# EUROPEAN PATENT SPECIFICATION

(11) **EP 2 670 861 B1**
(45) Date of publication and mention of the grant of the patent: **13.09.2017**
(21) Application number: 12702032.9
(22) Date of filing: 01.02.2012
(51) Int. Cl.: C12Q 1/68

(54) **MARKERS OF MELANOMA AND USES THEREOF**
MELANOMMARKER UND IHRE VERWENDUNG
MARQUEURS DE MÉLANOME ET LEURS UTILISATIONS

(30) Priority: 01.02.2011 EP 11152864
(43) Date of publication of application: 11.12.2013
(73) Proprietor: Azienda Ospedaliera Universitaria Senese, 53100 Siena (IT); Centro di Riferimento Oncologico - Istituto Nazionale Tumori - Aviano, 33081 Aviano (PN) (IT)
(72) Inventor: SIGALOTTI, Luca, 33085 Maniago (PN) (IT); MAIO, Michele, 33080 Roveredo in Piano (PN) (IT)
(74) Representative: Capasso, Olga
(86) International application number: PCT/EP2012/051663
(87) International publication number: WO 2012/104340

(56) References cited:
- WO-A2-2008/134596
- WO-A2-2009/086472
- US-A1- 2003 129 602
- LAHTZ CHRISTOPH ET AL: "Methylation of PTEN as a prognostic factor in malignant melanoma of the skin.", THE JOURNAL OF INVESTIGATIVE DERMATOLOGY FEB 2010 LNKD- PUBMED:19798057, vol. 130, no. 2, February 2010 (2010-02), pages 620-622, XP002636328, ISSN: 1523-1747
- TELLEZ CARMEN S ET AL: "CpG island methylation profiling in human melanoma cell lines", MELANOMA RESEARCH,, vol. 19, no. 3, 1 June 2009 (2009-06-01), pages 146-155, XP009148010, ISSN: 1473-5636, DOI: DOI:10.1097/CMR.0B013E32832B274E
- MORI TAKUJI ET AL: "Predictive utility of circulating methylated DNA in serum of melanoma patients receiving biochemotherapy.", JOURNAL OF CLINICAL ONCOLOGY : OFFICIAL JOURNAL OF THE AMERICAN SOCIETY OF CLINICAL ONCOLOGY 20 DEC 2005 LNKD- PUBMED:16361635, vol. 23, no. 36, 20 December 2005 (2005-12-20), pages 9351-9358, XP002636329, ISSN: 0732-183X
- JOHN THOMAS ET AL: "Predicting clinical outcome through molecular profiling in stage III melanoma.", CLINICAL CANCER RESEARCH : AN OFFICIAL JOURNAL OF THE AMERICAN ASSOCIATION FOR CANCER RESEARCH 15 AUG 2008 LNKD- PUBMED:18698035, vol. 14, no. 16, 15 August 2008 (2008-08-15), pages 5173-5180, XP002636330, ISSN: 1078-0432
- E. MERHAVI ET AL: "Promoter Methylation Status of Multiple Genes in Uveal Melanoma", INVESTIGATIVE OPHTHALMOLOGY & VISUAL SCIENCE, vol. 48, no. 10, 1 October 2007 (2007-10-01), pages 4403-4406, XP055023384, ISSN: 0146-0404, DOI: 10.1167/iovs.07-0272
- KORABIOWSKA MONIKA ET AL: "Analysis of adenomatous polyposis coli gene expression, APC locus-microsatellite instability and APC promoter methylation in the progression of melanocytic tumours", MODERN PATHOLOGY, NATURE PUBLISHING GROUP, US, vol. 17, no. 12, 1 December 2004 (2004-12-01), pages 1539-1544, XP002489097, ISSN: 0893-3952, DOI: 10.1038/MODPATHOL.3800238 [retrieved on 2004-07-16]
- HUNTER W. RICHARDS ET AL: "Epigenetic marks in melanoma", PIGMENT CELL & MELANOMA RESEARCH, vol. 22, no. 1, 1 February 2009 (2009-02-01), pages 14-29, XP055023516, ISSN: 1755-1471, DOI: 10.1111/j.1755-148X.2008.00534.x
- "Comprehensive DNA methylation analysis on the Illumina Infinium Assay Platform", Illumina Inc. , 25 March 2008 (2008-03-25), Retrieved from the Internet: URL:http://res.illumina.com/documents/prod ucts/appnotes/appnote_dna_methylation_anal ysis_infinium.pdf [retrieved on 2014-09-22]

## Description

### FIELD OF THE INVENTION

The present disclosure relates to the methylation of genomic DNA sequences, including *Long Interspersed Nucleotide Element-1 (LINE-1)* sequences and genome-wide gene methylation profiles. More specifically, the disclsoure relates to: i) the use of low levels of methylation of *LINE-1* sequences as a prognostic and predictive marker in human melanoma; ii) the use of multiple gene methylation signatures as prognostic and predictive markers in human melanoma; and iii) the use of combined *LINE-1* methylation and multiple gene methylation signatures as a further improved prognostic and predictive marker in human melanoma.

### BACKGROUND OF THE INVENTION

Cutaneous melanoma (CM) is characterized by a constantly growing incidence and high mortality rates in industrialized countries, representing the leading cause of skin cancer-related deaths worldwide (MacKie et al., 2009). Only surgery, in the initial phases of disease, may have a curative intent for CM patients; otherwise, advanced stage CM is characterized by a substantial unresponsiveness to conventional therapies (Bhatia et al., 2009). At present, the unique predictor of the 5-year survival of CM patients is the clinico-pathological staging of the disease, which defines overall survival (OS) rates ranging from 95% to 7% for stage I to IV patients, respectively (Balch et al., 2001; Balch et al., 2009). American Joint Committee on Cancer (AJCC) staging of CM identifies 4 stages of disease, stage I to stage IV, and respective substages, which are characterized by a progressively reduced survival of patients along with the increase in the stage of their disease (Balch et al., 2009). In detail, localized CM has no evidence of regional lymph node or distant metastasis, and is classified as stage I or II. Substaging reflects increased tumor thickness and presence of ulceration and/or mitoses, and identifies the following categories: stage IA, IB, IIA, IIB, IIC. Ten year survival rates range from 93% of stage IA to 39% of stage IIC patients. Regional metastatic CM is characterized by the absence of distant metastases, the presence of regional lymph node metastases and/or in transit/satellite metastases, and is classified as stage III. Substages IIIA to IIIC reflect an increasing involvement of regional lymph-nodes and/or in transit/satellite metastases. Stage IIIC is the more aggressive, with a 5 year survival rate of 40%, as compared to 59% and 78% of patients with stage IIIB and IIIA disease, respectively. Stage IV CM is the most advanced stage, characterized by the presence of distant metastases, and is substaged to M1a, M1b, M1c by the location of the metastases and the level of serum lactate dehydrogenase. One-year survival rates are 62% for M1a, 53% for M1b, and 33% for M1c CM.

CM patients within the same clinico-pathological staging category often behave extremely differently, and the current lack of established prognostic molecular markers greatly impairs physicians ability to identify CM patients who will evolve through a highly aggressive rather than through a more favorable course of disease (Jennings and Murphy, 2009). Thus, the definition of prognostic molecular markers that enable predicting survival of patients within the same clinico-pathological stage of disease is a mandatory requisite to boost the current clinical management of CM patients, providing physicians and patients with more efficient clinical, follow-up and treatment procedures.

In mammals, DNA methylation, occurring at 5C-position of cytosine in the context of CpG dinucleotides, represents a major epigenetic mechanism in controlling gene expression, chromosome X inactivation, imprinting and repression of endogenous parasitic sequences (Esteller, 2008). Alterations in the genomic DNA methylation patterns represent a hallmark of cancer, and include both global genomic DNA hypomethylation and gene-specific hypermethylation. Each of these apparently contrasting events are thought to play a major role in tumor development and progression.

Global genomic DNA hypomethylation (i.e., overall reduction of the 5-methylcytosine content) is a frequent molecular event in cancer and has been observed in neoplastic cells of different histotype (Gama-Sosa et al., 1983). Genomic hypomethylation might contribute to cancer development and progression through different mechanisms including generation of chromosomal instability, reactivation of transposable elements, and loss of imprinting (Esteller, 2008). Substantial decreases in the 5-methylcytosine content in the genome mainly reflect the hypomethylation of repetitive genomic sequences. Among these, the *Long Interspersed Nucleotide Element-1 (LINE-1)* family accounts for 17% of the human genomic DNA, is capable of transposition, and its methylation level may be utilized as a surrogate for the overall level of genomic DNA methylation (Yang et al., 2004). In accordance with the proposed role of genomic DNA hypomethylation in cancer biology, preliminary studies have reported *LINE-1* hypomethylation to be associated with a reduced overall (OS) and cancer-specific survival in patients with colorectal and ovarian cancer (Ogino et al., 2008; Pattamadilok et al., 2008). However, these studies did not investigate the role of *LINE-1* methylation as a prognostic factor in patients in specific stages of disease, but rather looked at the patients' population as a whole. Besides, no literature data is available on the prognostic role of *LINE-1* in CM.
In the application WO 2008/134596, the methylation status of *LINE-1* in breast, colorectal, esophageal and prostate cancer as well as in CM was investigated. No significant difference in *LINE-1* hypomethylation level was observed between primary CM and adjacent normal skin (Fig. 29), nor between different stages of disease other than between stage I and stage IV (Fig. 31). A significantly higher *LINE-1*hypomethylation of metastatic CM vs adjacent normal skin or primary CM (Fig. 30), and of stage IV CM vs primary CM or adjacent normal skin (Fig. 31) was observed. The application does not disclose any correlation between*LINE-1* methylation level and survival variables (OS, cancer-specific, or disease-free survival) in cancer patients. In addition, it does not provide neither suggest evidence of the existence of molecular markers able to predict different survivals of patients, in general, and, most importantly, of individuals within identical clinico-pathological stages of disease.Further, the data reported in WO 2008134596 would indicate that patients with low LINE1 methylation levels have a lower survival rate.

By contrast, the authors of the present disclosure invention examined the prognostic role of *LINE-1* methylation status on survival of CM patients, in particular at identical stages of the disease. The authors found that within identical clinico-pathological stage of disease (e.g., stage III or IIIC), CM patients which neoplastic cells have a higher *LINE-1* hypomethylation survive significantly longer, and a higher *LINE-1* hypomethylation level represents a positive, independent, prognostic factor for CM patients.

Besides the overall hypomethylation of genomic DNA discussed above, the aberrant gene-specific hypermethylation observed in human malignancies might dramatically impact their biology by affecting different key cellular pathways involved in cancer development and progression (Esteller, 2008). Along this line, initial evidences in CM have demonstrated that this malignancy is characterized by a profound epigenetic deregulation, characterized by the transcriptional silencing of over 50 genes through the aberrant hypermethylation of CpG islands located within their promoter regions (Sigalotti et al., 2010). The inactivation of these genes affects diverse cellular pathways including cellular proliferation/differentiation, apoptosis, DNA repair, extracellular matrix interaction, metastatization and immune recognition, and is likely to play a relevant role in CM development and progression (Sigalotti et al., 2010). Accordingly, initial studies, conducted on very small cohorts of patients, have examined the prognostic potential of the methylation status of single or limited panels of genes in CM, showing an association between the methylation of the Methylated IN Tumors locus 31 (MINT31) in tumor tissues and disease outcome in stage III patients, and a negative correlation between presence of circulating methylated DNA for RasAssociation (RalGDS/AF-6) domain Family member 1A (RASSF1A) and Estrogen Receptor alpha (ER-α) and survival of CM patients receiving biochemotherapy (Mori et al., 2006; Mori et al., 2005; Tanemura et al., 2009). Despite these initial evidences, no study has investigated the genome-wide gene methylation profile in CM patients and its prognostic/predictive potential. Thus, the authors have utilized a whole-genome approach to identify genes or gene signatures which methylation status has a prognostic/predictive role in CM, paying particular emphasis in identifying multi-gene methylation signatures able to discriminate survival of CM patients at identical stages of disease. With this approach the authors were able to define distinct methylation signatures that were able to sort stage III CM patients into categories with highly significantly different survival rates. Even more important, the gene methylation signatures the authors defined enable to identify patients with significantly different survival also in sub-satges of disease (e.g., stage IIIC), which currently represent the upper limit of our ability to segregate patients into groups characterized by different clinical outcomes using the tools that are at present available. In this context, the concomitant evaluation of both the extent of *LINE-1* methylation and of the gene-methylation signature(s), proved to have a further increased power to discriminate survival of patients as compared to each marker alone.

In consideration of such arguments, our invention has a significant industrial transferability to the clinical setting, where it will provide a key contribution in guiding the daily clinical management of CM patients.

### SUMMARY OF THE INVENTION

*LINE-1*sequences account for a substantial portion of the genome and are usually heavily methylated in normal somatic tissues. Reduced levels of *LINE-1* methylation (i.e. *LINE-1* hypomethylation) significantly contribute to the well-known global genomic DNA hypomethylation occurring in cancer tissues. Besides *LINE-1* hypomethylation, neoplastic tissues are characterized by a genome-wide alteration of gene methylation patterns that appears to profoundly contribute to the neoplastic phenotype. The inventors developed a method to predict survival in CM patients at identical stage of disease through the evaluation of: i) *LINE-1* methylation status; ii) multiple gene methylation signatures; iii) combined *LINE-1* methylation and multiple gene methylation signatures.

Assessment of neoplastic cells from CM patients within the same clinico-pathological stage of disease demonstrated a significant association between higher *LINE-1* hypomethylation and longer OS. *LINE-1* hypomethylation represents an independent factor predicting a more favorable prognosis of CM patients (Example 1). *LINE-1* hypomethylation can be used as a surrogate marker for the expression of different methylation regulated molecules, such as tumor-associated antigens, that are relevant for cancer therapy (Example 2).

Assessing with high-throughput approaches the genome-wide gene methylation status in neoplastic cells of CM patients at the same stage of disease, the authors have defined multi-gene methylation signatures that identify groups with significantly different OS within stage III and IIIC patient cohorts (Table 9, Example 3). In particular, the differential methylation pattern of: **i) signature A:** MGC29671, GJB5, KCNK16, IGLL1, ANXA8, OR12D3, TUB, CST11, TMEM10, CRHR1, TRIM40, GAS2L2, FOXN1, FXYD4, KRTHB2, FLJ33860, S100A9, FOXI1, GUCA2B, CYP2B6, IL8RA, CYP8B1, WFDC8, and SLPI genes identifies 2 classes of stage III patients with significantly different survivals; **ii) signature B:** FLJ25421, LAMC2, Clorf36, MGC29671, GJB5, KCNK16, ANGPTL3, IGLL1, DEFB4, STK38, SEMG2, ANXA8, OR12D3, PREPL, IL17F, DCLRE1A, FLJ20581, PTCD2, CX3CR1, TUB, UBE2E1, CST11, IFT140, TMEM10, SLC22A8, MLN, DIRC1, C1orf64, CRHR1, TRIM40, TNFSF13B, GAS2L2, FOXN1, CYP2B6, ACSL5, SERPINB12, APOBEC4, TNNI1, Clorf42, FXYD4, PPAT, PSD4, SCN4A, KRTHB2, IL8RB, UNC45B, Clorf201, SFTPG, LOC124751, FLJ33860, ANKRD1, FMO2, ARSG, GJB4, S100A9, LRRN5, MSMB, HLA-DOB, R3HDML, SPINK5, DLG5, PALLD, WFDC12, WDR20, FLJ32771, PKHD1, FOXI1, DEFB123, KRTHB3, GUCA2B, CYP2B6, BSND, NALP10, NR5A2, KRT25D, IL8RA, APOD, CLEC4F, THRB, KRTHB5, CYP8B1, WFDC8, SLC13A1, C3orf27, MGC35206, C3orf63, DARC, SLPI, SLC2A7, CPA3, MIS12, KRTHB4, MRVI1, MIA2, CYP2A7, ANGPT4, SLC44A5, and CST9L identifies 3 classes of stage III patients with significantly different survivals; **iii) signature C:** SLC18A2, GJB5, SLC6A18, IGLL1, ALOX12B, WNT10B, TUB, S100A9, CRHR1, TRIM40, SLC6A11, FLJ33860, FGF4, S100A9, MGC35206, PCDHAC2, and GRM4 identifies 2 classes of stage IIIC patients with significantly different survivals; **iv) signature D:** FLJ25421, ANXA13, SLC18A2, CTAG2, GJB5, KCNK16, SLC6A18, STK38, PVALB, OR12D3, ALOX12B, HAO2, DCLRE1A, WNT10B, TUB, UBE2E1, IFT140, SLC4A7, S100A9, DIRC1, CRHR1, TRIM40, SLC6A11, TDRD5, ACSL5, APOBEC4, FAAH, CD1D, Clorf201, FLJ33860, ANKRD1, FGF4, FLJ39501, S100A9, LRRN5, GLT1D1, IL28B, AVPR1B, GJB3, MGC35206, PCDHAC2, GABRG3, MIA2, CTAG1B, UNQ9433, GRM4, and CEACAM6 identifies 3 classes of stage IIIC patients with significantly different survivals. Furthermore, the concomitant evaluation of whole genome expression and methylation profiles allowed to identify 2 multi-gene methylation signatures of 51 and 73 genes that were constituted by the sole genes displaying a significant (p<0.05) correlation between expression and methylation. These latter signatures identify groups with significantly different OS within IIIC patient cohorts (Table 10, example 3). Specifically, the differential methylation pattern of: **i) signature E:** DENND2D, CTAG2, PGCP, KCNG1, FAM107B, FANCE, FAM38B, FOXF2, BGN, MAGEA10, SPATS1, PRRX1, MLSTD1, PDZD4, HCP5, OCIAD2, CSAG1, SLCO4A1, C9orf90, CSAG1, AMPH, MT1G, C20orf100, CST7, KIAA0367, RNASE1, HCP5, NDN, RAC2, AXL, BATF, EMILIN1, ARHGEF7, EVL, RAC2, KIAA0367, TFF3, CTSK, C21orf70, PRKCZ, MLSTD1, COL5A2, LY96, MAGEA1, MAGEC2, DENND2D, PPIE, SORBS2, PROCR, TMEPAI, SSNA1, and ZNF324 identifies 2 classes of stage IIIC patients with significantly different survivals; and **ii) signature F:** FLJ25421, DENND2D, CTAG2, PGCP, KCNG1, MGMT, FAM107B, FANCE, GAGE2, C11orf1, ABCB4, BGN, MAGEA10, SPATS1, CTNNAL1, PRRX1, EMP1, RNASE1, PDZD4, MAGEA6, CHMP4A, ADORA2B, HCP5, PLA1A, KIAA0141, OLFM1, ERO1L, CSAG1, SLCO4A1, CXorf12, C9orf90, CSAG1, AMPH, MT1G, ARHGDIB, C20orf100, CST7, KIAA0367, RNASE1, HCP5, NDN, RAC2, AXL, GRAMD3, ARSG, PLD1, HNMT, ZNF558, ATP6V1E2, EMILIN1, ARHGEF7, KRT25C, ZNF532, EVL, RAC2, SLAMF9, NBL1, KIAA0367, MAGEA10, TFF3, C21orf70, APOD, ABCA8, ACOX2, COL5A2, LY96, MAGEA1, MAGEC2, TIMP3, PPIE, RPUSD3, PROCR, and TMEPAI identifies 3 classes of stage IIIC patients with significantly different survivals. When, among the genes having significant (p<0.05) association between expression and methylation status, only those with a coefficient of correlation Rho<-0.4 were taken into account, an additional signature of 35 genes, **signature G:** PRDX2, DENND2D, CTAG2, PCOLCE, BGN, OCIAD2, RNASE1, MAGEA6, HCP5, OCIAD2, CSAG1, C9orf90, CSAG1, CTSK, ADM, RNASE1, HCP5, PAGE5, NNMT, EMILIN1, MAGEA3, RAB11FIP5, ARHGDIB, EMILIN1, LPXN, CTSK, ALDH1A3, SUHW1, ABCA8, COL5A2, LY96, MAGEA1, MAGEC2, GSTT1, DENND2D was defined, which identifies 2 classes of stage IIIC patients with significantly different survivals. The identified multi-gene methylation signatures represent independent factors predicting prognosis of CM patients.

In addition to the prognostic value of the multi-gene methylation signatures above described, the methylation status of specific genes taken from these signatures may also have a prognostic impact per se, as exemplified in Example 4. Similarly, "methylation scores", summarizing the above described genome-wide gene methylation profile of neoplastic cells, can also be utuilized to define prognosius of CM patients at the same stage of disease, see Example 5.

The extent of methylation of *LINE-1* sequences and/or the methylation pattern of the (multi-)gene methylation signatures can be used, alone, or in conjunction with other biomarkers, to predict prognosis and response to therapy of CM patients, thus enabling the administration of patient-tailored clinical, follow-up and therapeutic procedures.

Accordingly, the disclosure provides a method of prognosing CM or of predicting and monitoring the efficacy of a CM therapy, in patients at the same clinico-pathological stage of disease, comprising measuring *LINE-1* methylation and/or multi-gene methylation signatures in neoplastic cells. The term, "neoplastic cells" refers to any source of neoplastic cells from CM patients, including, but not limited to, primary cultures of autologous neoplastic cells, neoplastic cells purified from fresh autologous tumor tissues or from cryopreserved cell suspensions by immuno-sorting (e.g. using antibodies specific for cancer cells) or other means, cryopreserved tumor tissues, formalin fixed paraffin embedded (FFPE) tumor tissues, or other types of fixed tumor tissue, micro-/macro-dissected tumor tissues, circulating neoplastic cells present in body fluids (Tables 14-15, Example 7). Neoplastic cells may be obtained from a subject by any means known in the art.

As used herein, a "subject" is defined as a human. In a preferred embodiment the subject is a human with cancer. "Patient" refers to a human with cancer.

Methods for extracting genomic DNA are well-known in the art. Depending on the source of neoplastic cells, a preliminary step to remove embedding substances by organic solvents may be required. Classically, neoplastic cells from the subject are lysed using detergents and proteins are removed by different proteases. DNA is then purified from cell lysates either through extraction with organic solvents (e.g. phenol) or by affinity binding on silica-gel matrixes. DNA is then either precipitated with alcohols and dissolved in aqueous solutions, or washed with different solutions and eluted in aqueous solutions. Similar procedures are used to obtain genomic DNA from biologic fluids or circulating neoplastic cells.

The extent of methylation of *LINE-1* is then measured on the DNA extracted from neoplastic cells. Different methods can be used to determine the extent of methylation of *LINE-1,* and are well-known in the art. These include, but are not limited to, Methylight, bisulfite sequencing, bisulfite pyrosequencing, Combined Bisulfite Restriction Analysis (CoBRA). Level of methylation of *LINE-1* is typically defined as the ratio between the level of methylated *LINE-1* and the level of methylated or unmethylated *LINE-1,* but any other mathematical formula relating to the level of *LINE-1* methylation also applies. Furthermore, level of methylation of *LINE-1* may refer to the extent of methylation at a single or multiple CpG sites as well as to aggregating measurements of methylation of multiple CpG sites, including, but not limited to, mean, median, mode. Similarly, the methylation status of all or any of the genes included in the 7 methylation signatures is measured on the DNA extracted from neoplastic cells. Different methods can be used to determine the extent of methylation of the multigene methylation signatures, and are well-known in the art. These include, but are not limited to, microarray-based methylation analysis, Methylight, bisulfite sequencing, bisulfite pyrosequencing, CoBRA. Level of methylation of each sequence/gene included in the signature is typically defined as the ratio between the level of methylated target sequence and the level of methylated or unmethylated target sequence, but any other mathematical formula relating to the level of gene/sequence methylation also applies. Furthermore, level of methylation of any gene/sequence included in the signature may refer to the extent of methylation at a single or multiple CpG sites as well as to aggregating measurements of methylation of multiple CpG sites, including, but not limited to, mean, median, mode.

MethyLight is the quantitative implementation of Methylation Specific PCR (MSP) using real-time PCR technology. MSP is a PCR-based technique that amplifies the target sequence depending on its methylation status. The approach requires an initial chemical modification of genomic DNA by incubation with sodium bisulfite, which converts unmethylated cytosines (C) to uracil (U), leaving methylated ones unaltered. This methylation-specific difference in the primary sequence generated by treatment is exploited to design oligonucleotide primers specific for either methylated or unmethylated target DNA, which are then employed in PCR-amplification reactions to discriminate between methylated and unmethylated sequences. Performing MSP reactions through real-time quantitative PCR approaches allows an efficient and sensitive quantification of the extent of methylation of the target sequence (MethyLight). Furthermore, when performing MethyLight using fluorogenic hybridization probe approaches (e.g., TaqMan® technology), the quantitative discrimination between methylated and unmethylated sequences may rely on selectivity for different methylation status of primers, probe or both.

Bisulfite sequencing allows to determine the methylation status of each CpG dinucleotide in the target sequence by conducting standard sequencing analysis. The technique relies on the differences in the sequence between methylated and unmethylated DNA that are generated by bisulfite treatment. The sequence of interest is PCR-amplified typically with primers amplifying all states, irrespective of methylation status. Usually, PCR products are cloned into plasmid vectors and single colonies are subjected to DNA sequencing through different methodologies available in the art. The extent of methylation at each CpG site is then typically established by evaluating the percentage of colonies with methylated CpG (i.e., C base in sense strand sequence).

Bisulfite pyrosequencing. Pyrosequencing is a high-throughput nonelectrophoretic nucleotide extensionsequencing technology for various applications includingDNA methylation analysis. Pyrosequencing-based methylation analysis investigates quantitatively the degree of methylation at CpG positions after treatment of genomic DNA with sodium bisulfite. The target region is amplified by PCR using a pair of primers complementary to the bisulfite-treated DNA sequence, amplifying all states, irrespective of methylation status. Usually, one of these two amplification primers carries a biotin label at the 5'-end that is exploited to subsequently purify the PCR product by immobilization on streptavidin-coated sepharose beads. Immobilized PCR product is then rendered single-stranded by alkali and hybridized to a pyrosequencing primer complementary to a region in close proximity to the sequence for which methylation data is going to be analyzed. The pyrosequencing reaction is performed by the sequential addition of single nucleotides in a predefined order. Each incorporation event is accompanied by release of pyrophosphate (PPi) in a quantity equimolar to the amount of nucleotide incorporated. ATP sulfurylase quantitatively converts PPi to ATP in the presence of adenosine 5' phosphosulfate. ATP then drives the luciferase-mediated conversion of luciferin to oxyluciferin that generates visible light in amounts that are proportional to the amount of ATP. Light emission is detected by an instrument and reported as a peak which area represents the quantity of the respective nucleotide. The level of methylation at each CpG position in a sequence is usually determined from the ratio of C over the sum of C and T.

CoBRA is a technique that enables quantitative evaluation of DNA methylation by exploiting the sequence differences that exist between methylated and unmethylated DNA upon treatment with sodium bisulfite. In particular, it relies on the generation, or suppression, of a recognition site for restriction enzymes depending on the methylation status of the sequence under analysis. The target region is amplified by PCR using a pair of primers complementary to the bisulfite-treated DNA sequence, amplifying all states, irrespective of methylation status. After amplification, PCR products are digested with a specific restriction enzyme that cuts only DNA molecules that were methylated (or unmethyated) before bisulfite treatment. Digestion products are then separated by conventional, microfluidic or capillary electrophoresis, and the proportion of digested versus undigested products is quantitated and used to determine the extent of methylation at that particular site.

High-throughput DNA microarray technologies can be efficiently used to define genome-wide gene methylation profiles. Different methodologies either rely on the enrichment of genomic DNA for methylated sequences, through methylated DNA immunoprecipitation (MeDIP) approaches, or on the modification of the genomic DNA by sodium bisulfite. MeDIP can be conducted utilizing either anti-5-methylcytosine antibodies or proteins which selectively bind to methylated DNA (e.g., methyl-CpG-binding domain (MBD) proteins). Capturing of the genomic DNA with these reagents bound to an insoluble matrix allows to enrich the sample for methylated sequences. Classically, for each test sample, genomic DNA immunoprecipitated (IP) and DNA non-IP, utilized as control, are labelled with different fluorescently-coupled nucleotides (e.g., Cy5-dCTP for IP DNA and Cy3-dCTP for non-IP DNA), and co-hybridized to a microarray containing the probes for the sequence set of interest (e.g., CpG island arrays representing over 27,000 CpG sites distributed genome-wide). Fluorescence intensity data are then extracted from the scanned microarrays, pre-processed and analyzed using different software packages to provide a methylation score for each analyzed CpG site. On the other hand, the treatment of genomic DNA with sodium bisulfite translates the methylation data into primary DNA sequence modifications that are interrogated using oligonuclotide probes. Typically, bisulfite-modified genomic DNA is evaluated at each locus through hybridization of a pair of oligonuclotide probes constituted by one probe specific for the methylated and one specific for the unmethylated target sequence. Probes can be elongated by a polymerase only in case of match, so that probes for methylated or unmethylated sequences are quantitatively elongated only in the presence of methylated or unmethylated sequences in the test genomic DNA, respectively. A classic application employs single-base probe extension using fluorescently-labelled nucleotides to reveal the methylation status at each locus. The level of methylation for the interrogated locus can be determined by calculating the ratio of the fluorescent signals from the methylated vs unmethylated probes. In a typical application, this technology enables to interrogate the genome-wide methylation status of over 27,000 CpG sites covering over 14,000 genes. Besides microarray technology, the identity of methylated sequences can also be determined by applying next generation sequencing approaches to the MeDIP-enriched or the bisulfite-modified genomic DNA. The identification of methylation profiles (multi-gene methylation signatures) specifically associated with different prognosis (e.g., OS, time to progression (TTP), CM-specific survival) of patients may be obtained through different approaches including, but not limited to, that illustrated in Example 3.

The invention provides a method for determining prognosis (including, but not limited to, OS, disease- and progression-free survival, CM-specific survival) of subjects with CM within a clinico-pathological stage of disease. In one such method, in a neoplastic cell sample obtained from a subject the level of *LINE-1* methylation is determined. If the level of *LINE-1* methylation in the sample is lower than a reference value, the subject survives longer. On the contrary, if the level of *LINE-1* methylation in the sample is higher than a reference value, the subject survives less. The reference value of *LINE-1* methylation is determined in a test population of subjects with cancer as the value that more specifically and sensitively discriminates patients with different survivals. This reference value can be defined through any approach available in the art, including, but not limited to, receiver-operator characteristic (ROC) curve analysis, mean, median, mode value of *LINE-1* methylation of neoplastic cells from the population of cancer patients under evaluation. In an additional method for determining prognosis, a neoplastic cell sample is obtained from a subject affected by CM, and the methylation profile of any, multiple or all of the genes included in the multi-gene methylation signatures (Table 3) is evaluated. The methylation profile obtained from the neoplastic cells of the patient is then fed into a methylation-based classifier algorithm that will assign patients at the same stage of disease into either a high- or a low-risk group. Patients assigned to the high-risk group based on their (multi-)gene methylation profile will survive less, while patients assigned to the low-risk group will survive longer. Furthermore, combining both *LINE-1* methylation and gene methylation signature classifications of patients results in a stronger prognostic marker, which, for instance, predicts that patients whose neoplastic cells have *LINE-1* methylation lower than a reference value and a low-risk methylation signature have a longer survival (Example 6).

In another method of determining prognosis, in a biological fluid sample obtained from a subject *LINE-1* methylation is evaluated and/or (multi-)gene methylation signatures. A "biological fluid" refers to any fluid present in the subject in which acellular DNA or cancer cells may be present, including, without limitation, blood, serum, plasma, bone marrow, cerebrospinal fluid, peritoneal/pleural fluid, lymph fluid, ascitic, serous fluid, sputum, lacrimal fluid, stool, and urine. Body fluid samples can be obtained from a subject using any of the methods known in the art.

Accordingly to the above reported role of *LINE-1* methylation level and of (multi-) gene methylation signatures as prognostic markers, the invention includes the measurement and use of *LINE-1* methylation and/or of (multi-)gene methylation signatures to define differentiated therapeutic treatments and differentiated follow-up procedures and schedules for CM patients, according to their different prognosis as defined by *LINE-1* methylation and/or (multi-)gene methylation signatures.

The disclosure further provides *LINE-1* methylation level as a surrogate marker of presence and levels of expression in neoplastic cells of different therapeutic targets regulated by DNA methylation, including, but not limited to, Cancer Testis Antigens of the MAGE, GAGE, NY-ESO, SSX families, High Molecular Weight-Melanoma Associated Antigen, class I and II Human Leukocyte Antigens, and Intercellular Adhesion Molecule-1. In such a method, a neoplastic cell sample is obtained from a subject/patient and the level of *LINE-1* methylation is determined. If the level of *LINE-1* methylation in the sample is lower than a reference value, neoplastic cells are likely to express a higher number of distinct methylation-regulated therapeutic targets and/or increased levels of their expression. As a consequence, this population of patients is most likely to respond to any therapeutic treatment (including, but not limited to, chemotherapy, immunotherapy, biologic therapy, radiotherapy and targeted therapy utilized alone or in combination) involving methylation-regulated molecules, and thus it represents the preferred cohort of individuals to be treated with these therapies utilizing a patient-tailored approach. Accordingly, the disclosure includes the use of the evaluation of the extent of *LINE-1* methylation to identify patients to be treated with therapeutic approaches targeting one or multiple DNA methylation-regulated molecules, including, but not limited to, Cancer Testis Antigens of the MAGE, GAGE, NY-ESO, SSX families, High Molecular Weight-Melanoma Associated Antigen, class I and II Human Leukocyte Antigens, and Intercellular Adhesion Molecule-1.

The invention also provides a method for predicting response to therapy. In such a method, in a neoplastic cell sample obtained from a subject/patient the level of *LINE-1* methylation and/or the (multi-)gene methylation signatures are determined. The level of *LINE-1* methylation and/or the (multi-)gene methylation signature is associated with the fact that the patient is more likely to respond better to different therapeutic approaches, including, but not limited to, immunotherapy, chemotherapy, radiotherapy, biologic therapy, targeted therapy utilized alone or in combination. Accordingly, the disclosure includes the use of the level of *LINE-1* methylation and or of (multi-)gene methylation signatures for selecting patients to be treated with specific therapeutic approaches.

Patients whose neoplastic cells have a level of *LINE-1* methylation lower than a reference value have a more favorable prognosis, surviving longer. Similarly, patients whose neoplastic cells have more hypomethylated multi-gene methylation signatures (see Examples 3 and 5) are characterized by a longer survival. Accordingly, one aspect of the disclosure includes the administration of compounds able to reduce the extent of methylation of genomic DNA (DNA hypomethylating agents, DHA) in cancer patients whose neoplastic cells have levels of *LINE-1* methylation higher than a reference value and/or hypermethylated (multi-) gene methylation signatures. The hypomethylation of genomic DNA, of *LINE-1* DNA, and of the genes included in the methylation signatures in neoplastic cells of these treated patients is expected to drive cells to a less aggressive phenotype and to provide patients with a longer survival. Treatment of patients may be done with DHA alone or in combination with any other therapeutic option available. In one, not limiting, embodiment, pharmacologic DNA hypomethylation could be used as a feasible chemoprevention approach in the initial phases of disease and/or in patients at high-risk of disease recurrence. It is therefore an object of the present invention an in vitro method of prognosing melanoma within a clinico-pathological stage thereof **and/or predicting** and/or monitoring the response to a melanoma therapy in a subject and/or to identify a subject to be treated with a DNA hypomethylating agent comprising determining the methylation status of at least one cytosine residue in a methylation relevant region of the genes ABCA8, ADM, ALDH1A3, ARHGDIB, BGN, C9orf90, COL5A2, CSAG1, CTAG2, CTSK, DENND2D, EMILIN1, GSTT1, HCP5, LPXN, LY96, MAGEA1, MAGEA3, MAGEA6, MAGEC2, NNMT, OCIAD2, PAGE5, PCOLCE, PRDX2, RAB11FIP5, RNASE1 and SUHW1 (signature G) and allelic variants thereof or the genes ABCA8, ABCB4, ACOX2, ADORA2B, AMPH, APOD, ARHGDIB, ARHGEF7, ARSG, ATP6V1E2, AXL, BGN, C11orf1, C20orf100, C21orf70, C9orf90, CHMP4A, COL5A2, CSAG1, CST7, CTAG2, CTNNAL1, CXorf12, DENND2D, EMILIN1, EMP1, ERO1L, EVL, FAM107B, FANCE, FLJ25421, GAGE2, GRAMD3, HCP5, HNMT, KCNG1, KIAA0141, KIAA0367, KRT25C, LY96, MAGEA1, MAGEA10, MAGEA6, MAGEC2, MGMT, MT1G, NBL1, NDN, OLFM1, PDZD4, PGCP, PLA1A, PLD1, PPIE, PROCR, PRRX1, RAC2, RNASE1, RPUSD3, SLAMF9, SLCO4A1, SPATS1, TFF3, TIMP3, TMEPAI, ZNF532 and ZNF558 (signature F) or alleleic variants thereof in a biological sample obtained from the subject.

Preferably for each of said genes, the methylation relevant regions are comprised in the nucleotide sequences as indicated in Table 3 signature G, or in complementary sequences thereof, more preferably for each of said genes, the methylation relevant regions consist of: nt 940 to nt 1061 of SEQ ID No. 109, nt 940 to nt 1061 of SEQ ID No. 125, nt 940 to nt 1061 of SEQ ID No. 132, nt 940 to nt 1061 of SEQ ID No. 138, nt 941 to nt 1062 of SEQ ID No. 139, nt 940 to nt 1061 of SEQ ID No. 140, nt 941 to nt 1062 of SEQ ID No. 142, nt 941 to nt 1062 of SEQ ID No. 143, nt 941 to nt 1062 of SEQ ID No. 149, nt 941 to nt 1062 of SEQ ID No. 150, nt 941 to nt 1062 of SEQ ID No. 155, nt 941 to nt 1062 of SEQ ID No. 161, nt 941 to nt 1062 of SEQ ID No. 165, nt 941 to nt 1062 of SEQ ID No. 166, nt 941 to nt 1062 of SEQ ID No. 167, nt 941 to nt 1062 of SEQ ID No. 168, nt 941 to nt 1062 of SEQ ID No. 169, nt 941 to nt 1062 of SEQ ID No. 181, nt 941 to nt 1062 of SEQ ID No. 182, nt 940 to nt 1061 of SEQ ID No. 201, nt 940 to nt 1061 of SEQ ID No. 205, nt 940 to nt 1061 of SEQ ID No. 206, nt 941 to nt 1062 of SEQ ID No. 207, nt 940 to nt 1061 of SEQ ID No. 208, nt 940 to nt 1061 of SEQ ID No. 209, nt 940 to nt 1061 of SEQ ID No. 210, nt 941 to nt 1062 of SEQ ID No. 211, nt 941 to nt 1062 of SEQ ID No. 212, nt 941 to nt 1062 of SEQ ID No. 213, nt 940 to nt 1061 of SEQ ID No. 214, nt 940 to nt 1061 of SEQ ID No. 215, nt 941 to nt 1062 of SEQ ID No. 216, nt 941 to nt 1062 of SEQ ID No. 217, nt 940 to nt 1061 of SEQ ID No. 218, nt 940 to nt 1061 of SEQ ID No. 219, an allelic variant or a complementary sequence thereof.

Preferably for each of said genes, the methylation relevant regions are comprised in the nucleotide sequences as indicated in Table 3 signature F, or in complementary sequences thereof, still preferably for each of said genes, the methylation relevant region consist of: nt 940 to nt 1061 of SEQ ID No. 25, nt 941 to nt 1062 of SEQ ID No. 61, nt 941 to nt 1062 of SEQ ID No. 80, nt 940 to nt 1061 of SEQ ID No. 109, nt 940 to nt 1061 of SEQ ID No. 125, nt 940 to nt 1061 of SEQ ID No. 126, nt 941 to nt 1062 of SEQ ID No. 127, nt 941 to nt 1062 of SEQ ID No. 128, nt 941 to nt 1062 of SEQ ID No. 129, nt 940 to nt 1061 of SEQ ID No. 132, nt 940 to nt 1061 of SEQ ID No. 133, nt 941 to nt 1062 of SEQ ID No. 134, nt 940 to nt 1061 of SEQ ID No. 135, nt 941 to nt 1062 of SEQ ID No. 137, nt 940 to nt 1061 of SEQ ID No. 138, nt 940 to nt 1061 of SEQ ID No. 140, nt 940 to nt 1061 of SEQ ID No. 141, nt 941 to nt 1062 of SEQ ID No. 142, nt 941 to nt 1062 of SEQ ID No. 143, nt 941 to nt 1062 of SEQ ID No. 144, nt 941 to nt 1062 of SEQ ID No. 145, nt 941 to nt 1062 of SEQ ID No. 146, nt 941 to nt 1062 of SEQ ID No. 147, nt 941 to nt 1062 of SEQ ID No. 148, nt 941 to nt 1062 of SEQ ID No. 149, nt 941 to nt 1062 of SEQ ID No. 150, nt 940 to nt 1061 of SEQ ID No. 151, nt 940 to nt 1061 of SEQ ID No. 152, nt 940 to nt 1061 of SEQ ID No. 153, nt 941 to nt 1062 of SEQ ID No. 155, nt 940 to nt 1061 of SEQ ID No. 156, nt 940 to nt 1061 of SEQ ID No. 157, nt 940 to nt 1061 of SEQ ID No. 158, nt 941 to nt 1062 of SEQ ID No. 159, nt 940 to nt 1061 of SEQ ID No. 160, nt 940 to nt 1061 of SEQ ID No. 162, nt 941 to nt 1062 of SEQ ID No. 165, nt 941 to nt 1062 of SEQ ID No. 166, nt 941 to nt 1062 of SEQ ID No. 167, nt 941 to nt 1062 of SEQ ID No. 168, nt 940 to nt 1061 of SEQ ID No. 170, nt 941 to nt 1062 of SEQ ID No. 172, nt 940 to nt 1061 of SEQ ID No. 173, nt 941 to nt 1062 of SEQ ID No. 175, nt 941 to nt 1062 of SEQ ID No. 176, nt 941 to nt 1062 of SEQ ID No. 177, nt 940 to nt 1061 of SEQ ID No. 178, nt 941 to nt 1062 of SEQ ID No. 179, nt 941 to nt 1062 of SEQ ID No. 180, nt 941 to nt 1062 of SEQ ID No. 181, nt 941 to nt 1062 of SEQ ID No. 182, nt 940 to nt 1061 of SEQ ID No. 183, nt 940 to nt 1061 of SEQ ID No. 184, nt 940 to nt 1061 of SEQ ID No. 185, nt 940 to nt 1061 of SEQ ID No. 186, nt 940 to nt 1061 of SEQ ID No. 187, nt 940 to nt 1061 of SEQ ID No. 188, nt 940 to nt 1061 of SEQ ID No. 189, nt 940 to nt 1061 of SEQ ID No. 190, nt 941 to nt 1062 of SEQ ID No. 191, nt 940 to nt 1061 of SEQ ID No. 192, nt 940 to nt 1061 of SEQ ID No. 193, nt 941 to nt 1062 of SEQ ID No. 194, nt 940 to nt 1061 of SEQ ID No. 195, nt 940 to nt 1061 of SEQ ID No. 196, nt 941 to nt 1062 of SEQ ID No. 197, nt 941 to nt 1062 of SEQ ID No. 198, nt 941 to nt 1062 of SEQ ID No. 199, nt 940 to nt 1061 of SEQ ID No. 200, nt 940 to nt 1061 of SEQ ID No. 201, nt 940 to nt 1061 of SEQ ID No. 202, nt 940 to nt 1061 of SEQ ID No. 203, nt 940 to nt 1061 of SEQ ID No. 204, an allelic variant or a complementary sequence thereof.

Yet preferably the method of the invention further comprises determining the methylation status of at least one cytosine residue in the methylation relevant region of the *LINE-*1 repetitive sequence as in SEQ ID No. 220. Yet preferably the method of the invention further comprises determining the methylation status of at least one cytosine residue in a methylation relevant region of:
a] at least one of the following genes: *ALOX12B, FGF4, IGLL1, OCIAD2, PAGE5, S100A9, SLC6A11*, *SLC6A18, TUB, BATF, CRHR1, CTSK, FLJ33860, GJB5, GRM4, MAGEA10, MGC35206, MLSTD1, SLC18A2, SORBS2,* and *TRIM40*;

Preferably the melanoma is a cutaneous melanoma, preferably at stage III or IIIC.

Still preferably the method of the invention comprises the steps of:
(a) isolating DNA from a sample obtained from a subject;
(b) subjecting said DNA to methylation-sensitive sequence modification through incubation with suitable chemicals, or contacting said DNA with a reagent capable of selectively binding to methylated DNA;
(c) optionally amplifying said DNA as obtained in b), and
(d) determining the methylation status of said DNA.

It is a further object of the invention a kit for performing the method of the invention, comprising a chemical able to differentially modify DNA sequence depending on methylation status, or a reagent capable of selelctively binding to methylated DNA, and probes, primers and/or aptamers specific for each of genes or **repetitive sequence** as defined above.

Preferably the kit is in the form of an array. It may also be in the form of a microarray or microchip.

In the present invention, 6 genes are common between signatures A, B, C and D (Table 1) and 14 genes are common between signatures E, F and G (Table 2).

**Table 1: Probes/sequences/genes shared among signatures A,B,C,D**

| GeneSymbol | AccessionNumber | GeneID | IlmnID |
|---|---|---|---|
| GJB5 | NM_005268.2 | 2709 | cg01333788 |
| TUB | NM_177972.1 | 7275 | cg05492113 |
| CRHR1 | NM_004382.2 | 1394 | cg08929103 |
| TRIM40 | NM_138700.2 | 135644 | cg09196959 |
| FU33860 | NM_173644.1 | 284756 | cg14236389 |
| S100A9 | NM_002965.2 | 6280 | cg16139316 |

**Table 2: Probes/sequences/genes shared among signatures E,F,G**

| **GeneSymbol** | **AccessionNumber** | **GeneID** | **IlmnID** |
|---|---|---|---|
| DENND2D | NM_024901.3 | 79961 | cg00619207 |
| CTAG2 | NM_020994.2 | 30848 | cg01325515 |
| BGN | NM_001711.3 | 633 | cg04929865 |
| HCP5 | NM_006674.2 | 10866 | cg07971188 |
| CSAG1 | NM_153478.1 | 158511 | cg08977028 |
| C9orf90 | NM_197956.1 | 203245 | cg09296212 |
| CSAG1 | NM_153478.1 | 158511 | cg09917461 |
| RNASE1 | NM_198232.1 | 6035 | cg13718960 |
| HCP5 | NM_006674.2 | 10866 | cg13784557 |
| EMILIN1 | NM_007046.1 | 11117 | cg15895197 |
| COL5A2 | NM_000393.2 | 1290 | cg22774472 |
| LY96 | NM_015364.2 | 23643 | cg23732024 |
| MAGEA1 | NM_004988.3 | 4100 | cg23776892 |
| MAGEC2 | NM_016249.2 | 51438 | cg23813564 |

In the present invention, the "Methylation status" refers to the presence, absence and/or quantity of methylation at a particular nucleotide, or nucleotides within a portion of DNA.

The methylation status of a particular DNA sequence (e.g., a methylation relevant region as described herein) can indicate the methylation state of every base in the sequence or can indicate the methylation state of a subset of the base pairs (e.g., of cytosines or the methylation state of one or more specific restriction enzyme recognition sequences) within the sequence, or can indicate information regarding regional methylation density within the sequence without providing precise information of where in the sequence the methylation occurs. The methylation status can optionally be represented or indicated by a "methylation value." A methylation value can be generated, for example, by quantifying the amount of methylated DNA by quantitative MSP. In this particular example, distinct primer pairs can be utilized to selectively amplify methylated or unmethylated target sequence. The amount of methylated and unmethylated DNA could be measured, for instance, by extrapolation of the values (e.g., the n. of PCR cycles at which the quantity of amplification product reaches a particular threshold level) obtained for the test sample against standard curves generated with different known amounts of methylated and unmethylated target sequences. Accordingly, a value, i.e., a methylation value, for example from the above described example, represents the methylation status and can thus be used as a quantitative indicator of methylation status. This is of particular use when it is desirable to compare the methylation status of a sequence in a sample to a threshold value.

The "methylation relevant region" is defined as a region containing CpG dinucleotides potential targets for DNA methylation which may extend from over 5000 bases 5' to over 5000 bases 3' respective to the putative transcription start site of the gene, and may include gene promoter(s), enhancer(s), exonic and intronic sequences.

As used in the present invention, the term "biologic sample" refers to any sample taken from a subject or derived from it, including, without limitation, any source of neoplastic cells from CM patients, such as primary cultures of autologous neoplastic cells, neoplastic cells purified from fresh autologous tumor tissues or from cryopreserved cell suspensions by immuno-sorting (e.g. using antibodies specific for cancer cells) or other means, cryopreserved tumor tissues, FFPE tumor tissues, or other types of fixed tumor tissue, micro-/macro-dissected tumor tissues, circulating neoplastic cells present in body fluids. The term further applies to biological fluids, including, without limitation, any fluid present in the subject in which acellular DNA or cancer cells may be present, such as blood, serum, plasma, bone marrow, cerebrospinal fluid, peritoneal/pleural fluid, lymph fluid, ascitic, serous fluid, sputum, lacrimal fluid, stool, and urine. Biological samples can be obtained from a subject using any of the methods known in the art.

**Table 3:** Signatures A, B, C, D, E, F and G associated with their SEQ ID No. to be analyzed for methylation status. * GeneBank accession number, † Illumina unique gene identifyer, § Illumina unique probe identifier that defines the exact CpG dinucleotide that is evaluated.

**Signature A**

| **Seq ID no**. | **Gene Symbol** | **Accession N. *** | **Gene ID†** | **IlmnID§** |
|---|---|---|---|---|
| 1 | MGC29671 | NM_182538.3 | 201305 | cg01138020 |
| 2 | GJB5 | NM_005268.2 | 2709 | cg01333788 |
| 3 | KCNK16 | NM_032115.2 | 83795 | cg01669948 |
| 4 | IGLL1 | NM_020070.2 | 3543 | cg02415431 |
| 5 | ANXA8 | NM_001630.1 | 244 | cg03399459 |
| 6 | OR12D3 | NM_030959.2 | 81797 | cg03608577 |
| 7 | TUB | NM_177972.1 | 7275 | cg05492113 |
| 8 | CST11 | NM_080830.2 | 140880 | cg06489008 |
| 9 | TMEM10 | NM_033207.2 | 93377 | cg06825166 |
| 10 | CRHR1 | NM_004382.2 | 1394 | cg08929103 |
| 11 | TRIM40 | NM_138700.2 | 135644 | cg09196959 |
| 12 | GAS2L2 | NM_139285.1 | 246176 | cg10016608 |
| 13 | FOXN1 | NM_003593.2 | 8456 | cg10213812 |
| 14 | FXYD4 | NM_173160.2 | 53828 | cg12619162 |
| 15 | KRTHB2 | NM_033033.3 | 3888 | cg13696887 |
| 16 | FU33860 | NM_173644.1 | 284756 | cg14236389 |
| 17 | S100A9 | NM_002965.2 | 6280 | cg16139316 |
| 18 | FOXI1 | NM_012188.3 | 2299 | cg19233472 |
| 19 | GUCA2B | NM_007102.1 | 2981 | cg19728577 |
| 20 | CYP2B6 | NM_000767.4 | 1555 | cg19756068 |
| 21 | IL8RA | NM_000634.2 | 3577 | cg21004129 |
| 22 | CYP8B1 | NM_004391.1 | 1582 | cg21979032 |
| 23 | WFDC8 | NM_181510.1 | 90199 | cg22021786 |
| 24 | SLPI | NM_003064.2 | 6590 | cg23889010 |

**Signature B**

| **Seq ID no.** | **Gene Symbol** | **Accession N. *** | **Gene ID†** | **IlmnID§** |
|---|---|---|---|---|
| 25 | FU25421 | NM_152512.3 | 150350 | cg00044729 |
| 26 | LAMC2 | NM_005562.1 | 3918 | cg00208830 |
| 27 | C1orf36 | NM_183059.1 | 343035 | cg00658007 |
| 1 | MGC29671 | NM_182538.3 | 201305 | cg01138020 |
| 2 | GJB5 | NM_005268.2 | 2709 | cg01333788 |
| 3 | KCNK16 | NM_032115.2 | 83795 | cg01669948 |
| 28 | ANGPTL3 | NM_014495.2 | 27329 | cg02218214 |
| 4 | IGLL1 | NM_020070.2 | 3543 | cg02415431 |
| 29 | DEFB4 | NM_004942.2 | 1673 | cg02658251 |
| 30 | STK38 | NM_007271.2 | 11329 | cg02964385 |
| 31 | SEMG2 | NM_003008.2 | 6407 | cg03312792 |
| 5 | ANXA8 | NM_001630.1 | 244 | cg03399459 |
| 6 | OR12D3 | NM_030959.2 | 81797 | cg03608577 |
| 32 | PREPL | NM_006036.2 | 9581 | cg03894103 |
| 33 | IL17F | NM_052872.3 | 112744 | cg04063348 |
| 34 | DCLRE1A | NM_014881.2 | 9937 | cg04281204 |
| 35 | FU20581 | NM_017888.2 | 54988 | cg04428453 |
| 36 | PTCD2 | NM_024754.2 | 79810 | cg04527989 |
| 37 | CX3CR1 | NM_001337.3 | 1524 | cg04569233 |
| 7 | TUB | NM_177972.1 | 7275 | cg05492113 |
| 38 | UBE2E1 | NM_182666.1 | 7324 | cg06282059 |
| 8 | CST11 | NM_080830.2 | 140880 | cg06489008 |
| 39 | IFT140 | NM_014714.2 | 9742 | cg06730286 |
| 9 | TMEM10 | NM_033207.2 | 93377 | cg06825166 |
| 40 | SLC22A8 | NM_004254.2 | 9376 | cg06917325 |
| 41 | MLN | NM_002418.1 | 4295 | cg08332212 |
| 42 | DIRC1 | NM_052952.1 | 116093 | cg08654334 |
| 43 | C1orf64 | NM_178840.2 | 149563 | cg08887581 |
| 10 | CRHR1 | NM_004382.2 | 1394 | cg08929103 |
| 11 | TRIM40 | NM_138700.2 | 135644 | cg09196959 |
| 44 | TNFSF13B | NM_006573.3 | 10673 | cg09646392 |
| 12 | GAS2L2 | NM_139285.1 | 246176 | cg10016608 |
| 13 | FOXN1 | NM_003593.2 | 8456 | cg10213812 |
| 45 | CYP2B6 | NM_000767.4 | 1555 | cg10322876 |
| 46 | ACSL5 | NM_016234.3 | 51703 | cg11152574 |
| 47 | SERPINB12 | NM_080474.1 | 89777 | cg11435943 |
| 48 | APOBEC4 | NM_203454.1 | 403314 | cg11505048 |
| 49 | TNNI1 | NM_003281.3 | 7135 | cg12114524 |
| 50 | Clorf42 | NM_019060.1 | 54544 | cgl2588301 |
| 14 | FXYD4 | NM_173160.2 | 53828 | cg12619162 |
| 51 | PPAT | NM_002703.3 | 5471 | cg13086586 |
| 52 | PSD4 | NM_012455.2 | 23550 | cg13273136 |
| 53 | SCN4A | NM_000334.3 | 6329 | cg13694749 |
| 15 | KRTHB2 | NM_033033.3 | 3888 | cg13696887 |
| 54 | IL8RB | NM_001557.2 | 3579 | cg13739417 |
| 55 | UNC45B | NM_173167.2 | 146862 | cg13859324 |
| 56 | Clorf201 | NM_178122.2 | 90529 | cg13980834 |
| 57 | SFTPG | NM_205854.1 | 389376 | cg14034870 |
| 58 | LOC124751 | NM_213597.1 | 124751 | cg14069619 |
| 16 | FU33860 | NM_173644.1 | 284756 | cg14236389 |
| 59 | ANKRD1 | NM_014391.2 | 27063 | cg14558138 |
| 60 | FMO2 | NM_001460.2 | 2327 | cg14721213 |
| 61 | ARSG | NM_014960.2 | 22901 | cg15308737 |
| 62 | GJB4 | NM_153212.1 | 127534 | cg15554401 |
| 17 | S100A9 | NM_002965.2 | 6280 | cg16139316 |
| 63 | LRRN5 | NM_006338.2 | 10446 | cg16456919 |
| 64 | MSMB | NM_002443.2 | 4477 | cg17030820 |
| 65 | HLA-DOB | NM_002120.2 | 3112 | cg17103109 |
| 66 | R3HDML | NM_178491.2 | 140902 | cg17692403 |
| 67 | SPINK5 | NM_006846.2 | 11005 | cg17788013 |
| 68 | DLG5 | NM_004747.2 | 9231 | cg17791617 |
| 69 | PALLD | NM_016081.2 | 23022 | cg17925436 |
| 70 | WFDC12 | NM_080869.1 | 128488 | cg18241647 |
| 71 | WDR20 | NM_181291.1 | 91833 | cg18412984 |
| 72 | FU32771 | NM_145017.1 | 220004 | cg18432384 |
| 73 | PKHD1 | NM_138694.2 | 5314 | cg18885346 |
| 18 | FOXI1 | NM_012188.3 | 2299 | cg19233472 |
| 74 | DEFB123 | NM_153324.2 | 245936 | cg19241311 |
| 75 | KRTHB3 | NM_002282.2 | 3889 | cg19258973 |
| 19 | GUCA2B | NM_007102.1 | 2981 | cg19728577 |
| 20 | CYP2B6 | NM_000767.4 | 1555 | cg19756068 |
| 76 | BSND | NM_057176.2 | 7809 | cg19971655 |
| 77 | NALP10 | NM_176821.2 | 338322 | cg20311730 |
| 78 | NR5A2 | NM_003822.3 | 2494 | cg20406878 |
| 79 | KRT25D | NM_181535.2 | 162605 | cg20484002 |
| 21 | IL8RA | NM_000634.2 | 3577 | cg21004129 |
| 80 | APOD | NM_001647.2 | 347 | cg21084260 |
| 81 | CLEC4F | NM_173535.2 | 165530 | cg21148892 |
| 82 | THRB | NM_000461.2 | 7068 | cg21303011 |
| 83 | KRTHB5 | NM_002283.2 | 3891 | cg21518208 |
| 22 | CYP8B1 | NM_004391.1 | 1582 | cg21979032 |
| 23 | WFDC8 | NM_181510.1 | 90199 | cg22021786 |
| 84 | SLC13A1 | NM_022444.3 | 6561 | cg22024657 |
| 85 | C3orf27 | NM_007354.1 | 23434 | cg22068211 |
| 86 | MGC35206 | NM_178552.2 | 339669 | cg22088368 |
| 87 | C3orf63 | NM_015224.1 | 23272 | cg22332306 |
| 88 | DARC | NM_002036.2 | 2532 | cg23507131 |
| 24 | SLPI | NM_003064.2 | 6590 | cg23889010 |
| 89 | SLC2A7 | NM_207420.1 | 155184 | cg24027679 |
| 90 | CPA3 | NM_001870.1 | 1359 | cg24290574 |
| 91 | MIS12 | NM_024039.1 | 79003 | cg24445405 |
| 92 | KRTHB4 | NM_033045.2 | 3890 | cg24461814 |
| 93 | MRVI1 | NM_130385.1 | 10335 | cg24541550 |
| 94 | MIA2 | NM_054024.3 | 117153 | cg24603941 |
| 95 | CYP2A7 | NM_030589.2 | 1549 | cg25427638 |
| 96 | ANGPT4 | NM_015985.2 | 51378 | cg26540515 |
| 97 | SLC44A5 | NM_152697.2 | 204962 | cg27336379 |
| 98 | CST9L | NM_080610.1 | 128821 | cg27655855 |

**Signature C**

| **Seq ID no.** | **Gene Symbol** | **Accession N. *** | **Gene ID†** | **IlmnID§** |
|---|---|---|---|---|
| 99 | SLC18A2 | NM_003054.2 | 6571 | cg00498305 |
| 2 | GJB5 | NM_005268.2 | 2709 | cg01333788 |
| 100 | SLC6A18 | NM_182632.1 | 348932 | cg02064402 |
| 4 | IGLL1 | NM_020070.2 | 3543 | cg02415431 |
| 101 | ALOX12B | NM_001139.1 | 242 | cg03742272 |
| 102 | WNT10B | NM_003394.2 | 7480 | cg05164634 |
| 7 | TUB | NM_177972.1 | 7275 | cg05492113 |
| 103 | S100A9 | NM_002965.2 | 6280 | cg07039113 |
| 10 | CRHR1 | NM_004382.2 | 1394 | cg08929103 |
| 11 | TRIM40 | NM_138700.2 | 135644 | cg09196959 |
| 104 | SLC6A11 | NM_014229.1 | 6538 | cg09395732 |
| 16 | FU33860 | NM_173644.1 | 284756 | cg14236389 |
| 105 | FGF4 | NM_002007.1 | 2249 | cg14578030 |
| 17 | S100A9 | NM_002965.2 | 6280 | cg16139316 |
| 86 | MGC35206 | NM_178552.2 | 339669 | cg22088368 |
| 106 | PCDHAC2 | NM_031883.2 | 56134 | cg24076884 |
| 107 | GRM4 | NM_000841.1 | 2914 | cg26424956 |

**Signature D**

| **Seq ID no.** | **Gene Symbol** | **Accession N. *** | **Gene ID†** | **ILmnID§** |
|---|---|---|---|---|
| 25 | FU25421 | NM_152512.3 | 150350 | cg00044729 |
| 108 | ANXA13 | NM_004306.2 | 312 | cg00283535 |
| 99 | SLC18A2 | NM_003054.2 | 6571 | cg00498305 |
| 109 | CTAG2 | NM_020994.2 | 30848 | cg01325515 |
| 2 | GJB5 | NM_005268.2 | 2709 | cg01333788 |
| 3 | KCNK16 | NM_032115.2 | 83795 | cg01669948 |
| 100 | SLC6A18 | NM_182632.1 | 348932 | cg02064402 |
| 30 | STK38 | NM_007271.2 | 11329 | cg02964385 |
| 110 | PVALB | NM_002854.2 | 5816 | cg02978737 |
| 6 | OR12D3 | NM_030959.2 | 81797 | cg03608577 |
| 101 | ALOX12B | NM_001139.1 | 242 | cg03742272 |
| 111 | HAO2 | NM_016527.2 | 51179 | cg03762535 |
| 34 | DCLRE1A | NM_014881.2 | 9937 | cg04281204 |
| 102 | WNT10B | NM_003394.2 | 7480 | cg05164634 |
| 7 | TUB | NM_177972.1 | 7275 | cg05492113 |
| 38 | UBE2E1 | NM_182666.1 | 7324 | cg06282059 |
| 39 | IFT140 | NM_014714.2 | 9742 | cg06730286 |
| 112 | SLC4A7 | NM_003615.2 | 9497 | cg06798189 |
| 103 | S100A9 | NM_002965.2 | 6280 | cg07039113 |
| 42 | DIRC1 | NM_052952.1 | 116093 | cg08654334 |
| 10 | CRHR1 | NM_004382.2 | 1394 | cg08929103 |
| 11 | TRIM40 | NM_138700.2 | 135644 | cg09196959 |
| 104 | SLC6A11 | NM_014229.1 | 6538 | cg09395732 |
| 113 | TDRD5 | NM_173533.2 | 163589 | cg09656934 |
| 46 | ACSL5 | NM_016234.3 | 51703 | cg11152574 |
| 48 | APOBEC4 | NM_203454.1 | 403314 | cg11505048 |
| 114 | FAAH | NM_001441.1 | 2166 | cg12671744 |
| 115 | CD1D | NM_001766.2 | 912 | cg13765621 |
| 56 | Clorf201 | NM_178122.2 | 90529 | cg13980834 |
| 16 | FU33860 | NM_173644.1 | 284756 | cg14236389 |
| 59 | ANKRD1 | NM_014391.2 | 27063 | cg14558138 |
| 105 | FGF4 | NM_002007.1 | 2249 | cg14578030 |
| 116 | FU39501 | NM_173483.1 | 126410 | cg14851685 |
| 17 | S100A9 | NM_002965.2 | 6280 | cg16139316 |
| 63 | LRRN5 | NM_006338.2 | 10446 | cg16456919 |
| 117 | GLT1D1 | NM_144669.1 | 144423 | cg16717225 |
| 118 | IL28B | NM_172139.2 | 282617 | cg17982671 |
| 119 | AVPR1B | NM_000707.2 | 553 | cg18992688 |
| 120 | GJB3 | NM_024009.2 | 2707 | cg21151355 |
| 86 | MGC35206 | NM_178552.2 | 339669 | cg22088368 |
| 106 | PCDHAC2 | NM_031883.2 | 56134 | cg24076884 |
| 121 | GABRG3 | NM_033223.1 | 2567 | cg24244000 |
| 94 | MIA2 | NM_054024.3 | 117153 | cg24603941 |
| 122 | CTAG1B | NM_001327.1 | 1485 | cg25531166 |
| 123 | UNQ9433 | NM_207413.1 | 389658 | cg26021627 |
| 107 | GRM4 | NM_000841.1 | 2914 | cg26424956 |
| 124 | CEACAM6 | NM_002483.3 | 4680 | cg26813458 |

**Signature E**

| **Seq ID no.** | **Gene Symbol** | **Accession N. *** | **Gene ID†** | **IlmnID§** |
|---|---|---|---|---|
| 125 | DENND2D | NM_024901.3 | 79961 | cg00619207 |
| 109 | CTAG2 | NM_020994.2 | 30848 | cg01325515 |
| 126 | PGCP | NM_016134.2 | 10404 | cg01892689 |
| 127 | KCNG1 | NM_002237.2 | 3755 | cg02305261 |
| 128 | FAM107B | NM_031453.2 | 83641 | cg02876062 |
| 129 | FANCE | NM_021922.2 | 2178 | cg03030757 |
| 130 | FAM38B | NM_022068.1 | 63895 | cg03426225 |
| 131 | FOXF2 | NM_001452.1 | 2295 | cg03848675 |
| 132 | BGN | NM_001711.3 | 633 | cg04929865 |
| 133 | MAGEA10 | NM_021048.3 | 4109 | cg04950711 |
| 134 | SPATS1 | NM_145026.2 | 221409 | cg05010967 |
| 135 | PRRX1 | NM_022716.2 | 5396 | cg05593300 |
| 136 | MLSTD1 | NM_018099.3 | 55711 | cg05697976 |
| 137 | PDZD4 | NM_032512.2 | 57595 | cg06051662 |
| 138 | HCP5 | NM_006674.2 | 10866 | cg07971188 |
| 139 | OCIAD2 | NM_152398.2 | 132299 | cg08942875 |
| 140 | CSAG1 | NM_153478.1 | 158511 | cg08977028 |
| 141 | SLC04A1 | NM_016354.3 | 28231 | cg09210315 |
| 142 | C9orf90 | NM_197956.1 | 203245 | cg09296212 |
| 143 | CSAG1 | NM_153478.1 | 158511 | cg09917461 |
| 144 | AMPH | NM_001635.2 | 273 | cg09966445 |
| 145 | MT1G | NM_005950.1 | 4495 | cg09985279 |
| 146 | C20orf100 | NM_032883.1 | 84969 | cg11319389 |
| 147 | CST7 | NM_003650.2 | 8530 | cg11804789 |
| 148 | KIAA0367 | NM_015225.1 | 23273 | cg11880010 |
| 149 | RNASE1 | NM_198232.1 | 6035 | cg13718960 |
| 150 | HCP5 | NM_006674.2 | 10866 | cg13784557 |
| 151 | NDN | NM_002487.2 | 4692 | cg13828758 |
| 152 | RAC2 | NM_002872.3 | 5880 | cg14072120 |
| 153 | AXL | NM_021913.2 | 558 | cg14892768 |
| 154 | BATF | NM_006399.2 | 10538 | cg15645309 |
| 155 | EMILIN1 | NM_007046.1 | 11117 | cg15895197 |
| 156 | ARHGEF7 | NM_003899.2 | 8874 | cg16427670 |
| 157 | EVL | NM_016337.2 | 51466 | cg17813891 |
| 158 | RAC2 | NM_002872.3 | 5880 | cg18265887 |
| 159 | KIAA0367 | NM_015225.1 | 23273 | cg19282250 |
| 160 | TFF3 | NM_003226.2 | 7033 | cg20488657 |
| 161 | CTSK | NM_000396.2 | 1513 | cg20802392 |
| 162 | C21orf70 | NM_058190.2 | 85395 | cg20884362 |
| 163 | PRKCZ | NM_002744.4 | 5590 | cg21137823 |
| 164 | MLSTD1 | NM_018099.3 | 55711 | cg21522988 |
| 165 | COL5A2 | NM_000393.2 | 1290 | cg22774472 |
| 166 | LY96 | NM_015364.2 | 23643 | cg23732024 |
| 167 | MAGEA1 | NM_004988.3 | 4100 | cg23776892 |
| 168 | MAGEC2 | NM_016249.2 | 51438 | cg23813564 |
| 169 | DENND2D | NM_024901.3 | 79961 | cg24641737 |
| 170 | PPIE | NM_006112.2 | 10450 | cg24920358 |
| 171 | SORBS2 | NM_021069.3 | 8470 | cg26583078 |
| 172 | PROCR | NM_006404.3 | 10544 | cg26806924 |
| 173 | TMEPAI | NM_020182.3 | 56937 | cg26912636 |
| 174 | SSNA1 | NM_003731.1 | 8636 | cg27291231 |
| 175 | ZNF324 | NM_014347.1 | 25799 | cg27401095 |

**Signature F**

| **Seq ID no.** | **Gene Symbol** | **Accession N. *** | **Gene ID†** | **IlmnID§** |
|---|---|---|---|---|
| 25 | FU25421 | NM_152512.3 | 150350 | cg00044729 |
| 125 | DENND2D | NM_024901.3 | 79961 | cg00619207 |
| 109 | CTAG2 | NM_020994.2 | 30848 | cg01325515 |
| 126 | PGCP | NM_016134.2 | 10404 | cg01892689 |
| 127 | KCNG1 | NM_002237.2 | 3755 | cg02305261 |
| 175 | MGMT | NT_008818.15 | 4255 | cg02330106 |
| 128 | FAM107B | NM_031453.2 | 83641 | cg02876062 |
| 129 | FANCE | NM_021922.2 | 2178 | cg03030757 |
| 176 | GAGE2 | NM_001472.1 | 2574 | cg04431776 |
| 177 | C11orf1 | NM_022761.1 | 64776 | cg04545708 |
| 178 | ABCB4 | NM_018849.1 | 5244 | cg04737046 |
| 132 | BGN | NM_001711.3 | 633 | cg04929865 |
| 133 | MAGEA10 | NM_021048.3 | 4109 | cg04950711 |
| 134 | SPATS1 | NM_145026.2 | 221409 | cg05010967 |
| 179 | CTNNAL1 | NM_003798.1 | 8727 | cg05485060 |
| 135 | PRRX1 | NM_022716.2 | 5396 | cg05593300 |
| 180 | EMP1 | NM_001423.1 | 2012 | cg05885720 |
| 181 | RNASE1 | NM_198232.1 | 6035 | cg05958352 |
| 137 | PDZD4 | NM_032512.2 | 57595 | cg06051662 |
| 182 | MAGEA6 | NM_005363.2 | 4105 | cg06899808 |
| 183 | CHMP4A | NM_014169.2 | 29082 | cg07296772 |
| 184 | ADORA2B | NM_000676.2 | 136 | cg07677850 |
| 138 | HCP5 | NM_006674.2 | 10866 | cg07971188 |
| 185 | PLA1A | NM_015900.1 | 51365 | cg08141463 |
| 186 | KIAA0141 | NM_014773.2 | 9812 | cg08158289 |
| 187 | OLFM1 | NM_014279.2 | 10439 | cg08268099 |
| 188 | ERO1L | NM_014584.1 | 30001 | cg08554114 |
| 140 | CSAG1 | NM_153478.1 | 158511 | cg08977028 |
| 141 | SLC04A1 | NM_016354.3 | 28231 | cg09210315 |
| 189 | CXorf12 | NM_003492.1 | 8269 | cg09275137 |
| 142 | C9orf90 | NM_197956.1 | 203245 | cg09296212 |
| 143 | CSAG1 | NM_153478.1 | 158511 | cg09917461 |
| 144 | AMPH | NM_001635.2 | 273 | cg09966445 |
| 145 | MT1G | NM_005950.1 | 4495 | cg09985279 |
| 190 | ARHGDIB | NM_001175.4 | 397 | cg10925082 |
| 146 | C20orf100 | NM_032883.1 | 84969 | cg11319389 |
| 147 | CST7 | NM_003650.2 | 8530 | cg11804789 |
| 148 | KIAA0367 | NM_015225.1 | 23273 | cg11880010 |
| 149 | RNASE1 | NM_198232.1 | 6035 | cg13718960 |
| 150 | HCP5 | NM_006674.2 | 10866 | cg13784557 |
| 151 | NDN | NM_002487.2 | 4692 | cg13828758 |
| 152 | RAC2 | NM_002872.3 | 5880 | cg14072120 |
| 153 | AXL | NM_021913.2 | 558 | cg14892768 |
| 191 | GRAMD3 | NM_023927.1 | 65983 | cg15165084 |
| 61 | ARSG | NM_014960.2 | 22901 | cg15308737 |
| 192 | PLD1 | NM_002662.2 | 5337 | cg15329866 |
| 193 | HNMT | NM_006895.2 | 3176 | cg15441973 |
| 194 | ZNF558 | NM_144693.1 | 148156 | cg15751406 |
| 195 | ATP6V1E2 | NM_080653.3 | 90423 | cg15759873 |
| 155 | EMILIN1 | NM_007046.1 | 11117 | cg15895197 |
| 156 | ARHGEF7 | NM_003899.2 | 8874 | cg16427670 |
| 196 | KRT25C | NM_181537.2 | 342574 | cg16488522 |
| 197 | ZNF532 | NM_018181.4 | 55205 | cg17675150 |
| 157 | EVL | NM_016337.2 | 51466 | cg17813891 |
| 158 | RAC2 | NM_002872.3 | 5880 | cg18265887 |
| 198 | SLAMF9 | NM_033438.1 | 89886 | cg18913171 |
| 199 | NBL1 | NM_005380.3 | 4681 | cg19136075 |
| 159 | KIAA0367 | NM_015225.1 | 23273 | cg19282250 |
| 200 | MAGEA10 | NM_021048.3 | 4109 | cg19964192 |
| 160 | TFF3 | NM_003226.2 | 7033 | cg20488657 |
| 162 | C21orf70 | NM_058190.2 | 85395 | cg20884362 |
| 80 | APOD | NM_001647.2 | 347 | cg21084260 |
| 201 | ABCA8 | NM_007168.2 | 10351 | cg21660392 |
| 202 | ACOX2 | NM_003500.2 | 8309 | cg22012981 |
| 165 | COL5A2 | NM_000393.2 | 1290 | cg22774472 |
| 166 | LY96 | NM_015364.2 | 23643 | cg23732024 |
| 167 | MAGEA1 | NM_004988.3 | 4100 | cg23776892 |
| 168 | MAGEC2 | NM_016249.2 | 51438 | cg23813564 |
| 203 | TIMP3 | NM_000362.4 | 7078 | cg24080529 |
| 170 | PPIE | NM_006112.2 | 10450 | cg24920358 |
| 204 | RPUSD3 | NM_173659.2 | 285367 | cg26417554 |
| 172 | PROCR | NM_006404.3 | 10544 | cg26806924 |
| 173 | TMEPAI | NM_020182.3 | 56937 | cg26912636 |

**Signature G**

| **Seq ID no.** | **Gene Symbol** | **Accession N. *** | **Gene ID†** | **IlmnID§** |
|---|---|---|---|---|
| 205 | PRDX2 | NM_005809.4 | 7001 | cg00155609 |
| 125 | DENND2D | NM_024901.3 | 79961 | cg00619207 |
| 109 | CTAG2 | NM_020994.2 | 30848 | cg01325515 |
| 206 | PCOLCE | NM_002593.2 | 5118 | cg02797569 |
| 132 | BGN | NM_001711.3 | 633 | cg04929865 |
| 207 | OCIAD2 | NM_152398.2 | 132299 | cg04961553 |
| 181 | RNASE1 | NM_198232.1 | 6035 | cg05958352 |
| 182 | MAGEA6 | NM_005363.2 | 4105 | cg06899808 |
| 138 | HCP5 | NM_006674.2 | 10866 | cg07971188 |
| 139 | OCIAD2 | NM_152398.2 | 132299 | cg08942875 |
| 140 | CSAG1 | NM_153478.1 | 158511 | cg08977028 |
| 142 | C9orf90 | NM_197956.1 | 203245 | cg09296212 |
| 143 | CSAG1 | NM_153478.1 | 158511 | cg09917461 |
| 208 | CTSK | NM_000396.2 | 1513 | cg11946165 |
| 209 | ADM | NM_001124.1 | 133 | cg12320306 |
| 149 | RNASE1 | NM_198232.1 | 6035 | cg13718960 |
| 150 | HCP5 | NM_006674.2 | 10866 | cg13784557 |
| 210 | PAGE5 | NM_130467.3 | 90737 | cg13991029 |
| 211 | NNMT | NM_006169.2 | 4837 | cg14209518 |
| 155 | EMILIN1 | NM_007046.1 | 11117 | cg15895197 |
| 212 | MAGEA3 | NM_005362.3 | 4102 | cg16390856 |
| 213 | RAB11FIP5 | NM_015470.1 | 26056 | cg18943195 |
| 214 | ARHGDIB | NM_001175.4 | 397 | cg19826026 |
| 215 | EMILIN1 | NM_007046.1 | 11117 | cg20516209 |
| 216 | LPXN | NM_004811.1 | 9404 | cg20654468 |
| 161 | CTSK | NM_000396.2 | 1513 | cg20802392 |
| 217 | ALDH1A3 | NM_000693.1 | 220 | cg21359747 |
| 218 | SUHW1 | NM_080740.3 | 129025 | cg21604856 |
| 201 | ABCA8 | NM_007168.2 | 10351 | cg21660392 |
| 165 | COL5A2 | NM_000393.2 | 1290 | cg22774472 |
| 166 | LY96 | NM_015364.2 | 23643 | cg23732024 |
| 167 | MAGEA1 | NM_004988.3 | 4100 | cg23776892 |
| 168 | MAGEC2 | NM_016249.2 | 51438 | cg23813564 |
| 219 | GSTT1 | NM_000853.1 | 2952 | cg24330042 |
| 169 | DENND2D | NM_024901.3 | 79961 | cg24641737 |

**Table 4:** Genes of the various signatures and relative SEQ ID No. Genomic coordinates are given respective to assembly GRCh37, in the form of: assembly n:chromosome n: start position-end position
**Seq ID no.1, MGC29671 -** GRCh37:17:4336094-4338094, Probe design region (genomic position GRCh37:17:4337034-4337155, nt 941 to nt 1062 of SEQ ID No. 1), investigated CpG (genomic position GRCh37:17:4337094, nt 1001 of SEQ ID No. 1)
**Seq ID no.2, GJB5** - GRCh37:1:35219815-35221815:1, Probe design region (genomic position GRCh37:1:35220754-35220875, nt 940 to nt 1061 of SEQ ID No. 2), investigated CpG (genomic position GRCh37:1:35220814, nt 1000 of SEQ ID No. 2)
**Seq ID no.3, KCNK16 -** GRCh37:6:39289878-39291878, Probe design region (genomic position GRCh37:6:39290817-39290938, nt 940 to nt 1061 of SEQ ID No. 3), investigated CpG (genomic position GRCh37:6:39290877, nt 1000 of SEQ ID No. 3)
**Seq ID no.4, IGLL1 -** GRCh37:22:23922255-23924255, Probe design region (genomic position GRCh37:22:23923195-23923316, nt 941 to nt 1062 of SEQ ID No. 4), investigated CpG (genomic position GRCh37:22:23923255, nt 1001 of SEQ ID No. 4)
**Seq ID no.5, ANXA8 -** GRCh37:10:47746083-47748083, Probe design region (genomic position GRCh37:10:47747022-47747143, nt 940 to nt 1061 of SEQ ID No. 5), investigated CpG (genomic position GRCh37:10:47747083, nt 1000 of SEQ ID No. 5)
**Seq ID no.6, OR12D3 -** GRCh37:6:29341680-29343680, Probe design region (genomic position GRCh37:6:29342619-29342740, nt 940 to nt 1061 of SEQ ID No. 6), investigated CpG (genomic position GRCh37:6: 29342680, nt 1000 of SEQ ID No. 6)
**Seq ID no.7, TUB -** GRCh37:11:8059333-8061333, Probe design region (genomic position GRCh37:11:8060272-8060393, nt 940 to nt 1061 of SEQ ID No. 7), investigated CpG (genomic position GRCh37:11: 8060333, nt 1000 of SEQ ID No. 7)
**Seq ID no.8, CST11 -** GRCh37:20:23432349-23434349 - Probe design region (genomic position GRCh37:11:8060272-8060393, nt 941 to nt 1062 of SEQ ID No. 8), investigated CpG (genomic position GRCh37:20:23433349, nt 1001 of SEQ ID No. 8)
**Seq ID no.9, TMEM10 -** GRCh37:10:98118079-98120079, Probe design region (genomic position GRCh37:11:8060272-8060393, nt 941 to nt 1062 of SEQ ID No. 9), investigated CpG (genomic position GRCh37:10:98119079, nt 1001 of SEQ ID No. 9)
**Seq ID no.10, CRHR1 -** GRCh37:17:43859355-43861355, Probe design region (genomic position GRCh37:17:43860295-43860416, nt 941 to nt 1062 of SEQ ID No. 10), investigated CpG (genomic position GRCh37:17: 43860355, nt 1001 of SEQ ID No. 10)
**Seq ID no.11, TRIM40 -** GRCh37:6:30103441-30105441, Probe design region (genomic position GRCh37:6:30104380-30104501, nt 940 to nt 1061 of SEQ ID No. 11), investigated CpG (genomic position GRCh37:6:30104441, nt 1000 of SEQ ID No. 11)
**Seq ID no.12, GAS2L2 -** GRCh37:17:34078666-34080666, Probe design region (genomic position GRCh37:17:34079605-34079726, nt 940 to nt 1061 of SEQ ID No. 12), investigated CpG (genomic position GRCh37:17:34079666, nt 1000 of SEQ ID No. 12)
**Seq ID no.13, FOXN1 -** GRCh37:17:26849322-26851322, Probe design region (genomic position GRCh37:17:26850262-26850383, nt 941 to nt 1062 of SEQ ID No. 13), investigated CpG (genomic position GRCh37:17:26850322, nt 1001 of SEQ ID No. 13)
**Seq ID no.14, FXYD4 -** GRCh37:10:43866156-43868156, Probe design region (genomic position GRCh37:10:43867095-43867216, nt 940 to nt 1061 of SEQ ID No. 14), investigated CpG (genomic position GRCh37:10:43867156, nt 1000 of SEQ ID No. 14)
**Seq ID no.15, KRTHB2 -** GRCh37:12:52799423-52801423, Probe design region (genomic position GRCh37:12:52800363-52800484, nt 941 to nt 1062 of SEQ ID No. 15), investigated CpG (genomic position GRCh37:12:52800423, nt 1001 of SEQ ID No. 15)
**Seq ID no.16, FLJ33860 -** GRCh37:20:58630039-58632039, Probe design region (genomic position GRCh37:20: 58630978-58631099, nt 940 to nt 1061 of SEQ ID No. 16), investigated CpG (genomic position GRCh37:20:58631039, nt 1000 of SEQ ID No. 16)
**Seq ID no.17, S100A9 -** GRCh37:1:153329759-153331759, Probe design region (genomic position GRCh37:1:153330698-153330819, nt 940 to nt 1061 of SEQ ID No. 17), investigated CpG (genomic position GRCh37:1:153330759, nt 1000 of SEQ ID No. 17)
**Seq ID no.18, FOX11 -** GRCh37:5:169531752-169533752, Probe design region (genomic position GRCh37:5:169532692-169532813, nt 941 to nt 1062 of SEQ ID No. 18), investigated CpG (genomic position GRCh37:5:169532752, nt 1001 of SEQ ID No. 18)
**Seq ID no.19, GUCA2B -** GRCh37:1:42618164-42620164, Probe design region (genomic position GRCh37:1:42619103-42619224, nt 940 to nt 1061 of SEQ ID No. 19), investigated CpG (genomic position GRCh37:1:42619164, nt 1000 of SEQ ID No. 19)
**Seq ID no.20, CYP2B6 -** GRCh37:19:41496223-41498223, Probe design region (genomic position GRCh37:19:41497162-41497283, nt 940 to nt 1061 of SEQ ID No. 20), investigated CpG (genomic position GRCh37:19:41497223, nt 1000 of SEQ ID No. 20)
**Seq ID no.21, IL8RA -** GRCh37:2:219031025-219033025, Probe design region (genomic position GRCh37:2:219031964-219032085, nt 940 to nt 1061 of SEQ ID No. 21), investigated CpG (genomic position GRCh37:2:219032025, nt 1000 of SEQ ID No. 21)
**Seq ID no.22, CYP8B1 -** GRCh37:3:42916518-42918518, Probe design region (genomic position GRCh37:3:42917457-42917578, nt 940 to nt 1061 of SEQ ID No. 22), investigated CpG (genomic position GRCh37:3:42917518, nt 1000 of SEQ ID No. 22)
**Seq ID no.23, WFDC8 -** GRCh37:20:44206289-44208289, Probe design region (genomic position GRCh37: 20: 44206289-44208289, nt 940 to nt 1061 of SEQ ID No. 23), investigated CpG (genomic position GRCh37:20: 44207289, nt 1000 of SEQ ID No. 23)
**Seq ID no.24, SLPI -** GRCh37:20:43881991-43883991, Probe design region (genomic position GRCh37: 20:43882933-43883051, nt 940 to nt 1061 of SEQ ID No. 24), investigated CpG (genomic position GRCh37:20: 43882991, nt 1000 of SEQ ID No. 24)
**Seq ID no.25, FLJ25421 -** GRCh37:22:40289977-40291977, Probe design region (genomic position GRCh37:22:40290916-40291037, nt 940 to nt 1061 of SEQ ID No. 25), investigated CpG (genomic position GRCh37:22:40290977, nt 1000 of SEQ ID No. 25)
**Seq ID no.26, LAMC2 -** GRCh37:1:183153778-183155778, Probe design region (genomic position GRCh37:1:183154718-183154839, nt 941 to nt 1062 of SEQ ID No. 26), investigated CpG (genomic position GRCh37:1:183154778, nt 1001 of SEQ ID No. 26)
**Seq ID no.27, Clorf36 -** GRCh37:1:211665129-211667129, Probe design region (genomic position GRCh37:1:211666069-211666190, nt 941 to nt 1062 of SEQ ID No. 27), investigated CpG (genomic position GRCh37:1:211666129, nt 1001 of SEQ ID No. 27)
**Seq ID no.28, ANGPTL3 -** GRCh37:1:63061489-63063489, Probe design region (genomic position GRCh37:1:63062429-63062550, nt 941 to nt 1062 of SEQ ID No. 28), investigated CpG (genomic position GRCh37:1:63062489, nt 1001 of SEQ ID No. 28)
**Seq ID no.29, DEFB4 -** GRCh37:8:7750367-7752367, Probe design region (genomic position GRCh37:8:7751306-7751427, nt 940 to nt 1061 of SEQ ID No. 29), investigated CpG (genomic position GRCh37:8:7751367, nt 1000 of SEQ ID No. 29)
**Seq ID no.30, STK38 -** GRCh37:6:36514677-36516677, Probe design region (genomic position GRCh37:6: 36515616-36515737, nt 940 to nt 1061 of SEQ ID No. 30), investigated CpG (genomic position GRCh37:6:36515677, nt 1000 of SEQ ID No. 30)
**Seq ID no.31, SEMG2 -** GRCh37:20:43848760-43850760, Probe design region (genomic position GRCh37:20:43849699-43849820, nt 940 to nt 1061 of SEQ ID No. 31), investigated CpG (genomic position GRCh37:20:43849760, nt 1000 of SEQ ID No. 31)
**Seq ID no.32, PREPL -** GRCh37:2:44585902-44587902, Probe design region (genomic position GRCh37:2: 44586841-44586962, nt 940 to nt 1061 of SEQ ID No. 32), investigated CpG (genomic position GRCh37:2:44586902, nt 1000 of SEQ ID No. 32)
**Seq ID no.33, IL17F** - GRCh37:6:52108348-52110348, Probe design region (genomic position GRCh37:6:52109287-52109408, nt 940 to nt 1061 of SEQ ID No. 33), investigated CpG (genomic position GRCh37:6:52109348, nt 1000 of SEQ ID No. 33)
**Seq ID no.34, DCLRE1A** - GRCh37:10:115611803-115613803, Probe design region (genomic position GRCh37:10:115612742-115612863, nt 940 to nt 1061 of SEQ ID No. 34), investigated CpG (genomic position GRCh37:10:115612803, nt 1000 of SEQ ID No. 34)
**Seq ID no.35, FLJ20581 -** GRCh37:16:20419653-20421653, Probe design region (genomic position GRCh37:16:20420592-20420713, nt 940 to nt 1061 of SEQ ID No. 35), investigated CpG (genomic position GRCh37:16:20420653, nt 1000 of SEQ ID No. 35)
**Seq ID no.36, PTCD2 -** GRCh37:5:71613753-71615753, Probe design region (genomic position GRCh37:5:71614692-71614813, nt 940 to nt 1061 of SEQ ID No. 36), investigated CpG (genomic position GRCh37:5:71614753, nt 1000 of SEQ ID No. 36)
**Seq ID no.37, CX3CR1 -** GRCh37:3:39320450-39322450, Probe design region (genomic position GRCh37:3: 39321389-39321510, nt 940 to nt 1061 of SEQ ID No. 37), investigated CpG (genomic position GRCh37:3:39321450, nt 1000 of SEQ ID No. 37)
**Seq ID no.38, UBE2E1** - GRCh37:3:23845693-23847693, Probe design region (genomic position GRCh37:3: 23846632-23846753, nt 940 to nt 1061 of SEQ ID No. 38), investigated CpG (genomic position GRCh37:3:23846693, nt 1000 of SEQ ID No. 38)
**Seq ID no.39, IFT140 -** GRCh37:16:1656373-1658373, Probe design region (genomic position GRCh37:16:1657312-1657433, nt 940 to nt 1061 of SEQ ID No. 39), investigated CpG (genomic position GRCh37:16:1657373, nt 1000 of SEQ ID No. 39)
**Seq ID no.40, SLC22A8 -** GRCh37:11:62782123-62784123, Probe design region (genomic position GRCh37:11: 62783063-62783184, nt 941 to nt 1062 of SEQ ID No. 40), investigated CpG (genomic position GRCh37:11:62783123, nt 1001 of SEQ ID No. 40)
**Seq ID no.41, MLN -** GRCh37:6:33769851-33771851, Probe design region (genomic position GRCh37:6: 33770790-33770911, nt 940 to nt 1061 of SEQ ID No. 41), investigated CpG (genomic position GRCh37:6:33770851, nt 1000 of SEQ ID No. 41)
**Seq ID no.42, DIRC1 -** GRCh37:2:189654590-189656590, Probe design region (genomic position GRCh37:2:189655529-189655650, nt 940 to nt 1061 of SEQ ID No. 42), investigated CpG (genomic position GRCh37:2:189655590, nt 1000 of SEQ ID No. 42)
**Seq ID no.43, Clorf64** - GRCh37:1:16329717-16331717, Probe design region (genomic position GRCh37:1:16330656-16330777, nt 940 to nt 1061 of SEQ ID No. 43), investigated CpG (genomic position GRCh37:1:16330717, nt 1000 of SEQ ID No. 43)
**Seq ID no.44, TNFSF13 -** GRCh37:13:108920053-108922053, Probe design region (genomic position GRCh37:13:108920992-108921113, nt 940 to nt 1061 of SEQ ID No. 44), investigated CpG (genomic position GRCh37:13: 108921053, nt 1000 of SEQ ID No. 44)
**Seq ID no.45, CYP2B6 -** GRCh37:19:41495749-41497749, Probe design region (genomic position GRCh37:19:41496689-41496810, nt 941 to nt 1062 of SEQ ID No. 45), investigated CpG (genomic position GRCh37:19:41496749, nt 1001 of SEQ ID No. 45)
**Seq ID no.46, ACSL5** - GRCh37:10:114133551-114135551, Probe design region (genomic position GRCh37:10:114134490-114134611, nt 940 to nt 1061 of SEQ ID No. 46), investigated CpG (genomic position GRCh37:10:114134551, nt 1000 of SEQ ID No. 46)
**Seq ID no.47, SERPINB12 -** GRCh37:18:61221687-61223687, Probe design region (genomic position GRCh37:18:61222627-61222748, nt 941 to nt 1062 of SEQ ID No. 47), investigated CpG (genomic position GRCh37:18:61222687, nt 1001 of SEQ ID No. 47)
**Seq ID no.48, APOBEC4 -** GRCh37:1:183621727-183623727, Probe design region (genomic position GRCh37:1:183622666-183622787, nt 940 to nt 1061 of SEQ ID No. 48), investigated CpG (genomic position GRCh37:1:183622727, nt 1000 of SEQ ID No. 48)
**Seq ID no.49, TNNI1** - GRCh37:1:201389794-201391794, Probe design region (genomic position GRCh37:1:201390734-201390855, nt 941 to nt 1062 of SEQ ID No. 49), investigated CpG (genomic position GRCh37:1:201390794, nt 1001 of SEQ ID No. 49)
**Seq ID no.50, Clorf42** - GRCh37:1:152486336-152488336, Probe design region (genomic position GRCh37:1:152487275-152487396, nt 940 to nt 1061 of SEQ ID No. 50), investigated CpG (genomic position GRCh37:1:152487336, nt 1000 of SEQ ID No. 50)
**Seq ID no.51, PPAT -** GRCh37:4:57302150-57304150, Probe design region (genomic position GRCh37:4: 57303089-57303210, nt 940 to nt 1061 of SEQ ID No. 51), investigated CpG (genomic position GRCh37:4:57303150, nt 1000 of SEQ ID No. 51)
**Seq ID no.52, PSD4 -** GRCh37:2:113930566-113932566, Probe design region (genomic position GRCh37:2: 113931505-113931626, nt 940 to nt 1061 of SEQ ID No. 52), investigated CpG (genomic position GRCh37:2:113931566, nt 1000 of SEQ ID No. 52)
**Seq ID no.53, SCN4A -** GRCh37:17:62049038-62051038, Probe design region (genomic position GRCh37:17: 62049978-62050099, nt 941 to nt 1062 of SEQ ID No. 53), investigated CpG (genomic position GRCh37:17:62050038, nt 1001 of SEQ ID No. 53)
**Seq ID no.54, IL8RB -** GRCh37:2:218989627-218991627, Probe design region (genomic position GRCh37:2:218990567-218990688, nt 941 to nt 1062 of SEQ ID No. 54), investigated CpG (genomic position GRCh37:2:218990627, nt 1001 of SEQ ID No. 54)
**Seq ID no.55, UNC45B -** GRCh37:17:33473693-33475693, Probe design region (genomic position GRCh37:17: 33474632-33474753, nt 940 to nt 1061 of SEQ ID No. 55), investigated CpG (genomic position GRCh37:17:33474693, nt 1000 of SEQ ID No. 55)
**Seq ID no.56, C1orf201** - GRCh37:1:24726851-24728851, Probe design region (genomic position GRCh37:1:24727790-24727911, nt 940 to nt 1061 of SEQ ID No. 56), investigated CpG (genomic position GRCh37:1:24727851, nt 1000 of SEQ ID No. 56)
**Seq ID no.57, SFTPG -** GRCh37:6:30899198-30901198, Probe design region (genomic position GRCh37:6:30900137-30900258, nt 940 to nt 1061 of SEQ ID No. 57), investigated CpG (genomic position GRCh37:6:30900198, nt 1000 of SEQ ID No. 57)
**Seq ID no.58, LOC124751 -** GRCh37:17:8274274-8276274, Probe design region (genomic position GRCh37:17:8275213-8275334, nt 940 to nt 1061 of SEQ ID No. 58), investigated CpG (genomic position GRCh37:17:8275274, nt 1000 of SEQ ID No. 58)
**Seq ID no.59, ANKRD1 -** GRCh37:10:92679511-92681511, Probe design region (genomic position GRCh37:10:92680450-92680571, nt 940 to nt 1061 of SEQ ID No. 59), investigated CpG (genomic position GRCh37:10:92680511, nt 1000 of SEQ ID No. 59)
**Seq ID no.60, FMO2** - GRCh37:1:171153612-171155612, Probe design region (genomic position GRCh37:1:171154552-171154673, nt 941 to nt 1062 of SEQ ID No. 60), investigated CpG (genomic position GRCh37:1:171154612, nt 1001 of SEQ ID No. 60)
**Seq ID no.61, ARSG -** GRCh37:17:66302328-66304328, Probe design region (genomic position GRCh37:17:66303268-66303389, nt 941 to nt 1062 of SEQ ID No. 61), investigated CpG (genomic position GRCh37:17:66303328, nt 1001 of SEQ ID No. 61)
**Seq ID no.62, GJB4 -** GRCh37:1:35225892-35227892, Probe design region (genomic position GRCh37:1:35226831-35226952, nt 940 to nt 1061 of SEQ ID No. 62), investigated CpG (genomic position GRCh37:1:35226892, nt 1000 of SEQ ID No. 62)
**Seq ID no.63, LRRN5** - GRCh37:1:204654379-204656379, Probe design region (genomic position GRCh37:1:204655318-204655439, nt 940 to nt 1061 of SEQ ID No. 63), investigated CpG (genomic position GRCh37:1:204655379, nt 1000 of SEQ ID No. 63)
**Seq ID no.64, MSMB -** GRCh37:10:51548260-51550260, Probe design region (genomic position GRCh37:10:51549200-51549321, nt 941 to nt 1062 of SEQ ID No. 64), investigated CpG (genomic position GRCh37:10:51549260, nt 1001 of SEQ ID No. 64)
**Seq ID no.65, HLA-DOB -** GRCh37:6:32784142-32786142, Probe design region (genomic position GRCh37:6: 32785081-32785202, nt 940 to nt 1061 of SEQ ID No. 65), investigated CpG (genomic position GRCh37:6:32785142, nt 1000 of SEQ ID No. 65)
**Seq ID no.66, R3HDML -** GRCh37:20:42963960-42965960, Probe design region (genomic position GRCh37:20:42964899-42965020, nt 940 to nt 1061 of SEQ ID No. 66), investigated CpG (genomic position GRCh37:20:42964960, nt 1000 of SEQ ID No. 66)
**Seq ID no.67, SPINK5 -** GRCh37:5:147442252-147444252, Probe design region (genomic position GRCh37:5:147443192-147443313, nt 941 to nt 1062 of SEQ ID No. 67), investigated CpG (genomic position GRCh37:5: 147443252, nt 1001 of SEQ ID No. 67)
**Seq ID no.68, DLG5** - GRCh37:10:79627941-79629941, Probe design region (genomic position GRCh37:10:79628881-79629002, nt 941 to nt 1062 of SEQ ID No. 68), investigated CpG (genomic position GRCh37:10:79628941, nt 1001 of SEQ ID No. 68)
**Seq ID no.69, PALLD -** GRCh37:4:169417492-169419492, Probe design region (genomic position GRCh37:4:169418431-169418552, nt 940 to nt 1061 of SEQ ID No. 69), investigated CpG (genomic position GRCh37:4: 169418492, nt 1000 of SEQ ID No. 69)
**Seq ID no.70, WFDC12 -** GRCh37:20:43752347-43754347, Probe design region (genomic position GRCh37:20:43753287-43753408, nt 940 to nt 1061 of SEQ ID No. 70), investigated CpG (genomic position GRCh37:20:43753347, nt 1000 of SEQ ID No. 70)
**Seq ID no.71, WDR20 -** GRCh37:14:102603722-102605722, Probe design region (genomic position GRCh37:14:102604662-102604783, nt 941 to nt 1062 of SEQ ID No. 71), investigated CpG (genomic position GRCh37:14:102604722, nt 1001 of SEQ ID No. 71)
**Seq ID no.72, FLJ32771 -** GRCh37:11:61247085-61249085, Probe design region (genomic position GRCh37:11:61248024-61248145, nt 940 to nt 1061 of SEQ ID No. 72), investigated CpG (genomic position GRCh37:11: 61248085, nt 1000 of SEQ ID No. 72)
**Seq ID no.73, PKHD1 -** GRCh37:6:51952114-51954114, Probe design region (genomic position GRCh37:6:51953054-51953175, nt 941 to nt 1062 of SEQ ID No. 73), investigated CpG (genomic position GRCh37:6:51953114, nt 1001 of SEQ ID No. 73)
**Seq ID no.74, DEFB123 -** GRCh37:20:30027893-30029893, Probe design region (genomic position GRCh37:20:30028833-30028954, nt 941 to nt 1062 of SEQ ID No. 74), investigated CpG (genomic position GRCh37:20:30028893, nt 1001 of SEQ ID No. 74)
**Seq ID no.75, KRTHB3 -** GRCh37:12:52713892-52715892, Probe design region (genomic position GRCh37:12:52714831-52714952, nt 940 to nt 1061 of SEQ ID No. 75), investigated CpG (genomic position GRCh37:12:52714892, nt 1000 of SEQ ID No. 75)
**Seq ID no.76, BSND -** GRCh37:1:55463928-55465928, Probe design region (genomic position GRCh37:1: 55464867-55464988, nt 940 to nt 1061 of SEQ ID No. 76), investigated CpG (genomic position GRCh37:1:55464928, nt 1000 of SEQ ID No. 76)
**Seq ID no.77, NALP10 -** GRCh37:11:7984184-7986184, Probe design region (genomic position GRCh37:11:7985123-7985244, nt 940 to nt 1061 of SEQ ID No. 77), investigated CpG (genomic position GRCh37:11:7985184, nt 1000 of SEQ ID No. 77)
**Seq ID no.78, NR5A2 -** GRCh37:1:199995238-199997238, Probe design region (genomic position GRCh37:1:7985123-7985244, nt 940 to nt 1061 of SEQ ID No. 78), investigated CpG (genomic position GRCh37:1:199996238, nt 1000 of SEQ ID No. 78)
**Seq ID no.79, KRT25D -** GRCh37:17:38955500-38957500, Probe design region (genomic position GRCh37:17:38956440-38956561, nt 941 to nt 1062 of SEQ ID No. 79), investigated CpG (genomic position GRCh37:17:38956500, nt 1001 of SEQ ID No. 79)
**Seq ID no.80, APOD -** GRCh37:3:195310868-195312868, Probe design region (genomic position GRCh37:3:195311808-195311808, nt 941 to nt 1062 of SEQ ID No. 80), investigated CpG (genomic position GRCh37:3:195311868, nt 1001 of SEQ ID No. 80)
**Seq ID no.81, CLEC4F -** GRCh37:2:71046650-71048650, Probe design region (genomic position GRCh37:2:71047589-71047710, nt 940 to nt 1061 of SEQ ID No. 81), investigated CpG (genomic position GRCh37:2:71047650, nt 1000 of SEQ ID No. 81)
**Seq ID no.82, THRB -** GRCh37:3:24536178-24538178, Probe design region (genomic position GRCh37:3:24537117-24537238, nt 940 to nt 1061 of SEQ ID No. 82), investigated CpG (genomic position GRCh37:3:24537178, nt 1000 of SEQ ID No. 82)
**Seq ID no.83, KRTHB5 -** GRCh37:12:52760417-52762417, Probe design region (genomic position GRCh37:12:52761356-52761477, nt 940 to nt 1061 of SEQ ID No. 83), investigated CpG (genomic position GRCh37:12:52761417, nt 1000 of SEQ ID No. 83)
**Seq ID no.84, SLC13A1 -** GRCh37:7:122838956-122840956, Probe design region (genomic position GRCh37:7:122839895-122840016, nt 940 to nt 1061 of SEQ ID No. 84), investigated CpG (genomic position GRCh37:7:122839956, nt 1000 of SEQ ID No. 84)
**Seq ID no.85, C3orf27 -** GRCh37:3:128294141-128296141, Probe design region (genomic position GRCh37:3:128295081-128295202, nt 941 to nt 1062 of SEQ ID No. 85), investigated CpG (genomic position GRCh37:3:128295141, nt 1001 of SEQ ID No. 85)
**Seq ID no.86, MGC35206 -** GRCh37:22:37403888-37405888, Probe design region (genomic position GRCh37:22:37404828-37404949, nt 941 to nt 1062 of SEQ ID No. 86), investigated CpG (genomic position GRCh37:22:37404888, nt 1001 of SEQ ID No. 86)
**Seq ID no.87, C3orf63** - GRCh37:3:56697168-56699168, Probe design region (genomic position GRCh37:3:56698108-56698229, nt 941 to nt 1062 of SEQ ID No. 87), investigated CpG (genomic position GRCh37:3:56698168, nt 1001 of SEQ ID No. 87)
**Seq ID no.88, DARC -** GRCh37:1:159173156-159175156, Probe design region (genomic position GRCh37:1:159174096-159174217, nt 941 to nt 1062 of SEQ ID No. 88), investigated CpG (genomic position GRCh37:1:159174156, nt 1001 of SEQ ID No. 88)
**Seq ID no.89, SLC2A7 -** GRCh37:1:9085621-9087621, Probe design region (genomic position GRCh37:1:9086561-9086682, nt 941 to nt 1062 of SEQ ID No. 89), investigated CpG (genomic position GRCh37:1:9086621, nt 1001 of SEQ ID No. 89)
**Seq ID no.90, CPA3 -** GRCh37:3:148582153-148584153, Probe design region (genomic position GRCh37:3:148583092-148583213, nt 940 to nt 1061 of SEQ ID No. 90), investigated CpG (genomic position GRCh37:3:148583153, nt 1000 of SEQ ID No. 90)
**Seq ID no.91, MIS12 -** GRCh37:17:5390239-5392239, Probe design region (genomic position GRCh37:17:5391178-5391299, nt 940 to nt 1061 of SEQ ID No. 91), investigated CpG (genomic position GRCh37:17:5391239, nt 1000 of SEQ ID No. 91)
**Seq ID no.92, KRTHB4 -** GRCh37:12:52778282-52780282, Probe design region (genomic position GRCh37:12:52779221-52779342, nt 940 to nt 1061 of SEQ ID No. 92), investigated CpG (genomic position GRCh37:12:52779282, nt 1000 of SEQ ID No. 92)
**Seq ID no.93, MRVI1** - GRCh37:11:10714360-10716360, Probe design region (genomic position GRCh37:11:10715300-10715421, nt 941 to nt 1062 of SEQ ID No. 93), investigated CpG (genomic position GRCh37:11:10715360, nt 1001 of SEQ ID No. 93)
**Seq ID no.94, MIA2 -** GRCh37:14:39702303-39704303, Probe design region (genomic position GRCh37:14:39703242-39703363, nt 940 to nt 1061 of SEQ ID No. 94), investigated CpG (genomic position GRCh37:14:39703303, nt 1000 of SEQ ID No. 94)
**Seq ID no.95, CYP2A7 -** GRCh37:19:41388357-41390357, Probe design region (genomic position GRCh37:19:41389297-41389418, nt 941 to nt 1062 of SEQ ID No. 95), investigated CpG (genomic position GRCh37:19:41389357, nt 1001 of SEQ ID No. 95)
**Seq ID no.96, ANGPT4 -** GRCh37:20:895349-897349, Probe design region (genomic position GRCh37:20:896289-896410, nt 941 to nt 1062 of SEQ ID No. 96), investigated CpG (genomic position GRCh37:20:896349, nt 1001 of SEQ ID No. 96)
**Seq ID no.97, SLC44A5 -** GRCh37:1:76077230-76079230, Probe design region (genomic position GRCh37:1:76078170-76078291, nt 941 to nt 1062 of SEQ ID No. 97), investigated CpG (genomic position GRCh37:1:76078230, nt 1001 of SEQ ID No. 97)
**Seq ID no.98, CST9L -** GRCh37:20:23548455-23550455, Probe design region (genomic position GRCh37:20: 23549395-23549516, nt 941 to nt 1062 of SEQ ID No. 98), investigated CpG (genomic position GRCh37:20: 23549455, nt 1001 of SEQ ID No. 98)
**Seq ID no.99, SLC18A2 -** GRCh37:10:118999236-119001236, Probe design region (genomic position GRCh37:10:119000176-119000297, nt 941 to nt 1062 of SEQ ID No. 99), investigated CpG (genomic position GRCh37:10:119000236, nt 1001 of SEQ ID No. 99)
**Seq ID no.100, SLC6A18 -** GRCh37:5:1224720-1226720, Probe design region (genomic position GRCh37:5:1225659-1225780, nt 940 to nt 1061 of SEQ ID No. 100), investigated CpG (genomic position GRCh37:5:1225720, nt 1000 of SEQ ID No. 100)
**Seq ID no.101, ALOX12B -** GRCh37:17:7989706-7991706, Probe design region (genomic position GRCh37:17:7990645-7990766, nt 940 to nt 1061 of SEQ ID No. 101), investigated CpG (genomic position GRCh37:17:7990706, nt 1000 of SEQ ID No. 101)
**Seq ID no.102, WNT10B -** GRCh37:12:49365538-49367538, Probe design region (genomic position GRCh37:12:49366478-49366599, nt 941 to nt 1062 of SEQ ID No. 102), investigated CpG (genomic position GRCh37:12:49366538, nt 1001 of SEQ ID No. 102)
**Seq ID no.103, S100A9 -** GRCh37:1:153328013-153330013, Probe design region (genomic position GRCh37:1:153328953-153329074, nt 941 to nt 1062 of SEQ ID No. 103), investigated CpG (genomic position GRCh37:1:153329013, nt 1001 of SEQ ID No. 103)
**Seq ID no.104, SLC6A11** - GRCh37:3:10856457-10858457, Probe design region (genomic position GRCh37:3:10857396-10857517, nt 940 to nt 1061 of SEQ ID No. 104), investigated CpG (genomic position GRCh37:3:10857457, nt 1000 of SEQ ID No. 104)
**Seq ID no.105, FGF4 -** GRCh37:11:69590383-69592383, Probe design region (genomic position GRCh37:11:69591323-69591444, nt 941 to nt 1062 of SEQ ID No. 105), investigated CpG (genomic position GRCh37:11:69591383, nt 1001 of SEQ ID No. 105)
**Seq ID no.106, PCDHAC2 -** GRCh37:5:140345922-140347922, Probe design region (genomic position GRCh37:5:140346861-140346982, nt 940 to nt 1061 of SEQ ID No. 106), investigated CpG (genomic position GRCh37:5:140346922 nt 1000 of SEQ ID No. 106)
**Seq ID no.107, GRM4 -** GRCh37:6:34100527-34102527, Probe design region (genomic position GRCh37:6:34101466-34101587, nt 940 to nt 1061 of SEQ ID No. 107), investigated CpG (genomic position GRCh37:6:34101527, nt 1000 of SEQ ID No. 107)
**Seq ID no.108, ANXA13 -** GRCh37:8:124748565-124750565, Probe design region (genomic position GRCh37:8:124749504-124749625, nt 940 to nt 1061 of SEQ ID No. 108), investigated CpG (genomic position GRCh37:8:124749565, nt 1000 of SEQ ID No. 108)
**Seq ID no.109, CTAG2 -** GRCh37:X:153880680-153882680, Probe design region (genomic position GRCh37:X:153880619-153880740, nt 940 to nt 1061 of SEQ ID No. 109), investigated CpG (genomic position GRCh37:X:153880680, nt 1000 of SEQ ID No. 109)
**Seq ID no.110, PVALB -** GRCh37:22:37215968-37217068, Probe design region (genomic position GRCh37:22:37216007-37216128, nt 940 to nt 1061 of SEQ ID No. 110), investigated CpG (genomic position GRCh37:22:37216068, nt 1000 of SEQ ID No. 110)
**Seq ID no.111, HAO2 -** GRCh37:1:119910481-119912481, Probe design region (genomic position GRCh37:1:119911420-119911541, nt 940 to nt 1061 of SEQ ID No. 111), investigated CpG (genomic position GRCh37:1:119911481, nt 1000 of SEQ ID No. 111)
**Seq ID no.112, SLC4A7 -** GRCh37:3:27497660-27499660, Probe design region (genomic position GRCh37:3:27498599-27498720, nt 940 to nt 1061 of SEQ ID No. 112), investigated CpG (genomic position GRCh37:3:27498660, nt 1000 of SEQ ID No. 112)
**Seq ID no.113, TDRD5 -** GRCh37:1:179560500-179562500, Probe design region (genomic position GRCh37:1:179561440-179561561, nt 941 to nt 1062 of SEQ ID No. 113), investigated CpG (genomic position GRCh37:1:179561500, nt 1001 of SEQ ID No. 113)
**Seq ID no.114, FAAH -** GRCh37:1:46858774-46860774, Probe design region (genomic position GRCh37:1:46859714-46859835, nt 941 to nt 1062 of SEQ ID No. 114), investigated CpG (genomic position GRCh37:1:46859774, nt 1001 of SEQ ID No. 114)
**Seq ID no.115, CD1D** - GRCh37:1:158148228-158150228, Probe design region (genomic position GRCh37:1:158149168-158149289, nt 941 to nt 1062 of SEQ ID No. 115), investigated CpG (genomic position GRCh37:1:158149228, nt 1001 of SEQ ID No. 115)
**Seq ID no.116, FLJ39501 -** GRCh37:19:15617560-15619560, Probe design region (genomic position GRCh37:19:15618499-15618620, nt 940 to nt 1061 of SEQ ID No. 116), investigated CpG (genomic position GRCh37:19:15618560, nt 1000 of SEQ ID No. 116)
**Seq ID no.117, AGLT1D1** - GRCh37:12:129336768-129338768, Probe design region (genomic position GRCh37:12:129337708-129337829, nt 941 to nt 1062 of SEQ ID No. 117), investigated CpG (genomic position GRCh37:12:129337768, nt 1001 of SEQ ID No. 117)
**Seq ID no.118, IL28B -** GRCh37:19:39734568-39736568, Probe design region (genomic position GRCh37:19:39735508-39735629, nt 941 to nt 1062 of SEQ ID No. 118), investigated CpG (genomic position GRCh37:19:39735568, nt 1001 of SEQ ID No. 118)
**Seq ID no.119, AVPR1B** - GRCh37:1:206222242-206224242, Probe design region (genomic position GRCh37:1:206223181-206223302, nt 940 to nt 1061 of SEQ ID No. 119), investigated CpG (genomic position GRCh37:1:206223242, nt 1000 of SEQ ID No. 119)
**Seq ID no.120, GJB3 -** GRCh37:1:35245607-35247607, Probe design region (genomic position GRCh37:1:35246547-35246668, nt 941 to nt 1062 of SEQ ID No. 120), investigated CpG (genomic position GRCh37:1:35246607, nt 1001 of SEQ ID No. 120)
**Seq ID no.121, GABRG3 -** GRCh37:15:27669336-27671336, Probe design region (genomic position GRCh37:15:27670275-27670396, nt 940 to nt 1061 of SEQ ID No. 121), investigated CpG (genomic position GRCh37:15:27670336, nt 1000 of SEQ ID No. 121)
**Seq ID no.122, CTAG1B -** GRCh37:X:153812580-153814580, Probe design region (genomic position GRCh37:X:153812520-153812641, nt 941 to nt 1062 of SEQ ID No. 122), investigated CpG (genomic position GRCh37:X:153812580, nt 1001 of SEQ ID No. 122)
**Seq ID no.123, UNQ9433 -** GRCh37:8:53477706-53479706, Probe design region (genomic position GRCh37:8:53478646-53478767, nt 941 to nt 1062 of SEQ ID No. 123), investigated CpG (genomic position GRCh37:8:53478706, nt 1001 of SEQ ID No. 123)
**Seq ID no.124, CEACAM6 -** GRCh37:19:42259486-42261486, Probe design region (genomic position GRCh37:19:42260425-42260546, nt 940 to nt 1061 of SEQ ID No. 124), investigated CpG (genomic position GRCh37:19:42260486, nt 1000 of SEQ ID No. 124)
**Seq ID no.125, DENND2D -** GRCh37:1:111742369-111744369, Probe design region (genomic position GRCh37:1:111743308-111743429, nt 940 to nt 1061 of SEQ ID No. 125), investigated CpG (genomic position GRCh37:1:111743369, nt 1000 of SEQ ID No. 125)
**Seq ID no.126, PGCP -** GRCh37:8:97656073-97658073, Probe design region (genomic position GRCh37:8:97657012-97657133, nt 940 to nt 1061 of SEQ ID No. 126), investigated CpG (genomic position GRCh37:8:97657073, nt 1000 of SEQ ID No. 126)
**Seq ID no.127, KCNG1** - GRCh37:20:49637967-49639967, Probe design region (genomic position GRCh37:20:49638907-49639028, nt 941 to nt 1062 of SEQ ID No. 127), investigated CpG (genomic position GRCh37:20:49638967, nt 1001 of SEQ ID No. 127)
**Seq ID no.128, FAM107B -** GRCh37:10:14815654-14817654, Probe design region (genomic position GRCh37:10:14816594-14816715, nt 941 to nt 1062 of SEQ ID No. 128), investigated CpG (genomic position GRCh37:10:14816654, nt 1001 of SEQ ID No. 128)
**Seq ID no.129, FANCE -** GRCh37:6:35419754-35421754, Probe design region (genomic position GRCh37:6:35420694-35420815, nt 941 to nt 1062 of SEQ ID No. 129), investigated CpG (genomic position GRCh37:6:35420754, nt 1001 of SEQ No. 129)
**Seq ID no.130, FAM38B -** GRCh37:18:10696032-10698032, Probe design region (genomic position GRCh37:18:10696972-10697093, nt 941 to nt 1062 of SEQ ID No. 130), investigated CpG (genomic position GRCh37:18:10697032, nt 1001 of SEQ ID No. 130)
**Seq ID no.131, FOXF2 -** GRCh37:6:1388146-1390146, Probe design region (genomic position GRCh37:6:1389086-1389207, nt 941 to nt 1062 of SEQ ID No. 131), investigated CpG (genomic position GRCh37:6:1389146, nt 1001 of SEQ ID No. 131)
**Seq ID no.132, BGN -** GRCh37:X:152759079-152761079, Probe design region (genomic position GRCh37:X:152760018-152760139, nt 940 to nt 1061 of SEQ ID No. 132), investigated CpG (genomic position GRCh37:X: 152760079, nt 1000 of SEQ ID No. 132)
**Seq ID no.133, MAGEA10 -** GRCh37:X:151305719-151307719, Probe design region (genomic position GRCh37:X:151306658-151306779, nt 940 to nt 1061 of SEQ ID No. 133), investigated CpG (genomic position GRCh37:X:151306719, nt 1000 of SEQ ID No. 133)
**Seq ID no.134, SPATS1 -** GRCh37:6:44309640-44311640, Probe design region (genomic position GRCh37:6:44310580-44310701, nt 941 to nt 1062 of SEQ ID No. 134), investigated CpG (genomic position GRCh37:6:44310640, nt 1001 of SEQ ID No. 134)
**Seq ID no.135, PRXX1 -** GRCh37:1:170631093-170633093, Probe design region (genomic position GRCh37:1:170632032-170632153, nt 940 to nt 1061 of SEQ ID No. 135), investigated CpG (genomic position GRCh37:1:170632093, nt 1000 of SEQ ID No. 135)
**Seq ID no.136, MLSTD1 -** GRCh37:12:29375483-29377483, Probe design region (genomic position GRCh37:12:29376423-29376544, nt 941 to nt 1062 of SEQ ID No. 136), investigated CpG (genomic position GRCh37:12:29376483, nt 1001 of SEQ ID No. 136)
**Seq ID no.137, PDZD4 -** GRCh37:X: 153095786-153097786, Probe design region (genomic position GRCh37:X:153096726-153096847, nt 941 to nt 1062 of SEQ ID No. 137), investigated CpG (genomic position GRCh37:X:153096786, nt 1001 of SEQ ID No. 137)
**Seq ID no.138, HCP5 -** GRCh37:6:31430101-31432101, Probe design region (genomic position GRCh37:6:31431040-31431161, nt 940 to nt 1061 of SEQ ID No. 138), investigated CpG (genomic position GRCh37:6:31431101, nt 1000 of SEQ ID No. 138)
**Seq ID no.139, OCIAD2 -** GRCh37:4:48907902-48909902, Probe design region (genomic position GRCh37:4:48908842-48908963, nt 941 to nt 1062 of SEQ ID No. 139), investigated CpG (genomic position GRCh37:4:48908902, nt 1001 of SEQ ID No. 139)
**Seq ID no.140, CSAG1 -** GRCh37:X:151882197-151884197, Probe design region (genomic position GRCh37:X:151883137-153096258, nt 940 to nt 1061 of SEQ ID No. 140), investigated CpG (genomic position GRCh37:X:151883197, nt 1000 of SEQ ID No. 140)
**Seq ID no.141, SLCO4A1** - GRCh37:20:61272056-61274056, Probe design region (genomic position GRCh37:20:61272995-61273116, nt 940 to nt 1061 of SEQ ID No. 141), investigated CpG (genomic position GRCh37:20:61273056, nt 1000 of SEQ ID No. 141)
**Seq ID no.142, C9orf90** - GRCh37:9:130827969-130829969, Probe design region (genomic position GRCh37:9:130828909-130829030, nt 941 to nt 1062 of SEQ ID No. 142), investigated CpG (genomic position GRCh37:9:130828969, nt 1001 of SEQ ID No. 142)
**Seq ID no.143, CSAG1** - GRCh37:X:151902447-151904447, Probe design region (genomic position GRCh37:X:151903387-151903508, nt 941 to nt 1062 of SEQ ID No. 143), investigated CpG (genomic position GRCh37:X:151903447, nt 1001 of SEQ ID No. 143)
**Seq ID no.144, AMPH -** GRCh37:7:38670659-38672659, Probe design region (genomic position GRCh37:7:38671599-38671720, nt 941 to nt 1062 of SEQ ID No. 144), investigated CpG (genomic position GRCh37:7:38671659, nt 1001 of SEQ ID No. 144)
**Seq ID no.145, MT1G** - GRCh37:16:56701134-56703134, Probe design region (genomic position GRCh37:16:56702074-56702195, nt 941 to nt 1062 of SEQ ID No. 145), investigated CpG (genomic position GRCh37:16:56702134, nt 1001 of SEQ ID No. 145)
**Seq ID no.146, C20orf100** - GRCh37:20:42543648-42545648, Probe design region (genomic position GRCh37:20:42544588-42544709, nt 941 to nt 1062 of SEQ ID No. 146), investigated CpG (genomic position GRCh37:20:42544648, nt 1001 of SEQ ID No. 146)
**Seq ID no.147, CST7 -** GRCh37:20:24928716-24930716, Probe design region (genomic position GRCh37:20:24929656-24929777, nt 941 to nt 1062 of SEQ ID No. 147), investigated CpG (genomic position GRCh37:17:24929716, nt 1001 of SEQ ID No. 147)
**Seq ID no.148, KIAA0367 -** GRCh37:9:79327644-79329644, Probe design region (genomic position GRCh37:9:79328584-79328705, nt 941 to nt 1062 of SEQ ID No. 148), investigated CpG (genomic position GRCh37:9:79328644, nt 1001 of SEQ ID No. 148)
**Seq ID no.149, RNASE1-** GRCh37:14:21270313-21272313, Probe design region (genomic position GRCh37:14:21271253-21271374, nt 941 to nt 1062 of SEQ ID No. 149), investigated CpG (genomic position GRCh37:14:21271313, nt 1001 of SEQ ID No. 149)
**Seq ID no.150, HCP5** - GRCh37:6:31429916-31431916, Probe design region (genomic position GRCh37:6:31430856-31430974, nt 941 to nt 1062 of SEQ ID No. 150), investigated CpG (genomic position GRCh37:6:31430916, nt 1001 of SEQ ID No. 150)
**Seq ID no.151, NDN -** GRCh37:15:23931374:23933374, Probe design region (genomic position GRCh37:15:23932313-23932434, nt 940 to nt 1061 of SEQ ID No. 151), investigated CpG (genomic position GRCh37:15:23932374, nt 1000 of SEQ ID No. 151)
**Seq ID no.152, RAC2 -** GRCh37:22:37639403-37641403, Probe design region (genomic position GRCh37:22:37640342-37640463, nt 940 to nt 1061 of SEQ ID No. 152), investigated CpG (genomic position GRCh37:22:37640403, nt 1000 of SEQ ID No. 152)
**Seq ID no.153, AXL -** GRCh37:19:41724333-41726333, Probe design region (genomic position GRCh37:19:41725272-41725393, nt 940 to nt 1061 of SEQ ID No. 153), investigated CpG (genomic position GRCh37:19:41725333, nt 1000 of SEQ ID No. 153)
**Seq ID no.154, BATF -** GRCh37:14:75987747-75989747, Probe design region (genomic position GRCh37:14:75988687-75988808, nt 941 to nt 1062 of SEQ ID No. 154), investigated CpG (genomic position GRCh37:14:75988747, nt 1001 of SEQ ID No. 154)
**Seq ID no.155, EMILIN1 -** GRCh37:2:27300490-27302490, Probe design region (genomic position GRCh37:2:27301430-27301551, nt 941 to nt 1062 of SEQ ID No. 155), investigated CpG (genomic position GRCh37:2:27301490, nt 1001 of SEQ ID No. 155)
**Seq ID no.156, ARHGEF7 -** GRCh37:13:111765903:111767903, Probe design region (genomic position GRCh37:13:111766842-111766963, nt 940 to nt 1061 of SEQ ID No. 156), investigated CpG (genomic position GRCh37:13:111766903, nt 1000 of SEQ ID No. 156)
**Seq ID no.157, EVL -** GRCh37:14:100531037-100533037, Probe design region (genomic position GRCh37:14:100531976-100532097, nt 940 to nt 1061 of SEQ ID No. 157), investigated CpG (genomic position GRCh37:14:100532037, nt 1000 of SEQ ID No. 157)
**Seq ID no.158, RAC2 -** GRCh37:22:37639251-37641251, Probe design region (genomic position GRCh37:22:37640190-37640311, nt 940 to nt 1061 of SEQ ID No. 158), investigated CpG (genomic position GRCh37:22:37640251, nt 1000 of SEQ ID No. 158)
**Seq ID no.159, KIAA0367 -** GRCh37:9:79327522-79329522, Probe design region (genomic position GRCh37:9:79328462-79328583, nt 941 to nt 1062 of SEQ ID No. 159), investigated CpG (genomic position GRCh37:9:79328522, nt 1001 of SEQ ID No. 159)
**Seq ID no.160, TFF3 -** GRCh37:21:43735927-43737927, Probe design region (genomic position GRCh37:21:43736866-43736987, nt 940 to nt 1061 of SEQ ID No. 160), investigated CpG (genomic position GRCh37:21:43736927, nt 1000 of SEQ ID No. 160)
**Seq ID no.161, CTSK -** GRCh37:1:150779684-150781684, Probe design region (genomic position GRCh37:1:150780624-150780745, nt 941 to nt 1062 of SEQ ID No. 161), investigated CpG (genomic position GRCh37:1:150780684, nt 1001 of SEQ ID No. 161)
**Seq ID no.162, C21orf70** - GRCh37:21:46357703-46359703, Probe design region (genomic position GRCh37:21:46358642-46358763, nt 940 to nt 1061 of SEQ ID No. 162), investigated CpG (genomic position GRCh37:21:46358703, nt 1000 of SEQ ID No. 162)
**Seq ID no.163, PRKCZ -** GRCh37:1:1980271-1982271, Probe design region (genomic position GRCh37:1:1981210-1981331, nt 940 to nt 1061 of SEQ ID No. 163), investigated CpG (genomic position GRCh37:1:1981271, nt 1000 of SEQ ID No. 163)
**Seq ID no.164, MLSTD1 -** GRCh37:12:29375873-29377873, Probe design region (genomic position GRCh37:12:29376812-29376933, nt 940 to nt 1061 of SEQ ID No. 164), investigated CpG (genomic position GRCh37:12:29376873, nt 1000 of SEQ ID No. 164)
**Seq ID no.165, COL5A2** - GRCh37:2:190043636-190045636, Probe design region (genomic position GRCh37:2:190044576-190044697, nt 941 to nt 1062 of SEQ ID No. 165), investigated CpG (genomic position GRCh37:2:190044636, nt 1001 of SEQ ID No. 165)
**Seq ID no.166, LY96 -** GRCh37:8:74902801-74904801, Probe design region (genomic position GRCh37:8:74903741-74903862, nt 941 to nt 1062 of SEQ ID No. 166), investigated CpG (genomic position GRCh37:8:74903801, nt 1001 of SEQ ID No. 166)
**Seq ID no.167, MAGE1 -** GRCh37:X:152484938-152486938, Probe design region (genomic position GRCh37:X:152485878-152485999, nt 941 to nt 1062 of SEQ ID No. 167), investigated CpG (genomic position GRCh37:X: 152485938, nt 1001 of SEQ ID No. 167)
**Seq ID no.168, MAGEC2 -** GRCh37:X:141292020-141294020, Probe design region (genomic position GRCh37:X:141292960-141293081, nt 941 to nt 1062 of SEQ ID No. 168), investigated CpG (genomic position GRCh37:X:141293020, nt 1001 of SEQ ID No. 168)
**Seq ID no.169, DENND2D -** GRCh37:1:111742271-111744271, Probe design region (genomic position GRCh37:1:111743211-111743332, nt 941 to nt 1062 of SEQ ID No. 169), investigated CpG (genomic position GRCh37:1:111743271, nt 1001 of SEQ ID No. 169)
**Seq ID no.170, PPIE -** GRCh37:1:40203286-40205286, Probe design region (genomic position GRCh37:1:40204225-40204346, nt 940 to nt 1061 of SEQ ID No. 170), investigated CpG (genomic position GRCh37:1:40204286, nt 1000 of SEQ ID No. 170)
**Seq ID no.171, SORBS2 -** GRCh37:4:186876779-186878779, Probe design region (genomic position GRCh37:4:186877718-186877839, nt 940 to nt 1061 of SEQ ID No. 171), investigated CpG (genomic position GRCh37:4:186877779, nt 1000 of SEQ ID No. 171)
**Seq ID no.172, PROCR -** GRCh37:20:33759017-33761017, Probe design region (genomic position GRCh37:20:33759957-33760078, nt 941 to nt 1062 of SEQ ID No. 172), investigated CpG (genomic position GRCh37:20:33760017, nt 1001 of SEQ ID No. 172)
**Seq ID no.173, TMEPAI -** GRCh37:20:56285392-56287392, Probe design region (genomic position GRCh37:20:56286331-56286452, nt 940 to nt 1061 of SEQ ID No. 173), investigated CpG (genomic position GRCh37:20:56286392, nt 1000 of SEQ ID No. 173)
**Seq ID no.174, SSNA1 -** GRCh37:9:140080916-140082916, Probe design region (genomic position GRCh37:9:140081856-140081977, nt 941 to nt 1062 of SEQ ID No. 174), investigated CpG (genomic position GRCh37:9:140081916, nt 1001 of SEQ ID No. 174)
**Seq ID no.175, MGMT -** GRCh37:10:131263840-131265840, Probe design region (genomic position GRCh37:10:131264780-131264901, nt 941 to nt 1062 of SEQ ID No. 175), investigated CpG (genomic position GRCh37:10:131264840, nt 1001 of SEQ ID No. 175)
**Seq ID no.176, GAGE2 -** GRCh37:X:49225695-49227695, Probe design region (genomic position GRCh37:X:49226635-49226756, nt 941 to nt 1062 of SEQ ID No. 176), investigated CpG (genomic position GRCh37:X:49226695, nt 1001 of SEQ ID No. 176)
**Seq ID no.177, C11orf1 -** GRCh37:11:111749355-111751355, Probe design region (genomic position GRCh37:11:111750295-111750416, nt 941 to nt 1062 of SEQ ID No. 177), investigated CpG (genomic position GRCh37:11:111750355, nt 1001 of SEQ ID No. 177)
**Seq ID no.178, ABCB4 -** GRCh37:7:87108371-87110371, Probe design region (genomic position GRCh37:7:87109310-87109431, nt 940 to nt 1061 of SEQ ID No. 178), investigated CpG (genomic position GRCh37:7:87109371, nt 1000 of SEQ ID No. 178)
**Seq ID no.179, CTNNAL1** - GRCh37:9:111775779-111777779, Probe design region (genomic position GRCh37:9:111776719-111776840, nt 941 to nt 1062 of SEQ ID No. 179), investigated CpG (genomic position GRCh37:9:111776779, nt 1001 of SEQ ID No. 179)
Seq ID **no.180,** EMP1 - GRCh37:12:13347429-13349429, Probe design region (genomic position GRCh37:12:13348369-13348490, nt 941 to nt 1062 of SEQ ID No. 180), investigated CpG (genomic position GRCh37:12:13348429, nt 1001 of SEQ ID No. 180)
**Seq ID no.181, RNASE1 -** GRCh37:14:21269924-21271924, Probe design region (genomic position GRCh37:14:21270864-21270985, nt 941 to nt 1062 of SEQ ID No. 181), investigated CpG (genomic position GRCh37:14:21270924, nt 1001 of SEQ ID No. 181)
**Seq ID no.182, MAGEA6 -** GRCh37:X:151866333-151868333, Probe design region (genomic position GRCh37:X:151867273-151867394, nt 941 to nt 1062 of SEQ ID No. 182), investigated CpG (genomic position GRCh37:X:151867333, nt 1001 of SEQ ID No. 182)
**Seq ID no.183, CHMP4A -** GRCh37:14:24682529-24684529, Probe design region (genomic position GRCh37:14: 24683468-24683589, nt 940 to nt 1061 of SEQ ID No. 183), investigated CpG (genomic position GRCh37:14:24683529, nt 1000 of SEQ ID No. 183)
**Seq ID no.184, ADORA2B -** GRCh37:17:15846413-15848413, Probe design region (genomic position GRCh37:17:15847352-15847473, nt 940 to nt 1061 of SEQ ID No. 184), investigated CpG (genomic position GRCh37:17:15847413, nt 1000 of SEQ ID No. 184)
**Seq ID no.185, PLA1A** - GRCh37:3:119309382-119311382, Probe design region (genomic position GRCh37:3:119310321-119310442, nt 940 to nt 1061 of SEQ ID No. 185), investigated CpG (genomic position GRCh37:3:119310382, nt 1000 of SEQ ID No. 185)
**Seq ID no.186, KIAA0141 -** GRCh37:5:141301730-141303730, Probe design region (genomic position GRCh37:3:141302669-141302790, nt 940 to nt 1061 of SEQ ID No. 186), investigated CpG (genomic position GRCh37:3:141302730, nt 1000 of SEQ ID No. 186)
**Seq ID no.187, OLFM1 -** GRCh37:9:137965585-137967585, Probe design region (genomic position GRCh37:9:137966524-137966645, nt 940 to nt 1061 of SEQ ID No. 187), investigated CpG (genomic position GRCh37:9:137966585, nt 1000 of SEQ ID No. 187)
**Seq ID no.188, ERO1L** - GRCh37:14:53162205-53164205, Probe design region (genomic position GRCh37:14:53163144-53163265, nt 940 to nt 1061 of SEQ ID No. 188), investigated CpG (genomic position GRCh37:14:53163205, nt 1000 of SEQ ID No. 188)
**Seq ID no.189, CXorf12** - GRCh37:X:153237580-153239580, Probe design region (genomic position GRCh37:X:153238519-153238640, nt 940 to nt 1061 of SEQ ID No. 189), investigated CpG (genomic position GRCh37:X:153238580, nt 1000 of SEQ ID No. 189)
**Seq ID no.190, ARHGDIB -** GRCh37:12:15113704-15115704, Probe design region (genomic position GRCh37:12:15114643-15114764, nt 940 to nt 1061 of SEQ ID No. 190), investigated CpG (genomic position GRCh37:12:15114704, nt 1000 of SEQ ID No. 190)
**Seq ID no.191, GRAMD3 -** GRCh37:5:125758291-125760291, Probe design region (genomic position GRCh37:5:125759231-125759352, nt 941 to nt 1062 of SEQ ID No. 191), investigated CpG (genomic position GRCh37:5:125759291, nt 1001 of SEQ ID No. 191)
**Seq ID no.192, PLD1 -** GRCh37:3:171454827-171456827, Probe design region (genomic position GRCh37:3:171455766-171455887, nt 940 to nt 1061 of SEQ ID No. 192), investigated CpG (genomic position GRCh37:3:171455827, nt 1000 of SEQ ID No. 192)
**Seq ID no.193, HNMT -** GRCh37:2:138720318-138722318, Probe design region (genomic position GRCh37:2:138721257-138721378, nt 940 to nt 1061 of SEQ ID No. 193), investigated CpG (genomic position GRCh37:2:138721318, nt 1000 of SEQ ID No. 193)
**Seq ID no.194, ZNF558 -** GRCh37:19:8933543-8935543, Probe design region (genomic position GRCh37:19:8934483-8934604, nt 941 to nt 1062 of SEQ ID No. 194), investigated CpG (genomic position GRCh37:19:8934543, nt 1001 of SEQ ID No. 194)
**Seq ID no.195, ATP6V1E2** - GRCh37:2:46745935-46747935, Probe design region (genomic position GRCh37:2:46746874-46746995, nt 940 to nt 1061 of SEQ ID No. 195), investigated CpG (genomic position GRCh37:2:46746935, nt 1000 of SEQ ID No. 195)
**Seq ID no.196, KRT25C -** GRCh37:17:38939133-38941133, Probe design region (genomic position GRCh37:17:38940072-38940193, nt 940 to nt 1061 of SEQ ID No. 196), investigated CpG (genomic position GRCh37:17:38940133, nt 1000 of SEQ ID No. 196)
**Seq ID no.197, ZNF532 -** GRCh37:18:56528784-56530784, Probe design region (genomic position GRCh37:18:56529724-56529845, nt 941 to nt 1062 of SEQ ID No. 197), investigated CpG (genomic position GRCh37:18:56529784, nt 1001 of SEQ ID No. 197)
**Seq ID no.198, SLAMF9 -** GRCh37:1:159924416-159926416, Probe design region (genomic position GRCh37:1:159925356-159925477, nt 941 to nt 1062 of SEQ ID No. 198), investigated CpG (genomic position GRCh37:1:159925416, nt 1001 of SEQ ID No. 198)
**Seq ID no.199, NBL1 -** GRCh37:1:19967885-19969885, Probe design region (genomic position GRCh37:1:19968825-19968946, nt 941 to nt 1062 of SEQ ID No. 199), investigated CpG (genomic position GRCh37:1:19968885, nt 1001 of SEQ ID No. 199)
**Seq ID no.200, MAGEA10 -** GRCh37:X:151306054-151308054, Probe design region (genomic position GRCh37:X:151306993-151307114, nt 940 to nt 1061 of SEQ ID No. 200), investigated CpG (genomic position GRCh37:X:151307054, nt 1000 of SEQ ID No. 200)
**Seq ID no.201, ABCA8 -** GRCh37:17:66949546-66951546, Probe design region (genomic position GRCh37:17:66950485-66950606, nt 940 to nt 1061 of SEQ ID No. 201), investigated CpG (genomic position GRCh37:17:66950546, nt 1000 of SEQ ID No. 201)
**Seq ID no.202, ACOX2 -** GRCh37:3:58521690-58523690, Probe design region (genomic position GRCh37:3:58522629-58522750, nt 940 to nt 1061 of SEQ ID No. 202), investigated CpG (genomic position GRCh37:3:58522690, nt 1000 of SEQ ID No. 202)
**Seq ID no.203, TIMP3 -** GRCh37:22:33196035-33198035, Probe design region (genomic position GRCh37:22:33196974-33197095, nt 940 to nt 1061 of SEQ ID No. 203), investigated CpG (genomic position GRCh37:22:33197035, nt 1000 of SEQ ID No. 203)
**Seq ID no.204, RPUSD3 -** GRCh37:3:9885233-9887233, Probe design region (genomic position GRCh37:3:9886172-9886293, nt 940 to nt 1061 of SEQ ID No. 204), investigated CpG (genomic position GRCh37:3:9886233, nt 1000 of SEQ ID No. 204)
**Seq ID no.205, PRDX2 -** GRCh37:19:12911528-12913528, Probe design region (genomic position GRCh37:19:12912467-12912588, nt 940 to nt 1061 of SEQ ID No. 205), investigated CpG (genomic position GRCh37:19:12912528, nt 1000 of SEQ ID No. 205)
**Seq ID no.206, PCOLCE -** GRCh37:7:100199010-100201010, Probe design region (genomic position GRCh37:7:100199949-100200070, nt 940 to nt 1061 of SEQ ID No. 206), investigated CpG (genomic position GRCh37:7:100200010, nt 1000 of SEQ ID No. 206)
**Seq ID no.207, OCIAD2-** GRCh37:4:48907469-48909469, Probe design region (genomic position GRCh37:4:48908409-48908530, nt 941 to nt 1062 of SEQ ID No. 207), investigated CpG (genomic position GRCh37:4:48908469, nt 1001 of SEQ ID No. 207)
**Seq ID no.208, CTSK -** GRCh37:1:150780435-150782435, Probe design region (genomic position GRCh37:1:150781374-150781495, nt 940 to nt 1061 of SEQ ID No. 208), investigated CpG (genomic position GRCh37:1:150781435, nt 1000 of SEQ ID No. 208)
**Seq ID no.209, ADM -** GRCh37:11:10325924-10327924, Probe design region (genomic position GRCh37:11:10326863-10326984, nt 940 to nt 1061 of SEQ ID No. 209), investigated CpG (genomic position GRCh37:11:10326924, nt 1000 of SEQ ID No. 209)
**Seq ID no.210, PAGE5** - GRCh37:X:55245456-55247456, Probe design region (genomic position GRCh37:X:55246395-55246516, nt 940 to nt 1061 of SEQ ID No. 210), investigated CpG (genomic position GRCh37:X:55246456, nt 1000 of SEQ ID No. 210)
**Seq ID no.211, NNMT -** GRCh37:11:114165636-114167636, Probe design region (genomic position GRCh37:11:114166576-114166697, nt 941 to nt 1062 of SEQ ID No. 211), investigated CpG (genomic position GRCh3 7:11:11416663 6, nt 1001 of SEQ ID No. 211)
**Seq ID no.212, MAGEA3 -** GRCh37:X:151866333-151868333, Probe design region (genomic position GRCh37:X:151867273-151867394, nt 941 to nt 1062 of SEQ ID No. 212), investigated CpG (genomic position GRCh37:X:151867333, nt 1001 of SEQ ID No. 212)
**Seq ID no.213, RAB11FIP5 -** GRCh37:2:73338228-73340228, Probe design region (genomic position GRCh37:2:73339168-73339289, nt 941 to nt 1062 of SEQ ID No. 213), investigated CpG (genomic position GRCh37:2:73339228, nt 1001 of SEQ ID No. 213)
**Seq ID no.214, ARHGDIB -** GRCh37:12:15113394-15115394, Probe design region (genomic position GRCh37:12:15114333-15114454, nt 940 to nt 1061 of SEQ ID No. 214), investigated CpG (genomic position GRCh37:12:15114394, nt 1000 of SEQ ID No. 214)
**Seq ID no.215, EMILIN1 -** GRCh37:2:27300944-27302944, Probe design region (genomic position GRCh37:2:27301883-27302004, nt 940 to nt 1061 of SEQ ID No. 215), investigated CpG (genomic position GRCh37:2:27301944, nt 1000 of SEQ ID No. 215)
**Seq ID no.216, LPXN -** GRCh37:11:58341997-58343997, Probe design region (genomic position GRCh37:11:58342937-58343058, nt 941 to nt 1062 of SEQ ID No. 216), investigated CpG (genomic position GRCh37:11:58342997, nt 1001 of SEQ ID No. 216)
**Seq ID no.217, ALDH1A3** - GRCh37:15:101419636-101421636, Probe design region (genomic position GRCh37:15:101420576-101420697, nt 941 to nt 1062 of SEQ ID No. 217), investigated CpG (genomic position GRCh37:15:101420636, nt 1001 of SEQ ID No. 217)
**Seq ID no.218, SUHW1 -** GRCh37:22:22873678-22875678, Probe design region (genomic position GRCh37:22:22874617-22874738, nt 940 to nt 1061 of SEQ ID No. 218), investigated CpG (genomic position GRCh37:22:22874678, nt 1000 of SEQ ID No. 218)
**Seq ID no.219, GSTT1 -** GRCh37:22:24383160-24385160, Probe design region (genomic position GRCh37:22:24384099-24384220, nt 940 to nt 1061 of SEQ ID No. 219), investigated CpG (genomic position GRCh37:22:24384160, nt 1000 of SEQ ID No. 219)
**Seq ID no. 220, Human LINE-1 transposon (L1Hs) DNA,** GenBank accession n.: X58075, assay design region (nt 111 to nt 358 of SEQ ID No. 220), investigated CpGs (nt 252, 256 and 270 of SEQ ID No. 220).

The following examples are intended to illustrate, but not to limit, the scope of the invention. The examples are illustrated by reference to the non limiting following figures.

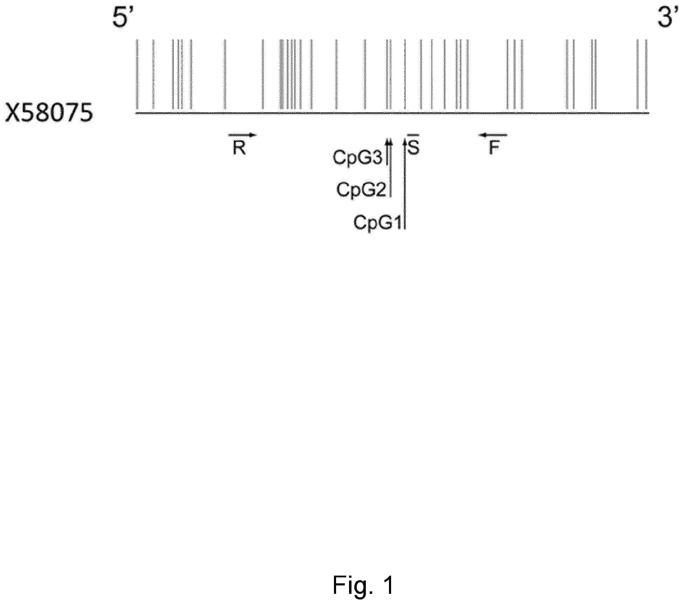

**Figure 1****.** *LINE-1* bisulfite pyrosequencing assay map.
   The region of the *LINE-1* sequence (GenBank accession n. X58075) utilized for the design of the assay is reported. Vertical bars indicate individual CpG sites. Horizontal lines indicate forward (F), reverse (R) and sequencing (S) primers. Vertical arrows indicate the CpG sites (CpG1, CpG2, CpG3) analyzed by pyrosequencing (adapted from Aparicio et al, Epigenetics, 4:176-84, 2009).

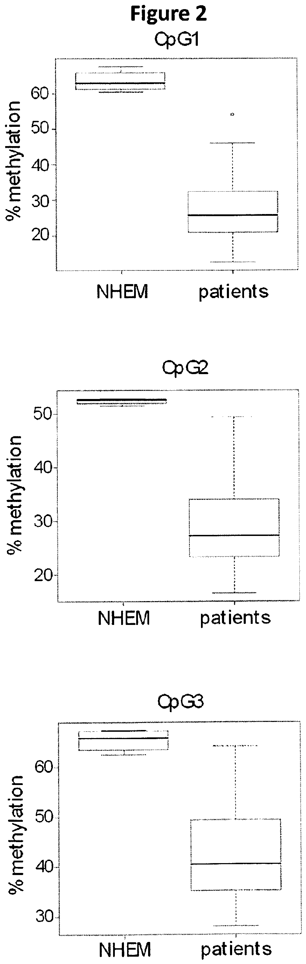

**Figure 2****.** *LINE-1*methylation in stage IIIC CM patients
   *LINE-1* methylation at 3 CpG sites (CpG1, CpG2, CpG3) was evaluated by bisulfite pyrosequencing analysis in 4 independent primary cultures of Normal Human Epidermal Melanocites (NHEM) and inshort-term cultures of CM cells generated from neoplastic lesions of 42 stage IIIC melanoma patients. All cells were analyzed at 6^{th} *in vitro* passage. Separate box plots have been generated for each of the CpG sites under analysis. Black horizontal bars represent the median values of methylation for each group.

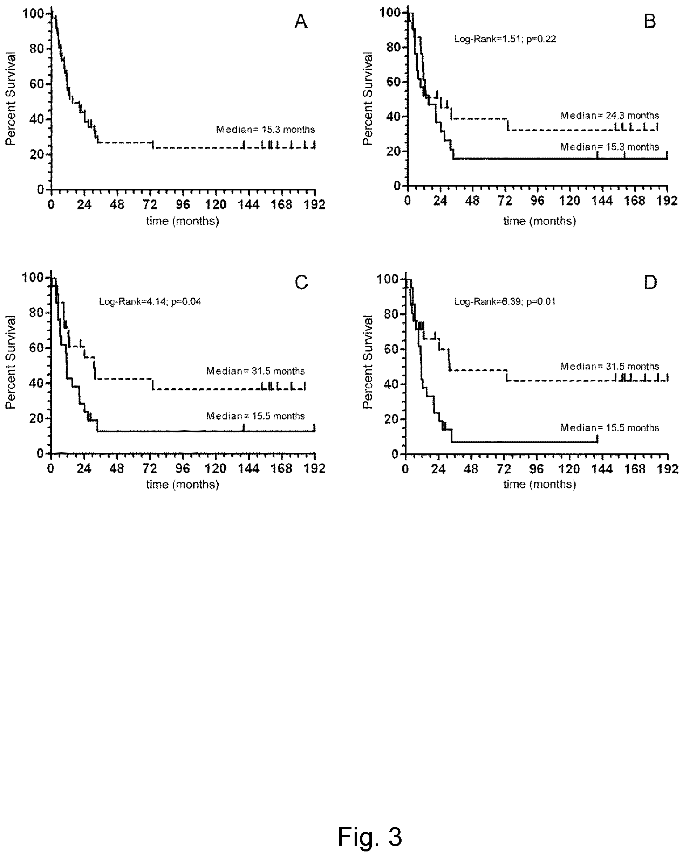

**Figure 3****.** Kaplan-Meier analysis of CM patients survival according to *LINE-1* methylation
   *LINE-1* methylation at 3 CpG sites (CpG1, CpG2, CpG3) was evaluated by bisulfite pyrosequencing analysis in short-term cultures of CM cells generated from neoplastic lesions of 42 stage IIIC melanoma patients. Cells were analyzed at 6^{th} *in vitro* passage. Kaplan- Meier function for OS was calculated for CM patients either unstratified (A) or stratified according to median methylation of CpG1 (B), CpG2 (C) or CpG3 (D) site of *LINE-1*elements. Dashed and solid lines refer to patients with *LINE-1* methylation below or above the median, respectively. Vertical bars represent censored patients. Cumulative survival by *LINE-1* methylation level was evaluated using the Log-Rank test, reported P values were two sided.

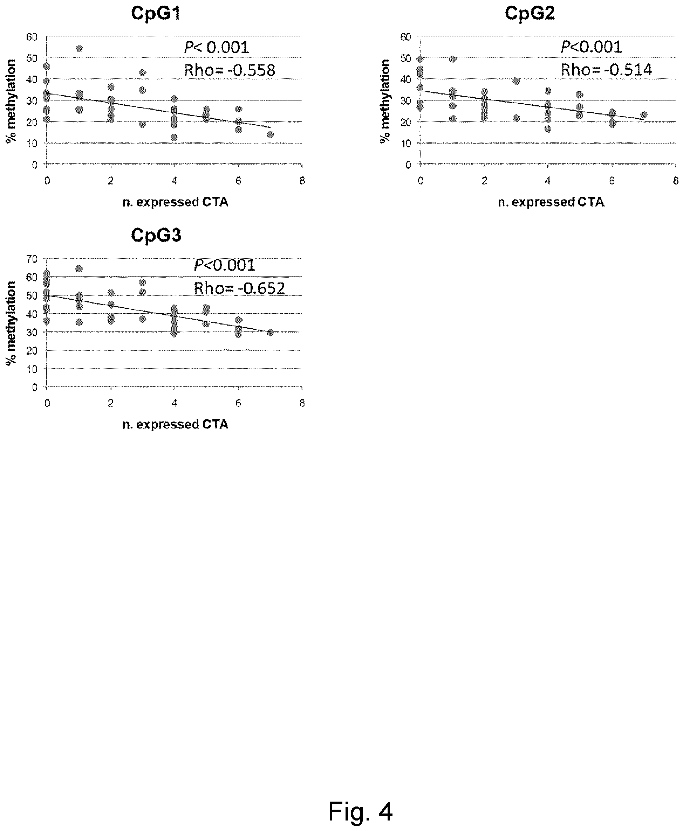

**Figure 4****.** *LINE-1* methylation correlates with the number of CTA concomitantly expressed by CM cells.
   *LINE-1* methylation at 3 CpG sites (CpG1, CpG2, CpG3) was evaluated by bisulfite pyrosequencing analysis in primary cultures of CM cells at the 6^{th} *in vitro* passage. Total RNA was extracted from the same cell cultures and subjected to RT-PCR analyses for the expression of the CTA MAGE-A1, -A2, -A3, -A4, -A10, SSX-2 and NY-ESO-1. For each cell culture, the number of different CTA expressed was evaluated. The correlation between *LINE-1* methylation at each CpG site and the number of expressed CTA was evaluated through Spearman's rank correlation test. P values < 0.05 were considered to be statistically significant.

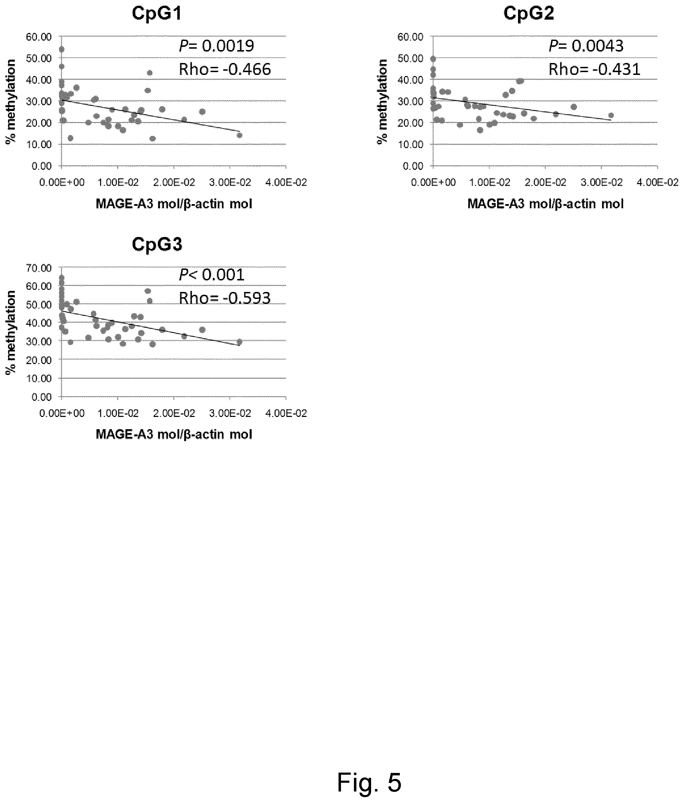

**Figure 5****.** *LINE-1* methylation correlates with the quantitative expression of MAGE-A3 by CM cells.
   *LINE-1* methylation at 3 CpG sites (CpG1, CpG2, CpG3) was evaluated by bisulfite pyrosequencing analysis in primary cultures of CM cells at the 6^{th} *in vitro* passage. Total RNA was extracted from the same cell cultures and subjected to quantitative RT-PCR analysis for the expression of the CTA MAGE-A3 and of the house-keeping gene β-actin. MAGE-A3 expression in each sample was normalized to that of β-actin, and reported as MAGE-A3 molecules/β-actin molecules. The correlation between *LINE-1* methylation at each CpG site and the quantitative levels of expression of MAGE-A3 was evaluated through Spearman's rank correlation test. P values < 0.05 were considered to be statistically significant.

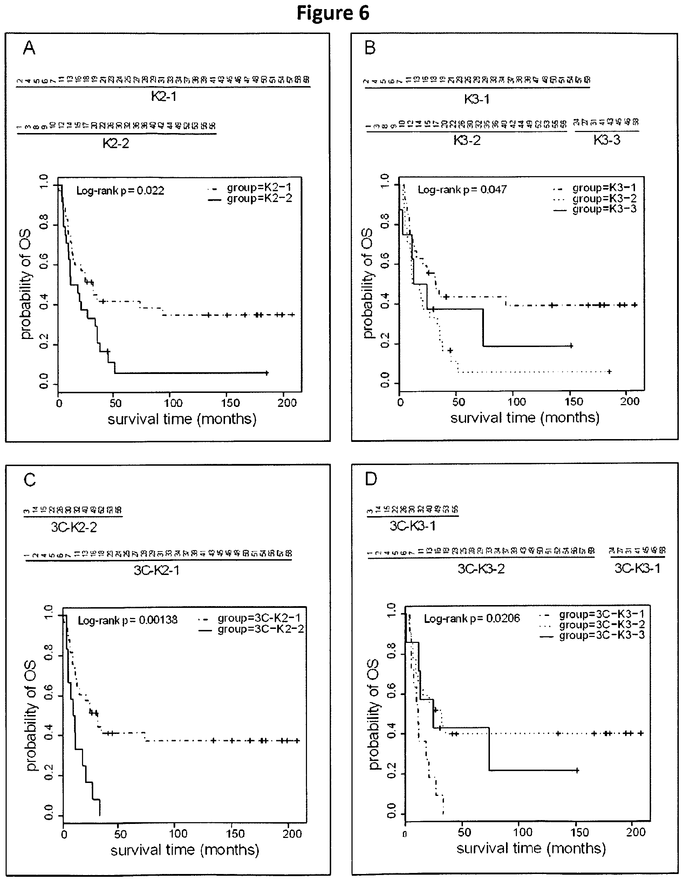

**Figure 6****.** Kaplan-Meier analysis of CM patients survival according to genome-wide methylation profiles
   Genome-wide DNA methylation profile was evaluated by Illumina HumanMethylation27 Bead-Chip whole-genome assay in short-term cultures of CM cells generated from neoplastic lesions of stage III CM patients. Cells were analyzed at 6^{th} *in vitro* passage. Patients were divided in either 2 or 3 classes based on the whole genome methylation profile of their tumor cells through the k-means algorithm. Kaplan-Meier function for OS was calculated for stage III and stage IIIC CM patients stratified according to k-means-defined methylation classes. Vertical bars in the Kaplan-Meier curves represent censored patients. Cumulative survival by k-means-defined methylation group was evaluated using the Log-Rank test, reported P values were two sided. Numbers above the horizontal bars in the upper portion of panels A-D represent the patients' numeric identifiers, horizontal bars and text below identify the k-means-defined methylation classes. Panel A, B, stage III patients stratified according to two (K2-1, K2-2) or three (K3-1, K3-2, K3-3) k-means-defined methylation classes, respectively; Panel C, D stage IIIC patients stratified according to two (3C-K2-1, 3C-K2-2) or three (3C-K3-1, 3C-K3-2, 3C-K3-3) k-means-defined methylation classes, respectively.

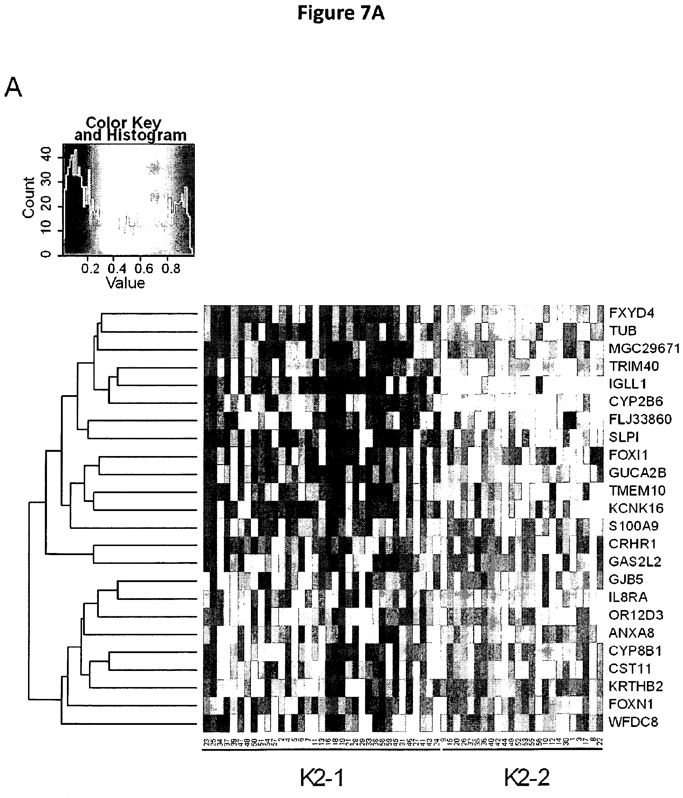

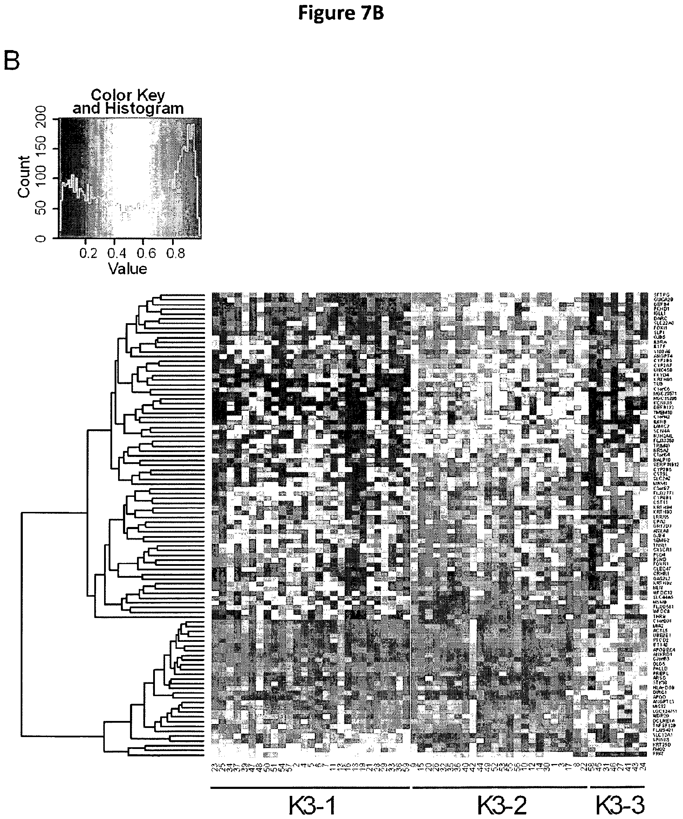

**Figure 7****.** Prognostic methylation signatures identified by the "nearest shrunken centroid" algorithm in stage III CM patients.
   Pre-processed genome-wide methylation data from stage III CM patients under study was analyzed through nearest shrunken centroids algorithm to identify genes best characterizing the k-means defined prognostic methylation groups. The methylation values of the genes composing the methylation signatures in the patients' population have been reported as a heatmap. Genes defining the signature have been reported as Gene Symbol on the right of the heatmap, while patient identifier numbers and the k-means-defined classes have been reported on foot of the heatmap. Each color patch represents the methylation level of one gene in each patient, with a continuum of methylation levels from dark blue (completely unmethylated, β=0) to dark red (completely methylated, β=1). Panel A, 24 genes signature classifying patients into 2 k-means-defined classes (K2-1 and K2-2) with an overall error rate of 0.05; Panel B, 98 genes signature classifying patients into 3 k-means-defined classes (K3-1, K3-2, and K3-3) with an overall error rate of 0.034.

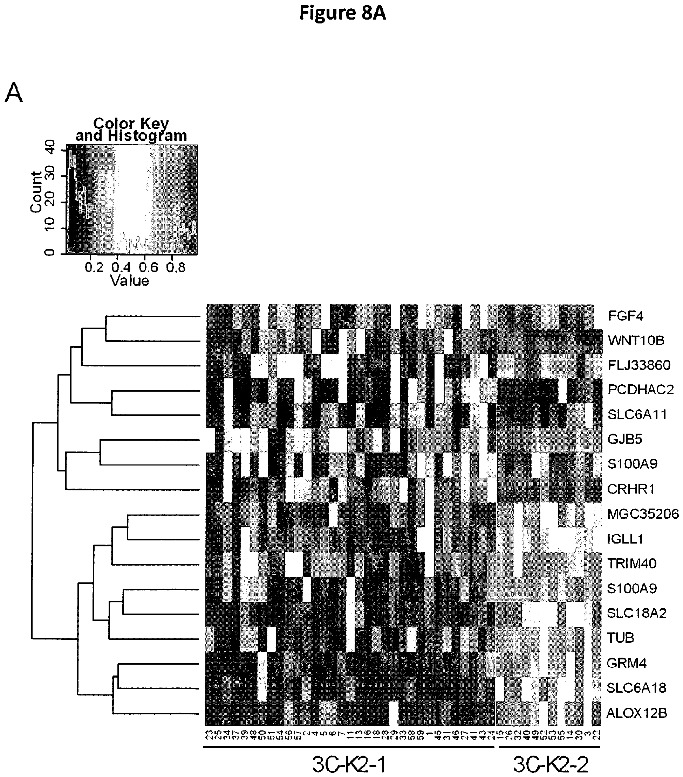

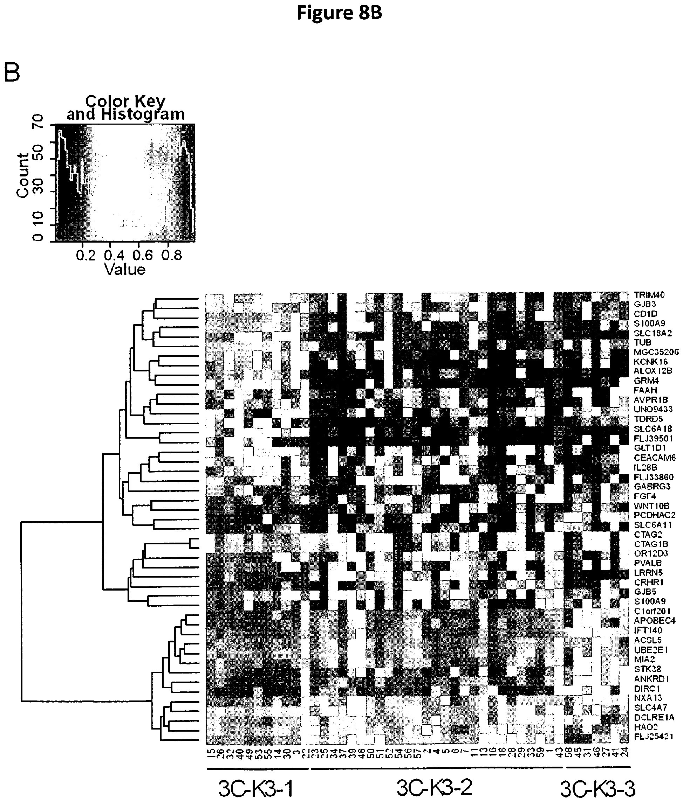

**Figure 8****.** Prognostic methylation signatures identified by the "nearest shrunken centroid" algorithm in stage IIIC CM patients.
   Pre-processed genome-wide methylation data from stage IIIC CM patients under study was analyzed through nearest shrunken centroids algorithm to identify genes best characterizing the k-means defined prognostic methylation groups. The methylation values of the genes composing the methylation signatures in the patients' population have been reported as a heatmap. Genes defining the signature have been reported as Gene Symbol on the right of the heatmap, while patient identifier numbers and the k-means-defined classes have been reported on foot of the heatmap. Each color patch represents the methylation level of one gene in each patient, with a continuum of methylation levels from dark blue (completely unmethylated, β=0) to dark red (completely methylated, β=1). Panel A, 17 genes signature classifying patients into 2 k-means-defined classes (3C-K2-1 and 3C-K2-2) with an overall error rate of 0; Panel B, 47 genes signature classifying patients into 3 k-means-defined classes (3C-K3-1, 3C-K3-2, and 3C-K3-3) with an overall error rate of 0.022.

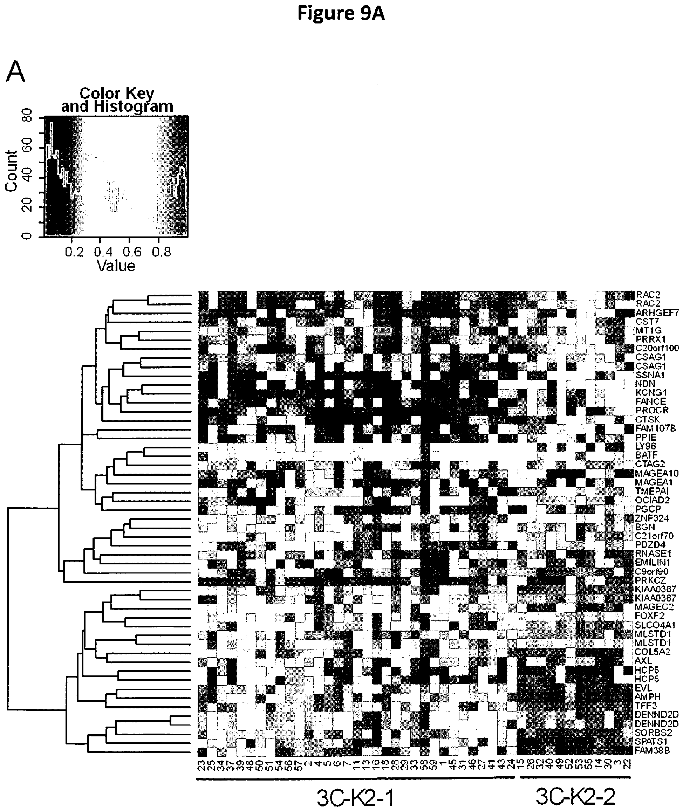

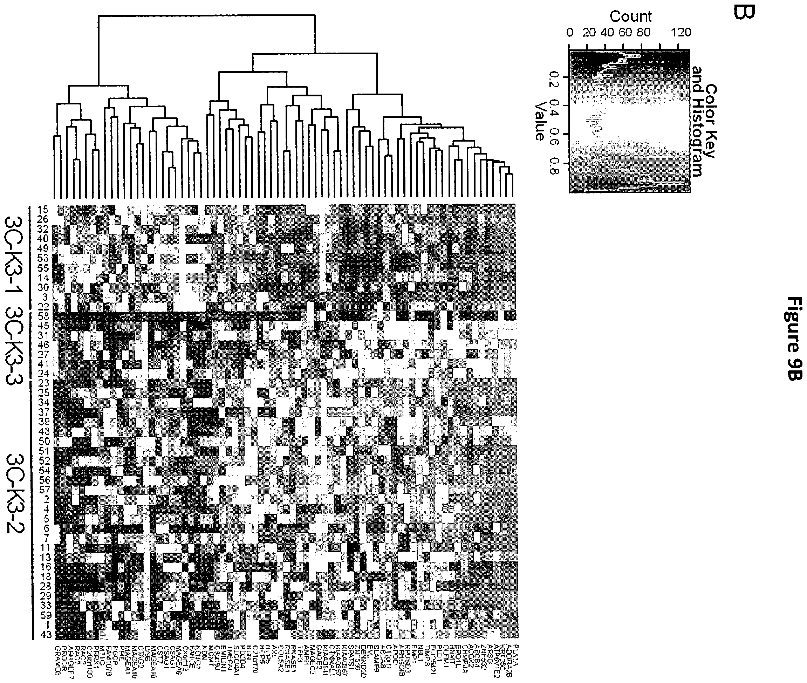

**Figure 9****.** Prognostic methylation signatures identified by the "nearest shrunken centroid" algorithm in stage IIIC CM patients using correlation between gene methylation and gene expression (p≤0.05).
   Pre-processed genome-wide methylation data from stage IIIC CM patients under study was analyzed through nearest shrunken centroids algorithm to identify genes best characterizing the k-means defined prognostic methylation groups. The analysis has been restricted to 820 methylation probes showing significant association between gene methylation and gene expression values in the same patient population, as evaluated by Spearman test. The methylation values of the genes composing the methylation signatures in the patients' population have been reported as a heatmap. Genes defining the signature have been reported as Gene Symbol on the right of the heatmap, while patient identifier numbers and the k-means-defined classes have been reported on foot of the heatmap. Each color patch represents the methylation level of one gene in each patient, with a continuum of methylation levels from dark blue (completely unmethylated, β=0) to dark red (completely methylated, β=1). Duplicate Gene Symbol entries refer to methylation data read for the same gene from different probes. Panel A, 51 genes signature classifying patients into 2 k-means-defined classes (3C-K2-1 and 3C-K2-2) with an overall error rate of 0.022; Panel B, 73 genes signature classifying patients into 3 k-means-defined classes (3C-K3-1, 3C-K3-2, and 3C-K3-3) with an overall error rate of 0.

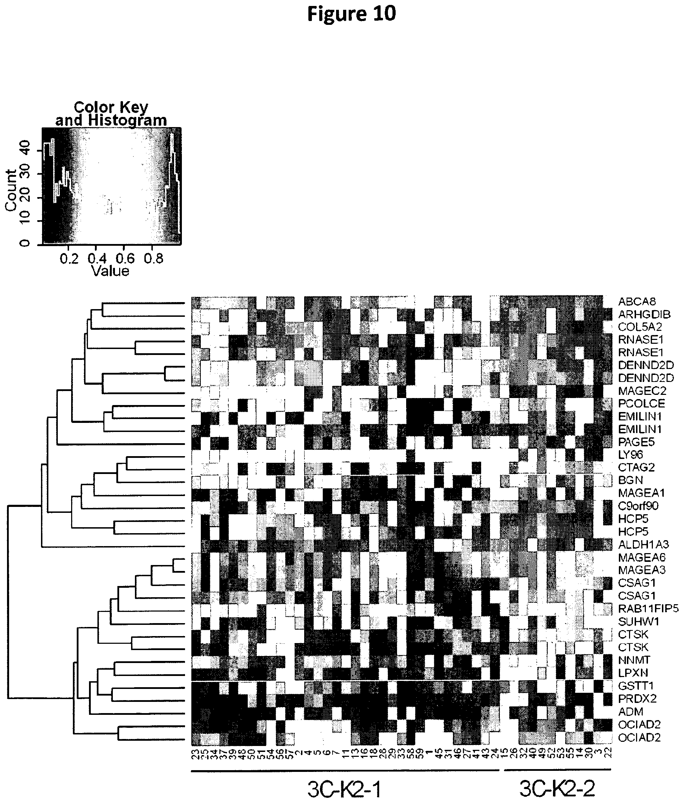

**Figure 10****.** Prognostic methylation signatures identified by the "nearest shrunken centroid" algorithm in stage IIIC CM patients using correlation between gene methylation and gene expression (p≤0.05, Rho≤-0.4).
   Pre-processed genome-wide methylation data from stage IIIC CM patients under study was analyzed through nearest shrunken centroids algorithm to identify genes best characterizing the k-means defined prognostic methylation groups. The analysis has been restricted to 274 methylation probes showing significant (p≤0.05) association between gene methylation and gene expression values in the same patient population, and with a coefficient of correlation Rho≤-0.4, as evaluated by Spearman test. The methylation values of the genes composing the methylation signatures in the patients' population have been reported as a heatmap. Genes defining the signature have been reported as Gene Symbol on the right of the heatmap, while patient identifier numbers and the k-means-defined classes have been reported on foot of the heatmap. Each color patch represents the methylation level of one gene in each patient, with a continuum of methylation levels from dark blue (completely unmethylated, β=0) to dark red (completely methylated, β=1). Duplicate Gene Symbol entries refer to methylation data read for the same gene from different probes.

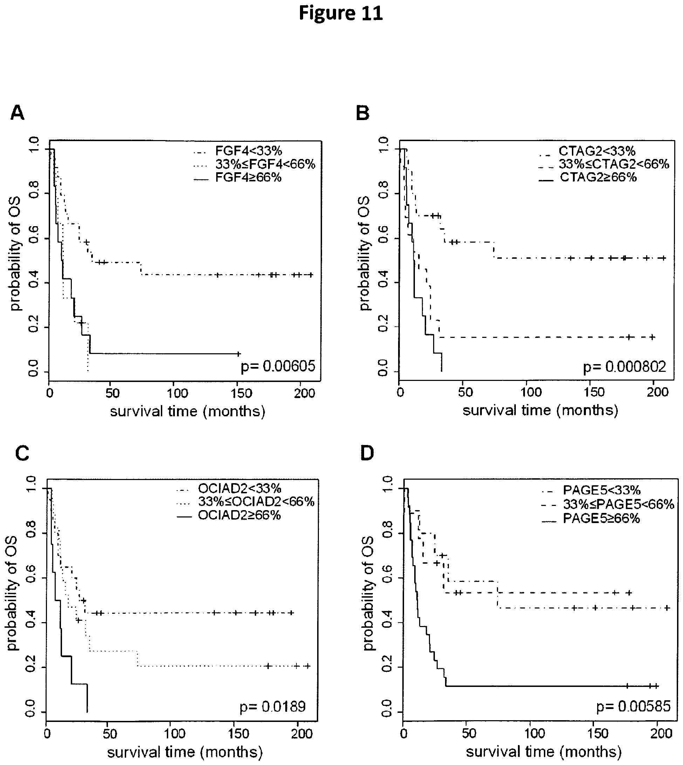

**Figure 11****.** Kaplan-Meier analysis of stage IIIC CM patients survival according to *FGF4, CTAG2, OCIAD2* or *PAGE5*methylation status. Genome-wide DNA methylation profile was evaluated by Illumina HumanMethylation27 Bead-Chip whole-genome assay in short-term cultures of CM cells generated from neoplastic lesions of stage IIIC CM patients. Cells were analyzed at 6^{th} *in vitro* passage. The methylation level of the genes*FGF4*(panel A), *CTAG2*(panel B), *OCIAD2* (panel C)and *PAGE5* (panel D), selected from the whole-genome defined prognostic methylation signatures E and G, have been utilized to subdivide patients according to the methylation level of each gene being <33%, between 33% and 66%, or >66%. Kaplan-Meier function for OS was calculated for stage IIIC patients stratified according to the methylation level of each gene. Vertical bars in the Kaplan-Meier curves represent censored patients. Cumulative survival by methylation group was evaluated using the Log-Rank test, reported P values were two sided.

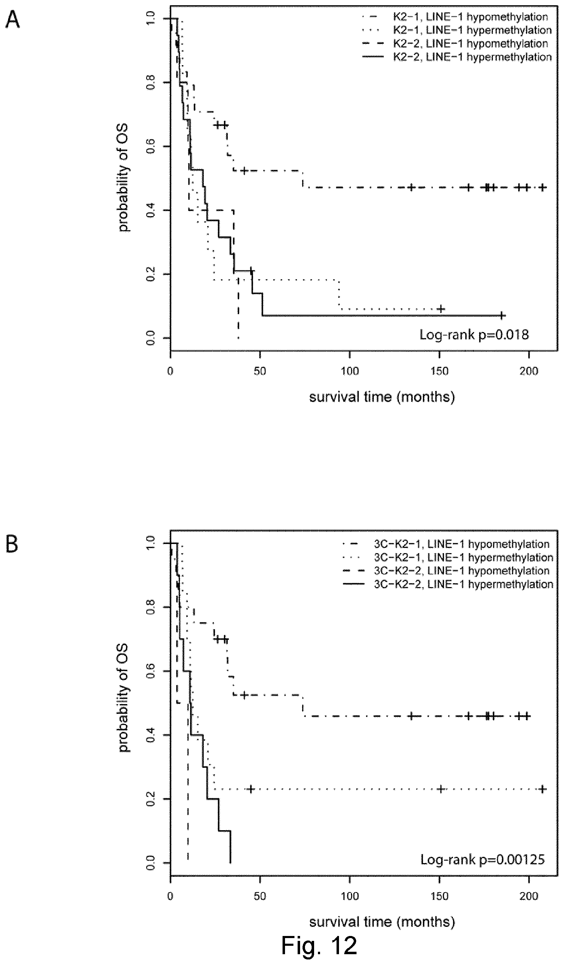

**Figure 12****.** Kaplan-Meier analysis of CM patients survival according to genome-wide methylation profiles and *LINE-1* methylation status
   Genome-wide DNA methylation profile was evaluated by Illumina HumanMethylation27 Bead-Chip whole-genome assay in short-term cultures of CM cells generated from neoplastic lesions of stage III CM patients. Cells were analyzed at 6^{th} *in vitro* passage. Patients were scored as K2-1 or k2-2 (stage III patients) or as S3-K2-1 or S3-K2-2 (substage IIIC patients) based on the whole genome methylation profile of their tumor cells through the k-means algorithm. Concomitantly, short-term cultures of CM cells were evaluated for the extent of *LINE-1* methylation by pyrosequencing, and scored as hypomethylated or hypermethylated depending on their value being < or ≥ median value, respectively. Kaplan-Meier function for OS was calculated for stage III (panel A) and stage IIIC (panel B) CM patients stratified according to both genome-wide methylation profile-defined classes and *LINE-1* methylation. Vertical bars in the Kaplan-Meier curves represent censored patients. Cumulative survival by methylation group was evaluated using the Log-Rank test, reported P values were two sided.

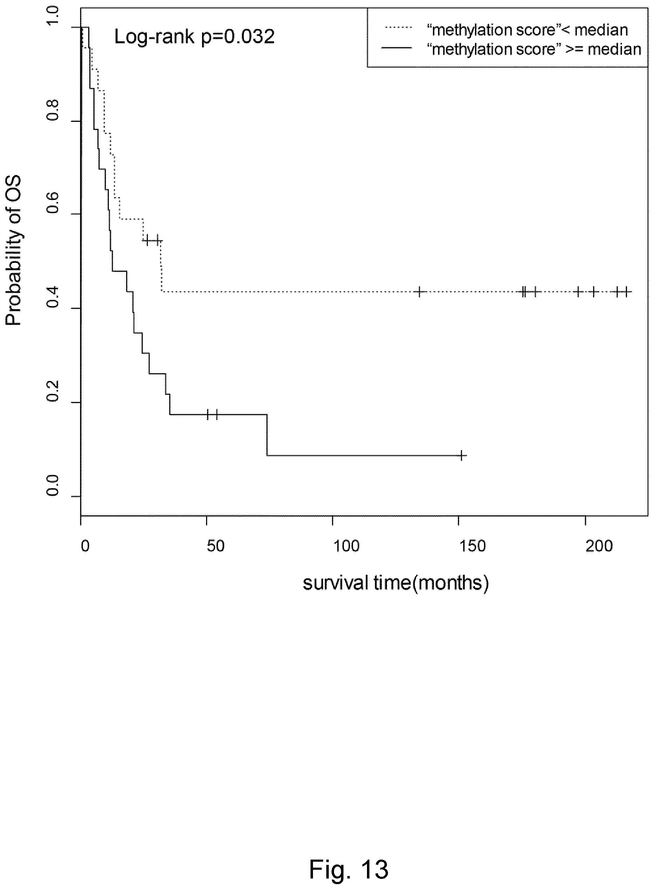

**Figure 13****.** Kaplan-Meier analysis of CM patients survival according to genome-wide methylation profile defined "methylation score".Genome-wide DNA methylation profile was evaluated by Illumina HumanMethylation27 Bead-Chip whole-genome assay in short-term cultures of CM cells generated from neoplastic lesions of stage III CM patients. Cells were analyzed at 6^{th} *in vitro* passage. The genome-wide methylation profile of each patient was summarized by the "methylation score". Patients were divided into 2 classes based on the "methylation score" of their tumor cells being < (dotted line) or ≥ (solid line) the median value of the patients' population. Kaplan-Meier function for OS was calculated for stage IIIC CM patients stratified according to the "methylation score". Vertical bars in the Kaplan-Meier curves represent censored patients. Cumulative survival by "methylation score"-defined groups was evaluated using the Log-Rank test, reported P values were two sided.

### DETAILED DESCRIPTION OF THE INVENTION

### Example 1 - Methylation levels of the "long interspersed nucleotide element-1 "repetitive sequences predict survival of melanoma patients

The prognosis of cutaneous melanoma (CM) differs for patients with identical clinico-pathological stage, and no molecular markers discriminating the prognosis of stage III individuals have been established. Genome-wide alterations in DNA methylation are a common event in cancer. This example defines the prognostic value of genomic DNA methylation levels in stage III CM patients.

Overall level of genomic DNA methylation was measured using bisulfite pyrosequencing at three CpG sites (CpG1, CpG2, CpG3) of the *Long Interspersed Nucleotide Element-1 (LINE-*1) sequences in short-term CM cultures from 42 stage IIIC patients. The impact of *LINE-1* methylation on overall survival (OS) was assessed using Cox regression and Kaplan-Meier analysis.

Here the authors provide the first evidence that the methylation level of *LINE-1* repetitive elements has a significant prognostic value in stage IIIC CM patients. Specifically, patients whose tumors exhibit more hypomethylated *LINE-1* sequences had a significantly longer overall survival. The reported data have significant prospective practical clinical implications. Indeed, it can be expected that the evaluation of *LINE-1* methylation status will: i) provide CM patients with improved clinico-pathological sub-staging information; ii) allow physicians to personalize follow-up procedures for CM patients ; iii) guide the future selection and/or stratification of patients for trials of adjuvant treatment; iv) select patients who are most likely to benefit from therapy, including DNA hypomethylating drugs. Overall, the evaluation of *LINE-1* methylation in the routine clinico-pathological ascertainment of CM patients is anticipated to greatly help in personalizing their comprehensive clinical management.

### Materials and methods

### Patients and cell cultures

Short-term cell cultures were established from metastatic lesions removed surgically from consecutive CM patients referred to the National Cancer Institute of Aviano (Italy) for stage III surgery from 1991 to 2007, as previously described (Altomonte et al., 1993). Autologous tumor cell cultures were successfully established from 30% of patients. The micrometastatic nature of lymph-node tumor tissues from AJCC stage IIIA patients precluded their use for cell culture generation, while short-term CM cultures were available only from 12 stage IIIB patients, and were excluded from the statistical analyses. Thus, the planned studies were conducted on a total of 42 available short-term cultures, identified as having been generated from CM patients classified as AJCC stage IIIC. Short-term CM cell cultures were grown in RPMI 1640 Medium (Biochrome KG, Berlin, Germany) supplemented with 20% heat-inactivated fetal calf serum (Biochrome KG) and 2mM L-glutamine (Biochrome KG). Four independent cultures of NHEM were purchased from Invitrogen (Milan, Italy), Gentaur (Brussels, Belgium), Provitro (Berlin, Germany), and ScienCell (Carlsbad, CA, USA), and were maintained in M254 Medium supplemented with Human Melanocyte Growth Supplement (Invitrogen). To minimize alterations potentially arising with extended *in vitro* culturing, all cell cultures were utilized for molecular assays at the 6^{th} *ex vivo* passage.

### LINE-1 bisulfite pyrosequencing analysis

Genomic DNA was extracted from short-term cultures of CM cells by proteinase K treatment followed by standard phenol/chloroform extraction and ethanol precipitation (Ausubel et al., 1998). Bisulfite conversion was carried out on 500 ng genomic DNA using EZ DNA Methylation-Gold™ Kit (Zymo Research, Orange, CA, USA), according to the manufacturer's protocol. Methylation analysis of the *LINE-1* elements was performed as previously described (Yang et al., 2004), with minor modifications. *LINE-1* elements were amplified using 50 pmol each of forward primer 5'-TTTTTTGAGTTAGGTGTGGG-3' (SEQ ID No. 221) and reverse biotinylated primer 5'-TCTCACTAAAAAATACCAAACAA-3' (SEQ ID No. 222) in a 50 µL reaction volume containing 2.5 ng of bisulfite-treated DNA, 1x PCR buffer, 1.5 mM MgCl₂ and 1.25 U of Platinum Taq DNA polymerase (Invitrogen). PCR thermal amplification profile consisted of an initial denaturation step of 5 min at 95°C, followed by 50 cycles of 30 s at 95°C, 30 s at 58°C, and 1 min at 72°C. The PCR product was purified using Streptavidin Sepharose High Performance beads (Amersham Biosciences, Uppsala, Sweden) and denatured using 0.2 mol/L of NaOH solution. Next, 0.3 µmol/L of the sequencing primer (5'-GGGTGGGAGTGAT-3') (SEQ ID No. 223) was annealed to the purified single-stranded PCR product and the Pyrosequencing reaction was performed using the PSQ HS 96 Pyrosequencing System (Pyrosequencing, Inc., Westborough, MA). The level of methylation for each of the 3 analyzed CpG sites (CpG1, CpG2, CpG3) was expressed as the percentage of methylated cytosines over the sum of methylated and unmethylated cytosines (Figure 1).

### Statistical analysis

The primary objective was to determine differences in survival among various *LINE-1* DNA methylation level groups. *LINE-1* DNA methylation levels were dichotomized using median value into the following categories: CpG1 (<25.68, ≥25.68), CpG2 (<27.26, ≥27.26), and CpG3 (<40.46, ≥40.46). The characteristics including age, gender, primary tumor localization, Breslow thickness, Clark level, and ulceration of the primary tumor, number of lymph nodes involved, and pre-operative serum LDH values were examined. Survival time was calculated in months from the date of stage IIIC diagnosis until the date of death. According with the specific goals of the analysis, the authors did not classify the deaths considering their cause. Patients were censored at the last follow-up date or the last date the patient was last known to be alive. Median survival duration was determined by the Kaplan-Meier method (Kaplan and Meier, 1958). Cumulative survival by DNA methylation level was evaluated using the log-rank test. P values were two sided and values <0.05 were considered to be statistically significant. Cox proportional hazard method (Cox, 1972) was used to examine the effect of DNA methylation level on survival and results were presented as Hazard Ratios (HR) with corresponding 95% Confidence Intervals (CI). *LINE-1* methylation was also entered in the model as a continuous variable with the unit set at 10% of methylation. Patient characteristics and clinical and pathologic features were examined by univariate Cox model. The statistical analyses were carried out using the SAS Software version 9.13 (SAS Institute Inc., Cary, North Carolina, USA).

### Results

### Patients

The study was conducted on CM patients who underwent radical lymph node dissection for stage III disease at the Centro di Riferimento Oncologico National Cancer Institute between 1991 and 2007. Patients diagnosed with a stage IIIC disease, and for whom a short-term cell culture had been successfully generated from the surgically removed autologous neoplastic tissue, were included in the study. Table 5 summarizes the 42 patients under study and their clinico-pathologic characteristics at presentation.

**Table 5. Characteristics of the 42 AJCC stage IIIC melanoma patients**

| **Variable** | **n. patients** | **%** |
|---|---|---|
| Age, years | | |
| Median | 54 | |
| Range | 29-83 | |
| | | |
| Gender | | |
| Male | 27 | 64 |
| Female | 15 | 36 |
| | | |
| Localization of primary tumor | | |
| extremities | 14 | 33 |
| trunk | 23 | 55 |
| head & neck | 3 | 7 |
| NA* | 2 | 5 |
| | | |
| Breslow thickness of primary tumor | | |
| ≤2.0 mm | 13 | 31 |
| >2.0 mm | 22 | 52 |
| NA | 7 | 17 |
| | | |
| Clark level of primary tumor | | |
| 1- 3 | 12 | 29 |
| 4-5 | 24 | 57 |
| NA | 6 | 14 |
| | | |
| Ulceration of primary tumor | | |
| No | 10 | 24 |
| Yes | 30 | 71 |
| NA | 2 | 5 |
| | | |
| N. lymph nodes involved | | |
| 1 | 9 | 21 |
| >1 | 33 | 79 |
| | | |
| LDH | | |
| Low_{†} | 28 | 67 |
| High | 11 | 26 |
| NA | 3 | 7 |

| | | |
|---|---|---|
| *NA, not available ^{†} low LDH is established as LDH values ≤ 0.8 times the upper limit of normal, high LDH is established as LDH values > 0.8 times the upper limit of normal | | |

### Extent of LINE-1 methylation in CM patients

To define if CM undergoes changes in the overall content of 5-methylcytosine, bisulfite pyrosequencing analyses were utilized to compare the extent of methylation of *LINE-*1 repetitive elements in the 42 short-term CM cell cultures under study to that of primary cultures of NHEM. *LINE-1* sequences were heavily methylated at all investigated CpG sites in NHEM (CpG1: median 62.82%, range 60.43%-67.53%; CpG2: median 52.57%, range 51.37%-52.87%; CpG3: median 65.77%, range 62.40%-67.33%; Figure 2).

On the other hand, much lower and largely heterogeneous levels of methylationof the *LINE-1* elements were observed in CM cells from stage IIIC patients (CpG1: median 25.68%, range 12.45%-54.05%; CpG2: median 27.26%, range 16.50%-49.43%; CpG3: median 40.46%, range 28.10%-64.15%; Figure 2), demonstrating that highly variable alterations in the overall level of genomic DNA occur in CM.

### Prognostic value of LINE-1 methylation in CM patients

The highly heterogeneous levels of *LINE-1* methylation observed in CM cells from stage IIIC patients led us to investigate whether they correlated with a different clinical outcome of patients under study.

Kaplan-Meier analysis indicated that median OS for stage IIIC CM patients under analysis was 15.3 months (95% CI, 11.0-31.5; Figure 3A). To evaluate the association between *LINE-1* methylation status and OS, patients were divided according to the median value of methylation of each analyzed CpG site (CpG1=25.68%; CpG2=27.26%; CpG3=40.46%). Patients were defined as having hypomethylated or hypermethylated *LINE-1* sequences, depending on the methylation level being below or above the median value for each group, respectively. Kaplan-Meier analysis showed a trend towards an increased OS rate for patients with hypomethylated CpG1, however, the difference did not reach statistical significance (P=0.22, log-rank=1.51; Figure 3B). On the other hand, a significant survival advantage was observed in patients with CpG2<27.26% as compared to patients with CpG2≥27.26% (P=0.04, log-rank=4.14) (Figure 3C). Similarly, the survival rate of patients with CpG3<40.46% was significantly higher than that of patients with CpG3≥40.46% (P=0.01, log-rank=6.39) (Figure 3D). In line with these data, median OS of patients with hypomethylated CpG1, CpG2 and CpG3 sites was 24.3, 31.5, and 31.9 months, respectively, as compared to 15.3, 11.5, and 11.5 months of patients with hypermethylated *LINE-1* CpGs (Figure 3, Table 6).

**Table 6. OS of stage IIIC CM patients according to LINE-1 methylation**

| **LINE1 CpG site** | **Extent methylation*** | **Median OS (95%CI)^{†}** | **5 year OS (%)** |
|---|---|---|---|
| **CpG1** | <25.68 | 24.3 (11.1-inf) | 39 |
| | ≥25.68 | 15.3 (6.8-26.9) | 16 |
| | | | |
| **CpG2** | <27.26 | 31.5 (12.5-inf) | 43 |
| | ≥27.26 | 11.5 (6.8-20.9) | 13 |
| | | | |
| **CpG3** | <40.46 | 31.9 (13.1-inf) | 48 |
| | ≥40.46 | 11.5 (9.2-20.6) | 7 |

| | | | |
|---|---|---|---|
| * Patients were divided according to the % of methylation of the specified CpG site being < or ≥ the median % methylation measured in the examined patients' population; ^{†} Survival functions were calculated by the Kaplan-Meier method. Data are reported as Median OS in months, together with the corresponding 95% Confidence Intervals (CI). | | | |

Accordingly, the 5 year OS was 39%, 43%, and 48% for patients with hypomethylated CpG1, CpG2, and CpG3 sites, respectively, as compared to 16%, 13%, and 7% of patients with hypermethylated *LINE-1* CpGs (Table 5).

Cox univariate analysis was carried out to identify patient characteristics and clinico-pathologic factors that predicted survival. Among all factors examined, including age, gender, localization of primary tumor, Breslow thickness, Clark level and ulceration of primary tumor, number of lymph nodes involved, and level of pre-operative LDH, only CpG2 methylation (HR=2.12 for CpG2≥27.26% vs. CpG2<27.26; 95% CI: 1.01-4.44; P=0.04) and CpG3 methylation (HR=2.63 for CpG2≥40.46% vs. CpG2<40.46; 95% CI: 1.21-5.69; P=0.01) were associated with statistically significant differences in OS (Table 7). When *LINE-1* methylation was analyzed as a continuous variable, a statistically significant inverse association emerged between OS and an increase of 10% of methylation of CpG1 (HR=1.51; 95%CI:1.06-2.16; P=0.02), CpG2 (HR=1.60; 95%CI:1.02-2.52; P=0.04) and CpG3 (HR=1.49; 95%CI:1.06-2.09; P=0.02) (Table 7).

**Table 7. Cox analysis of the influence of LINE-1 methylation on OS of stage IIIC CM patients**

| **LINE1 CpG site** | **Extent methylation*** | **HR^{†}** | **95% CI; *P* value** | **HR_{cont.}^{‡}** | **95% CI; *P* value** |
|---|---|---|---|---|---|
| **CpG1** | <25.68 | 1^{§} | | 1.51 | 1.06-2.16; 0.02 |
| | ≥25.68 | 1.57 | 0.76-3.24; 0.22 | | |
| **CpG2** | <27.26 | 1 | | 1.60 | 1.02-2.52; 0.04 |
| | ≥27.26 | 2.12 | 1.01-4.44; 0.04 | | |
| **CpG3** | <40.46 | 1 | | 1.49 | 1.06-2.09; 0.02 |
| | ≥40.46 | 2.63 | 1.21-5.69;. 0.01 | | |

| | | | | | |
|---|---|---|---|---|---|
| * Patients were divided according to the % of methylation of the specified CpG site being < or ≥ the median % methylation measured in the examined patients' population; ^{†} Cox proportional hazard method was used to examine the effect of *LINE-1* methylation on OS. Results were presented as Hazard Ratios (HR) with corresponding 95% Confidence Intervals (CI); ^{‡} *LINE-1* methylation was also evaluated as continuous variable. The HR value is that of the *LINE-1* methylation relative to an increase of 10 %; ^{§} set as reference. | | | | | |

In the present disclosure, the authors demonstrate that the global level of *LINE-1* methylation of short-term tumor cell cultures grown from patients with nodal disease is a significant predictor of OS in stage IIIC CM patients. This finding is of remarkable clinical relevance, since, to the best of our knowledge, it provides the first evidence of a molecular marker capable of differentiating the prognosis of CM patients in this high-risk substage. These results are of particular emphasis given the conduct of this study in subjects within a single clinically well-defined clinico-pathological staging sub-group, which has become the focus of several ongoing clinical trials in the US and Europe (i.e., ECOG intergroup trial E4697, EORTC trial 18071, GSK trial 111482 "DERMA").

Genomic DNA hypomethylation has been proposed to have an important impact on tumor biology through the generation of chromosomal instability, reactivation of transposable elements, and loss of imprinting (Esteller, 2008). Thus, a negative correlation between genomic hypomethylation and survival of CM patients could have been expected. Instead, the authors found that hypomethylation of *LINE-1* elements at CpG2 or CpG3 sites was associated with a significantly better OS, as demonstrated by Kaplan-Meier analysis and log-rank test. The positive prognostic value of *LINE-1* hypomethylation the authors have identified in CM is in sharp contrast with data most recently obtained in colon and ovarian cancer patients, in which *LINE-1* hypomethylation in neoplastic tissues was associated with a poorer prognosis(Ogino et al., 2008; Pattamadilok et al., 2008). Since CM behaves differently from other solid tumors, the underlying biological effect(s) of *LINE-1* hypomethylation on patients' outcome could depend on the tumor histotype. Nevertheless, it should be emphasized that our findings are generated from patients in the same clinico-pathological stage of disease, while the studies on ovarian and colon cancer were conducted on the heterogeneous patients population as a whole, and did not investigate the prognostic potential of *LINE-1* methylation in specific clinically defined stages of disease.

The mechanism(s) through which *LINE-1* hypomethylation affects survival of CM patients remains to be fully explored; however, some speculation can be made, based on recent data in the literature. Tellez *et al* (Tellez et al., 2009) have demonstrated that higher levels of *LINE-1* methylation correlate with an increased number of aberrantly hypermethylated tumor suppressor genes (TSG) in cultured melanoma cell lines. Thus, epigenetic inactivation of TSG might account for more aggressive disease the authors have observed in patients with elevated *LINE-1* methylation in their neoplastic cells. This notion is in accordance with initial studies reporting a negative association between survival and the presence of hypermethylated *ER-α, RASSF1A*, *RAR-β2, or MINT31* DNA in neoplastic tissues or sera of stage III/IV CM patients(Mori et al., 2006; Mori et al., 2005; Tanemura et al., 2009). On the other hand, hypomethylation, and consequent transcriptional activation, of *LINE-1* elements might *per se* reduce the tumorigenic potential of neoplastic cells by triggering apoptosis and a senescence-like state through the activity of the second open reading frame of *LINE*-*1*(Wallace et al., 2008). Furthermore, genomic DNA hypomethylation has been associated with the *de novo* expression of tumor associated antigens belonging to the Cancer Testis Antigen (CTA) class by neoplastic cells of different histotype (De Smet et al., 1996; Sigalotti et al., 2002; Woloszynska-Read et al., 2008), and the authors have recently identified a significant correlation between a hypomethylated status of *LINE-1* elements and increased presence, levels and total number of CTA concomitantly expressed in short-term cultures of CM cells (see example 2). Thus, it is intriguing to speculate that a better immune recognition of *LINE-1* hypomethylated CM cells might contribute to the improved survival of these patients.

Irrespective of the underlying biological mechanism(s) triggered by *LINE-1* hypomethylation, the prognostic value of *LINE-1* methylation here identified for stage IIIC CM patients bears several important practical clinical implications. Among these, the goal to provide CM patients with improved clinico-pathological sub-stage and/or follow-up-procedures would be enhanced using *LINE-1* methylation status, and these findings might be used to select and/or stratify patients for adjuvant treatment based on the methylation level of *LINE-1* in their tumors. In addition, the significant positive prognosis of *LINE-1* hypomethylated patients should prompt the incorporation of this in new studies aimed at understanding whether pharmacologic DNA hypomethylation (Sigalotti et al., 2007) could be regarded as a feasible chemoprevention approach in the initial phases of disease and/or in patients at high-risk of disease recurrence.

The authors' present findings will be further investigated in prospective multicenter studies in which the prognostic significance and the predictive value for different treatments of CM will be validated. Providing further support to our data will undoubtedly add the evaluation of *LINE-1* methylation into the routine clinico-pathological ascertainment of CM patients, helping to personalize their comprehensive clinical management

Hypomethylation (i.e., methylation below median) at CpG2 and CpG3 sites significantly associated with improved prognosis of CM, CpG3 showing the strongest association. Patients with hypomethylated CpG3 had increased OS (P=0.01, log-rank=6.39) by Kaplan-Meier analysis. Median OS of patients with hypomethylated or hypermethylated CpG3 were 31.9 and 11.5 months, respectively. The 5 year OS for patients with hypomethylated CpG3 was 48% compared to 7% for patients with hypermethylated sequences. Among the variables examined by Cox regression analysis, *LINE-1* methylation at CpG2 and CpG3 was the only predictor of OS (Hazard Ratio=2.63, for hypermethylated CpG3; 95% Confidence Interval: 1.21-5.69; *P=*0.01)*.*
*LINE-1* methylation is identified as a molecular marker of prognosis for CM patients in stage IIIC. Evaluation of *LINE-1* promises to represent a key tool for driving the most appropriate clinical management of stage III CM patients.

### Example 2 - Methylation levels of the "long interspersed nucleotide element-1" repetitive sequences correlate with presence and levels of expression of cancer testis antigens in cutaneous melanoma

Cancer Testis Antigens (CTA) represent an important class of immunogenic tumor antigens that are currently utilized as targets in several cancer immunotherapy approaches. The expression of CTA has been shown to depend on the methylation status of their promoters. In view of the therapeutic relevance of CTA expression and of its regulation by promoter methylation, the authors asked the question whether *LINE-1* methylation might correlate with CTA expression in CM cells, thus representing a surrogate marker for their presence and levels of expression. To this end, *LINE-1* methylation was measured in CM cells by pyrosequencing assays and CTA expression was evaluated by qualitative or quantitative RT-PCR analyses. The correlation between *LINE-1* methylation and CTA expression was evaluated through Spearman's rank correlation test.

### Materials and methods

### LINE-1 methylation

The extent of methylation of *LINE-1* elements was measured in primary cultures of CM cells at the 6^{th} *in vitro* passage by bisulfite pyrosequencing as reported in example 1.

### Qualitative and Quantitative RT-PCR analyses

Total RNA was extracted from CM cells at the 6^{th} *in vitro* passage using the TRIzol reagent (Invitrogen, Milan, Italy) following manufacturer's recommendations. RT-PCR reactions were performed as previously described (Coral et al., 1999). The oligonucleotide primer sequences and gene-specific PCR amplification programs utilized have been defined for MAGE-A1, -A2, -A3, -A4, -A10(De Plaen et al., 1994; Rimoldi et al., 1999), NY-ESO-1 (Jager et al., 1998), SSX-2(Gure et al., 1997). The integrity of each RNA and oligo-dT-synthesized cDNA sample was confirmed by the amplification of the β-actin housekeeping gene (Coral et al., 1999). Ten µl of each RT-PCR sample were run on a 2% agarose gel and visualized by ethidium bromide staining.

Real-time quantitative RT-PCR analyses were performed as previously described(Calabro et al., 2005). Briefly, total RNA was digested with RNase free DNase (Roche Diagnostics, Milan, Italy) to remove contaminating genomic DNA. Synthesis of cDNA was performed on 1 µg total RNA using MMLV reverse transcriptase (Invitrogen) and random hexamer primers (Promega, Milan, Italy), following manufacturers' instructions. TaqMan quantitative RT-PCR reactions were performed on 10 ng of retrotranscribed total RNA in a final volume of 25 µl TaqMan Universal Master Mix (Applyed Biosystems, Milan, Italy) at95°C for 10 min, followed by 45 cycles at 95°C for 15 sec and at 60°C for 1 min. TaqMan primers/probe sets were as follows: β-actin forward CGAGCGCGGCTACAGCTT (SEQ ID No. 224), β-actin reverse CCTTAATGTCACGCACGATT (SEQ ID No. 225), β-action probe FAM-ACCACCACGGCCGAGCGG-BHQ1 (SEQ ID No. 226); MAGE-A3 forward TGTCGTCGGAAATTGGCAGTAT (SEQ ID No. 227), MAGE-A3 reverse CAAAGACCAGCTGCAAGGAACT(SEQ ID No. 228), MAGE-A3 probe FAM-TCTTTCCTGTGATCTTC-MGB (SEQ ID No. 229). Measurement of gene expression was performed utilizing the ABI PRISM 7000 Sequence Detection System (Applyed Biosystems) and the copy number of MAGE-A3 and of the reference gene β-actin was established in each sample by extrapolation of the standard curve. The number of MAGE-A3 cDNA molecules in each sample was then normalized to the number of cDNA molecules of β-actin.

### Statistical analyses

The correlation between *LINE-1* methylation at each CpG site and the number of expressed CTA or the quantitative levels of expression of MAGE-A3 was evaluated through Spearman's rank correlation test. P values < 0.05 were considered to be statistically significant.

### Results

Results demonstrate a significant inverse association of *LINE-1* methylation with both number of CTA concomitantly expressed (Figure 4) and level of expression of the CTA MAGE-A3 (Figure 5).

### Example 3 -whole-genome methylation profiles, and derived methylation signatures, predict survival of melanoma patients Introduction

Cutaneous melanoma (CM) is a very aggressive neoplasm of growing incidence and mortality rates in industrialized countries, and the leading cause of skin cancer-related deaths worldwide(MacKie et al., 2009). Surgery, in early phases of disease has curative potential for patients; for advanced CM conventional therapies have failed to prolong survival (Bhatia et al., 2009). At present, the best predictor of 5-year survival is the clinico-pathological stage of disease, which defines overall survival (OS) rates ranging from 95% to 7% for stage I to IV patients, respectively(Balch et al., 2001; Balch et al., 2009). However, within the same clinico-pathological stage category, patients often behave radically differently, and the current lack of prognostic molecular markers impairs our ability to identify CM patients with highly aggressive as opposed to more indolentcourses of disease(Jennings and Murphy, 2009).

Methylation of genomic DNA in mammals occurs at 5C-position of the cytosine in the context of CpG dinucleotides, and results in gene silencing through different mechanisms (Sigalotti et al., 2010). Recent studies have demonstrated that alterations in genomic DNA methylation patterns represent a hallmark of cancer cells, being proposed to actively contribute to cancer development and progression mainly through inactivation of tumor suppressor genes by aberrant promoter hypermethylation (Esteller, 2008). In particular, epigenetic alterations are emerging as important players in CM tumorigenic process, as demonstrated by the continuously growing list of genes that are transcriptionally inactivated by aberrant DNA hypermethylation in CM, and which potentially affect key cellular pathways such as cellular proliferation/differentiation, apoptosis, DNA repair, extracellular matrix interaction, signaling, metastatization and immune recognition (Sigalotti et al., 2010). The increasing role of aberrant methylation in CM biology strongly suggests for the opportunity to test methylation markers as potential indicators of disease prognosis. Along this line, preliminary investigations are suggesting a possible prognostic role of the methylation status of selected genes in CM patients. Indeed, Lahtz *et al* have most recently evaluated a total of 230 CM patients at stage 0 to IV of disease, demonstrating *PTEN* methylation to be an independent negative prognostic factor, though it did not outperform the strongest traditional markers of tumor thickness and ulceration (Lahtz et al., 2010). Similarly, methylation of the tumor suppressor gene *TSLC1* was found to be significantly increased in advancing tumor stage and to associate with a reduced disease-related survival of CM patients (You et al., 2010), and the methylation of the methylated in tumors (MINT) locus MINT31 has been recently shown to be significantly associated with advancing clinical stage in 107 stage I to IV CM patients, and to predict improved disease-free and overall survival in the 25 analyzed stage III patients (Tanemura et al., 2009).

In line with the above reported information, initial studies have also investigated the methylation status of selected tumor suppressor genes in sera of CM patients. Among the genes more frequently methylated in CM, a negative correlation between presence of circulating methylated DNA for RASSF1A and ER-α and survival of CM patients receiving biochemotherapy was found, suggesting for the potential usefulness of soluble methylation markers as prognostic factors in this malignancy(Mori et al., 2006; Mori et al., 2005).

Despite these promising initial results, the studies so far available have several limitations, including: i) investigation of only single/few genes; ii) reduced number of patients evaluated; iii) rarely investigated the prognostic significance of methylation markers in patients at the same stage of disease.

Furthermore, to the best of our knowledge, no studies have investigated the influence of the genome-wide gene methylation profiles on CM prognosis. Accordingly, the authors investigated whether whole-genome methylation profiles may account for the differing survival patterns of CM patients of identical clinico-pathological stage of disease. The study was conducted on a series of 59 consecutive stage III CM patients for whom the autologous short-term cell cultures were available. The latter were analyzed early during *in vitro* passage, and utilized instead of tumor tissues to overcome possible alterations in the evaluation of genomic methylation profiles due to the unavoidable presence of contaminating normal cells.

Results demonstrated that whole-genome methylation profiling is a powerful tool to identify CM patients with a significantly different prognosis, and that methylation signatures of 17 to 98 genes can be utilized to assign CM patients to distinct prognostic groups. These findings demonstrate that evaluation of whole-genome-defined methylation signatures may greatly help in guiding the daily clinical management of CM patients, and provide a strong rationale for the development of a large prospective validation study.

### Materials and methods

### Patients and cell cultures

Short-term cell cultures were established from metastatic lesions removed surgically from consecutive CM patients referred to the National Cancer Institute of Aviano (Italy) for stage III surgery from 1991 to 2007, as previously described (Altomonte et al., 1993). Autologous tumor cell cultures were successfully established from 30% of patients. The micrometastatic nature of lymph-node tumor tissues from AJCC stage IIIA patients precluded their use for cell culture generation. Thus, the planned studies were conducted on a total of 59 available short-term cultures, identified as having been generated from CM patients classified as AJCC stage IIIB or IIIC. Short-term CM cell cultures were grown in RPMI 1640 Medium (Biochrome KG, Berlin, Germany) supplemented with 20% heat-inactivated fetal calf serum (Biochrome KG) and 2mM L-glutamine (Biochrome KG). To minimize alterations potentially arising with extended in vitro culturing, all cell cultures were utilized for molecular assays at the 6^{th} ex vivo passage.

### Whole genome methylation profiling

Genomic DNA was extracted from short-term cultures of CM cells by proteinase K treatment followed by standard phenol/chloroform extraction and ethanol precipitation (Fratta et al., 2010).The 59 samples under analysis were evaluated for genome-wide promoter methylation using the Illumina Infinium HumanMethylation27 Bead array (Illumina Inc, San Diego, CA), which allows the interrogation of 27,578 CpG dinucleotides, covering 14,495 genes. The assay is based on the conversion of unmethylated C-nucleotides into U(T) nucleotides by the bisulfite treatment, leaving unaltered methylated ones. Accordingly, the EZ DNA methylation kit (Zymo Research, Orange,CA, USA) was used for bisulfite conversion of 500 ng of genomic DNA, and the remaining assay steps were performed as per Illumina protocol. Briefly, the bisulfite-modified genomic DNA was whole-genome amplified, enzymatically fragmented, precipitated, resuspended, and hybridized overnight to locus-specific oligonucleotide primers on the BeadArray. Each interrogated locus is represented by specific oligomers linked to two bead types: one representing the sequence for the methylated DNA (M) and the other for unmethylated DNA (U). After hybridization, the C or T nucleotides were detected on M- or U-beads, respectively, by single-base primer extension. The arrays were then imaged using a BeadArray™ Reader. Image processing and intensity data extraction were performed according to Illumina's instructions. The methylation status of a specifc CpG site is calculated from the intensity of the M- and U-beads, as the ratio of fluorescent signals: β=Max(M,0)/[Max(M,0)+Max(U,0)]. DNA methylation β values are continuous variables between 0 (no methylation) and 1 (completely methylated) representing the ratio of combined locus intensity.

### Gene expression profiling

Total cellular RNA was purified from short-term cultures of CM cells by TRIzol reagent (Invitrogen, Milan, Italy) following manufacturer's recommendations. Gene expression profiling was conducted on Illumina human WG-6 version 3 arrays using standard Illumina protocols.

### Statistical analysis

All statistical analyses were performed using the R, version 2.11.1, statistical environment (http://www.R-project.org) and bioconductor2.6 packages (http://www.bioconductor.org). The authors used the methylumi package for importing and pre-processing the methylation data, the package lumi (Du et al., 2008) for importing and pre-processing the gene expression data, the survival package for Kaplan-Meier estimates and the pamr package for the shrunken centroid supervised analysis. In brief, the methylation data exported from the Illumina Beadstudio software were imported in R, checked for quality and normalized using the methylumi bioconductor package. K-means partitioning clustering was used to divide the transformed data into respectively 2 and 3 groups. The survival times of each of the 2/3 groups were evaluated using a Kaplan-Meier analysis. The characteristics including age, gender, primary tumor localization, Breslow thickness, Clark level, and ulceration of the primary tumor, number of lymph nodes involved, and pre-operative serum LDH values were also examined. Survival time was calculated in months from the date of stage III or IIIC diagnosis until the date of death. According with the specific goals of the analysis, the authors did not classify the deaths considering their cause. Patients were censored at the last follow-up date or the last date the patient was last known to be alive. Median survival duration was determined by the Kaplan-Meier method. Cumulative survival was evaluated using the log-rank test. P values were two sided and values <0.05 were considered to be statistically significant. Methylation patterns (signatures) related to the discovered groups were determined using the nearest shrunken centroid classification algorithm (PAM). As the algorithm requires a threshold to balance between the number of sample correctly classified and the subset of features representing the methylation patterns, the authors determined this threshold by cross-validation as recommended by the inventors of the algorithm (Tibshirani et al., 2002).

### Results

### Patients

The study was conducted on CM patients who underwent radical lymph node dissection for stage III disease at the Centro di Riferimento Oncologico National Cancer Institute between 1991 and 2007. Patients diagnosed with a stage III disease, and for whom a short-term cell culture had been successfully generated from the surgically removed autologous neoplastic tissue, were included in the study. Table 8 summarizes the 59 patients under study and their clinico-pathologic characteristics at presentation.

**Table 8. Characteristics of the 59 AJCC stage III melanoma patients**

| **Variable** | **n. patients** | **%** |
|---|---|---|
| Sub-stage | | |
| IIIB | 13 | 22 |
| IIIC | 45 | 76 |
| NA* | 1 | 2 |
| | | |
| Age, years | | |
| Median | 52 | |
| Range | 27-84 | |
| Gender | | |
| Male | 35 | 59 |
| Female | 24 | 41 |
| | | |
| Localization of primary tumor | | |
| extremities | 21 | 36 |
| trunk | 31 | 52 |
| head & neck | 4 | 7 |
| NA | 3 | 5 |
| | | |
| Breslow thickness of primary tumor | | |
| ≤2.0 mm | 20 | 34 |
| >2.0 mm | 29 | 49 |
| NA | 10 | 17 |
| | | |
| Clark level of primary tumor | | |
| 1-3 | 19 | 32 |
| 4-5 | 31 | 53 |
| NA | 9 | 15 |
| | | |
| Ulceration of primary tumor | | |
| No | 20 | 34 |
| Yes | 36 | 61 |
| NA | 3 | 5 |
| | | |
| N. lymph nodes involved | | |
| 1 | 19 | 32 |
| >1 | 39 | 66 |
| NA | 1 | 2 |
| | | |
| LDH | | |
| Low_{†} | 37 | 63 |
| High | 19 | 32 |
| NA | 3 | 5 |

| | | |
|---|---|---|
| *NA, not available ^{†} low LDH is established as LDH values ≤ 0.8 times the upper limit of normal, high LDH is established as LDH values > 0.8 times the upper limit of normal | | |

### Unsupervised analysis of whole-genome methylation profiles identifies CM patients subgroups with different prognosis

In light of the likely influence of altered genome-wide methylation profiles on CM biology, the authors sought to investigate whether distinct methylation patterns might associate with a different clinical outcome of CM patients.

Genome-wide gene methylation profiles were evaluated in the 59 short-term CM cell cultures under study using the Illumina HumanMethylation27 Bead-Chip whole-genome assay, which interrogates the methylation status of 27,578 CpG sites, corresponding to 14,495 genes. Patients were then grouped according to their whole genome methylation profile by applying a k-means clustering, which is a non hierarchical unsupervised method that allows partitioning of data into a predetermined number (k) of groups (Figure 6). K-means clustering algorithm was applied by requiring a separation of all stage III CM patients under analysis into 2 or 3 subgroups (Figure 6). Accordingly, the classification split the population in either 2 subgroups (group K2-1 and K2-2), consisting of 35 and 24 patients, respectively, or in 3 subgroups (group K3-1, K3-2, K3-3), counting 27, 24 and 8 patients, respectively (figure 6).

The prognostic relevance of the genome-wide methylation profile-based classification was assessed by evaluating the obtained subgroups of patients for overall survival (OS) through Kaplan-Meier analysis. Results demonstrated a significant survival advantage for patients classified as K2-1 as compared to those belonging to group K2-2 (P=0.022, log-rank=5.2) (Figure 6A). The statistically significant difference in OS among groups was maintained also when splitting the population in 3 classes (P=0.047, log-rank=6.1) (Figure 6B). In line with these data, an increased median OS was observed for patients classified as K2-1 (31.5 months) as compared to patients in the K2-2 group (14.8 months) (Table 9). Similarly, patients in the K3-1 class had a superior median OS (31.9 months) as compared to K3-3 (18.8 months) and K3-2 (14.8 months) (Table 9). Accordingly, the 5 year OS of patients classified as K2-1 and K2-2 was 41.8% and 5.6%, respectively, while that of patients classified as K3-1, K3-3 and K3-2 was 43.7%, 37.5%, and 5.6%, respectively (Table 9).

**Table 9. OS of stage III CM patients according to genome-widemethylation profiles**

| **stage** | **group*** | **Median OS (95%CI)^{†}** | **5 year OS (%)** |
|---|---|---|---|
| **III all** | K2-1 | 31.5 (13.5-NA‡) | 41.8 |
| | K2-2 | 14.8 (10.4-35.4) | 5.6 |
| | | | |
| **III all** | K3-1 | 31.9(15.3-NA) | 43.7 |
| | K3-2 | 14.8(10.4-35.4) | 5.6 |
| | K3-3 | 18.8(11.5-NA) | 37.5 |
| | | | |
| **IIIc** | 3C-K2-1 | 31.5(13.12-NA) | 41.2 |
| | 3C-K2-2 | 10.4(5.29-NA) | 0 |
| | | | |
| **IIIc** | 3C-K3-1 | 11.0(7.3-NA) | 0 |
| | 3C-K3-2 | 31.5(12.5-NA) | 39.9 |
| | 3C-K3-3 | 24.5(11.5-NA) | 42.9 |

| | | | |
|---|---|---|---|
| * Patients were divided by the k-means clustering algorithm in either 2 or 3 groups, according to their genome-wide methylation profile; ^{†} Survival functions were calculated by the Kaplan-Meier method. Data are reported as Median OS in months, together with the corresponding 95% Confidence Intervals (CI). ‡ NA, not available. | | | |

In light of the above reported prognostic significance of genome-wide methylation profiles in stage III CM patients, the authors asked the question whether methylation profiling could allow to identify different survival classes also in sub-stage IIIC patients, which are characterized by highly homogeneous clinico-pathological characteristics. Thus, the same analytical procedures employed for the whole population of stage III patients were applied to the subgroup of 45 stage IIIC patients. K-means classification split the population in either 2 subgroups (group 3C-K2-1 and 3C-K2-2), counting of 33 and 12 patients, respectively, or in 3 subgroups (group 3C-K3-1, 3C-K3-2, 3C-K3-3), including 11, 27 and 7 patients, respectively (figure 6). Kaplan-Meier analysis demonstrated a significant survival advantage for patients classified as 3C-K2-1 as compared to group 3C-K2-2 (P=0.001, log-rank=10.2) (Figure 6C). Similarly, a significant difference in OS was also observed among groups 3C-K3-3, 3C-K3-2, and 3C-K3-1 (P=0.021, log-rank=7.8) (Figure 6D). An increased median OS was observed for patients classified as 3C-K2-1 (31.5 months) as compared to patients in the 3C-K2-2 group (10.4 months) (Table 9). Similarly, patients in the 3C-K3-2 class had a superior median OS (31.5 months) as compared to 3C-K3-3 (24.5 months) and 3C-K3-1 (11 months) (Table 9). Accordingly, the 5 year OS of patients classified as 3C-K2-1 and 3C-K2-2 was 41.2% and 0%, respectively, while that of patients classified as 3C-K3-3, 3C-K3-2 and 3C-K3-1 was 42.9%, 39.9%, and 0%, respectively (Table 9).

Laying on the above reported information, Cox univariate analysis was carried out to identify patient characteristics and clinico-pathologic factors that predicted survival. Among all examined factors, including age, gender, localization of primary tumor, Breslow thickness, Clark level and ulceration of primary tumor, number of lymph nodes involved, and level of pre-operative LDH, only the classes defined by k-means algorithm on the genome-wide methylation profiles were associated with statistically significant differences in OS, both when evaluating the entire population of stage III CM patients (HR=1.99 for group K2-2 vs. K2-1; 95% CI: 1.09-3.64; P=0.025), and the sole substage IIIC patients (HR=3.2 for group 3C-K2-2 vs. 3C-K2-1; 95% CI: 1.51-6.79; 0.0023) (Table 10).

**Table 10. Cox analysis of the influence of genome-wide methylation profile on OS of stage III CM patients**

| **stage** | **group*** | **HR^{†}** | **95% CI; *P* value** |
|---|---|---|---|
| **III** | K2-1 | 1^{§} | |
| | K2-2 | 1.99 | 1.09-3.64; 0.025 |
| | | | |
| **IIIc** | 3C-K2-1 | 1 | |
| | 3C-K2-2 | 3.20 | 1.51-6.79; 0.0023 |

| | | | |
|---|---|---|---|
| * Patients were divided by the k-means clustering algorithm in 2 groups, according to their genome-wide methylation profile; ^{†} Cox proportional hazard method was used to examine the effect of methylation-based k-means clustering on OS. Results were presented as Hazard Ratios (HR) with corresponding 95% Confidence Intervals (CI); ^{§} set as reference. | | | |

### Supervised analysis of whole-genome methylation profiles by shrunken centroids identifies the methylation signature of CM patients subgroups with different prognosis

To define the methylation signature, that is the minimal number of methylation markers succinctly characterizing each of the CM patient prognostic group defined by the unsupervised clustering analysis, the authors have applied the "nearest shrunken centroid" algorithm (Tibshirani et al., 2002) (Figure 7, 8).

Using this approach the authors have identified different methylation signatures able to correctly classify patients into the previously defined prognostic classes, with acceptable error rates (Figure 7, 8). Indeed, methylation signature A, consisting of 24 genes, had an overall error rate of 0.05 and classified stage III patients into classes K2-1 and K2-2 with a total of 3 errors, while methylation signature B, including 98 genes, had an overall error rate of 0.034 and classified stage III patients into classes K3-1, K3-2, and K3-3 with a total of 2 errors (Table 3, Figure 7). The same approach conducted on sub-stage IIIC patients led to the identification of a methylation signature of 16 genes (signature C) able to sort stage IIIC patients into groups 3C-K2-1 and 3C-K2-2 without any misinterpretation, and a methylation signature of 47 genes (signature D) able to sort stage IIIC patients into groups 3C-K3-1, 3C-K3-2, and 3C-K3-3 with a total of 1 error (overall error rate of 0.022) (Table 3, Figure 8).

The initial analysis was further refined in the attempt to identify methylation signatures that were likely to be directly associated to a biologic effect through the transcriptional regulation of the respective genes. To this end, a Spearman's correlation analysis was conducted comparing genome-wide methylation data and gene expression profiles obtained from neoplastic cells of the stage III CM patients under study. This analysis yielded a set of 820 methylation probes which methylation status was significantly correlated (p<0.05) with the expression of the respective gene. By restricting the application of the "nearest shrunken centroid" algorithm to this sub-set of methylation signals, the authors were able to identify methylation signatures capable to correctly classify stage IIIC patients into the previously defined prognostic classes (Table 3, Figure 9). Of these, a 51 gene methylation signature (signature E) was able to assign stage IIIC patients to groups 3C-K2-1 or 3C-K2-2, committing only one error (overall error rate 0.022), while a 73 gene methylation signature (signature F) was able to sort stage IIIC patients into groups 3C-K3-1, 3C-K3-2, and 3C-K3-3 without any misinterpretation (Table 3, Figure 9).When, among the genes having a significant (p<0.05) association between expression and methylation status, only those with a coefficient of correlation Rho<-0.4 were taken into account, an additional signature of 35 genes (signature G) was defined, which was able to assign stage IIIC patients to groups 3C-K2-1 or 3C-K2-2, committing only one error (overall error rate 0.022) (Table 3, Figure 10).

Whole genome methylation profiles identified classes with significantly different OS in stage III CM patients. As an example, stage IIIC patients with a "favorable" methylation profile had increased OS (P=0.001, log-rank=10.2) by Kaplan-Meier analysis. Median OS of stage IIIC patients with a "favorable" vs "unfavorable" methylation profile were 31.5 and 10.4 months, respectively. The 5 year OS for stage IIIC patients with a "favorable" methylation profile was 41.2% as compared to 0% for patients with an "unfavorable" methylation profile. Among the variables examined by Cox regression analysis, classification defined by methylation profile was the only predictor of OS (Hazard Ratio=3.2, for "unfavorable" methylation profile; 95% Confidence Interval: 1.51-6.79; P=0.002). Different methylation signatures, consisting of 17 to 98 genes, able to correctly assign (overall error rate ≤0.05) stage III or IIIC patients to distinct methylation-defined prognostic groups, were identified.
Discrete whole-genome methylation signatures have been identified as molecular markers of prognosis for CM patients in stage III. Their use in the daily practice will drive the most appropriate clinical management of stage III CM patients.

### Example 4 - Prognostic significance of the methylation status of single genes taken from whole-genome defined prognostic methylation signatures.

In light of the prognostic relevance of whole-genome methylation profiles that the authors have identified (see example 3 above), the authors have addressed the question whether specific genes taken from the prognostic methylation signatures may retain a prognostic information. To this end, the authors selected genes *ALOX12B, CTAG2, FGF4, IGLL1, OCIAD2, PAGE5, S100A9, SLC6A11, SLC6A18,* and *TUB* from signature E and G as playing a major contribution in the classification tasks. The 45 stage IIIC patients under study were scored accordingly to the methylation of each gene in their tumor cells being <33%, between 33% and 66%, and >66%, or < and ≥ 50%. The impact on patients' survival of *ALOX12B, CTAG2, FGF4, IGLL1, OCIAD2, PAGE5, S100A9, SLC6A11, SLC6A18,* and *TUB* methylation levels was evaluated by Kaplan-Meier analysis and Log-rank test, showing a significantly improved survival for patients with unmethylated vs methylated genes (Table 11, Figure 11). Similar results were found for other tested genes, including: *BATF, BGN, CRHR1, CSAG1, CTSK, DENND2D, FLJ33860, GJB5, GRM4, LY96, MAGEA1, MAGEA10, MGC35206, MLSTD1, SLC18A2, SORBS2,* and *TRIM40* (data not shown).

**Table 11. OS of stage IIIC CM patients according to methylation status of selected signature genes**

| **gene** | **extent methylation*** | **Median OS (95%CI)^{†}** | **5 year OS (%)** |
|---|---|---|---|
| | <33% | 35.2(24.5-NA‡) | 49 |
| *FGF4* | ≥33%, <66% | 11.5 (6.8-NA) | 0 |
| | ≥66% | 9.8 (5.3 -NA) | 8 |
| | | | |
| | <33% | NA(31.9-NA) | 58 |
| *CTAG2* | ≥33%, <66% | 15.3 (4.5-NA) | 15 |
| | ≥66% | 11.3 (7.3-NA) | 0 |
| | | | |
| | <33% | 27.0(11.5-NA) | 44 |
| *OCIAD2* | ≥33%, <66% | 18.1 (11.1-NA) | 28 |
| | ≥66% | 9.4 (5.0 -NA) | 0 |
| | | | |
| | <33% | 73.9(24.5-NA) | 58 |
| *PAGE5* | ≥33%, <66% | NA (15.3-NA) | 53 |
| | ≥66% | 11.1 (7.3 -24.5) | 12 |
| | | | |
| | <33% | 35.2 (12.5-NA) | 48 |
| *S100A9* | ≥33%, <66% | 20.9 (11.5-NA) | 24 |
| | ≥66% | 11.1 (7.3--NA) | 0 |
| | | | |
| | <33% | 31.5 (12.4-NA) | 41 |
| *SLC6A18* | ≥33%, <66% | 9.8 (5.3-NA) | 0 |
| | ≥66% | 11.5 (11-NA) | 0 |
| | | | |
| *ALOX12B* | <33% | 24.5 (13.1-NA) | 39 |
| | ≥50% | 8.5 (5.3-NA) | 0 |
| | | | |
| *IGLL1* | <33% | 28 (13.1-NA) | 40 |
| | ≥50% | 11 (5.3-NA) | 0 |
| | | | |
| *SLC6A11* | <33% | 31.5 (15.3-NA) | 40 |
| | ≥50% | 9.8 (7.3-NA) | 0 |
| | | | |
| *TUB* | <33% | 31.5 (13.1-NA) | 41 |
| | ≥50% | 10.4 (5.3-NA) | 0 |

| | | | |
|---|---|---|---|
| * Patients were divided according to the % of methylation of genes selected from prognostic methylation signatures in their tumor cells being <33%, between 33% and 66%, and >66%, or < and ≥ 50%. ^{†} Survival functions were calculated by the Kaplan-Meier method. Data are reported as Median OS in months, together with the corresponding 95% Confidence Intervals (CI). ‡ NA, not available. | | | |

### Example 5 -prognostic utility of methylation scores defined by whole-genome methylation profiles in melanoma

In light of the prognostic relevance of whole-genome methylation profiles that the authors have identified (see example 3 above), the authors have addressed the question whether simple scores summarizing the whole genome methylation profiles could retain a prognostic information. To this end, the genome-wide methylation profile of tumor cells from each patient (defined by Illumina Infinium HumanMethylation27 Bead array, see example 3 above) was summarized with a "methylation score" as follows: methylation for each gene among the patients was standardized by the Z score method, calculated by (X-µ)/σ where X stands for methylation data of each gene in each sample, µstands for mean of methylation of each gene among all samples, and σstands for the standard deviation. Each patient was then assigned a "methylation score" consisting of the average of Z scores for all genes.The 45 stage IIIC patients under study were divided accordingly to the "methylation score"of their tumor cells being below or above the median value of the patients' population. The impact on patients' survival of the whole genome methylation profiling-derived "methylation score" was evaluated by Kaplan-Meier analysis, Log-rank test and Cox regression analysis, showing a significantly improved survival for patients with low vs high "methylation score" (Table 12, Figure 13).

**Table 12. OS of stage IIIC CM patients according to whole genome methylation profiling-derived "methylation score"**

| **methylation score*** | **Median OS (95%CI)^{†}** | **5 year OS (%)** | **HR^{‡}** | **95% CI; P value** |
|---|---|---|---|---|
| <median | 31.5(13.1-NA^{§}) | 44 | 1** | |
| ≥median | 12.5 (9.8-33.5) | 17 | 2.15 | 1.05-4.44; 0.04 |

| | | | | |
|---|---|---|---|---|
| * Patients were divided according to the "methylation score" being < or ≥ the median "methylation score" measured in the examined patients' population; ^{†} Survival functions were calculated by the Kaplan-Meier method. Data are reported as Median OS in months, together with the corresponding 95% Confidence Intervals (CI); ^{‡}Cox proportional hazard method was used to examine the effect of *LINE-1* methylation on OS. Results were presented as Hazard Ratios (HR) with corresponding 95% Confidence Intervals (CI); ^{§}NA, not available; ** set as reference. | | | | |

### Example 6 - Prognostic significance of combined evaluation of LINE-1 methylation levels and multigene methylation signatures in stage III cutaneous melanoma patients.

In light of the prognostic relevance of *LINE-1* methylation status and of whole-genome methylation profiles that the authors have identified (see examples 1, 3 above), the authors have addressed the question whether combining these two markers would result in an additional prognostic information. To this end, 59 stage III melanoma patients were scored according to their whole genome methylation profile as K2-1 or k2-2 (stage III patients) or S3-K2-1 or S3-K2-2 (substage IIIC patients), and as having hypomethylated or hypermethylated *LINE-1* sequences, as detailed in examples 1 and 3. The impact on patients' survival of the combined evaluation of the 2 markers was evaluated by Kaplan-Meier analysis and Log-rank test (Figure 12, Table 13). Results demonstrated that the combined use of the 2 markers allowed to further discriminate survival groups within the population under study (P<0.05).

**Table 13. OS of stage III CM patients according to genome-wide methylation profiles and LINE-1 methylation status**

| **stage** | **group*** | **Median OS (95%CI)^{†}** | **5 year OS (%)** |
|---|---|---|---|
| **III all** | K2-1, *LINE-1* hypomethylated | 73.9 (31.5-NA‡) | 52.4 |
| | K2-1, *LINE-1* hypermethylated | 12.5 (9.4-NA) | 18.2 |
| | K2-2, *LINE-1* hypomethylated | 10.4 (9.8-NA) | 0 |
| | K2-2, *LINE-1* hypermethylated | 18.1 (11.01-45.6) | 7 |
| **IIIC** | 3C-K2-1, *LINE-1* hypomethylated | 73.8 (31.5-NA) | 52.5 |
| | 3C-K2-2, *LINE-1* hypermethylated | 12.5 (9.3-NA) | 23.1 |
| | 3C-K2-2, *LINE-1* hypomethylated | 6.8 (3.7-NA) | 0 |
| | 3C-K2-2, *LINE-1* hypermethylated | 11.3 (5.3-NA) | 0 |

| | | | |
|---|---|---|---|
| * Patients were scored by k-means clustering algorithm as K2-1 or K2-2 (stage III patients) or as S3-K2-1 or S3-K2-2 (sub-stage IIIC patients), according to their genome-wide methylation profile, and as having hypomethylated (< median value) or hypermethylated (≥ median value) *LINE-1* sequences. Combined evaluation of this 2 markers resulted in the definition of 4 classes. ^{†} Survival functions were calculated by the Kaplan-Meier method. Data are reported as Median OS in months, together with the corresponding 95% Confidence Intervals (CI). ‡ NA, not available | | | |

### Example 7 - Analysis of LINE-1 methylation in neoplastic material from different sources

DNA was extracted from neoplastic cells and evaluated for LINE-1 methylation at 3 CpG sites (CpG1, 2, 3) by pyrosequencing or for mean LINE-1 methylation by quantitative Methylation-Specific PCR (qMSP).

Neoplastic cells were purified using anti-HMW-MAA antibody-coated magnetic beads from cryopreserved cell suspensions obtained by mechanical and enzymatic dissection of neoplastic lesions surgically removed from CM patients.

Surgically-removed neoplastic tissues from CM patients were flesh-frozen in liquid nitrogen and maintained at -80°C until use.

Surgically-removed neoplastic tissues from CM patients were formalin-fixed and paraffin-embedded. Ten micrometer sections (total 2 to 6) were cut from paraffin embedded lesions and used for evaluation of LINE-1 methylation.

Neoplastic tissue of different extensions (area 9-100 mm2) was scraped from 15 micrometer sections of formalin-fixed paraffin-embedded CM tissues, and used for evaluation of LINE-1 methylation.

In a further technical validation, neoplastic cells were purified from hematoxylin and eosin stained formalin-fixed paraffin-embedded CM tissues by Laser Capture Microdissection (LCM) using an Arcturus System (Applied Biosystems). Genomic DNA was extracted from LCM tumor cells using the PicoPure DNA kit (Applied Biosystems) and subjected to bisulfite modification by the EZ DNA Methylation-Gold Kit (Zymo Research). The extent of methylation of LINE-1 elements was measured by a qMSP assay using primers: L1M fwd CGCGAGTCGAAGTAGGGC (SEQ ID No. 230) and L1M rev ACCCGATTTTCCAAATACGACCG (SEQ ID No. 231), specific for the methylated sequences; and L1U fwd TGTGTGTGAGTTGAAGTAGGGT (SEQ ID No. 232) and L1U rev ACCCAATTTTCCAAATACAACCATCA (SEQ ID No. 233), specific for the umethylated sequences. SYBR green qMSP reactions were performed with methylated- or unmethylated-specific primer pairs on 2 µl of bisulfite-modified genomic DNA in a final volume of 25 µl 1X Power SYBR green mastermix (Applyed Biosystems) at 95°C for 10 min, followed by 45 cycles of 15 sec at 95°C and 1 min at 60°C. Real-time measurement of fluorescence intensity was performed utilizing the ABI PRISM 7000 Sequence Detection System (Applyed Biosystems), and the copy number of methylated or unmethylated sequences for each target gene was established in each sample by extrapolation from the standard curves generated with plasmids carrying fully methylated or unmethylated LINE-1 sequences. The % of methylation was defined as the ratio between methylated molecules and the sum of methylated and unmethylated molecules.
The results are shown in Tables 14 and 15.

**Table 14. Pyrosequencing analysis of LINE-1 methylation in neoplastic material from different sources***

| **neoplastic cells immunosorted from cryopreserved cell suspensions^{†}** | | | | |
|---|---|---|---|---|
| **patient** | | **CpG1** | **CpG2** | **CpG3** |
| 403 | | 28.4 | 23.2 | 34.0 |
| 390 | | 44.3 | 41.1 | 60.1 |
| 554 | | 21.9 | 26.2 | 42.0 |
| 578 | | 32.2 | 39.7 | 59.5 |
| 475 | | 40.3 | 33.9 | 47.3 |
| 514 | | 38.6 | 28.7 | 43.9 |
| | | | | |

| **cryopreserved CM neoplastic tissues‡** | | | | |
|---|---|---|---|---|
| **patient** | | **CpG1** | **CpG2** | **CpG3** |
| 588 | | 68.7 | 51.4 | 67.2 |
| 603 | | 29.0 | 25.7 | 39.3 |
| 629 | | 48.0 | 37.9 | 58.0 |
| 790 | | 50.0 | 32.9 | 47.3 |
| 816 | | 42.3 | 34.9 | 50.5 |
| 11766 | | 50.6 | 32.0 | 49.9 |
| 2605 | | 37.4 | 26.3 | 40.4 |
| | | | | |

| **formalin-fixed paraffin embedded neoplastic lesions§** | | | | |
|---|---|---|---|---|
| **patient** | **n. of sections** | **CpG1** | **CpG2** | **CpG3** |
| 11482 | 2 | 40.5 | 25.7 | 38.6 |
| | 4 | 38.6 | 24.8 | 37.6 |
| | 6 | 37.8 | 24.9 | 38.7 |
| 11766 | 2 | 48.4 | 29.2 | 47.3 |
| | 4 | 45.5 | 30.4 | 44.2 |
| | 6 | 45.3 | 30.7 | 44.8 |
| 6432 | 2 | 46.9 | 32.2 | 50.1 |
| | 4 | 48.7 | 36.0 | 53.6 |
| | 6 | 50.1 | 37.6 | 53.2 |
| 2605 | 2 | 42.8 | 29.2 | 43.3 |
| | 4 | 39.5 | 27.3 | 41.6 |
| | 6 | 42.3 | 29.0 | 40.8 |
| | | | | |

| **macro-dissected formalin-fixed paraffin embedded neoplastic lesions^{††}** | | | | |
|---|---|---|---|---|
| **patient** | **area (mm²)** | **CpG1** | **CpG2** | **CpG3** |
| 17193 | 9 | 22.8 | 30.2 | 44.2 |
| | 25 | 25.9 | 32.4 | 50.4 |
| | 100 | 28.8 | 28.1 | 47.6 |
| 6432 | 9 | 29.6 | 36.3 | 54.6 |
| | 25 | 23.6 | 35.2 | 55.0 |
| | 100 | 25.3 | 36.0 | 53.6 |

| | | | | |
|---|---|---|---|---|
| * DNA was extracted from neoplastic cells and evaluated for LINE-1 methylation at 3 CpG sites (CpG1, 2, 3) by pyrosequencing; t neoplastic cells were purified using anti-HMW-MAA antibody-coated magnetic beads from cryopreserved cell suspensions obtained by mechanical and enzymatic dissection of neoplastic lesions surgically removed from CM patients; ‡ surgically-removed neoplastic tissues from CM patients were flesh-frozen in liquid nitrogen and maintained at -80°C until use; § surgically-removed neoplastic tissues from CM patients were formalin-fixed and paraffin-embedded. Ten micrometer sections (total 2 to 6) were cut from paraffin embedded lesions and used for evaluation of LINE-1 methylation; ^{††}Neoplastic tissue of different extensions (area 9-100 mm2) was scraped from 15 micrometer sections of formalin-fixed paraffin-embedded CM tissues, and used for evaluation of LINE-1 methylation. | | | | |

**Table 15. qMSP analysis of LINE-1 methylation in laser capture microdissected tumor cells***

| **% LINE1 methylation** | | | |
|---|---|---|---|
| **patient** | **cell culture (6p) ^{†}** | **LCM ‡** | **Correlation §** |
| 629 | 89 | 74 | R=0.99 (95°/ CI: 0.86 1.0) |
| 767 | 76 | 67 | P=0.001 |
| 790 | 48 | 55 | |
| 812 | 22 | 43 | |
| 816 | 58 | 63 | |

| | | | |
|---|---|---|---|
| * DNA was extracted from neoplastic cells and evaluated for LINE-1 methylation by qMSP. † autologous neoplastic cell cultures were evaluated at the 6^{th} in vitro passage; ‡ neoplastic cells obtained from formalin-fixed paraffin-embedded CM tissues by Laser Capture Microdissection (LCM); § correlation between % LINE-1 values obtained from 6^{th} passage neoplastic cells and autologous LCM neoplastic cells was evaluated by the Pearson's correlation test. Coefficient of correlation is reported with the corresponding 95% Confidence Interval (Cl). Reported P value is 2-sided. | | | |

### REFERENCES

Altomonte, M., et al. 1993. Cancer Res. 53:3343-3348.
Ausubel, F.M., et al. 1998. Current Protocols in Molecular Biology John Wiley & Sons, NY.
Balch, C.M., et al. 2001. J Clin Oncol. 19:3635-3648.
Balch, C.M., et al. 2009. J Clin Oncol. 27:6199-6206.
Bhatia, S., et al. 2009. Oncology (Williston Park). 23 :488-496.
Calabro, L., et al. 2005. J Cell Physiol. 202:474-477.
Coral, S., et al. 1999. J Immunother. 22:16-24.
Cox, D.R. 1972. J Roy Statist Soc B. 34:187-220.
De Plaen, E., et al. 1994. Immunogenetics. 40:360-369.
De Smet, C., et al. 1996. Proc Natl.Acad Sci. U.S.A. 93:7149-7153.
Du, P., et al. 2008. Bioinformatics. 24:1547-1548.
Esteller, M. 2008. N Engl J Med. 358:1148-1159.
Fratta, E., et al. 2010. J Cell Physiol. 223:352-358.
Gama-Sosa, M.A., et al. 1983. Nucleic Acids Res. 11:6883-6894.
Gure, A.O., et al. 1997. Int J Cancer. 72:965-971.
Jager, E., et al. 1998. J Exp Med. 187:265-270.
Jennings, L., and G.M. Murphy. 2009. Br J Dermatol. 161:496-503.
Kaplan, E.L., and P. Meier. 1958. J. of the Am. Statistical Association. 53:457-481.
Lahtz, C., et al. 2010. J Invest Dermatol. 130:620-622.
MacKie, R.M., et al. 2009. Ann Oncol. 20 Suppl 6:vi1-7.
Mori, T., et al. 2006. Cancer Res. 66:6692-6698.
Mori, T., et al. 2005. J Clin Oncol. 23:9351-9358.
Ogino, S., et al. 2008. J Natl Cancer Inst. 100:1734-1738.
Pattamadilok, J., et al. 2008. Int J Gynecol Cancer. 18:711-717.
Rimoldi, D., et al. 1999. Int J Cancer. 82:901-907.
Sigalotti, L., et al. 2002. J. Immunother. (1997.). 25:16-26.
Sigalotti, L., et al. 2010. J Transl Med. 8:56.
Sigalotti, L., et al. 2007. J Cell Physiol. 212:330-344.
Tanemura, A., et al. 2009. Clin Cancer Res. 15:1801-1807.
Tellez, C.S., et al. 2009. Melanoma Res. 19:146-155.
Tibshirani, R., et al. 2002. Proc Natl Acad Sci USA. 99:6567-6572.
Wallace, N.A., et al. 2008. Gene. 419:75-81.
Woloszynska-Read, A., et al. 2008. Clin.Cancer Res. 14:3283-3290.
Yang, A.S., et al. 2004. Nucleic Acids Res. 32:e38.
You, Y., et al. 2010. Melanoma Res. 20:179-183.

## Claims

1. An in vitro method of prognosing melanoma within a clinico-pathological stage thereof and/or predicting and/or monitoring the response to a melanoma therapy in a subject and/or to identify a subject to be treated with a DNA hypomethylating agent comprising determining the methylation status of at least one cytosine residue in a methylation relevant region of the genes ABCA8, ADM, ALDH1A3, ARHGDIB, BGN, C9orf90, COL5A2, CSAG1, CTAG2, CTSK, DENND2D, EMILIN1, GSTT1, HCP5, LPXN, LY96, MAGEA1, MAGEA3, MAGEA6, MAGEC2, NNMT, OCIAD2, PAGE5, PCOLCE, PRDX2, RAB11FIP5, RNASE1 and SUHW1 (signature G) or allelic variants thereof or the genes ABCA8, ABCB4, ACOX2, ADORA2B, AMPH, APOD, ARHGDIB, ARHGEF7, ARSG, ATP6V1E2, AXL, BGN, C11orf1, C20orf100, C21orf70, C9orf90, CHMP4A, COL5A2, CSAG1, CST7, CTAG2, CTNNAL1, CXorf12, DENND2D, EMILIN1, EMP1, ERO1L, EVL, FAM107B, FANCE, FLJ25421, GAGE2, GRAMD3, HCP5, HNMT, KCNG1, KIAA0141, KIAA0367, KRT25C, LY96, MAGEA1, MAGEA10, MAGEA6, MAGEC2, MGMT, MT1G, NBL1, NDN, OLFM1, PDZD4, PGCP, PLA1A, PLD1, PPIE, PROCR, PRRX1, RAC2, RNASE1, RPUSD3, SLAMF9, SLCO4A1, SPATS1, TFF3, TIMP3, TMEPAI, ZNF532 and ZNF558 (signature F) or alleleic variants thereof in a biological sample obtained from the subject.

2. The method of claim 1 wherein for each of said genes, the methylation relevant regions are comprised in the nucleotide sequences as indicated in Table 3 signature G, or in complementary sequences thereof, more preferably for each of said genes, the methylation relevant regions consist of: nt 940 to nt 1061 of SEQ ID No. 109, nt 940 to nt 1061 of SEQ ID No. 125, nt 940 to nt 1061 of SEQ ID No. 132, nt 940 to nt 1061 of SEQ ID No. 138, nt 941 to nt 1062 of SEQ ID No. 139, nt 940 to nt 1061 of SEQ ID No. 140, nt 941 to nt 1062 of SEQ ID No. 142, nt 941 to nt 1062 of SEQ ID No. 143, nt 941 to nt 1062 of SEQ ID No. 149, nt 941 to nt 1062 of SEQ ID No. 150, nt 941 to nt 1062 of SEQ ID No. 155, nt 941 to nt 1062 of SEQ ID No. 161, nt 941 to nt 1062 of SEQ ID No. 165, nt 941 to nt 1062 of SEQ ID No. 166, nt 941 to nt 1062 of SEQ ID No. 167, nt 941 to nt 1062 of SEQ ID No. 168, nt 941 to nt 1062 of SEQ ID No. 169, nt 941 to nt 1062 of SEQ ID No. 181, nt 941 to nt 1062 of SEQ ID No. 182, nt 940 to nt 1061 of SEQ ID No. 201, nt 940 to nt 1061 of SEQ ID No. 205, nt 940 to nt 1061 of SEQ ID No. 206, nt 941 to nt 1062 of SEQ ID No. 207, nt 940 to nt 1061 of SEQ ID No. 208, nt 940 to nt 1061 of SEQ ID No. 209, nt 940 to nt 1061 of SEQ ID No. 210, nt 941 to nt 1062 of SEQ ID No. 211, nt 941 to nt 1062 of SEQ ID No. 212, nt 941 to nt 1062 of SEQ ID No. 213, nt 940 to nt 1061 of SEQ ID No. 214, nt 940 to nt 1061 of SEQ ID No. 215, nt 941 to nt 1062 of SEQ ID No. 216, nt 941 to nt 1062 of SEQ ID No. 217, nt 940 to nt 1061 of SEQ ID No. 218, nt 940 to nt 1061 of SEQ ID No. 219, an allelic variant or a complementary sequence thereof.

3. The method of claim 1 wherein for each of said genes, the methylation relevant regions are comprised in the nucleotide sequences as indicated in Table 3 signature F, or in complementary sequences thereof, still preferably for each of said genes, the methylation relevant region consist of: nt 940 to nt 1061 of SEQ ID No. 25, nt 941 to nt 1062 of SEQ ID No. 61, nt 941 to nt 1062 of SEQ ID No. 80, nt 940 to nt 1061 of SEQ ID No. 109, nt 940 to nt 1061 of SEQ ID No. 125, nt 940 to nt 1061 of SEQ ID No. 126, nt 941 to nt 1062 of SEQ ID No. 127, nt 941 to nt 1062 of SEQ ID No. 128, nt 941 to nt 1062 of SEQ ID No. 129, nt 940 to nt 1061 of SEQ ID No. 132, nt 940 to nt 1061 of SEQ ID No. 133, nt 941 to nt 1062 of SEQ ID No. 134, nt 940 to nt 1061 of SEQ ID No. 135, nt 941 to nt 1062 of SEQ ID No. 137, nt 940 to nt 1061 of SEQ ID No. 138, nt 940 to nt 1061 of SEQ ID No. 140, nt 940 to nt 1061 of SEQ ID No. 141, nt 941 to nt 1062 of SEQ ID No. 142, nt 941 to nt 1062 of SEQ ID No. 143, nt 941 to nt 1062 of SEQ ID No. 144, nt 941 to nt 1062 of SEQ ID No. 145, nt 941 to nt 1062 of SEQ ID No. 146, nt 941 to nt 1062 of SEQ ID No. 147, nt 941 to nt 1062 of SEQ ID No. 148, nt 941 to nt 1062 of SEQ ID No. 149, nt 941 to nt 1062 of SEQ ID No. 150, nt 940 to nt 1061 of SEQ ID No. 151, nt 940 to nt 1061 of SEQ ID No. 152, nt 940 to nt 1061 of SEQ ID No. 153, nt 941 to nt 1062 of SEQ ID No. 155, nt 940 to nt 1061 of SEQ ID No. 156, nt 940 to nt 1061 of SEQ ID No. 157, nt 940 to nt 1061 of SEQ ID No. 158, nt 941 to nt 1062 of SEQ ID No. 159, nt 940 to nt 1061 of SEQ ID No. 160, nt 940 to nt 1061 of SEQ ID No. 162, nt 941 to nt 1062 of SEQ ID No. 165, nt 941 to nt 1062 of SEQ ID No. 166, nt 941 to nt 1062 of SEQ ID No. 167, nt 941 to nt 1062 of SEQ ID No. 168, nt 940 to nt 1061 of SEQ ID No. 170, nt 941 to nt 1062 of SEQ ID No. 172, nt 940 to nt 1061 of SEQ ID No. 173, nt 941 to nt 1062 of SEQ ID No. 175, nt 941 to nt 1062 of SEQ ID No. 176, nt 941 to nt 1062 of SEQ ID No. 177, nt 940 to nt 1061 of SEQ ID No. 178, nt 941 to nt 1062 of SEQ ID No. 179, nt 941 to nt 1062 of SEQ ID No. 180, nt 941 to nt 1062 of SEQ ID No. 181, nt 941 to nt 1062 of SEQ ID No. 182, nt 940 to nt 1061 of SEQ ID No. 183, nt 940 to nt 1061 of SEQ ID No. 184, nt 940 to nt 1061 of SEQ ID No. 185, nt 940 to nt 1061 of SEQ ID No. 186, nt 940 to nt 1061 of SEQ ID No. 187, nt 940 to nt 1061 of SEQ ID No. 188, nt 940 to nt 1061 of SEQ ID No. 189, nt 940 to nt 1061 of SEQ ID No. 190, nt 941 to nt 1062 of SEQ ID No. 191, nt 940 to nt 1061 of SEQ ID No. 192, nt 940 to nt 1061 of SEQ ID No. 193, nt 941 to nt 1062 of SEQ ID No. 194, nt 940 to nt 1061 of SEQ ID No. 195, nt 940 to nt 1061 of SEQ ID No. 196, nt EP 12 702 032.9 (Clean copy)
941 to nt 1062 of SEQ ID No. 197, nt 941 to nt 1062 of SEQ ID No. 198, nt 941 to nt 1062 of SEQ ID No. 199, nt 940 to nt 1061 of SEQ ID No. 200, nt 940 to nt 1061 of SEQ ID No. 201, nt 940 to nt 1061 of SEQ ID No. 202, nt 940 to nt 1061 of SEQ ID No. 203, nt 940 to nt 1061 of SEQ ID No. 204, an allelic variant or a complementary sequence thereof.

4. The method of any of previous claims further comprising determining the methylation status of at least one cytosine residue in the methylation relevant region of the *LINE*-1 repetitive sequence as in SEQ ID No. 220.

5. The method of any of previous claims further comprising determining the methylation status of at least one cytosine residue in a methylation relevant region of:
a] at least one of the following genes: *ALOX12B, , FGF4, IGLL1, OCIAD2, PAGE5, S100A9, SLC6A11, SLC6A18, TUB, BATF, , CRHR1, , CTSK, , FLJ33860, GJB5, GRM4, , , MAGEA10, MGC35206, MLSTD1, SLC18A2, SORBS2,* and *TRIM40;*

6. The method of any one of previous claims wherein the melanoma is a cutaneous melanoma, preferably at stage III or IIIC.

7. The method of any one of previous claims comprising the steps of:
(a) isolating DNA from a sample obtained from a subject;
(b) subjecting said DNA to methylation-sensitive sequence modification through incubation with suitable chemicals, or contacting said DNA with a reagent capable of selectively binding to methylated DNA;
(c) optionally amplifying said DNA as obtained in b), and
(d) determining the methylation status of said DNA.

8. In vitro use of a kit for performing the method of any one of previous claims, comprising a chemical able to differentially modify DNA sequence depending on methylation status, or a reagent capable of selelctively binding to methylated DNA, and probes, primers and/or aptamers specific for each of genes or repetitive sequence as defined in claims 1 to 5.

9. The in vitro use according to claim 8 wherein said kit is in the form of an array.

## Patentansprüche

1. In vitro-Verfahren zum Prognostizieren von Melanomen innerhalb eines klinisch-pathologischen Stadiums davon und/oder zum Voraussagen und/oder Überwachen des Ansprechens auf eine Melanomtherapie in einem Patienten und/oder zum Identifizieren eines Patienten, der mit einem DNA-hypomethylierenden Mittel behandelt werden soll, umfassend das Bestimmen des Methylierungsstatus mindestens eines Cytosin-Restes in einer für die Methylierung relevanten Region der Gene ABCA8, ADM, ALDH1A3, ARHGDIB, BGN, C9orf90, COL5A2, CSAG1, CTAG2, CTSK, DENND2D, EMILIN1, GSTT1, HCP5, LPXN, LY96, MAGEA1, MAGEA3, MAGEA6, MAGEC2, NNMT, OCIAD2, PAGE5,PCOLCE, PRDX2, RAB11FIP5, RNASE1 und SUHW1 (Signatur G) oder allelische Varianten davon, oder der Gene ABCA8, ABCB4, ACOX2, ADORA2B, AMPH, APOD, ARHGDIB, ARHGEF7, ARSG, ATP6V1E2, AXL, BGN, C11orf1, C20orf100, C21orf70, C9orf90, CHMP4A, COL5A2, CSAG1, CST7, CTAG2, CTNNAL1, CXorf12, DENND2D, EMILIN1, EMP1, ERO1L, EVL, FAM107B, FANCE, FLJ25421, GAGE2, GRAMD3, HCP5, HNMT, KCNG1, KIAA0141, KIAA0367, KRT25C, LY96, MAGEA1, MAGEA10, MAGEA6, MAGEC2, MGMT, MT1G, NBL1, NDN, OLFM1, PDZD4, PGCP, PLA1A, PLD1, PPIE, PROCR, PRRX1, RAC2, RNASE1, RPUSD3, SLAMF9, SLCO4A1, SPATS1, TFF3, TIMP3, TMEPAI, ZNF532 und ZNF558 (Signatur F) oder allelische Varianten davon in einer biologischen Probe, die von dem Patienten erhalten wird.

2. Verfahren nach Anspruch 1, wobei für jedes dieser Gene die für die Methylierung relevanten Regionen in den Nukleotidsequenzen wie in Tabelle 3 Signatur G angegeben sind, oder in komplementären Sequenzen davon, mehr bevorzugt bestehen für jedes der Gene die für die Methylierung relevanten Regionen aus: nt 940 bis nt 1061 von SEQ.-ID Nr. 109, nt 940 bis nt 1061 von SEQ.-ID Nr. 125, nt 940 bis nt 1061 von SEQ.-ID Nr. 132, nt 940 bis nt 1061 von SEQ.-ID Nr. 138, nt 941 bis nt 1062 von SEQ.-ID Nr. 139, nt 940 bis nt 1061 von SEQ.-ID Nr. 140, nt 941 bis nt 1062 von SEQ.-ID Nr. 142, nt 941 bis nt 1062 von SEQ.-ID Nr. 143, nt 941 bis nt 1062 von SEQ.-ID Nr. 149, nt 941 bis nt 1062 von SEQ.-ID Nr. 150, nt 941 bis nt 1062 von SEQ.-ID Nr. 155, nt 941 bis nt 1062 von SEQ.-ID Nr. 161, nt 941 bis nt 1062 von SEQ.-ID Nr. 165, nt 941 bis nt 1062 von SEQ.-ID Nr. 166, nt 941 bis nt 1062 von SEQ.-ID Nr. 167, nt 941 bis nt 1062 von SEQ.-ID Nr. 168, nt 941 bis nt 1062 von SEQ.-ID Nr. 169, nt 941 bis nt 1062 von SEQ.-ID Nr. 181, nt 941 bis nt 1062 von SEQ.-ID Nr. 182, nt 940 bis nt 1061 von SEQ.-ID Nr. 201, nt 940 bis nt 1061 von SEQ.-ID Nr. 205, nt 940 bis nt 1061 von SEQ.-ID Nr. 206, nt 941 bis nt 1062 von SEQ.-ID Nr. 207, nt 940 bis nt 1061 von SEQ.-ID Nr. 208, nt 940 bis nt 1061 von SEQ.-ID Nr. 209, nt 940 bis nt 1061 von SEQ.-ID Nr. 210, nt 941 bis nt 1062 von SEQ.-ID Nr. 211, nt 941 bis nt 1062 von SEQ.-ID Nr. 212, nt 941 bis nt 1062 von SEQ.-ID Nr.213, nt 940 bis nt 1061 von SEQ.-ID Nr. 214, nt 940 bis nt 1061 von SEQ.-ID Nr. 215, nt 941 bis nt 1062 von SEQ.-ID Nr. 216, nt 941 bis nt 1062 von SEQ.-ID Nr. 217, nt 940 bis nt 1061 von SEQ.-ID Nr. 218, nt 940 bis nt 1061 von SEQ.-ID Nr. 219, eine allelische Variante oder eine komplementäre Sequenz davon.

3. Verfahren nach Anspruch 1, wobei für jedes dieser Gene die für die Methylierung relevanten Regionen in den Nukleotidsequenzen wie in Tabelle 3 Signatur F angegeben sind, oder in komplementären Sequenzen davon, wobei immer noch bevorzugt für jedes dieser Gene die für die Methylierung relevante Region besteht aus: nt 940 bis nt 1061 von SEQ.-ID Nr. 25, nt 941 bis nt 1062 von SEQ.-ID Nr. 61, nt 941 bis nt 1062 von SEQ.-ID Nr. 80, nt 940 bis nt 1061 von SEQ.-ID Nr. 109, nt 940 bis nt 1061 von SEQ.-ID Nr. 125, nt 940 bis nt 1061 von SEQ.-ID Nr. 126, nt 941 bis nt 1062 von SEQ.-ID Nr. 127, nt 941 bis nt 1062 von SEQ.-ID Nr. 128, nt 941 bis nt 1062 von SEQ.-ID Nr. 129, nt 940 bis nt 1061 von SEQ.-ID Nr. 132, nt 940 bis nt 1061 von SEQ.-ID Nr. 133, nt 941 bis nt 1062 von SEQ.-ID Nr. 134, nt 940 bis nt 1061 von SEQ.-ID Nr. 135, nt 941 bis nt 1062 von SEQ.-ID Nr. 137, nt 940 bis nt 1061 von SEQ.-ID Nr. 138, nt 940 bis nt 1061 von SEQ.-ID Nr. 140, nt 940 bis nt 1061 von SEQ.-ID Nr. 141, nt 941 bis nt 1062 von SEQ.-ID Nr. 142, nt 941 bis nt 1062 von SEQ.-ID Nr. 143, nt 941 bis nt 1062 von SEQ.-ID Nr. 144, nt 941 bis nt 1062 von SEQ.-ID Nr. 145, nt 941 bis nt 1062 von SEQ.-ID Nr. 146, nt 941 bis nt 1062 von SEQ.-ID Nr. 147, nt 941 bis nt 1062 von SEQ.-ID Nr. 148, nt 941 bis nt 1062 von SEQ.-ID Nr. 149, nt 941 bis nt 1062 von SEQ.-ID Nr. 150, nt 940 bis nt 1061 von SEQ.-ID Nr. 151, nt 940 bis nt 1061 von SEQ.-ID Nr. 152, nt 940 bis nt 1061 von SEQ.-ID Nr. 153, nt 941 bis nt 1062 von SEQ.-ID Nr. 155, nt 940 bis nt 1061 von SEQ.-ID Nr. 156, nt 940 bis nt 1061 von SEQ.-ID Nr. 157, nt 940 bis nt 1061 von SEQ.-ID Nr. 158, nt 941 bis nt 1062 von SEQ.-ID Nr. 159, nt 940 bis nt 1061 von SEQ.-ID Nr. 160, nt 940 bis nt 1061 von SEQ.-ID Nr. 162, nt 941 bis nt 1062 von SEQ.-ID Nr. 165, nt 941 bis nt 1062 von SEQ.-ID Nr. 166, nt 941 bis nt 1062 von SEQ.-ID Nr. 167, nt 941 bis nt 1062 von SEQ.-ID Nr. 168, nt 940 bis nt 1061 von SEQ.-ID Nr. 170, nt 941 bis nt 1062 von SEQ.-ID Nr. 172, nt 940 bis nt 1061 von SEQ.-ID Nr. 173, nt 941 bis nt 1062 von SEQ.-ID Nr. 175, nt 941 bis nt 1062 von SEQ.-ID Nr. 176, nt 941 bis nt 1062 von SEQ.-ID Nr. 177, nt 940 bis nt 1061 von SEQ.-ID Nr. 178, nt 941 bis nt 1062 von SEQ.-ID Nr. 179, nt 941 bis nt 1062 von SEQ.-ID Nr. 180, nt 941 bis nt 1062 von SEQ.-ID Nr. 181, nt 941 bis nt 1062 von SEQ.-ID Nr.182, nt 940 bis nt 1061 von SEQ.-ID Nr. 183, nt 940 bis nt 1061 von SEQ.-ID Nr. 184, nt 940 bis nt 1061 von SEQ.-ID Nr. 185, nt 940 bis nt 1061 von SEQ.-ID Nr. 186 nt 940 bis nt 1061 von SEQ.-ID Nr. 187, nt 940 bis nt 1061 von SEQ.-ID Nr. 188, nt 940 bis nt 1061 von SEQ.-ID Nr. 188, nt 940 bis nt 1061 von SEQ.-ID Nr. 189, nt 940 bis nt 1061 von SEQ.-ID Nr. 190, nt 941 bis nt 1062 von SEQ.-ID Nr. 191, nt 940 bis nt 1061 von SEQ.-ID Nr. 192, nt 940 bis nt 1061 von SEQ.-ID Nr. 193, nt 941 bis nt 1062 von SEQ.-ID Nr. 194, nt 940 bis nt 1061 von SEQ.-ID Nr. 195, nt 940 bis nt 1061 von SEQ.-ID Nr. 196, nt 941 bis nt 1062 von SEQ.-ID Nr. 197, nt 941 bis nt 1062 von SEQ.-ID Nr. 198, nt 941 bis nt 1062 von SEQ.-ID Nr. 199, nt 940 bis nt 1061 von SEQ.-ID Nr. 200, nt 940 bis nt 1061 von SEQ.-ID Nr. 201, nt 940 bis nt 1061 von SEQ.-ID Nr. 202, nt 940 bis nt 1061 von SEQ.-ID Nr. 203, nt 940 bis nt 1061 von SEQ.-ID Nr. 204, eine allelische Variante oder eine komplementäre Sequenz davon.

4. Verfahren nach einem der vorigen Ansprüche, ferner umfassend das Bestimmen des Methylierungsstatus mindestens eines Cytosin-Rests in der für die Methylierung relevanten Region der wiederkehrenden *LINE*-1-Sequenz wie in SEQ.-ID Nr. 220.

5. Verfahren nach einem der vorigen Ansprüche, umfassend das Bestimmen des Methylierungsstatus mindestens eines Cytosin-Rests in einer für die Methylierung relevanten Region von:
a) mindestens einem der folgenden Gene: *ALOXI2B, FGF4, IGLL1, OCIAD2, PAGE5, S100A9, SLC6A11, SLC6A18, TUB, BATF, , CRHR11,, CTSK, , FLJ33860, GJB5, GRM4, , , MAGEA10, MGC35206, MLSTD1, SLC18A2*, *SORBS2, und TRIM40;*

6. Verfahren nach einem der vorigen Ansprüche, wobei das Melanom ein kutanes Melanom ist, bevorzugt in Stadium III oder IIIC.

7. Verfahren nach einem der vorigen Ansprüche, umfassend die folgenden Schritte:
(a) Isolieren von DNA aus einer Probe, die von einem Patienten erhalten wurde;
(b) Unterziehen dieser DNA einer Methylierungssensitiven Modifizierung durch Inkubieren mit geeigneten Chemikalien oder Inkontaktbringen der DNA mit einem Reagens, das in der Lage ist, selektiv an methylierte DNA zu binden;
(c) optional Amplifizieren der in b) erhaltenen DNA, und
(d) Bestimmen des Methylierungsstatus der DNA.

8. In vitro-Verwendung eines Sets zum Durchführen des Verfahrens nach einem der vorigen Ansprüche, umfassend eine Chemikalie, die in der Lage ist, DNA-Sequenzen abhängig von dem Methylierungsstatus differentiell zu modifizieren, oder ein Reagens, das in der Lage ist, selektiv an methylierte DNA zu binden, und Sonden, Primer und/oder Aptamere, die spezifisch für jedes der Gene oder jede der wiederholenden Sequenzen sind, wie in Anspruch 1 bis 5 definiert.

9. In vitro-Verwendung nach Anspruch 8, wobei das Set die Form eines Arrays aufweist.

## Revendications

1. Procédé in vitro de pronostic d'un mélanome à un stade clinico-pathologique de celui-ci et/ou de prédiction et/ou de surveillance de la réponse à une thérapie contre le mélanome chez un sujet et/ou d'identification d'un sujet à traiter avec un agent d'hypométhylation de l'ADN comprenant la détermination du statut de méthylation d'au moins un résidu cytosine dans une région importante pour la méthylation des gènes ABCA8, ADM, ALDH1A3, ARHGDIB, BGN, C9orf90, COL5A2, CSAG1, CTAG2, CTSK, DENND2D, EMILIN1, GSTT1, HCP5, LPXN, LY96, MAGEA1, MAGEA3, MAGEA6, MAGEC2, NNMT, OCIAD2, PAGE5, PCOLCE, PRDX2, RAB11FIP5, RNASE1 et SUHW1 (signature G) ou des variants alléliques de ceux-ci ou des gènes ABCA8, ABCB4, ACOX2, ADORA2B, AMPH, APOD, ARHGDIB, ARHGEF7, ARSG, ATP6V1E2, AXL, BGN, C11orf1, C20orf100, C21orf70, C9orf90, CHMP4A, COL5A2, CSAG1, CST7, CTAG2, CTNNAL1, CXorf12, DENND2D, EMILIN1, EMP1, ERO1L, EVL, FAM107B, FANCE, FLJ25421, GAGE2, GRAMD3, HCP5, HNMT, KCNG1, KIAA0141, KIAA0367, KRT25C, LY96, MAGEA1, MAGEA10, MAGEA6, MAGEC2, MGMT, MT1G, NBL1, NDN, OLFM1, PDZD4, PGCP, PLA1A, PLD1, PPIE, PROCR, PRRX1, RAC2, RNASE1, RPUSD3, SLAMF9, SLCO4A1, SPATS1, TFF3, TIMP3, TMEPAI, ZNF532 et ZNF558 (signature F) ou des variants alléliques de ceux-ci dans un échantillon biologique provenant du sujet.

2. Procédé selon la revendication 1, dans lequel, pour chacun desdits gènes, les régions importantes pour la méthylation sont comprises dans les séquences nucléotidiques telles qu'indiquées dans le tableau 3, signature G, ou dans des séquences complémentaires de celles-ci, de préférence pour chacun desdits gènes, les régions pertinentes pour la méthylation sont constituées de : nt 940 à nt 1061 de SEQ ID NO : 109, nt 940 à nt 1061 de SEQ ID NO : 125, nt 940 à nt 1061 de SEQ ID NO : 132, nt 940 à nt 1061 de SEQ ID NO : 138, nt 941 à nt 1062 de SEQ ID NO : 139, nt 940 à nt 1061 de SEQ ID NO : 140, nt 941 à nt 1062 de SEQ ID NO : 142, nt 941 à nt 1062 de SEQ ID NO : 143, nt 941 à nt 1062 de SEQ ID NO : 149, nt 941 à nt 1062 de SEQ ID NO : 150, nt 941 à nt 1062 de SEQ ID NO : 155, nt 941 à nt 1062 de SEQ ID NO : 161, nt 941 à nt 1062 de SEQ ID NO : 165, nt 941 à nt 1062 de SEQ ID NO : 166, nt 941 à nt 1062 de SEQ ID NO : 167, nt 941 à nt 1062 de SEQ ID NO : 168, nt 941 à nt 1062 de SEQ ID NO : 169, nt 941 à nt 1062 de SEQ ID NO : 181, nt 941 à nt 1062 de SEQ ID NO : 182, nt 940 à nt 1061 de SEQ ID NO : 201, nt 940 à nt 1061 de SEQ ID NO : 205, nt 940 à nt 1061 de SEQ ID NO : 206, nt 941 à nt 1062 de SEQ ID NO : 207, nt 940 à nt 1061 de SEQ ID NO : 208, nt 940 à nt 1061 de SEQ ID NO : 209, nt 940 à nt 1061 de SEQ ID NO : 210, nt 941 à nt 1062 de SEQ ID NO : 211, nt 941 à nt 1062 de SEQ ID NO : 212, nt 941 à nt 1062 de SEQ ID NO : 213, nt 940 à nt 1061 de SEQ ID NO : 214, nt 940 à nt 1061 de SEQ ID NO : 215, nt 941 à nt 1062 de SEQ ID NO : 216, nt 941 à nt 1062 de SEQ ID NO : 217, nt 940 à nt 1061 de SEQ ID NO : 218, nt 940 à nt 1061 de SEQ ID NO : 219, un variant allélique ou une séquence complémentaire de celles-ci.

3. Procédé selon la revendication 1, dans lequel, pour chacun desdits gènes, les régions importantes pour la méthylation sont comprises dans les séquences nucléotidiques telles qu'indiquées dans le tableau 3, signature F, ou dans des séquences complémentaires de celles-ci, de préférence encore pour chacun desdits gènes, les régions pertinentes pour la méthylation sont constituées de : nt 940 à nt 1061 de SEQ ID NO : 25, nt 941 à nt 1062 de SEQ ID NO : 61, nt 941 à nt 1062 de SEQ ID NO : 80, nt 940 à nt 1061 de SEQ ID NO : 109, nt 940 à nt 1061 de SEQ ID NO : 125, nt 940 à nt 1061 de SEQ ID NO : 126, nt 941 à nt 1062 de SEQ ID NO : 127, nt 941 à nt 1062 de SEQ ID NO : 128, nt 941 à nt 1062 de SEQ ID NO : 129, nt 940 à nt 1061 de SEQ ID NO : 132, nt 940 à nt 1061 de SEQ ID NO : 133, nt 941 à nt 1062 de SEQ ID NO : 134, nt 940 à nt 1061 de SEQ ID NO : 135, nt 941 à nt 1062 de SEQ ID NO : 137, nt 940 à nt 1061 de SEQ ID NO : 138, nt 940 à nt 1061 de SEQ ID NO : 140, nt 940 à nt 1061 de SEQ ID NO : 141, nt 941 à nt 1062 de SEQ ID NO : 142, nt 941 à nt 1062 de SEQ ID NO : 143, nt 941 à nt 1062 de SEQ ID NO : 144, nt 941 à nt 1062 de SEQ ID NO : 145, nt 941 à nt 1062 de SEQ ID NO : 146, nt 941 à nt 1062 de SEQ ID NO : 147, nt 941 à nt 1062 de SEQ ID NO : 148, nt 941 à nt 1062 de SEQ ID NO : 149, nt 941 à nt 1062 de SEQ ID NO : 150, nt 940 à nt 1061 de SEQ ID NO : 151, nt 940 à nt 1061 de SEQ ID NO : 152, nt 940 à nt 1061 de SEQ ID NO : 153, nt 941 à nt 1062 de SEQ ID NO : 155, nt 940 à nt 1061 de SEQ ID NO : 156, nt 940 à nt 1061 de SEQ ID NO : 157, nt 940 à nt 1061 de SEQ ID NO : 158, nt 941 à nt 1062 de SEQ ID NO : 159, nt 940 à nt 1061 de SEQ ID NO : 160, nt 940 à nt 1061 de SEQ ID NO : 162, nt 941 à nt 1062 de SEQ ID NO : 165, nt 941 à nt 1062 de SEQ ID NO : 166, nt 941 à nt 1062 de SEQ ID NO : 167, nt 941 à nt 1062 de SEQ ID NO : 168, nt 940 à nt 1061 de SEQ ID NO : 170, nt 941 à nt 1062 de SEQ ID NO : 172, nt 940 à nt 1061 de SEQ ID NO : 173, nt 941 à nt 1062 de SEQ ID NO : 175, nt 941 à nt 1062 de SEQ ID NO : 176, nt 941 à nt 1062 de SEQ ID NO : 177, nt 940 à nt 1061 de SEQ ID NO : 178, nt 941 à nt 1062 de SEQ ID NO : 179, nt 941 à nt 1062 de SEQ ID NO : 180, nt 941 à nt 1062 de SEQ ID NO : 181, nt 941 à nt 1062 de SEQ ID NO : 182, nt 940 à nt 1061 de SEQ ID NO : 183, nt 940 à nt 1061 de SEQ ID NO : 184, nt 940 à nt 1061 de SEQ ID NO : 185, nt 940 à nt 1061 de SEQ ID NO : 186, nt 940 à nt 1061 de SEQ ID NO : 187, nt 940 à nt 1061 de SEQ ID NO : 188, nt 940 à nt 1061 de SEQ ID NO : 189, nt 940 à nt 1061 de SEQ ID NO : 190, nt 941 à nt 1062 de SEQ ID NO : 191, nt 940 à nt 1061 de SEQ ID NO : 192, nt 940 à nt 1061 de SEQ ID NO : 193, nt 941 à nt 1062 de SEQ ID NO : 194, nt 940 à nt 1061 de SEQ ID NO : 195, nt 940 à nt 1061 de SEQ ID NO : 196, nt 941 à nt 1062 de SEQ ID NO : 197, nt 941 à nt 1062 de SEQ ID NO : 198, nt 941 à nt 1062 de SEQ ID NO : 199, nt 940 à nt 1061 de SEQ ID NO : 200, nt 940 à nt 1061 de SEQ ID NO : 201, nt 940 à nt 1061 de SEQ ID NO : 202, nt 940 à nt 1061 de SEQ ID NO : 203, nt 940 à nt 1061 de SEQ ID NO : 204, un variant allélique ou une séquence complémentaire de celles-ci.

4. Procédé selon l'une quelconque des revendications précédentes, comprenant en outre la détermination du statut de méthylation d'au moins un résidu cytosine dans la région importante pour la méthylation de la séquence répétée LINE-1 comme dans SEQ ID NO : 220.

5. Procédé selon l'une quelconque des revendications précédentes, comprenant en outre la détermination du statut de méthylation d'au moins un résidu cytosine dans une région importante pour la méthylation de :
a] au moins l'un des gènes suivants : ALOX12B, FGF4, IGLL1, OCIAD2, PAGE5, S100A9, SLC6A11, SLC6A18, TUB, BATF, CRHR1, CTSK, FLJ33860, GJB5, GRM4, MAGEA10, MGC35206, MLSTD1, SLC18A2, SORBS2, et TRIM40.

6. Procédé selon l'une quelconque des revendications précédentes, dans lequel le mélanome est un mélanome cutané, de préférence au stade III ou IIIC.

7. Procédé selon l'une quelconque des revendications précédentes, comprenant les étapes de :
(a) isolement d'ADN d'un échantillon provenant d'un sujet ;
(b) soumission dudit ADN à une modification de séquence sensible à la méthylation par incubation avec des produits chimiques appropriés, ou mise en contact dudit ADN avec un réactif capable de sélectivement se lier à l'ADN méthylé ;
(c) amplification facultative dudit ADN tel qu'obtenu à l'étape b), et
(d) détermination du statut de méthylation dudit ADN.

8. Utilisation in vitro d'un kit pour la mise en oeuvre du procédé selon l'une quelconque des revendications précédentes, comprenant un produit chimique capable de modifier différentiellement la séquence d'ADN en fonction du statut de méthylation, ou un réactif capable de sélectivement se lier à l'ADN méthylé, et des sondes, des amorces et/ou des aptamères spécifiques de chacun des gènes tels que définis dans les revendications 1 à 5.

9. Utilisation in vitro selon la revendication 8, dans laquelle ledit kit se trouve sous la forme d'une puce.


### SEQUENCE LISTING

<110> Azienda Ospedaliera Universitaria Senese Centro di Riferimento Oncologico - Istituto Nazionale Tumori - Aviano
   Sigalotti, Luca
   Maio, Michele
<120> MARKERS OF MELANOMA AND USES THEREOF
<130> PCT 115407
<150> EP 11 152 864.2 <151> 2011-02-01
<160> 233
<170> PatentIn version 3.5
<210> 1
   <211> 2001
   <212> DNA
   <213> Homo sapiens
<400> 1
   aaattaaaaa aaaaattttt ttttttgagt tggggtcttg ctttgtcacc cagcctggag      60
   tgcagtggca cgaacatggc tcactgcagc ctcgacttcc caggctaagg cgatccttcc     120
   tacctcagcc tcccaagtag ctgggactgt ggacgcacac cactacaccc ggctattttt     180
   ttttaaaatt ttttattttt ttggtatttt tgtagcaatg gggtttcacc atgttgccca     240
   ggctggtcac aaacttctgg gcttggatga tcctcctgtc ttggcctccc agtgttagga     300
   ttacaggtgc gagccaccgc gtctggctga tgtcttctca tgatgagatt ccggttgtgc     360
   cttcctggct ggaatgccac atagatgatg ctgtgacctg cgaagacctt cacatctgga     420
   ggcacacgac accctcctgc ctctcacagg tgatttattt taaccccttg gtcaagatgt    480
   ctgattcctc tactgtatag ttactctttt tccttttgtt actaataagc aatctatcaa     540
   gagatgcttc gtgaccgtgc tgacaccctc ccccctcact gaagtcttcc ccccagattt     600
   agcccccact gatgagtttt gcacacaggc tgtcttcttg taggcagccc cctctgctac    660
   ctttgttttt ccgggaagga ccttagatcc tgggtaggtg ctcatcctgc ctggcagtcg     720
   agtgcaatgc tgctgtcctg gccaatgccc agccttcctc atttgccccg gcttcaaggc     780
   attggttcac agtcccgtac gggcagctag accatctcac agcaagcagc actctgggag     840
   gctcatctca tttcctgccc tggaagcttc ccttcctcac tgcagccact gaagctgcag     900
   gctagatcaa ggggaccaca gagttgtcca gtccccaggg tgggggtgcc aggagccctt     960
   gtcctccgta gcaggtctca gaggctgacg atggggaaat cgcactggtg aagctgccgt    1020
   ggggtcagga ggaacctgcg ataagacgcc caacagttag ggcaggtgct ttgaggtttg    1080
   gagcctggga ccccacagga agtgacttcg tgctgagcta atgttgcttc atcctggcgc    1140
   cagtctcagg ccaagagctg caggctggca tggctggggg gatgtcagcg gagtgccctg    1200
   agcctgggcc aggaggtctg cagggccagt ccccagggcc aggcaggcag tgtccccctc 1260
   ccatcacgcc cacctcctgg agcctgcccc cgtggagggc ctacgtggct gccgccgtcc 1320
   tctgctacat caacctcctg aattacatga actggttcat cattgcaggt gaggagggga 1380
   tggctaccct gggcagtacc tgctgctgtg cccaccagcc aggggctgca gatccatggt 1440
   ggccttccag ccatcaccca tctttctgct cctgggccca tctctggtgt tctcacctct 1500
   agaggtggtt ttcagctgtg ctcgaatcgg ggaggaccaa ctgatgttcc caatccagcc 1560
   tcaactaaga agggacacag gctgggcatg gtggctcccg cttgtaattc cagcactttg 1620
   ggaggctgag gcaggaagat tgcttgagcc caggagtttg agaccaacct ggacaacata 1680
   gtgagactct gtctctattt atttattttt ttttttgaga cagagtctcg ctctgtcgcc 1740
   caggctggag tgtagtggcg caatctcagc tcactgcaac ctctgtctcc caggttcaag 1800
   tgattctcct gcctcagcct cccgagtagc tgggattaca ggcgtgcacc accacatccg 1860
   gctaattttt gtattttgag tagaggagga gtagggacgg ggttttacca tgctagtcag 1920
   gctggtctcg aactcctgac ctcaggtgat ccacccgcct cggcctccca aagtgctggg 1980
   attacaggca tgagccaccg t 2001
<210> 2
   <211> 2001
   <212> DNA
   <213> Homo sapiens
<400> 2
   attgtggcct gtgtgtaaga gtatgctcat gtgaagctac attgtgtttg cttgtggaca      60
   tgaggtgttt gtggctcctg gatgctttgt ctgttgaatc ctgaccccaa gtgacctgct     120
   gatacagggc accttggaag tcaattaggc caaaagaccg tgtctgtgca ctaatgagaa     180
   gctttcccca ggtgcccctg cccaccttct ccacctctcc tgtggttcct gcctggcttg     240
   ggtttggcct ttttaaccca aagacaacat gcatccaaat gagattttct ctagtgcata     300
   cgtctctctc tccctccctt ctcctctgcc ttccctgtat ctcagaaaga aaaggcctag     360
   gaataaggaa aaggaggagg aagaaacatt tttttgagga cataccatgc ggcaggcctg     420
   tgctgggcag tatacacgca ttataggcaa ttgtagacag ggcttccctt gtgtcaggca     480
   ctgttctaag cacttcaccc ctaaggctgg gttaatccac gtatccaccc tatgaggtag     540
   gtgttgttct tttcccactt tcacagagaa ggcaagaaat cacacagtga gaaagtggca     600
   gagctgggat tcaggcctca gcttctccca cacaagccca cagatgggag gatgaacaag     660
   gagtcagagt ggacctgtgg ctctcccctg agaggatacc aggattcgag gctcagcgcc     720
   ctgggaggag tgaacccctc agtttcctga gcctcatcag aagcagtctg gggaggctgg     780
   ggcccagcca atggcagtgc aggtcccagc tcctcctcct cccaggctgg gccagcacac     840
   ccggcaggct ctgtcctgga aacaggcttc aacgggcttc cccgaaaacc ttccccgctt     900
   ctggatatga aattcaagct gcttgctgag tcctattgcc ggctgctggg agccaggaga      960
   gccctgagga gtagtcactc aggtaagggc atctgtgtcc gcatgtgccc tgccccttgg     1020
   catacgtggc aggaggacac ttctctgctg gggagtggtg ggatgtggcc tggacacatc     1080
   tgccaccctg ggtcctgatg gtggaggaat aaggggctct gggttctttc aggtctcccc     1140
   agccgggcaa aggtaggagc aggggccagg ccggaagctc agcaggcagg gtagctctgc     1200
   ctgggcctgc aaagccctgc cccactcctc cccaagtagg gatgggctgg gtgagagccc     1260
   ctggggctaa gggcggggcc ttcctgggtg ttccctgaga agctgcagga gtcctacctt     1320
   agacaggact gagcatgcaa gatggtgatt caaaacagag ctccccccaa ccccggcccc     1380
   cacctccacc catcccccgt ccaagtggcg caggtggtgt ggagacaggg tggtgggcaa     1440
   aggttccccc agccaccctc atccaggatc cacacacaag gggaaggaca gaatccgtct     1500
   gcacacaaac aggcaggcag ctcattgaaa cttgaggaaa gggtctgagg cctaaggctg     1560
   ggcagttccc ggagggtggg ctctagagcc agcctgcctg ggttcaaatc ctagctctgc     1620
   cacttattag ctgtgtgacg ttgagcaagt taattaatcc ctctgtgttc ctctttcctc     1680
   atctgtaaat tgggaataaa aaatatgccc tttcctaagg ttgctgtgaa ggaataatgt     1740
   actgattcat acaaagaact tagagcggtg cctggcacgg catgaatgct tcatggatat     1800
   tggctgttat cactactctg ctttgcagtt gatcatgcag aggctccaga gagcctggtg     1860
   agcttcagcc tggaccagca tgggaaggcc atcatgagcc ctttagtggc taggcagaca     1920
   ggttccttgg ggtcccaggg tgcacagttg gttgaaacca cagtgcatgg gtcttaatgg     1980
   acacaccccc atctccagga a                                               2001
<210> 3
   <211> 2001
   <212> DNA
   <213> Homo sapiens
<400> 3
   tgggtccaga gaggttaagt gacctgttca aggtcacaca tcaatccagt tgtagacctg      60
   gggccagaat ccaggattag atctcaccag gctctctgtt cccaccacac tcttcttcca     120
   aatggggcat cagctcctgg gtgaggactg tggatctgcc acaggaaccc cattccacca     180
   accttcccaa gcctctccat cccaatccca catcgctccc cttcacctgc acaaactgct     240
   ccatggccca ctggtccagg caggtgtagt tctccaggaa gcgcagcttc tccaactgaa     300
   actggtccct ggactgagcc tccgcctgcc tctctagcag ctggaagata gtggcaccga     360
   gcagcaggta gcagacatag gccagcagca ggggcagcac ccggccaccc cagcagctgc     420
   agagcccagc actgggcatg ctgtggccag gaccctgcca gggccgtggg gctggctatg     480
   ggggagaggt gggaaacagg tgaggagtaa gcacctaggg gcctgtgccc aggctgccgc     540
   ctgccctgcc ctcctcctcg gcacaggtgt ctggaggctg gggagactgt ttgaacagtg     600
   cggctcagct tggctgcact aagtgctatg ctgtgagtgc aaacaggccg gccaccccca 660
   cccggccttt gtttccatgg acagcagcaa gtcagtgtca gggacccttc aaagagacct 720
   accaggttct cctgagtgct tcatgtctct ctctctgtct ccaagttgga tagatgtttc 780
   tgggctaaat cggataggtg tgctcactct gtccaggccc cagtgggctc tctcggccgc 840
   ccccagcgtc ttcacccttg aaggctgtaa gaccccagct taggagctgg gaagaggtca 900
   gaatgggtat gaataaacct tctactcagt tgccttggct acaaaatggg gcaggataat 960
   tgaactgcct attgggggaa cctcaggatg agtaaactgc gtgacaaatc agggcaaagt 1020
   aaatagacag gtgattgtct cactcagcct gatggtaaga gtatcagggt tgattctgct 1080
   gataagttgg attcattggt tttataactc ttgctaattt gtcttgcatg aagccctcta 1140
   gtgggttacc ttcagaggat atgtcggtga aatatatgtg tgaattaaaa agaaactata 1200
   agtatatcct gatcctggga tggtaagagg ggctgtctct gagtggatgg aattgagagt 1260
   gctttccttt ctctttgcct gtgtgtgtgt gtgtacacat gcttgtttat gtacatgtat 1320
   aacagcttta tacagatata attcacatgc tgtatcgtcc acacatttaa agtgtgcaat 1380
   tcagtgggtt ttagtatatt cacagggtct tcaagatcca gccctgttgc aacatgtaac 1440
   agtacttctt ccttctttct ggctgagtaa tatttcattg tgtggctata acacatttgt 1500
   ttatccattt gatttctagt tgatggacat ttgggttgtc cctgtttcct tctattaaga 1560
   acaaagcagc tatgaacctc tgtgtatgag ttttttttgt ggacatgtgt ttgcattact 1620
   cacaggtaca tacctaggag tagaatttct ggactatctg gccactctgt attgaacttt 1680
   ttgaggaact gccaggctat tttccaaagt ggctgaacca ttttatattc tcactagccc 1740
   agggcgagga ttccaatatc tctgctgtgt tttctgctgt ttcctttaat gagctgtggc 1800
   tgcagacact gtgagttgcc tgctgagtat cctttccctc ctccccagct agcagaaccc 1860
   cggctttgta agtgttaacc ccaggtaatt gagcataatt ggtctaagtc atagtgctaa 1920
   tcttgctccc ctctgtctca gcccctgaga gcagtgaagt cactgaagac ccataagaca 1980
   gtgggcaaga gggccaggct g 2001
<210> 4
   <211> 2001
   <212> DNA
   <213> Homo sapiens
<400> 4
   tgggcgcagc aggccatggg ttaccacggc cagacccagc agcagcaggg gccagcgctg        60
   cctgaggttg gggcctggct caccaggggc ctcaaggccc ccctggcctg tccctggcct       120
   catcggccct cagggtcaga ggtccttgtg gcctgacttg cagtgtgggc tccctagaga       180
   gtggtgccct ggtccctctc gctggcagca gctgtcccat tgcaccccag tccatgtggc       240
   cccatcctca tcctctggtc cctgccccca gccaggcccc ttccagagtc catgaccgtt       300
   acccctgggg ttctgtccca gaggcacatc tgccaactgc cctgctgtgg tcaggatcca      360
   gggctcaact gcctcccaca cctgtcagct tggccttgcc cctggggccc tgggtgcttt      420
   gtttggggac ctcccaccac aggcagaggg tcagcaagag ctgaggtcac acagggaggt      480
   gggatgcgcc atggcccccc aagaccctcc cttggggctt tcctttggag gcacctgcgc      540
   cctggatggg cttgagtccc ctcggggctg gtcccaagag gtgatgcacc tgacttccct      600
   ccttccccac ttccccgagg gcgccctggg tctgtggagc atctcactgg ggtccagaga      660
   gctgcccagc aggagtcccc cagggcttga gctcaggggc ctgggctgag gctcctccaa      720
   ccggtcctag agggaggtgt ggttagagag caggaaacag gtttgggttt agccagaagc      780
   cactcaacgg ttcaagtcca ggaatcatga gataagaatg aaaatgctgt ttaatgcacc      840
   atgttcagca cctcactgac ctcacatcac acagacacac acacacacac caaccacccc      900
   agcttcctga aggtataatt gacaaaactg tatatatatt taccacctac agtgcgatgc      960
   tttgccacac tgcaaaatga ttaaatcaag ctgacacaca cgtccgtcat ctcacatact     1020
   tatgcttttg tagtgagaac atggaagatc taatcttagc aattttccag tatacaatac     1080
   agtttttaac tgtagactcc atgttgtacg atacatctcc agagttcatt cttatagatt     1140
   tcactcaaag gccagtagct tcctcctgag cggtgagtgc atcatgtcat ggcttctgac     1200
   aggcccccgg tcctctccca gcccagcatc cccgaccctc tcccaacatc tcgcgaccct     1260
   gtgctttggt gttgagggag cagtggcagt caggagggac tgagcacctg tgctgggcgg     1320
   ccagggccag gctcttttgc tggtgctgcc agagacgcct caggtcaccc aagagtctca     1380
   ggttcctctc cttcctccag aggcggggct ggaaatgctg ctggctctgc tcacactcca     1440
   gggctgggtt gaggttggtg ttgggagata atgcatgtca gtgttgtttt cttcatctgt     1500
   gaaaaagcag taacatacac acaacctaaa atttatcatc ttgacctttt tttttttttt     1560
   gagagtcttg ctgtgtcgcc aggctggagt gcagtgtggc gtgatctcag ctcactgaaa     1620
   cctccgaatc cctggttcaa gcgattctcc tgcctcagcc tcctgagtag ctgggattac     1680
   aggcatgcac caccacgaca agctaatttt tgtattttta gtagagatgg ggttcaccat     1740
   gttggccagg ctggtctcga tctcctaacc tcatgatcca cccgcctggg cctcccaaag     1800
   tgctgggatc acaggtgtga gccaccgcgc ctggcccatc ttgaccattt ttaagtacac     1860
   agttcagtgg cattaactac attcacaatg tcgtgcaacc atcaccacca tccatttcta     1920
   gatttttctt ttttcagagt cttgctctgt tgcccaggct ggagtgcagt ggcgcaatct     1980
   tagctcatgg caatctcttt c                                              2001
<210> 5
   <211> 2001
   <212> DNA
   <213> Homo sapiens
<400> 5
   attttttgta tttttagtag agatggggtt tcaccatgtt agccaagatg gtctcgatct 60
   cctgacctca tgatccacct gccttggcct cccaaagtgc tgggattaca ggcgtgagcc 120
   accgcacctg gcccagaata tttatttaga gagtgatgtc aggcctaagt gttgatagtg 180
   ggtagtagtg ggcaagatgc ctggccctct ctctttcttc tcatgagaga aggtggaatg 240
   ccttccacca aagaaacgag acctccccat gaccagctgc tccactaacc acacatcctc 300
   cagtgatgcc tctgaatggt cccgaggggc tgtggtggcc gggcagagcc aggcaggagc 360
   cagagtcagc ctggggggtg atggagctga ggccatcacc ggtctgacag tggaccagta 420
   tggcatgctg tataaggtgg ctgtgccgcc tgccaccttc tcaccaactg gcctcccatc 480
   tgtggtgaat atgagcccct tgcccccgga aaaaaaataa tattgcatat gcagttgatt 540
   aaatggcata ctccatggct attaggaatg atgaaaatgg gctgggtgcg gtggctcaca 600
   cctgtaatcc cagcactttg gtaggccaag gtgggcagat cacctgaggt cgggagtttg 660
   acaccagcct aatcaacatg gagctgtgtt tactgagtgg ggctttcgca ggctggagct 720
   aagaatttcc agtatgcgta acagccacag cccaaatatc tgccagtgag ttgtgtaatt 780
   ccccagagca ggcctgggca gtgtctgggt ggggcctggg agccacagga gacgcccaaa 840
   gccaggcaga gcccgggggc gagggggcgg caggcaggtg tagcgctgcc ctgggcgggc 900
   ttgcaccccc acacccaagt gagcggcctg ctcactcctc agctgcagga gccagacgtg 960
   tggagtccca gcagaggcca acctgtgtct cttcatctcc gtgagaaagg tgcccccgaa 1020
   gtgaaagaga tggcctggtg gaaagcctgg gtaagtggga gctggcagga gatttgggtt 1080
   cctgcatggt gttgggtgct ggtcaccagg gagtcctgag aaatgtaaag tcgcccttgg 1140
   ctgggagtgg ctcagtctac atttggctgc ctcctgggcc aagcatggag ggtgggaagg 1200
   ggccgagaag aaacagtccc aggcctagag tgcccacggc agggatggga cctgggccgg 1260
   gagacaggct gatgaagaaa tctagaaatg caagggggaa gctgaggttt gagaagagtc 1320
   caggggaggg acccttgcag gggtggggcc actcaaagga gggggtatca tggttgagcc 1380
   ttgtgtgtaa atgtgtatgg ggtgcacaca cgtgtgcatt tggggagaat caggcatact 1440
   gctcctggca gagggaactg tgtgcagagg gtcagagggc ggggagcttg gctttgaggg 1500
   aagctgctca gcacagctca agggtcctgg taccggagga gatggacctg gggaagcagg 1560
   cagggccagg tcctgaaagg ccttgcctgg caggctgagg agttaggctt taccttaaga 1620
   gaagtgggaa ggcactgaag ggctttgagc atgagagtaa cctgaacctg ctgttgggga 1680
   cccatcaatg ccttcacctc agggatcaat gatgttcatt tgtcaagact ggagagggaa 1740
   gaggggaagc aggaggacct agcctgctag agcctggagg aacccggggc tcagggcagc 1800
   ccatgcccag cagaactgat gggttctcgg gagctttctg tctatggttg agcctgaggc 1860
   catcgctgtg ctaaggagtt gaatatccag aaggggacag aggtgacatc tcccggctgc 1920
   cggctctgca cacccttttc ctttcctccc aggcagcctc aggatgtcag ggctgcactt 1980
   tctgttttgc agatgatgaa a                                                2001
<210> 6
   <211> 2001
   <212> DNA
   <213> Homo sapiens
<400> 6
   aaacacaaaa ggaaaaaaaa taggcaaaaa taaagattat ctcatatgga atttcagtaa 60
   gatacttcta ttacttggaa aattccaaag gtttttgtcc taggagaaat gccacattct 120
   tttttacaca gcagattcct actctaaccc tccccatact tcctacctcc ttagttctcc 180
   ttgccaccga attatcctga catatgtact tacgcgtttg gttatttggg gcctccatca 240
   cattaaactt tatgtttgat gacattatgg aatttgtttt gcttgctcct gtgtttcttg 300
   aacctactac actgcctaat ataatatgtg ctcaaaattt ttgaaagaaa aatataaacc 360
   aggtaaacaa atcatttctt acagtgaagt gatggaaatc agtcttaata gaaatcagat 420
   atccctcatt agtcctagtg gtgttgctgc cagtctttaa acaacttcct accaaagatt 480
   ttcttcagag ccatcatcac ttctttgttc ctaagggtgt agattagtgg attcagtaca 540
   ggggtgacgg cgctatacat gatggccatt atccggtcct gaatcatgga ggtggctgaa 600
   gcaggacgaa tatatgtgaa gcccacaggt ccatagaaaa gacataccac cataaaatgg 660
   gaggcacaag tggacagagc cttgtggagt attctgcagg acctgttctt aaacagaagg 720
   aagccaatta catagaagca ggagagaaga gtcagaaaga aagctcccat ggatatgctg 780
   cctgtgacaa tggaaagaag ccattgattg agtaatgtgt cactacaggc caattctaag 840
   agcggcttga catcgtagaa gaagtgattg agtttctgag agccacaaaa actcaggtgt 900
   gcagtcatga cagaatgcat cagagcgtaa aagaagctga tgagccaggc cgcagctgcc 960
   aacagaatac acacctgggg gttcatgatg acagtgtagc gaagaggatt gcagatggca 1020
   acaaaacggt caaaggccat gatagccagt aaaatggcct ctgtgcttcc caaaaagtgg 1080
   aagaagtgta gctgggtgat acagcctaga aaagatatag ccctgcgact gcacacgagg 1140
   tttacgagca gcttgggcag tgtcactgaa gaataagaaa tatccagaca agaaaggttt 1200
   cccagaaaaa aatacatagg ggagtggagt tgtggttcca aaacaaccat caccaatata 1260
   gatccatttc caatcaagtt tatcaggtaa atgattaaga aaatcccaaa gaagaaaggc 1320
   tgcagctcct gaacaccagt caggccaagt agaagaaact cattcattgt agtgacattc 1380
   tccattgctc tgggaagcaa atttaacaat aacagaatta atttttctga atttttaatt 1440
   ttacactgtg aggattaaat aaagcaagtt tactatttgg aggaacctag gggtgagcaa 1500
   cagtgtgaag aatagttacg aacagtaaaa ctaaatttat gtacaacaaa attgaggaag 1560
   actaatatac ctgataaatt ctgagctgtt aaaatggcac tagactaata aaagcattat 1620
   attatgaaca aatcagatat agataaaggg catttgtttt caaaactagg aagtgcaaga 1680
   tttgatggac ataatcataa cctcatgtat ggcattagaa ttatgtatta caaaaatttt     1740
   aaacatacaa acagggaata gtttttcaac ttatgattct aggttctggc aaaaattcag     1800
   atgtagctca gtggggattt catcacttct tctaagatac ataaaagcca caagagagta     1860
   acacatgtct tctgacaacc caaactttca gtgaaagtaa gaaacataaa cacccacaaa     1920
   ccaaatgttg tatgagtaga ggagaaaaaa acagcaaaat tagcatcatt agtcacaaga     1980
   ctgtgtcata gcaatgaggc a                                               2001
<210> 7
   <211> 2001
   <212> DNA
   <213> Homo sapiens
<400> 7
   ccagaggaag tgggggtctt ctgaagggtt taagtaggct gggaggacat gatcttagga      60
   agctcactct ggtgtcagtt gcaggatgga tttgagagga gcaagttagg tgtagatgcc     120
   catgatgatg ccaagatctg ggcaacagac aggaaggccc tagctcagaa gtggctctag     180
   ggaaggtgag gtgcatagaa ttgagagatg ctcagtagat ggcatgagca gtgcttgatg     240
   attgtctggg ttggtggagg aaggtggaca gggaaaagca gaaagctacg atggtgcctc     300
   aaggggctga gtgatgtcac tcacagagac agaatgtata gagtgaatgt tcagactcac     360
   aggaagtcca aaactacata ccccaacgtg aggtgctgtg ggacatccgg ggtgcagggt     420
   ccagagagca ggtaggtaga gtttagaaga gggctgggtc cacaatgcag ccttggatgt     480
   tctcaatgta agagttgtgg gagatgaagc cttgtgagtg gatgggaaca cccaggtgca     540
   tttcaggtga agcaagggga caagaggctg aggacacaga caagcaaatc ctagatcttc     600
   catcagtccc tagaaggcac gatgtgtgcc cctcccagca cacagcctga gccctagcac     660
   agagctggcc gcagagaggg cagcagtgaa tgtgtcctcg gtggttcctc cagatggggc     720
   ctttgtccgc agtgcacttg tctctgcctg ggttgctata gtaacccaca gatgcagaga     780
   gacttggcct ccgtgttgcc atggaaacca gcaattgggt gtccctgtgt ggcatggcca     840
   ctgagacctt gaggattcag tctggtcctg aagggtttgg ggggagactg cgaccagaag     900
   atgtttccat gtcctaatta atgggtgatg gtggttgtta gtctgactgt tgccacggtg     960
   atgaagggag acatccaagt gctggtttca gtactgaggc gaatacaggg aatttcaaca    1020
   ggctccaggt cttactatgc agcctgaagt gggaccatcc cttaaaccca ctccatcctg    1080
   tggccacgat gggggccagg acacctttgc cttctttctg ggtttctttc tttgccgaga    1140
   cagggatttt gttcccagga ggcactccct ggcccatggg atctcagcat tcaaagcagc    1200
   acaggaaacc tgggcccctg aaacggggcc accgaagaga tcggtaagct ttcaacatcc    1260
   tgcctttagc ccatgggccc aaccattgcg tcagctccac ccacccaccc tcactgacct    1320
   caacccttta cagtcccaag gcctcccctt cctgatggtg ctgctcccct atcaccacgt    1380
   ggagccccac aggtatatcc ccgcaaaccc ctcttgctcc agacaggtga tcagggaggc 1440
   ttcattactc caggagctgc ccaggacctc agggaggtcc cccaccatgg gagtaagggc 1500
   ctctccccca gcctccaggg ctccggcagc tccacactcc ggccctctgc ctcttctgga 1560
   gggctcctcc ttccagcttc tgcctgtctc cccacccccc agtgcttcat tcccatggct 1620
   caggtcatca ctgccctcat ctggactgcc tgcctgcctg cctgccatcc ggggcccaca 1680
   gagtgagcac tctatggaag tccctctacc ctgatccatc acgtaactca cctactgagg 1740
   cagctgagac cagggcactg aaggaggcct tgagtgacca ggtgtggcca ggcagcaggc 1800
   agggatgatg aggtgaggct gcaaggatgt ggagagtcac cccttctttt cctcctcagg 1860
   agaacaacca ggaggaagta ctggaaggaa ggaagggaga tcgctcggtg agctggaggg 1920
   gaggagggcg tgccggccct gggaaagggc caaccacagg agactgtgag ggaggtgggc 1980
   ggaagacgtg ggtggctcag g 2001
<210> 8
   <211> 2001
   <212> DNA
   <213> Homo sapiens
<400> 8
   agcttgagta aattccaatt aattcttccc aactcattct aaagccacag aaaaacctca 60
   ctgaaaagga cttctgtcta cgttaaagtg cttttaccca ataccttgtg aagctccctt 120
   tcctggggga cacagttcgt ggtctctggc ttttggcagg tggtccactg catttccaca 180
   ttcaggtgat actccaggtg gtcagtgacc tgtgggcgcc aggcgtgggg gaggcacagc 240
   ttgtcccctt ctcccctgtc tgcggtttct gtgcctgggg gctccgctac cccacgtcca 300
   agggcaagga gctggtccta ggcggattag cgggctcctc tctcctctat ttgactcatg 360
   attgtttttg tcattccaag agaattaacc agaaaccatc agaaacatga actttccccc 420
   tgggcaaatc tatcccttgg gtaatgcaag tgacgctagt gattctggat aggggtctgt 480
   aaactttttc cgtaaagggc tacatagtaa acatcttacg ttttattggc catgtggtct 540
   ctgtaactac tcagttcggc atgagtgtgg cagcagcaag cagtggcaga caaagcacag 600
   gcatgctcat ggctaggtca caagaacacg acttacaaaa gtgggcggca ggcagttttg 660
   gcccttggga tgtaatgtgc tgaggatccc tggtcctggg ttttgtttaa gtgccttaat 720
   ttcatcaccc tagtgaattt atgcttctct taaagtgctc acatcaagct aagcaaggtt 780
   gaatttccct tgagtgggac tattcctgac accagtggcc acccgatgtg ggcgtatcag 840
   gcctggggag aggcgggaag tcatacactc acctgcctct ggactttaag gactcggaag 900
   atcctgaagt ggtacttgtc atcactttcc ttgttatact ggtcggtgat ccactgcaag 960
   ctgtccttcg catagttttc tactgccatc acttcatgga cgcttagaaa ggttttcttc 1020
   cttgcttggt aggggagggc catcagagtc aatagaatgg ccaacaggag ctgtagggcc 1080
   tgccagggct cagccatcat ccttcagctg cagaggaaca ggaagagtgt tctctgtact 1140
   cctcagggac aagtcgaatc acactgaccc tacctgccct ggcaggattt aagagcagct 1200
   ggtggtggac acccacagct ctcctctcct cctcctcccc cttcatcagc agctgaactc 1260
   gaggggagag cagcaagaga ctgtgcaatg gtttttgcat aagttcatgc cacacacaca 1320
   caggataaca cacacatttt ctcttttgag gggctgctcc ctcagcaaca cacaggcttc 1380
   tgtggaccca gcattgtctt ggtgggagcc aggcgagggc ccctgggagt cctggaggat 1440
   ggtagctgct ggagacacgg ggagattgga tgccaggact ggcgcattca taggtgagca 1500
   gtggggggtg ggcactgtgg ggaggggccg cctgcaccgg aagccactct cccccagcct 1560
   ggttcctgga gacgagttct ggaaaatccc ctcccttaga ggacaaagga gcagaggcac 1620
   cctacagaaa ggtgtgaagg gagggagagt caactctgac accgacgatt ccccagtacc 1680
   gacaagtcct agagtggcct ccgctggact tgcatgtttt ggaggaggag gcagtggcga 1740
   ggaagcttca ggtgtgagac acagggagct ttctggttgg acattctggt ctcctaaagg 1800
   tcccatcctg gggacctgat gggagtcccc agacttggct gccagttaga gaaggaagaa 1860
   gccactggtg ggtggaggct ggagcttgtg tcccacggca cacagaatgc aggcttagtg 1920
   cggttatgtt cgattcctag aaggcctgtt atggtgtctt ttgggaagat gcgatataaa 1980
   aatgtgcagt gatggctcct g 2001
<210> 9
   <211> 2001
   <212> DNA
   <213> Homo sapiens
<400> 9
   agggtctcct cctattctaa agctaatatt cagttgtata tatacatata aaccacagtc      60
   cctttatcca ttcatccact gatggacacc taggctgttt ccataattct ttagtaaatc     120
   acagaaccct cttgttttct cttctaactt agcaaacttc atttgattga gtcacaacaa     180
   aatatttatc acaagtaaac aagaaagatg gaaaatttct gatcaatgat gaccatccat     240
   gttgctgatg atggaacaaa aagccagatt aaacaagtaa gacagataaa atcgttgatt     300
   agatccattg cttcaactag aaagggaaca taggtgaaac atctactctg tactcttgta     360
   tgtggggttt taaaaacaaa ccttaatacc aaaatgaaag acaagatcag caaatattag     420
   gtgagaagtt taaaaaatta ttggtatttt gcatatagga aaataattta aaataaagtc     480
   cctttacttc attgtacatt taattgaatt tggaggtttt ttatagtaag aaatgaaagt     540
   tcgattgaga agagatgcca gctttcactc accatttctg acagtctccc aggaaccttc     600
   ttcgtacact gcctttggta agctcctttc tcactggctt tattggcttg tgtctttcat     660
   ttgtaggaac agttaactga caaagctgca gaggcaggct gagaggtggc tccaagagaa     720
   ccctggccaa gccatttatg gtcaggggag tagccaagcc ctcagtccat ccctcgggaa     780
   agatgggctg ctgctgcatg gccgacgtgt atggcctggt tcacccaccc tctttgcctt      840
   gattttctgt ccttactttt caggctgctt tcttggctat acctccatct cagcaaagaa      900
   gccacagctg gcctgagagc ttgggggctt ctgcagcctg cagccctttg gaaacaatgg      960
   gttacatgag gctgaggctg gccactccat ccccagctga cgggagtcca cattaaccat     1020
   gttactgata atgatggagt cattgtgtta catggacaac agcatggaca ttcgggatgt     1080
   ctggaggagg ggtgggggcc cattctgttg caggagtcac actgctgggg gctccagctg     1140
   ctgcaaggtg ctgcactcag tggccaatga gctgcctggt gtgagttcag cctcaccccc     1200
   tctctgtttt tgtattttgg gtgggaagct ctctagacct ggaggaagcc atggacttgg     1260
   gctgctttat aacttacaat ttccctgtta gggatttgcc tctttaaaat ggtgaatgag     1320
   ttgctaataa aacaacagaa ttaaaccaat aatttggcca ttgaaatgaa aaccactgtg     1380
   agaggagcag ataggagtgt aaggagagag cgactcaagt gacagaggag gccgttgttt     1440
   agatgactct gggagatggc actgaagctg tgcgtgaaag atgagaagag ggcagacatg     1500
   tgaagggttg agggaggaga gtcccaggca gaagccacag caaatgctaa gccctgtaag     1560
   cttggtgcac tcccagagca ggcaggaggc cggttggctg gaacaacata aaccagggag     1620
   tcacccggga aggggctaag gacggagtga gtttgagccc ttttgatgtg acatgggagg     1680
   gcatggtaag attttcatcc ttagatgaaa ccttttccaa gggtttttca gtgggagaga     1740
   tgcatgatgt aattgctggt gacgaggatt gctagggctt ctctgtggag ccaagattac     1800
   aggggaagag agtggaagca ggtggctggc tagtggagtc caggtgagcg atgaaggtgg     1860
   ctgctgtagg aagacagcag tgaatgattg tgctctctac tgtggaggtg gagccactgg     1920
   gattaaatga tgaaaaaaat atggggcggg gaagggcgag gtgagggaaa gagaagaata     1980
   aagccaactc ctgggtttga g                                               2001
<210> 10
   <211> 2001
   <212> DNA
   <213> Homo sapiens
<400> 10
   tatttaaaga tcatgtcaag gggaggataa tggggctcag agaggcaaaa tttgatggtg        60
   tttattaagc agagagaaac acagaagctg gagccgtcta gtccttagag agtgtgctcc       120
   aaggtgggac cagggaggga gcaaggcctt ggctgggcct gggcaaagct accagaggac       180
   caaccggctt cctctcctct gattccacag cctactgctg ctggtgaatt tctgatcaag       240
   aattctgctc cccacaaaga actgcctaga gggtgggacc aagccacccc tcacacttga       300
   gcatccagtc cactccatcg tgatgatgat gatgatcata gtattggtga ctacagctaa       360
   ccctcattaa gctctcacta tgtgccaggc tccgttccaa gctctttaca aataccaact       420
   ccttcaaact ccccaaccac cctcagaagt agcgtttcta ccccgatttt atagatgaga       480
   aaattgaggc attgttaaat aactctccaa gtttatacag cttgtaagtg gaaaagccaa 540
   gattcaaact caggccatct gactccagaa cctgcacatc agactgcccc gcacttaact 600
   gttgttggta acagttagtt gtccatctgc ttagcatgtc catcccttct ggggttggct 660
   ggggcaagtc agggcccaac tcggcacagc cagctccgga gcactaacct aatgggggtt 720
   ttgcaagact cgggactgtg tgcttcacct ggaaagtgcc aggtgtgtgg ataagccatg 780
   tgagcagctg gcttcctggg caatgcccct ccccacttca gtgtcccagg cccctttagg 840
   gcccagccct ccaggcactg tgagctggga atgaggtgaa ggaggactag gcttaccctc 900
   tgtgtgcctc cattttctca ccagtaaact gtgtgtgttg gatcttacga tcttctaaaa 960
   taactccagc tccaaatgaa ggccagtgga cccatgaaca cgctggctct tgcccagtgt 1020
   gctgagaaga gggcaggagg gaggagtagg gtgggcacgg cagggcacag ggtgcagggc 1080
   aggtgggttc agtttgattt caaagccgac tgcccgccgc cccaggtgct ctggtgtacc 1140
   caataggggc tgcattggcc aggcggccta ggctctgcgg gtggagtgaa gatttctcta 1200
   cctcccagtt tctccctttt cagctctggt aggagagagg aagagtgaag aggaagtcaa 1260
   aagttctgtt catctggaag ggctgggagt tgcttcctgg gatggggagc tccgaagcca 1320
   attcagaaga cggccccggg gtctggcggg gaggcccacc gctgcccaga gggctcggcc 1380
   ggccgggagg gggcgctgtg tcctccccac gccctgcccg cgggctccta gcagcgttcg 1440
   ccgcgctgca acgcaatgcg cgcccggctt tcccctgggc agcgttcagt gcccgcttgt 1500
   cctgcagggc accgccgtct ccctttcatc ctggggctcc caggtccaaa gggttcccag 1560
   atccagcctc ttgcctgaac caaagtggcc aggcactcag tttgtaagat gaacaaatga 1620
   aggaaagaag gaacgaatga ataagggctc atcttgcaga gcgttcgcgt ctcagcgcgc 1680
   accaggcaca tcagtgccaa aggtgctggg ggtcgggccc agcgcccggc gcggctggaa 1740
   tgggggcggc gaggagctcc tcctagccag ccgcagggcg ggaactgccc aaacgcaggt 1800
   cgcagcctca ctgggttccg cggcggctcc gctcagtagg gaccgccctg ttccctagcc 1860
   cccaatagcc tcgccctcat aaattcacct gagcaccctg gcccaagtca ccccagatcc 1920
   ctgggacttc ccaggccccg acctcggccg ggcgggaggc tgatccttta ccgcggcccc 1980
   cttcccccat tcccggcttc t 2001
<210> 11
   <211> 2001
   <212> DNA
   <213> Homo sapiens
<400> 11
   aatgaggttt tcaacaagcg tcttggaccc ttagaagttt caagtgacag cccttattag     60
   tggcacccct taggggctcc ctcaaactca atgccaagac tagcctgact ggagggaact    120
   tagacaatgg agtgggcatt tgatgttcca gcatcttgag tgggtgccat tattctgtga    180
   cacctggatc ctggggttcc ataagctggg gttctaggat gtccattctt agttgagtct     240
   catttcctgt catatcgacg tcaaaggccc aagaccttcc tcctcccctt gagcaaacaa     300
   gccacgccct gcaccaaagt ccatctcacc ttccagctac cctaggtaat tttcctggta     360
   attcagtgtt tctaggaaag catgagtctt tccacccccc gccctgagat ttactgacat     420
   atgaacacat atgattggca tgatataaga accactcatc tgctggccat ctctccaggt     480
   ctggagccag agaagatgat ctgaaattgt agcaggagaa attgaggtag gatactaaga     540
   aagcttttca ggagtggggc taggcaagag gtgcagcatg agggaataag agtgaatcct     600
   cagtatctaa gggaggtggc aggtggcggg ggacttcttt ctttgggtca tctttggtgg     660
   tgatttgaca ggaaggaaca aagtggcttc aaacattata caagtctcta gtctgttctg     720
   tgtcctgttt tctttctcat tctttcagtg tggaatctat atgaccctgg gagggatgtt     780
   ggttggaaga atgaccagct gatggagatg ctgctgtaat tattggtggt aataatgggc     840
   agcagtgagc cacccggtgt gacagtgtag gagaaaacag tccaaactcc tgccaaactc     900
   tctctactga tggcaaatca gaggagactc aaattgtaag tttatagtgg tctggctttt     960
   ggccatgaca atgacacctt gcccttttaa tttggggccc gtgcaaatat tcactgaaag    1020
   ctgtcaagag gaaaacagaa ttggttattg aatcacttgc ttcctctagg tgtatgaaaa    1080
   ataatttcaa gtttaacaaa cacaaggaaa ccgcagggtc catgtcaaag ctgatgagct    1140
   atttctgaaa ctcgtgcaga attgtggttt gtgtggtcta tgtcacggca cccttgaggg    1200
   agagtgggca attgcctgaa cttggaggct gtgtcctgtc cccaggctgc tccagggctg    1260
   cctccttccg actgggcctt cttatctggg actgttgagg gcaacaggcc ttccgaagac    1320
   cagtgaagaa ggaggccctg caaacaggag gctgacaggg taggaacgag gccatgatcc    1380
   ctttgcagaa ggacaaccag gaggagggtg tctgccccat ctgccaggag agcctgaagg    1440
   aggccgtgag caccaactgc ggacatctct tctgtcgagt gtgcctgaca cagcatgtgg    1500
   agaaggcctc agcctctggg gtcttctgct gccccctctg ccggaagccc tgttctgagg    1560
   aggtgctagg gacaggctat atctgcccca accaccagaa gagggtgtgc aggttctgtg    1620
   aggagagcag acttcttcta tgtgtggaat gcctggtgtc ccctgaacac atgtctcatc    1680
   atgaactgac cattgaaaat gccctcagcc actacaaggt aagcctgggt caccgcagcc    1740
   aggccctgcc tccacctcgc tgaggtgctg catcctacat gttcatcatg cctggcacct    1800
   cagagtagct caacaatgga catctctctt tgtttcttct gcttcatcct gttttggacc    1860
   cttgtcttgc ttttctgtgt atattttgag gctgatgttt ccatgcatta atgtgagtct    1920
   gtctaaaaga ggatattgtc agtgtgatgt tagagtccca gtctgctcat ctgtagaata    1980
   gagtaattgg actaactaat g                                              2001
<210> 12
   <211> 2001
   <212> DNA
   <213> Homo sapiens
<400> 12
   gacagatcta gactcagttt caaaaaaaaa aaaagactca ggccaaatct ccttccttta        60
   tgatgcaaac tttctcggcc ccattccacc taatcaggct gctatttcct cccctggctc       120
   ccagcattac acctctaggt caagcctttg ccacagtagg ctataattta tgtatctatc       180
   cctctacctc tctctctctc tctttttttt tttttttttt ttttttttga gacagactct        240
   cactctgtca cccaggttgg agtgcagtgg cgccatctca gctcactgca acctctgcct       300
   cctgggttca ggcaattctc ctatctcagc ctgccactat gcccagctaa tttttgtatt       360
   tttagtagag acagggtttt gctatgttag gccgggctgg tcttgaactc ctgacctcag       420
   gtgatccacc ggcctcagcc tcccaaagtg ttgggattac aggcatgagc caccacaccc       480
   agccttctgc ctctcttgct agaccatgaa tttgttgggc gctgggtctc tttcctctct       540
   ttccttggtg cccagcaaag tgctcagcac agaagaggtg cttgatatat gcttattgaa       600
   gtaatcatca acaaacattt atcaatcatc aatatattta tttattgagg atttactatg       660
   aggtggggaa actgaggcat gaaaaaagag ctggttcaag gcaaatctag aatgaggatc       720
   caggctcctg ccccccagag ctagcctgtg catttgagaa aggaccaata ccccccaaga       780
   taagcatctg gaatggggac gagggtgcag cgtggttacc ttggatgccc atctcctttc       840
   gacaccactg gatgaagtta gagacattgt ccctggcctg gaaggtacct ggctgggcgg       900
   ccccattgca ggagaccccg acccggggca tgggaatctt ctgggcttgg gcaggtgcct       960
   cagccaggaa ggccagggca gcgtcagtga caacgttggc gtgttggcac agcaccaggc      1020
   ccgtttccag cacctgcagg aagttggctg cgtcgatgtc cagcccatag aggtcgcgaa      1080
   gccactcagc caggtcttcc ttcatggcct ccaggtactg ctcactcgac ttgaaaggcc      1140
   ggatactgca cacaggcggc cctagggtcc tgggcttcct cctgcctccc gcaggctggg      1200
   acatggctgg accccagcag ggcaggaggt gggcacctcc cctctcccac tgccgcctct      1260
   ttcctcccgc tgctgctggg tctcggctgg gttcttcctg attctgaggt tcccgtgtac      1320
   tcgctgagag cccgggacct ccagtgctgc tacttgccac tggcttcagc tgccaggcct      1380
   ggcccagccc ctgccccccc acattcctca gtccttgcca gagctttccc tggtggccct      1440
   gcagcaacct tgctctcagg ctgcccagtc cacttggagt tcacccctct gaggccagat      1500
   ggaagtgagg acactcctct ctttccccag agctggagag atcaagctgg ctgcctccta      1560
   ccctctgctg gctgcggcca cagagcctgg ggtggctgcc ggcctcccca ctctgctttc      1620
   ctcccctctc cttggagaca gcggctgcta caaacactcc ttgttaaccc ttcccagcag      1680
   gcgaggactg ccctgcagaa ccaagggaga gggaggagag gttggggctg gggctggctt      1740
   ctctcacggg gctggccaaa tgggaacttt gggggagtgg agggaaagca ggggtagggg      1800
   ttgccatacc ccttggtgtt tagtgagtac aggagaatgg gaatgaaaca gacatagaaa 1860
   tagggcccaa aactcataag tgtagcctta aatcctgtgt gaccatcata tactacctag 1920
   cctctctgtg ccttagtttc tcaactgtaa aatgggggta ataatgttta cctttttggt 1980
   gtgttgtgag ggataaagaa a 2001
<210> 13
   <211> 2001
   <212> DNA
   <213> Homo sapiens
<400> 13
   taagaggaat tgctcttgca ggtggtaaag gaaggcaaaa agctgaggat agtaagagtg 60
   agaggccggg cacagtggct aggctgtaat cccagcactt tgggaggcca aagcaggcag 120
   atcatctgag gtcaggagtt ggagaccagc ctggccaaaa tagtgaaatc ccttctctac 180
   taaaaataca aaaattagcc aggcatggtg gcatgtgcct gtagtcccag ctactcttga 240
   ggctgaggca ggaaaatcac ttgaactcag gaggtggagg ttgaagtgag ccgagagcat 300
   gccaccgcac tccagcctag gcaacagagg gagactccgt ctccaaaaaa acaaataaat 360
   aaaaataaaa acaaaaatac aaaaaatagc caggtgtggt ggtacaagcc tgtagtccca 420
   gctactcttg aggctgaggc aggagagttg cttgaacccg ggaggcagag gttgcagtaa 480
   gccaagatga tgccactgca ctccagcctg ggtgacagag agagactcca tctcaaaaaa 540
   aaaaaaaaag tgagaaccac actcacaata atattcataa tgagaactgg catttatggg 600
   gcacggactg tgcaccaggc actgtgctga atgctttctg cgcatgattt tgtttgacac 660
   tcacaacatc tgtgtgaggt ggggcctgtc actctcccag ttagatgatg acgctgaagt 720
   tcagagagct ttgctggcac tgcttgagag gaagggccaa ccccggcttt gaggcctgag 780
   tcctcttaac caccccactg tgttagggac agggttcccc tatgattgca gaaggtctga 840
   gcagaaaaag ccctccgata tcatgtaccc aaggctctag gtcttcatgg ggaaactgaa 900
   gatcagaaag gggcagtggc ctgcacaaag tcacaggtta gagtttggag gacacaggtg 960
   gaacagcact tgccaggcgc tgcagtgtgg gtggtgacat cgtcagggcc cgctgggctc 1020
   acagggcctc caggcaaccg ggggcctttg ggtccccagt tacacactgt ggtcacatca 1080
   gccaggctga cctgagggtc actgcaggac cagcctctgc tgagggctgg ctcacctgca 1140
   tggagggggt tgctcccacc ccaagcatga gtctgaccca cgcaccagct cactctcccc 1200
   tcaggaccac cgacttgccc agtcactgaa tggagaccac ctgctgtggt caccttgcca 1260
   ggcccttctc tgtagacaca ggagaccgat cttgaggagc cagaggcaga gcccagctgg 1320
   gagccggggt ttcactggtc agtcggtcag tctctgcagc tgcagcctga gatcagccaa 1380
   gcccctcctg aggctcctac ctggctcact ttgccctttc tccacagggg tctgatctct 1440
   ctgctctgct tttccaaact atttctcttt ctctctctcg ctctctggtt ctctctctct 1500
   ctctctctct ctctctctct ctctctctca tcagatggct gactggaggc agggtcccag     1560
   cccaaggatg gggttggggt ggaggtggcg aacctgggtt ggtccccact ggatgctggt     1620
   cctcactctc atggcagacg gctttctttg aggccaggac tgggtgatgg tgtcgctacc     1680
   cccgccgcag tctgacgtca cgctgccggg ccccaccaga ctggagggcg agcgccaagg     1740
   ggacctcatg caggcaccgg gcctcccagg ctcccctgcc ccacagagtg taagtacccg     1800
   gcatctgggc ctgggtttag gccaaggcct gcggctgccc aggcaacaag aagaccagtc     1860
   accttaccgc ccccagggac ctcccagggc ctgtcttgtc ccctccacca gcaaacaagt     1920
   cctcagggac cccgagatcc agaggggtca ctcctcatcc cttcagtctg agcctgtccc     1980
   agggctgtga gccccatcag a                                               2001
<210> 14
   <211> 2001
   <212> DNA
   <213> Homo sapiens
<400> 14
   aggggtaaca gcagactagg gaggcctggg gtcctccaca cgcctgtgtg gggtgacttt      60
   ggcccccagc aacaaggcag gcctgaggtc tgcagaggga ggcagcccac tcctcagccg     120
   ggggcgtcta tcctcctcct ggccccatcc ctcacctgtg ggggaggcta agtgggtgtc     180
   agggccctgt cagagggtga gccacccccg ggggtgccca tcggccacca gcctcccttc     240
   agtagacacc cagcccaagt gacagcagtg gcatggcact gctgagcaca gggagcctca     300
   aggtcagtgc ctggctcctg gtatcctccg tggtccccag gacaccccca cacagaggtc     360
   ccagaacccc caaagctggc acaagagccc aggaacaccc tgcccagcac cctccaggtc     420
   cctcttttag gatctgggtg agctaaagaa gccaaaagaa aggctgggcg cggtggctca     480
   cgcttgtaat cctagcactt tgggaggccg aggtgggcgg attgcctgag ctcaggagtt     540
   ggagaccagc ctgggcaaca cattgaaacc ccatctctac taaaatacaa aaaaaattag     600
   ccaggtgtgg tggcgtgcac ctgtagtccc agctactcgg gagcctgagg caggggaatt     660
   gcttgaaccc aggaggcaga ggttgcagtg agctgagatt gcatcactgc actccagccc     720
   gggtgacaga gtgagactcc atctcaaaaa aagaaaaaaa aaaaaaaaaa aaaaaaaaaa     780
   agaagccaaa agagggtctt cggcccagcc cgcccctccc caacagccct gggtgtggca     840
   gggagcctga ccagttccct ccagcctgcg tcatggagcc ctgggcgtgg ctgcagggtt     900
   taaagagccg acccacgtgc ccagcagcct cctcagatcc gttctctgcg ctgccagctc     960
   aggtgagccc tcgccaaggt gacctcgcag gtgagcaggc gtccactttg gggctgctgg    1020
   gtgaaggggg tgggtgccca agaattctga gtaatgagga gcctgttgag gggtctgagg    1080
   acagatatgg ctgcagcagc cgcttgaagg cctcctgctt tgcacccact atcatatgga    1140
   tggtgggttg ggggttggag gaaacagaag ccagcccttg gctaaagaat atatgagctg    1200
   gtggggtata cctctggtcc caactgcttg agatactcag gtgaggagat cacctgagcc     1260
   aagagagttc aaggctgcag tgagccgaga tcacgccact gctctccatc ctgggtaaca     1320
   gagcaagacc ctgtctcaaa aaaaaaaaaa atagtaataa taataataat aaaataaaaa     1380
   atgtgaccta gagttgagac cctcgtattc caggaactcc aggttggtga aggtagagag     1440
   accagtgaag gctgggggaa gccagctttg gagactgctc ctagcccaag aaccgatgtt     1500
   cagaagcagt gcctaacagt ctctccctcc agtgcctccc tcccactgca gaaggaccat     1560
   gtgtttattt atggatgtat ttatttattt attttgagat ggagtctccc tctgttgccc     1620
   aggctggagt gcagtgccag gatctcggct cactgcaacc tccaccttcc aggtttaagt     1680
   gattctcctg cctcagcttc ccaagtagct gggaatacag gcgcctgtca ccacactcag     1740
   ctaatttttg tatttttagt atagatgggg ttttgccatg ttggccaggc tgatcttgaa     1800
   ctcctgacct caggtgatcc acccgcctca gcctcccaaa gtgctgggat tataggtgtg     1860
   agccactgcg cccggcctat ggatgtattt attgtaacag gtttttagtg cttactgtgt     1920
   gctaggcata gttcttaggc ttgacaaata gtagttcacg taattccatc gctataacct     1980
   tatgaggttg ctactattct g                                               2001
<210> 15
   <211> 2001
   <212> DNA
   <213> Homo sapiens
<400> 15
<210> 16
   <211> 2001
   <212> DNA
   <213> Homo sapiens
<400> 16
   acgtgaatac acacaaacac acaaacagct tggctgctct gggtgaagca tgtgtgtgtt      60
   tgggggaaat ttccttcccc acccgtggtc gtccatttgc ccatgagcag cttccgaggc     120
   aggtccgtgg accccaacgc tgtgtaacac cgtgtgaaaa acacaaaggc ttgcagccct     180
   ctcactgcag acgtgggaac acgactctgt ttaaaaccct tcaaagcccg ccttcttgcc     240
   gtgtgatgct gcctgggcca gcagggcagg tcaccacgct gtctcttcaa agcagctcgc     300
   tcatgcccac agcgctgggc acaagggcag ccacgagctg tttgatttct gcgtccccag     360
   cagatggctt ggtgcacagg ggcagggctc gtctgtccct ctcactgctg cgtcccaggc     420
   tatgtctcca ccatgtaact tcagacaagt tcctcagcgt ctctgtgtcc tggttccctc     480
   atttgtaaca tcaggatatt gataaggatc atagtaatag ggatgtcctt atggggcctc     540
   atgaaaatga agagttctct gtgatgtgcc tggaaccaca gagggcagca agtgggagcc     600
   acagaaagtc atctctttat catagttgtc attggtaact atgacggtta tcaagggcag 660
   gggtctccca ccttctgcat ccaggccttg tcattcctta taaaattttc tcctttcaag 720
   gactttcaat gccctgaaaa cggaatgctc ctccctgctc cccatcagct gctgcagagc 780
   cctgaacccc ttgaacttgc cttgcttagt tctttcaggg gctgccttgg atgccacctc 840
   acttccctct tcttggctct gtggctgtca gaggggctgg gcactgggtt acttcatcag 900
   ttcctgggtg ttgatcggca ggaaacagtg gtttcctgtt catatcctgc ctgagcctgg 960
   gccgggtgga ggtgtcctgc atggagtctt gtgaaatcgc gtggactgca acacccagca 1020
   gcgcggtgag tccaccctga aatccacagt gacccacgcg gcatgtgctg ggaattgaac 1080
   gcttggggaa agagctgggc tccagcggat cagccccgac caagtcgttc ccacagctca 1140
   ggaccggacg ctacaagccc actttgtagc agtttggcgg acggcaaact tttgcaacac 1200
   agtgcaaaaa ccatttaaca cctttaaggg aattggaaat gggtttttca tgtgtggtgg 1260
   tgctgaaaat tgagaataat gacagaatta ttaaaaacag ccttctcggc cttagactct 1320
   taattgcctc tggtctgtaa aactgacaaa taagcactgt agaagccaca gatgaaatat 1380
   gtcgtcttgg gagccctgga gaattgtctc tgaattcttt tgtttatgga ttgtagaaac 1440
   tgctttgggg gtagttggat gtagaagcag aaggacagag tggggagctg ggagcaggag 1500
   gcctgggttt gtctcctgag tctgtttcca ctggggcctg aggctcccgt gtgagggaac 1560
   atgaatgggc caggcacatg ggccctgtgg ggtgacagag ctgtgggctc catggtggct 1620
   ctgtgggcag ctagcttggg gatggctgag ataaagacaa gtagttttcc ttcctgcagg 1680
   acttctcaga gcctttagtg tggttataat gatagaaaac agtattaagt ccataccctg 1740
   aaccagaaac aatgctaaaa gctttgcact tggccttttt ttttcttttt cttttatcaa 1800
   catgtgtgag gacggaagct taggcttaca gatatggact cattgagagt gagtggacgt 1860
   gtctgatctt taacccagtt ctgtctgacc aaaaagcctg tgctctaacc actgtgcacc 1920
   cctgcctgtc tgtcctgttg agagaactgt agggaaagcc ttgagattat gtatccagtg 1980
   ctcccctcct cctgattgga a 2001
<210> 17
   <211> 2001
   <212> DNA
   <213> Homo sapiens
<400> 17
   tgaccttgct caggtccatc tccgggtgtc agtttcttca tctacaatgc aagagggttg      60
   cccacctctg agaacccttc taaccccaaa tctcacccta tgaatctaag aacaccaacc     120
   cctcgccatc ctaagtatca cagagccagg caagcatggg tgagagctca gaccatcctt     180
   gttggactaa aaggaagggg cagactgccc atggggggca gccgagaggg tcaggccccc      240
   ataggtcctc agcctgcttc aacctcaaag gggatggggg gctgagtggt gccagaggag     300
   cagcaggctc gctcggggag agtagggcct taggatagaa gggaaatgaa ctaaacaacc      360
   agcttcctcc caaaccagtt tcaggccagg gctgggaatt tcacaaaaaa gcagaaggcg      420
   ctctgtgaac atttcctgcc ccgccccagc ccccttcctg gcagcattac cacactgctc      480
   acctgtgaag caatcttccg gagacagggc caaagggcca agtgccccag tcaggagctg      540
   cctataaatg ccgagcctgc acagctctgg caaacactct gtgtggctcc tcggctttgg      600
   taagtgagct gccagcttcc ccaggcagaa gcctgcctgc cgattccttc tttccttccc      660
   tgacccaact tccttccaaa tcctcctcct agaagccctc cttggttggc cctgcctact      720
   ttaaagcttc tttcacattt tcttaggtca tgttcccctg gggcctcctg ccctcaaatg      780
   ctttgctttt tggcactctg tagatattct aaaaaatcat tttgtacatg tgtgtgacag      840
   gccatctccc agttaagttg cagcctgtgc tttcttttta ttttgcactt cccccactat      900
   ttctgtgagt gcttagtagg aagtgtcaaa gaagcttgac agcattttct tctaagtgtc      960
   ccaactcttg gttttccatt acacagacag agtgcaagac gatgacttgc aaaatgtcgc     1020
   agctggaacg caacatagag accatcatca acaccttcca ccaatactct gtgaagctgg     1080
   ggcacccaga caccctgaac cagggggaat tcaaagagct ggtgcgaaaa gatctgcaaa     1140
   attttctcaa ggtagggctg gactctggca ggtctgaccc agcctcaccg cagtttgggt     1200
   tgacaaggga ggatgggagt atgggctaca gcaatcaagg ggaagatttg agctcctgga     1260
   gcccagcccc aagacgcagc gagtgtcctg ttatacaggg caggtgctca cagttacaca     1320
   ggacgacagg gtcaagaaat tgctcaattg aacacctgct atttgtcggg ccctgttctg     1380
   ggcagaggga tgtagtggta aatggggagc ccactattcc agtggaggga gacacacagt     1440
   aaagttgttg gccaataaag agcacagata aagccaaatg ccaataagtg cctggaagaa     1500
   aatgagatag agtgcgctgt gggcaatggg gctgggtggg gtggaggtga ccagttaggg     1560
   tacatgagaa gggcctcttt gaggaggtaa catttgagct gagccccgaa tgttggggag     1620
   ggaagcccct gaggatgaca cttggcacaa agctgaggag accctaagcc tcaggcggga     1680
   acttggggtg gaagacttgg gggcttttct aatcctaagg gtctgcggtg gaaaatgaat     1740
   gcataaagag cacatggaga gcacctgcac agcactcagg gaactgggag gtttttcccc     1800
   cgctccaaaa atgattaggc agttctaaga aaaaggctga gcacttccaa cagccttttt     1860
   gttttctttt caaatttggg gaaagtcggg aaacagaggc ctgcattaag aagggtggaa     1920
   cacatgggtc tcagtctcag ttccagtccc ggagccagac atcctggggt aggtccccag     1980
   ccctcccagt gcccctccct c                                                 2001
<210> 18
   <211> 2001
   <212> DNA
   <213> Homo sapiens
<400> 18
   tccccttttg ctattatttt taagagcctt gctgctctcc aggcaaattc tcaggttctc 60
   tgcccgtctg cagcatgtat gcatatgcgt gtgtgtgtgc gtgcgtgtgt gcgcgtgcgt 120
   gcgtgggcgt gtgcgcgtgt gtgggcgtgc gcgtgtgcgt gtgcgcgtgt gtgcgtgtgc 180
   gcgtacgcgt gtgtgcgtgt atgtgtgtgt tgggggaggc tgcggtgcct atgactggtt 240
   gcccccttga accccaccag aagccagccc aggcccctct gcgccctctt ctgagtctcc 300
   agggccggtt ctctcctggc aactccagaa agtgcctcct cacctcaaaa gaccaagaaa 360
   cagggtttct gataaagtgt cccacccagt cccggctggg gcagttggtt agagaatgag 420
   gggacattgg ggggaagagg agggagaatg aggggatggg aagagaggcg cagagaaaat 480
   gagaggtgga gagaaaggga cagagtgaaa gagaaggaga aaggggagaa gccgggaaga 540
   caatgagaat ggaagcgaga gatggaagtg gaaagaagaa ctggaagcct agagacagag 600
   ggagagaggg agagagggag ggggagagag agagagagag agagagaacg ggaatgagca 660
   gctgtgatgt ggccagatca gttcatagaa aagaaacaca gttcagagca gaagggagga 720
   aagaaaataa gaaggaaaga aacagagaat gagatgcaga gaggagagga gggagagaga 780
   gaaggaaacc gaggtgggta gaaaaatgtg acgaaaggga aggacagaaa aaaaaatgag 840
   agaagtaaga cagaataaag gagaaaaaga agaagggaat catgaggatg gtgagaaaag 900
   acaggagaga aagccgggag agaggataga ggggcgttct cgctgcgctg cgtgtcagct 960
   ctggtaagcc gtctagcatg tcattagtgg ggacctgagg cgccagcggg aaagtcccct 1020
   ccccgtgggc tggaggctga gggagggcgg gccctgggct gggatccaca ggcaggtggc 1080
   cgcccctggg tggggccgcc cggccaagcc ctaggggtat aagtgggagg ccgggcctgc 1140
   tgagcacctg tcaggggcag ctccggggtg caggtgccag gcaggtggct ccggccagcc 1200
   cagccccagc atgagctcct tcgacctgcc ggcgccctcc ccacctcgct gcagccccca 1260
   gttccccagc atcggccagg agccccccga gatgaacctc tactatgaga acttcttcca 1320
   cccacagggc gtgcccagcc ctcagcggcc ctccttcgag gggggcggcg agtatggggc 1380
   cacccccaac ccctacctct ggttcaacgg gcccaccatg accccgccac cctacctgcc 1440
   cggccccaac gccagcccct tcctgcccca ggcctatgga gtgcagaggc cgctgctgcc 1500
   cagcgtgtcg gggcttgggg ggagcgacct gggctggctg cccatcccct cgcaggagga 1560
   gctgatgaag ctggtgcggc caccctattc ctactcggct ctcatcgcca tggccatcca 1620
   cggggcaccc gacaagcgcc tcactctcag ccagatctac cagtacgtgg ccgacaactt 1680
   ccccttctac aacaagagca aggccggctg gcagaactcc atccgccaca acctgtcgct 1740
   caacgactgc ttcaagaagg tgccccgcga cgaggacgac ccgggtaagg aggctttgag 1800
   tgtggggggt gtccccaagg aagactcact tccttcctcc ccaagacccc attctagaaa 1860
   gttctgacta gggctgtgcc ccccaagact gggggatgct accaggcagg acagagggga 1920
   gctcagcagt gaaggagagc atgggcctgg gggaagattt gggtttcagc cctggctgtg 1980
   ctcctgccca gtcagtgacc t 2001
<210> 19
   <211> 2001
   <212> DNA
   <213> Homo sapiens
<400> 19
   gtaagattca ggggctgcta tggatgcatt acccaggaga taagggcaac ctcaggtggg 60
   ccggggccct cacagcaacc ttacgagagg ggttgtatca tgcccatttg atggaagaga 120
   aaactaagat tccattagaa ttgatgtgac gtatcctaaa tcacacagct gcagcaggac 180
   taggatttga atgcgggtca atctgcctcc aacactactg ccttttcctc catgctggat 240
   taggctattg gcttctgaaa gcacagacag gtgagatggg tcaaacatgg cctgaagcac 300
   ccaggcacag gtgggagatg gcaagcagag gcaggttcaa aacgtggggc caggactggt 360
   ctgaaagata gtcgttaaat agcactgagg agagggagtg agagagatgt gggtcttggg 420
   ggagtggggt cttgagaacc ctgaaatcag cagtgtctgc ccactttgcc ctggaccagc 480
   ctctctgtct gtatccttcc cccagagaaa ggaccatggg ctgacctggg ccaaggacac 540
   ctggctggag ccccttctct gacattaact agctccgtga cctggggaaa gccaccctcc 600
   ctcctgagcc tcacttttcc tgtctgtaaa atggcctccc cgtcatcaca cccacacagg 660
   tgtcttgagg agtgaatgat ggtgtgctcc aaggccccca tggagagccc ggcacatggc 720
   aggcactcgg tgttcaagca ctgtgttccc atagactctg ggctcctgcc ctcctcgccc 780
   ccaaccccct tcctaggaac cagccccggc ttgtcagaga gccagctagc tgtcaacccc 840
   accccagctg gccagttaat gagtgaccag gactcctgag cctgacctat aaggaggtgc 900
   taggcaggga cacagatggg agacggtgga cagcggcagg gggaacccag ggagcgcgat 960
   gggctgcagg gctgcatcag ggctcctgcc aggagtggcc gtggtcctcc tgctgctgct 1020
   gcagagcaca cagtcagtct acatccaggt gagtcccttg gccagcgttc cctttgcctg 1080
   aagggcccca tggtgggagg ctaggctgga gagggtgtac tgggaattca gaggggcacc 1140
   gggggagcac ggggcccggg gctcagccca acacccatca cagcccagag accaaggcag 1200
   ctggtgggtg ggggcagcca gatgttgatg gcagtgatga tggcaatgac aacaccaaac 1260
   tgattcagcg ctgctcccct caaaggcccg tgctggtcac agaggtcagg gtgtggaaca 1320
   gaaggctcct ccttgcatgg gaggaggcca gaaagggaag gagacaccct gttatgctcc 1380
   cacagttccg tatattcacc cacatgtagg tggaggagga caggctccag aaaaattttc 1440
   ctgagaagta acccttgagc tggcattgca agatgaacag gaggttgcca gacacagatg 1500
   atggggaaac tgttccaggc caaggggcac agcatgtgca aagaccagat agaagactat 1560
   gggggttcca gtgcccttcc agggggcatg ggagagtggc agtagtcgtc acagcagggc 1620
   ctgtctgcca cgtcacaggg gatgctgctg gcctcgccca gcatagcaga gacgtctgtg 1680
   gccctgggtg tcacatccct gactctgggc atggtctcct tgggaagaac aaagtgggtc 1740
   ccagagaggg gtgcaggaga cttggcctcc ccaacagccc tggcctcaga gaccagctac 1800
   ttggggctgc catcactctg ccatgtgagc tcaggcaggc gtactcagtt agtgattgtg 1860
   ccctggtacc aggcccccac gggagatgtc agatgaggga actgaggctg agtggggaac 1920
   agacaggcag ccggtcatgg gcagaggtgg gatttgaacc agggcttgtc tgtctctaaa 1980
   gcctcgaacc actgtgctcc a 2001
<210> 20
   <211> 2001
   <212> DNA
   <213> Homo sapiens
<400> 20
   ctatgctaca aaggcagtag gccaaatttg attgaattga ataattcctt attttcatca      60
   gcttctcctt tttttttttt tttttttttt tttgagatgg agtattgctg tgtcacccag     120
   gctggagtgc agtggtgtaa tcttggctca ctgcagcctc cacctcccag gttcaagtga     180
   ttctcttgcc tcagcctccc gagtagctgg gattaaaagt acccaccatc acgcccggtt     240
   aatttttgtg tttttagtac agatggggtt ttgccatgtg ggccaggatg gtctcgaact     300
   cttgacctca attgatctgc ccccctcagc ttcccaacgt gctgggatta caggtgtgag     360
   ccaccgcacc cagccagcct ctcagttttg aacatgcact accaccacct ccacaacaca     420
   caaatgtaaa tgcactttcg tatataaaac tgtataaata caaggaagct catacacatg     480
   caaggataca cacataagca cccccagatt caaccacaga aatatacgcc agtacatttg     540
   cataaattca aacacccctt tacatgtaaa aatcatataa gcacatacag ggatgcaagc     600
   aggcatggac aaatgcatgc aagcacagac aaacagacaa agctaagtaa aaaagtgcaa     660
   gctcacctat gcttacaaaa atagacatac atatacccac aaacccacac acccacacat     720
   tcacttgctc acctggactt tgatatctct accactgtat ccctgccaat atctacagag     780
   tgggtaaagg gataggcatc aggtcactgg gttgcccaag caggaagtct gggttcccta     840
   acaacttttt ctaagctaat gctcctggat gatgatgaaa aaggaggtgg ggaatggatg     900
   aaattttata acagggtgca gaggcagggt caggataaaa ggcccagttg gaggctgcag     960
   cagggtgcag ggcagtcaga ccaggaccat ggaactcagc gtcctcctct tccttgcact    1020
   cctcacagga ctcttgctac tcctggttca gcgccaccct aacacccatg accgcctccc    1080
   accagggccc cgccctctgc cccttttggg aaaccttctg cagatggata gaagaggcct    1140
   actcaaatcc tttctgaggg taagacacag acgaatgggg tctgagggtg agctgcttct    1200
   tgccttggta cttggggaag cttcaccaaa cagaatgagg cagacttcca gagtcagggg    1260
   tggcacgggc atggttggtg agtacggagc atggtgaagc atgatgggtg gtattattag    1320
   gagaaaagca tcaaattaaa tttagcagag tttatttgag caaagaagtg actcatgagt    1380
   tggacagctc cctaaaccag gaaagacacc acacggcagt atggtcaagt ggtatttaca 1440
   ggcagaaaaa ggaggtgaca tacagaaaca gcctgattgg ccacagatca cagcttgcct 1500
   tacttggtca caatctgagc agtttgcagc ctgtgtggac tgaaagccca gctgctctga 1560
   ttagccaaca cttggctact tgtcacaaga atatattcat ttgggccagg tgcagtggct 1620
   catgcctgta atcccagtgc ttttggaggc cgaggtggtg gatcacctga ggtcaggagt 1680
   tcgagaccag cctggccaac atggtgaaac cttgtctcta ctaaaaatac aaatattagc 1740
   tgggcatagt gatgcgtgcc tgtaatctca gctacccagg aggctgagga aggagaatca 1800
   cttgaatcca ggaggcagag gttgcagtga gccaaaatct taccactgca ctccatcctg 1860
   ggtgacagag tgagactcct tctcaaaaaa aaaaaaaaaa aaaagaatat actcccaagt 1920
   taggttgcag ttcactctac agagagagct ttaggtcaaa tttaatttaa ttaaacaatt 1980
   ctcccctttt ggtcagcctc a 2001
<210> 21
   <211> 2001
   <212> DNA
   <213> Homo sapiens
<400> 21
   gtggcgtgca cctgtaatcc caactactca ggaggctgag gcaggagaat cacttgaacc        60
   cgggaaatgg aggttgcagt gagccgagat cgtgccacag tactacagcc taggcgacag       120
   agtgagactc catctcaaaa taaataaata aataaataaa taagtctcat gaccttgagt       180
   ggggtctcgt acccctatag atttcagtct tctcatctgc aaatcgagga agacagaatc       240
   atccacctct catttgctgc caggcctgag ggtgtatgat gagtccactc agatccacac       300
   attttagtgt gttccctccc taccagtatt tgcattttca ctgaagacaa agtcttaagg       360
   tgaagcaata aacatcaaat tggggaatgg tcagcctctt cccacaaaag ggcaaggtat       420
   aaaaagaggg gatgaggagg ctgtcctctt cacctgctaa ctccatgtat gagtggtggt       480
   cacaagagag gaagcgggca gcagaaatcc tcagctccca cagaaatgaa tacattttcc       540
   aaaataaagt caagccaagc agccctaccc cacagaaagt cctagcaaac agaggtggct       600
   tcctacctga agcaccggcc aggtgtgtcc aacagtagga gctgcagtca gagatcagag       660
   tgacttaaca gctagagggc acttgatgaa taaacggaaa taaggaaacc acatcagatg       720
   acacctccct tctgagcccc acccatgtct acatttgggg aagaacagtt taagtcaagg       780
   gatcacagac ttgtgattag agactgccag ggtccatatg accaagtggg ggtcccaggt       840
   gtgaagctgg ggttgagggt ccattatctg aattctccac tctatggatg atcattttta       900
   ttattttcct tttcttgaat ttatttccat ttgtttattc ctaaattccc tagtatatca       960
   cctgtggaag cttgcaactt cctgaccaag tataaagggc gaaggatttg acttacagca      1020
   gagacttcag aaggagtccc ccataggagg gacttggggg caatccagtg ggaaagagga      1080
   ggaggactgc gcttgagttg tgtttgaaca gcaggcactc aatctctact tattttgcag 1140
   gcttctttga ggtggcctag ctcggattca aggcattctc atcaaggatg tgagagcagt 1200
   tactgggata tccttggctt ccctgttggt ttgccaacga gagctcccct tcctcctttg 1260
   cctccaacct ggtcttcttt ggaggttggc ttcccagtgt tctccacctt ccacaactcc 1320
   aaggcctggc ctcaatctct acttattttt caggcttttt gagggtattt gaatggaaca 1380
   caaaccctcc tttgccgcca gccactcttt cgcacaatca ctgccaatga ccagttggtt 1440
   taaatggccc tgaggctgac ggcataggcc actcccagag tctggctggt tagcagagag 1500
   accacagagc tggggcagtc catgaatgga agagctgtgg tcaaggtcac tgcagccccc 1560
   atctcagtaa cggagaatgg cttctctggg aacccacctt agctgcctcc agcagctaat 1620
   ggctgtcccc tagcttgagt cttctggcac cttcgtcact tctccatata acatgaaccc 1680
   tgctgtgagg catttacaag tcagccctct tctagcttgg gagccatgag acagcaagaa 1740
   ccttcttcaa ttcatgttta tgttctcttt cagggaatat agtaggtgct caatcaatgg 1800
   ttgtggaata aatgaaggga gacatttctc ctgtgattca tggaccataa gtcaccacca 1860
   cgattcagtg tctattgctt ttggctacac actaaagtca ctggtatgcc tgatagcttc 1920
   acttccacac agcccagttg gtctgcctgg cattgcttct ttccaacctt cctctaatgt 1980
   ttggagctaa tgccagtctg t 2001
<210> 22
   <211> 2001
   <212> DNA
   <213> Homo sapiens
<400> 22
   tccctcagaa actgaagcat gttgcccagc cagttgctga tgccctcctt ctcctggctg       60
   tggctcacgg agagcatctt gtgaaagaga cgctggagtc ggcccacttc tagccactcc      120
   cggggccaca gcagggagta gacaaacctt gggaaaagaa ggtcaaactt gcggaactcc      180
   atgaataact ctcctgcctg tagcaggtcc tgctccttgt ccttcgtgta gccgaacaag      240
   ctcaggtagc cagctgtgaa caagatgtaa tagcagaagc gaaagaggct gtcctcatgc      300
   cagcaactgg catccagact ccagcctttg gacgtcagca ttacaaagga caggctgtcc      360
   agcatggtct cattaagatc cttcaagcca tcccccctca gatgcttggt gctggctgag      420
   tgtatcatct catggtcccc ttgcactgaa cggtatccaa ataccttcag caccagtttt      480
   tttgcatatt gcccaaagtc tagttttctc tgtgtgtcct tgaggatgga gccaaaggag      540
   agggggtcca tgacgaaggt gaagtactgg ccccctagct gcactgtgaa cacatcccca      600
   tgcttggtcc tcatgcgctt cagaaattca aacatattct tccggaaagc catggcatgg      660
   ccaagccagg gcacggtacc cttgtccaga gggggctccc atggcctgcg ttgtcggagc      720
   atccctggca ggcacaggta tccagcaatg accaccagca gagctcccag cactggaccc      780
   cagagaacca tggctatgct cctgcggatt aagctctcga cacgcacact gttccctggg 840
   tgccagagag ctttgggagg ccttggaaag tccaggatgt aaaggtctga gcacctggac 900
   cttgccctgt ggccgcaagt tatggcaaat aacactagtg agacagtact tggaagcagc 960
   aaaatgttcc cctggactta acacttggct gactggtcac gtaggggagg aggaggtaac 1020
   caggagaaag ctcagagaga agcttccagc tatggtcaca gctgctcacc agcttcatgc 1080
   tttgctctga ccttttcaga cagctcttgc accttctctc taagcctgcc ttgagcccac 1140
   tcccaccccc atagctgaca gactatttat acccccattt actcatgctt ttatttattt 1200
   gccagttcag ttggtagaaa tgaatcaagc acttacaaaa tctcacttcc actctttcct 1260
   catctttaga agactcaaca aatgcctcca ttcctacttg gcccccttgg tcaagtaagt 1320
   taacctcact gtggctccac tccctcatct gtgaagtagg gataattaca gtgccttgta 1380
   ggtgctgttg tgaggcaaaa agagataaca cctgaaaagc acttagaata gtgcctggct 1440
   catgctagtg ctccatatat gttagttagt atcctaaagc cttttctctt acctttgcct 1500
   gctcccctgg acctcacagc agcccgtgct tatctcctca ttctttccct agtctgtctt 1560
   cctctctggt ctcaaagatg cttgccaagg gggcatgacc ttgttgaagt catgtctgct 1620
   cctacctcca tgtgcacaga gtacacagtc aaaggcaagt gaagaatgac tgtatgccct 1680
   tccacaacag ctctgacccc taacccaaaa aaagaggcat tgatgctggg gctttagatg 1740
   ctgagaaggc tgagatggtg gagggggtgt tgactccatc ttggggtatg agtgtgaatg 1800
   aactcatctg aggaggccac agagcctccc agatgatgac tcttcaagca gaggctgagc 1860
   cctcagtctc tgagcctctc acccggagcc ctctcacctg cccaactgct tgcctataca 1920
   cttctctgac aagaaggcag aatcctggaa ttgtaaaatg tcagagacaa tagagacctc 1980
   actcaatcac ttcatacttc a 2001
<210> 23
   <211> 2001
   <212> DNA
   <213> Homo sapiens
<400> 23
   agcctcccaa agtgctggga ttacaggcat gagccaccgc gcctggccta tatacatgtt      60
   taaaaaataa agacagggcc agcacggtgg ctcatgcctg taatcccagc actttgggag     120
   gctgaggcag gcagatcacc tgtggtcagg agttcaagac tagcctggcc aacacggtga     180
   aaccctgtct ctactaaaaa tacaaaaatt agcctggagt ggtggtgcac acctgtagtc     240
   ccagctactc aggaggctaa ggcaggagga tcccttgaac ccagaaggtg gaggttgcag     300
   agagccgaga tcacaccact atgctccagc ctgggggaca gagagagatt ccctctcaaa     360
   caaaacaaaa aaaataaaaa taaaaataaa taaataaata aagtatgtag agttttattt     420
   aataattaac cacacagatt aaaaaataga aagtagccag cttcccagaa gtcttctgtg     480
   tgcaccttct aattaccatc tctgtcatgt gtcttcagac agaagcacat ataaaaacaa      540
   ggttatgtat tgatcaaatg atgaaaatgt gtggtcagag gcttgcattt tccctaggag      600
   caatggctca gtatttgcta atccggtgtt cacagtgact ttacagaaca taactacccc      660
   caagtaatgg gaatcaactg tatttttatt actttggtgt acaaagaact ttgagctctc      720
   catggcttgc cacaacaaaa gacttcctag gcaaattgtc tactgctttg ttccaagtgg      780
   gggctctact tgaggctgtg ggaccaagac aaaagaagca gcctctatgt ggattaggcc      840
   ccttctcctg gcagagagaa agcacaaagg agctggcaga aactcacagt gcaaaactca      900
   ctatgcatat gctacatctg ctcatactcc aataggcaaa gcacagctga cagcaaaccc      960
   aaagtcaatg agacagaaat gtgtcttcct tccacgggcc gttctaaaat tgcaaggtaa     1020
   tggacaggag tgtcctctta aaggaaagat ggaatgaata gttgtgaata ataatataac     1080
   ccatcactac tactcctgtt ttaatttttt gaaaatgttt agatacatat tatatgcttg     1140
   tttaatttaa aatatcaaac tttaccttgc tcaggctctg cccctccaca ctgggccact     1200
   caaagatgcc caacttaccc tacttgggct ccaacagcca tatcaggttg cattcgagac     1260
   cagcctggcc aacatggtga aaccccgtct ctactaaaaa tgcaaaactt agccgagcgt     1320
   ggtggcgcac acctgtagtc ccagctacta gggaggctaa ggcaggagaa tcgctggatg     1380
   cattgatgcc ctcttcaccc tgcttaggct ctgattcctg acacaagaca gctctgttct     1440
   gtggaacccc tcctcactat gcttggccca actccccaca gccgaccact ctaacttcta     1500
   tgtatcctcc tcatctcctt gggctcagac cctccatgtc tggctaccca cccccataga     1560
   caccctcctc accctccttc agttcggaca gtccacatca ggcctcttcc ctatggagac     1620
   agcttcctca ctttgctcca gctctaaggc tcctccctca tctgctcccc tgcctggaca     1680
   ctctgctcac cccactcagg ctctcattct atgccagtct gcccctctag ccccaggggg     1740
   atgcctttct caacatgggc aggctggtct ccatgctatg ctgcttcttg cctcttcact     1800
   gggcattctc atctacccct ggctccagca caccaggcca gcagccctcc tacagagaca     1860
   ccctccttga cctgttccag ctctgatgcc cacacggagc cagccgtggc tcccttcccc     1920
   ttggggcaaa cgcctacctt gctaaatgtg cctatctaat ggctttagga ctgactagtt     1980
   cagttagtaa agaaaaggag a                                               2001
<210> 24
   <211> 2001
   <212> DNA
   <213> Homo sapiens
<400> 24
   tgcaggtggc cagccaccca gtgctgacta ggtgggaggc ttccaactac ctggctatct      60
   aaatgcccgc tgcttcacca gctgctggct accttcctgc ttagggcagt gtgactcact     120
   gacggattga caaggaggcc tggcaggacc catcacccag ttccatccct ctaatgctgt     180
   gtccccaggc tcactcctct ctacccagtt ccccgacctg cttacttggg tttggggtgt      240
   caacaggatc caggcatttg atgccacaag tgtcaggaca acatctcttc ttccctggac      300
   actgccagtc actctggcac tcaggtttct tgtatctaag gcactgggca gatttcttag      360
   gaggacagac tccagctttg aaggcttttg aagagaaagt caagaggtca ggaggaggct      420
   gggtactgag gacccatcat gcctcctgaa caaagcacca cttttgaggg ggagagaccc      480
   ttacctcagt agctgtccac aaagcccaaa gggtcatgcc tgcctgatct ccaggtgtat      540
   tgatgggctt gattaaatta gacaaatgga caggggacgt ttcacaccca agaaaagggc      600
   agcaggcttc tagtcaagac ctcccaccca agagcaaaac aaggctacac aaaggcagag      660
   aggaaggctg ggagtgatgg agatggagag aggtaaacaa agataaatag aaccagtgtg      720
   agagaagttg agagctaaag cccaaggcag aagcacagag acaaaatagc agaagcaaag      780
   acacaaggaa gcagagtcag aaactaatga tttgagagtc gcagacaggt gaagaagaag      840
   gcggagtatg agagagagag agagagagag agagagagag agagagtggg acctgggctg      900
   acagcatggc tgcatagagg aagcatttcc agaagctcaa actgtcacac aaaccttgga      960
   gctcagccag acatgtattc caccctcagg gaggtctccc gaaactaagc tgagggatca     1020
   gagcctacag aaggggacac acccacacca caaggagacc ccacctctga ccctgcagcc     1080
   cagattagac cagagtgact ccaacttaca ctttccagag ccttccacag cccaaggtgc     1140
   cagagttccc agggcaagca gcaccaggaa ggggaagagg ccgctggact tcatggtgaa     1200
   ggcaggagtg actctgatgg ccaatgccaa acctcactat ttattctttc accagtgggt     1260
   gtggccccac cagaattctt tcggcctctc ccagctacta ttgcataagc aggaaacgta     1320
   gccagagacc agaaatcctg acaccaagga gattgtttat ctttcctatg ccaggtaagg     1380
   ctatgaggcc aacacagcaa ctatcagaaa agaaagggaa gagattggcc tgggaagttg     1440
   gttgattaaa gaggccatct cagtgtccat ctgagcccta aacatcacaa gatcccaggc     1500
   cattagcctt ataggtggag agagggaaga agtggtcaat tgcaaaggtg ctacccggac     1560
   ctggaactaa aaccagacac agactccctg ctgagacttt gtgcaataca ttctattctc     1620
   acattgctaa ttctgattct cacattactg gactctcagg cattgtgaaa ctctgctgat     1680
   tactcaatgc agtttctttc tcaaaagttc agctcgttca accaactgat acttagttcc     1740
   ctgaaatatt tacaccgtta tatccccagt gcctgttctg tgcctgacac aggaaaagca     1800
   ctcacaaatg aaagctgaag aagagctccg ctctccacaa atctctctgg agctcagttg     1860
   tctcagctgt gaaatgaaat gatactaaca agtcagccct tctcagaggg ttatgaaatt     1920
   aagggcatga agggactctg aatgaaaaga acaaatgaat agtatccctt cccactaaag     1980
   agaattgaaa tgaatctgag a                                               2001
<210> 25
   <211> 2001
   <212> DNA
   <213> Homo sapiens
<400> 25
   gagatctggg aaatagttta atcagacact agcatttatt cttagctgct ttcttaatta 60
   aataaaaatg agtataaatt aatttactaa atgttcacaa cgtaaaaacg gacattaaaa 120
   aaagaaaaaa aatgccaagg taagctgata atgtttttaa aaagtagaca ataaaataat 180
   gtatgtaact taaaatttgc catgataatt tattcttaaa atggaaaatc agtaaaatac 240
   tcagacttct agcataatat ctcttttttt ttatttatta ttatttaagt tttaaggaca 300
   tggatgaaat tggaaatcat cattctcagt aaactatcgc aagaacaaaa aaccaaacac 360
   cgcatattct cactcatagg tgggaattga acaatgagaa cacatggaca caggaagggg 420
   aacatcacac tctggggact gttgtggggt gggggagggg gggagggata gcattgggag 480
   atatacctaa tgctagatga cgagttagtg ggtgcagcgc accagcatgg cacatgaata 540
   catttttaaa agtgtgttac tactgtcatt tcaactgact agaaatacca ccagcacgtg 600
   attgccacct caaaggactg agaatgtaat agtagtctat gaagagcagt gtgtgtgatg 660
   gacaatgatg gggtagctcc agtgactgat ggcttcaggt gccactgacg ggtaattgtt 720
   gatgtattca actgattttc cgtgagcacc cactttgtgc cagacagtaa ggactcggag 780
   ataaaaagca gccctctgct ggatacgttt acaggctagt ggaggtgaca ggcaccgtca 840
   gagaagattg taacaagata tgtatggcat agtaagagag aagagatcat ctaattctgc 900
   cactgagtat ttaagggcag gtttcacaga agagtttaca tctgaattga atcttaaagg 960
   atgacaggga gttcactgga tagacaaagt agaggaggac gtaagaggca aaggacacat 1020
   catgtgtaaa ggcatgaaag ctgagccagc ctattatctt ggaaaaacta catgtactgt 1080
   agttactaca actgtcacaa aggctattag tggagcagca gaggaaggtt aaataacaga 1140
   gggaagttgg atcatgaaga accttatcca tactacaagg tgattttatc cagtaggcat 1200
   tgaaaaatgt aatcgaacct gtgttttaat ggaatattga ctaggatgag gggaaaagac 1260
   agaggataac aaaacactta gtagacaaaa gcaacatttg tttctatctc ctactacttc 1320
   agtcataatt ataataataa tagcccacat tattgatact gacatgtaga taggtgaatc 1380
   caatataaaa tgataggagg tatgaaagaa gttggtgagt ttggaatatt atttgactgg 1440
   ctataatata gagtaatggg gtgggagcca gagggaaatc gaaaaggtga cctggaaaac 1500
   attgttcagg gccttgaatg ctatgttgag gtattttgac tttataaaat agtgggggac 1560
   aaactacaag tattttatac attgaggtat agtttatgaa gtttttttat atgtatagct 1620
   tatgtaaatc tcaaaggaat gctgagaagt aggcgttatt atcccattgt acaggtgaga 1680
   aagctgagtt tgtggaatat gcatacattc tacattattg agcacctgct atttgccaag 1740
   cactatgctg agctatgaga ctagaaggaa gttgtagttt aatactctaa tagaggaaac 1800
   aggaataaat cagcaattgt aagtagttgc tataataaag gcaaacaatg ttacgggagt 1860
   gaaaggggga cttgtctata tttgcatagt cagggaaggc ttcctggagg aggtgagctt 1920
   aaactgagtt tggagaaaca aatagattac agtaattgcc cagagatatt taactaggaa 1980
   gcagtttacc tgaaacttga a 2001
<210> 26
   <211> 2001
   <212> DNA
   <213> Homo sapiens
<400> 26
   aataggtgtc agccagtttg cagcaacctg tcttgcccct accattcttg ggttctgtat      60
   tcagacccca gctattccta acacccagtt catactccac gttctgtaca aagccatccc     120
   tagctatccc agtcagccca cactggtctc catgacatgc atgaaccgaa gtttttagaa     180
   cctttgacac ttatgtctgc cctggatgat ttgttcatta tcttatgttg ttgtcatcca     240
   cagaacaatc tggagaatat tcatatattt agtcagcaaa tagctattta ttgggggcct     300
   ttgtaggcct caatgttgag aagaagggtt ggttgttttg gacagattgt gtatttataa     360
   agctctagca atttttaaat ctctagcagg aaccaaagcc ttcttgacag ccacctctct     420
   gaattattgt cactagcctc cttaggagaa gagacaatat agtttatacc aagatttgca     480
   gttgtctaga tattactcac ttcagctagc actgtgctag tagataacat tcaaagttgg     540
   tctcaacaaa tattttctct agtgcccatg gagagtggct gagaaataca ctctaagatg     600
   taaaagagtt tggtatctag atcctctttc ttattcattt caagccaata tttattaagc     660
   accaactgca agctagatac tattatagtt aggaatataa agtgggccag ggatggtgtt     720
   tatgcctata atcccagcac tttgggaggc caaggcagga ggattacttg aggccaggaa     780
   ttcaaggtca gcctgcccaa cacagcaaga cctcgtctct acaaaaaatt aaaaaattag     840
   ctgggtgtgg tggcatttgc ctgtagccct agctactcaa gaggctgagg tgggaggatt     900
   gcttgagccc aggggttgga ggctgcagtg cgctatgatg gtgccattga aaacaaaaac     960
   aaaaaccaac caacaaacaa aaaaacaaac aaaaagaaaa cgatactcag tctttatagg    1020
   gaggttggcc agtcaatagg ttactttatg agttgctaac cctggtgagc aggaagttat    1080
   gtggaccagg agagaaaccc ttggttcagc ctggagaaag gagaggttga ccctaaactg    1140
   gagggtggag aggaccctgt tgtgactctc cgactgactt gtcttccttg atgtccttta    1200
   agccggagct gattcgggct gctgccttat ttctgagtta gcgctcttaa gattgggcct    1260
   cccagtttga ggaaggggcg ggctgctgtc tacctctgtg aatctgccct ggaccacccc    1320
   gggagagaag gagggctccg gggaatctcg cacattccag gcaaaggctc ccgggccgca    1380
   gcctctgtgc cacacccttg gcccgggcca ggtgtgcgcc ctcctcgctg cgagggggag    1440
   cgggcggctg cggggagcga ttttccagcc cggtttgtgc tctgtgtgtt tgtctgcctc    1500
   tggagggctg ggtcctcctt attcacaggt gagtcacacc ctgaaacaca ggctctcttc      1560
   ctgtcaggac tgagtcaggt agaagagtcg ataaaaccac ctgatcaagg aaaaggaagg      1620
   cacagcggag cgcagagtga gaaccaccaa ccgaggcgcc gggcagcgac ccctgcagcg      1680
   gagacagaga ctgagcggcc cggccccgcc atgcctgcgc tctggctggg ctgctgcctc      1740
   tgcttctcgc tcctcctgcc cgcagcccgg gccacctcca ggagggaagg tgagtcggct      1800
   tccacaagga aacatctcag ccctgggctg agaatctgcc tttctctgca gaccgtgatg      1860
   gctgaacgta cctccgagga ttcccacttt gtccagaaac ttgttagagc tcccttctcc      1920
   tcccctatct ggggctcctc cagagacgga ttttaaatga gactaatata taaagctatc      1980
   tgctttttaa aaatagtaag a                                                2001
<210> 27
   <211> 2001
   <212> DNA
   <213> Homo sapiens
<400> 27
   tcaggtgcag cgtcacagca gggggcctgc gttctgcttc ccagtctggg agaagagagt 60
   gtctggatat tttcaggaaa aaaaaagaaa gttgagcagc cacagcaggg cagggcagtc 120
   tacatcctca agtctccctc agcttctctg ctgctgccgg ctggcctcaa attttgcctc 180
   tctttggttc tctgagaagt tttaccaaga gaagagacag gaggagagat ccagcctgga 240
   ctattgtttg tggggtgtgt aggtgtgtgt gtgtgtgtgt gtgtgtgtgt gtgtgtgtgt 300
   gtgtgtatgc gtgtatgtat cccgcgaggc tggagccaca gcttccctga tgaaccatgg 360
   aggcgggcca gctcctgagc tgcggttgcc ctggagaccc acctaagcct ctttgcctgg 420
   agcagcatta tctgtagagg gagtggctga tctgacacaa aagcctgagg cgacccaggc 480
   ccaggcagtg agggagcact tgtgtccaga tggcattctg tccgcttcct cctccagccg 540
   gtgctcagtg tggcacaaac tgggcaagac cattctcacc caaccctctg ctgagcccta 600
   ggtgtacctg ctgttggcaa atgtttccag ctgcctggtg ggtggctcct ggatggagat 660
   cccctctttc tatcagctcc ctcctacagg gaaggggtga ttctgggtgc tgaggacagg 720
   gacctgtgaa gatacatctc cgcctttgcc ccaccacatc ccttgcctgt cttcctctct 780
   gcttatctaa attatttcct tccctcaagg caagctcaag agcagacagt tctgactttc 840
   agaaggaggg gctgtccagg gagaagagga acatggaaca gaaagcattg acgttgaggt 900
   atttgagata acatttgggg atagcagaag catcctgttg ctgttctgca gcatcccaca 960
   ctcatgtctg ggcctccttc ctgtctctgc tctctggtca cgctgtcact tgtagactgt 1020
   tctgcctaga agccatgtgt gggccagctt gcacctaaca gacattaggt agcactaaaa 1080
   cagatgcaca ctgccctgtg aggggcatgg gaatactgta atgggcatga agctgcatta 1140
   taatagatct taggtaatcc tgtgctgggt gatagcgtga cttttgcaca catctggttt 1200
   tgattgtgtt ggaaataaag cagaaagtac aaccaagaat aaattaaaca atggctgatg 1260
   aaatggactc aggttgccca cctgcctgcc tgcatgattt cgtccctggg tgatttgtca 1320
   cttcgtgctt gctctgtggc tggttccact cagctggttc catccgcctt ccctcttcat 1380
   ccatcaagca ggcaagattc ttgagtcagg agttggatca tatacttctc ttgaccagtt 1440
   cacagcacga cagccccaaa gattgagtgg gtcacttggg gatggaagat ccccacattt 1500
   gagggagctg aagctgactt cctctgaccc acagttccca ggcggcctcc acctcagggc 1560
   cagctaaggc cagagtcacc caaagcaggt aactgggagt ccccacctct gagcacccca 1620
   ccactatcct ggcactttcc caaggggcgt ggcttggagg tccaagggca gaaattccca 1680
   ggcatttcca cactgtaggg aagggagcag ggggatgaca agactttacc ttccctttag 1740
   gaaagttgct ggaggcaatg aggagaggag ggcatggact aagtcacatg ctcatctgtg 1800
   cagcacgtat ttctaaagca ccattctaca ggggcaccaa gctaggagct ggctgagcct 1860
   ggctctcctc tctgcacaca tcccagccaa gcctgtgtgt gctggattgc tggatcagat 1920
   gaggaggagg tggaagtctg gcaggcaggt ggggagcagg gacagcatta gggcccagag 1980
   ttaaagactt ttatctccaa g 2001
<210> 28
   <211> 2001
   <212> DNA
   <213> Homo sapiens
<400> 28
   acttgggaga cagagggagg aggcttgctc gagcccagga gttcgtggct atagtgagct      60
   atgatcatgc cactgcactc cagcctggac aacagagcaa gaccctgtct ctaaaaaaga     120
   aaagaaattt ggaaatggtt tattttgtat taacaattta taatttacac tgaaatttat     180
   tatgataaaa cttttccctg tgttaaaaag ctattaactt tatgaaaaat ttcttttagg     240
   taaggttgat tatatatacc cacacacata cacaggttaa aagttagttt catgtgacat     300
   aataactagc attttgagca ctacctgttt gcccagcact gttctaagtg ctctacatgt     360
   attattgtta aattatcata acactatgaa ttatgtacta taattacccc agctttacag     420
   atgaggagac taatccatgg ggaggttaag taacttgtcc aaggccagac agctagagcc     480
   ggcttttgga cccacaccac agtctgactc cagcacccat attcttaaca atttcaccat     540
   attaatatgt caagattaag cagttttaaa ggatgctatt ttctcacaaa tttcttaata     600
   tgaacactca ataagaataa tcactaatat aagcatttag tattttttta acactaagtt     660
   ggaagcatag tggaacattt atttttagaa atattattaa ttggctgggc tcacgcttgt     720
   aatcggctgg gctcatgcct gtaaattttg ggaggccaag gtaaaagaat tgcttgagcc     780
   cagtatttcc agaccagcat gggcaataca ttaagacatc atctttaaaa aaaaaatgtt     840
   attaatctcc tctttttgtt aaatgtatat tatcaaaatt gttactaagc taacaaactt     900
   cagaaaaact tatgatgggc aagctgcttg tgacattgaa ggtatttaag attcaattct      960
   agtttggtcc tagatgacca catatccatt gttccttcaa cgagcacatg gtaaagagcc     1020
   tagaacacag agacacagaa cacagtggag aaaagggagt gaaatgtctt taatgacact     1080
   tactatatat gggattttgt gacaatatac aaggatggtt aagacatata aggtgatgca     1140
   aaaaaacata ttaacaatta tagtgacaaa aaatgaggag catataatta tacattgatt     1200
   tatacagagt accagaggaa cacagcattg agagccgtaa caccacctga gggagtggag     1260
   aaaggcttca gagagaaagt gttttttgga atggatcact gtttccaaaa gaactaaagt     1320
   acagtttgag aaatgcatac ttaattcatt acttttttcc cctcaacttt aataataaat     1380
   ttacccaaca aaaaagttta tttttgactt gtaaatctct taaaatcata aaaaagtaaa     1440
   attagctttt aaaaacaggt agtcaccata gcattgaatg tgtagtttat aatacagcaa     1500
   agttaaatac aatttcaaat tacctattaa gttagttgct catttctttg atttcattta     1560
   gcattgatct aactcaatgt ggaagaaggt tacattcgtg caagttaaca cggcttaatg     1620
   attaactatg ttcacctacc aaccttacct tttctgggca aatattggta tatatagagt     1680
   taagaagtct aggtctgctt ccagaagaaa acagttccac gttgcttgaa attgaaaatc     1740
   aagataaaaa tgttcacaat taagctcctt ctttttattg ttcctctagt tatttcctcc     1800
   agaattgatc aagacaattc atcatttgat tctctatctc cagagccaaa atcaagattt     1860
   gctatgttag acgatgtaaa aattttagcc aatggcctcc ttcagttggg acatggtctt     1920
   aaagactttg tccataagac gaagggccaa attaatgaca tatttcaaaa actcaacata     1980
   tttgatcagt ctttttatga t                                              2001
<210> 29
   <211> 2001
   <212> DNA
   <213> Homo sapiens
<400> 29
   tttcatattc catttctctg tatgctttct tcaccaggaa gaaatagttt tagtgtcagg 60
   aatgaatgag tctgcccctc aattccagcc tgctcagcac acaaggaaac aaagccctga 120
   caatcagagt gactccctgg tgactaagct ccagtcctgg atgcatattt gtttagcagt 180
   tctgacagca tctgacccag ccctctcttt gcatacccca ccagaacctt cttttttttt 240
   ttttttcttt gagactgagt cttgctctgt cggaagcgat tcccgtgcct cagcctccca 300
   aatacctgga attataggcg taagccatca tgcctggcta atttttgtat ttttcatgga 360
   gatggggttt tgccatgttg gtcaaattgg tctcacactc ctgacctcat gtgatccacc 420
   tgcctcagcc tcccaaagtg ctgggatgac aggtgtaagc caccatgcta ggctcagaaa 480
   tttcctttta taaaaatgtc attaaggatc ttggctgcac aatatcgtta ccagcttcct 540
   ttaaatccac ctctggcctg ccaggaatca gggttcttca gaacctgaca ttttaaatga 600
   agaggtcagg caggtcatga ggaaagcctc attgtcccca tgtctctgtc actgctgcac      660
   ccctgagaca tcacagacat ggacactggg gcctgcttgt ttctcaaact gcccttagat      720
   cgaaagaggg aggaaccagg atgaatgcca ctcattttcc caagaaaggc cctctcctga      780
   gtgcccggga tggggctctg tccattgcct ggggccgcca attgctactc tgggttacgg      840
   aagaaggaca gggtcctgag agacaccaga gacctcacac agccctgaaa acatggggct      900
   ccttcataag tgtttcccat caccaacagg gagaccacgt ggaggccttg cagccctact      960
   cggtgcttct ccaccaaatc ccaagggcag tgacgctgac gtctgtggaa agcagagaaa     1020
   gccctggctc ccaaagccct gaagtcctgt ggagctgaca ttccctgagt gacggtgtga     1080
   atggaaggaa ctcaagtgcg ggtggtaggc cacctcctgg cccaggcctg ggtgaactct     1140
   gaggggacac atgtagtcac aatcccatcc tcccattctc cttctcagag gaaggaagtg     1200
   ggcatccatc tgcctcatct ctctcccgtg gggaagatgg ggagtttcag gggaactttc     1260
   acataaattt caccagctca gatctcctgt gaggatgggg cccaccatgc tcccggtgct     1320
   gccagaggcc ctgagcccct ccagggtccc tgggtttgag ccagccctgt atcatcccca     1380
   ggagctgaat gtccgagcaa tggatagaat tagatggaaa gagctctcaa tttggcctga     1440
   gactgtcccc agatactcag gaaaaacagg acgtcgcaca gagtgggcag caggtgagtg     1500
   gcaggttata ggtcctgagt ttgagtttgt tctcacgtga gacagaccca gcccctcact     1560
   ccattcacac actgggtttt aaatggtgca agataggagg aattttctgg tcccaagagc     1620
   aggaggaagg gattttctgg ggtttcctga gtccagattt gcataagatc tcctgagtgt     1680
   gcattgttct ttgaggacca ttctctgact caccaggtaa gtggctgaat tctaacctct     1740
   gtaatgagca ttgcacccaa taccagttct gaactctacc tggtgaccag ggaccaggac     1800
   ctttataagg tggaaggctt gatgtcctcc ccagactcag ctcctggtga agctcccagc     1860
   catcagccat gagggtcttg tatctcctct tctcgttcct cttcatattc ctgatgcctc     1920
   ttccaggtga gatgggccag ggaaatagga gggttggcca aatggaagaa tggcgtagaa     1980
   gttctctgtc tcctctcatt c                                              2001
<210> 30
   <211> 2001
   <212> DNA
   <213> Homo sapiens
<400> 30
   gaaaggaaag cctggctccc gctccaactg caaaaggaga aagataaagg atggggtgga      60
   aaggctggga ctggaaaaat gtggagaggg ccaacaaggg gcatctggag acgggggaag     120
   aagggggtta aggagagggg cggagagagg ccaagaggct gagaggttag ggcttgaaag     180
   agggcgccgg cgagcaggag aaagcccaag aaggagtcgg aggttgggga aggaggagat     240
   aaaggaaaaa ccccgggatg gagtgaagac ggcggcagca agcaactcac cagagcccag     300
   ggagaggcag ggctggggac ggcggagagc cgcagacgcc ccttcggcca gtagtctcga 360
   cttgaaggct ggcgaagccc cggcggcggc agccaacgag ccaaagacgg gaggggacgc 420
   gtgagggact ctgagacagg gggcaacagg cggttacggg tgaggacaac aggaagcgtt 480
   ccaactgccg gaaaagacgc gagcgctgag gggccaaggc agcccggtcc cccgccgcgg 540
   agacgggctg gcgcggcagc ccggggccga gactacgcat gcgcgacttc ccggagcggc 600
   cgggattgcg gaactgcgct tgcgtggccc cgccctgccc ctcccttcac cccctgccta 660
   gggctggctg gacctgggtg aggccagatg tgtgagtgtg aggcggtctc gaagtaggct 720
   gggctgaggg ggaagttcca ttgttttctt ttgttttact ttttacttcg aaataattat 780
   ggactcacaa gaagttgcaa aattaataca tacaggttcc ctataccatc acccacccag 840
   cttacctcaa catttttctt aagatacaac attctcaaag ccaggaaatt gacattcgca 900
   caatacaatt aactaaacca atacaattaa ctaaaccaat acaattaact tcattacaga 960
   ccacactcgg atttaacctg ttgggtatgc agtcatgtgc gtgtgtgtga gtgtgtgtgt 1020
   agttctatga aatgttgtca cctgtatagt tcccacccac ccccctaccc cgtgtagaac 1080
   aggtctgctg gagcccttgg cccccctcct cttccttaaa gtaaggacct tacctgggat 1140
   aaggtgagct ctcataggtt tgggaggtta cccagcgggc gacccagtgt ggacagaaga 1200
   aggtgctttt caaggccacc caactcttat tctgtactac ttttgggggt aaagaggtcc 1260
   caagtttaat agcctttggg cacttgtcgt ttcttgagaa tggagcacaa agccctcaac 1320
   tacaaagtgt caagctcctg gaaactaaga tgaacgagac atggtccctg actgcctgca 1380
   aaggggccat ctagggagag aaggaagcat gtgctgcgag ttattcattc atttcatctt 1440
   caaagcccag tttccttttg tctagcctct taacaaaaat cacttctcgc caggcgcggt 1500
   ggctcctgcc tgtaattcca gcactttggg aggtccaggt gggaggatag cttgagccta 1560
   ggagtttgag accagtctgg gcaacataag gagaatccac ctctaaaaaa atttttgtaa 1620
   ttggccagca tggtggcaag tgcctgtggt cccacctact gtggaggctg aagcaggagg 1680
   attgattgag tccaggagtt cagggctgct atgagccatg atcatgacac tgcaccccag 1740
   cctgggcaac ggagggagac cctgtctcaa tcagtcacta catacatatc aatcaatcac 1800
   ttcgcctttc attctctctg cccagaggcc cttctcccca aatgcctgtt gtcaaagtcc 1860
   tacccattct taagctttta ataatagtcc aagatgtctg cataatgctt tacagtttac 1920
   aaacaacttt cacttattta atctcacttg gtcctcactt tggcagtgac acacgaggtt 1980
   ttgcagatga agtaattgat a 2001
<210> 31
   <211> 2001
   <212> DNA
   <213> Homo sapiens
<400> 31
   gccgagatgg tgccactgca ctccagcctg ggcgacagag cgagactctg tctcaaaaac 60
   aaacaaacaa aaaaaacaaa acaaacaaac aaacaaacaa aaaaccttcc ttggattttt 120
   caagaagttt tcacaattgc aaaaaaacaa aacaaaacaa cacaacacaa caaaacaaaa 180
   caaaagcaaa aaaaccctat gcttccaccc aaggtaaaaa ttttagcttt aggtccactc 240
   tcaatacatt atttaatgaa ctgaagttgg caaatattcc tcacagcctg ttggagggtt 300
   cagcagttta ttacagaagt atgaaatgct tttatttaaa aaatgtattt tggtaaatac 360
   atttttgttt aggtaaatat catcaaataa tccaatttgg aaaccaacat tcttacttct 420
   ttttccaaca gttgttccta tcatcataaa aacatgttaa atttttctca tcctttcaaa 480
   aaatctctgg agcttacctc accctccaga tacagcctca cctctcattc caccatgaaa 540
   tcagacttct tgagatggtt tcagctggac tccatccttc aactgacaac ccaacacaac 600
   tgtattcatc tctcgttaac attactagca gtgagtaaca tcagaaagtt tttgacacat 660
   gttagtcttt tttgtgatga acttcacaga tacatttgac attggtatgc ctcatttatt 720
   tgttgaaatt tttttctttg gcttccatga agtttctttc tcttctgtat cattctactt 780
   ctatgactgc tccttctcga gtaaaacaga atgtgtctca ggattacttt aaaacaagac 840
   aaagtataga gttataccta aaatttagta tttaagttat tggatcagaa aggaaactcg 900
   catttagagt atgaaggcat tgtcagccac caattacttt tgtaacctga agctagtctc 960
   cttcctactc cggactgaat ttcttctgta taatgcaagc gatctggcat gatgatatac 1020
   aaagaccgat aaaattttgc tggggattct gaaagtaaaa aaaattgcct ttgatattat 1080
   gtccccatgc taagtccctg gggactttga cattatcccc cactgagcag gggtgaggaa 1140
   gctggcattt actaataagc tatgaaaggg cagtgccttt tgacatttca gctccaccca 1200
   tagcacaccc actcaaggaa catataaatg aagagatccg ctcagttctc agacaagatt 1260
   tttcaagcaa gatgaagtcc atcatcctct ttgtcctttc cctgctcctt atcttggaga 1320
   agcaagcagc tgtgatggga caaaaaggtg agtggagagg gtaagccttg gggaaagcta 1380
   ctttaaaaaa atggcctcta aggatattca gggtgcaaac agtaacctgt tcaggcacag 1440
   attcttctcc ttgatgagaa ttgatttttc tccacccaac gctgtaggct tttggaaata 1500
   tcagaaattt gttgggaaaa ggtgggaggt aagagttgca agagagcttt ggagataatg 1560
   aatgcataca tttctattat caattaccag gtggatcaaa aggccaattg ccaagcggat 1620
   cttcccaatt tccacatgga caaaagggcc agcactattt tggacaaaaa gaccaacaac 1680
   atactaaatc caaaggcagt ttttctattc aacacacata tcatgtagac atcaatgatc 1740
   atgactggac ccgaaaaagt cagcaatatg atttgaatgc cctacataag gcgacaaaat 1800
   caaaacaaca cctaggtgga agtcaacaac tgctcaatta taaacaagaa ggcagagacc 1860
   atgataaatc aaaaggtcat tttcacatga tagttataca tcataaagga ggccaagctc 1920
   atcatgggac acaaaatcct tctcaagatc aggggaatag cccatctgga aagggattat 1980
   ccagtcaatg ttcaaacaca g 2001
<210> 32
   <211> 2001
   <212> DNA
   <213> Homo sapiens
<400> 32
   ctgacagtgt cttcctgaat gactcaagtg gtcaaaaacg caagattatt tttcattttt 60
   ttacagagaa ataaaataca attcatggcc tttactgaag tttactgatt tgtgattcat 120
   aaaacaatta gcgtatcatt agattttatt aagagctata atttagtgtt ctaggaaatt 180
   tagaaggccg aattatactt tgaaatgcac tggcaggcat actaaggaag tcctgaggtg 240
   aatcattttt tataaaccta catttttata tattacatta gagccctata aatatatgta 300
   tacacttttt tttttttttt ttttttcaga cagagtctcc ctgtcaccca ggctggagtg 360
   ctgtggtgcg atctcagctc accgcaacct ctgcctcctg ggttcaagag attctctgcc 420
   tcagcctccc aagtagctgg gattacaggt gggtgccatg acgcctagct aacttttgta 480
   tttttagtag agacagggtt ttaccatgtt ggccaggctg gtctcgaact cctgacctca 540
   ggtgatctgc ctgcctcgat gtatatactc ttttatactt acttttgact agagacaatt 600
   gagacttgaa aattagaaaa atcaagttag aattactgag aaaattagta gctctgatat 660
   tctacccatt ctttattttt aaattaaaat taatcttaat gacctacaaa taggttaact 720
   taaacagtcc ataccttaca tgagaagctc cgacttggga tgtttcttgc taactctgat 780
   atcttgggtt tattctgaag acacttggtt aggtgatatt ttttcaattt tattctattg 840
   taacaatgat cagcgaagtt atagtgattc aaatgctgtt tctgcatgca ttttccaagg 900
   tgaggaatac tatacttcaa agcttggaga aataatttgg tcttctgctg catgatatca 960
   aagtccctgg tttatgctcc ttttggggct gccagcacac gaatgatttt ataggtatga 1020
   ttacagagta gtgggttctc agagtaacta agatcagcag ttctcatccc tccttttcag 1080
   ggccatgccc cacttaggtt atgaataact tcagacagct gataacttag gctaactaaa 1140
   tctaaaaacc tgtgtaagtt aaagtttcat tcaaatcata attcttggca gatgtgactt 1200
   aaatgtctca tttgtaaagt taatcctatc aggctcaaac tatgcagcac tgactctggc 1260
   acacataggg gttaaactaa aacaaaaaac aaaacaagaa acaaaatata aatttgcaaa 1320
   taagtacaca gacttaatgg ggtatctcag gaactcaggg ctgtaaaagg taaaaagccc 1380
   tttggtctta cataattatg aacgtatctt tggtatatat gtggaagata aactggaaaa 1440
   gcctaattat ttaataaagg tgggggaggg aagattaatt attgttgagt gttaactaac 1500
   tgccaggtgc ttttatcttg tttgtctaat ttaaatttta tatggactta ataaagtaga 1560
   tattaccatg cacattttac aggttgccaa actgtggctg tgggaggttc taagcaactg 1620
   ttccaggtca cttgtacagt aagctgtata gcaaaaaatc caatttggtt cggttccaaa 1680
   tactggacat actcaacttc ggatttttaa tgttaaaaag gtatttagcg ctttaattaa     1740
   aaacaacaaa aaacccctgc aagatgcaca aataaaatgt aaggccatct ctggtttgtc     1800
   aggaggtagc ttttattaac cttgattgct aataagtagt taagagtcag agagacctgg     1860
   gttgaggtct tagctcctac acttaactgt gtctccgggc ctcagtttct tcatctgcaa     1920
   aaatgaaatg acaccattta ccttctaggg ttgttgttgt aaagacaaaa tgagacagct     1980
   taacattaag accatacagt g                                               2001
<210> 33
   <211> 2001
   <212> DNA
   <213> Homo sapiens
<400> 33
   ccccagtttc cttggttaaa atgtatatgt acataatccc taaagtgctg gccaagctcc      60
   caccatgaga taagaatttc aggccaacct gactctataa tggatggcca caggaaagaa     120
   catgctagtt gtctgtgatg ccaaggactg cacacactaa caccacctgg gcacacacta     180
   acaccacctg ggcaaaagcc attgggtgag gagctgcatt tttttttctt tgagacagag     240
   tctcgctatg tcacccagga tggagtgcag tggcgtgatc ttggctcatt gcaacctccg     300
   cctcccaggt tcaagccatt ctcctgcctc agcctcccga gtagctggga ttacaggcaa     360
   ctgacccatg cccagctaat ttttgtattt ttagtagaga cagggtttca agttagtcag     420
   gctggtctca aacccctgac ctcaagtgat ccacccacct cagcctctca aagtgctggg     480
   attacaggcg tgagccacca ggctcgacct aatttctgta tttttagtag agaccgggtt     540
   tcgctatgtt ggtcaggctg gtctcgaact cctgaactca tgtgacccgc ccgccttggc     600
   ctcctaaagt gctgggatta caggcatgag ccaccacgcc tggccaggag ctgcattttt     660
   atgccattat aacttttcta gttatttttt tctttttctc caccagacag gagtcaaaat     720
   taaagtcatc tctgtttttt aagaagtaat tccaaaaaga ccccaatcaa acctaattcc     780
   ttaggattct gttttctgaa atcctaggca tgacagtcct taaaccagaa tgattgctgc     840
   tactcaccat ggctgggcca tgcagggtct tcactgtcat gttgcgctgg tggcttactt     900
   tgtgcaggaa gctctttctg tgaatgtatc ttcctgtgta tgcctgtgtt ctatcaatga     960
   gagtacctgt tagttttata gcaatcattg cccctgtttc gattgacctg cttactgtct    1020
   ttaagttgtg gttgacccgg agttactgac gaatgcaggg gttttttttt ttattccatt    1080
   tcccgaaggg gaacaaaagg gggaccctaa agaaagggat gacaggaaaa ggaccaaatt    1140
   tttctttcct tctaccctac ttccaccccc attgcccaca aatcagagca aaatttttaa    1200
   tttactacag agatgaacca agttagaaat tgttgtcaaa tcttttttgt tcttccacaa    1260
   attgccaaca cccttgtcac ggaccatatg ttctactttc cagtcacgca atatgaatcg    1320
   tgatttaaat gggagaaaac tcatccttag aggagccttg gtactaaact atctttacct    1380
   ccattttatg aatgggaaaa caaaactaaa gaaatgaatg acataagtga aaaattcaga 1440
   aatgatatac cctggtagtt ttgttatctc cctgaactaa ttatttttta accccatggt 1500
   tagactccta ctggtgctta ataaataatc actattcatt gtgttgataa aacaaactgt 1560
   tttcctctgc tctcacacca caaccagcaa cacaaaagat ttccatgacc atgtatgtgg 1620
   gcatttatct ccatcaacca gcaagcaatc aattctgcat ccaacgccag ctaagcatct 1680
   tctaatccaa ttcaattctg acgctatctc caggtaggta gagtcagatc ccacaggctg 1740
   aaggctcagt cccacaaaac tgctcccact ttaagcacca atcacaggtc caagcctctg 1800
   ggacttctga ctgactggcc tcaagttcag gttcccacaa accgcttgag tttgattagg 1860
   cttgattaat ttgttagaac agctcacaaa acttagggaa cactaattta tatttaccag 1920
   tatattctaa aggatatttt taaggataca agtcaacagc cagatgaaaa ggtacacagg 1980
   gcaaggtttg aaagggtcac a 2001
<210> 34
   <211> 2001
   <212> DNA
   <213> Homo sapiens
<400> 34
   ccaccaactg gggccgggcg cggtggttca tgcctgtaat accagcactt tgggaggccg      60
   aggcgggcag atcacgaggt caggagatcg agaccatcct ggctaacacg gcgaaacccc     120
   gtctctacta aaaatacaaa aaattagccg ggtgcggtgg cgggcgcctg tagtcccggc     180
   tactcgggaa gctgaggcag gagaatggca tgaacctagg aagcggagct tgcagtgagc     240
   caagatagcg ccactgcact ccaatctggg caacagagag agactccgtc tcaaaaaaat     300
   aaataaccaa ttggacaagt tatctgactt ttaagaactt tcatctataa aatgtcaata     360
   ctagctacct tatatggtga ttgtgaaagt tgggtaagta gacagtgtga aagtacccag     420
   catagtaagg attcagttaa catttatgga aaataatcca gatacttagg aacttttatg     480
   aaataacaca atgtatttaa actgttttct agtctgccat ttgctttaca attataccgt     540
   tgactggtat ggtgtttccc agtgtctcat ctctctttta ggttactgct tgccttcctt     600
   gtttcactga attggtatgt ctgacctaat gaagactttt tagattattt agaaactact     660
   acgttttctg cagtcttacc tgtttcagag cgtggtggag aatccaaaca ttcaaaaaca     720
   tgccatcgag gtgtctgccc tatcaatgag gaaaaaggca tctggcaatt tggacagtat     780
   ccatcataaa ctggacgtat ctttggggac acgtgttggc ttttttgagt tctacagagt     840
   tttcctggag tagtttcctt gtcttgggtc tgctgaatac catctccaca acttgaattc     900
   tgactagaag caacagaagt ctgacaacct gcatttccaa ggggcacttc atggtccttc     960
   acctctttag cttctgcggc tctttttctg tttctactcc gttttgactg gtattttcca    1020
   tctgttgctt tttcaacaga ttttagaata tttttagagc cattatttgg atcaactcgt    1080
   tttggttttc ttttagattt gtattcccaa atgtcttctt cggaaatgtc ttctaacatg 1140
   gcaaaatgat tttatcattc aagaagtatt aatcttaagt aaactgaaag tccatttctt 1200
   gtcacaaaca aaaagttata gaattatttt gctgagaaaa aaaacaaagg taacaaaacc 1260
   cccaccaact caatgggaat ctgcagtgtt ggtaatgaac atattggcaa gctacaaacc 1320
   ttgtacctga caacacctgc tattccattt ataccacaat gaaactaaga taaacctatc 1380
   acaggagtag caactgtgaa gttctccatt atgggtgcta tttcattcaa atatcttgtc 1440
   cttttccagg gcttgattca taaaaataat acttcaaaaa ttaagcgcta atggcaaata 1500
   taggttcaac agcttaatct tagaacgaga tttgtaacat tgttatgaag ctactgtcag 1560
   ttctttcaag tggacaaccg tccaaataat tcataaatcc aaccacagtc cctggacaaa 1620
   gggcttaaca ggtatcccaa acactaaatg actatctaac aaaactagtc acaacataat 1680
   caaagagatg tgtaattcgc aaaaaactgc gaagtcactg ccctcatcca cgactagtgg 1740
   atactcaacc acttcagttt cccgcgacag tccatgatct tcctccagca gcaatgaaca 1800
   atccctgcac agtgaacaaa aaaagttgat atgtgaaaaa agttcaaata tgtgaaagag 1860
   tgggcttgta ttgaggtctg actccgtatt gcacccatgg ttccgaaggt ggccagccct 1920
   ctgtccacct gactcaaaac ggggtcacac gccagcctct cccttatctt ttggcattag 1980
   aggctgggag gccaggaagc g 2001
<210> 35
   <211> 2001
   <212> DNA
   <213> Homo sapiens
<400> 35
   gtgatccgcc cgccttggcc tcccaaagtg ttgggattac aggcgtgagc catcccaccc      60
   ggcaatcgtc aggactttgg cttggactgt gagtgagata gggagttgca ggaagctttt     120
   tcgaaaaacc acctttgggc caggtgcagt ggctcccacc tataatccca gcactttggg     180
   aggccaaagc aggcagatca cttaaggtca ggaatttgag accaacctgg ccaacatgtt     240
   gaaaccccat ctctaccaaa aaaatacaaa aattagccag gcatggtggt gcatgcctat     300
   attccccgct actcgggagg ttgaggtggg aggatcgctt gaacccggga ggtggaggtt     360
   gcagtgagcc aagattgcgc cactgcactc cagcctgggc gacagagcaa gagcctgtct     420
   ccaaaaacaa acaaaaaaac ccaaaaaact tcttttggca gtgtacaatt attattacta     480
   ttttttgtag taaatttaca gagatctgca gtcatgacca caatccaaat ttagaacaat     540
   tctatgactt caaagggatg ccttagagcc gttccctgga accactcctc tgttctctct     600
   gtatctactg gagggttcta agcagaaaag aaacaaagtc tgacttatat tattatcact     660
   attgcacctt ttctgttaaa acatcataat cccagcactt tgggaggccg aggcgggcgg     720
   atcacgaggt caggagatcg agaccgtcct ggctaacacg gtgaaacccc gtctctacta     780
   aaaaatacaa aaaattagcc gggcatggtg gtgggcgcct gtagtcccag ctactcggga      840
   gcctgaggca ggagaatggc gcaaacccgg gaggtagagc ttggggtgag ccaagatcgc      900
   gccactgcac tccagcctgg gccacagagg gagactccgt ctcaaaacaa acaaacaaac      960
   aaaaaaaaaa acccgtcata atcttcctgt tgctttttcc gattagatgg cgacttgaat     1020
   ttttagggaa tacatccacc agtttgggca cacccaccag gcacgacagt tgaggtctga     1080
   tcttccagcc actgtgagta ctgagaaaac ttggcagggg gccactctca ctggctcaat     1140
   gcaccctgac cactggaagc cactgtgtca gctgctgggt tctggctctg cccactcttg     1200
   gctgcatttg gggctgaagt tccctgtggg aggctgtttt ctgagggagc tgagtgttta     1260
   cagccactca gccctgctct gctcagctga agcagaaaac agagaccttt tgcattactt     1320
   tggttcaaga gcaaggtaat ttggaaagaa atttttcaga cgaaggtatc agctgcctct     1380
   gaggcctttg ctgattaaat caagaagtga catctgaaat ggtgtcgggg aatgggaagg     1440
   aggcctgggg attctaaatt atcagctgtt taggtggctg aggaatccag gggtgggaga      1500
   ctgaggaaaa ggactggggc agagctcagt tcaggagagg tgaggggcgg gagaggtgag     1560
   gggcgggaga ggtgaggggc gggagaggtg aggggcggga gaggtgggtc agagaaagga     1620
   agtctctgag gctgcaggcc taggcctggt ggccactgca gaggctgggg cctatgtttt     1680
   tccaggaagt gaaacctgaa agtcactttt cccctttaag cctcattttc tttatcttta      1740
   aatggagaaa ataacattta cagcatagag ttgttataaa gatttcatga aataatgcct     1800
   ctaagcactc ttaaaatgct tggtacacag taggccttca atagactgaa gctgttatgg     1860
   tttttcttgt tgtgccacgt tattattgtt ggttgttatt accatatttt gttattatta     1920
   ttactaagag gaatttaatg caaggaaact gaaactgagg tcacacgcag ctcccgggcc     1980
   agtgcctctc aaactttaac a                                               2001
<210> 36
   <211> 2001
   <212> DNA
   <213> Homo sapiens
<400> 36
   ggggtgtcca tttttttggc ttccctggtc acattggaag aagaacaatt gtcttgggcc 60
   acacatacat aaactacact aacactaatg atagctgatg ggcaaacaaa caaaacaaaa 120
   aatgctaaaa atctcctaat attttagtta agtttacaaa tttgtgttgg gccacattca 180
   aaactgtcct gggctgaggg ttggacaagc tgctttaagt catttgtaga ttacttataa 240
   tacctaacac aatgtaaacg caatgtaagt ggttgtcatg ttgtattttc tttgtattat 300
   tttactgttg tattattttt tattgttagt ttttgttttg ttttgtagta ttttgatccg 360
   ctgttggttg actctgcaga tgcagaatgc tcggataagg aagccaactg tactcccgca 420
   taccagctac ttgcttactc tctatttatt atctgttatt ctctatgcct tcattctagc 480
   atatgcttca ttctagcata agtaatcacc tttctacatt ctggagtcag gacaatatct 540
   agatcattgc tcaagagttc tgaactggga gattaaaaag ctgggccagc tgggcacagt 600
   ggctcacgcc cgtaatccca gcaccttggg aagtagaggc gggtggatca cctgaggtca 660
   ggagttagag accagcctga ccaacatgga gaaaccccgt ctctactgaa aatacaaaat 720
   tagccgggag tggtggcgca tgcctgtaat ctcagctact cgggaggctg aggcaggaga 780
   atcacttgaa cccgggaggc agaggctgca gtaagccgag atcgtgccat tgcactctaa 840
   cctgggcaac aagaacgaaa cccgaaaaaa aaaaaaaagg ctggggaggt gattgtgggg 900
   aaagacaggt ctcccaaaat gccctctgtg tgtagttgtt cacggaaacc acataaacag 960
   aaaatagcta caacagctaa ttcttactaa ggtgtcaagc gttgtgcata acactgccta 1020
   atgtgtgaca ttaaacgttt tcaccaggtc agccacttaa tcttcagaaa actggtggcc 1080
   aactctgagg tttcacattg acgtttaatg gctgttccta aagaagtgcg tgctttttaa 1140
   aaaaatagag gatgggaggt aggttggaaa gggaagataa gcggcaatgg aaaaatcgag 1200
   tgtgattatt gcatttttat caaatatgtc tgtctggatt atgtggttag aatcactgca 1260
   aatttggctt ctataaatac tttaaaactt ttttccaatg ttttcattat cagatccact 1320
   atgcaggctt tgagtgggct gccatttagt tcagatatca aagtatttta caagtaacta 1380
   atagcattta ttatctacac gtggaaaact atagcaaaga aaggtcaatc aaaagttgtg 1440
   ctctaggtca tcagataaaa tgagattaat actgaaaaat tcttccacga agaccagggg 1500
   tccagctaaa tgtgtggtgg tgggaaaaat gtttatctga cgtccctcta aagaaacctc 1560
   agatgggaga aaaatgtccc agccccagga atggacacat ttaggttttt cctttttttt 1620
   tttttttttt ttttttgaga cagggtctcg ctctgttgcc caggctggag tgtagtggcg 1680
   cgatctctgc tcactgcaac ctccgcctcc cgagttcaag cgattatcct gcctcagccc 1740
   cccgagtagc tgggattaca ggcgcgcgcc accaggccca gataatttct ttgtattttt 1800
   tagtagagat ggggtttcgc cctgttggcc aggctggtct cgaactcctg gcgatccgcc 1860
   cgcctcgcgt tcccaaagtg ctgggaccac aggcgtgggc caccgcgccc ggcgcacatt 1920
   tgggtttttc gtggtcagca aaagcacgac caccccaaca cataaatctg gcgaatcata 1980
   cgcaaattcc ctcatgctat a 2001
<210> 37
   <211> 2001
   <212> DNA
   <213> Homo sapiens
<400> 37
   tgtgccagga atttattaat ggagaaaccc cgcctctact aaaaatacaa aattagccgg      60
   gcggtggtac atgcctataa tcccagctac ttgggaggct gaggcaggag aatcacttga     120
   acccaggagg cagaggttgc agtgagccga gactgtgcca ttgcactcca gcttaggcaa     180
   caagagcaaa actccgtctc aaaaaacaaa acacaaacaa gaaagaaaac ccaaaaaaca      240
   aaaaacaaac aaaaacccat agatggtaca aaaagtggaa gaaaaaggaa aaaaaaaccc      300
   atgaaactat ctgcaatgca gtttcctaga atcaaccaca gtaatctttt agcttttctc      360
   tatctaaatg ttatacatat tttatattta tattaagtgc atttatattt atattaaaat      420
   acagatacaa atttaaaaac tctttattat taaattttca aataaaaagc aacttttcaa      480
   ataaaagcag cgagaacctc cttataccca acacctggct tcaactatta gcaacccatg      540
   gtcaatattt cctctatttc ccacttaatg tctctactga attatttaat ttcacccata      600
   aacacttgtg tgcatctcta aaagttaagg actctttttt gaaaaagact ttaatacaat      660
   ttctcatctc aaaaaattag caataattcc tcaacagcat ccagatgcaa tttttcatgc      720
   ttttttcagt tagtttaaaa tcatgagctt ttctccatgt ctctaaaatg taattactaa      780
   tgcttctaat actattccac caggttgagt gtattataaa tgatctgatc accccttgcc      840
   tcccactctt ccatcccctt ggacatattt aggctacaac atccccagga aaatggtttc      900
   cattgtctgc acgtaagcaa acaggcatta ttaacctatt agctgtccac tgctccaccc      960
   cacccacagg tacccaacta gtcctgtgca cctacctggc gtggactgcc aagggaacct     1020
   ctggatctgc cagtcagcca ccctgtcctg ctcagacttt accagagacg agtatttccc     1080
   tttccttccc tctaagagca ttttatttgc agagttagcc aatagtatct tgtttcctgt     1140
   aagggagact atctttagat gctgccacag gcaagctcag atactccagg gtggaggcca     1200
   gctgccaaca ggagggcctg actttataaa cctggaagat ctaaacctca gggaagactg     1260
   cggagctgga gggagagagg tgccacttac cccaacctgg aggtgggggg cagggagcag     1320
   gccctgcagt cagcaaagca cagcacagtc agctctcatt aatggggagc cttcaggaag     1380
   ctgggagagc tcatggggcc ctcagcttcc ctgcccacga gagggagccc agagtactca     1440
   ctggaccaca ctggggcaca cacagctctt ctcctcaaag tgcagtccca ggcactggga     1500
   gcttattaaa gacagcacct caagccctgc cctatatctg ctgaattaga atctgcattt     1560
   taatgaaatt cttagaagag tgatatgcat actaagtttg agaagctttg gtactgaagg     1620
   catctagtga gtaccttgca ccttgtagat gctcggtaaa tatttttttc ctcacctctc      1680
   tgccccaagg catttagtgg ctccctatac ccatcagtca aggctgagga cacagctttc     1740
   acccaggacc tggcctatgc tcctttcaca gtctctcccc ctgccactct tgccctgcag     1800
   tggggagcct ggtgggctcc ggcatccagc catgcttctg ctccctggcc tggccagctt     1860
   ggggcgactg caacgtgaat ctcaactgcc actgcctcat caccttccga tcacttcatt     1920
   acctgcctga cccctggcat atgctatata attctgatcc atccctaact agggcctggc     1980
   ccatggtggc cttcaataca c                                               2001
<210> 38
   <211> 2001
   <212> DNA
   <213> Homo sapiens
<400> 38
   ctcatcctga gtttatttca tcttcttatt gttttcttct gaatttacat cttttcatca      60
   gaacaaaaag aatacaatca gtttttcaga agtttaaaaa attttttcaa atatatagca     120
   aagaatgtca tcatttttat atactctacc aggactcctg aaaccaaaca cttaagaaag     180
   gttatgtctg gaaatttttt caaaatctat atgcatttca gtggtggaga aatagttctg     240
   tctggaagca ggaggctgaa attatgaccc tgaagatttc ttccagcttt tgaacttctt     300
   attctatgac aactcctggt agatacaggt ttcctggtag ggattgagca agaagcatgc     360
   acaattactt tatgagtctt gataaagctg gaatttcagg ttgatggtta gaaggagttc     420
   ggctgggtcg tgagaatgct tgttttcctg gttactggat atgccatggg ctgtatatct     480
   tgtgttgtcc tgtggtaact gaagccactg ttgctgtgga aaatgatggc agtttgaatg     540
   agataattta gagtgaccta tggaaatctg aggctgtctc cttaatgtac aagaccttgt     600
   atgtgtggca ctgttgtatt tgaatggcac acatctcttt agttcccttc aactgaaatg     660
   acaaaaggtg agaaactgaa tcgtaatttg atggcttaat attaaaaaaa aattggcaaa     720
   aatgctaaat aaggaacaat tcagaagaat tgctgatcat ttcaaaaata ttatttgcat     780
   agcatttata ggtagctaaa gaaagagaaa aatggcttaa tattttaaaa ataatttggt     840
   caatttagcg atctggggat tctgcgatcc ccagaacttt gcatccaatt tccaaaaacc     900
   aaaccttaca tctaatgtaa aatgacacaa aaaattctaa atccagtcta attcagtagt     960
   atatagctta ttcccagtga tgtctcacat tcgcaagccc gttacacgtt ggttttacct    1020
   gtcaccaaaa cctttgtatc actcttataa aaaaccacat cactttctat aaaaagtgag    1080
   tggaagaatc taatattgta aaactgcacg tgcctcgcgt gacctgagca gaaaattcct    1140
   agtttgggat tttctcacag gcatacctaa actgacctct gctcctagca ggtaaattgc    1200
   tagaaggaga agcaagtgtc agtccaaaga aaacccttgt tgtgtgattt tttttttctc    1260
   tttaatcgct ttttaggtgg agtacccgct gcattctcta aagtctttgc caccaccaga    1320
   ttaccaacgt tttcaccaat ttgccacctc cctactcctc tcctttccaa aagccctcat    1380
   tggaaagaac tttctctcgt gaccccccta ttccatctag ttccaatcac tcccagcgcc    1440
   ccccatctcc atcggcttcc cctctcccgc ccagaatcca ggttcccaaa ccacttaaga    1500
   gaaaattcac aaccctcgcc cccttcccgg gcgtccggag gaggcgctgg ggtgggggtg    1560
   tctgtggggg acagggtttc ctggggtctc ctgggtcttc ctcgcgcacc cccaggaggt    1620
   gttgcagggg gaggggagcg cgggtgggtg ggaccgggcc gggtggggga ggggtgaggg    1680
   cgaggaggag gaggaggagg aggcggcggc ggaggagtgg tgtgtgtgcg agcgtgtgtg    1740
   gctgggggaa gccattgcct gtttaatagt tgctgttgct gcacttccgc ttctctccca    1800
   gcgagagaga gacacgagtg gccaggccca gccgcagccg cagcagcagc cgccgcggcg      1860
   gcacggagga gccagacaca aagagaggta cggggatccc cccagcggcc cgccgccccc      1920
   cggccgcgcc gccgggcctc ggggacaccc ctcgccgcct cccccgactc gcgccgccgg      1980
   ggccgccggg ccgcagggca c                                                2001
<210> 39
   <211> 2001
   <212> DNA
   <213> Homo sapiens
<400> 39
   aatagccagc gtgaaagaac tgcccacata ctagggcttt tataaccctg ggatttggcc      60
   tctgtgaaaa ctgatgacgg atgtcgtttt cttttttttc ttttctgaga cagagttttg     120
   cttttgttgc ccaggctgga gcgcaatggc gtgatcgccg ctcactgcaa cccctgcctc     180
   ctgggttcaa gcgattctcc tgcctcagcc tcccgagtag ctgggattac aagcatgagc     240
   caccatgccc ggctaatttt gtatttttag tagagatggg gtttctccat gttgatcagg     300
   ctggtctcga acccctaacc tcaagtgatc cacccacctc ggcctcccaa agtgctggga     360
   ttacaggcgt gagccactgc gcctggcctc atagatgtcg ttttcttcat gaataagcta     420
   gagtagagtt cgtttgaaaa tacacacacg caccacacgc acacacacac acgtgtgcac     480
   acacacgcat acaccccccc acatacaagc atacagatgc acacacacac ccatatgcac     540
   acacccacgt gtgcgcacac acacacagat gcagacatac atatgcgcat acacacacca     600
   atacacatac acgcacacac agatgcatgt atacacacac acacccatag gcacacacac     660
   acatgtgcac acacacaagg acaacagcaa cacaaacaac atgagccaga agctaccaca     720
   gtcaggctcc ttgcttctga gcactcactt gctccaggta aatatccacg ctgcctgttg     780
   aggttgtgct gatgtaagca actgccaaga atggatggac agggtgccag ctgataaatg     840
   agggtgaccc tgctgcatcc ggggcttcta tctggtggtc ataatagagg gccatgacgg     900
   aactcaggcc tcctcagcgc tgaaacctgc agggaaaaaa aaatgaccaa gtccaatcag     960
   ttttaaataa aacaaacaat atgactgtac atggaatgac gaaaaggaag catcccaaag    1020
   ccaccatcgg aagaccatcg gcaacttgaa aatgccttgc ggactgagca ggcaacctga    1080
   tgcgaggaga tctccaacag ggagacatgc agaagagaag cagttagacc cggaatcacc    1140
   caggctgtga cagaaacgaa tacaactcat ggccatcaaa ggaggaaaga aagaaaaggc    1200
   ctgcagccta actacacacg caatcctcag taactttcat tccagtcaca ataaggacaa    1260
   gttctatttc tgtagttccc tttaactttt tagaccaagt cttgctctgt cacccaggct    1320
   ggagtgcagt ggtgtgatca cggctcactg cagcctcaaa ctcctgggct caagtgatcc    1380
   tcccacctca gcctctcaag tagctgggac tacagacatg cactaccatg cccagctaat    1440
   ttttaagtaa tttgtgtaga cacaaggttt tactatgttg cccaggctgg tcttgaaccc    1500
   ctggctcagg taatcctcct gccttggcct cccaaagtgc tgggattaca gccgtgggcc      1560
   accactgcct tttaaacaac attttcaaac aaaataaatg caaactgctc cagaaaagag      1620
   tactggtaca gaaaaaaaac cagtactgaa tttccaataa agcataaaat caaagactgt      1680
   cttttacctc tgactcacag gtggtagaag acagaagtct tcctctatta gagagtcaag      1740
   aatgcacagc cgcagcagct gtgagaagtt gagcttgatc acttacgtga acttaatctc      1800
   tctctttcat tcttcctttc agatctggaa aagatataca catcgtccct tttcaggcgg      1860
   gttagggtga taaataaagt gacggtctgg ttactgctca cttttttaca cctcagtcaa      1920
   tttggggaag ttaactggca gagaagaaca tacttgtagc cgggtttgcc accatttaaa      1980
   attatacagg ctcatgcctg t                                                 2001
<210> 40
   <211> 2001
   <212> DNA
   <213> Homo sapiens
<400> 40
   ctgttgtaga cccagccatc caggcatggc tccatggccc tctgggtgtc attgggcagg 60
   ctggcattgg gcggatgtac aaaacggagg cacctctcag gcttcccatt tgggcccatg 120
   gggagcaccc aaggccctgt ggaggcattg tggggcgggc gacagtggtg gacaggggtg 180
   gcggctgtga agatctgcag caggttgtgg ttggccatgt tgaggatcgg gaggcccagt 240
   atggctacat gcaggaactg gaaatggccc atgcttccca cacggtccag gatctccgag 300
   aaggtcatgg cactggggca agacgagcca gagctgtggg cagggcatag gccagggaga 360
   ggtatatttg tgcctggtag tgcgtgggtc tatggaacga tatgtgggag ggtgaaccag 420
   gtggatatgc gagcacaggt gtgtggagca gagcatgtgt aagtgtgtaa agatgtggaa 480
   gctcaggcat gtggagccgc agattcttgt gtggatgtct gaggaagagc ccagctgcac 540
   ggagggctgt gggatcaaag gtggaagtgt gtgtccaggt gtgtggacac aaaggtatct 600
   atgggtatgt gctcatttag gctgcagcat aaggacacag ctgcgtgagc tcgggtatgt 660
   gttgtgagcg tgtgcacctg cggacacgag catgtgtgtg ctcagctagg ctggttgcac 720
   atgctgcatg catggagcag tgcaggacac gtagcaggga ccaaggggcc cagccttgga 780
   tgttggctac aaaggacaga ttagagggtg ggtggtgaat gtccccagtt gctccaggca 840
   gatacacagg gttagcagag ttcaggttgt ctgctgggaa aggcctcccc aagctcagag 900
   cctcctggct tctggcagtt ccctggggct gggcccacca tgctggagta tctgtaagag 960
   cccgaagcag gtctcagagg gcactagggg cttggctagt cgcaagtgga gcctggcctt 1020
   gggagatccc tgggattcct aggtgcctgg gctggggagg ccctccatcc ctcctcacct 1080
   ggtggccggg tctggatccg atggggctgg tgatcagtgt agttgaggtc cctgctctgt 1140
   cctaggggcc ggcagctgct gtagtagggc agctcagctc taacaagctg tgtttgtgcc 1200
   tcccccaggg gggcttatat aaggcataag ctgtttcccg ggctaatgtt tgactttctt 1260
   ttgcggatta acgtcactgg cagggagagt gctgtggacc caccaagccc agctctcctc 1320
   aaagggctgc ctcagcgagg gccgctgggg gtggggcagc gtggctgagc ctagcagggc 1380
   tgctggctgg acaccacggg cccaaatggg aagtctgaga cgtgcctccg ttttgtacat 1440
   ccgcccaacg cgagcagccc tggcaggctc accgagggga gattctggga aggaatctgg 1500
   cctgacctct gagggttcaa ggtccctctc aactgatgtt cagaggactt gatgctgagg 1560
   acaaccaatg ggaaaggcac tggtgcccgt aagcaggcag cattctcctg tgtgctacct 1620
   cccctgcctg gcacctcccg agtaggggaa ggggatttgc ctctcaggaa gtgttagggc 1680
   acacccagac agaaaatgtc actttcatct atccctgagg acgcagggct gccatctcct 1740
   gggcccctgg ggcagccagg acctccgtgt ctgtgtctct gacacaggtg aactgccccc 1800
   ttccatccca ccctcaatgg acaggcttct ctcctgatca gccgcttccc cctggggacc 1860
   aaatgtagtc cctgctgttt tattttcact ctgaatatta ttttaattat ataataatgt 1920
   gtgtttcctg cataaaacat gggaaataca gacaagccaa acaaaacaaa tcaaaacaaa 1980
   gcaaaatgtc cagtctcacc t 2001
<210> 41
   <211> 2001
   <212> DNA
   <213> Homo sapiens
<400> 41
   atggcctagc tgagtgccag ccaaggttga ctcattcaaa ggcagcaggg gctggaacct      60
   tggccttttg aggcccagtg cttttcccaa taccccactc tacgttgcct ctgcccatag     120
   taggcagagg aaggcacagg gggcaggcct gttgaatgca tcttgggcac cactgttcct     180
   gcgctgggtg gactgctgag tgcaggggcc aggcctccaa gcagggaggg gaggccggga     240
   cagcccctcc agcagtaggg gtaggggtgg aggtagttac aaggtttcag ataaagcgct     300
   cctccagtcc ccaggcagcc tggattctgt cggcaggaga gggtctgccc tggaaaggcc     360
   tcggggcttc tctgcaggtc tcaacctggc tccccctttg aacagaacct tccaaggctg     420
   caggcttgca tcctgcctct cagccaagac ctgtctcctc agtcatctga aaccacccta     480
   ccacgccaga cgctccctct gccttcttgc tattccagct ctctcttctt cccacttcct     540
   attttctctc cattttgttc aaactccatg aacaagaaca acagagctca gacagcgaaa     600
   gcctccccat cccagctgta tgcaaattcc cagcaggctg aaaggaccac ccgcccaccc     660
   aagaccacct cccaggaagc cgaggccttg tgtgcagccc cagaggagct tgagctgaac     720
   ggagaatggg ttgaggtggg tgggacccat cactgttctt cccttctcat tcctgcgcgt     780
   tccctgaaga gtctctgggg cctgcttttc cccccagcag agacggatgt gtcacattta     840
   tggcatccca tttccttcac agagaggcta tagatcctgt ttcattctgg tgtatttaat     900
   tcactccata ggaatcaata aaaactttaa attaaaaaaa aggcaaaatc caattgcttc      960
   tcattttctt gatccaactg gcagtcggca tgtcacctac gttcatgtgt gttctggtga     1020
   gctggaggtg ggagaaggag cttttcattg tacagtgagg cactaacttt cctttcccat     1080
   catcagagat ggggggcttc aaccttcaag tgtccagctc tgcatgaagg gggtgaccgt     1140
   ctgccctccc ttgcagcacc cctggccggg ctagccttgc ctccccatgt gctctccctc     1200
   ggggtcaact ttttcttttc cttttctttt cttctttttt tttttttttt ctgagatgga     1260
   gtctcgctct gtctcccagg ctagagtgca gtggcgcgat ctcggctcac tgcaacctct     1320
   gcctcccagg ttcaagcgat tcttctgcct cagcttcctg agtagctggg actacaggca     1380
   tgtgccacct cgcatggcta atttttgtat ttttagtaga gacagggtgt caccatattg     1440
   gcctggctgg tctcaaactc ctgaccttgt gatccgccca cctcagtctc ccaaaatgct     1500
   gggattacag gcatgagcca ctgcagggcc aaatttttct tacctttcca gagcaaagcc     1560
   accatgccaa gaggggccct gcgcccatga tgagctctgg cttgggttcc cggcccaccg     1620
   tggatgtgcc agacccaccc acaacagcct ctgctttgcc aggagttgta cccacctgct     1680
   tgcctccagc gtctgtgcct cagccctgcc tctgctcact gatgccctgg gaatcagcgc     1740
   gggcactcag tgcgaagccc ggctgggcac tggaaggtgg cagagggccc tggaccaggc     1800
   tggctctggc ttgcaccagc gttctgcttt agggcttatg ctgaccactc cccacccact     1860
   ttctccaagg gctacttacg gcgtgcacgt ggtctgagtg ggtctggagg agtctctctg     1920
   cttgtcttct ggtgtccggc tgatctgacg ggttcttata tacctcccaa ccctgggagc     1980
   tgcccttgga gcccattaac c                                               2001
<210> 42
   <211> 2001
   <212> DNA
   <213> Homo sapiens
<400> 42
   ctgaattttt ccttctggtg atgagaagca atgccagaat ttctactcca gccatcttca        60
   cacttgtgga gagaaattca atagtgatac atttgtagtg aggagaaaga tgtgttagct      120
   taacaactga tgcagggtct atcttttaag gaagatattg tgtttgtgga ggaaaacatc      180
   ttggaaggta tttagaagtg gaaagtaaga ctttaagggc aagagaaact ctgcccatgt      240
   caagagacac aactaaactg ctttgtattt ggaaatcaat aggaatttgg agagagaaac      300
   taaaaacatg agtaatcaac aaaagatgat ttaacctaca tgatcggaat ctgaagccca      360
   agatttggaa ggagagaggg aactttagga acatcaatat ctaacataga atattacatc      420
   ttgagccttt tggcagtgta aatctctaca tcccctataa ctcagtagtc ctttacacat      480
   ataagagttt ctataagtag tttggcataa ctaagaaaaa gtatagtgat ccacatctaa      540
   aacaatataa tgcaaaacat aataatacat attggacatt tagattatcc ctaaactgat      600
   tcacactaag caggacttaa gagctgaaag tgatcataaa ttctgatgaa atttgaaaat      660
   tcttgagata caggttgaac tcagtggaaa atacattgaa tcaatcttac tgatctcaaa      720
   aggcagggaa atgctgttga aatttagttt acataattat tttctctttt tctggctgtc      780
   cttaaagcta acacttggca tgatactaaa aacattgttt taagagaaaa tctattgtgg      840
   acaaatacaa gctttaggaa taaattaaaa tgtttagagg aattaacaca tctgtgtaaa      900
   attttacatg tgtgccaaac tggtccccaa gctaaaacta gtgacatatt aaaacccaac      960
   atattagctc acacatttac aatgaaatac aacacttttc gtaatactat agtttgtgac     1020
   agttcaagaa gtgatgaatt ggaaaacatg ctatgatagg catgggcaat ttagagctca     1080
   gtctggacaa tcggtgatga ttaatcttaa gaggatttct gaagttcagg caacaaatga     1140
   ttaaaaatca acttcaacaa tagtcaatga tggctgaatg taagatattt aaacatgcat     1200
   tggaaaatac aatggaacta attgtagtca tggacattga gagaagactt ctggattcag     1260
   acaaattttt gctcactcaa atccctgtaa cattgtttac cttatattac atgtcataaa     1320
   ttgactacgt tatgaaatag gaagggtaat atctattttt aaatgtaata attataaaca     1380
   atgtataaaa tatgtgacac aaagaagact ggcagtttat agtgactatt gtattattat     1440
   tttgatttta gtagttgaac aggaaaaaga gagaacagca cagactgaag tatttatatc     1500
   tcaataatta aacttactta atattttcta ttatacattt acctaaaaag tacagggctg     1560
   atacttccat atttatatgg aataacagtt cttaaaatat ataaagcaaa atattattga     1620
   aataaagaga tgatcgataa acttgtaaaa ttatcatcag agatgtaaat accacctcta     1680
   caataggtag accaaaatta gtaaagatac aaaataattt aacaacacaa taaacttgaa     1740
   ctaattaaaa tacaaggtct ctatcccaaa gaactacaaa ataaatatcc tttttaagta     1800
   gaaaaaaaat tcactaaaaa tatatatttc aagccataag acaagtctga ataaatttca     1860
   acaacagaaa tcattcatag tacaaaccct gactacaaag gaagaaagtt agaaatctcc     1920
   caattatttg taaacaatgc actcctaatc aacccaagag tcaaagaaga aatcagaatt     1980
   tgcacaaaac agctgaaaga a                                               2001
<210> 43
   <211> 2001
   <212> DNA
   <213> Homo sapiens
<400> 43
   ctgtaatccc agcactttgg gaggccaagg caagcagatc acctgaggtc aggagttcga      60
   gaccagcctg accaacacgg agaaaccctg tgtctattaa aaatacaaaa ttagccgggt     120
   gtggtggtgc atgcctgtaa tcccagctac tcgggaggtt gaggcaggag aatcacttga     180
   acctgggagg tggaggtttc agtgagctga gattgtgcca ttgcactcca gcctgggcaa     240
   gaagagcgaa actcagtctc aaaataaata aacaaataaa taaataaagt gcacttctct     300
   ctctctcttt ctttctttct ttttttttct tttttctttt gagatggaat ctcgttctgt 360
   cactcaggct ggagtgcagt ggcgcaaact cggctcactg caagctccac ctcccgtgtt 420
   cacgccattc tcctgcctca acctccggag tagctgggag tacaggtgcc cgccacctcg 480
   cccggctaat tttttgtatt tttagtagag acggggtttc accgtgttag ccaggatggt 540
   cttgatcttc tgaccttgtg atccacccgt cttggcatcc caaagtgctg ggattacagg 600
   catgagccac cgcgcccggc caagtgcact tctttttcag taaaaacagt tggctgactt 660
   caaggacatg tgaattaagt agcattacgt atagttccta gacaggggaa aaagccaaac 720
   tggtggaacc actggataat gactgaggtt tgggaaactg aggctggatg agaaggttcc 780
   agaggcctac cctaaatcag gaagcagctg cgcttggtag aaatagtgtc tgtttggtct 840
   gtttggtagg aggcctgcct cccattgagc caacaataat cctgttgaca taaccctgtt 900
   gatcaaagcg atcccctgac tgctctgggc cagttccttt ggctcctggt ggaatgcact 960
   cagagaatcc ggatccggtc agtataaaag gtctggtccc gcagtggggg agccacatcc 1020
   tggaagagtg gcctaggaca gctcctctcc tgccagagct aggcaggcgc cgaagtagcc 1080
   gcatggcccc gtcagaagac cccagggact ggagagccaa cctcaaaggc accatccgtg 1140
   agacaggcct ggagaccagc tccggtaaga ggcggcaaag ggaccccagc cccacagaaa 1200
   caccagctgg gggccaggtg cggtgcctca tgcctgtaat cccagcactt tgggaggccg 1260
   aagcgggcag atcatgaggt caggagttcg agaccagctt ggccaacata gtgaaacccc 1320
   atatctgcta aaaatacaaa aaattagctg ggtgtggtgg cgggtgccta taatcccagc 1380
   tactcaggag gttgaggcag gagaatcgct tgaacccagg aggcggaggt tgcagtgagc 1440
   tgagatcgca ccactgcact ccagcctgag caacagtgtg agactccatc tcaaaaaaaa 1500
   aaaaaaaaaa aaaaaagcaa gaccagctgg gggtgggtgg gggtctgatg gcaaacatgc 1560
   tacctccctt gggttgctgg gattctccag ccaagggcgg gctcgtttta tttcctgtgc 1620
   tagccaacct actagaacat aagcttcctt ctgagcccac gtcgtgtttc acagcacgta 1680
   gctaagagaa tggatgtgta tgttatttgg gtaactcaaa aagttacctt tttaagagca 1740
   caggctttgg attcagatac aattgctcag acacctgctt cattgataag tctattggct 1800
   ggtttgtcca tccctctacc ctgtgctggg ctctgagcgg ggggattgcc agggcaaatg 1860
   ccaccaggta ctgatgggat tctctgaggc tgtggacaag attatctggg gagggggaga 1920
   gtagcaagat gctccctgag acccaccaac acttgtgcag gctgagcctc agtttcctca 1980
   cctggaaaga tggccagggc t 2001
<210> 44
   <211> 2001
   <212> DNA
   <213> Homo sapiens
<400> 44
   taaagaataa aagtgaatga aaaaatggaa gcattgcacc gtgcttttga tttacatttc 60
   cctgatcatt tagtacaacc accatggaaa acagtgtgga gattccttaa agaactaaaa 120
   gtagaactac catttgatcc agcaatccca ctactgggta tctacccaga ggaaaagaag 180
   ttgttatacg aaaaacatac ttgcacacac atgtttatgg cagcacaatt cacaactgca 240
   aaaatataga accagcccaa atgcccgtca atcaatgagt ggataaagaa actatgatac 300
   atatatatga tggaatacta ctcacccata aaaaggaatg aattaatgat gttcgcatca 360
   gcctggatgg gattggagac aattattcta agtgaagtaa ctcaggaatg gaaaaccaaa 420
   cattgtatgt tctaactcac agtgggagct aaattacaaa gatgcaaaga cataggaatg 480
   atctaatgga ctttagggac tcaggggaaa gggtgggagg ggggtgagga ataaaagact 540
   aaaaaatggg ttcaatgtgt acactgctcg ggtgatgggt gcaccaaaat ctcacaaatc 600
   accactaaat aacttaacca tgtaatcaaa taccacctat tccccaaaaa cctgtggaaa 660
   taattttttt ttaaatggaa gcatccaaaa tgtaatttgt ttccacattc ttgtaagcct 720
   cattatgtat cccagggaca gttaatatcg tcatacgaat tcacaggtga ggaaatggaa 780
   tcacagagag attatgtact ttgcataagg agagcgaggg tgaggcagga tttgcagtct 840
   agaagcctgg gtctggagtt ctccactttg cactatatca tttctgtaaa cttcttactt 900
   aagactgtgt ggaaatgtag agtgagacag aactaaagct cactattctt agtccacagg 960
   ggagtagtga ctacccaggg cttggtaagt gctcagtaac gtttgttgaa agatgaatca 1020
   atatttcaat gctggggcaa agcagtgaaa aactggggaa tgtccaggaa ccagccatca 1080
   ggataaggca ggactagaat gaatgcaaca gtcctcacag gtccaccaag tcaacaacag 1140
   aaaaatccaa agctaaggtg gctcttgtgt gatcaaggac ctcttgtaca ccgacctgtt 1200
   aggctgtggc acagtcaaca tgggagttgt agacaagcta cttgagagtg gttaatggta 1260
   gcagtcaggc atttggagct ggaggcaagg ctgattctcc tcaactagta tcatattgag 1320
   cggggactta aagcctcaga agtctgagca gaaaagctga gaccaggctc cttgggacag 1380
   gctgttcata tgcatgattg agtttcagtg atgagtccca gaaggaaaaa gtttatgcat 1440
   gttttcctta aaaatatatt cacttatatt tagagaatcc ttcagatatc taacctaaat 1500
   acgtctgtaa catttactct cattcttttt atgacattat tagtataata atatgaataa 1560
   ttcccagtgg acttacttcc acacttcctt caaggattct ccattttatt tcataattga 1620
   agactgttaa taaatcactc tctagctaag aacaattcaa tgctaaaaca cttagcttat 1680
   ctgaccttca cattaattat ttttatgaca gcagcaggaa ctttcagctg cttttccccc 1740
   ttccttatga agccatgtgg tcccaagcag acagagttcc cttgctacac ttaagggtgg 1800
   gcttctcaga cctgagcata tcttaaaagc tttcccttga ctgtgccaat ccaaactcca 1860
   agtttcctca gcagatttaa ggggttttaa atctacttga gcatgagtgt tatgagaaga 1920
   ctttgaaatt cttacaaaaa ctgaaagtga aatgaggaag acagattgag caatccaatc 1980
   ggagggtaaa tgccagcaaa c 2001
<210> 45
   <211> 2001
   <212> DNA
   <213> Homo sapiens
<400> 45
   gtccagtaga catcaatcaa actggactca gtttgcacac acctggagct caagagtctc 60
   cagggggaaa acagagacac aaagtcagac agagagagag ccagagaaat ttcctgcacc 120
   gtgaagatag tcagaggcag ggaagaaact ccttagcact agttagagtg atcagaaacc 180
   aagaggacct gatcgctgta cctgccaggt ctcagtttct gtctccttcc aactgaccac 240
   ctcttcctct gagactcacc agttctgcat ctcttgctcc tccttctgtt tctccgacca 300
   cttccacctg tggctgtcac agaagggcgg atgaaggagg ggacactgga gatagactca 360
   gcatctgcag gcttccaaag agaggggcta ggagatccac caacacacca gcacaaatac 420
   accagcacac acagatacac acaattggtt catgtattgc taggttacag tttgctatgc 480
   tacaaaggca gtaggccaaa tttgattgaa ttgaataatt ccttattttc atcagcttct 540
   cctttttttt tttttttttt tttttttgag atggagtatt gctgtgtcac ccaggctgga 600
   gtgcagtggt gtaatcttgg ctcactgcag cctccacctc ccaggttcaa gtgattctct 660
   tgcctcagcc tcccgagtag ctgggattaa aagtacccac catcacgccc ggttaatttt 720
   tgtgttttta gtacagatgg ggttttgcca tgtgggccag gatggtctcg aactcttgac 780
   ctcaattgat ctgcccccct cagcttccca acgtgctggg attacaggtg tgagccaccg 840
   cacccagcca gcctctcagt tttgaacatg cactaccacc acctccacaa cacacaaatg 900
   taaatgcact ttcgtatata aaactgtata aatacaagga agctcataca catgcaagga 960
   tacacacata agcaccccca gattcaacca cagaaatata cgccagtaca tttgcataaa 1020
   ttcaaacacc cctttacatg taaaaatcat ataagcacat acagggatgc aagcaggcat 1080
   ggacaaatgc atgcaagcac agacaaacag acaaagctaa gtaaaaaagt gcaagctcac 1140
   ctatgcttac aaaaatagac atacatatac ccacaaaccc acacacccac acattcactt 1200
   gctcacctgg actttgatat ctctaccact gtatccctgc caatatctac agagtgggta 1260
   aagggatagg catcaggtca ctgggttgcc caagcaggaa gtctgggttc cctaacaact 1320
   ttttctaagc taatgctcct ggatgatgat gaaaaaggag gtggggaatg gatgaaattt 1380
   tataacaggg tgcagaggca gggtcaggat aaaaggccca gttggaggct gcagcagggt 1440
   gcagggcagt cagaccagga ccatggaact cagcgtcctc ctcttccttg cactcctcac 1500
   aggactcttg ctactcctgg ttcagcgcca ccctaacacc catgaccgcc tcccaccagg 1560
   gccccgccct ctgccccttt tgggaaacct tctgcagatg gatagaagag gcctactcaa 1620
   atcctttctg agggtaagac acagacgaat ggggtctgag ggtgagctgc ttcttgcctt 1680
   ggtacttggg gaagcttcac caaacagaat gaggcagact tccagagtca ggggtggcac     1740
   gggcatggtt ggtgagtacg gagcatggtg aagcatgatg ggtggtatta ttaggagaaa     1800
   agcatcaaat taaatttagc agagtttatt tgagcaaaga agtgactcat gagttggaca     1860
   gctccctaaa ccaggaaaga caccacacgg cagtatggtc aagtggtatt tacaggcaga     1920
   aaaaggaggt gacatacaga aacagcctga ttggccacag atcacagctt gccttacttg     1980
   gtcacaatct gagcagtttg c                                               2001
<210> 46
   <211> 2001
   <212> DNA
   <213> Homo sapiens
<400> 46
   agtaatctag accttcctct tcctatcaca gtctcaagga ttgtgccccc aaagcagatg 60
   caaagccctg tctacccctt ctccttcccc tgaggtagag ctttatcgaa ggtttctctt 120
   ggtaaacgtt agctttctga actaagggcc gtcttttttt tttttcccgc ttaagcccat 180
   tgcataacaa tctcttgttt taatcttttg cccaacactg cttttcttac tgtcagaggt 240
   tactacaagt gtttgaacag gtaacttcta cctttgaact tctgttgtga cagattcaaa 300
   ggttccaggg gagtgtgttg gccactctca ggacagtaca cagtagcttc gggtgtgtcc 360
   cagtcagtcc taggagctgt ggaaagagta gaagtgcctg aatgtggtgc tgaatcaata 420
   cagccagctg tgaggggagc acttcctgga cccaggaagg gagagtcttc ttccaaggta 480
   agctgcaatg atactacctg cactatattg aatatatctg tgagatagta gggaggggat 540
   tggtgtgggg aaagaaagtg ggatttgtta tgctcaacct aaggctcttt aataacatga 600
   aattacattt agttgaatag taatctcagc tgacttgtgt gggaggcaaa gaaaaaaatc 660
   agttggaaaa ggggtggaga tatatgagct atccaaatgc tagcagggga tggaattgga 720
   agggaacact gattttggta agtgtactaa agcacaccaa gccagaatgt gggtctcctt 780
   catcttccat taagaaattt ctgtctcagt gtagttagaa ggatcgtgcg tcagctgctg 840
   agggcagaga gtcccatttg gcagacagag aggaagggaa tcagcagata ttcgattagg 900
   tgctggggac tgttctgggt agattatgta tgttacttca ttggtgtatc tcatcctata 960
   aagcaggtgg tatccttccc atttcacaga ggcatagtgc gtctccaggc tgttaaacag 1020
   catggcctaa gctacatagc tccaaggtgg tgtggctaga atttgagact tgattggtca 1080
   gtttccacag cctgcccaat aatgcagaat tgttgtgggt tgccaaagct ttcagttacc 1140
   ccctgaaatc ctgagggtgt cactcatgtg cacagaggat tgataggaat acaactctga 1200
   tgggccttta gaatgactca accttcctga acttgagcat ttttactgga ggaggtcaat 1260
   aaaaaaaaaa gaaataaaac ctcatcttcc cctggccctg agccctttgg tgaaggaaga 1320
   cagcacccgg tgaagggctc agaggtatta gtgtggctga gattcgacac ccactctgcc 1380
   atcctctgct ctcctctaga ggaagttgcc tcttggggag ctgagttcct gaaactaagc      1440
   aaatgacaag tgctcctcca gccgtgagat gagccagagg atggaatgcc acaggcccca      1500
   cttcgtctcc cagcccttca tctttcttcc atgtccaaag gcggctcccc agaggccaag      1560
   ggctcagctc ttgtgttctc tgagactgtg gcacagctca ccctggcttc cttagtagga      1620
   agcttttaag aactgctctg tagaaagaac aaaccctaat ctagctccag caaagagcca      1680
   ggatcatgct gaacttcctg tgagccttcg tgggggatcg aggctggaga aagtgctgct      1740
   tcattctgag gtatctgtga ggtggggaag gggcaggaaa atttgtcatt tcggcttcct      1800
   aatctgagaa tggtagctcc agggcccccc tgtagccaca gccttaggcg gatcaaaaat      1860
   gagactaata caggtcctgg taatactggg ggtgaccaag gcgaaagaat cgatttttaa      1920
   gttaagtttt gaggtgtgac tgtatcagag ggaaagaaac catgccaatt gaacgactca      1980
   cagccctgtc gttcctgact t                                                 2001
<210> 47
   <211> 2001
   <212> DNA
   <213> Homo sapiens
<400> 47
   acaaattaca ccattccatt caatgcagca atatttcttc atgcttatgg tgtgctgtat 60
   ctgatgataa aagcacaaat aagaggattt acatttctat cttggaaata cacaatatct 120
   ataggcaaag agacaaaaga aaaggcattt catttgaaag gcataggtca gtggtgagat 180
   ctacatattc tcttgatact ctggaataga ttgggtgggg aaggcaagat acctgttctt 240
   aggaagtcac tatctcactc tggttctata ttatctgtag ttctacactg tcaaagagga 300
   cattgaacct ggtgatatgg tttggctctg tgtccctact caaatctcat cacacattgt 360
   gatccccata atctccacgt gttgagggag agacctggtg ggaggtgatt ggatcatgga 420
   ggaagtttcc cccatgctgt tcttgtgata gtgagtgaat tctcatgaga tctgatggtt 480
   ttataagtgt ttggcagttc ctccttttct ctctctctct cctgccgcct aatgaagaag 540
   gtacttgttt ctccttctcc ttccgccatg attgtaagtt tcctgaggct tccccagcca 600
   tgcggaactg tgagtcaatt aaaactcttt cctttataaa tgacacagtc tctggtattc 660
   tttgtagcag tgtgaaaatg gactagtaca cctggtgaca tcaagacccc cagcatcatt 720
   caagatttat tgtctcttga acaatatgaa tgactgacta aagtgcctca aattctgtag 780
   tttgcatgtg ggatgagtca aaagggggtg agaatgttaa caaggcacat gacccatggg 840
   aatttgctgg gcatatcctc agtcacagag caatgagggg agagtacagg caaaaggtgt 900
   aagcagaacc agccacaatc tgtggagaag aatgagatgg gatgatgttt gcttggctgc 960
   taacttgata ataagaatgt tagcctttgg cttcatttta cgtgaataca gggagttcca 1020
   ccacaaagtg ggtaggatta tgtcccctaa aaatatagtt taagtcctaa tccttctacc 1080
   tgtgaatatg accttgtttg gaaatagggt ctttgcagat gtggtcaaat taagatgagg     1140
   ttgtactggc ttaggatggg ctctaattca gtgactgctg tctttaaaag aagagggaaa     1200
   tttggataca tacatggaca ataaagagaa ggatgtgaag atggaggcag agacttgagt     1260
   gatttgtcta caagtcaagg aatgtcaaag tttgctggca accaccacaa gctgagagag     1320
   aggcatgaaa cagattttcc ttgagagtct tgaaatgaac caaatctgct gatatcttga     1380
   ttttggacgt ttgtccccca gaaccatggg agaataaact cctgctattt aaaccactca     1440
   gtctgtggta atttcttatg gcagccccag gaaacgaatt cacactacta aatgaaaata     1500
   gatctctgtc tatcaatatc aacaccaata tttatataca gggatgataa tgaccttagt     1560
   tttccagttc ttaatagcat actgaataat tgtgcatgat tgtatttcag gtatgtaaaa     1620
   taaaattgtt tgttcactta ttttcttcca atcaccattt tctctttctc cttttttttt     1680
   ggttttagat cgttataagt tttacaatgg actctcttgt tacagcaaac accaaatttt     1740
   gctttgatct ttttcaagag ataggcaaag atgatcgtca taaaaacata tttttctctc     1800
   ccctgagcct ctcagctgcc cttggtatgg tacgcttggg tgctagaagt gacagtgcac     1860
   atcagattga tgaggtacgt gtccactagg gtgctacaca gggtcctaaa ctctgggtcc     1920
   acactcaaag tcagacccta atcccactta ggcaagccaa ggggcttatt gcaccctggg     1980
   cttggtcaga acccatccct g                                               2001
<210> 48
   <211> 2001
   <212> DNA
   <213> Homo sapiens
<400> 48
   tttattctcc ccaaccaggt ggaaatgtac cctgtttagg cccaggacct accttagcaa 60
   ccttgattgg ctcacaagcc aggttgctta agacttgttt ccccagcagc tggtgtccct 120
   acttcctgcc tagccctttt ccttttgctt tgtctgtgct tgggaaatac atgttatact 180
   ctgtagagca gatttgctgg cagtaagaac agagatgtgt agagcccagc gtgctggagg 240
   gctctctgca tctggctgtg agtggcccct ggactgggac cgtgggagga cagctttcca 300
   tagtttaaca taaatgaacc acaccataag gacaatctgc tctcatttca ctcaactagg 360
   atttagtgag tactgttaaa gcacagtttg cactggagta attggagaat ggagtttgcc 420
   cttcacaatc tgtgcacaat ctgttagcag ctaaaaacaa atgttagcag ttataattac 480
   catcaatttg tctccaggct tggatcacat tccaggtgtt agaagagatg gtccatttcc 540
   ttttttttct cctttaagtc ccatgccctg tttcaagtag cttcagaagg caattgtgct 600
   gtaaagattc accttgtaat gggttcagga ctctttcaca gtttgtgtct attcgttcat 660
   ttgaactttg ctgttccaag ttagaaagcc cttgctgagg tgtggctacc tctaagaaca 720
   ctgttgattg ggtgccatgg taacgtttgc aaagtagcta ctgctaggat acagctctga 780
   cacttattac tttatttgca tatcaagcca cctgttccaa agatgaaaat atacaggact      840
   gcatatccca gtctgggtaa aaggataata gctgactgtt gaagagaaca tgttgcagtt      900
   tttctcctgc tagctgaaga atcaagttct gagggcctgc tcctcagcac ctttctcctg      960
   gtctagagta ggtgccaggc ctggcggttc tgttgcctgc gtgggtgcca gggcttctcc     1020
   ttgggaggct tgccctctgt gagagggagc agagagttga cttcctccaa gaggcccata     1080
   gtccctgaag ggggtgtttt tgattctgtc taaacctagg ctatgtgtta gcggtttccc     1140
   tcctcttaga aattgcctgg aagactcttg ctattggtat gtatgtggtt ccctccagtt     1200
   taagacaaga tttgttccag gatcggttgt agccccttct ctgtggctaa cggaagagcc     1260
   acttgtcctt ctccattctt ttccttagtc tcccttgacc cccacatcat ctcctagaca     1320
   caaggcacat tgatagtcac aattcactgt caatcactct ggattattcc ctcattcccc     1380
   tttcccaggc cacctgaagg agtgagtggg gattgctatt gtgtacattc cagtaataac     1440
   tattggctgt aagcagacat actctaaaaa taactcaggg atactgagca cccagagggc     1500
   accttctgtc ttctcgaagc ttcgccacgc ctgtttccac tgtcagactt ccagtctgca     1560
   ccacagcaca catcctttct catccagagt gctcctacta gacgagatgt ttttgtctcc     1620
   taagggcctc aggtgtattc atgaactcct cagctggtta ttttcagttt catcttcctt     1680
   ggtgcttgct ctggcacatg gtcactgctg catcctgtcc cttccccaca gtttgccatg     1740
   ggtcatccct tttttctcat tgcttgcatc ttctaactga atgaacttgt aaatatggaa     1800
   ctgaaagtca atcctcatag ctcatctttt atttaatcct aataaatgac aatctattgc     1860
   aaacattact ttaaaaatat tacttatcaa aagctgggtg aaaggataat agctgactgt     1920
   tgaagagaac atgttggggt ttttctcccg ctagctgaag aatcaagttc tgagggcctg     1980
   ctcagttctg taagaaagta t                                               2001
<210> 49
   <211> 2001
   <212> DNA
   <213> Homo sapiens
<400> 49
   ttaggctcat gtgcttcccc ctgctccccg ccccctgacc ttggaccccc attcaagggc      60
   atccctgtct tccttgactg acggttaaca ctcccaagtg catggcgctg ggctgccccc     120
   atcttgagtt ctgaacagca aagcctaatc tcagagctgc tgtgggactg tgactcccaa     180
   acctggtggc aggtgctgcc ttcagccccc acctgagtca gggaccgcca cacggccccc     240
   tgccccaagc cttgggttac cctgtagata ctgccctggc ttatgtgttc actggctcct     300
   gtgccccaac ccaaaatgcc gccaccattg ccttcctcca agaggacccc cagtatctac     360
   tgctgcttct cagagccagg agctctgagc aggagcagtc aagaggggtt gccggtctgc     420
   cctcctctgc tcttgtgcta cactacagag ggataggcaa agggagctaa cagcgtcgct     480
   cagagacaaa gaacacagaa gcaagatggt ttccttggcc agcagggagc tctggccctg 540
   ccttctaggc tccctgggag tcctgcgcgt caggcaccct ccttctcttc cgctgcaggc 600
   attgctgact ttgagcactc accctggctc tctgatccca gggccacccc ttcccctggg 660
   tgcgctctcc ctcccacact ctgctgggca tcctgtgtga aaacatcatc ccttaggctc 720
   tgccctgggg ctcctaagga acctcacgga agattctgct ccttgctcac agcctccaaa 780
   tggccaggaa aaagtttcac ctctcacaac agttttgcag gagagatgcc attctgtggg 840
   acaaaatgct gtgggagtat tttaggaatg ttctaccaac aaaccaactg cacaggcaag 900
   aaatggtttt ctccagctcc aagacctcgc tttctcctta aacaaaagcc ccaggttctc 960
   tgcctgggat caacctacat ctgaagttct gggctgcaga cgcttacctt gttcagtcct 1020
   cgtggagctg ggctggcctg tgcctcgccg tagactgcag actgtgaggg gcagagccga 1080
   ggggaggcta tgaaaactgt gtccagcagc aactggctgg taaaaataga acagacccac 1140
   tgccccctcc tgctgcccct cctccctccc acccttcaca cacacacaca cacacacaca 1200
   cacacacaca cacacacaca cctcacaggg cagatgcgtg ctgcaatgca cacaacaggc 1260
   acataggcgg aaagccatgt gaatttgtgg ctcaggagca gaggcaaatt ctgtgcacat 1320
   aggtgccacc aactgagtga aaccagcacc cacctgggga gactcgagct gcagaaaggg 1380
   aaagaatgag cagccagacc aggtgtgggg actgagcaga gatgggactg ggagggggaa 1440
   tgagggagag caaagggcca cagtgaaggc ccagaggggc aagtagcagc tgggggaaga 1500
   ggaccattgg gctaaccctg catctcttat aaggcaatat ggaaatgagg cccctgaacc 1560
   catgccccaa aagcccaaga gggctaaaga ctcagctttc agggattttt cagtggctct 1620
   ggtgcacgtg ggatacgagg gtgcctggct gtggggagtg atgtgagcag tgttgcaggc 1680
   tgaccccagt gagtgtttcc agggctgagc agaatggcag tggcagcagg gcacaccttt 1740
   atgctcaggg aggtctgagg gtctgcagac actctgcatc cccctccaga tgggtttcct 1800
   ggaatctaga tttcccaggt tccaaaggac acccgagtct catgcctgga actcagtgag 1860
   actaattcac ctctcctctg ccctaatctt catctccagc cagaagccaa cagatcccag 1920
   gggactggag ccacaggggc tgcacctgtt taccgggtat ttttaggatg gttgatgaac 1980
   acataatacc caccctatag t 2001
<210> 50
   <211> 2001
   <212> DNA
   <213> Homo sapiens
<400> 50
   ggttccttaa ctttgcctag gtttttcttc ctgtctccag tgacagagag aaatcacccc 60
   tggggttccc aaaggtgcat ccccgtcatc atcttcactc actgcatctt gaagcatatg 120
   gatgaaaccc agcaggctta tcgcatagct tgtggtctgc cctggggagc caaggaccaa 180
   gagccagaga gatgagaatc aagataaaaa taggtgaact accacctacc aagtagatga 240
   ctacatagga atggatgtgt gggcaagtgg agccgtctgt gtgtgtgtgt gtgtgtgtgt 300
   gtgtgtgtgt gtgtgtgtgt attgaagaca agggcagggg ttccagacct ccctgcattt 360
   gagccccatg ccctctctta gcatacacac agcctcccag gtacaaacag tgccctgttt 420
   ggaaagtaaa catgccatag caatcctggc acctctgtga agtgacttag atgcccacat 480
   gcaccaccta caatgctacc tgagtgatga atgtctctgg gatgtgagtg tcagctgagc 540
   acgtctgttg ggaaatgggc ccctcccaag agcctgaacc tgaatacttc ttataaaaag 600
   gttctctggc tggacgctgc tcaatccact gcctagcagg tggcccattc cagttggaga 660
   acgtagtgag tctttcagtg gagccagggt ctggtaagtc tcctagcagg gtctggggga 720
   gagggtggga tgagttgagg gcacagggga ggagcaaaca gaacacaggc agcagataga 780
   tgtggaaggg gtgtcccgca aggcacctac gcttctagtg agccaacctc ttaaggtact 840
   aatgaggaca taaaggcaac ataggaggtg gtttttgcaa gggtacacag caagtccgca 900
   gaagagctgt gactagaacc caggtctttc agctccctgg atggggttat ttatattggc 960
   acaactctcc tttcccacat ggttgtgggg catgagaggc gtaggcaaga aagaatgatc 1020
   aggacatgct tctggcctgt ggattattta gacccagcag gtgggaccgc ctgttggact 1080
   tgggtgtttt ctaactgatg ggggtgtagt aactaggaag atacagtcga aggtcggtag 1140
   gaaagtaaaa tgagggacaa gaaaaaagcc aagtggagat tttggactgg atcatacaga 1200
   taagttgccg cagtgtgctg tttattttcc actgtggaga acttgagcag tcttcttcta 1260
   gtcagagaaa atggaaatgc attgattcat tgcatggatt gtgtcctgct ctgagcatgt 1320
   ttaactcact ggaaacacct gcttggattt gcttctttgc gtccgctctt tctgggtgct 1380
   tttccctcct tgtcccaggc caggctgact tgtacactaa gccgaatctc aaaagcccag 1440
   acagagttcc attggtggaa agaaaaagcc ggctcgcctt tgaaaggctg cctttcttcc 1500
   aggtttgtcg tgaggagctc cgcgatgtcc tctcaacaga gcgccgtttc cgccaaaggc 1560
   ttttccaagg ggtcgtccca gggccccgct ccgtgtcccg ccccggcgcc caccccggcg 1620
   cccgcctcct cctcctcctg ctgcggctcc ggcaggggct gctgcggcga ctcaggctgc 1680
   tgcggctcca gctccaccag ttgctgctgc ttcccaagga gacgccgccg acagcggagt 1740
   agtggttgct gctgctgcgg gggcggcagc cagaggtccc agcgctccaa caaccggagc 1800
   tcaggatgct gctccggctg ctgagaggcc cgcaaccccc agcgctgcgc tagagaaacc 1860
   cgcccagccc agagcgggcc cgccccgctg cggctcccac gcggggctgg gcctcggagt 1920
   ttgccccgta aagcgaattg cactttgatg ttcagaaacc cactttgttc tcagccacgc 1980
   aaaactccct gaccccgatg t 2001
<210> 51
   <211> 2001
   <212> DNA
   <213> Homo sapiens
<400> 51
   gcttccgcag agtggggagg ggccgccagt gcgcgccact tttcgcctgt ccgggcactg 60
   cgccagcgcg gggcggcccg gaggcggggt cgcgtctctg cgcctgcgcg gcgggaagcc 120
   agtggggcgt tgtttcgtcc gatatccgcg tttcagtctc cgcccatacc cctccgggtt 180
   aggcggctgt agcggagctc gaaaagagtg gcgcagggtc gcgcggcccc gcctccttcc 240
   ccgcccagcg aagctctctg accacccctc ttttctagag ttctgcctcg cttcccggcg 300
   cggtcgcagc cctcagccca cttaggataa tggcgacagc tgagggtgag taacagggat 360
   ccgggccctt cacggtctcc ctgaccccca ggccgtgagc tcgcggccac gtgcgagccg 420
   cgaaactctg tcccgcctcc ctgcagcagc gcctctaggc agccgcgcgg gcgcacacgt 480
   ggcccaggcg ctcccgggcc tccccgaact ttactgcctc ggggatgggg agaaggagca 540
   aagtagagga gaacgagaaa tggccaaggg gtggaaaggt gcctggaagt attatttata 600
   atcaatagct attgcaaaat acaagcaaat cagtcttgat tcgtcaaaac ctgagtcttg 660
   ctttgcccgt taggttaatg acctttctaa gaaggttaat aattgaaact tttgaaagac 720
   ctgtctagag gccgggcctg gtggctcacg cctgtaatcc cagcactttg ggaggccaag 780
   gcgggcggat cacctgaggt cagtagtacg agaccagcct ggccaacatg gcgaaacccc 840
   gtctcttact aaaaatacaa aaaattagct ggacgtggtg gcgcgcgcct gtaatcccca 900
   gctactcagg aggctgaggt aggagaatcg cttgaacccg gaggcggagg ttgcagtgag 960
   ccgagatcgc gccattgcac tccagcctgg gcgacaaagc gagactccgt ctcaaaaaag 1020
   aaaaagaaaa acctgtctag ggggcgtgtg tgtaagtagt ctttgatgcc atggtgtgtg 1080
   tgtgtgtgtg cacgcgcgcg tgaagtagtc tttgatgcca cgatagcttg gggtgtgtgt 1140
   acgtgcgtgt gcgtgtttaa gtagtctttg atgtcatggg tgtgtatgtg tgtgcgcgcg 1200
   cgtgttgaag tagtctgatt ccatgatggc tggggagggg ggctgttttt aggtagtctt 1260
   tgatgccatg atggtgtgtg tgtgtgtgtg tgtgtgtgtg tgtgtgtgtg tgtgttcgtt 1320
   ccagtagtct ttgatgccac gatggctttg aagaactcca aaacctttag gcaaaaaagt 1380
   tgcactatca ggaaagattg ttcttgctat ttatgctgga aaaaaacaaa tgctggtttt 1440
   ttccacacac ccagttgcta tatgtatagt gaggcaaatt gcttttgtgg ctgctgggta 1500
   cattcttttc cacatagaaa gtaactgaat tcagatttta aaaagctgta gactcaggcc 1560
   gggcgctgtg gctcacgcct gtaatcccca gcactttggg agtcccaggc gggcggatca 1620
   cgaggtcagg agatcgagat catcctgacc aacatggtga aaccccggct ctactaaaaa 1680
   tgcaaaaatt tgctggacgt ggtggcgtat gcctgtaatc ctagctactc gggaacctga 1740
   ggcaggagaa tcacttgaac cggggagtca gaggctgcag tgagccggga tcgcgccact 1800
   gcactccagc ctggcgacag agagagactc tgtctcaaaa aaaaaaaaaa aaaaaaaaaa 1860
   aagctgtaga ctcagcaaaa aagattcaga tgatattgag tatcaactcc ctagaatcaa 1920
   ggcaaggacc agaatttttc ttgcaatcat ggtacctgta cctctaaaat ggcaaatctc 1980
   taaaatcttt gctcttccac t 2001
<210> 52
   <211> 2001
   <212> DNA
   <213> Homo sapiens
<400> 52
   accctctcaa ctctgggtca gggtggctgg tccccactgc tgtgtggaca tgcaagtgag 60
   ggagtctctg ggtcagctgg aatcatgaag acgagagttc agggagggtg ggagtggtgg 120
   agcctcagca gagaagttga cggtcattga tgttgaggaa gtagattgtg tggagggagg 180
   agaatggcat ggtgtttgcg ctccccacca gcctcactta cacttgtgga cactgcggcc 240
   ggtgtcctga ggacgtgtgt tgggaattca gccacgggga atggaggtcc cttggttcat 300
   atagtcacag gctctgtatt ctaaccccag ctttgtaatt ttccagctgt gcaaccctgg 360
   gcagtggctt agcatctctg agcttgcctc cagatccgga tctgtatgat ggggttatta 420
   atgcttcata aagtaggtgt gaggagggat tgaggtacta ttgggagaaa ctttgtaact 480
   aaaaggcaat tgtatcattt gcattattac cagtgacatg agacatagtc acacaaaaat 540
   tgaaatgtca tttgttttaa aaccagaaat tgcccaactt agggccctcc tgtctctcac 600
   tgggtgccct gcaggggacc aagagccaga gcaagactga gaacctgggg cagaagcgca 660
   gggaggaagg aggtggggcc tgcttaccaa ccaggcctcg gccccttccc ctttcaatcc 720
   caggcccctg catggcaggg agaggccagt tccaccatgg caggttcttt ctaggctggt 780
   gtgcagggtg ggggcctcca aggagcaggg gcatggggaa aggggctgca ggcgctgggc 840
   ctaggaagag aggctcttat cttgtgggca ggaagtgtga ggtgttgagc agctagggaa 900
   ccctggggag gcggcgggga gcaggagggg tgtgtgttgt ggtgaggagg agcggaagtc 960
   ggaagctcca gccgtcacag ccacattcac tgggcaagcc gactgtgagc caggaagtgc 1020
   tcttggggag cccaggccaa gccatccatt cttgggtcct ttggaggtga gctaagtggg 1080
   tctgcctagg ttggggctgg tggaacctgt gggagcaggg aatgtggaga gtcacatgtg 1140
   ggtccttgtg gcaggggctg gggcaggtgg gcaggggaga ggcgagggct ctggcagtgc 1200
   cgtgtgcacc tgccatgagc ctgggctagg ctaggcccct ccttccccct tcctgctctg 1260
   tgtcccgtcc ccctcctccc tcagcctacc cctcctcatc tgacccttct ctgattccct 1320
   gccctggccc cacggacctc tgttgtttcc cttccctggc tccccgcaga gcctctggag 1380
   gtctgcaata gtgaagggtg aaggaagctc cttgctaatc ctcttgtgag tgtcactgcg 1440
   tccccagtga ctgctctggc tgagtaccca gtgggggctt ttgtggccaa aggtcccaag 1500
   tgattctcga ccctagactc tggagagagg gagtctggaa ggcctgggtt tggggtgggg 1560
   gtggctctgg gtgtggttgc tgcaggagct cagagtcaac ttggccatca gggggctccc 1620
   agcttcatct tgccttccag gaccatcctg gagaagctcg aggagaggta aggggaccct 1680
   ccttactggt ttagctctgg cttaggcctg ttgtcctgat gggcaacagg aaccactgaa 1740
   ctctcaaagt gtgcctgtgt aggaaactaa ggcccactgt agtcattatt gttatagtga 1800
   aggtagctgc atggagggaa agtcccctga cactgccatt ccccccttat tccagggcct 1860
   aaaattccac agttgaagtc actcctctgc tagaatgcag ggaagggtgg cctgtcatta 1920
   gctgatgata aatactcctc cgttcattgg accttggacc agctttaaaa agtggtcccc 1980
   aagcaggcct gtctcccagc t 2001
<210> 53
   <211> 2001
   <212> DNA
   <213> Homo sapiens
<400> 53
   cctcacccac cccagcctca ggatgtcctg gggcccctgc tcacgccatc atgatgacac      60
   tgaagtccag ccagttccag gggtcccgga ggaatgtgaa gtcgtcgaca cagaagcctc     120
   gggccagtat cttgatgagg gactcaaagg tgtagatccc tgtgaaggtg tacctggggg     180
   ggagagggcc ggccgggaca ggcatgtcac ctgggtaggg gtcagtgtgg caacgacaga     240
   cccccagaag ggagggacac agaaatgaaa tatgccactc aggtggctaa ggacactagc     300
   gagtgatagc atggccaccc cagggactca aggtgtggat gagggtcaca atgacagtgt     360
   gtctccctga aagacaagag cagcaccaca cagaggtgca aacacctgag atgggctgtg     420
   tcccagggct ggggagctca gggagcaggg agacttactc cacattcttg gaccagggag     480
   gcgggtcact catggtcatg aatacgcagt tggtcaagat ggtgatcatg atgaacatgc     540
   tgaacagcgt ggggcagggt caaggaaagt gaggaagcag ctaggatggg aggtgataga     600
   gggtctcctg ggccacagca ccccatctcg cccatcccta acccctcccg cctctcccat     660
   cttgggcagg atatgcatgg atgagcacct tgatggcccc gcgcctgact acgctgaagg     720
   ggctcagcag gtagagagca ggtgtggcgg agaagcggaa gatggccttg cccttgttga     780
   gtacgatgaa ggtctaaggt gggagagagg ctgtgagacc cagggacaga cagacaggca     840
   gacagatgga tggatggcag acagacagag gaagccttcc caggcccaga ccttcttatt     900
   gctgtagtag ggatccaggt cctccagggg gatgccgatg acctccggcg gggggtctcc     960
   gtagatcatg ggtaggttct tgccagcctc caagtcactt cgtggcttcc gttcgggctc    1020
   ctcaatctcc atctgcttat tccgctgcag ccgggcctcc tcctccaccg cccgctgttc    1080
   tatggctgcc agtgactccc gggtgaaggg gcgcaagcac tcagggccca gaggcaccag    1140
   ggtgcacaga gatggtctgg ccatcctcgc atcctgggct cagagaccag aagggtggtg    1200
   ggtggcctgg ggagctgcag tgcgcagccc cggggtgctg ggcggccgcc cctccctggg 1260
   cgggccgtcc actcctcacc tcctgcttgc ccagtggccg agccggggag gtgggcgtgc 1320
   agactctatc aggactggga gatgtgcctg caaggtgctg ctgctgcaca ggtgctgggc 1380
   acagagcatc acccctggcc cccccaccac actttcccca gaagtggcag cggctgccct 1440
   caggaccgac ggcaaaccaa ccagattctt tgtggccttg ggacttgggg tcaacacccc 1500
   agtgccttcg actgcctcct gggcccaagc tccgggaact gtgtggcttc agcctaatgg 1560
   gacagagaga gaaccacagc tggctgtgct caggcgacct ctggataggc caggatgagg 1620
   ggcctggttg gggggttaga tcaaagtggg actggcacag ggacttcacc ctgagggccg 1680
   acgtggatcc tgatattggc cacctggaga tgtaggtgga gaccatgaag gtggcagcca 1740
   ggctctgtgc aaaaaggttg ctgtgattga ttagtgatgt ctgccagggg cgcaaggagg 1800
   aagaccgtgg tgcctctgcc tgtgtttgcc atccctggcc tggagggaag ggaaaagaaa 1860
   gatgggggtg ggtgtggatt cctctttcct gatctttggg gtacttggtg ttgggctggg 1920
   agagggaaga agaaagggcc ctgggcccgg caggggtctc ctagcagttc cctcccacag 1980
   ccctgggggc tctagcccac t 2001
<210> 54
   <211> 2001
   <212> DNA
   <213> Homo sapiens
<400> 54
   tgggactcaa gccctgccat tcagattctt ttgttccaca gggtattccc ttgatgtgat      60
   gttcttcccc ttcctctagg aatgggactt cctggaagcc agactacagt gctcttctgg     120
   gtctagccac ccagtgggcc taccagactc tgggctggtg ctagggaatg tctgcaaggg     180
   atccagtggt gttacctgtc ttcaagactc ccagcagtga ataccagcac catctctgac     240
   agaagtggga ggggagtgat gcaaactctg caagattcct tggttgtaga tagacttaga     300
   gtattgactt tctcaaatgc tggttataat tatagtgaac ttatcacgtg gacagacgca     360
   ggacttctgg ttagccaggg tgttgtaagc agtggtgata gctgagaata tgcagccgtt     420
   ttctccttcc tgggtacagt gctattctgc ctagaggtga gtttttgcct gtctcacaga     480
   gtaggctgca gcctgccact tctttcaaaa gttctgtgga ttctttcagc tttcctgttc     540
   agttcctgca ttcgttcctg gagaaaaaaa aattatagtg tgaatctcta cacgctattc     600
   tgtccttcca gatgggagag gcatgctaat gctgcctcca atctgttgtc ttggggggaa     660
   agaaagcaat ttgtttttgt tttttttttt aatggatttg ctgggtttag taacccagag     720
   gtcattggga accttggcga aagttatttc agtggagcag taggaaggaa gccagttgca     780
   gtgtctttgt tgagcagcag aggggaggtg aggaagtgga gagagcagct gtggtggctg     840
   aagggtgcta ggacagaggg agcatgataa agccataagt aagatgtggt agaagggaaa     900
   gagccaggac agaggggaga gaaagcagga tcctcaagga ccagagaggc agtgggtccc 960
   acagtcaggt gcggggattc aacagaaaca gaaggaggga cgcttctcac tctgaccccg 1020
   gaaagaagga agaaactgcg tgcgagttca gacaagtccg ttggcggggg ctgggatgcc 1080
   tgcatcttcc ttgttaaaaa aggaagtgca ggttcaaaac attcagagac agaaggtgga 1140
   tagacaaatc tccaccttca gactggtagg ctcctccaga agccatcaga caggaagatg 1200
   tgaaaatccc cagcactcat cccagaatca ctaagtggca cctgtcctgg gccaaagtcc 1260
   caggacagac ctcattgttc ctctgtggga atacctcccc aggagggcat cctggatttc 1320
   ccccttgcaa cccaggtcag aagtttcatc gtcaaggttg tttcatcttt tttttcctgt 1380
   ctaacagctc tgactaccac ccaaccttga ggcacagtga agacatcggt ggccactcca 1440
   ataacagcag gtaggtgact tgttcagtct gagatgctgg caacatattt ctaatggaag 1500
   agatctggaa acttcagcct cctctgaggc ccctgagaac tggggtgatg aagtgtagaa 1560
   gcttagagct aaagacaact aagtcttcac ctagacagtg ttgcacaaaa gaattttctg 1620
   caataataga aatgtcctag acctgccctg cccagtatgg tagccactag tcacacgtgg 1680
   ccacggagca cttgaaatgt gactagtgtg actgcggatc tcaattttta tttcatttta 1740
   ttttatttta ttttttcttt tttgagacag gctctcactc caatgcccag gctggagtgc 1800
   tgtggcatga tcttggctca ttgcaacctc aacctacagg tctaggtgat tctcccacct 1860
   cagcctccca agtagctgca gctacaggca tgtgccgcta cactcagcta attttttggg 1920
   ttttgtttgt ttgtttgttt tgtttttgtt tttgtaaaga cagggtttcg tcatgttgcc 1980
   caggctgatc tcaaactcct g 2001
<210> 55
   <211> 2001
   <212> DNA
   <213> Homo sapiens
<400> 55
   ttcattcatc catccattca ttccattcag gaaacacact tcggacactg aatgtgccag      60
   aacctgtgca ggaagggggt tgggggacaa gctatatggg atgctgtctt tgccttccag     120
   gaggaagctc acagcccagg cttgagggca ctgaaatgca gaagagttct gggtccaact     180
   ggggacaacg tcataaattt gcttcaggaa ctcccagtta gctaaggaga aacattcctg     240
   cctcaggaga gacccctctg caatggtgaa tacatagttc ttgtcccagg aagaccccct     300
   tctgaggagg gagcctcagt gtgtcccagg atagtttcca gtcgggtggg gaggaccctt     360
   ggtctcgggg gaattcccag tctgacccag cccttgccct aagagatctc agcctgatgg     420
   gggatacaca aagcccactc tctgggagct aacagtcaga tgggagaggt aggctgaata     480
   gatccactct atacacaggg ggaggaacag gagaccgcat gtggaccgaa actaacgaac     540
   cactcactca cactaaactg agttagcaag tcctgttggt ctttctgaaa agcaaagaca     600
   ttttgaacag gtgaaagcag ggtgggttga ggtgttgcgc agaagtggaa aagggaagga 660
   actttcttcc cctgttcctt ccccgctccc tccagaaagg tatttctgga actctccagt 720
   tggtctggga tctgggcaac tgctgtgtgt cccagtctgt cagcccctca gctgtcactc 780
   tgctcccaaa aatagcaatg gttgagggga gggagaggaa gctgtttatc tgggatagat 840
   gtggagaggt gcattagagc tgtgcccagg gagggagtct gctggggggt tcctgtccct 900
   tctggatctt tctgtcactc tttggaatgt tagtgcctaa gccccccacc aggataaccc 960
   agagtggggt gcttgggcgg aggctggtca gcacctccac gtggtcatga ctgaccagtc 1020
   tggggagggg gagggccctg agtatcagtg acagactcca ggggcacctg cccctgtggc 1080
   caaatatggt ccagagccac tggctgaggc aagaaaatct cttgggtggg ggctgggagg 1140
   ggcagactga ggctggctgg ctggaggttg acacaggagt gctcagggga gcagcatcac 1200
   aagagggcag atcgaaagca tcgtccttgc tgaaaaagtg agcatgggcc tggggtgtgc 1260
   cctggactgg ggagggagac ggcaaacttc taagacctgc aatgagagtt caggcctttg 1320
   gcacaaacta agcagcttcg cctgctcagg ctgggaggga cagagagtga ggcacaggga 1380
   ctctcaactt gggccaagct ctgagcaaat gctataatgt cctcatgggg caatagattt 1440
   gggggttctg gggtgggtcc ccttccaggc agaatcccca ccatcctctc tggtgtgagg 1500
   ggaccctgga gctccggacc tcaggccagg agcccagccc tgggtctgca gtgaggggct 1560
   gaccagacag agcttctcct gtcccagcag atggcagagg tggaagcggt acagctgaag 1620
   gaggaaggaa accggcattt ccagctccag gactacaagg ccgccacaaa tagctacagc 1680
   caggccctga agctgaccaa ggacaaggcc ctgctggcca cgctttatcg gaaccgggca 1740
   gcctgtggcc tgaaaacggt ctggggcagg gcagggcaca gggtgggagt gaggcagagg 1800
   ggctttgggg ctgagtggca gccccttcac cctgatagaa attgcccttc atcccagccg 1860
   actctcctgg atgcctgggg ttggagaagg gtgcctccaa ccccctgacc catgccccgg 1920
   aaagacagaa attcatccag agcagaatca gcccaatgcc aggtgctttc atgtgttatt 1980
   catggcatct tactaatggc c 2001
<210> 56
   <211> 2000
   <212> DNA
   <213> Homo sapiens
<400> 56
   agggagataa tatctgagaa gggacaaggg cttttgagat gctgctgaca ttctgtcttg       60
   gtctgagaag ttattccata ggtggtgttt tctttgtgaa tattcattaa gctatacaat      120
   taggatttgt gcactttttc tgaatatata tttatgcttc aatttaaaga aatgttaaag      180
   aaaaagagag tgcaaaatca gaggatagga ggaagaaaga gagatctctc tggatttgag      240
   agcagcagcg actctggaag cccaccaggg aggtggtaac agcctcgaaa gaaaaagcgc      300
   tggagcctag ggggatctca aaatgaggtt tcctgtctca gcaataattc ctaagccaca 360
   ctaacgaacc agaacactag ttggcgaaca ctagcaaaac caaagggagc acatgagctg 420
   ggacactggg gatctcacca atgtggatgg ttgaccaatg acctgagtat tcctccccac 480
   cccaacccag cctcctggga tcacactaac tattgggaaa gttctgaact tgaaacaatc 540
   acgagggaga taggatttat gctgataatg gctggagtta tcctttccaa caacccagca 600
   gaatggggac ttgcagtcat aactcaagtt gagttctagg aaataaaaaa agatctctta 660
   catatctaaa tttgaggata gagattctta ccagatacat ggaactaaca agtctgggtt 720
   ttgtttatta gttttttatt ttttaacaga cttcctcctt cttctctcat cctttcttct 780
   cagtttcttc ccaatcgtca cacttgaaac agtagggcct aaagccacct atttggtatg 840
   cctgtaagga tggccctgtg gatataaccc aggacttact attcccccca aatttgctct 900
   tatgtactaa aataagcata tgtcaggagc tatctcctcc ctgcaaagac acatgtacct 960
   ttctgtactt cactggcacg tctgggatgt ttgccagtgc gttcattttt ctgtgcagag 1020
   ttgtccatgt tagcaaaatt ctgtgacgtg ttccatttgt ttgatgaaaa gttctactgc 1080
   atgttctcct aagcacctga aacagcaaaa cacagcattt gtagagatca tttcacattg 1140
   gttaattatt gcaaatgggt tctgttctat ctacagggta aactgagaaa tggtgtgggt 1200
   gccggctgag atctaccaag tgcagtgacc caaagtccca agcacattgt gctggtgctg 1260
   cctgcagtag gaactgcgta agaagtcact gttgatgttg cttacagaac tactgaaggc 1320
   ttggatttta tttttatttt ttattttttg atacggagtt ttgctcttgt cacccaggcc 1380
   ggagtgtaat ggtgtgatct tggctcactg caaccgctgc ccctgcctgg gttcaagtga 1440
   ttctcctgcc ttagcctcct cagtagctgg gattacaggc gcccaccatc tgtaatttgt 1500
   atttttagca gagatggggt ttcactatgt tggccaggct ggtctcgaac tcctgacctc 1560
   aggtaatctg cctgcctctg ccatccaaag tgctaggatt acaggtgtga gccaccatgc 1620
   ctggcctatt tttatttaaa tttagcttta ctgagacata atgtatacac aaataaaatt 1680
   catcagtgtt agttgtacaa ttccaagttt tggcaaatgt ttacagccaa gttaaatacc 1740
   accacaatta agatattaat ttatctatga tgctgagaag ctccctgtgt ccctttgcag 1800
   tcagcaccct cccaaccact gctctgctgt cacaattgtt gcctttctag aatgtcacag 1860
   aaatgcaatc atagacactc ttttttcact tagtgtaatg cttttgaaat tcttccatat 1920
   tgttgcatag atcagtactt tgttccagtt gttctacatc ctcaccaaca tgtgtttgct 1980
   caatcttgtt aattttaggc 2000
<210> 57
   <211> 2001
   <212> DNA
   <213> Homo sapiens
<400> 57
   cggggcctgc ctcctgcatc cccggcccaa aagcccgggc caagaaggac acaggcttca 60
   atggctgtca tgtgttgcag acaacatggt gttgagatct tgcatggtgg agggtgacgc 120
   tggtccctga agggagatgg aggaggaggc agagctggga acaaagggtt aaagggcgcc 180
   atgtaagaga gctctccatt cccaccacgg agacatccag accccagcag aggcccaaac 240
   tgactcacaa acacacagcc ccatctttcc ccttccaaag aactaccttt tcaagcaatt 300
   ccaggaagct ggactcatag gaggaatttg tcagaaaaga ctccttcagc ttcagttgca 360
   aagtcatacc cggccctgcg gatccagaag tacagcttag gacccagcag tcagggctga 420
   ttcctccgga gacacagaac cttcctgctc acgctccccg gcacagttcc tcttccccag 480
   caatgcccct ccagagcctc ttggaagctc aggactgggg tgtctctgtc actctcaggg 540
   accatgaagt cccacccctt ttctctggcc tcttcttgcc acagggaccc aggagtcctg 600
   ccctctagcc ctcacctgtt ccatgtgagc tgccaaggag ggtcaagagg aggacaaggg 660
   gcagcccaga ccccatagtg gccactgcgc tcctgggatg gaggagacac tgaagtcccg 720
   gtggcctctc cttaacaggc ctgtttctac accccactca agccttagca taagtgtttg 780
   aggggaaatg ggaggaggaa tctggtcaac tggattttcc agttctccca gtaagagagg 840
   acccaggaga ggaccattca ctgtgctttt ggggaaaata gaaggaagtc ccctcttttc 900
   cactctgtgt cccacccctc ccttgtgtct ccaggtttga gggagtgagt gcgggtccta 960
   cagacaggga aatggagagg gaagcgggga ccagggaggc gtccctcctg caggtgtctg 1020
   ggcccccacc catgcccggg cctcccaagg atctttagag attaattatt aactgcagct 1080
   aattttcatc attcttgaca ccgaagggct caggaatgtg ggcccaaaag ggaggggtgg 1140
   attaagccaa gtttcttcca gaacccaggt gtcctgctcc ctcaggtttt ttttttttaa 1200
   acactagtca agtgcagtag tgaaaagagt agaaggagta gaacaaggag ttgggtctat 1260
   aatggactgt gaacactgct tccccagcct tggtggcttt ctagatgaga aatctggtca 1320
   tgggaacttc cattgctgaa cattctgttt tacttgctct aacgcatcct ggattgtcag 1380
   ggggagacac gagtttcctc agacatcctc tcactcctgc cttgtgcacc catgaaaagg 1440
   ggacacccac ctactcccgg ccgagatgcc tacaggcaag cgctgggaca ggcaagcaca 1500
   ggacagatgt gcagagggag ttactgaccc tctttagaaa ctaggaaagt aaggcacaga 1560
   gagggtaagt gacctgctaa ggtcacccag ccaacaagta gcagagagat tagaacccat 1620
   ccctctagcc agacaaagag acacaccagc catggaaact tgcttgaaca cacaagggca 1680
   caggccagca tcttcctatc tgccacttcc ctatgcagcc tctccacctt ccccaaagcc 1740
   agcgggactg agcacagaga gacagaggca gagagagcac tgggcagagg tggggagagc 1800
   aaaagcaaga tggggaaaca gagatagagg cctggtctgc actgtaagtg taaagtgagg 1860
   caaccaatga ctcccagact tgcacaggac aacaggaaga ccacagtggg aagaaagcat 1920
   ggaggagaag agcagcaggc acaggaggga cgagcaggag gtagaattgg gaacacagag 1980
   agatcttcct cggagaagag t 2001
<210> 58
   <211> 2001
   <212> DNA
   <213> Homo sapiens
<400> 58
   gtcaggagtt caagaacagc ctggccaaca gggtgaaacc ccatctctac taaaaataca 60
   aaatttagcc aggtgtagtg gcaggagcct gtaatcccag ctactcggga gactgaggca 120
   gggagaactg cctgaacctg gaaggtggag gttgcagtga gccgagatta taccactgca 180
   ctccagcctg ggcgacaaag cgagactgtt tcaaaaaaaa aaaatttctt atggctgagg 240
   agtctgtcct tatcttgggg aatcgagaag aaagctgtgg ggatattttt aaatgacggt 300
   ttccccttga caattgtcca caagattaat aaaggaagat tctgagccct taccctgtgg 360
   gaccgtgttc tcataactgt ccagcattgt gtctctgtag caatcctttt gagagccttc 420
   taaataattc caatctttgg tcatattttc aaatgctgtt gcttcctgaa acaacattgg 480
   ctgctgccac agggaccata tagtcttggg gcctgggcta aagagcagct tcagcaggac 540
   tggggaagag acaagagagg gcaccaggaa tgaaggcatg cagcataaag gactctgaaa 600
   gcgagtcagg tgaataaatg gaagaaataa gaaataggtc tcacatctaa caagatgagt 660
   acagcttcat ggatgaggga aaggagtttt gctttcctgg gaggtctgtt ggtctggcca 720
   gttttgacgc tgccagagct gtagcccatt tattaatctt tccagaataa agcagagtcc 780
   aagtgaactt gggcgcaggc ttgatgactc ctaacagggg tggcaggatc tgtttccaat 840
   ggaaatatta tatctgtgta cataaagata tgtcaggaga ttatcctgca gaaaagagat 900
   accccaaatg gagtctacct gccactgtga tcagataatt ccttagggag agaacttgat 960
   actcagttgt ctaaactggg attccttagt cagaacagcc gtaagacagg atggtgatga 1020
   ggccagggga gttcacatga ggactgcagg aattccagca agcttaggaa aacaggatcc 1080
   cgagatccca gctgtcagat gcagagcgga ggggcagggc agaatcctga gaagcagaga 1140
   aagatggaaa cttttaaagg ctctgagcaa gagtctcagt taagtggggc ctgcttccag 1200
   gtccggaagg tccttcacta aaagggcttc aaactccctt cctcagcatt gctagttaat 1260
   catgaatatt cctgattcta agacattact gattgtaaat cactaactta ataacaactt 1320
   tcttttgaga ggaaaaacag tgtgtattaa atgaatgtat tgataagaca catccaaaat 1380
   ttaaaatgtg aaaaatatgc attttcattc tttttctttt tttgtatttt tttatagaga 1440
   tggggttttg ccatgttgca aaaaacccag gccggtctcg aactcctggg ctctagtgat 1500
   ccacctgcct cagcctccca aagtgaggtt ttgccatgct ggccaggctg gtcctcgaac 1560
   ttctagcctc aagtgatctg ctcgttttgg cctcccaaag tgttggaatt acaggtgtga 1620
   gccagcacac ccagccggtc ctactagttc taacagactt gaaaccacaa ggtattcaat 1680
   ggaatccaga aaaagcacct tccagattcc tttctgaaac cagcgtctct cagaacctga 1740
   gtgctgtgtt ggttgttatt tgagtccctt caggggaccg caaaggaagg aaaagcctca 1800
   tcttcattct ataaaataat gaaggcaact gggcatggtg gctcatgcct gtaatcctaa 1860
   cactttggta ggccaaggtg agaggattgc ttgaggccag gagttcaaga ccaacctagt 1920
   caacatagtg agaccctgtc tctaaaaata aaaaaaagaa aaaaaagaat gaaggcaaca 1980
   ggctagaaac aatttataat c 2001
<210> 59
   <211> 2001
   <212> DNA
   <213> Homo sapiens
<400> 59
   tctgtgttag taattaagga aaattcaccc agaaacacag attgtgtcaa cacagatagc 60
   agacagttaa acaatgatct atggaccaca gaccatgtta ttgagaaaaa gagatctccg 120
   ttgcttggga ttaactacat ggtgtgtaac atcaggaatt cactggacat gtgtatcttt 180
   caatcaaaac atggtcacag cacatttatt cattgattca gaattcagag gtcatagtct 240
   tgtcaaagag ttatttccca aaacctgttc catacaagtt gcctctttac gggttagaat 300
   ctggtaaaga gacatcttaa tgattgggtt tgtttctctc ttctccttcc ctgacatttc 360
   cattccctac cccagcccca acatcctact agtggattcc acagatggct ctcacctctg 420
   cctctcgttg tttctcgctt ttccactgtt gctcccccag ggtcacaggg tgggctagaa 480
   gtgtcttcag atcctcctgc ttctctaaag taacagcagc ttcatactct ccatctctga 540
   aatcctcagg aaggaattcc cctgcctccc cattgccatt cttctttcca gtgacctatg 600
   agggaagaag atggcagcgt cagaagcagc agcctcgtcc tgggtcattc ttcacagaca 660
   atgccgcagg aggctcttgg tccttctccc ctgggaacag ccacctttga gcagtgcact 720
   ctcagatctc aagctgcgat gcacactgct acaattgtcc cagaattacc ttcagatgct 780
   tgtcctactc tcttggttaa atcaaagctt gggttaggtt gacagattaa atctactttt 840
   aatctgctta atcacatttc ttttaggaaa atcaaaaacc ttctctgctt aattaaaact 900
   caactttagg tacttttgat gagtttcgcc ttattgtaaa gtttccgggc ttacattctt 960
   tcaataacac ttcttcccta agacattttg ataactacac gtgattccac actgtagtaa 1020
   taaaaccaaa ggagaagata ttaaatagga aatatgcaga gaattcatta ctggtagctc 1080
   catttttttt ttccaatttc acttgtttca tcacatcaac ctttttccat atatgacatt 1140
   ttcagagttc ccttgcatta cacctgaaag caaagggcat ttcaattttc aaataaaaac 1200
   caagatgaac cagttttcat tttataaaat taatgcatct tacataccag ttcctctact 1260
   ttcagtacca tcatgttggc tgaaggagtc ttgtatgttt ttctgtcgtg gaaggaatcc 1320
   ctggagttgg ccctgctggg cccctccaca ccggtcagct tatatagctg gggccgggtg 1380
   agataatctt ccaacctggg aaccgaagta acactcccac ccccattagg agccaactca 1440
   gaggcaggtg aattttcatt ccagactcag tgtctgggaa gctgaagagg gaggggagga 1500
   caagctaacc ctgctgaata tgtgaatcag gaagaaatgt gagagggcca ttcctttggt 1560
   ggtgatcaca tcgctcagca atgcaagtca tcctatttgt caagagtcag gggacagctg 1620
   tctgttgaca ttgcaccaca tcactgccct ttttttcttt gtcagctttc atatgactac 1680
   ctatcaagaa aatgtagatg ccctacatat cacaccccca gtaatatctt tctgataagc 1740
   agacttatca acacttcact taggggaaac ttgtcccagg acatcctatt ccctgggtaa 1800
   acagcctgag gggaagggat ctgggcccta aaggcacttg tctatatctg tttggaattc 1860
   tttggagtga tgtctggggc taaataggcc ccatgacatg ccaccttacc atcattataa 1920
   gaaattgaaa gcattcagta atgcactttt cttttatgat gtcactggcc ataattagca 1980
   catgacatgc ttaccgtgtt c 2001
<210> 60
   <211> 2001
   <212> DNA
   <213> Homo sapiens
<400> 60
   agaggtaatt atccagttct atatcaaggc aaaaagacag cagtggatag aaagatctta 60
   gaagtcccac taggttcatc caagccacca tacacatagg cagaaaaatc aaaataagat 120
   atgagcctgg acagggtgag caatctggga aaagatgaac acagtatgct aggacccaga 180
   aatcatcaag tctatgaaaa ctaagccaga acacaaatgt gaattccata agatcaggaa 240
   cataatctgt cttgttcatc caggcatggt aatctgccag aaatagtgct taactgcaag 300
   aactgaatat ttgttagata attaaaccat caactaaatg agattcatgc aaccatgaaa 360
   aatgctgcta taggtacaca atattgatat actagaaagt taaaaaatca agttggaaat 420
   tagactattc catttctgtt tgtgtgtatg tatctacaaa taggtggaag gatataccaa 480
   aatgtcaaca gcagttacct ctgggtggtg aggagtaatc ttaaccttgt tatttatccc 540
   tatatgttca tttgtgaatg aatatttatt acatcattat aaaaaggatt tttaaactat 600
   ctgtatgttt aagagtatat gttgctacta tgtaagagta tatgctgtta ctgtaaagac 660
   attgcattac tactgttgac ctcagagcac gagcctcttg cctaattcta ggactcctaa 720
   ctaagtcttt ggagtttcag ctggaagaat gctggaggaa tacggaactc ctcccatttc 780
   tcacagccac ctccaactct taaaaacgct tccaactgcc tcccagcaca caaccaaggg 840
   agaaaactat tctgtcaaag agacggtgcc aaaaggcaaa aacaaaggta aggatgatcg 900
   ctggggaaag aagctgaaaa ggaaaagctc agaactctag ctggaaattt ggctcacatc 960
   cctagtatgt tactgcatag tctggctttg ttcaatgggt cgcttttaaa tattaaagct 1020
   agatgtaagc aaggtttgca acaaagtcca taagaaactc agcttttctc aaaggcaaga 1080
   agagagcagg atttttgact ggctctttat tcaatagtgc tgcttattaa attaccactg 1140
   ctacaatgtt taaagccaat tacctgagca catcataagg attctcttac cggttgtccc 1200
   agttaagtaa tgttgattga tcaactcctt gacaggagct gatggcaaag aaggtagctg 1260
   tgattggagc tggggtcagt ggcctaattt ctctgaagtg ctgtgtggat gagggacttg 1320
   agcccacttg ctttgagaga actgaagata ttggaggagt gtggaggttc aaagtaagtg 1380
   agattttctt gggtcttgaa caggttgtgt tgttatttca gggtgaatca cagttactga 1440
   tgggtcatat tgagaaattt attaaacaac tctgatcaga ttttatttct acttattgat 1500
   gtggccataa tggaactgaa gtcataggct ggcatctctc ccccagtcaa tactaaccca 1560
   acccaggtag ctgacccagg catgtaaaag atctcttctt ttggattcag caattgtctt 1620
   acagcccata cttctgtcat tctttaatac gctaatatta gagaacattt tacaaaaata 1680
   gaagtaacag ggattcttct caagatatca cttctgtttc aattattaaa ccaaatgctt 1740
   ctttagagac catgctctta tcattactat ttttctctga caaatgaagc atgtttgttt 1800
   actgagcttt atcaatgaca ttctagtata actgctgtga aactctttgt taaatatgtt 1860
   ttattaaatt tattctatta atcaaaccaa aatattgata atgctatttg tctgtattag 1920
   tccattctca tgctgctatg aagaaatact gagactgggt gatttataaa ggaaagaggt 1980
   ttaattgact cccagttcca c 2001
<210> 61
   <211> 2001
   <212> DNA
   <213> Homo sapiens
<400> 61
   aactggggtt tctccatgtt ggtcaggctg gtctcaaact cccaacctca ggtgatccgc      60
   ccgccttgac ctcccaaagt gctgggatta caggtgtgaa ccaccgctcc ctgcgagaat     120
   ttcttgaacc cgggaggcgg aggtttcagt gagccgagat tgtgccattg cactccagcc     180
   tgggtgacag agtgagactc agtctcaaaa aaaaaagaat tttagctggt catagtggca     240
   catgcctgta atcccagcta tgtgggaggt tgaggcaaga ggatgacttg agccaggagt     300
   tcaagactgg cctaggcaac gtagcaggac cctatctcca aaaatttttt taaatgtaga     360
   aacattaggt tatagcttga ctgttacaaa taaccatgtc ttggtaataa gggtctcacc     420
   tctccttctg gaattctcag tggcacactg ggatttagca gcattggggg ctggtcctgg     480
   tcccacccag aacactgggc ctcaatatcc tcatctctac agtgagtggg ttggcagaga     540
   tcatctctaa tgttacttcc aaccctgtcg ctataggtcc attgcgaata ttattatcat     600
   cactattttt caggtggaga aactgatgaa ccagttaaat atttgaataa aaacatctgt     660
   tggcatggaa aagtgattat ttcctaagct actgagtaaa acacagctgc actggcagtt     720
   ttaactctgt tttcttgtca atttttgttc tttcaggaaa ccttacagaa acatgaagcc     780
   ctcaaccatc tgctactcag ttattcgggg ctgacggcgg cttctagaac atccaggtgt      840
   tctgcagatg cgagaactca tcctgtagtc accagatgga gtcccaaaca gccaagcaga      900
   tgtaaggcct gtgctgtggc tctgaggccc tgaatacaga agggtcactt tcttagtggc      960
   caaagagcag ttgttgacat tgatgtctaa ttattgaaca cgaccagtca ttttactgag     1020
   ctgcggtgag gaaacactga ccatagaaga tcaagccaaa tgagggattg caaatttcct     1080
   gattcttttg aattaggatt ccagatgggg gcctcatttc tacagccccc aacattccta     1140
   tagccgttat cactgccatc accactgcca ccagcatctt cttgcagatt ccacccctgc     1200
   tccccagaga cttcctgctt tgaaagtgag cagaaaggaa gctctcagaa aaatctctag     1260
   tggtggctgc cgtcgctcca gacaatcgga atcctgcctt caccaccatg ggctggcttt     1320
   ttctaaaggt tttgttggcg ggagtgagtt tctcaggatt tctttatcct cttgtggatt     1380
   tttgcatcag tgggaaaaca agaggacaga agccaaactt tgtgattatt ttggccgatg     1440
   acatggggtg gggtgacctg ggagcaaact gggcagaaac aaaggacact gccaaccttg     1500
   ataagatggc ttcggaggga atgaggtgag tcttgagatg ccaggccagc ctttctttgg     1560
   atgtcttact cccgttcttg agaggggaag gggccgtgca aagcacttaa agagtcattg     1620
   atggacccat gctgatttag ttaatttatt aattaatttg gtttgaagcc agcatagcat     1680
   cgtgaagaaa tccagccatc tagcacccat ccatctatct agtaacattc attatgtgtt     1740
   aggagctagg aatacagagg tgaacaagat aaaacatttt tgccctcaag ttattcattg     1800
   tctggtagag aagacaggtt aaaaggaaag taaggggcta ggtacagtgg ctcatggctg     1860
   taatcccaat gctttgggag actcaggtgg gaggttcacc tgagcccatg attttgggac     1920
   cagcttgggc aacctggcaa gatcccactt ctacaaaaaa taaaagcaaa ttagctagcg     1980
   gtggtggcac gctcctgtgg t                                               2001
<210> 62
   <211> 2001
   <212> DNA
   <213> Homo sapiens
<400> 62
   tccagacaag ggggtgtgga ggtcagagtc cacaggagag gggcctgaga ggaaataatg       60
   gaaatgcggc tagcaaacag ccactaaatg gacagaggag ggggtgcccc cagagaaagt      120
   aaatgggggg tccagaccat gccagaatcc atcctcagtc cttcctaaag ctggaatggt      180
   tggaattcca gaggaaccca ctgtggtcaa gacagtcagc cctcaggccc cagcaccagt      240
   acacgggttg gcagggatga tatcaagtcc ccgtgattcc aaagcacctc tgtttagttt      300
   gggagggaat gccagggctg agaaccttcc tggagccaga ggcaaggagt aaggatgcag      360
   attgttctgg gatttaccaa ggggctgatg tgaacgtccc caacacgggg ccaggctctt      420
   gttggtgttc agtaaaagtg ggatggatct gacaactgat aaggatacgt tcccacccct      480
   cctgccctgg cccagccctg gcagctgatg ggagagcaga tcgtccaatt caagccttcc 540
   tacggtctct ttagaagaca tgggctgaaa cttggggtaa tagaggttgg ctggcatatc 600
   catgcaggat gattgtcttc acatgtatct attaccttgt agaataaggt agaccctgat 660
   tttggaacct gaagaccaaa gtgcaagatt agctctgcta cttccatctg tggaccattg 720
   ggcaggtatc tctgggcctt cacttactct ttgtgaaatg aggacagggg caatccctac 780
   cctaccaagt cattgggagt gaagacatga tgacacggtg attgtgaaaa gattttgtca 840
   atcgcaccag cattaagggt gcccatctcc aggttccccc aggcctcaag gctcccaagg 900
   cctgagtggg caggtagcac ccaggtatag accttccacg tgcagcaccc aggacacagc 960
   cagcatgaac tgggcatttc tgcagggcct gctgagtggc gtgaacaagt actccacagt 1020
   gctgagccgc atctggctgt ctgtggtgtt catctttcgt gtgctggtgt acgtggtggc 1080
   agcggaggag gtgtgggacg atgagcagaa ggactttgtc tgcaacacca agcagcccgg 1140
   ctgccccaac gtctgctatg acgagttctt ccccgtgtcc cacgtgcgcc tctgggccct 1200
   acagctcatc ctggtcacgt gcccctcact gctcgtggtc atgcacgtgg cctaccgcga 1260
   ggaacgcgag cgcaagcacc acctgaaaca cgggcccaat gccccgtccc tgtacgacaa 1320
   cctgagcaag aagcggggcg gactgtggtg gacgtacttg ctgagcctca tcttcaaggc 1380
   cgccgtggat gctggcttcc tctatatctt ccaccgcctc tacaaggatt atgacatgcc 1440
   ccgcgtggtg gcctgctccg tggagccttg cccccacact gtggactgtt acatctcccg 1500
   gcccacggag aagaaggtct tcacctactt catggtgacc acagctgcca tctgcatcct 1560
   gctcaacctc agtgaagtct tctacctggt gggcaagagg tgcatggaga tcttcggccc 1620
   caggcaccgg cggcctcggt gccgggaatg cctacccgat acgtgcccac catatgtcct 1680
   ctcccaggga gggcaccctg aggatgggaa ctctgtccta atgaaggctg ggtcggcccc 1740
   agtggatgca ggtgggtatc cataacctgc gagatcagca gataagatca acaggtcccc 1800
   cccacatgag gccacccagg aaaaaaggca ggggcagtgg catccttgcc gtagcagggt 1860
   ggtgaggagg gtggctgtgg gggctcagga agctcgccca ggggccaatg tgggaggttg 1920
   ggggtagttt ggtccctggg tcctgagcct caggggaggg aggttgatag ctactgggga 1980
   ttttgtatat ggcaacagta t 2001
<210> 63
   <211> 2001
   <212> DNA
   <213> Homo sapiens
<400> 63
   gacctcggtg tccacgattc cccggcggcg gcggcgctgc ccaggtcagc cccgaagctg      60
   cacactcacc ccgtgggcgc cgggcaccgt ctgccgtctg ctgcccgcgc ggccgcgctc     120
   cgctcgcctt ccgcccgggg ccgggctggg gacgctggtc cgcgccctcc cgccgcgcgc     180
   gccgtctgca caggccgggg aggctcggcg ggcgccctcc ccaacgtgga gttatcctgg      240
   gagcggctcc agggcccagc cggtgagggc gatggcagaa tcccaatccg tccccacccc      300
   ctctccgcgc cctgggcgcg tcgcggccgg aggctggcgg gcgagagcca ggcgctcctt      360
   gagagcgccg cgcgttcgca ggtgcccgga gcaggccctc gcggcgcccg gcaccccctc      420
   cacgcgcgcc cctcttcccg gcttcgcgct cgccctcctc actcgcttct ccgccgctct      480
   cccaacctcc ccagcccact ccggtcgcct cctcccagtc tgcatgccac gccaaccccc      540
   tccagtcccg ctgccatttt aactcctgag tggccagagg ccccctctac ctaacgcaca      600
   cacacccggg aggaaggagt tgggtggggc cagacgctaa cgcttgagct gtccttgtgc      660
   ctagagacac aaggcttggc tgagagaagg ggcacagccc ttgggtccgg gaagagaagg      720
   gttggcttct ctcatactgc ctcggaccct cttgcaaagg ccaggagacg aatggggaat      780
   tcagcagctt tgtgaggaca ggtgcagctc acagtcacag catgcaccta agatcacatt      840
   tttgctttgc atggtgctac cctcccgaag ttaacagtgg actaaggaat gtagtggggt      900
   gggtgagaat ttgtcactgg gcccaggcac ctctcaccct aaagaatgga gtggaagacc      960
   cctttagttg tcatgctttt ccagcattgc atcctgagcc gtgcagctct agcattctgg     1020
   aagattccgt cctggctctc aatgaaggag gtcaatttca gtcaggaact ggcaggccca     1080
   gttaggggtt tggcttcagg gttccccact ctgccagctg gacatcatga agactggggg     1140
   aggaaagtaa gaaggtgcca aaggtgggtg ctaggctgct gcctggtggc ttagaaaagc     1200
   tgggtgactg ccactagagg agggcaaagg cccaatgccc aacacccatg ccttggtggg     1260
   gacttcagcc tggaccactc agaacagaca tcacaaactc ggctccatgg caggccacac     1320
   cctactgatg ggtctggacc agagcctgca gaaccaacaa caggggaatg gatgaggtgg     1380
   aggccaaggt gtggagtcct gtgaggagtg gcttccctgg gctgcctgcc gtcatccagt     1440
   gcttgagctt ctggccctgt cttttccaag ggtacagcca gtggtgactg ccatgcctgg     1500
   tggcttttgt gaagttggct tgaaggtctg agttctgtat gcacagaggg gattctcacc     1560
   ttcatatatt gaatgagcac atattaagtt cccactaaac cacccccatg ttcccactct     1620
   ccacccctgc tacggagcat aatggcctca aggagctcag agtgacaggt tggaactata     1680
   tgaaaccaat tttaatattt caagacgatg gaagtgccat agaaatacaa aaagaaacca     1740
   ccttaatgga ctagcattat tcacaaaagg tttaacggag gagctgagtc tccaactgag     1800
   cctagttgga gggataggat ttgaactgga agagaatatg gggaccagca aaggcaggaa     1860
   agtggagaca agcagagtag aatgggttgt ctggagggta gggggcaagg gaggaaaatt     1920
   atagagctac tattagatgc cagccttttc tcatctctgt catcttagac tctgagttga     1980
   ctaatatccc cttttaacag a                                               2001
<210> 64
   <211> 2001
   <212> DNA
   <213> Homo sapiens
<400> 64
   atggtgctgg aaagggccac tgtggccagc tcagatgagc ttcaaggatc ccctggttag 60
   gagcatgaac tctcatctgt gggcagaggg atctcaggtt aaattttagg aactgtagca 120
   agctgtggcg aggagtgagg ggactttttg ggataaaatt gtcttatgag ctcaattgca 180
   tcccctaaaa attcattggt ttaagcccta acccccaata tcccagaatg tgactgtaat 240
   ggagacacgg cctttaaaga ggtggcttag gtaaaatgag atagttagag tggaccctaa 300
   tccaatacag ctagtgtcct tatcagaaga ggaaatttgg acacagagag agatgccagg 360
   gatgcatgcc atgtaagagg cagcaagagc atggccatct gcaaaccaaa gagagagggc 420
   tcagaggaaa acaagccttc cccaatggct tgacctcaca tttctagtct ccagaactgt 480
   gagcaaataa atttctttag tcaaagccac ccagttgcag tgttttgttg tggtggcaga 540
   gcaaacagat aaagtagtaa gtccctccag caccctagga ggttctgctt gttgccattc 600
   cccaaaggcc atcctagcca tgatgagggc aggtgcggtt gtttgggagg agaccactgt 660
   ggaacaaaga gaaacaaggc ttgcttgatg cagcagggag tagactacag attcaagaag 720
   ggctctagaa aggtccagca attcagcttg tcagggaacc ccgagctgta tgtagttctt 780
   gctctcctgt gccctgcagg atggcttgac ccatttctgt tctcatgtca gaccctaagg 840
   gcagggtcat ctgggccaca gacccaggcc cggtgggcct ggtgggctga cctgtgggct 900
   attcttatgc cttcaggaaa cccaccttta ccacctggct gttgtgactg gcatcgctga 960
   ggctgtttct ttgtgtataa aatcgaataa ggacacatca cgtgctagca atccaccagc 1020
   atagggcgaa ggctcagcaa agagaagttc tctcctcctc tgctctttta ggtcagcagc 1080
   aaatgcagat cggggtgggg acaaggtaaa cacataactt gggtggagat gtgattgatt 1140
   aataactcat aaatcatctg aaaccatact ttccttttga tagtcaaccc tctgtaaaca 1200
   ctcaatgtgt tctcaccttg ttatcattcc caatgatgtc gaatgcgtgg ttgccctctc 1260
   cagtataaaa gtttgatgca gctttgcctg gatgtacctg tctataagga gtcctgctta 1320
   tcacaatggt aggtaactgg ttttatatat aaaggctaga aaagcaaaag agaatatgta 1380
   ttcctatttt aatatgcgtg tgcttctacc acatagcatt gaatggtaaa tgtgacctag 1440
   ttaggaacct tatgttatta cctgcaattc aatgtgcaga gtatgagagt ctgagagctt 1500
   gcgaaaattg taatgagttt agcactagtt ctgttctcta agatctaagt tgtttttcct 1560
   tccagtaatt ctggggttca attagatttc ataagtgtct ctaattcttg ggtcctatgt 1620
   gagtaatttg aaattaaaaa taatttcaga gctacatgac tgcatcgttt ttgagatgag 1680
   ggaactgagc ctggggaggg gaagaagctg ccactggggt ctcaccgcaa gcctgtgaat 1740
   gagctgagat aggaacccag gcattcctct ctccctgcca ggggctccca aacttggctg 1800
   tgcagtggaa actccggggg aattttgaag aacactgaca cccatcttcc accccgacac      1860
   tctgatttaa ttgggctgca gtgagaacag agcatcaatt taaaaagctg cccaggtcat      1920
   tttaatactc aagtttggga accatttccc taggcccttt tcactgtatc atgtgttaag      1980
   gaaaaatctt gggtttgggg c                                                2001
<210> 65
   <211> 2001
   <212> DNA
   <213> Homo sapiens
<400> 65
   aaaactgttt agtacaattc tgacaatata atagtggctt aataaatgac agaaggagca 60
   acctttgttt ccagtttcat ttgtccacat ataccccaac tgagatttgt ttccgtgtcc 120
   tgaccaaaaa atcacagatt gcctctgtga cccagcctac tgcaggttgt ttctcccagc 180
   aggctcgaac ccaagccaag gccttcaaca cgcccaggca ctgactgagg ttgatacaca 240
   caataaggat cctaaacaag acacaatgtt tccctcttcc tgcctcccta ccccttgaat 300
   aggtggctct ggtatatgaa gtccatccca tgtaaagagg caaggcgtac ctttctgcca 360
   aagggaagaa cactgttctt tgaaaccaaa agccacttcc agtctggtct gtggcctgga 420
   cttacaaaga aaggcatcac tcccccatgc caattcttgc atacacactg gaaaaaaaca 480
   attgctctgt tcttacctgg agagtctgtg ccttgagtca tggaggaatc cagtcgggtc 540
   agattcacta gcagagccac cacccagggg acccacccag aacccattct ggagaaagga 600
   aaaaaatgag atagtaaaat cgtcagcctc ttcagaatga gctcataaaa ttcagtcaga 660
   aagtacccat taagagtata aatcgctgtt tttctggctt ccccaggatt ggaaactcct 720
   cagattgaca accaatcaag atagaagagt tttgcatcat caggtactgg gtaggatact 780
   ttcacaaagt tgtgtcatac aactcaacca ttgtttgcct gcagaatcac tgacagtaat 840
   ttaggtatac taaaaatggg ctgggagaag aagtaaaaat atatgtttga catattatgg 900
   ggccctagaa gaactaggca gactgtttat tatgtattct tctattccct ggcctgttct 960
   gaccaatagg tctcccttct tattgggtgt tgacattgcc gacaggcagt gtgtaaatta 1020
   agaaggaatt aagagtatgt aaattaagca ttctgaagca tatgcctcaa gaagatttag 1080
   taaagggatt atcagagaag aaatagaaga catagagaac atttggagag tacgatttcc 1140
   tataagccaa aatgaccaca aaaaaagaaa agaaaagaaa acatggaaag agaattctca 1200
   aagaataaaa ttttgcaaaa aggctcacca ggatacaatc taagagagca ttattgtgtt 1260
   ttatggttag tagggcacca ataaccttca agaggacagc agagtgttaa aggtagaagc 1320
   catatttcaa aagaatgaat tggaaacaag aaaacaaagg ccgcagatta aaacagtctt 1380
   tcaagaattt tgaaagtgaa aacaaaggaa aaaatgggat acactttaga aaaaaacaac 1440
   actaagaaaa aatacttttc ttgggttttt taaattgaca cgtaataatt gtacatattt 1500
   atggggtaca gcatgatgtt ccaacacacc tatacattgt gtattgatca aatcagggta     1560
   attagtatgt gatatatata catgatggaa tactattccg ccataaaaaa gcatgaaatc     1620
   ctgtcatttt caacaacatg ggtgaacctg gagggctcta tgttaagtga aataagccaa     1680
   gaacagaagg actatattac gtgatttcac tcatatgtgg aatctaaaaa agttgacctc     1740
   atcgaagtaa agagtacgtt aggagttacc agaggctggg gagtcagggg tagaaatggg     1800
   tagaggttgg taacaagtac aaagttacag ttagacagaa ggaatgagtt ctggtgtttt     1860
   gggaaaagaa acttttaaga caggagagac ttcaacaagg ttgcaagtga agtgcaaggt     1920
   atctgtagaa aggagaaact gaagagaaaa aagagaagaa aggggacatt ttctccaaaa     1980
   tgctgtggtc tgaatgtcac t                                                2001
<210> 66
   <211> 2001
   <212> DNA
   <213> Homo sapiens
<400> 66
   aaatggcatc tttggctgat acaaaaaaat tagccaagta tggtggtgcg tgcccgtggc      60
   cccagctatt caggaggctg agatgggagg atcacctgag cctgggaggt ggaggttgca     120
   gtgagccgag attgtgccac tgcactccag cctggacgac agagcgagac tctgtctcaa     180
   aagaaaaaaa aagaaagaca gcctgtccaa tatggtgaaa ccccatccct actaaaaaat     240
   acaaaaatta gccaggtgtg gtggtgtgca cctgtagccc cagctactta ggaggctgag     300
   gcaggagaat cgaggttgca gtgagccgag attgcgccac tgcactccag cctgggcgac     360
   aaagtgagac aaaaaacaga aagaaaaaaa aaaggaaagg aaaaggaaaa ggcatccttt     420
   tagatactaa ccaggcctgg ggtcagatct ggacttaaat cctggcgtga ttgcagactg     480
   gctggcttga ctttgggctt gttctttctc ctctctgggg ctcagtttcc tcatctatga     540
   aatggggagc tatagtacct gcttctgaag gttggctggg cttttgtgta aagcactcag     600
   ccctgggcct ggcacatagc aggggctcac tagatgggcc atattaactg tctgctccaa     660
   ggctggaggg tgttataggg aaaacagtca gaaaatactc tccagatgca aggtggcctt     720
   aatatgtcag gatgtggaag gcagagagaa gcctcggggt ctgggatctc ccatagggga      780
   tgagtatttt ttcctggaat ccctgccctg ttgccacatc actaccctga ccatttgccc     840
   agcccagccc tacatgcttc aggtgaaagc agagagagag gggcgggaga gaaaagccca     900
   tcaagcagca ggcggagcct ccctgcagga gtctggcctg gctggaaggg gagggggctg    960
   tggggccagg gacgtcagat gccaacagca ccaggggacc gttaaagttg ctgcgccgcc    1020
   aggtgggcca gggtgtctgc agaggtcccc ggcctcctgg gctgccgcct gccaccgctt    1080
   gggctgcccg tcctgggtac gcaccgttgg agcctcctgc ctggtctctc gtgcttctct    1140
   gtctcttctc taagtcgatt tttcgctctt tcttctgttt gtgcccctct ctctgtctat    1200
   ttctgtcctc ccccagccac attgttgcct ctctctcatt ctctcttatc tctttacctt 1260
   tctatttctc attctttttt ctctgtaact ctttttttct gtcccctttt tggtctctct 1320
   tttttctctg cctctttcat tctctgccat tgggttttct ctgtgtctca ttctcaattc 1380
   ctaaattgca gcaaagcagg gacaaaagga ccttgcaata tgacttgggg taaggtgggc 1440
   cctgccctcc gagggcattg actgagcccg ctccagcagg attggtgaag aggatgtcta 1500
   gactggagag ggctgctgtc tgtctcctgg gctggaggga cagagcactg gctttgaagt 1560
   ggtggcacca tctcacccca gggccatgtc tggattagcc actgtctggg catttcttcc 1620
   ccttgcccaa cccactcagt cccctggggt ggaaaggaag gggtgctggg catctggacc 1680
   agagctgcat ctggaggtct ttgccaagtt cccaggaggc agcatcctct gggtcggcac 1740
   tgttggagaa cgctcagggt ctggtgctct cgtgcctgcc ccttccaggc agccggctct 1800
   gattgcacaa ggcagacctg tgactcctcc atccagctat gcccctgctg cccagcaccg 1860
   tgggcctggc aggcctgctc ttctgggctg gccaggcagt gaacgccttg ataatgccta 1920
   atgctacccc agccccggcc cagcccgaga gcacggctat gcggctcctg agtggcctgg 1980
   aggtgcccag gtaccgccgg a 2001
<210> 67
   <211> 2001
   <212> DNA
   <213> Homo sapiens
<400> 67
   tcatgagaga aaaaagaaag gacgtattat ctcactgtga taaatctcat aatattggca 60
   tactccctaa gttgggcaac tttgatcagt tttctggaac catcataaaa aggcctttaa 120
   ttggataaag tctcgaactt ttcttctgtg gatgtgaata tcttgagaag tactggaatg 180
   aaacaatttg gctctggttt cagtcatctt cacatgtttc ttcaggtgct ttcttaagct 240
   attagggcaa aataagctgg tagaaaaaag gtgttacttg taggatgaaa gaagcaacta 300
   aagagaagaa actttctcta gaaaaatgtt acatttacac atttataatt gttaacaact 360
   tttttcttct tttctttttg ggagactaaa taatgggatg atacaggaag aaatatgatt 420
   tagagaaaac tgattgagcc aaatttttca aacggtaata tgggtaatca taggctatgg 480
   aaagttaaac tgagtctcca atgtttatga ttatgatgtt tggctaggat ataaaataag 540
   gcttcagaat ataaaatatc ctgcagattg agaagtttta aaagtagtta catacaagtt 600
   ttatttttcc aacgttcctg ccttttctaa tgtctaagga tgaagcagtg tacagagagg 660
   aaagaatgtt atatgtgtgg tctgcttgca agtgctggat gcctcctctg cctgctccaa 720
   ctaaactgtc tgcttatgag aaagtcattg ctcctctgag ttcctgcctc cctatatatt 780
   caacattaga attttgccag gttatctttt agggtcgttc aactctaaca tatatatgag 840
   tctgtgacac tgaactcaga cacttcagaa caggaaccct ggatttctct ggatgtggaa 900
   atctttaaat ctatacctga ctggtaggac aaccaatctg tcttcctttc ctctcctctc 960
   ccacttatga agggagtcca gtcagaaaca tgtttgtcta cgaggtagta tatctggtat 1020
   ttctgaggaa ttcggttatt atataggatt atagaatatt caactttgag tagggtctgt 1080
   ggactccatc aattgcgttc tgggggaggt accatgaaag aacattccta ctttagaaat 1140
   agatattctc tgttcaaacc tgacaccctg acaggatcct aaaggatgga agctaaagtg 1200
   gtgtggtata taagtcccat gaccttgaac aactggtttt atgtgggtgt caccagtgct 1260
   ttataaaagt aaactgcatc gccccgagtt cagtcatact gcaccagctg agcaatgcat 1320
   ggagtggacc tgtaggcgac ttgcatcgtc ttcaacatga agatagccac agtgtcagtg 1380
   cttctgccct tggctctttg cctcatacaa ggtgagcaat ttgtgtgtaa tctaagcctc 1440
   ttgccacaca tctcaaagcc ctggggaagg gacttttctc cccctactcc tgccaaaaaa 1500
   tgtttacgta tgcatgtata atcctgaaat gtcttgccag acacagagtt ctgaagattt 1560
   atatatgtgg ctttataatt atgttgttat ctttagaaat ggcaagtagt ttgagataat 1620
   ctgaattttc taaccatgag aaattcttcc attaattctt tattaattca gaaaactgaa 1680
   tagattcttc ttcatcttcc tcttgctaaa tgtatcaatt cataatgcat attcttatat 1740
   atgtaataga catttaaaac ttaattgtaa ataatatatg tattttaatg ttataggtat 1800
   aaatgaaata ttttttaaaa ttcaatcatc tgttcttatt ttaaagcagt ggtatcttct 1860
   agcttatttt agtcttgtgt tttcaaatgc taaatgggtt gaaagcattt ctcccaaaga 1920
   gtgagggact tttgtgggag ctggaaggta gatttttata tttgctattt aaccctaaga 1980
   gaacacaaat tgcctagact t 2001
<210> 68
   <211> 2001
   <212> DNA
   <213> Homo sapiens
<400> 68
   gttatagcag ccctagaaaa ctaagatgga actcgccggc tggtcactgt cttccctctt 60
   atcacactac cttcccttac tgcagctact tatttaactg tttcccacca aaagacccta 120
   acatccagga gtgtggggcc cacatctgcc taattcgcca ctacggccca agcccacctc 180
   tggcacactg gaggtgttca agatgtgagc tgacatatga aagagctgtc ctgactagtt 240
   ctagaatctc aggcatgtga ctccccctcc cagaatgtca atttccccat ctctctgcag 300
   agggaaaaga aaataattgg agtagatgac ctctgtgagt ccttcgggct caaaagatgg 360
   ccgggactca ccactgaagg tgtctgcatc catcatggtc agatcaggcc atgagaaacc 420
   ccactagagg tgctaaacag agggaatggg tcacagaggt gtgggaagcc tgagaaacca 480
   ctgcggcctt cctgctggac ttcagagaag ggctggcctc attttatggg ctttctgaaa 540
   cccatattca gagaaacaca cccaattcag catggactgg caatgcctat ccccaaggaa 600
   actccaaaag gggtgagtct cctgggcagt ggtcactgag ttactacaag gcctttgggg 660
   ttggggtggg gatggtgggt caacaggctc ccctagaagc cctgtgccct gacaaagtaa 720
   acaatggcaa aacatgtgct tcccagcact cacctcccca cctccttcta acatcagaac 780
   tgggtttctg atgatgggag aaaatgtaaa tgaaatcaac ggaaggatga tcctgtgagt 840
   aatctgacaa gggagaacca gcttcctctc ccatgaaccc cagctgcctt ggctttcacc 900
   cagacccctg gcccacccgg tgtcctcccc agacagtggt actcacccac actgctgagg 960
   gagctgctgc tttctgagtc tgagggcatg gtggacagga cgctgtaggt agaacctggg 1020
   gaaagagggg agaccatgtt actaacccca aggggtcact cgtcttcacc ccaagccagc 1080
   tgatcatcag tcaatgccag gccatgagag ctacatgcac caggcactag aaatccacat 1140
   ccacgggcca agcagaggag gcgcgctcag cagatctgga cttcggtgtt tctgccaggc 1200
   ctcccacctc actttatcgg cccctcctgc tctgggcagg cgggcagagg ggaacttgga 1260
   gtcagtcagc tggcttcttg ttgggacatc tggggaaagg cttagcaagg gagtgacatg 1320
   ccccaggtaa acatggggtg tgggggtttc ctccacatct ctagcaatat gattctggct 1380
   tccatgcaga gggtaacaga gcagaaatta aagaacaact gaactatctg ttctttctct 1440
   gactgttctg ctctttaggt tctaactgta ttcaagctca gttcttaata atttacttag 1500
   tatctgcccc cttattaaaa gttactccta aaatgatgag caattgtacc atcaccaaag 1560
   ccagagggta gcactttttt ttgtccacac ttaatgttaa ttcctggttg tggacacaca 1620
   accactgtgg tttggtttga attcaaatat cttcaaaacc agatgttacc ctcccagcac 1680
   cgggaagccc agtttctgaa cctagctcta tgtaggtgcc catagctggg tcgtgctgag 1740
   caagtagctt ttgttttctg gaactctgtt tcctggtctg caaaatgaag gagttcaacc 1800
   agatggctta taaggtccct accccatccc ccatccccca tcccccatcc catttcagca 1860
   atttccatga ttaatggaaa cagaaggtac actttgctca tcaagactat ggatgacata 1920
   aaattaaaag ggagggcaaa ctgactcaga gactaaattg ggagtggaca aagcataaca 1980
   cacagagatg gcaggtcaga t 2001
<210> 69
   <211> 2001
   <212> DNA
   <213> Homo sapiens
<400> 69
   aaatgattgt gaaacatttt ttgcaactgt aagagggttg atacttttcc tgaatattat 60
   tcattcaaca ttcatttatt tctttgacaa atatttaaga gatgctcatg atgtcctagg 120
   cattgagcta agaactcaat gttgcaatgt ggataagatc cagtccctgt gtgttcatac 180
   ttatttaata ctattataga caaggccttt aatacatacc atagtcatat atcaattttt 240
   aattagcaat atgaaataag agaacaatgg gactttccac tcaatttgac aggtggctaa 300
   attttcagtt actgaattta ctattttagg gtaatctagc acatgaccaa aatttaaatg 360
   cacagaacag aagatcagag taagaagagc ttccatttgg attatcactt tttctacttg 420
   gagtcacata ctgtacttta aattgattaa gaacatgtta aaggtccagg tggtgatttc 480
   cagctacaag tccaaagtat agaagaatgt gtttaaatcc tacgtttatc agatccataa 540
   atatagttcc ttctatgtta agtggcttta tttctaagaa cccagccctc ggtcctgggg 600
   gtgcttccca gcccgctgct ggaattcctg atcgtggcag tctgctgaaa catgagctca 660
   gttaccaaat atagctgagt ggagtaagag gacagatgag agtctgaggc cacagttgcc 720
   tcaaagtgac cacggaccag gcagtctcta atgaataggc aaggccacaa cctccattct 780
   cccagaaaag aagaaatgct catctgaaat tcatcacctc tctggagtct tcaaactgac 840
   caagcattga aaaggtctgt aaggtgtatc ttaatatact aactagtagg gcaacatttt 900
   aaggacttaa aaaaaaaaaa actaaaaata caaaagtttt tctgaagata tgatactatt 960
   tacaagatac cttaattatt cttttgcttg gagaggaaac gtaatgagaa tgttacggtt 1020
   ttgccaaaaa cagataatac gtaagatttg cctgcccact tcatctctgc ctttgctgtc 1080
   agagaattac actttctgtt ataagcttaa gcatgctaag atggtgctat caattccaaa 1140
   aggattaaat gtatttatgg agccagcttt tgtcaagatc gtgtatgtgt tatgtttcac 1200
   agaaaaggaa aacgcagctt tctagaccct cacccatatt taagctagat aagtgatgat 1260
   ggcttaaagt gatagacctt agatttcagt aacattataa attgttaatt acactttcaa 1320
   aagattctaa aagtaactca ctggacataa ttaaatgagt tgggatagta acagcaggta 1380
   ttttaaatta tattcatgca taaaatttac atacatattt ttaataagat aacgcttact 1440
   aagggaaacc acagtgcaat ctcgagtcca gtcaagttta aacatcacag aataattttt 1500
   atcatgttgt tttaagttgc acatgaaatt gtcttgaatc caatatagga ggttgacata 1560
   aaatgatttc aggctcatat tacttattaa catttgctaa aaatactaat tgccttctag 1620
   tatgaataat gtggtgacat ataaagccat ttcttggcaa tataaaacta ttacaaatct 1680
   attcaatcac atatatgcag aaattaaatc ctcatttcat gataaatgtg ccttccaatg 1740
   ttttttctca cagaaagaac ttgaatactc acctcaatta ttcagatttt tttttcagat 1800
   ttttaaaatc aaagtctctt tgacaagtta ttgctaacag cagaaaaggc cgatgaggtt 1860
   tatctggcca ctaactgccc tgtaaataca tgattacaca ttcctagaat ataaattagt 1920
   cctctggcat cctagaatct ggtatggatt agggtctgta tcccaggggt tctgtgtcag 1980
   ctcttcctct gggtttctct g 2001
<210> 70
   <211> 2001
   <212> DNA
   <213> Homo sapiens
<400> 70
   cctctttttg gggaaaagga cttcaagctc aggttttctt tggtggggtt gggtattgct 60
   tcttccatat tccaagtctc aggaccctca ggggcaggtg ccaagtgaga gtgaccccca 120
   gaggtagaaa ggacccagca tcatttctga ggacacctgg tagaggagga gcctggacac 180
   ttggcctccc atcctggctc agggtatggc cttgacacat cttcatcctt gtttcctcct 240
   ttggacaatg gagatgttca gactaggtgg tctctgaggg ccctgccagc tttagccatc 300
   cctccccaca ccagctaaag ggactaattc ctccgaactt tcatgggtca gatagaggca 360
   gggaagggac agccccagcc ctgggaggca ggtctcctta ccttcttcca gttccttcac 420
   aggaatcaca cacttgaagc cacagtgcag gtaacaacac ttcctttccc ccagacagtc 480
   ctggtctgtg tgacactggg gaggatcgga cttgaagcag cgtacgttgt cagctgggca 540
   aacccctgct ttctctatac ctagtggagg aagccacaag gtgatatgag aactccagcc 600
   ccagagggcc cctcaggacc tctccccagc ccagccccac ccagctccac catgtctgct 660
   caccctcttt aactccttcc acagccacca gggtcacaag aacgagagac accatgagga 720
   ccaagaagct gctggacccc atgttgccag gcaggtgctt ggctgggagc tgggctgagc 780
   ctggctttat agtgttcctg ctgggcatgc cagtgggtgg agctgaaggc tcaaaatggc 840
   aagggaggct gcacttccca gctctatttg gaaatggccc cttctgagga gggacttggg 900
   gctttcccta aggctctgca ctggaataga tgaagaaatc caagagcgac cctcgttctt 960
   cacacgagga gaagaaatgg acacgtgatt gaccattagg cgccaccagg gccaaactat 1020
   cttatggaag gaggaaaaga agcacagaaa gggcatgaaa ttactcaagt tcacacacga 1080
   ggcagtggca gggccagact tcctcaactc caccgtgttc tctatcagtc cctcttcatg 1140
   cctgattcac ctgggaccaa aatcttccct ccttcctgtg ctgccccgga cttgttctca 1200
   gttctcacct caccccaagg aagaatctga atggcagtgc ttaaaatgtc tgtcctgaac 1260
   attcactctc agccttgcgc tccagcaccg tgtgctcctg ccccccgcca atctcatgtg 1320
   gcctggagga aagcagattt tggacctgtc accagctgtg tgactttgaa agcagcttca 1380
   cctttctaag cctccgtttc ctcattcata caatgagcat aataattata ctgtatcatc 1440
   ttcataggat ttgttttccc tcctctgccc ctgcatcccc caaattcctg gatttggaag 1500
   aagacatgga cgtgtgtgcg tgtgtgtgtg tgtgtgtgtg tgtgtgtgtg tgtgaccagg 1560
   cttttggggt cctttctttg aatgagtact aaagggagga aaatccttcc tgttggaaaa 1620
   gttgcagata taagcccaga aactccagaa atttggtcct ggccctggtg agagagaaat 1680
   tctgagaaga taaatctaag atgctgataa aaagagatga gagatggaca gggagtaatt 1740
   tagaggatat ctagtccttc actaaacttt ttcctgagat cagccccact catacccagg 1800
   gttatgttac cacccaatac attcaccttt ctgcctaagc tggtctgaat tggatttctg 1860
   tcatttacta acaggacccc tgagtaatgc aaagatttgg gtttggggtg ttccacatcc 1920
   tgaagtgtgg aagggactga gttggggtta agtggaggca gggaggatac ctccattcct 1980
   ggaagtgagc ctactcctta t 2001
<210> 71
   <211> 2001
   <212> DNA
   <213> Homo sapiens
<400> 71
   cctctttttg gggaaaagga cttcaagctc aggttttctt tggtggggtt gggtattgct 60
   tcttccatat tccaagtctc aggaccctca ggggcaggtg ccaagtgaga gtgaccccca 120
   gaggtagaaa ggacccagca tcatttctga ggacacctgg tagaggagga gcctggacac 180
   ttggcctccc atcctggctc agggtatggc cttgacacat cttcatcctt gtttcctcct 240
   ttggacaatg gagatgttca gactaggtgg tctctgaggg ccctgccagc tttagccatc 300
   cctccccaca ccagctaaag ggactaattc ctccgaactt tcatgggtca gatagaggca 360
   gggaagggac agccccagcc ctgggaggca ggtctcctta ccttcttcca gttccttcac 420
   aggaatcaca cacttgaagc cacagtgcag gtaacaacac ttcctttccc ccagacagtc 480
   ctggtctgtg tgacactggg gaggatcgga cttgaagcag cgtacgttgt cagctgggca 540
   aacccctgct ttctctatac ctagtggagg aagccacaag gtgatatgag aactccagcc 600
   ccagagggcc cctcaggacc tctccccagc ccagccccac ccagctccac catgtctgct 660
   caccctcttt aactccttcc acagccacca gggtcacaag aacgagagac accatgagga 720
   ccaagaagct gctggacccc atgttgccag gcaggtgctt ggctgggagc tgggctgagc 780
   ctggctttat agtgttcctg ctgggcatgc cagtgggtgg agctgaaggc tcaaaatggc 840
   aagggaggct gcacttccca gctctatttg gaaatggccc cttctgagga gggacttggg 900
   gctttcccta aggctctgca ctggaataga tgaagaaatc caagagcgac cctcgttctt 960
   cacacgagga gaagaaatgg acacgtgatt gaccattagg cgccaccagg gccaaactat 1020
   cttatggaag gaggaaaaga agcacagaaa gggcatgaaa ttactcaagt tcacacacga 1080
   ggcagtggca gggccagact tcctcaactc caccgtgttc tctatcagtc cctcttcatg 1140
   cctgattcac ctgggaccaa aatcttccct ccttcctgtg ctgccccgga cttgttctca 1200
   gttctcacct caccccaagg aagaatctga atggcagtgc ttaaaatgtc tgtcctgaac 1260
   attcactctc agccttgcgc tccagcaccg tgtgctcctg ccccccgcca atctcatgtg 1320
   gcctggagga aagcagattt tggacctgtc accagctgtg tgactttgaa agcagcttca 1380
   cctttctaag cctccgtttc ctcattcata caatgagcat aataattata ctgtatcatc 1440
   ttcataggat ttgttttccc tcctctgccc ctgcatcccc caaattcctg gatttggaag 1500
   aagacatgga cgtgtgtgcg tgtgtgtgtg tgtgtgtgtg tgtgtgtgtg tgtgaccagg 1560
   cttttggggt cctttctttg aatgagtact aaagggagga aaatccttcc tgttggaaaa 1620
   gttgcagata taagcccaga aactccagaa atttggtcct ggccctggtg agagagaaat 1680
   tctgagaaga taaatctaag atgctgataa aaagagatga gagatggaca gggagtaatt     1740
   tagaggatat ctagtccttc actaaacttt ttcctgagat cagccccact catacccagg     1800
   gttatgttac cacccaatac attcaccttt ctgcctaagc tggtctgaat tggatttctg     1860
   tcatttacta acaggacccc tgagtaatgc aaagatttgg gtttggggtg ttccacatcc     1920
   tgaagtgtgg aagggactga gttggggtta agtggaggca gggaggatac ctccattcct     1980
   ggaagtgagc ctactcctta t                                               2001
<210> 72
   <211> 2001
   <212> DNA
   <213> Homo sapiens
<400> 72
   aaaaaatacc taaaactcat caccccttaa ttatgtgagt acatttttct attatttgtg      60
   ctgtcgaggt tacttacgcc cattatgtct gcatgatggg actactcccg tacagtggtg      120
   agcgaccttc ccatgtcccg gttcaaagac actgtagtgg tggtttgaaa gcagccctga      180
   tgggaccact gacatcacag aaattggcaa atgctgcaaa gtagggctat cccaccccac      240
   ccagagatct aactgttaca acatttacaa gcctgaaagc caatttgagg caaaaaggct      300
   gtcatggaag aagattctca ctgtggagtt tgcgcactca gatttgagtc caggctccgt      360
   ctccacccac tgtggggcct gggctatttc cccttgagcc tcgcctttcc tatctgcaaa      420
   ataagactaa aagtacagca gcttttgcaa attgagcatt tgccgagtgt ggtggcttgc      480
   ccctgcaatc ccagcaactc aggaggctga ggcaggagga tcacttgagg ccaggagttc      540
   aagaccagcc tgagcaacat agcaagacgc tgtctctaca acaaataaat acataaataa      600
   gcaagtcagg caggggggag cacacctgta gttctggcta cttgagagtc tgaggcaaga      660
   ggattgcttg aacttaggag ttggaggctg gactgagcta tgatggtacc tctgtactcc      720
   aacccaggtg agagcataag atcctgtctc tcttaaaaaa aaaaaagagc atttactacc      780
   tgtcaggtgc tgaatccatg ctcttgctaa atctgccagc agccctgtga ggtgggtgca      840
   gtcacgtccc ttgcgaagat ggggatttgg gggcgtgaag aggataagta cctcatcaaa      900
   ggtgacagat cagatttgaa ccacactctt aactgctgcg gtttattggc cctacaataa      960
   tagtgcccag ttcacagggt ggtttggggg attacacgac gtaatgtgtg ttaaagggct     1020
   tagcacagat tcagctccag aggaacatag taaatgttga tttaccgaga ggcagaagag     1080
   cgggatccac atccacccac gtttgaatct caacttggct attttctgat tgcgagatct     1140
   ctggagtcaa gtcattttgc ttatctaagc ctcatgttcc cccagtgaaa atggggataa     1200
   tcatagctac tgaagagctg ctcagaggat ggagatagag gatgtaaagt atctgccgag     1260
   aagtgggtac tttatgggag tgagtgttcg ggattatctt ttattttatg tcacgcagac     1320
   agcaactctt cccagccctg tggaggaggc cggaggttgg ggccggagcc ctggaagcag     1380
   ggccttgctc gggcagcttc tgaccctccc ctcctggcca ggtcagcccc tcagtgggcc     1440
   ctggccctag aaggtctcag aagcctgggg acgctgttgc taggcaccag ttgctaggct     1500
   acacattgtt tctcctgctt ggcctcctcc aggagggctg gcttctctgc agagctctgc     1560
   ttcctgcagc ctctccccga cctcagagcc tctgcagagg aaaggtgagg gtggggccgc     1620
   tgtggttggg cctcggggag agtgcagcca attcccaggg gaggcaggca aggccagagg     1680
   cgggcaaggc catggagatg agggagagag aggagccggg ggctgggagt gagcgcctcg     1740
   gcctgacttc ttgggtgggg gccaagaatt ccccaggtag tgggttgtcg gctcattcat     1800
   tccgagaggt gggtatgtaa ggttcaggtc aggggccgag tagtcaaaca cctaggtctg     1860
   aatcccagca ctaccaccta ttatggagca aatcctttat cacttggaac ctcactgtcc     1920
   cagtggtaaa tggggacagg aagagttaat aagaaggtgt gggaaagtga gaggagacat     1980
   gaggactctt gggataagtg t                                               2001
<210> 73
   <211> 2001
   <212> DNA
   <213> Homo sapiens
<400> 73
   caaagttgca aggcactact atagatctcc catttcttcc ttttgttcac cagaacgtta      60
   acaagagata caacacctag aaaacagaga gcagagactt ttctggatca aacagtacct     120
   tttttttctg tttctgtctc cttgtgactc aagggagaaa tgatcctttc tttctaagtg     180
   atcttatttc tctctgtcaa gaatccgtgg acatgttatc tggttagagc tcagcctgca     240
   gccaccacgc aggtaactca ctcacgttag actataactc acctaactcg tttgtgtccc     300
   agccaaatgt gtaccagagt aaaagttcag acctgaccat gggtaagttt aatgattaac     360
   ataagccact caggcttttt cctcagggct tgttagtaat gaccaaagga aaagtgtctt     420
   acatttttct ccacttactg catgtgcccc agataagaat tttaagtaaa agtccaccca     480
   gtgagacaaa caggcattca acattgaaag aatttgccag caaatcagcg attgaacaga     540
   gggctcaaga gaaaggacca ctagagggca gatccccatt catgaacagt ctctcactct     600
   tcatcccgaa gagtgactcg taaataccag gttatgggct gttattcata tacccactgt     660
   aggggtatgg gtctaaagaa gtacagaagg gaagtaactt ggtccaagtc ttgctgagaa     720
   agggttgatt gattctcttc atttttctac tcctcatcct ccctcttgac cttgcttttt     780
   taaccaagag gaggtccttg acttgtactc atcaccctcc agtcgtaccc agaccatggg     840
   cctcagtcat cggttgaact ggtgggccta ggaaggtcaa caacaccttc ttacttggtc     900
   catgtctgac ttctctgttc ctgtcccact gcccattctg attcacatcc cccaattcct     960
   gatcatgttt gtctacgtct ggcgatacct gaccagaaga cgtccttatc ttctatcctc    1020
   tccctttcct ttgagggaga agtcattttg gtaaaatttg atagccctga gtctccacct    1080
   tcaaggacca gcagtagacc tggagaagca tctccagcaa aaccagatta aagccatgga     1140
   aatacgacca caactctgat gacagtagta aaactatcct ctagaaagtg tggaagttta     1200
   catcaactct gctgtatatc acagtttggc aacccgtgac aagctgaaaa atttttgtag     1260
   ccctgaagct agtggtgtag agagagggtg cttggccaga ttggggcagg tagggggtca      1320
   gtcaccccac caccctgcct gcctgctgaa cctctctcag tttccactct ggatctcttc     1380
   catgcaggtc aacaagcatc atttgaacca catttgtgtg cctggcatgt gggtaatggg     1440
   aaggaggggg aagactagtt tgggggtaaa ggagatttca acattattag taacttttat     1500
   ttttataaaa atgatatgaa tcacttagat aattcaaagt tatattttta aagagtatga     1560
   cacagtttct gccttcaaac tgcttaccat ctggcagagg agacagaaat ataatcagat     1620
   tattttatta tattgtagag tctcctgata agtttcctaa gacttatgtt ttatttcttg     1680
   ttatttcaac aagaggtttc cataaattag ccccacatct atccttggct aaaaggccta     1740
   taataaatat ttataagtat ttttggcagc ctagctatca aggccgcatc ctaaaatcac     1800
   cccaattttc ttatgaggag acagccacct ctctactatg gactaaaaga atcctaatga     1860
   cacccaggac tttggtttct gtgctctgtg ttataggtat ttcgtggtgg acaatcaagc     1920
   tctattgtaa aagaaataaa ggacaatgaa tcatttcttg ccttgaggcc taaaaggaaa     1980
   tgttgtagaa ctctagagaa a                                              2001
<210> 74
   <211> 2001
   <212> DNA
   <213> Homo sapiens
<400> 74
   ccaagaatga gttttcaaaa cccgagttca gaagaaggat tctgagtggt tggttctcag       60
   atctctgagg aagcggaagg atgaccctag ggggagggcc accgcatgtt agaacccagc      120
   agggggctgt gacagacagg ctggtgctcg gagcttcatc tctttctgcc gttttcctct      180
   cagtttttgc tctggttatg tcatatttct cattttcaac accaacatga gcctttgtac      240
   agacattcct ccacctggaa ccccatttca gctcatctac ctgttgcttt gtatttattc      300
   ttcaaaactc tgccgtcttc cctgaccttc cggagccggt gtttgtggcg cctcctgctg      360
   gtcactgggt tcaggcagtt tggagctggg tggaaggaac gggtagtata ttaagtcttg      420
   ggccctggac cttgttggct gtcctctccc tgctgaccta aatgacaaag ggaggggtgt      480
   ccagtcagag tgtgagggct gtacccgtgg gcagctgagg gagtgcgagc cacaggccag      540
   gcactctcct tctcccatta gctcagccgt ggcatcggac ttgcagcttc attttgggct      600
   gccttagcca tgaagctcct tttgctgact ttgactgtgc tgctgctctt atcccagctg      660
   actccaggta acctgaacct ccttaaggaa ggggcagggc ttagagacta aggcctggtc      720
   agagaatccc aagggctaga aggatgggct gcaggttgtc atctagcacc acgtatagga      780
   ggcaggaggc gcctcaccag cagcaggtgc cagctgatag atgacaacta caacatccag 840
   agctgctctc tcagcggctc catccctcct cttcctgccc agctccaggg agcatgtggg 900
   ggtggcctag tgtgtgagga ggagtgggag gggtgagcaa gagaagcagg aaagaggaga 960
   ggcaggtatg gcccttttgc aaaatagtcc ccagaatgaa cgaagctttg tgaagatcac 1020
   agctacctta atgggagaga aagctcattt gtacttagct gtctctgtgc cagactctgc 1080
   gctggaagct aggaccacgg agataaatga catggtgcca ggggaactaa ctggaggaga 1140
   cagttaaata gcatttataa cccagggtga cacggctgtg gcagtgggaa accagggggc 1200
   tgggtacaca tggtccagcc caggctgagg gaaaggagag tcctcctgga gggatatcct 1260
   gagtcctaaa aagtgagtcg aaaatagaaa aggaaggaga ggggaaagca gaagggtgtc 1320
   ccaggcagag aggtcagcct atgcaaaggt tggatgcggg agagagcatg ggagtgtcgg 1380
   ggacctgcat aacatggagg ggtgggttag gagagaggcg ggggtaaagc tgaggaagca 1440
   ttcaagctcc aggtctcagt gggcctcaaa tgccaggtga gggacccatg ggaaggttta 1500
   aacagagaaa gaccctggtc agacctacca cccaccaatt ccagcatgag ccacctatcc 1560
   cttcctgctc atctggcaga agtttccacc caccctaagg ctgtcggcta acctttctac 1620
   agagtcagaa acgtgtgaat ttcctaaaca agctgacctc cgtccttgtg tcttcctgag 1680
   gccctaaaac accactcacc tggagtaggg aggaaagccc agaccccggg ttaaaacctg 1740
   ccccatcatt ttctgactct gtgagcccca acaagaagct cagctgctca gagtatcaat 1800
   ttcctcattt ctcaaactgc aataacatca agtgtgtaat aatatcaggt tgagggaaca 1860
   acattgaaca cactgaaatt ttggtctgca aaagaaatat ggcttctcta ttttctctaa 1920
   ctcaccaaac tttcccagga acttccatcc tgactgtggg agttagtggt gggaggagca 1980
   ggactacaga gataaatgac a 2001
<210> 75
   <211> 1920
   <212> DNA
   <213> Homo sapiens
<400> 75
   cccttggtag gtcctatctt atgctatcct tggtctctca tgctattaga aattccacat      60
   ttttttttcc cgatcctttc tggtgggcat ctctctagtg tgactcctct cttgaggttt      120
   tggctctgga gggctctttc caaccccaga acatgttcct agtcagagcc ttttctccca      180
   gtctctcaca tcactgccac atggtggttg cctggattca gtggagaagg tggctgcaaa      240
   ctctagttgg acactgacta cacagatggt ttcaaccaaa cccagactgt gcactgcctg      300
   cctccccata aagcctgtga ggatggggac catgactctt gctcacccct ggatcctgag      360
   ccccaggaac agtggcttgg acaaaatact ctagaccctg ctcagtggcc aaatcactgt      420
   gtggtataga aagagttctg tgccatccac gagacccact tccagacagt tttccagcac      480
   cacacttatt agccctgcct tgatggagac agcatctcct tggcatttaa gcagttattt 540
   ggcttcaaat gccctgagcg cccacacagt ggtcctttgc tcactaggtg atattatcac 600
   tcagtgtgga aggctctgcc cctcgttctg cttttgtgtt ttcagttaat ctcccccatc 660
   agtctggtag ctcccggtcc catttttgtc tttctgtctg tgtcctagaa gtatgactac 720
   ctttcccttt ggcctgggaa cttctgtggg caagttcttg cctttgactt ccctgtcagt 780
   gcccagcctg agttctgaac atgagtaggg gttcaggaag ggtgtgatcc aggacgacac 840
   ccaccttgtc gatgaaggcc gcgaatctgc tgttgaggga cttgatctgc tccttctcct 900
   cctgcttcac gcactgcgcg ttggggtcta tctccaggtt gaggggcgtg aggaggctct 960
   cgttgaccga cacggtggtg atgcatgggg ggctgggtcc gcacacgccc ccggagcggt 1020
   agccgaagct gcgtccgcag gagccggcgc ggaagccccc gcacacgctg tggctgccaa 1080
   agcccccggt gaggccgcgg tagcaggaga tgccgcggta gggggcggcg gtgatgcagc 1140
   agcggcttgg ccggggcccg caggcagaga cacagctgaa gtttccaggg cggaacccac 1200
   agcctatgga gttgaagcca caggtcatga cggaggttag gaggtgtctg aacagtggag 1260
   tagatggcag aggatggaac caagggcctg tgctccctgg ctgatggcag gcccttttat 1320
   gtgccagggg cagctggtgt taaaagaggg agcaaagaga caatagctga attttattgg 1380
   cttggcatca ccctggcctc ttaagctaga accctgcttg cctcttcagt gtggctgcat 1440
   caacaatccc tggccacggg ccctgtttca tcttcaaagc cctttataag cttcctcctt 1500
   gctcctgccc ctcccactgg cactgctgtg gagcggggag aggggttcct gaaagcatta 1560
   gttgcattct gcggtccaag cgaggggaat gggtgcagtg acctggggta aagcctgctc 1620
   tctgaaccct gttctgaaag gggcagagct aggctatggg ccctggaccc atggccaagc 1680
   cctggggtgg agtggctggc ctgtcccaag gagggggctg cctctgagac aacctcttgc 1740
   tgtaggcttt cctgtctttt tctcaaaggc gggttccttt cctcattgtc aagattggcc 1800
   gctcctatcc tattcctgct cagagcctgg tcaaggatgg gagtgggggt agagatttgg 1860
   ggtgggagga gggcatgttc tttgcacttt gggtattcct agtgcaggga caccatctgg 1920
<210> 76
   <211> 2001
   <212> DNA
   <213> Homo sapiens
<400> 76
   cattattatt cccattttac agaggaggaa actgaggtaa cctgatcatg accacactgc 60
   cccacagtag aggagccaga atccaaaccc aggcagcctg ccttgccacc agagccacgc 120
   ttttcaccct caagctgcag ttaagaagta tcacgccgtg gctaggccca cggcctctgg 180
   cttcacatcc cactagctct gccatcttta gctgtgtgac cttgggcaag ttacttgtct 240
   gtgcccgagg gtcctctgta aagtggggat agtagtagct gcttcactgg gttcccttca 300
   atgagttaat agctgtaaca tgtttagaat ggtgccctgc acatagtcac cagccagtac 360
   atatgggcca ttattcctga accctggata ggcacatgtc cttgaagagt gtagaattct 420
   tcaggattct gaaatgccag cctgtgggat cctggaatcc tccaatagca ttctaacagc 480
   tctcagctgc agaactgtac cctgttttct agagctggaa ggtgtgaggc tgcctgtcca 540
   cccagctcaa ccccacagta ggcaaatgag gtgatgtcaa agccttggct ggggcccagc 600
   cccggtaacc tcccactctc cctcccctgt gtgctccctc gctcagattc acacacacac 660
   tccaggccag ccgggcccag agggaaggtg agagggcaag gagtaaaggt ggctgggtgt 720
   gggtccgttg aagcgagccg cctccagccc tgttgaactg gtgggcccag ggactggagc 780
   gggattgaaa gggatcttgc tctcccttga agccttgagt tgcagcgatt tcagtgtctt 840
   ctctccctgt gtaagcctgt ctcggtgttt aggctgaact acagccaccc cctctcccgg 900
   gggtgtgcag gccagggact ggccaggcag ccatggctga cgagaagacc ttccggatcg 960
   gcttcattgt gctggggctt ttcctgctgg ccctcggtac gttcctcatg agccatgatc 1020
   ggccccaggt ctacggcacc ttctatgcca tgggcagcgt catggtgatc gggggcatca 1080
   tctggagcat gtgccagtgc taccccaagg taggtggtag tggggctggg tggggccagg 1140
   tcagctgggg ccaggagggc tggacactgt gcctgtatgc catgcctcac cccctatcct 1200
   ttagttgtct tttcattctt ctcattttga ctcggtcttc tctctgctct cctgagcgtc 1260
   tgtcccatcc atccatccat ccatccatcc atccatccat ccatccctta gcctaagggt 1320
   tgatcagaag gcccctggat ctggagccct gagccggaag ctatggtata gacacagcaa 1380
   acacaacaca ccatgtgctt gaaacaaaag ctaatgagcg ctgaccccct gccaggcact 1440
   cctgcgttca gggtgcagtt cagccctcat cctaagcctt ttggtgcttt ctgtagttcc 1500
   accttctgag cctttctgag gtccagaaag agaggctgca cagctaacga gcaaggtttg 1560
   gagctcagat gcgtgtgact tacacgtcac ccttcacctt gggtcttcta cctctgggtc 1620
   ccagagcgac tgagagctcc agggtaggtc aggtctaacc ttggtggcat gcactgggca 1680
   gtatccttta gagtcttcat gtattctcag ccttatgtgt aatttagtga tgggaagagc 1740
   actggtttgg gagtcctgag ttctagttca agtcctgttc caaactctct atgacctttg 1800
   tctttctgag cctcattttt ttcatctgca aatgagatcc ctgcccctat tttccacaga 1860
   gcttcacagg gtcattataa agatcagaga agagataaga tataggaata ttttgtaaac 1920
   tgtaaggtgt tatttataga tgaagggcta ccactgttac ccatactgat cctcccaaca 1980
   gctctgtgga atggaaatgc c 2001
<210> 77
   <211> 2001
   <212> DNA
   <213> Homo sapiens
<400> 77
   gtagcagagg agctttcaag catgaaagga gcctatggga gggagcaaag gcaccaaaag 60
   atccctccct ccccccaggc acccacagga agaaccagca ggccatgagg tcccacacca 120
   cccattctgt agtgtggttc cttgagagat cagggccaca gaagacttag aagcgcctga 180
   gcagcttgcc aggaagcagc tagggacagg tctgacccct acatcctgat gctatttctt 240
   ctggttagtg gaaatccctt caccaacacc ctcccagtct ctaagaccct ttcttcagac 300
   tctccccttc tctgccactg cagctggagg taagatgtgg ggaagaaagg ctagtctttt 360
   cttaaataaa tcccagcttt ggctcagtcc ccctcctgac agttgttgga accctcaggt 420
   ggagaccacc aaggactctg gggcaccagg gactagagga caaggtgaca gaaggcataa 480
   ggtacaggct acaggcagct ctggaaatgc catggtggga ggggagtccc agtgccatcc 540
   tgccctcccc ttcccccgct ccacactcac catgcagaca aatatggctg agctggtcca 600
   caagttccaa caggttcatg accttcaagc ccttgaggac aactttcaca gcctcctttt 660
   ctccatactt tgaaatcagt aattctgcca ggtccaccgg aatcaggccc tccaactccc 720
   ctctggccag tgggggctgg ccctcagaca gggtcatatc ccgtaagtag aactttaact 780
   tcttgaaatc gttctcctca aggtcactca aggcccagag caatgcctcc cggggctttc 840
   tggccttggc catggccatg gtgatctggg ggaaggatca agtccagacc agaagaccag 900
   tgacctggag aaagaggcaa aaggagaggt ccactgctga gaggcccacc ctgctttctg 960
   ggggcttggc caagttatag agcggaggcc agggagagac gagatacatg tgcaaagctc 1020
   caagaaagac aagcagatga aacgcagggg aattttccta gtcattcccc tctgcctctt 1080
   agtcaggtga cacgtgactg tgttggcaaa tgggtaggaa ggtatgtagg tgtgttaatc 1140
   tctcaatctc tctctctctc ccccaaactc tatctctgtc tctctctctc cccccactcc 1200
   cttcctcttt ccctctcaac acgttcttta ttaggctctg tatgcctcct tagacggatg 1260
   gattaagaaa aaaaagtcta agttttttat tttatttttt ttggagacag agtctacctc 1320
   tgttgcccag gctggggagc agtggcatga tctcggctca cctcaacctc cacatcccac 1380
   gttcaagcaa ttctcctgcc tcagcctccc aagtagctgg aattgcaggt gcccaccacc 1440
   atgcccagct cacttttgta tttttagtag agatggggtt tcaccatacg ggcccggcta 1500
   gactcgatct cctgacctca ggtgatctgc ccgccttggc ttcccaaagt gctgggatta 1560
   caggcatgag ctactgtgcc cggccaagtt ttaagtttta aaagtgagac tgtccctggc 1620
   tgtgtgaacc tatagaaagc tggagcacat ttgctggaac tgctgtggta tggcaagaag 1680
   gtaaaaaaaa tctacactca acccattggc ctcacatgtc agcctcctgg cctcagtcac 1740
   acagggattg caaagctaga agtctcctaa ttcaaataag gaaacaaatc cagataatga 1800
   taggaagagg aaccgccacc tgtgagtgct gactttgtgc caggtgattt acatatcctt 1860
   tttgatcatc tcagaaatcg tctcaggaac tgttccacat gagaaatttg gcagtcagag 1920
   aaaaatggtt tttcagagtt ttccagagct aggccaccgg gcaaagaggc aggatttaaa     1980
   ctggtctatg ccctgggtgg a                                               2001
<210> 78
   <211> 2001
   <212> DNA
   <213> Homo sapiens
<400> 78
   aaattaatag accataaatt ctgtaatgtg aatctttaga caaagactgc taaatctatc      60
   atctgatata tgaagtttta ttacaacaaa tgtttatata ttgagttaaa aattatctgt     120
   acccatacac acaacctgca tttacttaat ttaaaaggag tgtcaatatt aaaattgagg     180
   attttggcca ggtgcggtgg ctcacgccta taattccagc actttgggag gccgggtaga     240
   tcatttgagg tcacaagttt gagaccagcc tggccaacac ggtgaaaccc cgtcttgact     300
   aaaaatgcaa aaattagccg ggcatggtgg tgtgcccctg taatcccagc tactggggag     360
   gctgaggcgg gagaattgct tgaacccggg agacagaagc tgcagtgagc cgagatcgtg     420
   ccactgcact ccagcctggg caacagagcg agaacccatc tcaaaaaaaa aaaattgtta     480
   ttttgttgtt attgttgttt ttgaagacag agtctcactc tgttgcccag gctggagtgc     540
   ggtggcatga tcttggattg tgttcttcca gggttgcaga gagaagtgca acagcgtctc     600
   attgcaacct ctgcctccca ggttcaagca attctcccgc ctcagcctcc caagtagctg     660
   ggattacagg agtgcaccat cacacccagc taatttttgc atttttagta gagatggagg     720
   tttcaccatg ttggccaggc tggtctcgaa ctcctgacct caggtgatcc gcctgccttg     780
   gcttcccaaa gtgctgggat tacaggtgtg agccaccatg cccagcctaa gaataataac     840
   tttataacta tgaaaaatgt gttacatatt caccattttg ccagcattaa tctcaaagca     900
   taagttttac cttttgggga taaagagata gattgtgttt tagccatatg accaatgtag     960
   aaacaagcta aggttaactc ctcatctaat gaataggccc gataaaaaag ttggttgacc    1020
   acaacatgaa taaataaata accataagct tggaaatgct acctgtagta tttcagagtg    1080
   tcgatcacat ttgtagtcat cacttaaaga attaatgtct gccaatgtta tcatctaaaa    1140
   tgtatttaca gaggtcagaa gagctatgtc tgtcctcctc tatgcaaaga ggtgatgaca    1200
   tcaaggggta tgtcacaagc caaagtttag ttagcctaag ttagttagct ggtttgcagt    1260
   ttttatccac taagtctgat cttctgaccc ctcagtttta caactttcca ctctctgtgc    1320
   tgcatagcac ctctccactt ctgagttaat catttctttg ccattatctg gcaagaattc    1380
   ttatctcttt tgctgtcact ttataacagg gtcctcttat caacctgcat agagtcatgt    1440
   gatgagtcaa agcctatcaa aagtttccat tcttctgatt agaaaccatc atgaaactgg    1500
   atacatggtt tacagcaggt cactaatgtt ggaaaaagta cagagtccag ggaaagactt    1560
   gcttgtaact ttatgaattc tggatttttt tttttccttt gctttttctt aactttcact    1620
   aagggttact gtagtctgat gtgtccttcc caaggccacg aaatttgaca agctgcactt     1680
   ttcttttgct caatgatttc tgctttaagc caaagaactg cctataattt cactaagaat     1740
   gtcttctaat tcagatactg gggatttaca agagtcttta aagcacggac ttacacctat     1800
   tggtaagtag gagtttctct attgatcatc tatttattct gctactacat acatataatt     1860
   taatcttgtt gatggatttt gcattttaag tcatggcttt tcctattatg tttattaaaa     1920
   gcacacaata agcctatgta tatatatcac acatttatct ttcttgcagt tcaaaaaaag     1980
   caaatggata tttcaacata t                                               2001
<210> 79
   <211> 2001
   <212> DNA
   <213> Homo sapiens
<400> 79
   aatggaacta ataacatttt aactacatta aaattcatga atcaaagggc ccatttttga      60
   atagtaggaa gaattgttta tctaaataaa taaatacaag ttataacagt catgaagcat     120
   ttcttattgt atgtcttttg ttaggtattt cttagtttta aatacttggg ttagttccaa     180
   atctgtgatt tctcacctta ttcttaagat cctcaattgt taggtgatat ctgctatagt     240
   catgatcaag tccacggcaa gatccaggtc cgtatttttc ataccaaccc ttgatttttc     300
   tctctaattc agcatttgcc tcctccagag ctcgcacatt atccaggtag gatgccaagc     360
   ggtcattaag attttgcatg gtcaccttct catttccaga gaggagtccc ccttcgcttc     420
   cagcaaagcc aatacaagca gcatttccaa gggcaccacc agcatggctc ccaccaggaa     480
   cactgcccaa gccccctccc aaggcacagg aaaattcact tccagcaaca gagccaccac     540
   atgcactgct gcctgcaaag cctgcacctc cattgagggg tctgacagat ccagctccag     600
   accttaagca aacatgcctg gatccattag aaaattggag agacatggtg ttttgagaaa     660
   tgttcacctt gtctatgcaa aactgtaatg tcccaagaga acagaatatc atgcactgat     720
   aatttctttg ggtttttata tatcattatt ggggcgttcc tatttgagtt atacatgcca     780
   aaagcaaaat ggcatcactt agattagcat aaaattatgt ataattgggt gattttaaaa     840
   ataattaatg tctaacatcc ctcggtgttg ctttgggcta tttcttatct taaatataat     900
   agggtttgtt ctatattaag tttattaatt taacttctaa ttttgagtga gtaatccttt     960
   cctttcatct agaccctagg catatactgt cattatctgg cgccaggtat tatcaaattt    1020
   tttataaagt taagtaagat tatgatagag ttctgtgagg tacagtaggg tctcaattac    1080
   catggagggg tataaaggac tgctaattac agaaacagat attttatccc accagaaaaa    1140
   aatgtgttat ttagaaaaca gaggctagtg acatctctgt tcttaccatg attataacat    1200
   ctatttatta tcttggacaa tgacacaaat attgacctat tattttgttt aaaatagcat    1260
   ggttatgatg tttggtgctc tatgctaagt gctaaaatga aagacatagt gttaactaaa    1320
   tgcagaagtg aaaaatgcgt gattacaatc tttaagaaat ctattgtcag gtccagacag 1380
   gggctggatc atggttagct ttctgtgctg gtctaaagag tttgtaatga atcctaagta 1440
   aaaagcagag ggcaaaatga tcaggttgaa atgaaacaag gtggtcaatt acaaggttgg 1500
   aataatggtc caggtgagaa ggatgaaggc agtggggatg cagagaagat ggctttgaga 1560
   gcatatggta atatgattga atttggagag tagggaataa ggcagaattg gggataactc 1620
   ttaggtaatt aattttagat tctgggaact cataatattc actgagatgg ggaatagagg 1680
   aggaggaaga cactgagttt aggtttggac caactggatt tgaggggttg ccagaatgtt 1740
   ccagatgaag gtgactgata gcaagaatga ctgtttagaa tatgtatact gaactggaaa 1800
   aatacttgac tagcaatgag taaaagaaaa ttggagggtc cagctctcat ttataatgat 1860
   gttcaactgc atatttggaa gtaatcctgg tgcaggaata gaaaggatga tgatcagatt 1920
   gatgtgggtt ttgggatttg aagacaggtc cagaagaagg aaatagtggc aaagaaattt 1980
   ctggtgttct tgagaaaaca a 2001
<210> 80
   <211> 2001
   <212> DNA
   <213> Homo sapiens
<400> 80
   ttttcatgca tgccacgccc gccgccccca tccagtcagg agggttggcc taaaagctta      60
   gtctgtatcg aaacacaaag atgggatgtt gtgtccatcc tcctcccccg atttccttca     120
   cagctgctcc ctgaagaaga aatgagatat tgttttcgcg cgcgcgtgtg tgtgtgtgtg     180
   tgtgtgtgtg ggtatgtgtg cgagagagag agagagtatg tgtgagagaa agataaagta     240
   tattttcatc caaaacattt ttttttttgc tatgtggatt ttctaattta atttttttgg     300
   tcagaatgca cttttaagtt ccagcaggac gtgttgcaac atgttttcaa acatctggta     360
   tttatttttc atttttgtgt aaatgaaaaa tcaacaagct catttcctga agtggaacat     420
   ggagttcctg actccactaa tgggagtcag gtaatggcgc gagggttatg agtcaaagct     480
   ggctttctcc aggatcctgg gcagccccct aacttctctg agccacagtc tcctcgttgt     540
   aagtaggggc tgtcattctt tctccaccag agccagacgt gtaggggaga taccagttct     600
   ctaacaactg gggctaaaag ttgagaatgc agaagacatc ttctgtcgct ctgttctctg     660
   tcccttttat gacccttgca gcgagaagtt caagacagtc tgtcactgct tgggggtggg     720
   ggccggggtg gtggggagtg tggacttaaa cccaggggtg cttttggtcc agccttatct     780
   ctcctggtgg aatgaacaac tttgactctt gtgagtgaga ttctatgtct ttgtgatagg     840
   caggaggtat gtttagagac atttagataa ggcagtgcct tcgtagtaga cttagactcc     900
   taaatgaaat tcattctaaa atatgtaagc aacagagata catttacagc actgagtcta     960
   taaacatgaa aacaacaaca cagatccgta acatcccaaa cgttgtgagc gctttcttta    1020
   agatgttctt tgccttttca gaaacataag aaattcctac taccacccag ggcctgcctc 1080
   tcgctgacct cctgtgctgc cctcccctaa gagctctgtg tagctccctt ccccactcca 1140
   ggtccctgat cagatgacac ttttccctaa aaagcttcct gacctttcaa aaatagcact 1200
   cttgtcactg tccctttccc tgcttcatta tatctttacc tgaaattctc tcctttattt 1260
   atacattatc ctgcttagta tgttttcccc aggaggatag gagcttgagg cgtgccagga 1320
   gtttgtctct ttgactctgt gttgtgcccc agtggctaag cacatactaa gtgctcagta 1380
   aatacttata aattaaatgg atgctatcca aagaacttaa tgagtttcta agatatacaa 1440
   aaggccccag aggtaatatt cacgcgattt tgatttcaat tgtatttctg gagaagcact 1500
   agcctgtatg tttctgtctt cttcacctgt atcacatttg ccaccaagaa ttgcctgtca 1560
   ttgaagaaat acacagttag cctcttttca gcctattaca aaaaattctc tctcctctaa 1620
   gcagttgcac agttttccac aaagcactgt tgcctaagtt tccaaatatg tatgttggag 1680
   ttggagatat ctatgtattt tagccctcag cactgaagtc ggcaaccatg gagctgaagg 1740
   tccaggtcta ggagtcacag cgggagaaaa gccacacgat cttcctaggt ctcagttcat 1800
   tttttctgta aatattggta agtgatatat tccttaggga aatcctctct tcacatgttt 1860
   ctggaatgta tagccagtaa caaattctta agcagcataa aacaaatcag acctatcttc 1920
   taagcaagtg tttattttta agtcccaaaa taaatacaca tttgttcaaa acaattaaaa 1980
   tagcaccaaa atgcaggact c 2001
<210> 81
   <211> 2001
   <212> DNA
   <213> Homo sapiens
<400> 81
   ggtagagttc cagaaccggg gagatagcag caaagaccct cccagggttt ccctcatgaa        60
   cctcagttct tgggaggctc ccccttggac agtggccttt atctgttccc agggaagctg       120
   tggcttgggc ctggagctgg aggtggaatg atctttctgg gagcagagag gcccagagag       180
   tgtgcatctg gggcccctgg ggactttgtg ctgaaaggag agaaagttag tgagatttgg       240
   gtcacctggg ggcttaccca ctacaaagag agtcacaaga gagaagacca aggtcacagc       300
   cataaatgcc ggggtagcct gaacgagcct cggtatcttg ggggctgcag gagccattgc       360
   cacagagtcc acctctgcag gggagaaggc agtgtccaag gtgagagggt gctgtgcaag       420
   gtaagagggt gctgtgcagg ctgagacaca gcctctctga ttctgtttcc tgggttgtct       480
   aatggggatg ataactcctg ccatgcttat catgcaggtg gttatggaga aggaagacaa       540
   aacactttgg gctgttcacc agagctagat gtggatatgg tagcaacaca gagctgagga       600
   gctctgagac cgagaccacc gatctccaaa tctcaaggag gctttggtgg tgcacagggc       660
   acagaacctg gcacagagca cagagcaaca aaagtgtagt ttgaatgaaa gaagaaagga       720
   agggagggag gcctcacagg accagctcag tgtgcagagc cgggattcca agccaggacc      780
   atctgaattc aaagttcatc ccctttctct tgccaggtct gcatccccaa ggacaagggt      840
   ctggggagag caataagaca gcaataagct gccttatggc agagggccaa aggacaggca      900
   ggctactccc tcactgggca agagctgggg ccccagtttt ctcaccttgg gggtgcaggg      960
   agacacactg gttatctgtg cagaagcgga ctgcctcacc gtccatctct gcttccttga     1020
   tcctccagca ctgctcccag ccactggctc ctggaagggc cgtcccgtgg accaatggca     1080
   gtggaagcaa agctgagaca cccacactta tatgctcctg accaccctcc cagctcttcc     1140
   tccctccctg cctgactctg cagaaacccg ccccagtggg cccctcccct agaggctccc     1200
   agatacccat ctgccctatt ttcttcctct tcccattagc accttcccgt cagttctcag     1260
   ccccatcatc ccagcctctg ccgcatccag cataacccca aacacacctc ttcttctctg     1320
   ggggctggga ctggcaccta ggaagggggt ctcagctttg cacagaaaac atccttatga     1380
   ctatggctga cctttaattc tagcctcaga acctcttaag taaaaggagt caggtaaaag     1440
   aggggccagg tcagtgcggc cgatatttgc aggtcaaagg aggaagcggg gtgaccagcc     1500
   acttggggac tcacaactga attatagtat acaggaggat gtgcggaggt tatataaaaa     1560
   tactacatga ttttgtacaa gggacttgag catctgtgga ctttggtacc catgggagtc     1620
   ctgaaacaaa tcccctgcag ataccaaggg acaactctgt aaaaaatata tctggtcttc     1680
   atctcagttc ctgacacaga actcctaaaa cgcttagagt ttcctgggtg acaggagtgt     1740
   tttttgttat tcataataag ccccttagga ctacacttgg gtttatgcta atgaggtgac     1800
   acagggtgga cccttagata atttcaggct ggaggctggt cccagaaaga ccaaacatgg     1860
   gattagactt tggaagactg ggactttcag ccacccactc ccattcccta ctcccagacc     1920
   tccaggtatg ggagatggcc tggggtctgg gctataacga ctcttaaaaa atgagattca     1980
   aggagcttag ggattggtga t                                               2001
<210> 82
   <211> 2001
   <212> DNA
   <213> Homo sapiens
<400> 82
   cagtgccctg gagccggttg ggttgagcgc ccctgcccgg ggaaggtagc ctgcgggctc       60
   ttgccccgag cccgcggagc aggaggtctc ttttccaagc gcactcacat tattcatgca      120
   aagttaatcc ccgccgcgtc acggccgccc gcccgaggat gtgcgccttc cttggccaag      180
   ccccgcagtc tccaccctcc tccgccccct cctcccggcg gcggggtccc ggggagcgcc      240
   ggcgactggg cgtcgcgcga gtcctgcaaa atgtcaactc cttgggcgaa gagaggcggc      300
   cgcagccagg gccgcgccgc acctccccgg tctctccact tttataggcg taggaagcta      360
   ctgcggctgg ggcaccagag tccccgcctc tccgggtccc ccgcgtgccc ggcccgcccc      420
   ggcccgcttc ccgggcgctg tcttactccg ggcccggggc gcctgctccg cgccgcgtct 480
   gcgaaccggt gacctggttt cccctccagc cctcacggct gtccgacttg cgcggcggtg 540
   gcggcggcgg ccaagagcag gcaaacccgg ctccgccagg ggcgcagcga ggaaatggcc 600
   tcctggcgca caccccgccg ccgccgccag ccatcgccac cgccccgggc tgcccagccg 660
   gtacagacgc cccctgccag gctccgggcc agcgctccga cgcgctcaca gcgcccggct 720
   tgccgacccc gaccccgacc ccgagtccta cgcactcgag gtccccgggc cggccgcctc 780
   cgcacctgcc tccccaggtg tgggcgctcc ccctcctcct aatcccggcc gtgccctccc 840
   agggttggtg acggtgctgt gggtttggtg gccgcgcgtc cacccacccg attgcctgcg 900
   cgcataggtg cgggtttggg cgtcattact ctagttggag aagaggaaag aatggggaac 960
   gagtgacacc gggaccggag ggcgagtctt ccaggagcac gtctcggcct tctttgcccg 1020
   gcccgaccgg cccgacccgt gccgcagcgc tcctccctcc gctcctaaac cacttgtggc 1080
   gcgtgtcggg aatacagggc gctctggctc ttcttggggc ctccgctggt ccgaccatct 1140
   ggcagctctc ttaaccacca cggccacccc acgaatggaa gtttgggacg tgcctgggtt 1200
   gcccttaagg aggacacccc cgcccccacc gccattatgc atatcgtggg agaggcgcgt 1260
   acagggctga gacactcctc aggtcccaga acggccgccc tattaatttg tctttgccct 1320
   ggaaatgtat agataaaacc aggaagacaa aaataatgta tactcttcaa gagatctttc 1380
   ccgggcaaat ataggtctct caaatgcccc cgaccttaag tcctggagac caggaagaga 1440
   cttgctttct gagctagaag gatacttggg aaataccgag tcctcactca gctaaaaggt 1500
   agtcaataag gaactatagt tgcaacaaaa ctactttgac aacagagtac aattaatttc 1560
   ttatagacag tattaaaata attccgacat cataatttaa tattcacatc tattcgtgta 1620
   acacgcagta taatagagtt atgacgatat ggatcctcag ctgcctgaca atgtataggt 1680
   tatttattgg aaggttctct agatttccag ccagtgacat gtatgtttta ttctcgcttc 1740
   ttctgagcgt cccagggaaa ggggtgagag gccccccacc cctgctgaca gcattaacaa 1800
   ccaccccgct ggataagcag ggtttcactg atgcactttt aagaaagttg caggccccag 1860
   agaccccatt tccctttggt cctcggtgag aagtccgtgc tttcttctct actcttaata 1920
   agtctgcacc ataggacttg caggcttctg tagttggaga gctgttctcc tttgcaggga 1980
   agttcctggg ctttgctcca a 2001
<210> 83
   <211> 2001
   <212> DNA
   <213> Homo sapiens
<400> 83
   gtacacactc acatgcatac acacacatat gctcacacac atgtacacac acacatatac       60
   acaccacaca ctaacatacg tacaggcgca cacaccgcta cacacacaca catgtacaca      120
   cacacacatg cacacacttt ggatcacagg ctcaatttct agaccacacc tggacctcgt      180
   taaatgctgc gggagttaag tgatggaaac tgacccttcc acctgtatgg gtctgcacgt      240
   cattccctct gctgcctcaa accggcagcc atcctgtctc ctcagcagac tgggacctcc      300
   ccaagggagg acagggctgg aggcttctct gggccgtctc aggcctcacc caccttgtcg      360
   atgaaggccg cgaacctgct gttgagggac ttgatctgct ccttctcctc ctgcttcacg      420
   cactgtgcgt tggggtcgat ctccaggttg aggggcgtga ggaggctctc gttgaccgac      480
   acggtagtga tgcatggggg gctgggtccg cacacgcccc cggagcggta gccgaagctg      540
   cgtccgcagg agccggctcg gaagccacct acagctatcc ggggcccgca ggagcccagg      600
   ttgcagaggc tgcggctgcc gaagcccgtc agccctcggt agcaggacac ccctcggtag      660
   ggggcggcgc tgatgcagca gcggttgcca gttttggggg ccacagctga gcaggagctg      720
   aagttcctgg tgaccccgca tcctgagctg atcctgtagg agcggcacga catcgtgtga      780
   ggctgagcag agtctgagag gcagcggaag gtggtgcggg cggcagagtg cgaggctcag      840
   gatccttctg ctctctctga tcctccctgg tccttttatt ggtggggaga gctggggttg      900
   gctgcataaa aggagtggaa agccatttta tgttgtttat gggcttgggg agtttgccag      960
   caactcccca cggactcatc ttaaaggtga gctcagcagc gactctgggg ctccctgaag     1020
   tcttgaagat gttattaagg agactgcatt tgaacttttc atcttcaaag ctcgtttaca     1080
   tacattaagt aattaatttt ctcacagtct ttctgtgaga tggcctcaac ccacacttgg     1140
   agacggattt gaactccaca acagcctggg ggtggctggg tttgggatac aggcaggagc     1200
   cattgtctca agggagaggt tggagggtcc tgggttaggt gagacttggg ggatcattgc     1260
   attcatctat ctggttccac agaggacaca ttctcaagta aatcattgca tttcaggggc     1320
   caataggtca ggcgaatccc accccatcta ggcagcattg cttggaaggt ggttcaggac     1380
   cagagcggtc attttgacca tgcccgtgcc aggataaaag gctggggcag atgatctctt     1440
   ggaaccttct ggattccagc ccagaaggcg ctggtccaga gctttcactt ctcacccagc     1500
   aaaagtgcaa accacagaac tctcagtggc cacaataatg gctacttgcg gattatgggg     1560
   agactccctc tagccacttt cttccattcg gtcaaaagga ttggagaact caaccttggg     1620
   ataacctggg ggctataaat ttgctgtggg cacctttgct ttgctcttta agaagaaaca     1680
   ggtcatgtga gtctcccttc ccagaccacc agctacaaga ggtattcctt ctgttggcca     1740
   gcccttgaat tgtcatccct agaggagtgt ttagagactg cattgcacaa agctggaagt     1800
   gggatagtgg tttattcttg tctaaggtgg ctatgacttc tggctcctgg gggttctggg     1860
   agccccatcc ttctagtttc tgcttccctg tgtgtccttg ggacacttat taggataaca     1920
   agtgctattc ttttaatgtt tatggagtac aaaccttggg gatgtgggtc tgcccccagg    1980
   aaggttagcc agacacccaa g                                               2001
<210> 84
   <211> 2001
   <212> DNA
   <213> Homo sapiens
<400> 84
   tgccaggcac tgctccaaat aagtgtttga agagtgtttc ttcatttcat tctcacaaca 60
   agcctgctac tatacacatc tttcatatga gaaaacagaa gcacagagac attaagtagc 120
   atgcccaggt gacacagttt gtaagtggta aatctgggac ctgaagcaga gaatctggct 180
   ccaaagcctg tcctcttact accaagtgac gtttgtgcct ggcacagaga aggtgctcat 240
   taaatgtttg ctgattcaaa tcattttacc aagaaattaa acatgcttga gcttattgtt 300
   ctggacttaa tgccctgact actcttaaaa agcaaacaaa aatgcttaca agtacaccac 360
   aatgaaacaa aacctggaat tatcagaaca tggtaaatgc tttattagat tttctgattg 420
   accaagggaa attactctaa gctttcaatg tcttcttttc ctacaaggga actaaatttc 480
   caggaagttc tgtgacaaag ccacagagct gagaagtaaa ttagtctgtg ctggacctag 540
   gatttccctt gttcttccac ctctgtcccc agcaattgct ttgctgccta ttctcagggg 600
   aaggcattgc actagccatg catgctccct ggagaatgac aacttgaact gcaaaatctc 660
   aaaggttgtg cttcttttgt tcttcctctc atctagttga taggcagaga acaattctga 720
   ttgaatattc tatttaatta tgtttaatca tactaagaat caaccaatgt taattattaa 780
   aaagggagaa aatggccaaa acatcatgta caaccatcct gactttaggt atgaagggtc 840
   aacattcatt cagctctgag ccaggcagtt aaacagcaaa cagttttaat aaaggacaat 900
   taaataagtc aggaaatcca ccagtctgtt ctccaggttc actcaccttg gtgtggagga 960
   cgatgggcag aggtagtaaa accaacacag tgaaaaccac gaagagaaat cggcgataaa 1020
   ccagaatgta actgaagaat ttcattgtcc tgagcaggtg gcttcaatag tgtcctccaa 1080
   ataaagcaga tgttaaataa ccaacttgga ttaatatttt tccagcctgt caccttcact 1140
   cactgcccta gaccgaccaa cacaagttaa aattaaatct cattttttat tgttttacta 1200
   tcacaattaa catgaagaga taggcccaag acttttcatg agaaatcaaa gaccatttgc 1260
   tcctctgggt tttcaaggaa aaactgttct ttccctgctt tctttgatca cattcactga 1320
   aatacagact ggacaagcct aggagaaggc aaaaccatga agctggttgt ttgttcacaa 1380
   tgaggcactt ccaacccttt tgatgtcctc tgacttccag actccctaac tggagtagtt 1440
   accacattgt catggtgcca tgagtgagtc tccacaggca gcctccatgg ccctttctaa 1500
   ggaagtatgt tttcagtgtg taaattgaaa tagcaactaa tagttggaat ttcagtgtat 1560
   gacagtgaaa gggaaacttt tttgtttaga gatggatttt taacggactt cttggacaat 1620
   gtattgcaag ggtcttctac aaaatttata gcaaatgtcc ctgttggtta gaagcaaagt 1680
   tttgtgtatt tcttggaccc tattgtctct tttgatagaa aaccaaaata accatgaagt 1740
   gttatataaa ccctctattt taagaccttt gaaggcttga tttgatactg ttgtgctgat 1800
   acagactaca taattatcag ggaatctgca taaagatgag tgattaggga cattcaagtg     1860
   gtgtgtgatg tgtgtgtgtg tatggtttgt gcacatgagc acatgggaag ttggagactg     1920
   ttccaacagt gatatggagg atgtattttg aaaatcttca gggataagga ccctggtttt     1980
   tttcacgttg tgttgtaaat t                                               2001
<210> 85
   <211> 2001
   <212> DNA
   <213> Homo sapiens
<400> 85
   ggagaattgc ttgaaccggg aggcagaggt ttcagtgagc caagatcatg ccactgcatt      60
   ccagcctggg tgacagagca agactccatc tcaaaaaaaa ttaaaagaca aaatgtggac     120
   acacacatgg ccccagcaag caagattgat tactaacttc gtggtggcgt gcacatgcac     180
   acgatgttcc acaagctcat catctattcg agtcgagagt attcagttat cagcctcaga     240
   aatccagaag gaacctcaca tcccaggagc ctctggcatt tcagggcctt tcactccaag     300
   gagccgcctc caccgggcct tccccagcag gatgtatatg gccctgtctg tgtgctagga     360
   ggagaagggc aggcccgtgt gctgtctggt gctaggtgtg agaccacagc ctttctggaa     420
   aggctttgta ctgtgctgct ggtgtgcaga ccccgtgcgt ggtaggggct tcaagaatac     480
   gcatcaaatg aacccgaacc caaggacgct tgctcacctg tgtcatctgg gggtctcctt     540
   ccacaggaag cacatctggg cagagtgaag gctcccaggg ctcaggtcca gaaagagctg     600
   gaggctcctg agagcaaggg gccctactcg gccatgagcc cagaacaatg cgagcttcac     660
   acaccgcagg tgtgtgcgtg tgggagcctc ttcctgaagc cttggggtca ggggccgggg     720
   cccagcaggc tgggtttatt gtccattgtc tcaggccggg acaggaagca ctttctgttc     780
   ccaattgctc gggtggatta atttacagtg gggagctcag agtcgcccac ggcacctgtt     840
   ttcccctctg aggggccagc ttccagcttg gagggtgttt caggaggggt ctcctctgca     900
   gagacgtggc ctcctccaga gacccttggc cacagcccct gcccgctccg tgctgggccg     960
   aggccaaggc cctggcagcc ctgcctgctt gcccgggtca cgctgcccac ccacggggaa    1020
   agctgagccc tgtggagtgc acaccatgct gtcagcagtg agccatggtg aggcagcctc    1080
   agcaatgtgt cttctctttg gttgggaatc cgctctctct tccaggttca ggggcaggtg    1140
   atgtatgctt gttttcattt ggtgaagatc gtgagtccca ttttacagat aaggacgctg    1200
   aggctcagca aggttgacta cacaggtcgc gaatgaagtg gtgggatgca aacccgagtc    1260
   tgctccaggt cccagagctc tcaacctcaa atgtctcagg agccaacgaa agcttgtgac    1320
   tgagtgtcac cagctggtgt acaaaggggg agtggcagga ggtggaggag aacccagggg    1380
   tgcaggcagg ggggaccctg tgccatctcc aagggcagca ctgctctacc cagcctgcca    1440
   tgccttgaag taaggtggac cttgtgtggc cacatttttc cttgttagta actcagaaga    1500
   tacatttcta gacacggggt aggtcacaaa agcctgtctg caggcggcac attgctttcc 1560
   acctctgctc tgtgccacca tgccaacacc aacagtgagc acctgctata tgcaggtgct 1620
   gcccgggcac ctccacaacg gggacccatc ctgtgtcttc aggccccccc aggcagcagg 1680
   cactgtgctt cctgtggaca cacaggggac tgaggctcaa aagagcaggc tcctgaccct 1740
   gggcaccagg ctggtctcgg cttgctcact ccacccctgc atccctgcat cggtttttca 1800
   tcaccactaa tgcaggaggc tgaggaagaa ttctggagtc tggtggagga tggggaagct 1860
   gaccctattt ctataggtac ctccctcatt tttgtaggat ggctccaagt cagccaccga 1920
   gactgccccc tctgcactaa ccattagcca gcactaccag gattggccag cttgctctgt 1980
   gccacttgtt ttatttattt g 2001
<210> 86
   <211> 2001
   <212> DNA
   <213> Homo sapiens
<400> 86
   gaagtggccc tccccaagat gttcattgga gttgccatgg tgatcactat acacagggct      60
   ctgttacctc agagggctga taggcccacc cgtagggaaa agaggccagg agaaagccag     120
   gattgaggcc aggaaaaggc aggaggggct tcaattattg ttcactctct catccactct     180
   agaatgtcag ttgagccccc actacatgcc aggtgtgcca ggcacagggg gctcagatgc     240
   taccaagtgc aaaagatgtt cttgggagct caaagcacta agtagggaga agaactagct     300
   ctgtgtagga aaaaggagga aggacttatg aaagagccat ttgagttgga tcttgaagga     360
   tgcctaggaa cttaacagtt ttgaagggat gagaatatta aaggcatgga tgtgtataac     420
   agcataaatg taggtcgagg tcaatgggga aggagaaaga tgcatctgga gctgtctgat     480
   ttggacaccc agggatcagg gacctgggag taaaactaaa ggaagggtcc tctctcctat     540
   tcctaaacaa caagggctac agaaggagat caacaaaatt aagtgacttc ttttctgcag     600
   agaagaagga ggcagaagtc attggtgcca gcactggccc attcctgtct ggctgttgct     660
   cagcctggac aaagagtggg tatagtgaac gcctcctgca agcccagcct ttcaggattt     720
   ccccaggaaa cagagagtgc tgtggttaaa cttggagtcc ccagaatctg ggcctgtcac     780
   ttattagata agagactggc caagtccttt agctgaactt ccttcacatg tgaaacagat     840
   atcacccaac actaaggata agggtgatgg gcgaataaaa caattttttg caaaatgtag      900
   cacattgcct tgcacagaga gagacatcac aaaactctaa gctacttccg caagcaaccc     960
   atcagcaagt ccttcgaagt acactagatt tggatgcctt cgtccacctc cacaactccc    1020
   cctactccct ggtccaagcc accctcatct cttgcctaag tattatctat tgctgcacga    1080
   caatcttgcc ataaacttcg aagcttaaca tacaactgtt atctcacagt ttctttgggt    1140
   cgggtgtcta ctcatggctt agctgagttc tctgcattgg ggtcttagca ggctacaatc    1200
   caagtattgg ccagggctgc agtctcatct gaggttccac tagggaagaa tatgcttcca 1260
   agctcatgtg gctgtgggag gattcagttc cttgcagtct gttagactga gggtctcagg 1320
   ctctctctgg ctgtcagcca gaggccatcc tcagttcctt gctatgtggg ccccccaaca 1380
   ccaacatgac actgacttca tcaaagccag caaagaggga gtccccttgc aagatggggc 1440
   attacaatgt tgtgtaccat agtcacagaa gtggcatcca tcacctttgc cgtaggctgt 1500
   tggttaaaag caagtcacgg gtcccacccg cagtgaaagg gagagggtca cacggggcat 1560
   gaacacgagg gggcagataa tatggggacc accctggagt ccaccatatc ctctgacagt 1620
   cttctcactc aacagcccag caatcctgtt aagacctata ttagatcata tgtcacccct 1680
   ctaaacaaaa ccttccagct caaccacagt aaaagctgac atcctcccat ttatctgcaa 1740
   ggccctaccc aacctgtcca ccatttttct ctgacctctg ctcctctcac tttcccaagt 1800
   gctccctggg tccctgccat gccaggctct gctgtttttt gggcacacca agcacagtcc 1860
   ctcctcgggg cctttgcacg ggctgttccc tctcctctcc tgcaacttcc ttcccccaca 1920
   tgcctacatg gcttgcctct tcacctcctt caagactctg ctcaccttcc cagaccacct 1980
   tttcttaaaa agcatcccag c 2001
<210> 87
   <211> 2001
   <212> DNA
   <213> Homo sapiens
<400> 87
   gccactgcac cccagcctgg gcgagagtaa gactctgtct cagaaaagaa aaaaaaacaa      60
   agaaacttta aaatagttta atgtcaatgc tactttcaat cttctctaat tctaagtcct     120
   acacctctgg tgaatttaat acttttaaaa agctgagtta tgaaacctgt tttatttttt     180
   atctttttct attaaagtgc taaaaccttt cagtctattc acagtagcag attttcaaag     240
   gagatcttat gcttggacaa ccaaatgcgc tgatgctaca ttataacttt tcatactttg     300
   cagattttca ttcattataa aaataataca aagcacttaa tcttttgcta tatgtactta     360
   ttctttccat ttataaaact aaagaaacat aaatctctta cctatgtttc tatctgtatt     420
   aaagctgtta gatctacaaa ttttttaaaa gaaaagaaaa acattactca ttagtataat      480
   cataaaaatt aacatgtaag atgatataag tgcaattttt aatggcatac tacagttaag     540
   tccttcctcc aggtaggaag aacaaagaaa ttactcatta caaagtctag taaatccaag     600
   tctacttaga gaaaacagaa gtacttactt cgctcaaatt accacattta cccaaattta     660
   agggagttgt tttttccaaa aatattaacc aagagagaac agtgtccacc aaagtctctt     720
   gtcaattatt ttgctgacat tagaagacac agcctcaata tataaatata ttaattcatc     780
   attcagtaaa tacatactaa gctcttattt accaggcaca gttctaggta ataggattat     840
   agagataaac agaacaaagt tccaacctac ataaatctaa tgaggaagac agaaaacaat     900
   gatatataac agatcatgat ggttttcagt gctataaaaa gagacataag agtatagagg     960
   taaaaaaaaa aaacagagaa acctcaatca taaggaaata cgtgaagaaa tgtagaatga    1020
   attatgcagc ttactgggga aagtgagaga acagcaaatg cagaggccct gaagcaggag    1080
   cattcatgga atattcaagg tacaaggatg gatgttcact caatgctcct ttgaaatgat    1140
   atagaggtca agagatggaa tcaaagagga ggcaaagaaa ttaacagtca ttattcctgg    1200
   gggcatgacc tcataatttt aagtgctgga tgtcttttaa agagtactta tgaaaacact    1260
   gtagtgaaag tatgtatcac agtgatcata aatttagagc cacaaaaaat gaaagccaca    1320
   actgtctgga tactatgtca attggtacct gtggctatag atggtttcca aggattattc    1380
   acctacataa aaggaccgta aatggaagag aagataattt gatctggaaa actgacaaag    1440
   aggtcaacct caaaaatgta catatatgtt tcatgatata tggttttaga acagcagtat    1500
   ttctaaatta atagatatca tacatgactt caaatatgta tacacaccca aagtaaaaaa    1560
   tgagcagaca tttgaccatc tcacctacat ccctataaat atacttattc catttggcta    1620
   aagcattttt agaatcaaca catggggaaa aaaaatggta actgcttcct atttggtcac    1680
   cttatactag agcttttaag aaatactcag taacacaaga acagaaaacc aaaaaaaagg    1740
   aaagaaatca tcaggccaag tgtggcagct catgcctata atcctagtgc tctggaatac    1800
   caaggcagga gaactgcttg aggccaggag tttgagacca gcctggccaa cacagcaaga    1860
   cccatttcca tgaaaactaa aaataaacca gacatgatgg tgcatgcctg tagtcccagc    1920
   tacttgggag gatcgcttgc acacaggggt ttgaggctgc agcaagctat gactgcaccg    1980
   ctttactcac tacagcctgg g                                              2001
<210> 88
   <211> 2001
   <212> DNA
   <213> Homo sapiens
<400> 88
   tggggggcat ctgactgctt ccccccaccc tctgccaggc attgctggag aatgttaaga 60
   cggcgatgga gatgccatca accccaccct gcagagcatc accagacacc accagaccaa 120
   attcactttc cagccccttc atgttgaacc tgaaacttga gctagtgtct tgggagaaaa 180
   gggggaaatc tctacgaggt acccatcctt ctgcacctta ggtctgaggt gcttggcccc 240
   ctaggaagcc ctacatgaat gggacagaag gtccttaaca acactggaga tgaagcagcc 300
   gatgctgttt tggacaaatg aaacagcgtc ccctaaccag ccctttctat ctcattgttc 360
   tgacttggac acgccatggc tcaccgctcc caaagtcccc actatgtctc cctagctgag 420
   gaaataaaag cagagagggg tgatgaaaca gtgacgatcc tggggaaaca gctgaggagg 480
   ggagggaggg ggaagaagcc actaaaaaag tgaaatgtgc ttgggagaat cggcctgcct 540
   gcagggtaga tgccctttct ctctgctggc cagctctgcc cctcagtgag aaactttaca 600
   tattgctaag atgcctggcc aatgaaacag ttccagagac tttatgtccc ccagtagaaa      660
   tatgaataga aatcaccctg tgggcaatgg tcccatttta aaatatgctg tcccattgtc      720
   ccctagagcc tactttaact tgtcagacca tgtattccac ttcatatgca agaggcatgc      780
   actgagccca taggtggcta ggcaaacacc caatagctcc ctgaaatggc ttcattatgc      840
   agcctcgaca gccaccccaa ccctcccact ctcacactga aacacccaga cctagagata      900
   gctagacaca cccagacacc cgccaagccc ctcacataca gatatgtgca caatgataca      960
   cagcaaacgt acacagagtt cagtacacac aaagagctca cgcccacgtg cacacacccc     1020
   tcagttggga cagagttgac caccaccacc tttctcccaa acacatggct tttgaactgc     1080
   ctttccttgg atccagttca aggggatgga ggagcagtga gagtcagccg cccttccact     1140
   ccaatttccc agcacctccc ttatctctgc ctcacaagtc acccagcccc cctctcttcc     1200
   ttccttgtgc ttgaagaatc tctccttgct ggaaagcccc ctgttttctc aatctccctt     1260
   tccacttcgg taaaatctct acttgctgga aagccccctg ttttctcaat ctccctttcc     1320
   acttcggtaa aatgcccact ttctggtccc cacctttttc ctgagtgtag tcccaaccag     1380
   ccaaatccaa cctcaaaaca ggaagaccca aggccagtga cccccatagg cctgaggctt     1440
   gtgcaggcag tgggcgtggg gtaaggcttc ctgatgcccc ctgtccctgc ccagaacctg     1500
   atggccctca ttagtccttg gctcttatct tggaagcaca ggcgctgaca gccgtcccag     1560
   cccttctgtc tgcgggcctg aaccaaacgg tgccatgggg aactgtctgc acagggtgag     1620
   tatggggcca ggccccagag tcccttatcc ctatgcccct catttcccgt gctgtttgcc     1680
   cctcagtctt tatatctctt ccttttcctc ctcatctttt ctcccttcct gcttttttcc     1740
   tcttccttca aagtcttttt ccttctctcc ttcctatgct agcctcctag ctccctcttg     1800
   tgtccctccc tttgcctttg agtcagttcc atcctggtct cttggtgcct tttccttctg     1860
   accttgcact gctcctccag ccccagctgc cctggcttcc ccaggactgt tcctgctccg     1920
   gctcttcagg ctccctgctt tgtccttttc cactgtccgc actgcatctg actcctgcag     1980
   agaccttgtt ctcccacccg a                                               2001
<210> 89
   <211> 2001
   <212> DNA
   <213> Homo sapiens
<400> 89
   caggcgccct ttgagccggg accagaatgg atgagaggga gcagctgtgt aagcatcggg        60
   gggaagagca tcccagacag agggactcat tgcttgcctc tgcctctaga acaggccctt       120
   aggaaaatcc cggcagtcgc ctgtgctcag ggccaggctt tccatggttg ccacaacacc       180
   cagggcagct tggaggggaa ctggcctgag ggccaaagtc tgaggagcag gtgggcaggg       240
   tctctgacca tgtcctagag ccaaggccct ggtgcatgcc ccctccccac tgccccctcc       300
   acccctcact cctgttccac atgtcctcac cccaaccctc acacttagct gcctggctgc 360
   ccacagcccg tagctctgca catccaggaa caaccagaca gaagggcagg attcgggcat 420
   tcttcagagg ccatctcccc tcgatcttta agggtagggg aagcggaggg gagcaggggt 480
   ggaagggaga gaggcatctc cagcaccatc cgctctccca gcattcgctc agccaccctg 540
   gcccatgtct gagccatgag gtttgagcat agctgcttct ccacatggct ctcccaggcc 600
   tggttatcct gaagaaaaag gaagcacgct acccgctcct tgctaaaagc attccacggc 660
   cttcacctcc ctccacaggt ctgcacagct ggtgggagag ggacagtgac aggttcctag 720
   tcttggcact taccccctcc ctggatggaa tgggtggagg ggttcccgcc tctttgttct 780
   ccatccttgt tcaggtggga gaacaggtgt gctctacctc ccaagtgaca cctgctctgc 840
   gccagccacc taaagaccgg ctgtttctcc cctgggccta cctggcttta tgatcctttt 900
   gctctctgga ccctgctgac tttccccagg agcaaattct ccttggaaat gttgataaga 960
   cacacgttgc cagtgcacaa atcatacaca gcacgaattt cgtctttgca cccaccaagc 1020
   cccaagccga aagctcccta gttgcaccaa ggagagagaa aaaacattgc ctgacattgg 1080
   tccgcagaaa tgaggacttt cagagaccca gtgaggctgt gacctggatc taggatgctt 1140
   tttctgtgtc atttttccat gtatgcctct tttttttttg agacagggtc ttcctttgtt 1200
   gcccaggctg gagtgcagtg gcgcaatctc agctcactgc aacctcggct tcctgggctc 1260
   aagctattct cctgccttag cttcccaagt agccgggatt ataggcacgt gccgccacac 1320
   ccagcttttt tttttttttt tttttttttt tgaggtggag tctcgctttg tcgcccaggc 1380
   tggagtgcgg tggcgcaatc tcagctcact gcaagctctg cctcccgggt tcacgccatt 1440
   ctcctgtctc agcctcctga gtagctggga ccacaggtgc ctgccaccat gcctggctaa 1500
   tttttttttt gtatttttag tagagacggg gtttcaccgt gttagccagg atggcctgga 1560
   tctcctgact tcatgatctg cccgcctcgg cctcccaaag tgctgggatt acaggcgtga 1620
   gccaccgtac ccagcctttt tttgtgtgtt tttagtagag atgaggtttc accatgttgg 1680
   ccaggctggt ctagaactcc tgacctctag tgatccgcct gccttggcct cccaaagtgc 1740
   tgggcttaca ggcatgagcc accgcacctg gctttttttt tttttttaac ttttatttta 1800
   ggttcagggt atatgtgaag gtttgttaca taggtaaact cgtgccacgg gggtttgttg 1860
   tacagattat ttcatcaccc aggtattaag cccacttccc agtgtttgtt tgctgctcct 1920
   ctccctcctc ccaccctcca gccggaagta gatcccagtg tttgctgttc ctttctgtgt 1980
   gttcctaggt tcttcatata t 2001
<210> 90
   <211> 2001
   <212> DNA
   <213> Homo sapiens
<400> 90
   tattttcttg ctgtcaggta gttctcaagt tgccttagaa ataggggtca cctctgagga 60
   cagagagaag agaagggcaa agagttaggg cagtgcttta ggtctttagt gtttatttct 120
   ttacccagag aagtacagga aaaatataac aaaaatgtta tcattgtaaa atctaaatgg 180
   tgaacatttc aatgttcgcc atagtactta cagcattttt agtacattta tattttgtaa 240
   tttaaatttt tggggttttt tttgcctccc aagtagctgg gattaaggca cctgccgcca 300
   cgcccggcta atttttgtat tttttagtag agacagggtt tcaccacgtt ggccaggctg 360
   gtcttgaact cctgacctca gatgatccac ccgccgcggc ctcccaaact gctgggatta 420
   caggcgtgag ccaccgtgcc cagtcaattt aaatgttttt aatttcacaa cttacattct 480
   agcttcaaaa atatatttta cattattacg aaccactcac caagatttta tggccagata 540
   cccaaattca tctcagtctc caagtgcaaa ttttaaaact aatttcttcc atcattcaat 600
   tcaagcagaa tggtagagta gtattttaga acagatagaa ctagtaaatc ccagctctgt 660
   gaacatactt tctctgttac cttggccaag ttatttgcta aaggcatttg cttcccatcc 720
   tgtatctgta aaatgggtat ttcctattgc aaaaggtaga taagtagcat gtgcttggtg 780
   ggttagtgtt tttccctctc cagctggaaa aaaaaaacct ggtgtggggg aagaagggga 840
   acagtttgtg caacagaacc ctaagaggaa atcagctgct catcaagata agggctgagg 900
   cataaaactg ccagagggtc tcaaggcagg caaagaagaa ccatgaggct catcctgcct 960
   gtgggtttga ttgctaccac tcttgcaatt gctcctgtcc gctttgacag gtaaatctta 1020
   cttcctgtgt tccagtctct gtgtagaaga gaattaaagg ttgtttcctt acagttcact 1080
   tttttttttt catttgcctc cacaaaggga gaaggtgttc cgcgtgaagc cccaggatga 1140
   aaaacaagca gacatcataa aggacttggc caaaaccaat gaggtaagca tttagaggga 1200
   tattttatct tttcttactg ttctctaaaa gatgcttctt cttattttac tgtcaatgcc 1260
   catgggacta caaaagacta ttgaacataa ggggaaagca ggagctatct ggataattca 1320
   gccaatggcc aatacttcac ctgttctggt tttggtcttg gtttccaaga ctaccacttc 1380
   tgtttttcct cccaaaagct tttcaaacac cagctttttc aaggtaaaac tatgaaatca 1440
   ggagctgttc ttggcctcct tatcagggca tctttctctt gcctacgggt gccattccct 1500
   ctccattctc cccactccga ccctcatctc ctctggtgcc ataatttctc ttgagtacta 1560
   actgaaaacg ttgaggcact ggctaatatc aagatgtgac cctttccaag agcaggtaca 1620
   ttttcaaata agacttttaa agttagatca atcctttaaa atccaacagc ctaatataat 1680
   tttatcatgt attcagctgc tcctatttat gttgcaccag ccaccttata tatgccattt 1740
   caaattctcc agcagattct caatgtcagg gttataatgt aactcatttg aaacagaaga 1800
   gctaaagtgc aaagacaata aattaaatga ccaagattac acatcaaatt taaacccaga 1860
   actgggtctg ggatttattc cagtcaaaca tacagttacc tctacatcaa atcaatcaaa 1920
   ggccctgcct attctaactc ccaaatgttt ctcaaatcca cacattgccc tccattccac     1980
   tgccactgcc aagttcatgt t                                               2001
<210> 91
   <211> 2001
   <212> DNA
   <213> Homo sapiens
<400> 91
   cagctcaact cgccgagatg acctgggcac ctctgcgttg aatcggcaaa tactgatcaa      60
   gccgcattta ttctgctctc aggaactcta agtctagcag agaagatgag gcggtagaag     120
   ttcatcagtc agtgcccgaa gagaggtcct aatgggaatt cgaagaaagg agttaatcaa     180
   ggaagtgtat ttttttgttg ttgtttgaga tggcgcccac ctgtaatccc agctacttgg     240
   gaggctgagc taagaggatc acttgaactt aggaggcaga ggttgtagcg agccgagatc     300
   ataccactgt actccagcct gggcgaaaga gtgacacttt gtctcaaaca acaacaacaa     360
   caagaaaatt agccaggcat ggtggcctgc acctgttgtc ccggctactt gggagactga     420
   ggtgggagga ttgcttgggc gcgggaggtt gaggctgcag tgatctgtga tcgtgccact     480
   gcattccagc ctgggcgaca gagcgacaac atgtctcaaa agaaaaaaag aaaaacagaa     540
   agaaatccta ccttacatat acagccaaac agttataacc actccatatt taaccattac     600
   aaacgtaaat ttttatttta ttttattttt tgagacagag tctcgctctg ttgcccaggc     660
   tggggtgcag tggcacaatc tcggctcacc gcaaccttcg cctctcgggt tccagtgatt     720
   ctcctgcctc agcctcccga gtagctggga ttgcaagtat ccaccatcac gcctgggtaa     780
   tttttgtatt tctaggagag atagggtttc gccatgttgg ccaggctggt ctcgaactcc     840
   tgacctcagg tgattcgtcc atccggcctc ctaaagtgct gagattacag gcatgagcta     900
   tcgtgctcag cctacaaaca taaattctta agattggctg catcttatct tcccataaga     960
   tttgactgta ctgcttattg caaattattt cataaagtgc gtcctaaatt cacctgataa    1020
   ttgaagtagc agtttgtttt agctaattgc ctttatctga aggaataata aaatttctct    1080
   tatttctatg acaagcaata tcatttcatc tcagaaaaag atgcctaggt tgaattccca    1140
   caatgacaag gagatagggg ttttattttc ctagatgttt atatttgaga ggaattactc    1200
   gcagaccctt gaaaaaacat gtcagggtta taaagctgac aagggactta ctcatcttct    1260
   aattgggttt ttttcagatg gcttggctgg aggacaagca aattgaggac attggcaacg    1320
   gagtgatcaa aatgatagat catgaggcct aaaatgaata aggaaagaag agaagtggca    1380
   gaggctgaga acagaaagag agggtggagg ggctgtaaat cttgaagatt agggtataat    1440
   atgagtatat gggtaagaat tggaagaatt gtgtaggagg cagtagtcaa aaagtagaag    1500
   cagtttggaa gagtagttac aaatatcaag agccaggtgg ctaaaaggtg gagctatagg    1560
   tcattgaagc tcaagaaact gagtctctag ggcattggtt aagtcatctg tctagacttc    1620
   aaagttgtct aggatgataa ttcagaagac tgatctgtgc caaagtcaca ggtaagggaa     1680
   gtgaacaacg aggaggggaa aagggagggt ataggctgat ggaaggagcc tcagagaggc     1740
   aaagagttta tatcctgttg tgggagtggc atcaggtata aaggaacaca aactccttat     1800
   tttttattga tttgcttctt tgtttgctat ctgtaatgtg attggttact tcctgcagaa     1860
   gatttttttt ttccaaatga attatttcct ttttacattt gcaggttttt cacgactgaa     1920
   aacaacatag caaaataagc caagatgtct gtggatccaa tgacctacga ggcccagttc     1980
   tttggcttca cgccacaaac g                                               2001
<210> 92
   <211> 2001
   <212> DNA
   <213> Homo sapiens
<400> 92
   gcaacttggc cgaattttga taccccatgt gggggtatga attcttcttc tttttttaat      60
   tactcaactc cattctctac ttttctaagc ctgaatagat gaggccactc agcctcagaa     120
   actatgaaaa cttcataagg aacaaaagga tctcaaagaa taaaacctct taagcatata     180
   ccagccaaca cctggctaaa gtcccagtgt ttccagcctg tttcagagac atttctaagt     240
   gaccagatgc aacatcttag tttgtcgaga tgccctcgag tttggctgga tgctcctctg     300
   tcctccagaa gatatcaaca ctgtcggaat ctatcccacc atgcagctcg tcctctggct     360
   tggccttgct ctgcagcaga gcaacccata aagcatacca caatgcctga ggtaggcact     420
   tgaaagatct gaggtcaagg tcaagtcccc agaaccgggc cagttctttt taagggaaag     480
   agcactctag cgcattttgg ctgtaatatt cctagaccca gagattcagc tatcataggt     540
   accttgtcaa tgaaggaggc aaacttgttg ttgagggtct tgatttgctc cttctcatcc     600
   ttcttcaccc tctgggcatt ggggtcaatc tccaggttga ggggggtcag taggctcttg     660
   ttcacagtca cagctgtgat agatggggct gctgggactc caacccctcc aactctgtaa     720
   ccaaagccag ggccaccaaa gccatagcca atgccactgc cagctccaaa gcccagacca     780
   acacagctgt cagccctagg ccccagacca acacctctcc catcaccaaa acccatccca     840
   cagccagcac caaagcggac tccacagtgg atgggccgag agcctacagc tgctatccgg     900
   ggtgagtacg atccaaaggt gatgacactc cgactaccaa agctgccaag gccccggaat    960
   ccaggcccac tccaacagga gacagagttg gcccggaagc gattcaggtt ctgtggtgtc    1020
   attgctgaac aagagctgaa gttgcccacc cggtgaccag agctgactcg gtaggagcgg    1080
   caagacatga tggcttcctg gttgggagca aaagagcaag tgtagaatgg gtgagctgga    1140
   gctgggagtc cctctgaggc cagtggaacc cttgcacccc ttttatgtgt gtggtgatct    1200
   gaggcttggc cccataaaaa tgaaaaaatc catttgcagg catttatgag cttgggtagt    1260
   tagcagaaac tctgcatgcc tataacctcc ttgacaatga gttaactcag acacacctag    1320
   tctcattggg atgagggctg taacagcaaa ccaataaaca ttaaaaacac ataaacatat 1380
   aaacattata gtcttttttt tccctataag tctaccacaa ttgccattaa ggggacatta 1440
   atttgctttg gtcataaatg caataggttt tttcactgtg agtgtggcta accaccagaa 1500
   agttaggatg gcgacagctc cagcaagagg ctcacatggg atgcatccat gaagatataa 1560
   ccccctaaag aggtgggagt gtgtgcacat ctccaagtaa gaagtgatat ggagaaaata 1620
   cagtttgttg catttctgca aatcaattaa atcatcaaaa aaccttctgc catgttttat 1680
   ccaatatggc tgtttagtat acaagagttg aatctacagt aaagctcatt tgggctagtg 1740
   tagacacatc tgtgatttga gacactcaga gatattcatt cattctacag ttttagagtg 1800
   cctgctatgt atccagcagt gtgcttgctg ctgagggtac agagattaca aagacagtct 1860
   ctatcttcat ggtagtaggg gaagatttat cttaaatttt tttttttttt ttagagacat 1920
   gatctagttg cccaggctgg tcttgaactc ctgggctcaa gtgatcctcc cgcctccgcc 1980
   tcccagatag ctgggactac a 2001
<210> 93
   <211> 2001
   <212> DNA
   <213> Homo sapiens
<400> 93
   tctctggcct ctggcttcca ctggcttttc ctttggaaaa agccagttca aatggtgctc 60
   actctaggaa tcgcttcaac tcggagccct ggaggtgagc agggccgggc acgggccggg 120
   cacatacacc ccctctaggg agcagggaag ggcacaggcc aggggaagct ggagacaaaa 180
   gaggagacag ggacagagat aaaaacaggg actgtgacaa gaacacagga tgggaagggc 240
   agagacagaa atagaggcag gcttaaggat ctagacaggt catgccagcc caggttcaag 300
   gcagaggggc gtagggggac agagagagag acacaggaag cacagagggg agaaagctct 360
   gcttagcaga cttgccaaag ctgaagaccc cactgttgcc acaaaggggc ccacaggaaa 420
   gctaggggtt gcaatcgcct gtcaggaccc acattttcac cctgagctgt gcacactttc 480
   caacggcaag caaaaggagc acaatctagt taagttagga ctggcaagat tcacttctga 540
   tttaaaactt aagacagcaa caataaagat actcagctga aatgccaccc aacaaagcca 600
   gctcccctgg aaagttaaag tttagcaccc ccatcccagc accctgtacc tttaaggaag 660
   agaaggttcc ctccgcttcc cagccgaaga ggcaggttat tctaggatat aggggaggct 720
   tacctaaaac tgagttattc tcctttcctc ctctggccag cctctcttca ctctggggag 780
   cttttaccat ttaaggtcaa tgttacatct ggctccggag ctcagagccg agaagcctct 840
   ggctgcagaa cctcggccgc acgcctcctc tgagaggggc tgggacttag agccgagagc 900
   tcctctggga gccccactcc ggcctggctc gggggataat ggcagggaaa gccgaccagc 960
   tggcctcagg gctcagggca ggctgggcct ttggggcagt cgctcaccct gggcccctcc 1020
   gatgggtatc cacacagcag atggcagtcc ccatggcaac tcgtgccact ggacactgtc      1080
   ctggccggac gggaacaaag ggaccctgca gatctcccca cccgcctgga gcctccagca      1140
   agcatgtgag cctggagaag accctgggcc agcgccaaga gaaactggga atggttcctg      1200
   tcctggggat tcgggggcag gggctgctcc cactgcgtta aactcttggg attgccagag      1260
   agaatggcct gggagattca gctactccca gacttggcac cagccccagg gatgtcaacc      1320
   ttgctcctcc attaaacagc ctgtggtctt aacatcgtgg agcctcagtc tcctcatctc      1380
   taaagtggaa ataataatga taatgcccga ctcactaggt agtcagggga ttaaatgaga      1440
   tgttagtcaa gtgtttaaca cactgccagc atctcataag agctagatca gtatgaatga      1500
   gcttcctgcc cctgcccagg aaatccatac cctgtccata cacactcttc acctgccttg      1560
   ggtatagggc ctacccaagg ccaggatcat ggagctccaa gctgggggca agccacatgg      1620
   ggacttcagc cccttctgtg gttagagcag cacagagaaa ccctaggtaa agttgctaga      1680
   taaattacag gacacccatt taaatttgga tttcagataa acacaggaca tacttgtatt      1740
   aaagactgtt atttatctga aattcaaatt taattagtca tcctctattt ttgtttgata      1800
   aatccgggaa ccctagcttt tggccatcat tagccaccca gaaaacattc agtagtcatg      1860
   gtagagagca ctagggattg gagctcccag gcctatggcc tcaagctctc ctggcaaaca      1920
   cagtccttat gctcagctcc tggccacaga tcagttgtgc agcagtggca gatgctctcc      1980
   actgagcctg gcccactgag c                                                2001
<210> 94
   <211> 2001
   <212> DNA
   <213> Homo sapiens
<400> 94
   actacaaaga agaaaataaa taccaacgga aatcacactg tatacttcta ccatgtgggg      60
   gggcacttat tcagatatct ctctttcatt ctctgtccac ttaaagaaat atgcatacag     120
   tttttaaagc atggagtaat actctacctg ctgtttctgg agtagtattg ttgtagacag     180
   agtgacctct ttcaatggca gatgacttga ttcttggtgg taaagcaagt ggttggttat     240
   aagtatgggg tctcattgtg cctaggttaa catttctttt ccactcatat gtgccattga     300
   gtaagtgatt taacccatct ctctgtgcct cagtttcctt atacacaaaa gtggggacga     360
   taatattacc tacctcagag agttggtgtg aaggttatat cagatcatat atttaagtac     420
   ttacttaaga cagtacctgg aacatagtaa gtacttacta catttcaact attgttctta     480
   actaaataag ttttaacttc atagtaacaa taaggggttt ttaaatagaa aagtggaatc     540
   tttttaaaaa tacttttcac catatttcaa agccattctt ttaacataaa ttattactct     600
   ttttgcacac agctatatac gttatccact gaaattagta tttgcaggta actacccttt     660
   tgcggaggcc tacttaaaaa ctatcctgtt gaacacaagg tacctttaag tgagacagtt     720
   ttacatgggc ttcctttata acacattagt ccaactaaaa atccttgtta attattaaac      780
   ccttaggtat tacaggaata cctgacactg cagattgaaa acagacagtg tttgtctctc      840
   aagttaaacc aacaagccga tagaaaaagg tagttatcaa gagattttta aaacttcaac      900
   cctttttctc ttatagttag tgaagagagt agaatatctc cagttttggc tgacatctct       960
   acaacctgaa caattggctt aaacttcact tgggattccc ggttgcttgt tttagcatgg     1020
   caaaatttgg cgttcacaga atccttcttc tggctatttc tctgacaaag tgtctggaga     1080
   gtacaaaact gctggcagac cttaaaaaat gtggtgactt ggaatgtgaa ggtaagtttg     1140
   cttcccccgc ttcttctcct cctaaattga ggctctgcaa tgacataaaa tcttattgtg     1200
   gtcacgcatg ataaaaatga gtattgatac cattgagaaa tatttggatt atgaagtaca     1260
   gcttggtata aaaaagaaat gttggaagca aagcattttg tggtagtatt taatacaaat     1320
   atacaaatag caaaaatcag tctgctatag aaatatatca gaagttaaaa aactagtcag     1380
   tccttttact atttttaaag attaatctag gagtctctga gcctattctg gctcagaagg     1440
   ctgcccatta aaaaaaaaag ataaatctaa ttgattcata taaaaaaaga catcaggcta     1500
   atttgatagg cctttattta tgaaggcctt ttacttttta gtttctgttt cacactcaga     1560
   actttctgat tctcatgacg ttcctaaaaa gtgtttttat ttcaaaccat catgatttat     1620
   tcttctaaac tttactaaat gttaatcatt tctgtgttat ttattatgta aaaatactga     1680
   aatcaggact agaatataaa ttttttattt atatcattga atataaagct ttctttactg      1740
   taaatggaaa ttaatttcca ttctattgtt aaagaactta tttctgttat tagttttatg     1800
   gtgcaaaata aattacatcc caatatatta gagttgcttt aaataagtta tttttccttt     1860
   tgtttttatt ttataataaa tacatatttt ttctctctca gcagaatcaa aaattgcttt     1920
   aaataaattc tacctttttg gttttttttt tttctgagat ggagtcttgc tctgtcaccc      1980
   aggctggagt gcaatggcac g                                               2001
<210> 95
   <211> 2001
   <212> DNA
   <213> Homo sapiens
<400> 95
   agggctgtgg atttaggagg gggcaaccga taaactgtag aactgaagag tttggaatat       60
   ttgcataggg gagcacttgg gctttggatt tggggtctga gagtgagaat gcaccccaaa      120
   gttgtggatt tgggcattcc aagggagagg aatccaaagg tctgggtttt attagggtga      180
   gatggaagtg tggctgtgca tctggaggtc ttctgttgcg gaggatgcag ggttaagggt      240
   ctcaggaggg gggactccag gcatatggac ttgaaagtct cggggctagg aagacccagg      300
   gctgtgggtg tggagtttct gggaggagga gtgtgtagca agccagcagg tgctcccaga      360
   aagcccagca gcccagcaat gggcccaggg gctgacttga ttttgctttt gcccagagct      420
   atctcttgat gcccagaatc ctcattggaa cctcagagtg aggccaggat gggtgccatg     480
   gaacctccac tgcccatctc tggttgggaa aggggtggaa gaccagggcc atatttcagg     540
   ggaactttcc ccacacctgg agaagagtac ctggagttag cggttggcag agggattggc     600
   taggtctgga gaaatagtaa taacaacccc caaatggtgg aaaaatctca agtgtcccaa     660
   gggttgtccg ctggtttcca acccagatcc tcagggaaac ccactagatt gattgtatca     720
   tccctattaa tattatgatt gttgtttatg tttctcattt tatataaaga tctacagagg     780
   acagaaaaca gacagtgggc aggggacatg tgtgagtggc cctgaactcc catctctcct     840
   gctctgtgta cttagttccc ctttctggat ttggttttcc tgccagtgga aagggagtgt     900
   ttgggaagag aagatcacat ttgagggtga gttaagtctc ctttaagggc gattcctgct     960
   ttgtcagggg ctactactat tagcaggcag agggaacagt cgcagaagag attggggtaa    1020
   actccagtgt taggcacaga ggcagatcct ggggtgggtg tgagtggaga agggatgagt    1080
   agccctcctg tccttccttc agggaaattc ttccttagta aacaggacag ttaccctttg    1140
   gtgagcatgg tcatatatgg gaagattaag tctaggtgtg tatgatctgg ttgaatcttt    1200
   gtaacagtga ggttggttca tcgtgatcat cctcattttg aagatgagca gatctagact    1260
   ccataaaccc tcttgagtga ggtcacacag ggagtaagtg agggagctgg gatgtgaact    1320
   ttctcctcgc cccccttcat tgactggcct agatcaccct gggagtcaag tgcgggccag     1380
   ccttagcagg aggtgagtgc tgagactggc atcagcagtg gacacactga tgacattggc    1440
   gggttctgat atcagcatca ggtatggcca gggagggaaa ggcatcgtgt tggctttggg    1500
   actgccccca ggtgtatgcg aggatgggaa acaagttggc actgaacttg acaccaggag    1560
   aatattgaag tcatgtttgg tgccaagagg gctctgtgtg ttgtctcagg tgtcattgag    1620
   ggcataagag tgtcctgctt caggagggca taagggtggc accatatagg gtctgaggtt    1680
   ggtcccagtg ggctctgaca tgagcatgag gcaggtgctg atattggtcc tatgtgggtc    1740
   tgagatgagc ctcagccaga ccctgagagg ggccccagat ggccacagga caggctctgg    1800
   tggttggacc atttagggtt gcacctgtcc tctgggtcac caactttgga caagtggagg    1860
   tgggtgaggt tggcaccccc aggactgaca tcttgctcaa actgttctag gatgctgagc    1920
   tctgagacct gtgtggaggg aacaaaggtc agagaacaag gcagagagag gaaagttgtt    1980
   tgttgatttt ggaggggaag a                                              2001
<210> 96
   <211> 2001
   <212> DNA
   <213> Homo sapiens
<400> 96
   ctttccttgg tagctctcac ttagggactc tgaagagtcc catctcagac cccccaggat 60
   gtctcctgta ccctcatgcc ccgccagggc caagcctgct ttgcacacct ctgtagtcag 120
   tgcttctccg agggtacaca ataggtgctc acactgctga aagacgaaag catgacaggg     180
   cattctacag cagccctggt cttgggccaa aaccacagtg gcatcctcag ctccttgctt     240
   cctcccactc cgcatccaat tcaccagcag agctgatggg ctctacctcc agaacccacc     300
   agaatcttac tggccacacc ccctcctagc cccgttctgc tccaggcctc cggtaccttg     360
   cacctgatgg ctgcagcggc cacctctttg ggcttcttcc ttccaccctg cctcctacag     420
   tctatgctgt tgaaatcgaa tcacgggtgt ctgtctctgc tcacaacctt gcagtggctc     480
   ccatgacact cagaacaaaa gctgaagtcc ttccagtggc ccccccccca gctgcatggc     540
   ctgccctgct gtcacctctc agacctcccc gcacccttgc tccctctcct ctgctccagc     600
   tacacaggcc ttcgccccat ggttctccag gcacacaccg ctgcggatgc attccctcct     660
   cacggtcaaa atacatgcag ctccctttgc agaacgttcc ctcaaatacc tgcctggccc     720
   actctatcat ttcccttgtt ctctgggttc aaatgtcacc tcctctgaga agtactctct     780
   aatgatctga cctcaagcag ccacccaggg ccgccacttg ccctctttct tcttcatttt     840
   gcttcaccat ctgacatagt agattatctc ttactgcctc tttcccctac tagtccagag     900
   ggagacatac tatactgtcg tatccccagc acccaggaag gcttggtccg ttcagccctt     960
   ggtcagggct gaatggattt ctgttggatg aatgaaggga cggcagttaa agagccaggt    1020
   tgccagctga cattgcccaa tcttgctgtg tggcctcaga cccacccctg cccctctttg    1080
   tgcagctcag agacagccct ctgtgctcag ggaaggcctc ttggatggac actccacctg    1140
   ctgattgtga cagaccccaa agccgccctc tgccccctgc aaacctccca tgccccgtac    1200
   cttcttcagc cactgcgtgt tgttctgcag tgcctgctcc agctgtttca cctgctgggt    1260
   gggcaacttc cccaggtgca gtgggttggc cagtgattct ctctggaggg tgttggagtc    1320
   cctggagacc tcaggccccg gagggcaggg ctcagacttg ggcagcaaga aggtgtagct    1380
   acagtggccg tgctggacta caagtgtctc gcagccccta tccgcctcct gccttgtctg    1440
   ttgagccaca gacatggtgg caaccacaag gaggaggctg ccctgcagca tggctagctg    1500
   ggagagcatc tgaagatgtg tcaatggcga gggatgtctg ctcagagccc taggggctgt    1560
   gcctgggatg tcctgctgca gccaacagtg gccaggcttg cctgcagctg cagctacaaa    1620
   cctctgtctg gccgagctct gtccaggcag ctatttatac ttgctctgaa taccacggct    1680
   gctctgcttg cttcctctca ccgcctccct ccggccccac ccgacaccct ctcacctcta    1740
   ttcttcactc cttcccttcc tccttcctca gtcttggtgg cacgtcgccc ttaccctaag    1800
   cgtgcaccaa ccttgctctg tctgcctcac ctctgagccc tctgattatc cagtgaggcc    1860
   tggcttccct gctggagggg agggaagaaa acagcacaca gacacgcgca cacactcatg    1920
   cacacactca cgagcacaca ctcacacgca cgctgacacg cacacacatt catgtacaca    1980
   caaacgccaa gctccaatgc t                                              2001
<210> 97
   <211> 2001
   <212> DNA
   <213> Homo sapiens
<400> 97
   ttaatatgtt catgctattt tcatttgtga ggtctgtaat ccatcagaac aacctgacag 60
   tgattaagta aaatcaaaga gactttgtaa acatctgtgt gcttttattg ttacaaatgc 120
   ttcattataa aaagatcagt caaaagcata aaagtaccct ggtttaaact ttttctgtgt 180
   ttgaatgaga atgtttctct tggaacatgc tcatatcagg cttccacttg gcaaaatcac 240
   gaattggctg atgttgtttt agaggctttc tcatgtcccc ttccaaacat cactcttctc 300
   cttctgtgga cccctttctg ttatctagga gcccctcatg ctgccttcca cattctgcct 360
   actgcacagt catgttctgg acccctcaga atccccctct accattacaa tatgtttttc 420
   ctaagactta cttgcattga aaggcttgcc agggaagtga ctaatcttat catcttgtac 480
   taaatgttaa attaatcctc attctctcta gaattgtttt ctaagcaggg attcctagag 540
   atgctatcaa tatctcagcc tgaaattgca taggtattct ggtgaatagc aagagaaaat 600
   aacgtgagta taactggacc ttttagaaaa gtgaagtcta ttaggtcacc ttagttggcc 660
   tagggagttg atgtaggaat gctcctctcc ctgaaataat cttccaggag aggaatgtat 720
   ccccaaatag caagtgaata tccaatggct tctcattgca cttaaactct atattccttt 780
   ttatgaccaa aaaggcctgg cttctcccta actgtctctt ccttacacat tacacctctt 840
   ccttttgtat gtgaatgtgc caagctacca cctcatttct accttaaggc ctgaagtgtt 900
   atgttccagt tcttggcatg caaattactc ttcatcactt gtttcccgac acaaatgcca 960
   cctttccaga caatctaaag taacctcaaa atcatttgct cgcacatcac tttgttttat 1020
   ttctcctggt attaatcact atgtgaaatt ttctggttca tattatttgt cacctgcttc 1080
   ttccaggcct cctcttactt cattgtctat catgttcatt accgtatccc tagtgttttg 1140
   cacagaatct ggtacatatt cagtgctcaa taaatatgtg ctaaatgaat aaatgaataa 1200
   attaacctaa acataagttt tacacttaag taattgagca agaagtcttt gttgtactaa 1260
   tattggaaaa ggcaggggtg ggggtggtga cgtgattata accaatgtaa ccagcttgca 1320
   tttataagac ttgtattgat tatgaactat agcatttttt cgttaaaaac attatcaata 1380
   ttggattgta aaagattcca ttggcctttc tgaaagactg tccttttgat gagatgtcac 1440
   aaatgaatgc taatgtccct tgagaaatcc tgccaacaga tcttattctt ctatcaatgt 1500
   taagaaactg catttctctt tggccaattt ctaggcttat ggatagtctc tcttgctagg 1560
   acttcagaag ccaatgagca acttgcctgt cacaagatcc gacgaatgat ctctccctgc 1620
   atgcggtgta gctaagcaga gaagcagctc ttgtaagaga ataaccaaca cagcatggaa 1680
   tattgctaaa tctacagtgt tttatccagc aaagttcacc ccagtagcct gcccctgcca 1740
   ataaagccta gaaatttttc ctaccaggca gtcccagttt agttggttgt agcttagaag 1800
   cagacatctg agagcagttc agcaagcctg cagtcagatg cttttgtaga gctctcctct     1860
   taaaaggcaa aattgaggca agtcaataga ttacaacttt ctgggtttgc ttctgtcatc     1920
   tatcaaatat cacaattcat cctgcttcgc taaagtgaaa ctccaaggat ttgtttttaa     1980
   aggtgtcctt ctttctgtga a                                               2001
<210> 98
   <211> 2001
   <212> DNA
   <213> Homo sapiens
<400> 98
   ttttaaatta tctctttttt attacatttt ttggaagtat ttctcaattt ctctctattc      60
   acattttctg ttcagccctt ttccttattc cgtatcttca ttttcttggt agtcctcatt     120
   ttgaagtcat tcttcagtta aataatgtca taaaaagatg gagattaaac aatatgtttt     180
   tcatgagagt tgattaggat caaattattt gaaatttcta acttagatgt tttcaaaatc     240
   aaaaactaac taaactacct tgtattttct taagtatgga ctacaactgg tgaacaagac     300
   tcttcaataa gaccccctgt ccccacccca ctcccatttt tcactgagca gcacatgcca     360
   gatgccaggg caggagggta cgtggtcacc aacctgctcc ttccaggaat tcaagatgtg     420
   ccccagtctg taggcatagt agtccttgct ctgttggttg aatgtgtgga cagcaaactc     480
   cactgtggca gggaggtaac gagccatgac attgtgttca tcacagtccc tttgctcgtg     540
   gaaatgccag gcatagatca gcaggatctg ggagcctaag agaagcagca gcagcgccca     600
   ggacagacct cccttccacg gcaggcccag catggtgctg actgtaggca ccgctgactt     660
   tgctcttccc agcccccgtg cccacaggag ttaattgagc acagacccag cccttaatct     720
   tatctgctca gacagccctg tgggacaaac ttgtctcacc ccacactggc aacaggcact     780
   cctacaggac aggaaggaca ggatcctgga ttaaagggag gcagctctgg atgtggagtc     840
   actcatctgc catccttctg ccctatactc tctaggccag cacaagccac aggcaggagg     900
   ggagctaccc tctgccacta gctttgaagg ctgctccttc agaacccaca ggttctggtt     960
   gtcctcaggt gcttggtgtc cttggatgat tctgtgcctt cgcctcccac agcgaactga    1020
   gataagacct ggggacatct cgactctcac tctcccctcc ttcaccccgt cgaagagagg    1080
   atctccgttg ttcagaaaaa taactaagat atcagtttgt tcttttgttg tataatacct    1140
   caacagttgt actctgtgaa ccaagggaaa aaatgaaaaa aataaatggc aatttttatc    1200
   aaccattttg acctgggatg gacaatgagt attgtgagaa aagggcagga aagacagaga    1260
   cccaagtcct gaggccccag cctggtcatg cccagcccag ccctgcttgc atttctaaac    1320
   cttgtcagcc ctggttccag ctgagccaga gccaaggttt ctacagggcc caccgcccta    1380
   catctgcagc cccaccacac acactcatgt tttattagac tggaatatca ttctagacca    1440
   aatttttaaa cacttctctg ggagatcaca tgtttctgcc tcagtgagat agaggttgga    1500
   ccattcttca gccccttgat catttttgtc tccatctgac catccttctt ggtgttctag     1560
   agtgagctat tttgccagca cttaggtgat actaggtggt tacttttaaa tcaatttcct     1620
   ggcaataact aacctgaaag aggaaataat aatattttgt tgctgtaaga atgaacatta     1680
   aatagtgcag gacaggtgaa gtatgcaaaa gtgagagctt tgagtttggc tgagaagact     1740
   atggtgctgc aatataggca agaacctagc attgttgcca gataacagaa tagaaagttc     1800
   agtcccaagt cagggagact gattctacaa gaactgttct gactgtgtcc cataccatgt     1860
   tcccagatca cagcagggcc aggcctggga agagcatcac tgcatctgtc cacactagca     1920
   cagcactggc caggtcaaga cagctgcagg tgaagagagt catgctgcct gacccaacac     1980
   tggggtggtc ccgagtgtct c                                                2001
<210> 99
   <211> 2001
   <212> DNA
   <213> Homo sapiens
<400> 99
   agtgataccc tcctgccatc ttaaacggct ctctgccatc tgcttcctcc tcctaagtgg      60
   ttttccttct gtcctccctg tttaatgttt aagttgtgca catcctgtga catcctggag     120
   acccatgttc ctgacctact tacctattca accatctctt ctcacacatg gggtagcaat     180
   gagaagcaca gggggtctct gcaagggagt agggaggtgg agagagcggg ggcagccaca     240
   catcacactg ctgttctcag tggcctcctg ctgtcaacaa gctaacatta ataaaacaag     300
   cccactcttc tgctaagaga ctgtgacagg gtaacattta ttctaggtag cttttcaaga     360
   aaacagctct tgctcccacg gtcccctcgg agggcctgca gagctccagc gatgtcagag     420
   ggcatctgtt tgtttctcct ttgcttataa aagggacctg gaaatcaaag gcaggctctt     480
   agcccttagc agtgaggatg acagctctgc accagccaca cgcaggcccc tggctctagg     540
   aaccacctct cacccagacg cccaggcaca gcatggtggg gagagagtac aggacacagg     600
   gcctaggcct gctgccccgc ccacagcccg ccccctagcc ctttctctga ggccaggtgt     660
   actcacctag cctccagggt cccctgctca ggtggcccca gaggcccaaa cccactgctc     720
   ccactttctg agacctggga ccctcacctc cccaactccc accatagggc gacactgaaa     780
   agtgtcccct tccccccccc gtttccaagt caccagcttg aggatgccca aggctcaaag     840
   gctcctccga gtggcacccc gcatcgggct gcagaggtcg gagaggggag gggatggtgg     900
   gtgcaaaggg tggcttcttc aggaaaggga agcctgtccc tggctcccag cacctgtcac     960
   ccctggcgat ctcaccccgc ggccatcatt cccgtcctca cggcccattt cagggacact    1020
   gaggctcaaa gagccacgtg atttattcaa agcagctcga gccaccactt ggagccacaa    1080
   gaaagccctg gtccgtgcct cctcgccttg gtccctggcc cttcactctt cgtccaggct    1140
   ctctgacctc tttccctctg cgcccctgcc tcttccccag gcctgggtcc ccaaggcact    1200
   cggccacgcg ctgggtccgg ggaagaggcg cggccgcggc cgaggcgggg acagggaggc 1260
   ggcagcccct cctgcgcagc gacggcgcgg gcgggcggag gccggggcgc cccagccccc 1320
   cgcccccgct ccctccggcc gtgacgtcag agccggacgg ggtaaactga gcggcggcgg 1380
   cggggcgctg gggcggagac tgcgacccgg agccgcccgg actgacggag cccactgcgg 1440
   tgcgggcgtt ggcgcgggca cggaggaccc gggcaggcat cgcaagcgac cccgaggtaa 1500
   cacggccccg gttccctgct cgcagggcgc ggtgccggag gggagcgggc ccgggggagg 1560
   cagggaggca gggaggcccc cagagctacg cgggcggggc tgcggggacc ccggggcagc 1620
   cggggcgcag ggcgcagggc gcaaggtgca cggggccgag cgtgcagttt gggaggtggc 1680
   acagttgggg gcggcgggga tcagggcagc gtcctggggg ttctaggttg ggtctccaga 1740
   ttgggtcccc gacggcgctc gcgggcgctc tttccaggga gcgcgggctc tcgtggggac 1800
   gcgctcgagg caggtgagcc gaggctgggg cgcccggggg tgtccccgga tgccggtgcg 1860
   cgcggctccc tgacccgccc ttccgcggcc tgggggacgg gagaatgggg ggtccacggc 1920
   cgccttgggt cctcaccgcg caccgcgccc gcagcggagc cccggagcca tggccctgag 1980
   cgagctggcg ctggtccgct g 2001
<210> 100
   <211> 2001
   <212> DNA
   <213> Homo sapiens
<400> 100
   ccttccaaaa atgtgtccac ctgggcccct gccctgggac tcatggattt ggagttgtgg       60
   ccacacggtt gaggggtgca gtgtccagtg gaatggggca attgcgggcc tgggggccct      120
   tggcctgtcc gtggcgggag catctgcaag gaggagcccc agagtccagg gagcactgtg      180
   gggagctcct tagagctgaa ctcacccggc gtcaactcat caaccctcca cccatggaca      240
   ggggtgcccc cagcacagga gaggactcag ccctctgccc ccacgcacgg tgggtgcctg      300
   tcaccctgtc ctgcccagcg gcccgagggc agcagtgggt gtgagggcag cccccggcct      360
   cccaagagca gctgagagga tccctgcggg aatccgggct tcgggtgcat gcgatctgat      420
   ctgagttgtt tctgacagtg acagagtgac aatctataag tatctcaaga tcaaatggtt      480
   aaataaaaca taagaaattt aaaacgatta aaatatgcct cagtcttggg aatcctggtg      540
   tctgcaacaa aaaaacttga ggctgtcctt gcaaacggtc ctcatggtgc tgggggtggt      600
   gccaggcccg gcgtttgcct ggaacacaga gcatgcgaga aggagcagag gacgtgggtg      660
   ccagggaagg accctggcgt cagagccagg gcctggacct gagttaccca ttaacggcac      720
   tccactggtt ttcctttaaa gaaatgaata aatacaactg cagtggggct gcttgtggtt      780
   tccaaacgtc ggcagaggct ggagacggct ctctagtgct gggtgtggag tgaggcacca      840
   cccttgccct gaagcctggg gcactcagtc accatggctc atgccccaga accggacccg      900
   gccgcctgcg acctcgggga tgagaggccc aagtgggaca acaaggccca gtacctcctg 960
   agctgcactg ggtttgccgt gggactgggg aacatttggc ggttcccata cctgtgccag 1020
   acctatggag gaggtaagca cccacctgcg tcctggggca gacccctaag caggggaggc 1080
   cgtctccacc ctgacctgcc aggcgcctgc cacgcatccc cagtgcttgg gcagagtcac 1140
   tcctcctgga aaccagggtg caccattgcc cacggcagag tcagtcccct cagggcccac 1200
   ctgcagtctt ctgggacctc agggcagctt caccactgcc cacagcacgg aggatccagt 1260
   cccctcgggg tccactatgg tcttccggga cctcagggca gcttcctgcc aggcgtcagg 1320
   ttgaatgggc cgccctgccc gacacacaca ccctacaaac agtgagcgct cttccatctg 1380
   aatgtcgtca gttctttaaa gaacacacaa tccgctctgg gtttcctcac cacagccttg 1440
   gtgtgtccag acatgtgctc aggtgccctt ctccgaggtt ctttgcagga atttgggggc 1500
   ccacccctcg cctccctggc tttggttctc tgggagagcc ctgtgggggg tgccaggatg 1560
   ttcagaactg tctgggtgac tttagtgacc ttggactcac cacccccaga agtgcattgg 1620
   tgactgaaat aggctcccac gcacctcgag cctcccctca ccctgtgcct gcttctcttc 1680
   atgcaggggt cacactctgc tctccatgtg gcgacacagg atgtagggac aggcatgaat 1740
   cactgctctc agggagccga cgtacaacat cacgcatgga cacccaggtc ttccccaggc 1800
   aacacgggca ggggcttccc aggccgcagg gtcctcctca ggccaggccg ggctgcttgg 1860
   tcagcctgtt ccccgggccc tcagctcctt ccctgagagc cccgcacaac tatcccttgt 1920
   tataaggtta ctcctttaaa gtgtttttcc tcctgcttaa gaaaagattc tcaacaggca 1980
   gaaaatgcag gaaatgcgca c 2001
<210> 101
   <211> 2001
   <212> DNA
   <213> Homo sapiens
<400> 101
   tctccagctt gggggtctcc tactggattc ctctacccag ggctatgtag gactccagcc 60
   cctccttctc tccatcccca gctcctgtct tctcccactc ccctctccag gcacctgagg 120
   ttgcaggaac ccttcagacc cctggggact tgggtaccac tctacagttt tcgagtgggg 180
   agaggcccag ccagcccctt ctcccattca tcaatgaaga tgcagatatt cagaatggcc 240
   acatagatag agtgacctat taaggtcgcc tagcagcagg tggctgggtc tgactgaaaa 300
   tcaggtctga ggacccgcaa agcgtgcctt ccactggaca ggctggtctg atgccatttt 360
   aagtcacaga gaatccagtg caaatgtgag acagttagga ggcagctgaa ttaggaagag 420
   tgactgagaa tggaagagag agggatgcgg gagagggggt aagacagaaa ggggaggaga 480
   gcaaagggga aggaaagcag gtggaaagga ttgagaaaag agagatgcag gataggcaga 540
   gacagggaga gagggctgag gaagagggag agagaagaaa aggatgggga gggagatgag 600
   gaatgcctga aagggttaga gatgaaggag caggatggag aaggatggcc cagagatctg 660
   ggacatgggc gaggggctgg aagccagctc tggacaaggc gtgtagagcc actggggagc 720
   ccactgcctt cggagctgtg cctcagctct ccagcctctc ctcgccaagc tcagctcacc 780
   tggctccccc tgcgcacctt cacccctccc ggggccacaa agacacacag acacacacag 840
   acacacacag acacacacac acacacacac agacacacac acacacacac acacacacac 900
   actcttaccg ccccagttgc aaagtctctc ccaaagtggt tcagcagctg cttatggctc 960
   tctccttgtg tccccacaat ggtcagtgag atggagtccc gtgttcccga caagaggtcg 1020
   gtgcctgtgg ccaccctgac tttgtaggtg gccatggctg ctcttcagga ggcaagaggg 1080
   gcactcagtc ccagacaccg tggaggggcc acacgaaggc ccaaggcagg caagagaagc 1140
   caggcacaga gtggagtgga cagggctggc ctccgaggtg cagtggtgag gtggcgaggt 1200
   ggggtgacta ggcctgccag ccaaattctg gaaaagctgc tgcccttggt ggccggggtg 1260
   ggtgccgggc aggcccaggc ggagcccagc tggtggtgag tgagcaggtg tctgggctcc 1320
   aagccttctg ggagctggtg gggaggaaga gcaggcaggg cactgggcct gcctctgaaa 1380
   gggcggatag aagatgccgt ggatgaaggc gccttcaggg tgggggagcc agtccagcct 1440
   gagccagggc cctagggaag ttgcgggagg gggcagggtg tggtccccag atctttatca 1500
   gactaataag ctccttcctc ctgccagtcc ccattggcta cagtgccagg cacccctact 1560
   aagcagggcc atgggcagtg agtgacttgc attggttctg aggccactgg tgggcggggg 1620
   agggtagggg gaggggcact aagtggcagc gcagctggcc agcctccagg ccattcctct 1680
   ctgggaaatg ggacaggtag tggccacagt gagaaagctg gcctgtcctt ctgccccagg 1740
   gcccagagtc tgtgactgga agggtgggag gtgttgaagc tagggctcgg ggaccacatg 1800
   ggtgcccaaa ggcctgaaga tgctgcgggg aggaacgatc agggtaggag aaggggatct 1860
   gttgcttcct gtggggccag gatccactgg tgagggattg gagttggata taaaatcaga 1920
   agcccaaaaa aaaaaaaaaa aaaactaaat tagaagacag agtgaaatct ggggcataag 1980
   aattaagtta aatagctggg c 2001
<210> 102
   <211> 2001
   <212> DNA
   <213> Homo sapiens
<400> 102
   agcccctccc cgagcctggg gcttccccac cctaccctcc actcccagcc ttgaccaggc     60
   gaatgcagcc tccccctcct cccagcgcgc gcgcgcgcgc gcgcgcgcgc acacacacac    120
   acacacacac acacacacac acacactctc tctctctcac acaccctctc ccccgacgcc    180
   tgggttcagg cttggcccca ctgaagaagg aggtggccag agttcctctc acctgatctg    240
   gggtcgccaa ggctgcaggg agggtgtagt aaccttccta gggagccaga atggggccca    300
   gatggccaca tctttcatca ctcctctttg cgtcagtttc tccaccaaga tcagggtggc 360
   tggaaggtgc ctttgacaag atgaggatgg agccagacag tctttgggct tctcggtgtg 420
   ggacagtcac atgtggcacc cacagcctga gcgcacccgg ggtactcaga gcgggcagga 480
   gaagcacacg tctgggcagc ttggttccca ccctcctcca agccctgcgc ctgccccttt 540
   ggaggggaca gctttggcct cagagtttgg ccctagcaga ggttcaagta gcaccaaaga 600
   ttgtcagcct gccccgatgc ttccttgcct ggcagggatg cttgccaaca gaggtatcct 660
   acccttctcg ggattccccc ggtctgcact ttgggtggcc tggattcctg ctccaagctg 720
   tagacttgct tcttatggga tgaggagggc cttgggatcc aaaattgagc caagcccaag 780
   ggcagccggg aaagagcctg gtcttggggt gcacaggcaa aggcaaaccg ccttagggag 840
   acccagtggc agcgaacggg ttaggcgcca ccgtgctccc gcctgactca ccctcgaagc 900
   tctaaccact gtgggctatt tttgccagag aggggcccca ggctttccag ccgccccccg 960
   cccctgccag accgcagccc aaggcacggc tggccccacg cgccccaggc acattgactc 1020
   agactgcccc caagcaagca gaggaacagg gatgataccc acaaccacag tcacctcttc 1080
   cttggtggtg gcgttgcagc tctgctgggc agccagcctc cagtctgtgc tgctggaggt 1140
   cctgcccgtg ctgcaggggt cactgttgga tagcgggcac agcagtctga gacaggagat 1200
   cgagcaaact tacatctgtt gggatattta cagatcacct ttacgtgcca ggctgtgagc 1260
   agtctgggga ggtgaaagtg ccatggtccc tgacactgga cagcctggac cagactgctt 1320
   ttctctgatt ccctacctct gggatgggga agtgggagca ctagagttcc tgaccctcct 1380
   tcccatttag aggacaagag ccaaaccatg tgtggttttg gaggtcacca gaatctgcaa 1440
   gccccatctc atagttttca tccttacacc tggattcatg atccccacca gtgtccttat 1500
   cctttttctt gcagtcaccc tgggattcct gccaggtcct tggaagaggg gaatgaggtt 1560
   gcctaagcct ctcttctcca tggggacctc atggacattt gtatttctcc atgtgtctct 1620
   gggtccccct taggtttggg attatttttt gccttctgat tctacccttg tcaggctcca 1680
   gcatacagcc aggtgccaag aaggggcgag gggcagaact actacaatag gtttcaactt 1740
   cctggtgacc ctaggtctcc ccaagcagga ctgttccaaa tccaaggacc cagagtcctg 1800
   ctccttctct ctactgaagc ccaactctgg gacaagaggc cacaggccgg cagactgggt 1860
   ggcctgagct ggcccgcagt gccctcagcc cgaccggctg gcctgagcac tgacccgcag 1920
   gcggctagag ggcggtggga caggcggggc ctggagaggg ctgtgggtgc acacccattg 1980
   cctaataccc caagcacgcg g 2001
<210> 103
   <211> 2001
   <212> DNA
   <213> Homo sapiens
<400> 103
   cacaagttgc cctactgctg ccgttgatgt tctcatgccc caccagcttc accttgggtc 60
   tggttcccgc aagggtgagt tggagatcct tggtcctgag tgagaagtgc tttgtcagag 120
   gcagcagagg gcgccatggg agcacacagg aagggcgcct ggggctgtgc agggactgca 180
   gaacaggctg cactggcaca cagggttggc tgctggctgc tggctgctgg ctgctggctg 240
   ggtttcctct attagcttaa acaatggaag gtggacttgg ataggaaatc tacctcatgt 300
   attcctgagc tgtgtctgca ccgttcattc attcgtaggc gactcatgta ttcaagcact 360
   catttattta ttagatgctt gctgtgtgct tagcactgtg ccaagccctg tccttagcta 420
   ccaagagttt aaaacgtaat ttggaagaca ggatataaga ttatgtctcc cctttgtatt 480
   gcgactcact catcaagttc tcccagcatc tagcattcat atctggcact cagtaggtgc 540
   tcaacaaata tttgttgcat aaatgaatga aaagattatt agtaataata ccttttattt 600
   actgagctct tcccaacttt ccatgtattg tcgcaatcct caaggtaagt cctgatatta 660
   tcctatttta ttgataagaa aagtaaggca cagagaagtt aagtaaactg cctaaggtca 720
   cacagacagt ctgaagtctt aaccactact ccaggtgcat ctcagcagtg tgaggcaggc 780
   catgcttagg gccaatgctt agcacacaca gagattgcct caggctttca gaggacaaag 840
   cagtcaggag gggctggggc aataaaggaa ggcttcatgg agacagaaca aaataaccag 900
   tgcaggtaga agaagagaac catctttcct gatagagaga gggggcagca agcaagaggg 960
   agtcccaggg tggatcatcc atactggctt ccaggggtga cgggaaagga tgccagcaag 1020
   gacttcagca aggatcaatc agcaaatccg agggtgtctc tcttgccaag ccccgggctg 1080
   tgtgcatagg agaaggagaa catgaatgag aacctacctt tgaggatcta cagccaattg 1140
   ttggacagag gcaaaatatg tatataattt tactccagga aagcaagtgt tgagagccag 1200
   aagtggtatg agaatcatag tcaggaagac agaaactgtt tccaaatggg aagcacagag 1260
   cagtctcctt ggaagagagt gcattagaac tgagccgtgg gggcgggcag gatatcactg 1320
   tggagtaggg gaagggcact cctggggtgg caaggtggga ggtgggccct gtgttcccac 1380
   agtgggcagg gaggtagtga aagggaagct ggccggacag gcagggccat tccaagaggg 1440
   ctttgtgcgc agggctaagc caagctttct ccataggcaa tggggagcaa ctggaggttc 1500
   gtagcaggag aaggacacat cttgcccacc aggaggctaa gtaaaaacag ttgtctccca 1560
   agttataagt tcctggaacc cttgctggga gcaggattta gaaaaatgat gctgagagat 1620
   gctagaaaca tattcgccct gaggctctct cactcagact gcaagaggaa ggtatcatca 1680
   gaattgccct taaccaggaa ccagaatagc tgggtcccct tcctgccaag tcagcaacca 1740
   gctatgtgac cttgctcagg tccatctccg ggtgtcagtt tcttcatcta caatgcaaga 1800
   gggttgccca cctctgagaa cccttctaac cccaaatctc accctatgaa tctaagaaca 1860
   ccaacccctc gccatcctaa gtatcacaga gccaggcaag catgggtgag agctcagacc 1920
   atccttgttg gactaaaagg aaggggcaga ctgcccatgg ggggcagccg agagggtcag 1980
   gcccccatag gtcctcagcc t 2001
<210> 104
   <211> 1925
   <212> DNA
   <213> Homo sapiens
<400> 104
   ctctttcaag aaatatgata gtggggggag gggaaacggg aagatggtgg aaggaagggc 60
   tttctgggga gatggaggtt gggagcgact tgagcagttg tgagccagaa tgaaaggaga 120
   agcggaaggc aggagtttga aggcttcaaa gcaaggagcc tgtccctaag agagctccag 180
   agggagctcc gagggacaga tggagagtag ggttggtggg ggctcaatgt ggctggactt 240
   ttccaggcct gtgcaatgga agaggtggaa gtgttcatct ctgtggcccc cagcagctta 300
   atagtctttc cttcttcccc ttttcctctt cctaccttcc tcccttcctc tctagccaca 360
   aacatttatt gagcacatac tatgcaccaa gtcttgtgct aggttatgtt gctattataa 420
   tgaacaggac agtgtatatc atccttacaa ctaagtgagg agacaaatat tatacaagaa 480
   gatggacaaa tgtgtgtagg tctacatatt gtataatact aacaatgggt aatgttcatt 540
   gggcactttc aaggtatcaa gacctttata agcatttctt aataaccttt tacctaaaaa 600
   tccatcccct acattaaaac ttacaagata ctactgatat taccttttgc agatcaggaa 660
   actgaggcac agagggatca gatgactggc caaaatcaca caacttagat tcacctgttt 720
   ccagacgcaa gcacttaact gccacacggt cctgactatg ttgagagctg cggacaccag 780
   tatgaacaag acctgcagag tgtctagggc tgggaagaag ccacgaattc atttactgcc 840
   ttttcctcaa ggtgtggtcc ctggaccaga catatcagcg tccctgggga ggtagtcaga 900
   cacgcaggct cttcagccta ctgcgaccta ctagaccgtg atgtgtagtt taaacaagac 960
   ccgcaggtgt ctggtgtgta gttcaagttg gagaagtctg gtctagagcc cattcctctt 1020
   attagaaaca ggaaaccgag gcccaagcat ttgaaggtta tggacggtgg tagaactttg 1080
   gggtttggtg agtggagccg gcctaggccg gaatgggcgc ttccactctt ggcccagcag 1140
   gccagtgggc agggccgcgg gaaggacggg cgctgcaggg gctccgcgcg ctcaaggtgc 1200
   tcgcgggctc ccgggcccag ccgggctgag gtggcgtggg cgccctgcgg gccgggcctc 1260
   cgcggcgcgt cccagtccca gtctccgcgc tggtctggcc cggtccctca gctccacccc 1320
   ctgcctcccc ctcccgcccg ctcgcgcctc ctgaaaagcc agctcgcccg gggcggcggc 1380
   gcagagccgg gccggcgcac gaggcagcca gcgcggccat gacggcggag aaggcgctgc 1440
   ccctgggcaa tgggaaggct gctgaggagg cgcgggagtc cgaggcgccg ggtggcggct 1500
   gcagcagcgg gggcgcggcg cccgcgcgcc acccgcgcgt caagcgcgac aaggcggtcc 1560
   acgagcgcgg ccactggaac aacaaggtgg agttcgtgct gagcgtggcc ggggagatca 1620
   ttgggctggg caacgtgtgg cgcttcccct acctgtgcta caagaacgga ggaggtgagg 1680
   tgatagtgag gagaagggga gggggcgcca accgcccggt gggggcgggg agccaggggc 1740
   gagcgcgaga ccccctcccg cgcctgcgtg gagcggaacc cgagcggaga acctcgactc 1800
   caggcacctc gcgtgtgagc tcgccccgga gcgcggccca cctgtgccag tgcgcacgcg 1860
   cacgtgccaa ttcgcacctg agggttccac ctgccagcgc ggggacttgc ccgcgttctg 1920
   tcccc 1925
<210> 105
   <211> 2001
   <212> DNA
   <213> Homo sapiens
<400> 105
   tttcagccag ttttgcctct tgcactttgt ccctgcctgt ccccgtcccc gtctccctct 60
   ctgctccggc cgccttggcg cagccctaac tcgggcatcc gaagctccta gtgggcgcag 120
   aagccagcgg ctgctgcctg cccttcgcct ttcggaggct cacgggaact tggttccttt 180
   gctggggcct tgaggaccgg cctgggaagg aggggtggat aagagcagta agcgacaccg 240
   gggcctggct gggcaaggga ggaccctttg gctacaggcg ggagggaggc cacccccacc 300
   ccctaggtct tggccccaca ggcttggcgc gtttctgggc tggagttctg aatacctggt 360
   ggaggtccac ctgagccaaa gggcaaggtg gcagagatgc acccagggag gcctggtcct 420
   gcacccccta cacacacacc cttccgtgaa aggacaggta gcagggcata gggccagagg 480
   ggatattcag catcccctgc tcctttgatt ccacagcaga aatatccatg taccatagcc 540
   agagccctca atctagcagg gacccaggcc aggcctctgg aagcctcccc tctctcactc 600
   atggttctac ctcccactgt gcccattatg gcctgatggg gactgtccta gctagatgca 660
   ggcccaccta ggtgtgaaaa cccctgttct ttgtggtccc tgaggagcct gcctggccct 720
   tcctgcaggc ccgcctagcc acagctgcag ggggcttggt gcctacccca tctctcatac 780
   caccagcacc tgtgaccaga gatgtcccca ggccaccgca gcctcctcca cctccaccgc 840
   atccccacag ggagattcct gggacccctt cagaaccctg ctggggctta ttcgtgcccc 900
   tggaggggac ctgggaggct tgagggctct tctctgaggt gcagaaaggg gggcacttgg 960
   gggtcatcaa gtggctttgg caggaccgga acctcagctc cgcacccctt gagaggatct 1020
   ttgttcctcc ttgggcctgc agcctgcttg ggcagcgcca cccagacctg gaccttcctg 1080
   ggctgcagct cacagcttcg agatgcaacc cggctgcctc tgggtcctgg ggtcccccag 1140
   atgagcctgc agatctggtg ccaaatagac atcgttggcc agggtgcagg gtgcagggtg 1200
   caggggacct agaacccatc cctcacttgc tgtgttgtct ttccatctct aggtccctga 1260
   ctccccattt gcaaaatgac atgggaggtg ggctttcctc cctcagcctg gggccaaccg 1320
   aacacaccct ctggggaagt gagacccagg cctggagacc cacctgtgcc agctgacctg 1380
   agcagaggga ctctgatgtc ttcccccggc ttttgatagg atgaggctcc tttcttccca 1440
   ggactcccac agcaagctca atttagggct tcccaaggac ccctccctgt ctctcctatt 1500
   tgctgtgagt tcagcaaagg acgctgcagg cagctgatat atctcccaga actgcaggcc 1560
   cacccacaca ggataggtgc ttgtggtgac tggggactga cagccagcag ggcttttggg 1620
   agctgggaat tcccagcaga actgatctgg aatctggctt ccactttgct ccgggtgctc 1680
   cctgagcaca ctctcaacct ccgcaggccc tggcctcctc agctgtgcaa tggaggcaac 1740
   aaaagcacag gtaaggaaga attgcggatg aaggatgcac caggaagcac tcacttatct 1800
   gcatctgacc cctttccgtg cccctggccc ttgacctggc ttcccacctg cttcctttct 1860
   gctcctgtgt tcccgccatg ctgaccgcgt catctctccc cttcccactg gatgaccccc 1920
   ggccccagat cttcctgcca atgacttatc ccattcaggt ttgcctgaat ggagaagcca 1980
   ctccattgga gtagccactc g 2001
<210> 106
   <211> 2001
   <212> DNA
   <213> Homo sapiens
<400> 106
   cagggtgaag gagatggggc tggagaggct gagcagtccg ggttcgctgt ccgccacttc      60
   ggcgcggaat cagagcagga cttgctgagc cctcctaccg ctccctttcc ccctccccct     120
   ctgtcttccc ttctgtttcc ttttccctcc ccctggagct gtagcggcag cagcagcagg     180
   aagccgagcc gggttgagcg actcggaggc gagcggagga gctggaatat ggggagtcag     240
   cgaggacggt ggggccagga gcccttggga gggcctacgg agggagcggc cccaggcgct     300
   ttctagagcg tgagcggtgg gggagcaggc gcagggtggc acgagcggag gcggggcccg     360
   ggcgtggggc acggctgggg aagctgccgc ctccggccct gcccggctgc ctccgccgcg     420
   gccagtggct atggagcagg cgggcaccag acctgcggcg acagagcatc cacggctccg     480
   gcggcccatg ccctggctgc tgctactgcc tctcctgctg ctgttgctgc tgctgctacc     540
   tggccctgcg gcctcccagc tgcgatactc tgtgccagag gagcaggcac ccggcgcgct     600
   cgtgggcaac gtggctcgcg cgctggggct tgagctgcgg cgcttggggc cgggttgctt     660
   gcgcatcaac catctgggtg cgcccagtcc gcgctacctg gagctggacc tgacgagtgg     720
   agcgctcttc gtcaacgagc gcattgatcg ggaggcgctg tgtgagcagc ggcctcgctg     780
   cctgctcagc ttggaagtgc tggcgcacaa ccccgtggcg gtgagcgccg ttgaggtgga     840
   aatattggac atcaacgaca actcaccgcg tttcccgcgg cccaactacc agcttcaggt     900
   aagcgaatcg gtggcgcctg gagcgcgctt tcacatagag agtgcgcagg accccgacgt     960
   gggcgccaac tcagtacaga cctacgagct cagccccagc gagcacttcg agctggacct    1020
   taagcccctg caggagaaca gtaaagtgct tgagctggtg ctgcgtaagg gcctagaccg    1080
   ggagcaggca gccttgcacc acctggttct cacagccgtg gatgggggca tcccagcccg     1140
   ctcgggtacg gcacagatct ctgtgcgtgt cctggacact aacgacaact ctcctgcctt     1200
   tgaccagtcc acttatcgcg tccagctacg ggaggactca cccccaggca cattggtggt     1260
   gaagctgaat gcctcagacc cggatgaggg ctccaatggt gagctcaggt actccttgag     1320
   cagctacacg tcggaccggg agaggcagct cttcagcata gatgccagta ccggggaagt     1380
   gcgagtaatt ggggggctgg attatgagga agcctcctcc taccagatct atgtgcaggc     1440
   gactgaccgg ggtccagtgc ccatggcagg tcactgcaag gtgctggtgg acatcgtgga     1500
   cgtgaatgac aatgccccag aggtggtgct cacggacctg tatagcccag tgcctgagaa     1560
   tgctacaccc aacaccattg tggccgttct cagtgtcaat gaccaagact caggccccaa     1620
   ccggaaagtg agcctgggtc tggaggccac actgcctttc cgactgaatg gctttggaaa     1680
   ctcctataca ctggtggtga gcggcccact ggaccgagag cgggtggctg tctacaacat     1740
   cacggtgaca gccacagatg ggggaatacc gcagctcaca tccctgcgta cactgaaggt     1800
   tgagatctct gacatcaatg acaatccacc aagcttcctg gaggactcct attccatcta     1860
   catacaggag aacaatttgc caggtgtgtt gctctgtact gtgcaagcca cagacccaga     1920
   tgaaaaggag aatgcagagg tgacctactc ccttctggag agggagattc aagggctgcc     1980
   agtcacctcc tatgtctcca t                                               2001
<210> 107
   <211> 2001
   <212> DNA
   <213> Homo sapiens
<400> 107
   cccgatgcct ttcatctgct gtgcctgtgc tgagaccaca agacaacata ctgaaaccag 60
   gaagagctgc cccaaattct cagacactgc tctgaggaaa aaactgtccc ttagagtgag 120
   gaaaaaagga ggaaaaaggg gctggtcggc caggcctggc accctgaagt ggggcgggca 180
   gagtccgttg ggggaagagc acctcagggg accaaccaag gcactgacct tgaagaggcg 240
   aaggatgttg gccaccatga tggagaccga gctccctgaa gcaccgatga cacccaccac 300
   acgttcaggc ttggtgatga tgggtgggcc gccactgcca cagcggacct ctgtgccatc 360
   cttctcgatg agcgcctgca caaaggtcag cgactgctcg agggcatggg tgtccctgga 420
   gcaggtgtcc agaatgcggg cgcccagcgt gatgttaggc agcaggtccg ggtcgttgtt 480
   gatgcgatcc agggcgaaca gcatggcctc cagccggtgg atgccctttt ccttcttaag 540
   ttctccacag ggcttgccct ctgagccccg gccatgcacc gggaacaggc ctcccagtgt 600
   gatgtcccca tctatgcgga tggaattcat gtgagggtgg cctttgggct ttcccaggga 660
   ggaaggcatc caggggccgt aaaggctgag gagcaggcaa aggggcagcc gggcccacca 720
   ccagcccaag cctctcttcc caggcatctc ggaaatccct agagacccat gaacggcagg 780
   cccactccta gccctggcag gcccctggcc ccacggcctg ggtgggcatg ggcagggcag      840
   cttcagcagc agggggactg agggcagcca accgcgtagc ccatgctgct accctctccc      900
   acctccttgt cactcgggcc aagcacagtt gcccgcacag tccaggccca cagacagcag      960
   gcagtggccg gggttgcagg aaggctctga tccttgtccc gtcctgcagg cctgttctcg     1020
   gtggatgact gtggaaaggg cagaatgctc ctagcttggc gtatcttggc atcaaggaga     1080
   aaacagctcc aatcctctcc tcctaccaac cttagaaggg gaagagagag agagaatatc     1140
   aaacagaagc ccaaagaaga cattagctag tctcatctct catcaggagc ctgagagaag     1200
   gagatgctag aggttatcgc cgaagattcc ataaatctcc tgaacttggt ttcctctccg     1260
   ccattgctca aatctggctt aaatgggaga gtgtatggca gaaattaggt catgtttgaa     1320
   gcttcctaag gcctgtaccc actgctatca gtagcccggg catcagagag cacaatcccc     1380
   aggaacagcc tcgaactggc ttggaaggaa gaggggcctt cctcaacaca tccacatgaa     1440
   ggagagcaag aaacaggtcc tatgggccca atcccattac agagatgagg aaactgaggc     1500
   ccagagcagg agagcatttt gcctgagtca cgcagggagt gtgcagttta attacaagct     1560
   cagataaagc ttggacaatg cctactgcag cccagctttc ctgctgtcag ctctggaacc     1620
   cgagacactt tggctcttcc gggactcaga gacccaggaa cacaaataag gccccctcaa     1680
   gaaatcatcc tggaacgcag tggtgtaggc tgggagctgc tcactctgaa ggccaaggga     1740
   aggccaggga ggagctgagg ctgtgggggt tctggatttt catcctgaat cagggagcgc     1800
   aaggtggacc ctgtactctc ctgcttccac cccagctaca cagctacccc aggcagagca     1860
   ggggaaaccc agcagcccct gcatgaggag caggcctcca ttcagcttgc acatcctccc     1920
   tccctccctc cctccctgac tttcttcatc tctccatctc tccactgctc tttctcccca     1980
   cccccacttc ctccctctga g                                               2001
<210> 108
   <211> 2001
   <212> DNA
   <213> Homo sapiens
<400> 108
   atttgtattg gaatagcttt agatatactt caatgtaaaa aatattcaag attcaactct      60
   gatctgtcct gtgtgtagtt ggttccaaaa ggtagtgttt tagtcagttt ttagtcatta     120
   actatttata gacacactat tcaggagcct aaacatttca aaagaaatgt aaattattaa     180
   gtccattcca tggatatttg caaatatgtg gatgtttaga aataataatg gaatattttc     240
   accttgcaat ccaagttgtg tggacttaca tgagtattta ccttggtcta ttaatatttg     300
   caacatgaac ctatctttcc cacataactc tgaatgggct tagtttatga agttttatac     360
   aaatttgcta caaactaaca gctttagtta tacattctac agatacatat taaccatttg     420
   ctctctgcaa ggcactattt actgccttat tttacatact tctttctctt tagcccctct     480
   taagagattt ttggccaata agggaataat atatccccag tgcttttttt tttttttttt      540
   tgggacggag tttcactctt gtagtccagg ctggagtgca gtggtgcaat ctcggcttat      600
   tgcaacctcc gcctcccagg ttcaaatgat tctcctgcct cagcctcctg agtagctggg      660
   actacaggca tgttccacca cacctagcta actttttttt tttttttaat ttttttagta       720
   gagacagtgt ttcaccatgt tggccaggct gacctcaaac tcctgacctc aagtaatccg      780
   cccactctgg ccttgcaaag tgctgggagt acaggtgtga gccaccgcac ccggcctcaa      840
   tgcattttaa aagcatggta tgctgttgct aaaaccaata ttaaagggta accatagtga      900
   taaagtatac aagtatcagt catgatttta tagtttaaat tcttccacat gctaaccaaa      960
   attaaaacca attcccaaag gcaatgagta cttacatgac gattgcccat tttccttttt     1020
   ctgagatggt ttttctgtat ttcatcaaga gatcagtcct cctacaggca aagtttacaa      1080
   cagttttccc cccggacata atatctcttg tgtttaataa gtaacttttg gcactgaatt     1140
   tgaatacatt gttggggatg ggtagggagt ggctctagct acagataaac ttagactgcc     1200
   caggtttcct tggtacccat gtttttaaaa taatatactt cttaataaat aaagtttagg     1260
   cttttaaagt tgaatagctt cttcccacct aaaagactaa acctgcctca cttctgaatt     1320
   gttgagttat tctttcatat tctggtgttc atttaaaata ttatgccttt tagtaatgaa     1380
   agcattaacc aaatagaaaa atattactat tttttactaa cattgttatt attattacag     1440
   agaatttttc gaaggctaca ttgcattaca ctagatcatt taaaaaattt tactgagttt     1500
   tgaatttaaa aagtaatatg tgcagatggt aaatttgttt ctaataatac ataaaggtcc     1560
   tgtgaaaagt cggcctactg acttcctttt tccttccccc agaggttttc cgtgagtcct     1620
   tcctgaagtg gtccatgcat attcagctat gtccgtcggt ctgcctgtct agttacctac     1680
   ctctctgttt atcttgatcc aacttgtctt ttcttcctca ccaaatgggt ttgtacaatg     1740
   tattctgttc catgccttgc atatttcttg tctttccttt ttttttctga gattattaca     1800
   acatgtttct ttttttattt gtacaaactt atgtggtaca taagacattt tgttacatgc     1860
   atataatgag tagtgatcaa gtcagggtat tgagagtgtt tgcctgagta caatagattt     1920
   ctgttgagta cagccaccgt actctactat ccaacactga atttattcct tccatcttag     1980
   tgtgtgtttg taccctttaa c                                               2001
<210> 109
   <211> 2001
   <212> DNA
   <213> Homo sapiens
<400> 109
   agctttctgc ccctccgggg aggcggatcc cagcccccaa cccaccaccc tcatccgccc        60
   agcgccttcc ctgtcctggt cccgaactga cataaacagt aggttgccgg acacggtgaa       120
   gtccttcaga accgcccctg gtcgggggag aggtgcggca tcccgggaca ggatcctgcg       180
   gaccagctcc gcttccatgg gcgacgagaa aggcatcgtg atgtgcctgg tggggaccca 240
   gttaaggtgc catctcctag gatttggccg gtgcccatct catcccctca ggctgtctcc 300
   ccacgcactg gcccaggtcc tgcagcaacc ccacccagct gtcttgattc cccactttcc 360
   tctgttgcac ctcaagctga ctcctgcgtg cccttccaag gccctcccca ccatgccccc 420
   tgccgcatca gccgggacct ctccccatcg cttgacacca cccccatcca gcccacaccc 480
   cagccaccgg ctcccacctt gcacgcttcc ccccaactcc tgatcccttc acacacccaa 540
   gaacccctgt ctcctgtccc cctgcccctc accccatcgc ccagcaccca actgtgcaca 600
   cgctccacct cccatcccct acctactccc ttcctctgag ccacccctcg acctccaact 660
   cccagccccc acccccaccc cacccggccc cttaccccca tctcccaaac cccaccttcc 720
   gcacctggcc ctgccctcac ccacctcctg gccctgcccc ccacctcttg accctgttgt 780
   ctccgtctca cggcccccac ctcgccacca gaacctccta gaacagaaca gaacaggata 840
   caactgaagc aggcggctgt ccggcctcct ggccccgcag gggcaccttc catcctgcgc 900
   agaagcggca ccgccatgcg gaccccgcgg ggcgcctcct ctcggccccg aggcccttgc 960
   tgcccctgcg ccccggggac ctctgccgcc cgtggcaccc gcctctcctg ggccgccagc 1020
   attgccccct gggccatcag gaatgccagg gcctcctggg ccatcagcat cgcccgtcga 1080
   accccctgtg ccctggcctt cggcctgcat ggctccggag cctctgcccg gctctcagag 1140
   agaaggtcag ggcccacgag gatgcggagg cagagaggct gcaggaagtt ccgccccctg 1200
   gcgtgagatg ggcagcccgg gatcctcagg gcgcctgcgc acaggggccc tacttccggc 1260
   cctgggagac cccgagtgag ccccggagca cgtgaccggt tctcaccaac cccgcccctc 1320
   cccaagagag cccgggccgg aaggtggccg caatgccagc ttggacccct cacccgtgag 1380
   cagccggctg tccgccggac ccctgtcccg ggagccctgc agggagtcag gcactgcggg 1440
   gcccagcctg tcccatcccc cgggtctccc tcacatcgag gagcaagacg ggcctgggaa 1500
   cacggggccg ggactgtgcg gccatcgtcc cggaccctgc ctgccctgtc cgtccttggg 1560
   ggagcgccca ggacagaccc cggggggcag gcctctaact gggctcagca gcctccgtcc 1620
   ctgtcctggt cgcccagctg gtggggtagc tggaactgca tgtctggtgg gcgtggcctc 1680
   gcgccagcca aggtctccca ggcgccaggt ccagccaagg tggacgggag ggcccagcgg 1740
   gacccggggc cggatctcac tcctgggttc aacgggttct gtccctgtcc tagtcgcccc 1800
   gcctggtggg ggagctggag ctgcgtgtgg ggtgggttgc gggtggcctc ccaccagcca 1860
   ggggctcccg ggctctaggc ctggcccagg tggacaggcg ggacctgctg gacctgaggc 1920
   tgggtcgggc ctggctcctg ggttcggcag tatcagtccc tgccctggtc gctcttgcct 1980
   ggtagggagc tgggaggtga c 2001
<210> 110
   <211> 2001
   <212> DNA
   <213> Homo sapiens
<400> 110
   cttgacaccc cctccgggac tgagccctcc cagccagagc atcaatgtca ttaggtcagc 60
   ccatcagccc tgggtagtcc tcctctgccc tctgggtcct ctctgatggc atcaccagag 120
   tccacagatg tctgactcca gccctctcgt tctaaggctg gggaatctga ggcccagcca 180
   cggggggcgg tgggtggggg gagtttgccc caggccacac caggagtctg ggtggagaag 240
   caggaatgca tgctctcagc cctacattcc acagctgccc tgcctccagc acccccaccg 300
   cacccccctc cccgggtcac tacggacagg ggagatgtag ctctcccttc cagctggctc 360
   atcctgctgc atccctctat cctccaacct cggtagctgc cactcacctc gggtgggggt 420
   ggagggctgg agcctgcact gaaaggctgg gctggtggga agtcctgtcg ggaagtgagc 480
   ctggaggtgg gatggccacc tatataggct tctgctctgg ctattttggg aggtggcact 540
   ccccgtagcc ccggccccct ccccttccgg tgtcaggtac tccccaaccg tggtgctggg 600
   tgtctgtatc acattcagcc tccctgggcc catcaggcca agactattaa taactcttgg 660
   cccctgcttc cctccagcac ccaaaattga ggcccaggca gggctcgcct cttccatgaa 720
   gccttccgtg accactgtag atggcagcga tgcttccttc ctccatattc tgccaggaaa 780
   ggaagagaaa cagcaaggaa aagaatgaaa caagaactgt cctctgtgca tggggctgag 840
   ccccattcct atgctgatat gatctctcac tacagccttg ccaggtagac atagctgctc 900
   tcgcacagat gaggaaacag cataaagacg ttagatgtta agtgacctgc tgctcaaagc 960
   cacgggcctg gggttgggag cggactcagc acttttgtgc gaggatctag tgcctggtag 1020
   aaggaaatgt tccgcatatt ctcagacaag tcccttcctc tctctttctc agtctttcca 1080
   cctgtaacat gagctcaatc agaggtaaac tgtgccctat agatggtcta gggcgagggg 1140
   aagatggacc ccctgaaatt gtatgcgtgt gacattgagg cacaaaggcc caagcccttc 1200
   atgatttgca aagagatctc tgacattgtc tgttttggat tggagaggac cagctgaggg 1260
   atgtgttttg agaagcatga atgaccaaat gaattcaata aatacgtagt gaatatctaa 1320
   tctgaccacc atgaaatgtt cctctcctca tccctaacct gaaaagaatc tccagggcac 1380
   gaagattgtc aatggtgggg tctagttctt cacagtgaca cgaggaatcc caggctccat 1440
   gcaaaggctg tttggaagct ccatggatag aaagggccga gggcaggctg catgggggaa 1500
   gctgggactg gggcctcctt gaggtgtggt gctggggcct gagctaggtt gctggtggtg 1560
   gcaacaggaa ggagtgtgca gataggggag cacctcttgg agagaagctt ccagaattgg 1620
   ggctgtggca tgaacaagag ggacaaatcc aagctgaccc taaatattca aggtggggtt 1680
   tgctgaagaa ggaaggtgtt gactttgaag gcagggagtg tagggaggag gtagtgctca 1740
   gtcgaaggga tgatagagta agtggtgttg aatcctagga tgttagggct gggacagcct 1800
   ccgtggtgat cggctacaaa cttctacaaa tgacatcatg ttatagatga agaaaccaag 1860
   gccagggtga cttacccaaa tcccccagag agtagtggaa gagccaagag ctgatgctgg 1920
   gtttagggga cctgaaggac accctggcgg agatgtgtct gaactctgga gtgaggtcag 1980
   ggctggaccc tgggggcttt g 2001
<210> 111
   <211> 2001
   <212> DNA
   <213> Homo sapiens
<400> 111
   caggatttcg gcctcctctg ctccgtgtgt tggctacagg ctaggttcaa atcccaattc      60
   tgctctttac tgctgaaagg ctttgggcat gttcgttaat ctcattttct cttctgaaaa     120
   agagacaatc ttgaaaagat tgaccttccg tgaagatcaa atgaagtaat gcgtgtagtg     180
   tgtctggcac gtcgtaagcg catcacacac gtgagctgtg gcaatactaa ggagcagtgg     240
   caagagctgg gggaggagtg gagacgaaca gacctggaca aattagtgac ctttctggga     300
   cccctacaga actcttctgg agaaattaat tctatttttc catgtacttt taagtttgct     360
   tttcctagca tttcttctac attcctgaat aattttccac tcaaaaattt cactggttct     420
   ccctgactcc catattaatc tagacataat tacttcccca gaacagttct gtgggcaggg     480
   agggctctac actatgcacc ctccaaaatc cccaaagctc ggaccgtcct ggcctgggtc     540
   cccactgctg tcctgcccat gggctcccag gctgtgcttg ctcttctccc accccaaccc     600
   catcaccaca aaaagcccct cctccaaccc ggggactttt gtaatttcct acctgaaatc     660
   atccactccc tacctctaac aatctgtgat gagcagagga caacatcaag tttgaattta     720
   gaggtaggga atgggtgatt tcaggtagga aattacaact tcactttgaa gtttaatttt     780
   gtaataaact tcacttaatt ttgaagtaaa tttgcaaagt cagcaaaatt ctttcaaagt     840
   caaccttgct ctggcctagt tgcttattta ctgctaagct ggatgttttc attaaaagat     900
   taactttgag acttcagcct cttctttagc ctgaactgtg ggtagtgatg tggtgtgttt     960
   cctaacacct ccggcagtga gccaggcttt gagtggctgc gtctaaacac ttctttccct    1020
   gaggactgga agacattaga ataaggtttg tcattgagtg tgttctgtgt gcttggaact    1080
   gagaagggtg atgaagtgcc agataggatg ggaatggaag atatcgtgga agccaactat    1140
   gagtttgcaa ggcaaggacc ttggcaaaga tggtcctagg gagacaggtg ttagggaact    1200
   cagagggcat gggcaaagga agatttggtt tcaaaaagga tacagacaga gaaggttaag    1260
   tctaggaagt tgagtcttag aattgtatgg tggaatattc gtgggtgtcc ataggaggaa    1320
   aaatgggtta aaatgttttc acttgacagc gttctgaaaa cctaaaaggt attgctattg    1380
   ccttcaggga tatatttagt attctgacca ctgcttcccg gccacaagca aaagaaaaca    1440
   gtgacatggc tatgctccta aaaaagtaaa gaagttaatg tgtctcaaac aatgtagttc    1500
   ctttgagcaa taagcttatt tggaacaaat gggttacaaa gtcccactaa tgaagagcct    1560
   ctgattgtgt taagttgaat ctacagatca acctgcggag aactgccaag aacgctaatg      1620
   aagacaggga ggtactggta taaagataga taacagatca atggaacaga ataaagcgtc      1680
   cattagcagg aagcaaaatc cgcaaacttt tattcttaac tactactata ttctgcattt      1740
   tacaagatcc ttcaattctc acaagatccc tatgagatag acagtaataa cctaatttaa      1800
   agatagatga gaattggagc ttgtctagaa tagattgagt ggaacatcac ttgtggtata      1860
   tcatttgaga ggtaatggga atattgccaa attcccaggt aactctcccc tcaaagggac      1920
   tcctgttgta ctctaaatcg tatcagctat caaatgatga gctatgaaag tctgtcttct      1980
   tcgactgtaa gcttcttgag a                                                2001
<210> 112
   <211> 2001
   <212> DNA
   <213> Homo sapiens
<400> 112
   cttttgacac cctaaggttt acaaaaatcc attaaaacag ccatcagaga tccctgtcac      60
   aaattaattt ttccattata taagacatat ggctatagcc taaagtaatg cctcccataa     120
   attatgatga accatatgga gtaaattcat agttatcgaa cactttagct tcaaaacaca     180
   cttaactatt aagataaagt gatttctttc ctaatatctt aaccaaataa tttttaaaga     240
   ctcgttcaag taatattaac tatccaaaga gttagtttta tagaagaata aagatgctac     300
   aattattttt aactttacac aaggaattac tcttcaacag atgacttcca cagccaagga     360
   cacaaaggat gatactattc taagccagtg ctaataagcc tgacatttct tcaagcttca     420
   ctattaaact ctgattcaaa ttcgtttgta aataaaagac aagatcataa ttaggtacat     480
   acaggtaact tcttctccag acgaaatctt tccatagtaa tatagaaatg ccttgtttct     540
   gatgatgtaa tgcagtaact cccttgaaga ttccagggac agtgagcaca tatctgattt     600
   aggtcactgt gctttgcaga gatgaattct tctaagttac ttttatggta cagccaatca     660
   ctgtgttcta agttacctaa tcttccaagc taataactac aacttacaca gggtcacagc     720
   ataacagatt tggaaagtaa tatacacatt tagttataag taaaaatacc aagaggaatg     780
   cagcaatccg ttttcaactg aaacctttct gcaatgttag tacaaagagt gcatactcta     840
   agaatatatg tggatggatg tgtgagagta tggtttataa ttatcattgt aaccaaaaaa     900
   caaagtatta atatggacaa tagctagaaa aaaaaaagag tagcacccct ctaactactg     960
   gacttgggat tctctacatt cttaaaaatt cctgaagacc gaaaaggacc ttggtttata    1020
   taagttacca catcaggatt aaaacagaaa acttcaaagt acttattcag aattatttac    1080
   acaataataa agccattaca tgttaatata cataacattt taatgaaaat aacttttcaa    1140
   aaacaagaca aaacttagag gaatacctct gttttacatt tttacaaatc tcttcaatta    1200
   tctaacttaa tagaagacaa ctggattctc atatctactt ctgcagtcaa tctgatgtga    1260
   tctgcccttg gttaaagtat atttaaaaat ccatattcac acagatattc tgctggaaag 1320
   gggaggcata tttcaatatt ctcttcaggt agttatggac attcctctga tagttcatca 1380
   aactcaacaa gtatattgtt tcttaatagt tgcaacgtag aatctgaaac cattccaaat 1440
   gaacatttta cacattgtta catcaaaatc catttgtcta ctatgcactt tgaatggatc 1500
   tctcacattt ttggaatgta tgtgttcaca gggtattaga gcttttccaa atgtcacatt 1560
   tcattaaaca atatcaaaaa tttttttaaa tcacacttgt taataatatc accaacaatc 1620
   tcataagtcc aagtattgag aaactgtcaa agtcaggtta gcagatgcaa gttttccaaa 1680
   attctaagtt ttgcctgaaa gtctgaatta ttatcatggg taacaaacac tatcagttgt 1740
   ttttcttgaa gtgacaagtt cacttcattc atttttgaca aaatatctgt caaatgccca 1800
   agtatgatta accaagtctg tcattttttc tttcaggtaa aaatggtgtt ccatgaaaat 1860
   aacagctagt tgagttcaca attcaggcaa ttaaccagtg atttttctcc aaacaatcat 1920
   tctatttgga tatgcagcag aaatgttttt acaaatgctt attttgtcat tcagaatatt 1980
   aaagcaatac acaaaggcaa a 2001
<210> 113
   <211> 2001
   <212> DNA
   <213> Homo sapiens
<400> 113
   ggagccagag ttgcctttag ctctgcaggg attctcagcc aagaatcaga attttctgtc      60
   tgtgattttc ctcaaataaa cccagaccta ttgaatgaga atctctgctt atcagacctg     120
   tgcatccttt tcgcctctta catagcccca caggtgattc tgaggcccac gcggcccgct     180
   tcccctccct gcgtcaggcc cggccctagc tctggaggtg gttggattgg ttcaagggct     240
   gcagccccac ctcgcgtctt cccaccgcgt cggaggacgt aggcgcctgg gatcctggaa     300
   ggcggccagc ctctgcagcc gggcccagcg gctcctgaga ctggggcgga cctcacacct     360
   gaccccggag cggctaatcc tgcccaaccg tcccacctgc cacgtgccct cggggctgga     420
   acgcggcgcc ccgggagagg cctgctggtc agggggcgct ggtgcgcagg cgcgcggggc     480
   cgccgtcgca gctaccttcc tgcgccgatt cggagagggc cccctaatct ctactcggcc     540
   cgcaccagca gcgctggcgt cagggcgcgg tgggggcaag acgggtgact ggcagcaggg     600
   cggtgccggg ccgagaaggg cgcctcacct ggcgcctcct gccttccttt ccacctcagg     660
   ttgcagagag ccgccagggc aggtggaagg gccacgcccc gctcccgtga gggtcaccga     720
   gagacgttgg gggtggggca gggaccccaa ccctagacca ggtagggggc gggcgaagat     780
   tggtggtacc ttcaggtaag agggagcggg gagcgccccc tcggcttggg gtgccagagg     840
   tgtgcggcgg gagcgccttt tttcgtgggg ccctcgccct gcagggcgga ggcggcattg     900
   cgttggcctt agttcctagt cctggttccc gaggagcccc tgaaggcttc ggatgggaag     960
   tttgcgttgg agtccaacac ccgacaggaa ccccagttaa cgcctccgca tggccagggg 1020
   cagagttctt gacacctgta gcttaatcaa cgcaggtgga gattcagggc ttgaatcgcc 1080
   cggccacgcg caagccgcag ggcaccctca tactactact tctgccttat tacccttaaa 1140
   actggagtct cagttatttg tatcccacta ttggtttttt tttaaatctt ttttcgtggg 1200
   tttgccccct tttcctcagc tgcgtttctg tcttcagtcc tgtagggcac aatgtctgaa 1260
   caagagcgta tacaggaatg tctgcggaag gaaataaggt cacttctcat ttccaccaaa 1320
   gatggtttga gcccacagga gttggagaag gagtaccttt tgatggttgg caaccatcta 1380
   ccactccgaa tccttgggta tcggtccact atggagctgg tattggacat gcctgatgtt 1440
   gttcgtgtct gccccggtgc aggtggtact gtaatactga aaggtaggtt taagattttt 1500
   gaaggtctat aaactttgta ataaaaacaa ttgcttagta aataattatt ctagttctgc 1560
   agttattatg catcccagcc agtggatttt aatttggtgg gggagggaga ctcataggtg 1620
   cttttgaggt tgtgggcaaa attggaacct ctgcaaaatt aacaaaccat tcccccaaac 1680
   aatcgtgcac tcacaaaagc tacattttta catatgattt caggttcttg caccttaagt 1740
   cgagagaaac aaaaaaagga cttcttaatt ataaaaacat gttttaaatt tcgtgaagca 1800
   caagaaaaaa ctattgtagg gaagtttgca gttcatccca agagtttcaa aaaatctgtc 1860
   aggggtcaat tagttgtaat gataatacat tgagttaact tttcttcagc tggcaggaga 1920
   gctagcaagc tgttgcccaa aatgcaagaa atcgaggaac caagagttac tgtttatcgt 1980
   gctgcctgga taaacagctc c 2001
<210> 114
   <211> 2001
   <212> DNA
   <213> Homo sapiens
<400> 114
   ggtagctacg gaaacgtgga gaggacccac tcactccgtc cagcagtagg acttgtcatc      60
   atccacacga acaacaccac aggtagggtt gtttgtgatc attcacgcac acacacattt     120
   agccctccag aatttgacca ccaaggaagt accttaccgg ctccctcggc ttcttccttt     180
   gtctgtgcac agagttgtct ctgcagtgtg tgaggatgct ttaccctagg ttgcctgctg     240
   gtttcttttc acattgctga gagctcaggt tatttcttgc actgggtgag tcctgatttc     300
   tcacccctga ggccatcaca agagggtggg gcatacctcc tcaggagaga gaaccagaga     360
   ctgcccctga aggggaatgt aatcacgagc aagcccccaa attgttataa ataaagtttc     420
   cccccagtgc cgcaaaagaa atagcactcg aatataaaat tttctttttt ttcttctcag     480
   caaggcaatt tacttctata gaagggtgcg ccctcacaga cggagcaaag gtgagcgcac     540
   acctggacaa gggaggggaa ggtggttctt atgcctgacc cacgtgattc ctgatgctgt     600
   gtccttcccc tgttggctag ggttagacct ataggctaaa ctaattccga ttggctaatt     660
   taaagacagt gagggggtga gtggtttggc gggaaaaatg gttaccgcag agcaggaaat      720
   cggaatgaac cacggtgaag aatgagtgag ggaggagcag gtaatcgaaa aatgttgctt      780
   tatgaggaag ttaagtttaa aagtagaagg caaagaattg aagatactga catattgatt      840
   cttttaagat aaatttagaa ctcatatcta acagctggca tgtctggcat ggggctccgg      900
   ctggggcgca gtcttcagca tttggggctc tggggtgcag ggggcagagg acggccgggg      960
   ctgtggtgcc caaccggtgc taaactcaag cttgggaggt cgcccacgag aaacagtcgc     1020
   ccttccccac ccgcctgctc ggcttggcgg ggagcgggat gtcgctgggg gcgatgccag     1080
   gcgggccgcc tctgatagcc tctaggctct ggcgcgcttc cccgcccctc ccgcgcaggc     1140
   gggcgtggga tcccggctga tccagtccgg gttttgcggc ggagcgggcg ggctgcgcgt     1200
   gcggcggctt caactgtcgc ggtaggcagc agcaggctga agggatcatg gtgcagtacg     1260
   agctgtgggc cgcgctgcct ggcgcctccg gggtcgccct ggcctgctgc ttcgtggcgg     1320
   cggccgtggc cctgcgctgg tccgggcgcc ggacggcgcg gggcgcggtg gtccgggcgc     1380
   gacagaggca gcgagcgggc ctggagaaca tggacagggc ggcgcagcgc ttccggctcc     1440
   aggtgactgc cggagcgtag tgggatgggc gcggcctgag ggtactcgca gcggcacttt     1500
   cagccgccgg acccgccgag ggacagggga tggggcgggc agagcagatg tgtaactctc     1560
   cagaatctct ccttgcccta agatattccg cactttctga gccaggtaga gcacggggga     1620
   agaaaggaaa cggtagtgtc aaggcaggtg gtggaaggcc tttccctcac tgggtcaaag     1680
   aggagttttg ctttgtgcca aagaatctca cgattgctgc ctattggaat gcgaaaacgc     1740
   aagcgtgaca accagtggga tgccaggggt tgaggacttt ggaatcgcag agtgtaatgg     1800
   ccaaggggat ctggagagct tagagttttg ttttttgttt ttttttgttt tgttttgaga      1860
   cggagtctcg ctctgactcc caggcaagag tgcagtggtg cgatctcggc tcactgcaac     1920
   ctccgcctcc cgggtgcaag cgattcttgc tgcctcagca tccccagtag ctgggattac     1980
   aggggcccgc cactacgcct g                                               2001
<210> 115
   <211> 2001
   <212> DNA
   <213> Homo sapiens
<400> 115
   acattaaatg gtctccttat ggttaggata acaaaaacat gaagaaagca tataagattc 60
   caactaattt gacgttgtga attgtgaatt ctgaactgaa accaagtaag tcccttatga 120
   tcaggctcca aggttttggt cagcctctca aaattgagag gctgaaaccc tttgaccaaa 180
   attaacacat ttgccccttc cctccagctc ctggaaacca ctaccctaac tctgtttcca 240
   taaaatagta aactatttta gagtccacat ataagtgaga tcatgtagta cccaccgtgg 300
   gcttatttta aggctctgag taaatcacca tctctctgtg ctagggtatt ctgagtcagt 360
   cttgttataa aatattttgt ttaggtaaca tattgcaaaa cgtttcataa ttcaatggtt 420
   cctttcccag aaaaaaagca gtgaaagttc caatagccaa aaagtgtcta actcaactga 480
   cctttgggaa aaaatatccg tataattcca gatagtccta acttctgaag gtaatatagc 540
   ctggcctctg ttgataaaca aacaaaaaca aaaaaaccac acacacacac acaaaccagc 600
   ttcttgtctt taagtgtgca agaatgttag cacttaaaat ttttaagata cacaaaaact 660
   aaacagaact gttcaagttt ttaaagggag cttggaaatt tgaagaaagt aaagttaaaa 720
   atattgcccc atgagtcata taattatttt tctttttatt tctcatagct tccctcctat 780
   ccattattta ggcaattttg tatccaaatt taataattta acaccttatt tgttacaaga 840
   acacgttact atgttatttt aagctttttt gggcaaatta cacataatta gataggctgg 900
   gttagggctg tctacaagat cccagcctag agtgtggtgc agtagcctat tttctctagg 960
   actcttgctg caacaggata tgggagaagg gaaacattaa cgttagcaga atgggaggaa 1020
   tcagattatg gagacatgat ccttgccttc tggcacctct ccagctgatt tagaagagga 1080
   cacagaggga gacagacata tggccatttc tataattata aactctgcta catgctgaga 1140
   ggatggcttc tcaaagccag ttagtgtaca agcagtggag gttgggactg ccctacttct 1200
   cctgcctagg gtcacacagt ctggcagatc tggaaagact tccctgagaa agtgaccttg 1260
   gaactgatac cttagtggaa ataactaggc attgggaggg ggtgaggtga ggagaaggtc 1320
   aagagctttc atgtagaggg aactttgtgc agagacctta gtcaggaaga ggttttgcat 1380
   agtggaggaa ctgaaagcta acagccaggc tacagtacag agcaaatgat gctggggtgt 1440
   gaggtgatgt caaaaggtag acaccagggc tagagggcgc ataatcttgc aggccgtgct 1500
   aatgattttg ttttctaccc aagaatgcta cttcaggaga tcagtcaaaa ggttagggtg 1560
   atggtgacgg tggaaatata gaaaaaggaa tttattcaag aggtattcaa aaagaagtga 1620
   aatagagatg agttggagat agattggata aaaagagtga gggagaggga ggtgtcaggc 1680
   tgcagcccag gttctgagtt gccaaagagg agtcttttcc tccaggtgag ctaaggctta 1740
   gaaccacgat ttttggtcac tttttcttat tttcagtgtg agaagtggcg agttggctgt 1800
   ccaggtacac actgtcatct tacacttact gtttaagaca gaggcgcata tttggaaagt 1860
   gaatgagtca ggagccagag gagaaggtat gccggcagag cctagggcgg agaggaggaa 1920
   gagagtctgg gggcgcgtcc ccaaaaagga gacagggaag acagagcaga ggccggggag 1980
   gggagagcaa ggaccatgac a 2001
<210> 116
   <211> 2001
   <212> DNA
   <213> Homo sapiens
<400> 116
   ccgggacctc atccttaacc ccttcctggg tctcagctac ccccaccctt tgtctctgtc 60
   ccctgcagcc actggagggg tccctctcaa acttcactct atgtggatct ctgcattttc 120
   caaagtgctt ctagtaagct cccccttggc cagaattact ggcaaaacaa aattcatatc 180
   cctaaaaaat aggctttcat gctgcgttaa ttatgaataa aaaattaata aaataagctc 240
   ttcctcagaa gtaaaaaaga ttaaagacac aaataaatat aaatattttt ttttaaaagt 300
   tctaggagct gggagaatgg cttgagccca ggagtttgag accagcctgg gcaacatagt 360
   gagaccctgt ctctaaaaaa atgaaaaaaa aaaattagct gggtgtggtg gcacgtgtct 420
   gtagtcccaa ctacccagga ggctgaggca ggaggatggc ttgagcccag gagtcacccc 480
   agcctggatg acagagtgag accaatcaac taacaggggg cacctcccct actcaagcac 540
   catccatggc tccccactgc ccaccagatc aagatccagc cattcaaact ggccttcaca 600
   gtcaactcct gcatccctcc agcctccaca cctctcacac tccaaggcca gggaaagcca 660
   tctgctaagt cacaccctcc agtttctctg tgcttgagct ctaccccctc tcctgcactt 720
   gttgaaatcc aacctctcca tgcagtccca gctcaagtgt cacatcctcc acgtcgcctt 780
   ccctgacctc atgtctgcct cctccaagca gcaaaacttc aattacttgt gtctcctctt 840
   cttcaccagg agacatgccc cacacctccc acgccctctt tccaagggcc cagcaccaaa 900
   tgagagcgta ttcttagaaa aaagagggat aaggaagtct tctaagttta ccacctgctg 960
   gggcaagtgg agattcttgt cttctcatct gtctgcccac gtgctaaggt cagccacaca 1020
   tccacccacc gactccggag gacagacaca tccagaggcg gagctgactc tgtctccaca 1080
   catctggagt agacgggttg gccaaggaca acatcctggt cagagggtca aggtgtctgg 1140
   cagtgggtgg ggaggagtgg cggagggcgg gttggcgggg agcagaacca gtctggtgct 1200
   gcttgttgag gttggaggtc ttcctggagg aagaggctgc agagcctcca ggggtgggtg 1260
   ccggggaagc ttaggcccag gatccagaga gaggggaagg gctggttccc aagggctggt 1320
   ggagccagag aattcctgag ggccccagag gaggcttctc gggggggtca gtggggcccc 1380
   agggggtgat ggtttagaga tcgctgtcca agcttgggag caaacctgga agtaagggcc 1440
   atggggggtg ttggtgtggg aggagtgtgg tccagagcgg gacttgggcg ctaatgcggc 1500
   ccagcagtgg caggagggcg gggagcctga tggtttggga cttctaggtg tccaagtctc 1560
   tgtctctctt catctctcca tctcagtctc agtctctctc tctttccatc gctctccacc 1620
   cctccacacg cctatttctt gttctctcct tttctagggg gcagcgctgg agtccccgcc 1680
   cagaaccctg cgggagcccg gggcgggatg tccgggggcg gacccctcgg cacggccggg 1740
   cggggttaaa gggttgggcc gtgggacggc gcggtcagat cccaggtagt ggcgccggtg 1800
   gatccggatc gagggcagga ggctgagacc cgcgggtgag tgcgcgcgtg gtgagggctg 1860
   taacctgagc gcggccgcgg cgctgcgctg gaacccgagg ctcgcgcggt tcggtgctgg 1920
   cggggcaagg gcgaaggtct gcgcgcggcg gcgggcgggg cgcagccggc cacggggacc 1980
   cccccaggac ccaggacggg g 2001
<210> 117
   <211> 2001
   <212> DNA
   <213> Homo sapiens
<400> 117
   ctccaggatg gacttttata tcttgataag cctgggggtc accatgctgc gtagggaggc      60
   cggagacctg ccactgacgg tggttatccc atgtcatggc tttcttcatt cccactttac     120
   ctttttgagg ctggaaggta tttaggggat gacagaaaat caagtttgaa ggtcaatgga     180
   aatattgggc acccactaga atgtcccaca tctgtaaata catgtgcatc tttgtaaaca     240
   cttaacagct gttttgggga gtgcttgttt aaatccacag atggcatttt gccagaggcc     300
   tccttcagtt ccctatttct ctcactcaag gtatcctagt ttggagatcc agaccaggtg     360
   cagcccatgt accctgatga accccaggtt cccctctcca acatctttct cacccgacag     420
   gtgcggccct gaaatgggtt tctactgtct cctcacacag tccccatgtt cccctcctcg     480
   ggttagtttt agggtagtct agcctgggaa ggaatttggg actgatcatg ccacgttccc     540
   tggaatggga ctagaaagga atcctgggct tcgtgcggac caaggcccca ctcaggttgt     600
   catggaaaat gaggctgaga gcccctctct gcagcaccct gtgggacgga acccgaggtc     660
   tctgctggtc ccggtgggca cagacctgca gccaacccgg tgttccgtga agccctgatt     720
   tcccaatgaa gcccatggaa gccatcactg gtttccatgg gagcggacca tggccacccc     780
   tgctcctgtt ccctgtggga aaggaccaag gccgcccctg gtcctggtca ctgtgggaag     840
   ggactgtggt caccgctggt cctggtcccc ttgggaggga ctgtggtcac cgctggtcct     900
   ggtccccgtg agaatggtct gtggtcaccg ctgcctgttc cctgtggaag ggaacgtggt     960
   cactgctggt cctagtcctc ttgggaggga ctgtggtcac cgctggtcct ggtccccgtg    1020
   tgagggactg tggtcaccga tggtcctggt ccccgtggaa gggactgtgg tcaccgctgg    1080
   tcctggtcca ggtggaaaca gaccgcggct accctggtga tggaccccac ggggcacacc    1140
   cgcgccaccc cgcacccagg gctcgcgccg cccgaggccc ccggttcctg tccccctccc    1200
   cgcctggcgc cggaacctgc gagctggggc gcggcctcgg ggaggggcgg gcgggacaga    1260
   cccagccgcc ccggctcccc cgccgtccgc gtctgcgccg gccccggggc ctggtcggcg    1320
   gcggcggggc cggtcgatgg cccgggcggc ggcggcggca tgcggctcct gttcctggcg    1380
   gtgctgcggc cacacaccgg caacgcggtc acggcccagc gcgttcggta ggtgcagggc    1440
   gccggggcct acgaagcctg ggccgggggc cggggggcgg ggacgcggga cccggggacg     1500
   cggggcgctc agccaggccc cctccagccg cgccggggcc gtcccgagcc gcgcgcacaa    1560
   acggatgggc cggtgccgcc tgccgggcgc gcgggggtcg gtgccttctg cgtggcgcgc    1620
   gtgtccccgg gtctccgtgc ggccggcgca ttggcctcgc gctctccgga ggggactgag    1680
   caggtgaaca ggcccggagc ctgtcgtgga ggggcccggg aaggcctcgc tgcagaagca      1740
   ggatgggagc aggatccgca gggatgcgca gcggggtccg cggagctccc ggcgggggcg      1800
   cgtttccagg ccggggaccg cggtgccagc cctgccctcg cggatcgggt ctcggctgtg      1860
   tgccggcgtc cggggtcctg tccccgaagt ccctttccca cctcccctct gccctgtcta      1920
   ttctcgtctt cctggctggt tagctccttc acactcatct ttttctgcct ttgtcttatt       1980
   tgtttacttg ttggtctgtg t                                                2001
<210> 118
   <211> 2001
   <212> DNA
   <213> Homo sapiens
<400> 118
   tgtgtgagcc ggggccttgg ccaggggaag gacgctgctc agagctcaca gacctgggtg 60
   cccgggccct gacgactcac acaggcccgg agctgggaga ggatatggtg cagggtgtga 120
   aggggctggt ccaagacatc ccccagggct gggtcagtgt cagcggtggc ctccagaacc 180
   ttcagcgtca gggccagctc agcctccaaa gccacggggc gctccctcac ctgaggagag 240
   gtgagaaaga gcaggtgagg ggggaggtga ggggaacagg ttgggggagg aggatagaga 300
   ggaacaagtg aaggtgacag gcacagggga gagggcacag ccagtgtggt caggtaggag 360
   cagagggaag gggtagcagg tgtggggaga ggagagaggg acaatggaga aggagaaggt 420
   gaaggggcca ctacagagcc aggtgagcag ggctgggagg gcaggggtgg gcctgactcc 480
   ccctctcacc tgcagctgcc tcaggtccca ggtcctgggg aagaggcggg agcggcactt 540
   gcagtccttc agcagaagcg actcttccta gacagcaaag gcacaggtta gccccagcag 600
   gaggggtgga ggttagacca ctctgatggg attggaggat ggctgacaac aggatagggg 660
   aggttaactg ctgggaggat ggtagaggac cctcttcttc aggaaaacat gagtcagtcc 720
   ctgcagtagg agcatgagat agcccactgc agggagggtg gaggctagcc cagtgaagga 780
   agctgggagt gggaaagcat ggtgaccctt ggagtgcggg tggaggctag tccatggcag 840
   gagggcaggg ggagactcac taaggcatct ttggccctct taaaggcctg cagctcctgt 900
   ggagacaggg acttgaactg ggctatgtgg cagccccttg catccgggag agccccgcgg 960
   agcctggcga caggaactgc tccagtcacg gtcagcactg cggccatcag caccagcact 1020
   ggcatgcagt ccccggtcat gtctgtgtca cagagagaaa gggagctgag ggaatgcaga 1080
   ggctgcccac tgagggcagg ggctgcagga gctgagcacg gacagagatg tgggactcac 1140
   ctagtttcat tcctgatctc tggtctttgt cagcagaaga aacactctga ggctgtcacc 1200
   cagggtctgt ttgggtcttg tctggagtct ctgttctttt cagtcccctc ttctggatct 1260
   cagactgtgt cctggctttg actctttctg tcagttttga gctctgtctg ggatgtaatt 1320
   cctgcctgag ctccatgggg cagcttttat ccctgacaga agggcagtcc cagctgatgt 1380
   aggaaaagtg aaaacacagc ctcaggtaag acaccggcca ccaggggagc cccaggctgg      1440
   gaaagcccag agcagggcag ggctagtgag ccagatgagc agctggaggg aaagagaaac      1500
   tgatggagac tcaggggtaa cctacaggaa ggtatgttcc caagaggatt ccacctgctc      1560
   tggttttgtt gttgtttaag acagggtctc actctgtcac tgcagtgcaa tggcatgatc      1620
   acagctcact gcagcctcaa actcctgggc tcaagccatc ctcctcaccc agcctattaa      1680
   agtgctgaga ttacaggcct gagccaccac tcctggcata tatatatata tatatatata      1740
   tatattttga gacagggtct tgttcggtca cccaggctgg agtgcagtgc aaaatcttgg      1800
   ctaactgcaa cctccgcctc ctgggttcaa gcaattctct tgccttagcc tcccaagtag      1860
   ctgagattac agatttgtgc caccattccc agctaatttt tatattttta gtagagatgg      1920
   cgtttcacca tgttggcaag gctggtctca aattcctgat gtcaagtcat ctctctgtct      1980
   aggcctccca aattgctggg a                                                2001
<210> 119
   <211> 2001
   <212> DNA
   <213> Homo sapiens
<400> 119
   ggtggatcac ctgaagtcag gagttcgaga ccagcctggc caacatggcg aaacccccct      60
   ctactaaaaa tacaaaatta gctgggcgtg gttcgggtgc ccgtgatccc agctacttgg     120
   gaggctgagg agggagaatc gcttgagcct gggaggcaga ggttgcagtg agccgagatt     180
   gtgccactgc actccagcct ggcgacagag caagactctg tctcaaaaaa aaaaaaaaaa     240
   aaaaaaaaag caagacaaaa ggacttctct cctgtcacag actctgccct ttctgagagt     300
   ctgggaccct actattgtca ctcctaaatt ttgtttgttt gtttgtttgt ttgtttgttt     360
   gtttgttttt gagacagagt ttcactctgt cgcccaggct ggagtgcagt ggtgtgttct     420
   cggctcactg caagccctgc ctcccgggtt cacgccattc tcctgcctca gcctccccag     480
   tagctgggac cacaggcgcc cgccaccacg cccggctaat tttttgtatt tttagtagag     540
   atggggtttc accgtgttag ccaggatggt ctcgatctcc tgacctcgtg atctgcccgc     600
   ctcggcctcc caaagtgctg ggattacaga cgtgagccat cgcgcccagt ctgtcactcc     660
   taacttctaa tctgctctag ggagagtccc aatagtccat cttgcatact gaagattaag     720
   tgaatttcac agccaacacc acacaatacc ttctctattg agctgtaagc tgcctctgga     780
   ctggatggtg ttttgttttt atttttaccg tcagcaccag tgcctctcac atagaattta     840
   gccaagccgg ttaggtgtta agtgctgctg ctgcgctgtc agaagggcac acggggctgg     900
   ttctagaccg gcctctgctg cttactcgtt cagtaatctt ctacagcaag ttcttgaact     960
   tctctgggcc tgttttctac ctcgccgagt ggttaactgc gtttaggatc ttggaagcac    1020
   ctaagcacga aaagcacata ggccagaagt taagatgtcc cttggggacg caaataccgg    1080
   gaatttctcc ggctgttccc accgatcaag caccagggct ctcaggtcta gctccttcgt      1140
   ctccccaccc ccacatatac cctcactttt tcttcctttc cctatgcctc gcagtccctc      1200
   aacagggtct gcggtcagac gtcactccca ggtgactggc aatcagggat gccgcctcgc      1260
   tcacctggcc acccttgaag attgtggatc ctgcaccggc tagccggctg gcagagggcg      1320
   cgccaacagc cgccagccga agtgcccccg cgtaggtggg aggagtgact gcctctcacc      1380
   atcccccggg gatgcacctg tggagagacg gcccgagagt tccagggtga gtgcgcagag      1440
   gagcttatct gcctgcctgc gggtcggtgt gtcctgccgg ctgctgggcg ctggcctgcg      1500
   tcgcctgctt gtctcgctcc ctgtccctcg tccgctaccc cctctgcccc ccctccctcc      1560
   agcagccggc ctctcggtca tcccgccggg tgggtgcctg cgaacctcac tcctccctcc      1620
   tctgcaggag ccaactggcg agccgcaggc cgccgggctc ggcctgggag cccataaaac      1680
   ccgcccgagc cgcgccgctg cgctccagcc gctgctcacc aggcagagcg agcgggcttg      1740
   gctggggctt cctgccctga gcgcgacacc gactgccccg gaccgcgcct ccaagcaggc      1800
   tgaagggctt ccgctcttgg cttccagaaa agtttggaga aagagaattt gaggcggatt      1860
   ggagggtggt agcccctccc cagccttctt tccctcccag aagcctcact ctgcacagcg      1920
   tcccccattc ttcccgtcct gattccccat cttcctgacc cctccttctc cctctcctgg      1980
   gtcgatccca gtcacatttt c                                                2001
<210> 120
   <211> 2001
   <212> DNA
   <213> Homo sapiens
<400> 120
   gtagcgggga gcaagtcttg ctctgacagg gagcagagca gcaggcggca gagtccaggg      60
   ccccacaccc tcatgctcac ttccccgctg cccatgcaca gagcatagca catgcacctg     120
   agccacctgg cactcctcaa tctcagcaaa gattttcaag ggccacgacc ccagtgagta     180
   caagggagcc tctgccctgc ggcttttctg tatctggaaa caacactgct gaggcctcca     240
   gagctgggtc tgctgcttat tatctgtgtg accctgagca agtcacctca cccctgtgtg     300
   cctcagcttt ctcattcata aaagtaggag ggattggacc ttcctcatct ggatgctgcc     360
   atgatgaaat aaagcatgtg ggacagtgtc gggtgcaaag atgccgctgg gctcccaact     420
   cagtgctcat tctctaccaa aatgaatagg aggagggtga ccataattga tgctcctaac     480
   tttggggcat ttttgaaagt aaaagggggc attgttaatg atgccagcac tgcaaacata     540
   aaccagtaca gtcctgggca aacaccatgt ggagccacca tagtgccagc aatatcatct     600
   ctgtggagac aggactcggg ccccacccta agatccaggg actcccaagg gaggctggaa     660
   accctggggt tttccaaaag aggctggggc actgcaggct tttattagtt cctagtcagg     720
   acacactagt aaataagaca atgtgtcctt atccccaggg cacagcccag ccccacccca     780
   gccgcagacc cacctgcttt ggcagaggct atttactgca atggtatttg taatagaatg 840
   aaataaaaaa ctagaaacag ccttaatttt tatcagagta ggactgataa aatatattac 900
   agtacatata ctattcagct gtttttaaaa tgagagagca aggcagctct atatcgactg 960
   atgggagaaa tgtcgtattt tgctaagtga aaaaaagcaa cgtgcagaag agtgtagata 1020
   gcatacttcc acaggttaag actatatagt tgaatatgaa cagacgattc aggaaggaaa 1080
   acaagaaact agggaccctg gtgttcaggt aggcagctcc ggttgcagtc tgcccctctg 1140
   agtcactcca ggcggtgacc tcccccaacc cgccctcatt cctgaacttc tttcctggca 1200
   caggactcac tgtgcccctt cccgctgtgg gtacaaggtc tgccccccac cccagctctc 1260
   caaagcccac cggcctccct ggaggccgag gtcgacggcc cgtcgcaccg ggaggggggg 1320
   ctcccagggg tgccccacgc acggtcaagg tcccgcgcca agcggggacc gggctgggcc 1380
   ggaagcgggc acggtactcg cggcaaacta gcgtgggcga gtcctgattg cagtcggacc 1440
   tgccgccgcg gcacttaaca gtttgcagag tgcttcccgc ccctgatctc attggagcct 1500
   tcggacagcc cagcccatgg ccaccgatgc ccccatttca cgcctgagga agcggaggct 1560
   cagacgggcc accagcccct ccggaggctg gcccgggagc gcctggcagc gtcgggtcta 1620
   ggagccggct ccctcctgct ccctcctccg cgccgcccgg ggtgtgcccg ccgtctgtgt 1680
   gcaccactgc tgagcccagc tccggcgccc tcgcctctgc tgtgggcccc ggggacgcgg 1740
   ggtcaggcca ccgcgttggc caggccgctg caggtaagtg ctggacgggg cggggcgagg 1800
   ctgggcggga ccctcagggg cgccaagcgc tcctggcgac cccctgcggc agggctgggg 1860
   cttgagcctg ccagtgcgct gggatggggc gcgaagcggg ggtgcgggga gcccgccacc 1920
   ccgtacctct caatcctccc tctcgcctct caattctggg cccattaggg gtcccggctg 1980
   cgggaaggtt ccgagacacc c 2001
<210> 121
   <211> 2001
   <212> DNA
   <213> Homo sapiens
<400> 121
   tatgatgggg tcttgggtca caccatatag cctatgcaaa tagtgtgatt tgtagctagg        60
   gctgtcttag ttcatttgtg ctgctataac aaaataccca agactgggta atttataaat       120
   aacagaattc tttctcatag ttaaggaggc taggaagtcc aagatcaagg tgacagcagg       180
   ttcagtgtct actgagggct gccctctcca cttctagatg gtgcctagct gctgtgtcct       240
   cacatggcag ggtcggaggg cagatgggag ggagacagct cccttgtacc tctttcataa       300
   gggcattaat cccattcatg agagatctac tctcatgact taatcacctc ctaaggtttc       360
   accttttttt tttttttttt tgagacagag tctcactctt gtcacccagg ctggagtgca       420
   atggcatgat ctcagctcac tgcaacctct atctcccagg ttcaagcaat tctcctgcct       480
   cagcctcccg agtagttgag actacagacg cctgccacca cacctggcta atttttgtat      540
   ttttagtaga gatggggttt caccatgttg gccaggctgg tctcgaactc ctgacttcag      600
   gtagtctgcc cgtcttggcc tcccaaagtg ctgggattac aggcgtgaac caccgaacct      660
   ggccaggttt cacctcttaa taatatcaca ttggtgatta catttcagcg tatgaactac      720
   ggggacacat tcagagcata gcaggggcct agggccatgc gatatcagct tgacctctgg      780
   aggggctaag actcaaccag tcacatttgc acttgaccat gtctccaaga caaatctcca      840
   gtggaatctc tggacatcaa ggcccaggtg agcttccctg attggcagta ctccagctgt      900
   cttttcatac atcttgctga aagaagtaag agctgtccgt acaactcccc tgggagacga      960
   cagctggagg cccctggctt gttgactgct ggaccctgcc gtatgtactt gtgccctggg     1020
   ctgactgtag tctgcatttt ttgctgtata gactctaacc atgagtataa gtggcttgac     1080
   tgagttctga gtccttctag tgaattgtaa acctggggat ggttttgggg atccctgacc     1140
   ttgcagttag tgtcagaagt gatggtggcc actgggaggc cgaggcaggc ggatcacgag     1200
   gtcaggagat tgagaccatc ctggctaaca aggtgaaacc gtgtctctac taaaaataca     1260
   aaaaaaaaaa aaaaaaatag gcgtggtggt gggcgcctgt agtcccagct actcgggagg     1320
   ctgaggcagg agaatgatgt caaccctgga ggcagagctt gcagtgagct gagatcacac     1380
   cactgcacac cagcctgggt gacagagcga gactccatct caaaaaaaaa gaagtgatga     1440
   tggaaccctc aaactttgta tttcataaag cctaaaatct atttattctt gcatcagcta     1500
   cttaaaagtt gttcctaagt tggtgcaaat aaggcttttg agagaatctt ttcttattac     1560
   ttctccttgg agaatgtaat tgttgttgtg taattggtat gaaggtttag gcctggaatg     1620
   gactattttt agtaaattag atataactct tttctaaatc ttcttctttt attcaatgtt     1680
   tgaaactttg atttgttaaa cacacaagaa agtggttcac tttttgaaaa gagcttgact     1740
   taacaagtga agaagctggt agagtgtggt aagtaccctc tcccatctgg ctacaatggc     1800
   aatggagaat gttatagaac aatcactcgt tagcacagtc tgagtttgca ccaatttgaa     1860
   tactgaattc aaatactcag atatacctga agattcctct cctagaataa aaatgtcaga     1920
   atctctgcaa ggaactaagg atatcagagc tctggtccaa gatgaagagt ctatcaccaa     1980
   gtgcctcctc cccacagaaa g                                               2001
<210> 122
   <211> 2001
   <212> DNA
   <213> Homo sapiens
<400> 122
   gtcacctccc agctccctac caggcaagag cgaccagggc agggactgat actgccgaac        60
   ccaggagcca ggcccgaccc agcctcaggt ccagcaggtc ccgcctgtcc acctgggcca       120
   ggcctagagc ccgggagccc ctggctggtg ggaggccacc cgcaacccac cccacacgca       180
   gctccagctc ccccaccagg cggggcgact aggacaggga cagaacccgt tgaacccagg 240
   agtgagatcc ggccccgggt cccgctgggc cctcccgtcc accttggctg gacctggcgc 300
   ctgggagacc ttggctggcg cgaggccacg cccaccagac atgcagttcc agctacccca 360
   ccagctgggc gaccaggaca gggacggagg ctgctgagcc cagttagagg cctgcccccc 420
   ggggtctgtc ctgggcgctc ccccaaggac ggacagggca ggcagggtcc gggacgatgg 480
   ccgcacagtc ccggccccgt gttcccaggc ccgtcttgct cctcgatgtg agggagaccc 540
   gggggatggg acaggctggg ccccgcagtg cctgactccc tgcagggctc ccgggacagg 600
   ggtccggcgg acagccggct gctcaggggt gaggggtcca agctggcatt gcggccacct 660
   tccggcccgg gctctcttgg ggaggggcgg ggttggtgag aaccggtcac gtgctccggg 720
   gctcactcgg ggtctcccag ggccggaagt agggcccctg tgcgcaggcg ccctgaggat 780
   cccgggctgc ccatctcacg ccagggggcg gaacttcctg cagcctctct gcctccgcat 840
   cctcgtgggc cctgaccttc tctctgagag ccgggcagag gctccggagc catgcaggcc 900
   gaaggccggg gcacaggggg ttcgacgggc gatgctgatg gcccaggagg ccctggcatt 960
   cctgatggcc cagggggcaa tgctggcggc ccaggagagg cgggtgccac gggcggcaga 1020
   ggtccccggg gcgcaggggc agcaagggcc tcggggccgg gaggaggcgc cccgcggggt 1080
   ccgcatggcg gcgcggcttc agggctgaat ggatgctgca gatgcggggc cagggggccg 1140
   gagagccgcc tgcttgagtt gtattctgtt ctgttctgtt ctgttctgac agttctggtg 1200
   gcgaggtggg ggccgggaga tggggagggc agggccaggt gggggaggag gcgggggaga 1260
   tgcgagtaag tggttgggtg ggggtggggg tgggggttgg gaggtgggga tatgagaggc 1320
   cagctgcgag gaggggagga ggtaagggct gggaggtaga gggggtgggg gtgggggaga 1380
   ggggctgggt gattgggtga ggggcaccgg gtcatgcggg gaggggagca ggggccagga 1440
   gataggggga cccaggtgta ggaggggatc aggaggtggg gggaaggggg caagatggga 1500
   gcgggtggcg ggaggtggtt gggatggagg gtcgagtgat gaggaggggg ccgggtgatg 1560
   ccacaggggg cagggtgggg aggggtcttg taagggcatg gaggagtcag cttggggtgc 1620
   agcaaaggaa agtggggaac caagacagct gggtggggtg tgctactggg ccagtgcctg 1680
   gggagacagc ctggggggga tgggctgggc aatccggggt aggggggagg ggtgggggag 1740
   tgggggaggg ggaagctggc ctggggtaag cagcaggccg gagccacagg ccaagtccta 1800
   ggagaggatg ccttaactgg gcccccacca gctacctcgc catgcctttc gcgacaccca 1860
   tggaagcaga gctggcccgc aggagcctgg cccaggatgc cccaccgctt cccgtgccag 1920
   gggtgcttct gaaggagttc actgtgtccg gcaacatact gactatgtca gttcaggacc 1980
   aggacaggga tggggcctgg g 2001
<210> 123
   <211> 2001
   <212> DNA
   <213> Homo sapiens
<400> 123
   gacgcgcgct cccctgcgcc cccggggcct cccgtgggct ccgtgcgggg acaaagccag 60
   cgccagcagg aagagtgcgg gcaaaggggc gccgggctta aggggccgca tgttcgcaag 120
   ccgggaggag agagcgggag actccgggag gatcccgacg caggtccgga gggtgcgcgg 180
   cccaagagaa ggccagcggg accacagcgc ggctacgcgg ccggccgcag tcttcaccgc 240
   gcgcctgccc ttgtctacgt cccgggggtc ggctggagct gcactgggac tcggtcctca 300
   gtgtgccgaa gcctaagcgc tgcggggcgc ggggcgggaa cgggaggcgg tgcctggggc 360
   cacggggtcg tcccccagga tgagggcgtg tcccagcgcg cgggaccctc ggaagtccgc 420
   gctgggccgg gcgggcacca gcctcggact cagcgggtct cagggctccc tgcgcaacgc 480
   ctgcctcgga tccggacccc gggctcgctc tctggtcgcc gtccccggga ggacccagta 540
   gggtaactgc cgcgtcgccc cggcggttct ccctgggctc tgtctcccgc cgcctccacc 600
   ccccgagcct cggggtccgt cacggcttcc cctggctggc ggggtcagta gaacccgcgg 660
   cgcctaggtc cggacggaaa aaagcagggc cggggtgcgg cctggatgag cggagatctc 720
   cgcgccttgg gctcaaaggt gcggggtgcg ctctgctgcc gagcccctgc tcgctcagga 780
   acactggcca cgccgtcacg ccagccgccc ctgccccagg tctggaggcc cgacctgctc 840
   tcctaggcgc agcaccgcgt tctcttccgc gtgggggagc ggcgggcgga agaggtctgg 900
   ggctgggcac cggggacacg cgcccagctc ccctggcctc cctgggggga gtggccggtt 960
   tcagtgcttc cccaggtgaa atcgccaagg ttgaccagtg cgcccaaaaa agcagccggc 1020
   tggatttttc ttcaaattag ggaactctta taaagagttt tagtgttaac aacttaaaaa 1080
   atgaaattaa ggccagtcgt tctgattaaa gtgcgcctct ctgatcaaac cgcgctctgt 1140
   aaactgttgt tgttttagtt gtttcagccg cctgctttat tgctttatgt taagtatatt 1200
   gaaaaatgtt gaaattgctc gctaatcaat tgactactaa ttatctcctc atatgcaaag 1260
   tgcaaacagg tgaaaggaaa aaggcaggaa taactaaggg aactgaggct ccattactcc 1320
   ttaggcggtg cccaacgtta cattactcta caaaaagaaa tcttgtgggt cacagatcct 1380
   gaataatgac ctcaactcct tcaaatagtt tatctaggct tggcatgtcc aacatgccta 1440
   agaaattggg aagcaaagca agctttgcag gagccctgtg aaatcgtgcc tttggatgaa 1500
   gctcttttga ggaagttgtt acacaggtga agactgcttc ttccaatcgt ttcactgtta 1560
   agcacgcata ggaatctggt atttcctaag caaacacagc agtgtaagag aatggcttcc 1620
   accaagggag acccacaaga gtcttcctag aaaaacaggt taagggtctg aaggccctac 1680
   ttaggtggaa taaacgcttt ctcacagctc tactgaagtg aaataagaaa acattagaag 1740
   ttcacagaca ttgacaggca gttcaaaaat taattttaat tggaaaataa aaaaataggg 1800
   actagtccga aaaacgatgg ggaaatgcag tatgaaataa tgctggagaa taagtgatac 1860
   tgaaaccatg acaaatggac tttttaatca accatctatc cggagcaaaa gacagcaaaa     1920
   cctggaagct gaattatttc cctcttcctc tacgttgaaa gctagaggaa ataaacaaag     1980
   caatgaagtt ttcaagcctg a                                               2001
<210> 124
   <211> 2001
   <212> DNA
   <213> Homo sapiens
<400> 124
   agagcattcc tggagctcaa gctcctctac aaagaggtgg acagagaaga cagcagagac 60
   catgggaccc ccctcagccc ctccctgcag attgcatgtc ccctggaagg aggtcctgct 120
   cacaggtgag gggaggactc cctcggagtg gatgggagga gggagcacag agactggcta 180
   gggtctcctg gggaggacaa ggctctgaga ggagacagag ggcttttgtt gaagcctgag 240
   gaaacagaac accagagagg gacaggggtc acaacaggaa agtcacacta aactgggatt 300
   gataaaaagg gaggaaaatc aattgatcat gttttccaag ttaatcatca tttgtcatta 360
   ccatttgaaa aaaaagaaaa atgatagaaa tcagaactgc attaggatga cactccaaat 420
   aaaaatataa caaggaaact aaatgctgcc cttactcacc aatcagaagt tgaaaaataa 480
   ccaccagata cactcattaa ctcatccaca agcatttgca atcaatttta gtcaatggca 540
   tacaacaagc atcagacaag tctcagtcat cacagagctt atgctgtcat gaagaggaaa 600
   acacacacac aaagagatat agaatgtgag gtcaggtgtt gacaagagcc ctggaaggaa 660
   cagagcaggg aaaggtcaga aagaaaagac ccagggtctg tagagggggt gtcagggaag 720
   ggatctccca agaatgccct gatgtgagca ggacctgagg ccagtgggga gggagccatg 780
   cagacccctg gggaagagca ttccacacag ggaaatgcca aggtcaaagg tgctgaagga 840
   atgggggtgt cacactgctg actttgactc agtaggacac acacacacac acacacacac 900
   acacacacac acacacgctc caacgtggag gggtgaagag acctgctcag gacccagggc 960
   cctgtttttc caccctaatg cataggtccc aatattgacc gatgctctct cctctctcct 1020
   agcctcactt ctaaccttct ggaacccacc caccactgcc aagctcacta ttgaatccac 1080
   gccgttcaat gtcgcagagg ggaaggaggt tcttctactc gcccacaacc tgccccagaa 1140
   tcgtattggt tacagctggt acaaaggcga aagagtggat ggcaacagtc taattgtagg 1200
   atatgtaata ggaactcaac aagctacccc agggcccgca tacagtggtc gagagacaat 1260
   ataccccaat gcatccctgc tgatccagaa cgtcacccag aatgacacag gattctatac 1320
   cctacaagtc ataaagtcag atcttgtgaa tgaagaagca accggacagt tccatgtata 1380
   ccgtgagtat ttccacatga cctctgggtg ttgggggtca gttctacttc ccacatacgg 1440
   gattgtcagg cctgggttgt gcctgtggcc ctctctgcat tacatcctgt atcagggttt 1500
   ggacatttag tgcaggacac acacggggga gacaaacttc cacagatcag aattcctttc 1560
   ctggatccag accctgcaga cactcgctgc agaggaagga cagtctgatg gggggactca     1620
   gcagggcgag gtcagtgtca gacaagcacc ccatggtctc cccatggacc tgaccctgag     1680
   aaagaccctg gagaactgga tcagggcctg gcctgagggg tccccctagg gacccctcag     1740
   agagaagctc aactcttccc tgaactgagc ccctgtacaa agccctgtcc ctggttcctg     1800
   attccaggtg atcctgggga gtctgtgcca gggctgtgtg gtggcctctt gggcagggct     1860
   ggctgggagc aaggatgtga gctatccaag ggccatgact cctggagttg gtcaccagcc     1920
   aggtccgagc acccagagcc tcatctagct gaggataggg cctcctcctt cacttgaggc     1980
   tcagcatgga aaggacagat g                                               2001
<210> 125
   <211> 2001
   <212> DNA
   <213> Homo sapiens
<400> 125
   tgggcccttt ctggttcctt taaagcttcc cctgaattgt cctggggtgg tcctgaaatc      60
   aagccagagc ccatttagta taatctcatt gatccattcc tggagtggag tctgtgagaa     120
   tccgggtgac ctccacacct caaggccgcc ttgccaatgc tgagatacct ggatactggc     180
   cagaaccacc agtcactcag gatcagggcc aaatgggcag tttccacacc tgccctggta     240
   gacttgaaac catggggagc caagcagagg aaagaaacca ctgacacaga agccacggaa     300
   atgtgctgga gaggccacat ggaggccaag gccccagggt accacagaga ctgcctaacc     360
   tgtcgggtgc ttctggcagt cctggaacaa tatggcctga gagtttagtt catactaagt     420
   gaataagaac aggctcaaag ggcatgtgac tgtgaccaca ctagccccag agagggcatg     480
   accagctctt aaaaaccagc agaggaagcc tcctgaccca gtcacatggg gaaccatgtg     540
   gaggccgaga tataaaggaa gaggaggaag gagcatttca aggaagaaac agtcccgcct     600
   aagagggtca ccctaaaaac aaggaagtag gcagtccagt cctgacctgc tcggagttga     660
   agcagtcggc gttggaagag cctgaacacc cggcctacca cttgtccttc catctctggg     720
   ccttcaggac agagcggact cccctctccc ctaacacaga cagactggtg acagtaagcc     780
   tgagaaaggg gctgcttcgg tctccagcca caactctgtg aagccaagcc acgcgctgtc     840
   ttccagagag ggaatagaag tttccatcct gtgcacccag tggttgagca agaaggatgt     900
   gacgggaccc aggaaggcag cagaagccag caccaacaga gtcagcatcc tggcactgca     960
   gcgcagatct cagctcctcc caagagcccc agggtgtggc gagagaaaag gtcatgagca    1020
   aacacctctg gctgaaagta gccggaaagg cctggctact tatgggctaa gggttttgaa    1080
   tctgggctgc agattgtgca gagagttaat ttacaacctt agaacagtgg acaatgcttg    1140
   atagtgtcag actgtgggca ctggaaaccg gctggtctca ggatagggat cctatacctc    1200
   caacacttgg attgctgacc tttcacttct caacttggtc aacggaagtc aaaaacccca    1260
   gcaaggccac ctgctgaatt ctagaacttc caggacttcc cgacctagag cacatggcca 1320
   aacactttct gcagccagga tgtcactgcc ctcccctgat taagataaat agacttggcc 1380
   taataattat ccaaatagat taattcttcc aacaaaagaa agagttctgg aagtcaatac 1440
   ccaatagcct tctttcctga tggtgcagta gagagcaaaa tccatctcta agcccagagt 1500
   tcgtgatatt ttggaaatag ctacaatggt gaatttcacc ccctggagag ttctgtcact 1560
   ggcacttgtt ccagggttcc agcagagtcc ctcttctcaa ataggaagca ctggctcaga 1620
   agactgtccc cagcataagg taggggactg attgcaccag cagcctgagg gtgattccat 1680
   ggcagtggaa aacatctggg tgatctccac ccaaccccca cgcctgctca gccttcctcc 1740
   gggtcaggcc aggtcagctg ttctgcccaa gctacaggca cagctgctag cccactgctg 1800
   tgtctggaga ctaggtaaga gagaaggaat agcaaaaaaa tcaccttgga ttctcacaga 1860
   aacccctgaa tgagtaaata cagggaaggc ttttaccagg aagctgcctc aaagggaaag 1920
   cagggggcaa aagggtttga cgccagtgtt tgccactcct gctaagaata agcacccctc 1980
   tggaagagag tggggtgcta g 2001
<210> 126
   <211> 2001
   <212> DNA
   <213> Homo sapiens
<400> 126
   aattaaagaa cagcaagtca gtgccactta aacggtaatc caaccaccgc cagtcagtga      60
   acagcttttt actgctccat gaacaggtaa gtatagaaat tgtgagtaag catttagaaa     120
   taaaaacaac aacaacaacc aataattgaa aaactaattt tatacttttt ggcatactca     180
   ttgtacactt gtaataaaaa ctgggcttgt agtttttgtc tcttttctta tttgctagca     240
   ggttttgatt tgtattttac agaaataatg gtccatgtca gattggaaat aataaaaact     300
   tgtcttttac ccttgatagt ttgagaaaaa agtaattcag tggggttaga tatggcatcc     360
   gagagatgat tcgggaaagg tgggccaggg ccatgtttat gtggaccctc caatgcctca     420
   gagttcacac ttgtttcagt gggcaacagg actagtaagg tttagaggga ctgttaaccg     480
   aaggtgagtg gactttgcac attttatggt attttttttt tcccagtagg catgtgctta     540
   ttctttcttt tcttttctaa ttaaaagttc tgtttggttt ttttagttaa gaggagagac     600
   tggaactgct cagtgtttca caaagataac tgtttgtatt tgataagagt ttcgttaaac     660
   aaaggaattt gcacttatca aaacataaag cattgaaaac actgagcaca tactctgtga     720
   ccagaatgcg tgtgacttgt tgagataagc cacaccgggt aatcagatga gtctcctttc     780
   ttcttgggca ccctcatata attcaggcac aaacgaaaga gaaaacgctt tttctgatct     840
   ctcagttcgg gtcatctctg ccttccatca tcaccgccta acaatcctac acctggcagg     900
   gtgggaaaac ggtttaataa tctcttccct ttgggaggcg tttgacagct cagaaagcat     960
   ggcctttact caggctcaga gacacacaaa aacctttcac gttttccttt cagcttcgca 1020
   gccccgtcct tctccacctg cacgggcacc cactgaagca caggaccctt ctgccaagcc 1080
   atgagccttc tcaggcccct gccgcccgcc tcgcctgagg ctgcgcttct gaaaagcacg 1140
   cggccctggc ctgcgcacca cccagtctga aggcgacgcc cctgcacgaa ggagtcaggc 1200
   ctaggggctc gggacccagg gcgacactca attttctaac aggagccaca gcccagaagc 1260
   tgagaagcct tccggccgat ctgtggccct gaggtcgccc ttggagggcg agtgagaacc 1320
   ccccaggcgt tccggaggtg gcccccagcc cgaacccggc gcccgccccg gcttcccgag 1380
   tcaggtcttt gccccgcctc tcggccccgc ggcctggccg gcaagcaggg ctgcagtcac 1440
   ggggcggcgc ggagggcccc agcccagtca ggggtgtggc cgccgccacc gtaaggctag 1500
   gccgcgagct tagtcctggg agccgcctcc gtcgccgccg tcagagccgc cctatcaggt 1560
   gtgtttgcag ccgaactggc agtgcccgcg ggcggctggg ccgaggcctt ggctcctacc 1620
   cgcgggggtc gggcgggctg aggctggagc aggccagggg cgggggcaga gggggacgga 1680
   cttccagcat cgctcattcg ggggcgtgac gggaggacct ggctggggag cggcgggttg 1740
   ggagcctgtg gctgcccctt cggtgacagt ggagtcaccg agcctagaaa tggggaagct 1800
   gtgacgtggg ggtggtggag ggtgctgcgg gacagcagcg agcacggccc tcagccgtcc 1860
   ttcccaacac ggtttcactt cccctcagct ccagggctca gccccaagcc gcccccttca 1920
   tcccccatct gcaaacaatc ctggtccccc tgagcctcca atatgcctta tgcagttctt 1980
   ttggggttcc tttcgcattt t 2001
<210> 127
   <211> 2001
   <212> DNA
   <213> Homo sapiens
<400> 127
   ggccccaatc agcagtgcca aacttttctt ggagatgtgc tgccggtgag gctcttcata      60
   gggactctgc catcatgggt atgggggtat caaacacaca ctaatgaata aacctctgct     120
   cagattcata gggcaggaac ttcccccacc ccacaatgaa tcagaccagt tcctgctccc     180
   tcctcttact tccccctgta cttatctcag ggagtcagca aagggggtgg gggcgtctgt     240
   gtctgtctcc ctcttctatg tgtctgtcat ttacctgggt aactttggtg cctggcaccc     300
   tttaagtgct taataacagc gagtgcttaa gaaaatgcct gctgaatgga caacctaact     360
   cataaacccc attggtggga acctgcattc ctcagtaatg tcggatcagt gtttctcaac     420
   tcctccccac cctaccccat cacccgcccc taaggagctt tgaaaagatc ttttcttcct     480
   attctagaac caattccagc ttccacacct cccaccttga gaaggaatgc attagatgaa     540
   ccaagacttc tgcctatata catgggctgc tacagttggc caccccggca gtctgcggcc     600
   tcccaagcgg gggcctgggt cttaatcctt acttctctct aagctgcatg aagggtgccc     660
   ccattccatc cctcttcact tctccccagc atagcgcctc cataaatgtt tgtgaactgg 720
   atgaatgact gttgagcgct tggtatgatg agctgcttac aataggtgct caaccaatct 780
   ttgtccaatc tatgaatgaa tgaatgaact tcaccctttt ccttccatac ctccgggctg 840
   agcctctctc ccacatggaa accctaaggc tgagaccacc accttcctac cagttcctgg 900
   gggtgcccag tgaactgtct cagtatcccc agtgcctagc acacagtagg cgctcaataa 960
   acgcttgctg tgttcataat gaatgactca atcaggccca cggtgggggt gaaggggggc 1020
   tccatactgt gcctaaggat acagtgaatg agcaagtgac acacactccc ccaccccaat 1080
   ccctaactct ccttcctccc aggatcgggg gagaaacctc ggcccacagc cgggatgcgc 1140
   ccccgccggg gtcaccgcgg cggcgaacgt cccggacttc gcatcccggt gaccccttgg 1200
   gatgaaccga agccggaccc gcaccttggc cgtaacaagc gcgctccacg ccgccacgca 1260
   ccagcggtga ctcccgagtc ctctgtcgcc ctggcctccc cgcaggcggg tttcgaggcc 1320
   cccgcaggga gttcagggcc acggccacgt gcagccgccc ccgagcccga gtttgcaggc 1380
   tcggggtccg gttcgcgctt tggtggcgcc cggcgaggct gcagcgcgca aagttcccac 1440
   ctcaagttcc cgtcgcggcg tccccgcctc ggagttggcc cggccgcggc ttccgttccc 1500
   cccggctgcc cacgggtccg gggcgaggac gcgagagggg cctggaagtc gcccggggag 1560
   cccgctctgc ctcccggagc cgccgccaaa cttcggtccc ggggcccggc tcagctcccg 1620
   ccctcggagc cacggccgcc ccgtctctgc ctcccggtcc ccgccgggct ccgagtgcgc 1680
   cgggagcgcg gcgcagcagc cgccgaggga gggagcgagg caggaggcgg gacggaggga 1740
   ggggactgag cgcggggcgg ggagaagggg cgggcagccg aaagcgcagc gcctggcctc 1800
   gcctcgcttc gggacagtgg cagcggcgcc accgtcacgg ggcgtccagg cgcccaatcg 1860
   ggaccccgca ccttggatcc gggggccctg gcgcgcctgc tggattgcgc tcctcgcggc 1920
   cagttcctga gcccgcgtcc gggactgctc gcgctacgcc ctccctcccg ttgccttctt 1980
   ccccgtctgc tctcccagga g 2001
<210> 128
   <211> 2001
   <212> DNA
   <213> Homo sapiens
<400> 128
   tttttaaata acttcctcct cctgccttca actctcagaa gattatcaca aaaacatcca       60
   tattatgaat agttttatag gtcagttccc ttccgtacca ctaggcatac actgatgatc      120
   atttccaaat ttttccagac tgctttgata tcatgtgatt cattttaagt agaggcagtt      180
   ttgtgaattc cctagccaaa ctttttagat tgtggatttt catataaata aattgacaga      240
   ccggtggggg tggaggggga aatagaaaaa ggaaaaaaat gtttttgtaa actatcttac      300
   tcctaatctg ttattgagtg agccattttt ggctctaagg caccctagcc cgttaattca      360
   aggttacctt cggtcattgc catggctcca gtgcttatta tcattgcctc atccacatct 420
   tttgagaaca ctctgagaat ctacacggct ttaacacact tcttcgcact agtgagaacg 480
   cccgcaatgc cttattcagt aaggttaaat gagtttgttt gctgaaacat tcgccacatg 540
   atcccaacgc tcctccagct ccatcccgtc tataatcgca gagcgaacac tcactcacct 600
   tgggcgtgtc aataattgat ggtctgggga tggaagcgtg aaacaccact gcttcacagt 660
   ccgccccatc atccagggac ccgggcacat cttcaggcgt ctccgcgggc tgggccgcag 720
   tgcggtgact tgaattccga ttcgcactgc catttctctc tgcatgggtg ctcgaatctt 780
   gccttttctc tggagctcct tctgcgcttg ggtgtcttgg ctgtctgcct gctttggcca 840
   caggctgcac acggacggtg gaatgagtat cagccacgcc ggactgattg aaggaagcac 900
   tctccctcgt attcccaaaa caggcgagca gagctgagca cggaaatgga tgcatgcttc 960
   tagagggaga cttcagtctt ttggtgagtc ttgctttcca cgtggacatg acttgcagtg 1020
   aatcattgca aagttcaaac ggggcaagga aattttccag gggtccacat cacctccact 1080
   ttgcttatag gaactctttc caattgcccg aagagaagaa cttgctagtg gttgccccta 1140
   aatagaagtt gggatggcaa ggccaccttc cctgagagtc acttagccgc tggggcccct 1200
   ttgaaagtgt ccatcctggg caatttcgcg ctcttccttc tgtgatgctg tcagtggcta 1260
   aagaaagctg ttggggaggc atgtgagtgg ctccggttac agttcctagg cagggcagac 1320
   gcttcagggt agggtagtgc caagtcaata gacaattgct tcaggttacc taggaggcat 1380
   aaagataatg tagcctgata gcatctgcca taatagcgtc agcctgcgta tcaataacat     1440
   cataacagca gcaaaacctt ctttggttac cccccttcaa atatttattc ccccaaacaa     1500
   aacaaaaacg acccagttgt ccatcacaaa tgaagacccc tagaagggcc acgaccctag 1560
   cagctgtttc attattaaaa taggaggtag actcaatctt tcgcctggag agatgcggtg 1620
   tgatgtgttt tctgaatctt tctggacttt tcgggatctt tccggaatct tcccggaatc 1680
   ttccccccac cctcaaaaca agtttcctgg tttcaggatc cacattgcct tttctgataa 1740
   ctataaatag gtctttggta atttgatttt ttttttttta ctagctaggc taagttcttg 1800
   attagttttc aacaatacca aaaagaaaaa agagagccct acattaacaa ttatccttat 1860
   gtattatgtt attatgtgtg gaggtattat gtacatgtga agtattctac ctatatggta 1920
   tatatcacct actttcagaa agtaactcct ttttgaacat agatcttctg tttctcaaca 1980
   cattaatatg tgaatggtga a 2001
<210> 129
   <211> 2001
   <212> DNA
   <213> Homo sapiens
<400> 129
   aagtccatga caagcaacat gccgtcagcg taaatacagc gcgggtcctc tagcacaaca 60
   gaacccctga catccactcc attccactac tttggggagt gatgaagaaa gggagagggt 120
   tggggtcgtc agcggcggat ccacccctcc atcaaccccc gcccccgccc cgctccaggc 180
   tccctggccc gacaccgccc acaaactaac cacttgtttt gtccacgccc cctcccatgg 240
   gcacgcccca ggccttggcc ccgcccacct actccggctg gacttgtgcc cggccctgcc 300
   ccttgcgacc ggccctcgcg ccggcctcac gttcaggctc cgcccccggc cgcgcctccc 360
   tccttccctt tccgacagcg cgggaacggc tgcggcttcg ggcggccggg tttctccggt 420
   ctcccaacgc cgaggagagc ttgtaacagg cgctggagct ggcccgccac cgccgcgtca 480
   gggacggcgc tggagtcctc cgttcccctc agcctctgag ctgaggcccc acaccagagt 540
   agggggcggc gcggcacccg tgccccggca tggcgacacc ggacgcgggg ctccctgggg 600
   ctgagggcgt ggagccggcg ccctgggcgc agctggaggc ccccgcccgc ctcctgctgc 660
   aggcgctgca ggcggggcct gagggggcgc ggcgcggcct gggggtgctc cgggcgctgg 720
   gcagccgcgg ctgggagccc ttcgactggg gtcgcttgct cgaggccctg tgccgggagg 780
   agccggtcgt gcaggggcct gacggccgtc tggagctgta agtcctcgcc cgcggcccct 840
   tagcaggtat gggaggcggg gggctgtcgg gggaacgaca cacacagagg tgaaataagc 900
   cccttcagcg acggcctgcc gcgcacccta tgagcagggg cacccttcac tgagcacttg 960
   ctgtgtgcgg gacatcgagg atccgggaac tcgaggcaca cgccttctgt tcgctgtaag 1020
   gtatagtgat gataagtgga ggcaaatcac ttttgatcgc gtacttccat ctctaaaaaa 1080
   ctggactcgt acgtccagtg taagtatact tatgtataaa gtgtacacat gtgttaaaat 1140
   acgtacattt taaagtacaa aaatagatac ttaaaacgtg atggtggaat acgtatccgc 1200
   tatcctggtg gctgtatgct ctgcttttca aagcataacg ttcactttaa ggcaagattg 1260
   ctttctctaa gaatggtaac tgtgaatcca tattttagtg ttcttgataa tttctggctc 1320
   ttggatgaca ttgaactttt tttttaacct ctctgtggct gacgaaggat tcctactagg 1380
   agcagaggtg tcggccctgc cattttattt ggttgtgttt ctgttattga tattatctcc 1440
   aattttttgg caggcattct tttaagccac gtgcttattt gtgaggacaa cttttgctta 1500
   aaaaaaaaag agtacagtat ctacagatgc acttaactag caaagttaac taaaacacac 1560
   agaaaaatga gtgggtgagg tgccatgttc agctcctagt ggggccagcc ttccactcct 1620
   cttggatacc cctcccccat aatttatgct ggaaaccatc cagatgctaa atatttctaa 1680
   tgcttaattt cttatgtttt agataaatag gggaaaatgg gagttccaat attttcttcc 1740
   tacactccac tttgaatttc tttcaggaga tgggtcagga gaacttgtct gaatccccaa 1800
   attggaagct atagaaatgt ttgcccagct atctggctta ttctggatag agtaaggaga 1860
   gggcagggcc ttaacctggg gcccctggat agaagagtat ttcagcacaa ttgatttcct 1920
   ttataatcct agatatttta tcaaatgcat ttaacactca ttctgatcac aggtctctgg 1980
   cactaaaaca ggttaaaatt t 2001
<210> 130
   <211> 2001
   <212> DNA
   <213> Homo sapiens
<400> 130
   atggaggcag gttggtgcct ctggcttctg aagaccttac ctctcagtca caccaggtaa      60
   gatgaagaac atccagaagt gaattccgaa cacaagaatg acctggaaga tgacctttcc     120
   cagtacagtc ttcctgaggt agagggctcg gtccaccacc atggttccaa actgaatgag     180
   gaccatcacc aaaaacggcc ccgggacctg gtcctctgac agtgaagagg tgatgtctgc     240
   agctgctgag tgtttctggg gaagagacaa caaattcatg cccaagagag gcaattcatg     300
   gcaggaagcg ccatcgccat gggtcagggc gggtgctgtg gcctttgccc caacctaccc     360
   caaaggccca aaagccgaag acaatgatga tgaagtccac agtgtcagcc aggaacatga     420
   gtacatacac gtcagtcacg gcgctatact ccgggtggat gaggttgtaa aagaactgtt     480
   tgatgggcac atagatctcc agcgtcctgc aaaatggaga cccccacccc caacccactt     540
   gtttcaggaa gggcaggatg gcagaaatga ccagacactg ccatcatgcc actcctctgc     600
   attcctcttt ccagtcaggt cccaccttcc tctctctgcc tcaggatagt cccgctgtgg     660
   acagctcctg ttctaacgga gagcgtatac acatttactg ccaagatagg tactgcagcc     720
   ctctctgctg atggcaccac agccctctcc tcttgcttga agcataatag aatggtatga     780
   tgccctggga acgaggtgtt gggtttgtag cctgattcaa ttgctcacca ccggcgagaa     840
   ccatgagcat gctatgtaac ctctgtgttt agttttctca tctgtaaaac tggagtgatg     900
   agaagacctg atgcctaggg ccaaggtggg aagtaaatga gggaatgcat gagggcgctc     960
   agacttggag tgcttggtcc atgcgcgttt gaatgtgcaa cgcccctgct cctcctgttt    1020
   tagggcctgt gtccatgctg gctcccacat actcccctgg ccttttatct atgctttgct    1080
   ctgtgcctta gtgtggtgtc ctgttggctc ttgcttgtct gtctaacctt tgccctatct    1140
   ttgctctggg acctgccttc agttcaacgc cacgttcagg tgccactggc tctcctgctc    1200
   tcagagcctc tgtccagggc tccactccca gctggcagtc ccagtcccat gcaccacttc    1260
   catccccagc aggtggaggg acctgtgtag tgtgctgggt aacaggtgta tgaccatgga    1320
   caaagcattt ccccaccatg cacttccatc cccttctcag gataagagtg caggtctaag    1380
   gcccttccag cattgacact gtattcctct cagtctggct ggacttcacc atgataatgc    1440
   ctaagaatac aatccatgag cccttgaaat gacccattta ctctgttcaa atagaaaaag    1500
   catgtgcata aatgcaatgg atataaaatg aatcacactt acagaaaaat gatttttaaa    1560
   aacaagacaa aaaggggtaa tttcattctg ccttacgcag ataacatttg tgacacgaga    1620
   agttacttct ctgctctgct cctggtttat aggaaataat aaagcccaaa accctacaat    1680
   aaagcacaca tgccatatgt tctcatggac tcacttcttt atggtaaagg cttttgcttt     1740
   gattaaatgt tcttgaagct tttccatata aagttccctt ttgccttttt gcttaatact     1800
   caaaacactg cttccttttt gactgctgtt tcgggtgctt gtgctgccta gaaataaaaa     1860
   gagatcataa gatgggtggt ggagcctggg gtttgttcac ctaaatatcc aagaagcaga     1920
   acccagagcc cacatggtgg agggatagct caggactgtt tgggaggatg agtatgcacg     1980
   gccttgggat ttgtcctcaa c                                               2001
<210> 131
   <211> 2001
   <212> DNA
   <213> Homo sapiens
<400> 131
   gtaaactgac actcggtctc tgtgtgtggt atccaggcag ggtttgatgg aaaaagcctg      60
   cttccactca acctcaggtc actgtctggg gccagccccg gcccaccccc ctcccacata     120
   cccctcccct ccccacccgc cctctctgca tctacagccc caccagcaca ctcagccagg     180
   agcagtcccc cggcactcca ggggcacaca gggctcctgc cgccccaggg ggcctgccta     240
   ggctgtgcgg ctgtgaaggc tctttgcctg gtgtcttcct cctgattact gcacaacctc     300
   cctttctcct gcctcctgca actccccagg ctccccagca ggcacacccg tgcacctcgg     360
   tcctttcagc ctagacgcag cggggtgccc agctctacct cctgtttggt ccgctggtct     420
   gcagcacctt ctttctgggc ctcccccttc agcccttggg aggccccgcc agctccggcc      480
   tcctgctctc ctcctccctc tcccagctcc tatccctggt cggactacat ttccctgcac     540
   gtgtaatcca ataactaact gtcgggtctc attggaagtg attgctcccg agggcacatc     600
   tctctcagag cctgactctt acttagggag gaattagcca caactgggga gaagtgttct     660
   cggacaggag ttagctgaga cctggcacag ttttgggtgg gcaatgttaa gacggaggat     720
   gtaaacctag tgacagctgc acaccccttc aggactgact ataaatatag ccatctcgct      780
   tatctcaggg tctttctttt tctttctctt tctttctttt cttccttcct tcctttttct     840
   tttgcacttt tttcttacag aacactaact ggggtgcggg ggtgggggga agaagtggaa     900
   gcaaatgcaa ccctaagtcc catgagatcc aggtttccag agtctttgaa acttggccta     960
   ggtttctcag tgtaaatccc tctgctcaaa gttccgaaaa cgtccacgat cgccagcggc    1020
   ccgcaaccct gcggcggccc ccagtcgccc taacaattgt cggggagcct gcaggccctg    1080
   ccagaaagag agaggattgg cacgttacct ttgggcgtct ggcaccccgg cgtgtggctg    1140
   agggcggcaa agtctaaacg aagttgacag atggagtcag tatcggccct ggggagggcg    1200
   gccagggagg ctccgagcag ccgcacccgc agcagcacca ctcggcccgg cgggccagga    1260
   ggaggaaggg ggcgcggagg ggcggggctg ggtcctggga tgggacgcgg ggcttgctgc    1320
   ggggggcaaa cagaaagaat atttcatgaa gtcagaggtg gtcgagtttt gcgaggggtg    1380
   ggggtcgtcc ccctggcaga accggagtga gtctgtgatc cctcaattgg aaagagggtg 1440
   agtgagggaa aggggaggag gccgcgagag gaatgaagag cgaggagacc ctggagagcg 1500
   gggagcgaga taagagaggg aggcggccgc caggtcggga gggggaggag tgagcgcggc 1560
   ggcgcggagc gaggaggggg cagggcgggg tgggggggga gggagggagg gagggagcga 1620
   ggagggcggc cggggagggc gagggaggga ggaagtccga gagacagagc gaggagcgct 1680
   cctgaaggga gacgtcgggc tcgtccccgg ggtccaggcc gcggtcccac tcgcgggcgc 1740
   agtcgcccgg cgccggccgc tcggccccgc ggcctgggag gccgttgcgc aaggcagggc 1800
   ccgcggctcg gggagggatg cgccgggcgt tgcctcccgc ccgccgccgc cgccgccgcc 1860
   agaagcccca gaggagctga gggaggcgac gccgaagcgc tggcccggag tggccacggc 1920
   tgcagcccgg gcggcgggct agggcgctcg cagggcttct gggccgaccc cgctccggcg 1980
   cctccgcttc ccgcccgggg c 2001
<210> 132
   <211> 2001
   <212> DNA
   <213> Homo sapiens
<400> 132
   ttctccctgg cccagtctag aagcttcttg caggcaggta ccacagtttg gtcaaggtgc 60
   catgcgtccc aggtcctagc caggccctgg gctgccgctg gtgaacagga agggggtctg 120
   tgccctgcca tgcccccagg tagagctccc ctgggagcat cctccctggc ctgggacctc 180
   ccagacccca ccccccggtt gagtgatggc actgccaggg gttgaagacc ctcagccctc 240
   gacgttgtcc tctctccatt ggatgccgcc tctctctagc cacccctctc tccctctctg 300
   ccccttcgag ctttttctct caatatgcaa ttttctcttt tggtcttccg cactcttggc 360
   ccccagttct attgcagatc tgtttctcac tccatctaaa ctcttacccc tgtgtctcag 420
   gagctgctct tgctgaggga agaaggggac actacgggac aggggggcag tgtcgtacta 480
   aggacctggg ctctagccac tggaggaact ggactcattt gggccctcag gaagcggctg 540
   agtcttggtg gggtaacccg gttagccccc gtaagtgacc agcacagggc tgagcccaga 600
   ggaagtggcc acccacagag tggttctcat gtccgagggg acctgcaggg attgagcaag 660
   aagactgact cgctggatcc ttcgtctctg aatcagttca gggcaggcaa gctggggagc 720
   cccctgcccc gtcctgccac caccagccgg atcgggccct ctttttaagg gaagaaagtc 780
   tggagtggaa gggagggcac agggcgccag gagcctacat gaagtccttc cagaaatcca 840
   caacagctac ctctctgatc ctggagaaac cacctccttg cttaggccca agcaggttcc 900
   tggcaggctc aggaccaaat tccaggggcc actcatgggc ctagcagccc aaggccgcct 960
   ccccctcgtc tttcttccat ctctctttcc tctgcctggc gagatgccag ccagcacctc 1020
   agtgtcccca tctgggcagt ggaaagtttg actctctggg tccttgtttg agtgagtgcg 1080
   agtgtgtccg ttcctttgct gtctgcccca ggcgggggag ggggggggag gtggtggggg 1140
   cgagggggcg ggggctcagc tagtccagcc gtctacaaga aaattgctcc ctttgaagct 1200
   gccagggggg ccgggaagcc tgccccctcc tgctcgcccg ccctctccgc cccaccagcc 1260
   ccctccctcc tttcctccct ccccgccctc tccccgctgt cccctccccg tcggcccgcc 1320
   tgcccagcct ttagcctccc gcccgccgcc tctgtctccc tctctccaca aactgcccag 1380
   gagtgagtag ctgctttcgg tccgccggac acaccggaca gatagacgtg cggacggccc 1440
   accaccccag cccgccaact agtcagcctg cgcctggcgc ctcccctctc caggtagggc 1500
   tggcttcaag ctgcctcctc agcaacccag agatgcccct ggctctgctg cctccgctgt 1560
   cccaagccct ggtcctgctg tccccagtgc cgcgagggtg tccacagatt tccccggtgc      1620
   tctctgtagg ctgctgatcc acgccccttc atcgccaccc tgcggccccc ttggtccctg      1680
   tcaggcttct gcctcgtctc gccgccctcc aggcaccttt ccctcacccc ttcctctccc 1740
   tctcccttct gaccttgctc tgcttcatcc acctcttgtc tctctgcctc ccgctcgggg 1800
   tccgtcttct tggctaccac cctagagcgt ggctgggtga ctggtacccc agctttgcca 1860
   atggccctgt ttcatcattg caagtcccag gcgcatgctc cactccctca gcctcgctct 1920
   gcccaggcgc ctccttgctc cagggcttgg cgcctggccc gggttgggtc ggatcgggga 1980
   ggaccgccca gcgcccaccg a 2001
<210> 133
   <211> 2001
   <212> DNA
   <213> Homo sapiens
<400> 133
   ctgggactcc tccctctgtt gaccaagtgt ggaaccctgt aataacttct tagcaaccct 60
   cagaccaagg tcctcacctc cctgagaccc ggcaggcaga agtggggagg ggcaccacac 120
   gatcacacct gcatgggatt cccaaagctg acagaaggag cagacatttg gcgggggggg 180
   gggggggggt ggtgggcagg ggagggggac aagacgggga cttcctttat ttgggtgtcc 240
   tccaggtatt cagatttact cccgacaggg cccagccctt tccctcctgc tgaatcgtgt 300
   atgggtcttg cacgccaaag ccaccatctc cctgggatcc ccggtgagaa agtgggggcc 360
   acttctcttc ctcaccgtcc tgcctggggt tccagagctg ataagaggga cagatttcct 420
   agttctgaga tgcggggatc tgctgagtcc tccctcaggg ctctgggact cctccctctg 480
   ctgacctgag gctccactcc tcagaccata gccctctcct cattgacacc ccaaaggcac 540
   agcaaagaca catcacatgg gccaccgtgc ctgaaaccac ccagggctga ggtccccact 600
   gatctggact ccaaggtccc ttgagtcctt tctcttcttc ctccccttga ctcttggctg 660
   ggttgttctc ctctaccacg ccccgccgca gacctgagtc accgtccctt ggattcctca 720
   ggccgagtga agagacatct cggtctgaga cagaggtggg gtgggcaccc tgtcctggga 780
   tcccttcatg cctccctcat ttcccagcag ggcccgggcc ttccctctgc tcccctgaag 840
   ccgcattctt ggtaccaata tccccttccc tacgtcagcc tcagatgccg acgtcagaat 900
   ttgcgttcct ggtcgccatg accagggctt cccagggccg acatcagggg cagagccctc 960
   cctgagcgtc ccgacattga tgctggcaga acctaggcgc ggctcccgcc gaaccagccg 1020
   ggccctggtc acaccaagct ctgactccca gagtcccctg cggcttaagc ggcgggagag 1080
   agtgggggcg ggggtggggg gggggtgaca gcccagcctg agaagtctgc cctcaggtgg 1140
   tccagagctg gcagcagagg cagcgctgta ttatttaggg ctttctatct gggttggggg 1200
   cgtcctcagt cctccctcag cctctcacct tgcctcctca cagagcctgg gcccgctctc 1260
   ttcagccgat ctcaagcctg tccctcagaa cgagaacctc gcttctctct gatccccgag 1320
   gcgtaagtta gtggcgtcac atccggtccc cggggcttcc ctgggttgac agcaggggcg 1380
   gagtggattc tgccggaact ggggtgggag ttggggtggg agtgagggtg ggagtccggg 1440
   tggggtgggg tacgggtgag gatggggttg gggcaggggt ggtggtgaag atgagtgtgg 1500
   ggaggggcgt ggaggtggga actgggatgg ggatcttggg gctgtgggtc gtggtggggg 1560
   tgggggtggg gtctttcagt catccctcag tgtctcgacc ttgatgcctg gaaaagcctg 1620
   gacccctgtt ttgtgctgag gatgtcttct ccccttaaac caggtctctt gactcggagt 1680
   cttccaaggt ggcagtgagg ggagggttgt ggtaggggtg acaacattgc cagggtcttc 1740
   cagggctgac aagaggggct cagctggatt ctgtggcccc tttgtgaggg gtgggtggac 1800
   cctcggtcct cactcaggag ggtcttcctc ctggcttttg tctctttgat gaagtgatgg 1860
   gtgggagatg ctcagtttcc gtcacctctg atcatttgcc cagctgcatc ccttgcagag 1920
   catgggtgcg gttcccaggc cagatggtcc ctggggagct gcaacacccg tgattgtaat 1980
   gacctatggg agaagacgca c 2001
<210> 134
   <211> 2001
   <212> DNA
   <213> Homo sapiens
<400> 134
   tttaacttct gtatggtgag atcatggata ttaataatac ttttccatat tttccaatat        60
   ttctataatg accatttatt tctcttataa tcagtaacaa aagaagggaa aacttggtct       120
   aaacacgaat ttagggactt aaactagact tggagaaaag ctttctatgc aagatttatt       180
   agatactgga aaggatgagg aagacaaatt taagtcaata tgatggaaag agtggtggat       240
   ttggaatgag gaaacttaga aatgaatctg ttttctgggt gaccttgcgc aagcctttgc       300
   taaaatttgg ttttctattt tgaaaaacgc agctggctgt ttaaggaaga taatatccac       360
   cagggcgcct ggcccagaaa aggcgctctg taagtggtag gtggatcctg tctaatcaac       420
   gccaattata accaggcatc tgcaagcttt gtggagggcg catctttcac tgctaccaga       480
   gcctacagtt ctaataatga aaattgcctg gaaaagaacc acaactctta aaaaaaaaaa     540
   aaaagtccaa aatcacaaag tggcttttgc agcggtcctt gcctgtgacg ccgggccatg     600
   gctggagctc cttgctccgt acctctcagg agcctccatc ctgtaccaat ggccagagag     660
   ccggggggtg acagacaggg gggccggcag ccgagccagg caggacgctg cgcgccgcgt     720
   tactggagcc ggtgtcccgg caaccccgga acgcgcctct gtgggagaag caggcggctg     780
   cggtgtccct tttgccctag gctctcggtt ctcaggcctc ggcgtgcggc gtgcgttcgg     840
   cagttcagtt gccagttggt tcgttggtcc gtggaggcct ctgagcacag acccgggcct     900
   cccctgggga agggggtggg aagaccccga ggtggaaaag aaggagcacc ttgagctgga     960
   tgggggctgc agcgagcctg gttcgggctg ggggcggact cgctggggct cccagggctt    1020
   ccaaaccacg aaacgcccag gtggagggag taaggctccc gtttagaggc cagtcctgcc    1080
   ctcctgactt tcggtggctt gtggaatttg tttgttttga ctgatacctt tcttacgagg    1140
   gactttcttt gtctctgggt tgcttctaat atttaaaatc atcatgggca cagatgagtc    1200
   catccatgct cactggaaac agtccacggg gctgccgtct cccctccatc tcaagcacga    1260
   cctgcggcag acagctggag aaggttccag aaaaaaggga ctctggcatg accgaggtgg    1320
   agaggaccta cagttgagtt ctaagaaatc caggctgtgg agttggtggc cacatccaag    1380
   tatactacac ccgggggcgg gggcaggtgg ggggcaccat ttgaagttta gaaggaagct    1440
   agtttggggg catgtaaaaa gagctgcttg aatagtagct tatgtttgtg ctaacccaag    1500
   atttgattag gcagagagca ccaccaaagc agagagtctc ataaattacc tcactggatc    1560
   tcacagcagt ggagaagtat attacctatg agtaaaggga gccagagatt tgacctgccc    1620
   agtgcttggg ttaataaaag gtgagcctgg gatttgacat gagcctggga tttgacatga    1680
   gtctgccata ctttcgagcc tgtgtccttt catctttcct ttgctcagat attagtaggt    1740
   ttgtgtcatt gtttattgga tgggacagtt caggtgagaa ttatgaacac tctaaaaatt    1800
   aaataatggc tggtagctcc ataattggtt aaggccgagg agggtatttg ggggcacgtt    1860
   tgcctttgga gaacagtgtc atggcgctga acagccacag tctcccagaa aagctcagag    1920
   tgcgcctgcg ctcaagttct catttacctg ttatgggtga gtggcctgag tgctgcaaaa    1980
   aggggttgtg aaatatccag a                                              2001
<210> 135
   <211> 2001
   <212> DNA
   <213> Homo sapiens
<400> 135
   tataggacga aaaaaaaaaa aagtctagca atcaagggag tgcgggagta cggatgcgtg        60
   tgtgtgtgtg tgagtgcgcg tttaaagaac ataaaacgcc acaaataagc acttaatatt       120
   ttactgagtc gtcatacagt aactcatttc taatgagact gttaaagctg ttaccaaatt       180
   ccaacaggtc atgaaaaaac tgctcagcac ctctctgtct tctgtcaggt tagggctccg      240
   cagacggacg acagaggatt atccgtagac ttagatgggg gttcctccgc ccctctctgt      300
   cctcctgcca ccacagccag gctggacgca gaaccgcacg gaagcctccc caactcctgc      360
   ctctgggagt gctcctgcct gcttcaagga atctgggcag cgggaccctc tgggtttagc      420
   gtcctcgcct ggggaagcaa ctttggcctt accgtagaag ttacaaaatt ggcgggggta      480
   gggagaaaga ttataatata tatttggagt cctttcttcc agtttaataa acacgtactt      540
   ttctcattgc cctagtcctg gtatgcctaa tggcggggaa agcctcaaat aaactcggtg      600
   ccctctggat ctccccaacc tgcctttagt ccttgcgaga ggcgtgagag aacttcctat      660
   tatcatccct tttgttattt ttattacatt atttaatagg ctccctctac ggaaccaaaa      720
   atagccttaa tatgcttact ttctccctgt actccgcccc aaactgttag aacagcagaa      780
   aatttttttt tggggggggg cggtttgggg gaaggcagaa ggagaagaaa atttgattgt      840
   gatggtggtg gattttttcc aaatttcccc acagctgtta tcaaaacatg caaatgagat      900
   tttccaacag gatcttaaac aaatctggag gagttaatgc cgaagcaaaa taatcagcac      960
   gtttgaagtg actgtgagtt tcagtttgtc ttttctcccc gtgatattat tgcaggggaa     1020
   tgataattag acctttagaa atccaaaggg gctctcttgc tctcttccct ctctcatttc     1080
   tcttctctct ttctctctct ctctctctcc cttcctccct ccctccctcc ctctctccct     1140
   ccctctctct tgttccctcc ctctcccttt ctctctaact ctgatgttgg caaagggggt     1200
   tttcttaatc agactgtttt ttggtcccag ggaaaggagg aagaaggaga ttgtgatgga     1260
   gaaagggggt ctgtgaaacg tcaggcacag cacaaaggct ttgccacgta attacaggct     1320
   cctattaagt cgagatctgc cctcccaggg gtctccaatt ttcttgtatt ccctacaaag     1380
   cctcctctgc atgccagttt gtgccttttg aagtgccaga gagcttcttg atccaactga     1440
   gaaggaaaaa ggagcccagc aagaagaggg ggagagagag aaggggaaag gggggaaccc     1500
   accagcaccc tccgtcggac tcttgaagcc tttttttttt aattcttaat tttttttttt     1560
   actctttaca aaaagtaaag tgagaatcct gctctctaat acatctgcaa gacatcaccc     1620
   tctcctcctg aaactttagt cactcctgag aatccacagg agtgcagaga ggggggaaca     1680
   cgttttcttg aagatgtttt aaagctggaa caagccttct tctgttggtg cttgaactct     1740
   tgcctgggaa taactttttt aacctttaaa aaaaccattc actttgattc ttctctccca     1800
   ccccttcttc tctcttcttc tgtttgccta actcccccgc cctgctggcc tccgctttcc     1860
   tctctccccc ttgttattat ttttagtctg tgcgtgtgga cacttttgga gagttggaag     1920
   ggattttttt ctcctgactt gaacataggg tgacttttta atattgtatt ttactgtgga     1980
   ttatctcttt ggaccgcgcc g                                                2001
<210> 136
   <211> 2001
   <212> DNA
   <213> Homo sapiens
<400> 136
   atatctagag tgatatttta cctgtttctt tttcttcatc tggattcttc atgggacacc      60
   ttgatatcac tctagatatc aagagtgagg agaattctca ataaactggc ttaataggat     120
   tctttgctaa agcagggttc aggaggtcaa tgtcaaggct tagttcagaa gaggagcctg     180
   actaaagttg ggtcaaagag gaagtctttg tctttgttaa tctttcaaat ttgaattcac     240
   ccactaataa ggaccaattt agaatgctat atgtacttta caagttcttt tctagacctg     300
   gaaaataagc tccatcatta ctcataatga cattataggt aactgacgcc agctgtcttc     360
   ataagaacaa aattatctag cagaattgca cacacactga caatttggtc caaatgaatt     420
   ctaggaaatc tgattaaact cagagggtaa tgaaaatatt tgtagtattt atattgtgct     480
   tagtatttgc caggtactgt cctgaatcat ttgcatggtt taatctctta gtcctcccaa     540
   cagtctattg ggaggtatta ctaacagtct gtagggaagt actattacta gccccatccc     600
   aggattacag acagagaaac tgagacacat agcagtttca aagcttgtct gaagttactc     660
   aattataata tgtcaggttt gtgattggaa cctagcacta taacttttga gatgatgtgt     720
   ttactacagt gcattcctac ttctgtaaat ctcttctcct tcctttctct gagcaaacac     780
   ataaaactgg gaggtaggta ggagaattgg gatgacattc atttgtggaa gtccatatac     840
   tgtgtcatat tcttcatctt cagcactttt aatctctaaa acatagtgca gtatttagca     900
   ggtcatcaat aatttttgtt gttgggtaaa tggctgatcg aatgaattaa caaaaggact     960
   ggatactcca tttctgatca ccatagagat aaccaaatta cgtttctcaa actctgtcat    1020
   gctgagggct gcagtgacaa cattttagga ctagaacaca ttacttggat ttcacaatac    1080
   aaagatgatt gcagttgtcc ctggtaagct cagcccacag agcctttcaa ttcagcttgg    1140
   ggtgagctgc acttcaggca tggggatata atagtgtgat gaaccattct tctgaaggtt    1200
   cttctgaaaa gctgctggtc ttgggatact gtgagagccc ctctcattgc tctttagaag    1260
   taatttaaat ttttctgcta cattgtctgc tcagctcgta ttctggtcat agaggaacct    1320
   gaagccagag aaactagaca aaaaggtaag agaaaaccaa gcaacaaatg cagtctcagc    1380
   tgttgctaac gttattcaca ggagtgacaa actgaatgaa tcttagggtg agagactggt    1440
   ctttttacct catttacact tttccttggt ctgcactgta gatcagcaga actcatttat    1500
   cctaagaaat cgatgactaa ttttctgtga agcagaatca agaaatctgg tctacattgg    1560
   ttaaaaataa gtacttggtt caccttgtgc tgtgaagtcc aagccaaata attgttgcaa    1620
   gttgttgaag catttgagtg aagaaaaact gaccaagttc actatgactg catgaaatga    1680
   aatgctgaac tttttggggt tgagggggtt ggaaggatgg atggaggaag acagttgttt    1740
   ccagtctctg aaattcaatt ctgcagtaag tttctgtatc accaagtgtc actttggcat    1800
   acctcgtgca tatttatagt ctaaggcttt tcatatagga ttcaaacata gattaaagta    1860
   gcagggaact tgagagctcc agggaagaat atgatacttt aattgcattt gatcatttta 1920
   ttgaatttgc ctaggaaaat caattaaaac acaaaactta atttttgtac ttacagcaga 1980
   gattatacat tccatcttgt c 2001
<210> 137
   <211> 2001
   <212> DNA
   <213> Homo sapiens
<400> 137
   gaacgggaag acgcctttca ctgacccggg cgcgggacct cgggtcccgg gccggggcca 60
   ggggccatac cctggcgcgg ggggcgggcc gcctagcggg ggaggggggc acgcccccga 120
   ggtgggggca gcggctcgcc cctcaggtta actcttcgct ggccgaggcc aggcgcgggc 180
   atgctccctc gcacccggcc aggagaaaaa gggcagcggg ggatgggcgg gcgcgccggg 240
   cgttgccagg ctacggcccc ccagggagcg cgggggcgcg ccgggccgcc aggggccgtc 300
   aggggccgtg ggccgcgcgc ctccccgccc gcggggcccg ccgctgccgc tgccgcctcc 360
   ggctcctgcc catggccgcc agccccggga gcccggccct cgcgctcggc gccgcggccc 420
   ggcccgcgac aggggggcag cggcgcggcg gcggcccccg cccgcggcca ggccagcgcc 480
   cgcgctcccg cctcgcccgg ccgcgctctc ggagctcccg gcgcccgctc ttcgtccagc 540
   agccgctggc cgtctccagc tcccgcccgg gacgacgggc acgcgagcgc gcccacggcg 600
   gcgggggagg ggcgcgggca cggggcgcac gcgcgccggg aggggcgggg aggggagcgg 660
   ggccggctgc gggagggagc ggcgggggcg ctggagccgg cgcgggaagc cgggggcggg 720
   cgctcgacgg aagcgggcgg gggtcctagg caccccacga cacccggcgt ccggtcctga 780
   ggggcgggca ccaggcgcgt tgtctgcgcg atgccgctct gggagagagg ggtcctgcca 840
   ggaggagcag tctcctgggt cccccagaga gagtccgcga tttcagcccc tctccccggt 900
   cttggacgca gcagccctca cgcctctgct ccttcctcgg gcttcgcctt aaatgactgc 960
   atccctcctt caactcagcc attccctgct gtctttgaaa cggacacctt tgtttaaaac 1020
   aagaaagaaa gagagaaagg gaggaaagaa aagaaagaga gaaagaaaaa aggatgagcg 1080
   aagcgtgggg agggggtggg tcgtggggag agtggcctcg gaggccttgg gacccgggcc 1140
   tgcacacagc actggccagc ctgggcccgc cgcctacctg ttcctcagcc ccagccctca 1200
   cccggtcccc gctttcacag cccttccatc tcttcctctt ctctcccacc acctccccgt 1260
   cttcttggac aagcctggag ggagaaggcc tttgcctgga aagcagcagc gggtggaagt 1320
   ctgcgttccc agaggctgcc ccggctgcag gagatcactt ggagaaattg ctacatgctt 1380
   catttccagg tttgggcaag tgagggaacc cccagccaca cggggctgtt gaatcggagc 1440
   ccttcctttc ctccctggct cctcgttaga agagaaatca agaatggcgg gcctgcccag 1500
   aatgtggaga agagaggcga gtttcttatc cagggcttct cccagaaagc agaacttgag 1560
   acaagggctg gtgtgcaggt tgcaacctgc cacccaatgg ctggctagtc ttgaggaatg 1620
   gcgtcatatt gagggctcag catcggtctc tgcagctggt ggatggaaga acattcacta 1680
   gcggcagtgt ctagatgagc cgtggtaaga ggaagccctt gccgttgggc ttatacagag 1740
   cctccagccc tcccaccatg gccactggca tcgtgggtcc atcgcatcct ggggagaagc 1800
   tggctgacct ccaccagatg gtcccctctg ttcgctagct tctggcgcgc cccctccaca 1860
   ctgcatgctc cccagcggca tgggcgcgaa gacgagcacg gggccccaca ctctccctga 1920
   gcactcccgt aggtacctct tcgccaaacc tgtctcttct tccaatcttc ctccacccgg 1980
   gtccctgacc agtcagccca g 2001
<210> 138
   <211> 2001
   <212> DNA
   <213> Homo sapiens
<400> 138
   tcctattcct tggaaagttc cagtatctga gggaggaaca gtgacttctg gtcctgacct 60
   gagtggagac cgagggactc agaagagctg gaatcagacc cccacacaca ctgagtatga 120
   gtcagagaac aaggcctgag agaaaaagtc acagtgccca agactgctgc aggggtcaaa 180
   ggggactgct gatcagtgtt ccagggattt gctccagatt aatctgagtc atggaaaaac 240
   tgcctttctt ttgtttctaa acaaatacct gcaaagacaa aagaccacat aaatccccag 300
   gtgcacaact ttttcagatt taaagaaaaa acaacccccg tctttccctc cattcccagg 360
   agaagctcac tctgtggcat caagctgcct gggtgagctc tcttctagaa gagtccaggg 420
   ggacaggcaa ggaatgggag gcaggaagtc cagttcaggg agggggattc tgggatgaaa 480
   agtgaaggga gagggactga gcccatgctg agggtttttc tctggtttct cagacagctc 540
   cggggccaag attcggggag acattgagac agagcgtttg atataaaaga agggggtaag 600
   agccaagtcc cagggcccgg agcttggctc tctgggcttc aggccccgag ggcggtgcct 660
   gggatgggcc ggctcagctt tggggtttcc cgagctccgc tctctctctt ccacctttcc 720
   caacctgtgt caggtccttt tacctggata ctcatgatgc ggcctcagtt ctcacttcca 780
   ttgggtgttg ggttcctaga tcagccaatc agtgtcgccg cggttcctgg ttctaaagtc 840
   ctcgccggcc caccgggact cagattctcc ccagacgcca aggttgcggg tcatggagtc 900
   ccgaaccctc ctcctgctgt tctcgggagc cgtggccctg atccagacct gggcaggtga 960
   gtgcggggtc gggagggaaa aagcctctgc gggaaggagc gaggggcccg cccggaggag 1020
   taaatctgca catatattta attacagatt acaattacaa tcaaggcaga aatgatctca 1080
   tttttacatt acaactctgg aaaaggcaat agactgagat gcaagtgtgc ccccaagtga 1140
   tgggcagaag gagagaaggt gttttggatg cattctagaa cacaggtaat ctaaggagag 1200
   ttgatcaagg ccgtggaaga gtcctccagc cactattggc catcaaagga gtcctctgtg 1260
   tcccaggaat ggtcctgctt tggtgtccct ggtgtgaccc atcacccgct gggaacagcc 1320
   tgagagaagt agggcctctg caccaatgct gctgaggatg tcagagcaca ggaacgaggc 1380
   cttgggaaat tacctggaaa tgcgactgaa atcttccttc ctgaggggtc tgggctcttg 1440
   gaaatcaaac cctctcaggt tgggtggctg gacgattctc ctcacactta caatgggaca 1500
   aggggaacca ggaggccccc aaggggatcc ctgggttcca cacgaactcc tcctaccctc 1560
   attgtgtgac agcagccatg cctcctcctg gggatcagga tctattacct gtgcctggag 1620
   aggaggggac tcctcttctc acccgctggt ctctggacac atactgtcca attcccctgt 1680
   ggcagctgta atgtgtagtt caatgggcac tcatttgtcc ccttttaagg gtaccctcct 1740
   ttagaatcca ggaccttcta ccctgcagag tgtggttttg ggagagaagt gcaaaatccc 1800
   acgacaggtg agttgaagga atgggatatg gagccacatc cacttccacc ccttggtatc 1860
   tggacccacg tgttcttcct actgagatta cagaactgta gagatgtctt tgatttttaa 1920
   aatgcaccat gtcctgaaag atggcaccct cccacccgca gagtgcttcc tgcaagctgg 1980
   cgttgagctg tgcctataga a 2001
<210> 139
   <211> 2001
   <212> DNA
   <213> Homo sapiens
<400> 139
   tctctgcctc agctttctcc actgttaatt gggcataatg gacacaacaa tggtgagggg 60
   ctcttgcagg aatgaagact aggatgagga tgaggatgag ggatacgtcc ataaagcccc 120
   tgcacacctt ccatttcctg agcactccca ggaagtcttt gctgaccacg ccaaattaga 180
   ttatctttgc ttagccatta ggccgctgcc ctatattggg aggcaaatat acctctcagc 240
   ttcggtggcc aggtcccctg gcacccactt agtcctcagt ctgcacctaa aagcaagcct 300
   tccgagggtc ttgccttgca ggtgtagctc ccacccccca cgctggggtg gtccggggac 360
   cctctgtttc ctcagtggtg gggaagggac ccgtgggttc gtgggtgctg accgacgaat 420
   gtgcggagga gggcgcgccc cagcatgggg ggcgggggtg cttcttagga tggcttggag 480
   gggggcgcct gcagggctgg acggcggacg ggggacactg cactttctga tgcggtcggg 540
   gaagccggga gtcagcctac tggagtccgc tgccatccct ggccagaagc agccagcgcc 600
   accccaagct ccccagcccc tcgcccgtgc cggggccgcc ggggtccgag ttaggggaga 660
   gtggggggcg gccaccgctg caccgcgcgg ccttcgaagg actgccctgc ggcgccgcgt 720
   tcccccgagg ccaccccctc ccgccgtcaa cactccccgg ggcgggggct cacctgtgtg 780
   gtctgggtcg cctccgaggc cgagtccctc gttgccaagc ccagcaggcc tcgctccccc 840
   gtgactgcac cgccggcccg ctccagcttc ccgagtaccc cacctgtctc cccggcttcc 900
   ttccggccct ttgtgttttc ttctcttgct ccgccccctc gcctgcccgc taagcactta 960
   agctggtgaa gtcaccctac tgccacctgg cagtcacttt cgctgttggt ggcaacccag 1020
   gctggaaccc agctcttgcc ggaacccctg ccccatcctt gaggcttgtc ccgacaccga 1080
   gtaggttcta gttcctttgt gccttccaaa tgactccctt cctccagcaa tcacagccgc 1140
   aacagccatg tattgcgccc ccgccctagg ccaggcgtgg gcatcacctt tgcactcctt 1200
   tctgcagccg tgtgtggtgc ccactgtgac ctgcacttca cagacgggtc atctgagttg 1260
   tgcaggccac taagggagcc tggagtcaac cccaggcctg cttggctccc aggccttctc 1320
   tttccattgc tctttgtcgg tcccctgctg cctcctgtcc ctccccaact ctgacttgtg 1380
   gagggaagcc tttgacatgt gggggtacct ttaataccga tatcacttgt taccggagag 1440
   gggtcccgat ccagacccca agcgagggtt cttggacttc tcgcaagaaa gaattcgggg 1500
   tgagtccgta gagtgagagc aagtttatta aggaagtata gaaaacaggc cgggtgcggt 1560
   ggctcacgcc cataatccca acactttggg aggcaaaggc aggcagatca cttgagctca 1620
   ggagttcaga ccagcctggc caacatggtg aaacactctc tctactaaaa atacgaaaag 1680
   ttagccaggc atggtgctct atgcctgtaa tcccagctac tcgggaggct gaggcaggag 1740
   aatcgcttga actccggagg cagaggttgc agtgagccga gattgcgcca ctgcactcca 1800
   gcttgggcga cagagccaga ctctgtctca aaaaaaaaaa aaacaaaaaa aaaacaaaaa 1860
   acaagaagaa caacaacaaa aaagaatggc tactccactg gcagagcagc tgtgtgggct 1920
   gcttagctaa ttatactggt agttatttct tgattctatg ctaaacaagg ggtggattat 1980
   tcatgaattt tccaggaaaa g 2001
<210> 140
   <211> 2001
   <212> DNA
   <213> Homo sapiens
<400> 140
   atcgggttcc cttagagcta agaataaggg acagccagac tctgtggcat cccttctttc 60
   tgggccgggg gtatccccag tcctcatgaa gggggcacac cttgggtccg gcagatcctg 120
   ggattcctcc ctctgctgac ctgaagtgcc actcctcaga ccacagcccg caaatctgtc 180
   accctgaagt cgcaatgagc atggggcaca ttaggccact gtgcccagga tcccccagcc 240
   caaggttgcc actgatctgg actccaaggt cccctcagtc ctccctcttc ttcctcccct 300
   tgactcctcg cagggctggg ctccaactcc ctgctgaagt gggtctgtat ccctaggatt 360
   cccgcggccg aatgaaaagg tggctcagtt tgagacaggg gtactttggg cccctgtcct 420
   ggcatgctgg gagccctccg gactccatta tcttccagga ggggcttggg cccatcctct 480
   gcttcccaaa ggccgtactc acgataccga cccccttccc tccctgagcc acagatgtga 540
   acgtcaggat ccgcgtgctt ggccgccatt cccggagctt ccgtgggttg attgcagggg 600
   caaagctccg attctgccag gatgggtggg gatgggaatc ggggtttggg ggtcccgttg 660
   atgtttgagc gatggtggtg ggtttggtgt tgggggatgg gggtggggtt ggtgttgggg 720
   gatgggaatg gtggtggggg tgcggggggg ttaccctcag tcccctcttg gggtcctgac 780
   cttgattcct ggctgagtct gggccctgag cacttctaac cagccctgcc ctggtcacac 840
   aaagctctta caccagagtt ccctgcggtt aaagcggcgg ggggtggcgg ggtgaggtgg 900
   tggcgactga gcctgagaag tcttcccctg cagggtggtc caggcctggc agcagaggca 960
   gcgctggatt attgggggcc ctctgtctgg ggtgaggccc gcctcagtcc tccctcggca 1020
   tcttaccttg cctcctcacg gagcctgcgc ctgcttccct ccaccgacgt caggccgttg 1080
   ctcagggcga gaatctcgct gttctctgac ctccaacgcg caagtcagtg gcgtcacatc 1140
   cggccccgtg acttccctgg gttcacagca ggggtggaac tggattcttc ctggatgggg 1200
   atccagatgg aggtggaggt aagcatgaag atggtgttgg gggtaggggt agggataggg 1260
   acggggatcc tgaggctgta gatcctggtg gggttggggg agttgggggt gggaggggcc 1320
   tttggtcatc cctcaaggtc ctgacttgga tgccaggctg agcctggacc gccatcctct 1380
   gccgattgtg tctgctcccc tcagaccaag tctctgactc tgagtccccc tgaagtggca 1440
   ctgggggagg ggtgcggtcg gggtgacatt attgcctaag tttatcaggg ctgacaagag 1500
   ggactgagct ggattctgtg gtcccactac gtgggggagg gtggaccctc agtcctcact 1560
   caagagggtc ctcttcctca ctcctggctg ttggatgaag tgattggcag gagatactct 1620
   gtttctgtaa cgtctgtgca tttgcacagc tgcacccctt gtagagaatg gctgcgggtc 1680
   ccaggccagg agctccctgc agggcgggag ctcccacgat cgtattgacc tctggaagaa 1740
   gacagacact ttcccacggg agctcctctc cagccagagc tacacttggc aaacctttgg 1800
   tcctaaatga tttattcact gaattgaaga aatacggttt acatatcttc caagtatata 1860
   tgtaggtagc gatatatata tttatttcta cgaactaaca cctaccctta gtatgcatcg 1920
   ttcactctta ctatcctaat ctacttctat tccagtcttt gtgttctgtt gaaggaattt 1980
   ttttttaaac gtaattcctc t 2001
<210> 141
   <211> 2001
   <212> DNA
   <213> Homo sapiens
<400> 141
   tgacactgac tttttaaaaa ataaacttac ccttttaatc aaatgtgtga attgtttaaa        60
   ggaaaaacat tgtgtaaata atgttttcag ggggtagaat ccagacgtga gttttggaaa        120
   cagagtccag tcctgcttct gacatttgaa ttgcttctcc agcatcatca ccagaagatg       180
   ggctacctgt gctcacaagc ctcctgtaac acggagctca ccacctctca aaggcacctt       240
   ctgtcaggtc tgcctggaaa tgctgctccc gcagactccg cacctgaggc caggtctgca       300
   gcgaagctca tccacacctg aggccaggtc tgcagcgaag ctcagccccc ttccttcaca       360
   ggagaaatac ttctgggtgt gcaccttcca ctcctggcag ggccgtgagc ccctgtggca      420
   gtgcctcacc ctctgggagg gaccctctcc atctgggaac aatgtgggta cttttttctc      480
   ttaaagggca gtgtcaggaa tgaaagcaga tacccaaaag cagtgggtct cgtggcctcg     540
   tgtctgcttc cagaagtgga gggcggcctg ggacggcggg agccctgagg acaccaccgg     600
   gggggtcccg ggacctgata cgagtgggca ggagccccaa gccctggagg aaggaaaacc     660
   atttcataaa taactgcatg gggttgtggg ccttttctca caagccgcag cacctccttc     720
   gtctctccac ggtgaacccg cccgccacgc tatgccacca ggccaggtcc tgcagcaccg     780
   cctccgtggc taggacttcc tcaccagcct ggggccgtga ggtggtcatt tcccccctta      840
   cctgacttat ctcaggattc acacacacac acttgacttt cggaaacttc ttttaaatta     900
   atttcactga cactaaaagg gaagctttgt ggagtgtgat tgtggttacc tgttaatact     960
   tggcaacaat tatgagccct gatatgagcc aacttcttcc gtcttatccc ctttgaggac     1020
   gctctgttat cgcccccatt ttgcaaacga ggaaactgag gctcggaggc ctggagcaat     1080
   gtgttccagg acagacggca ggaagccggg gtaggaattc caacagggag ttccctctgt     1140
   ggaagggaac gtggctggca cctccctgcc ttccaccctc agagctagtg tgaaaaagaa     1200
   gacggggtta tggaaaaggc aacgggctgg agattccccg gattcccagg ctagggcgcc     1260
   ggtctcactg tgcgcgcagg tggggcgcgt gccagaacag ggcctggggg tcaggccgag     1320
   accggcgggc accttcacca gggccgcccg tggcgttccc ccagcccctc tgccgcgggc     1380
   cccacgtggc cccgaccctg cggcagcctt tcggaagggc ggagctgaaa aacggccgct     1440
   tggctccact cggccccaca gcgcccagga gcgaatcccg agtccgttcg gaaccgcctc     1500
   gccgcgtggc ctggggcggc gtcgccaccg cgcgttctcg gcctcccggc tgcggcagcg     1560
   atgccggtcc ttggagctgg agcggggagt ccaggaggcg gcagcgctcc ggagagagac     1620
   gccctggtcc cccgccgcgc gttcccgcct ctgtgccctg ggggggagga gggggcggtc     1680
   gccgctgcca ttggccgtgg ggggtgaggc gggccccgcg caggaaaccc cgcccctccc     1740
   tctgcggcgg gggcggcccc agcggatgaa tgaagcggcg cgtggctgcc ggggaggcca     1800
   gagcgtggag cgctgcgcgg cgcggcggcc gggccctcga gacggggacg gtgagtgcgc     1860
   ggggagcggg gagctggcgc gggtgcacag ggcccgacct gctggcgtag gagcgcggca     1920
   ggtgccgcca tcgtgggcag gggctggagc gccgggggga tgcgcgcggg ggcgggaggc      1980
   ctgggcaccg gccgagcgcc c                                               2001
<210> 142
   <211> 2001
   <212> DNA
   <213> Homo sapiens
<400> 142
   agaatcactg aatgatccag ctcacggtca tcacaaacga gcagaggtaa cagggcacaa 60
   actcttcttt gaactaaaaa atccaaccaa agttagttct gttgtcattc ctgagaacca 120
   cggaaaggat cctggctggg ctgtatgggc ctccgcgcat gtgggagagg tcaagctcct 180
   ccacccttca acgccaatgt cagctatgtg tccttatgcc accaagaagc ccccttcctc 240
   ttccggggcc tgaggttttc ccaactgatg gcgaagctat tcctatatgt gtcatctctg 300
   tcctactaga acataactgg cctgtatttt agacaacttg gtaatcccag tgcccagcac 360
   agggtttggt acagaataag tgctaatgtt tgatgaatga atgaatgaat gaatggagta 420
   gattaacacc aggaaccatg aggctcctga gcagcacctg atgaaagatc ccccacactg 480
   aaaacagaat ccagaatcgt tgtctcactt gggcagtatt attcccatca attttttcaa 540
   caaatatggc ctggctgtct actacaagcc gggccctgtg aaaagtcctg tggggataca 600
   gacacggtct cttgttttca aggaccaccc agttcggcaa gtggaggtaa gtcatggaca 660
   aacatggtgg caatgctcag tggcgagcta tccagcacgt cagaagtcca gcccacgtgc 720
   tgaggaagtc ccaaagagag caagatcact tctgtctggg tgctgctctt ggaaggcctc 780
   atggtcttcc aagaccccca tctgacctct gacccttggc agccaacctt ccccaggcca 840
   ccccagagct tgctggccct tcccacttat gcacgccgcc tgggcaggag aggtcactca 900
   ctctgtgtca gggtgaccgt ggctgcggct gcggtggccg agggtccgag ggccacaggg 960
   tggatctctg tggtgctggg caggctgatg atggtggcct cgcccagcag gttgcagata 1020
   cgttgttgca tgggggtcag cagcactggg gctacagctg ggccaccgcc accactgcca 1080
   ccgcctgtcc caggcccccc agctccgtcc tcctcagtgg gccccggcgc ctcaccaccc 1140
   tccacggcgg cccggacctg ggcaaccttg cgacggacct cggtcttgag gtcagaccac 1200
   ttcttcttga cctcaggcag ctctctgcgg caggtggcca cggcgttgac ccttctcagg 1260
   atgccgtgcc aggccgcact cttggcggcc aggggtaccc cggcgttgaa gtggttcacc 1320
   agcaggtgct tcttcagctc cagctcctcc acgatgatct ccacctcccg ctctgagaag 1380
   ttcatcttcc ttttcttggc tgggacggcc atggcctctc ccctcccctc tttttaaaga 1440
   aattataatc ccctcaagcg ccccaattcc ccttctcttc ttcctcagcc tcgcccctca 1500
   cccaccccct acaggggata ggccaggctt gctcgaggcc aagcactagg cctttggtaa 1560
   cccccctcgc tacgcaaagt gcgtagggcc cagccccgac cggcccggcc gctgccagcc 1620
   agcagcgtaa tggcttcctg aatctgcctc ttccgccgag gtgtgcggag atccgcccac 1680
   ttcccctctt cccgcctccc agcgcttctc cgggaaaacc ggccttcccg gttggtagct 1740
   gctcaaatat cacgtcgatc actggttctt tcgatctgtc gctcagctaa agagcacgcc 1800
   tgcttactgt aattcgcctg atcattggtc taaaattgtg tccgtcgccc agagggcccc 1860
   gcccacctaa cctttattca cttccattgg aaaatcattc tgtcgttcaa ttgtcagtat 1920
   tacgactatt aagccgttaa attcagctat tttattggtg gtttagggag ttagattacc 1980
   aaaaccctgc ttcctgagga t 2001
<210> 143
   <211> 2001
   <212> DNA
   <213> Homo sapiens
<400> 143
   gaatgaaaag gtggctcagt ttgagacagg ggtactttgg gcccctgtcc tggcatgctg      60
   ggagccctcc ggactccatt atcttccagg aggggcttgg gcccatcctc tgcttcccaa     120
   aggccgtact cacgataccg acccccttcc ctccctgagc cacagatgtg aacgtcagga    180
   tccgcgtgct tggccgccat tcccggagct tccgtgggtt gattgcaggg gcaaagctcc     240
   gattctgcca ggatgggtgg ggatgggaat cggggtttgg gggtcccgtt gatgtttgag     300
   cgatggtggt gggtttggtg ttgggggatg ggggtggggt tggtgttggg ggatgggaat     360
   ggtggtgggg gtgcgggggg gttaccctca gtcccctctt ggggtcctga ccttgattcc     420
   tggctgagtc tgggccctga gcacttctaa ccagccctgc cctggtcaca caaagctctt     480
   acaccagagt tccctgcggt taaagcggcg gggggtggcg gggtgaggtg gtggcgactg     540
   agcctgagaa gtcttcccct gcagggtggt ccaggcctgg cagcagaggc agcgctggat     600
   tattgggggc cctctgtctg gggtgaggcc cgcctcagtc ctccctcggc atcttacctt     660
   gcctcctcac ggagcctgcg cctgcttccc tccaccgacg tcaggccgtt gctcagggcg     720
   agaatctcgc tgttctctga cctccaacgc gcaagtcagt ggcgtcacat ccggccccgt     780
   gacttccctg ggttcacagc aggggtggaa ctggattctt cctggatggg gatccagatg     840
   gaggtggagg taagcatgaa gatggtgttg ggggtagggg tagggatagg gacggggatc     900
   ctgaggctgt agatcctggt ggggttgggg gagttggggg tgggaggggc ctttggtcat     960
   ccctcaaggt cctgacttgg atgccaggct gagcctggac cgccatcctc tgccgattgt    1020
   gtctgctccc ctcagaccaa gtctctgact ctgagtcccc ctgaagtggc actgggggag    1080
   gggtgcggtc ggggtgacat tattgcctaa gtttatcagg gctgacaaga gggactgagc    1140
   tggattctgt ggtcccacta cgtgggggag ggtggaccct cagtcctcac tcaagagggt    1200
   cctcttcctc actcctggct gttggatgaa gtgattggca ggagatactc tgtttctgta    1260
   acgtctgtgc atttgcacag ctgcacccct tgtagagaat ggctgcgggt cccaggccag    1320
   gagctccctg cagggcggga gctcccacga tcgtattgac ctctggaaga agacagacac    1380
   tttcccacgg gagctcctct ccagccagag ctacacttgg caaacctttg gtcctaaatg    1440
   atttattcac tgaattgaag aaatacggtt tacatatctt ccaagtatat atgtaggtag    1500
   cgatatatat atttatttct acgaactaac acctaccctt agtatgcatc gttcactctt    1560
   actatcctaa tctacttcta ttccagtctt tgtgttctgt tgaaggaatt tttttttaaa    1620
   cgtaattcct ctcccactat gttggtttca ggttgttatt agcagaactc tggagccatg    1680
   ctgccttaac aaacccaatt ttcctggtgt cctgctatta cctagggctc ttggcatcgt     1740
   gttttcagga gctgcctcag attgtgcaat gaagtgaagt ggaaatttta aaacttttat     1800
   tctacccatt ctgttatctg ccgtctaggc aggctacaaa cctccccaac atccctgccg     1860
   ctaaccccct ctaaccgaca cggcaagtgt cacacacaag taccagctca acaaggtctt     1920
   cagtctcacc ctggactggc cctgttttgt tgtgtagatt ttcttgctaa tactggtttt     1980
   ttactttctg tttccattct a                                               2001
<210> 144
   <211> 2001
   <212> DNA
   <213> Homo sapiens
<400> 144
   ggtccccggc gctgcgtcct cccgacccgc tccccgcggc gcccgggtcg cgccttgcgg 60
   cagtcaccga gctgagggca gggcgtccca gcgtccctgg ccccggcccc gctccgcagc 120
   gcgccgcacc ccgaggctcg ggccccgcac agctgcagcc ggtgccctcc ggccaagccc 180
   ccgcctggcg tccccggccc cagccccggc cgcccgccgc tgaccttttc ctgcgcgcgg 240
   ttgagtcgct tctggacgtt cttggcgaag atgcccgtct tgatgtcggc catggctgcg 300
   ggtccgggga gctgcgaaga gcagagcgcg cagcggggct ggcggcggcg cggaggagcg 360
   ggaggaggag cgggaggagg aggagagggc cgagcaaggg aggagcggga gaagcggcgg 420
   ggagccgcgg agcgcggagg ggtgagggcg gggcgcggcg gcgggaccag cttaaagcga 480
   cagagcgggg gcgggatgcg tcgggactgg ctgtgacatg gggagaaggg gcgggggaag 540
   aaagagaaag aggccaagaa aaagagaggg aaaagccaga agccagattg tctcctcgat 600
   gatagaagag tttcgagagg agaaagggaa ccaagcctag cagaagtccc tgggcccagg 660
   ggcagctcgg gggttctttt aatgcagttt gtatttgggg agagtgaggg gacctgattc 720
   tttccagcaa aaaaagaaat aggggagaca aaaaagggaa gaaccctagg gatgtggaag 780
   ccagctggat tggaactgct ctgaagtgga gctgatgggg ctggtccctt gctgctccca 840
   aaagtttcca gctaacagag gcccctccat acaccctggg tgttgcagaa gaaggtatgg 900
   aggtcgccct accctcagga ggctcacagc ccagtgaggg gccctggctg ggaaaccaac 960
   gaagtcagag ctgcaggaga gaggcgtgtg gagagcaaag cggggagaga ccatcgctga 1020
   cacttgttta aactttaaaa caagagttag cagggcgggg aggtccgcgt tgagaaaggt 1080
   cagcttccgg aagacaggcg atgtgttttg gtcatctcca aaattcctgt gctgggcaga 1140
   aaccttagaa tagactactc ttcaagaaat ggtgcatgga acagtcaagt cccttatagc 1200
   ttcgcatcaa ccatggaaga cccagtcccc cctaacttgt tgcttccttg gaaacctttt 1260
   ggcaacctta acagggactc accatcccta ttattcttat taatgcttgg gagaagtacg 1320
   aataataggg ataaacctgc cctacaccta gagctcttgt tggctctgac cagtggtcaa 1380
   actattcata ttccattctc actgagagtg tgaggagaag aagtcaggtg gtgccctgca      1440
   aacagaggtc agaagtggtc actcacagtg aatcctatga gtccaggtga gcatcctact      1500
   gtacctctgg gggaaactgt tacattggta gacctcagcg agctgccacc tggtgttcac      1560
   tcttgtgtca ccctgtagtc ccctcacctt gattctagaa tgatgctatg gccagcttta      1620
   atcaataaga tgtggtgaaa gtgacgccat ggtaattcca gtcccccccc cacccaccca      1680
   acctagaaac caggcaagtt ttgccctctg gggattcctg tgacaccata taggaaattc      1740
   agctaattca gctaccctgc tgcaaaaata aaataaaata aaataacaag gagaggccat      1800
   acagaaaggg agaggccctg agattacgca gagagagaga gagagagaga gagcagccaa      1860
   gatgtcccag ctgagcccat tctccaatca gttcactggc tggctgcagc cacacaagca      1920
   accatcagca agaccagcag aagaaccact cagctaatcc cagccagagt gcagaatgtg      1980
   agcaaacaaa acagttttta t                                                2001
<210> 145
   <211> 2001
   <212> DNA
   <213> Homo sapiens
<400> 145
   ttccccagaa gcacgcactc agggacagcc ttgggttccc tcccaacctt agccacagcc      60
   ccagattcct ggagatggcc ccgcactcac tcttcttgca ggaggtgcat ttgcactctt     120
   tgcacttgca ggagctggcg caggtgcagg agacacctgc tagaagagaa aaagccagtg     180
   aacggtgagt gagatgcaga aggtacagca gtgggtcaat gagtgtccac ttcccccttc     240
   tctgtgcaga gccagccctc agagctcctt tcccactcag agcagaggaa gagaagcccc     300
   atgtcctctc cattcatgct gggaaaacca agcaccctcc tatttctact ccaaaggaaa     360
   gtcccaggct gcagacatgg cccaggaaga cttcgacttt ctgtcctgaa cctggaagag     420
   gcaatgtccc ctcctatcca atgtctccta tccaatccgg tagctcagag tcaggttaga     480
   gaattggggc tcagccagca gccctgtgac caaccaggtg acgtggagca ggacagcctt     540
   gagcctcagg accctcaact ctgacataga ggaacaggga ggaggaaaat gccctcagga     600
   gcttggccaa caggaaagca ctggaccaat aaaggagtcc cctttacctc tgatagcaaa     660
   atacgacctc ctcaaaccca gggacactct ctatggtgtc tgggaatttg gcatcccaag     720
   gcacagaacc cgggcgtccc ttaccagcgg cacaggagca gttggggtcc attgcaaccc     780
   gaggcgagac tagagttccc aagcgagaag ggaagaggca gtgggtgcac gtggaaggcg     840
   gagtggagcc caacagccag cggctgtttt tatagtcagg agacggggcc gggcgcagac     900
   gccccgcccc gcccttgcac ccagcccgct gagtccgcac cgcccgcggt cccggcctgg     960
   gctgtgcgca ggagatgggc caagtgcaag gtcccttgag cgcagctggg cgcacaccgc    1020
   aggacggccc ctttcgcacc ggctcgcgag ggaggcgctg tgccccccgt gtgcggcttc    1080
   tctcaccctg ccaggccttc ccagcttccc tgaggttgcc tgctacaccc gccccttggc 1140
   gttccgccta ccccagtatt ctggctttcc cctcaccttc accaacgccc aggtctctag 1200
   tcaatagcta agcctttcct ggagtcgagg gcaaccccag tcttgtgctc cactttctgg 1260
   atctgccaaa gcggggagca tagcaaggga gtagtgtgca aagggcaggc caagggtgcg 1320
   aacttcgcat ctctggcagt atccgccctg tccccgttcc cacctcagtc ctttggtcac 1380
   gcttctcctg tgctcagtaa cacggaagaa acagtgatct gcctctctct tctattctcc 1440
   actcttgagg agctgaatgg gcccatttgt ccaggtcctc ctttgcataa atctctccag 1500
   ggtctcccca cagcctagcc gggtacccca ctacaggatg gcagtttgga aagtgtggca 1560
   agtccctaga agagggatca ttgaaggtga tatgaagatc ctgcattaaa ttagaaagca 1620
   agagcgctgg ttatttttcc ttccatattc ctattactct atgttacacg ggtctgtgtt 1680
   tggccctgtt taatcacttt ctgtctttga accctcacag caaccctaaa gggacatgag 1740
   tccttttcac gaacccgtat tcctaatctg gacaatgacg aggagttcaa gccactagcc 1800
   cagggtcagg gagctgggaa gtggcactgc ttggtgtgaa tcctgtcagc ttccctaggt 1860
   agagaggtca gtgatgaagc ttctctatgg cctaggtatg tttatctctt cactgcttga 1920
   aatctttcaa caaaaccaag cattacagtc agtcctggaa gcaaaacaga gacatagagg 1980
   gatatgaata agtatacttt g 2001
<210> 146
   <211> 2001
   <212> DNA
   <213> Homo sapiens
<400> 146
   ggggtccccg gcgggcgggg ccggagtcac ctggcagctc gggactcagg cgctcccggg 60
   gtcacacggg gccgcgcaca tcagccccgc cgacgggcac gggcggctca catcgatccc 120
   ctcgcggcca cgctcacgcc ctgagttgtt gggatcgcgg gcagaagtca tcccgacagc 180
   cacccgtgcg acgacacagt ggcccctcac tcgcgtcccc ctgacagcct cgcagtcatt 240
   cacaagccgt cacagtgacc cacagccaca cgcaggcggc caggcactgc ttacacggtc 300
   ccgccgtccc acgcaagtca ccctgacagt cacttataca caacgacacg caaccactca 360
   cccgcaaaca gcctcaccgc cacagacgag tcacccacag acgctccgat gccccacaca 420
   ccgtgacatg cccccacccg ctgtcacacc caggcacaac gggggacagt cacacaaaga 480
   agtcgcccga cagtcacact ctcgcagcca caacgcctca cccaggcgca cgctgtcaca 540
   cacagccacc cccctggacg gtgagcctca gacacagatc gtcctgacgg ccacacagtc 600
   acacgcggtc acacccaggg acggccacac ctcggtcatc cacactcgcg cgtcgaggtt 660
   taacttctca gcgccgcagc acactaattg ggcagtttac taattggccg tcactgcctg 720
   gcggggcctc ggattccgag taggctgcgg ccaatcgagg cctgtgtcct cccgggagcg 780
   gtgagccggc gtccctccag gctggggctg tagtgccgga caccctccct cctccatgtt      840
   tccaggatct atccccccac cccagccagg tcccagctgc gctgcgccgg gcgcattccc      900
   agtgatggag gcttgcgtgc ctggaacccc gaacccgagg cgtcaggtcc cactgccggg      960
   tctggcccag cctggatggg ttgggtgcct ctaagcagtc cgcgtcccct tcggtcgccg     1020
   gagagggaac tttcaaactt agttaggaag ccagactgtc cgcgcgtccg ccagtcggtg     1080
   cgtcggtccc gggccggctg gagccaagcg cgggttttcg tcactcggag cccggattga     1140
   acagcgcgcg tgggtttccc gcagccctgg cgcagacgcg tgggctccgt ggcgatgcgg     1200
   ggtgagtgcg cgtccagtgg ctggatcggc gccccccagg gtctctcccc aacctcgcag     1260
   gcttttcgtc aggcccctgg tagtggggat gaggcaggag agaggcgtcc cggcgcggat     1320
   cccggggcct cccgggctgg gcctccctga gtgcgccctc aggccccagg ggagcggctt     1380
   ctggcaaagc ctccctgcag ttcgccaggg gcagcaagca tccaggccac tagggcctgc     1440
   gcgatgtggg gcggtggtgg gcgacaagtg aggtctcgcg aagagtggcg gtggttctct     1500
   tttgtccagt gacctgcaaa gttagaagcc gatgaggggg gatggtagcc tcaggagaag     1560
   gccgggagct gcctccagtc tgatggagga aaatagatta aaagtagcaa gtggagtttt     1620
   cagggaggag aaggttgggt gtgggggcag ctggtctgtc ttctgccctt ggctttgtta     1680
   tttttcaaaa tgtgaagttg gcgaggtagg taccagcctc tggtttgcgg gtgtgggtgg     1740
   gctgggcatt tgtgcctcaa agtctgagtg ggtgggggtt tagcctggag cgctccgttt     1800
   gagggtggtg tttctataaa tctcgccagg gtggggctgt cccaaatagg ggcctttgga     1860
   ggcaggactc agccgaggcc cctgctttat tctgatgctc tgtaaacctc tgactcagca     1920
   ccagaacctg tgcgaatctc gccgggaagg gcccgtcagg gagggaatga gaagccggcg     1980
   attctggttt ctttctgtgt g                                               2001
<210> 147
   <211> 2001
   <212> DNA
   <213> Homo sapiens
<400> 147
<210> 148
   <211> 2001
   <212> DNA
   <213> Homo sapiens
<400> 148
   agtgatgaaa aaaccacagg ctggtgatgc aggtcagagg aggagatgct ctttcacatt      60
   cggacttgag agagcccgag agtacccata ggcatgggtc aagagagcta cagtggagct     120
   cttgggggac agaaaggagg atgtagaaaa gaaaggggca gagactgtgc ctctagccac     180
   ccatggatac ataaggcatt gaatccatta gtgaagagaa ataagtaatg aagcatctgg 240
   ggccccgtgt ggctcctatt ataatgaatc atgtatgttc tcatattagt tattaagtac 300
   acctggcttg gtatatccga tggcctattt tgattaattt atctaattct tttttttttt 360
   tatttcctag gccttgggtt aaaagactca gctatcaacc tggaggataa ctgattacct 420
   agtcagagtt ctggtacctg gagacaaact aactttgatt aaagcatcag atcaccaggt 480
   cttaaacact gggcaccaaa ccggtcctgg gacaggggct aaaccttgtc agttatgatc 540
   agggagcagt cctagaagaa aatagcaccc aaaggagaac ttgctctttt gggaatgaga 600
   aagagctaat gttttgcact atggattcag tcaagaagag aaatgctttt ggggctttct 660
   taaaaggcca aatgtaaaaa gtacccccat catttcctaa aactcaaacc tctaggactc 720
   tacctcttgg gctaattgct catgagccac caatctgttc tgtcttgcac tgtgtgttct 780
   gagagccagc aggttaggac agagggaaca tgacggggtc agaggagggg ctcacctgac 840
   tgcacagctc catgttttct gagtacacag caatctgtcg tctcggctgc tgctcctctg 900
   acagatagct ggagaacagg atgaggacat caaaaccaaa cttgtctgta aatgctttca 960
   agtcactggt aatattgctg tgaaatagac aattctgaaa cgacaacagg aaatttgata 1020
   ttaatgtcaa actgcccagc tgcggggagt tgttccacaa atttgtccag tcttatgatg 1080
   tatttaggag agatatttat tatttaaggc aagtgaaata cagtttgctt tgagaaggag 1140
   tggaaacttt catgctatgg cttaaaatac actttgcttg tttgttttcc caaaccatgt 1200
   aagaaaggag tttttgccat tattaataat agtttagaca aagaacagat atcctaaaca 1260
   tttttttcta aaatatttat tcagatgctt aatttgcaaa tcctattttc cctcttgtcg 1320
   cttataggat ctatatacga atgtctgcta ttgttacaat ttatcacaat aagaatagga 1380
   ggaagttatt tcttcctgta ttttttttct aatgattatt attaactcca aggagaaatt 1440
   cctacgaatc aacatagctg agtttactgg gtgatcttta ttccagtgta gaattctggg 1500
   agatttttaa aagagctgtg atgtcagatt tgcttgttct atgtggcagc tgtggaggaa 1560
   tgaataattg cagaaaggga tcccaatacc tcagattacc cagatgtggc tcatatcatt 1620
   tgcaaaaatg ttctggctgc ttcaagtttt atttactgta tgtatgtact tatttattta 1680
   tttttgagac agggtctcac actgtcaccc aggctggagt gctgtggcgt tatctcggct 1740
   cactgcaacc tatgcctcct gggctcaagc aatcctccca gttcagcctc ccaagtagct 1800
   ggggctacag gtgtgtatca ccatgcccag ctaatttatt gtattttttt gtaggcgtca 1860
   ggttctgcca ttttgcccag gctggtctcg aactcctggg ctcaactgat ccacccactt 1920
   catccttgca aagtgctggg cttacaggcg taagaccgca cccggtccgg tctttgagtt 1980
   ttattaacta caatgagagc c 2001
<210> 149
   <211> 2001
   <212> DNA
   <213> Homo sapiens
<400> 149
   acctatggct tgcctggttt acactttctg tttcaggtca gccagaaaga cgtccatcct 60
   acttcctgag gtttttcgtg taatgagaca ctcacgtgta cccttttcca cgagcgtttt 120
   attcccactc tccaaagcga ggtcttcctc ccctctaggt ggtgacctac aaagtgacct 180
   aaaatcctgt cagttcctat ctttgaatac tggagtttct ttgatccaaa atacatttgt 240
   acccagtgct caaatggctc atcgaaacat cagtttgaat aaatcatgaa ccaaattgta 300
   tccccgacat ccagggccat gggtgacaga gccttgttgt cttggagcat aagggaactc 360
   aggccagtgc ctgacactgg catcataaaa gattccattg tattccagag agaaacgctg 420
   gcctccccca atttggaggc tccttcttct ccagtcccat ggatgctctg cagcctgcac 480
   gttccaccct gacccctctg cccccagctg atgtttgcct gggccacatt ttttcagcgc 540
   tagcatcacc caatctttct cagtgctgtg gactggccct agctccctct tctgcaaccc 600
   tgaagcagaa gcggcacctg agaacacctc ttgggctgct tacctggatc tgcagcttgg 660
   tgtctgagag agatggaagg ccagtttctg caatcactca gctcccgggg tgtggacctt 720
   atacagatta caaaggggtt tggcttccaa gaggaaaagg gtgggaggag catctggagc 780
   tcagttcaaa taaaatcaag gacgtgagga aggagtggtg aatcacccag gccgccgtct 840
   ttctagggct gctgaagaaa gagcaaatct ttgcctacgt tagtctttct tcctatgtga 900
   accacatctg tctgctgatg cacaaggagc agcagagaaa gccactacag cactggtgcc 960
   caaacctggc acactaggaa caaagtcctt gttacttctt cgtgggcgtt ttcaccaagt 1020
   agacttggag gttaacaaag gacgcaaagg agaggttcta atgggagata gggaattctg 1080
   gccaaagcta ttccagggct cctagggagc aaaacccaag tctctttcta ggagggcatt 1140
   ctcccaacgc tacctctccc ggggatggga ggcggggtcg gggggaatgg aggctgtcct 1200
   gctgccccca gctggaaggc ttcccagatc tggagaagaa agggggagta actgaatagg 1260
   gaggaggagg aggcctctag ggtgcgctca cccagttctg gccacttccc acccaccctc 1320
   cacccctgct ctgggggcat cgagaggatg gggctgttct tatctacagt aaccactgac 1380
   cctgtaggtc ggaattgagt agaggaacta aagataaact taaatcccaa atctcaaatt 1440
   ctttttgggg aaaaaataca aagaatatca tatttattag cagcagaatg ccctcctaga 1500
   aagagacctg tttttttgct gagtaacaat ttggatacct ctaagaatta tcaaaatatc 1560
   ccttcgtgct gtgtattcag attgtttcag cctttctgtg taaaaagtgg ccatagtgaa 1620
   attatctgac ctaccttgtg tgattgtagg tcgtaagatc ctcattccag agagggtcct 1680
   gccccccata cccagaaaaa aaggacgaat gctcagagag gccaagaaga atctagacag 1740
   ggaggccttg ctgggtttcc ccactcagtc tattttcatt agatcgccct gttgtccagt 1800
   catatttcta cacagctgtt catactttgt tgatatccaa gtgtaagaat gcccactttc 1860
   ccctgttatc tttgggtctt ctgaaggctc ccgtgtcgtg taaaactacg atcaaatcaa 1920
   tttgtatgcg ttttttccta tcaatctgtc ttttgtcagt tgattttcag caaacctttg 1980
   gagggtgaaa ggggaatttt t 2001
<210> 150
   <211> 2001
   <212> DNA
   <213> Homo sapiens
<400> 150
   ggactgtgga ttctttctct ggggcaaata aaaatgtaga atcagaaaat tcaggcactt 60
   tgcactcctc atgggacact ccagcagcac tcacgtgacc atcctgagaa tggacaggac 120
   acctgaggtg gggaagggag cacagaaccc agacaccagc ctggacacag gcacctggga 180
   taatctccta ttccttggaa agttccagta tctgagggag gaacagtgac ttctggtcct 240
   gacctgagtg gagaccgagg gactcagaag agctggaatc agacccccac acacactgag 300
   tatgagtcag agaacaaggc ctgagagaaa aagtcacagt gcccaagact gctgcagggg 360
   tcaaagggga ctgctgatca gtgttccagg gatttgctcc agattaatct gagtcatgga 420
   aaaactgcct ttcttttgtt tctaaacaaa tacctgcaaa gacaaaagac cacataaatc 480
   cccaggtgca caactttttc agatttaaag aaaaaacaac ccccgtcttt ccctccattc 540
   ccaggagaag ctcactctgt ggcatcaagc tgcctgggtg agctctcttc tagaagagtc 600
   cagggggaca ggcaaggaat gggaggcagg aagtccagtt cagggagggg gattctggga 660
   tgaaaagtga agggagaggg actgagccca tgctgagggt ttttctctgg tttctcagac 720
   agctccgggg ccaagattcg gggagacatt gagacagagc gtttgatata aaagaagggg 780
   gtaagagcca agtcccaggg cccggagctt ggctctctgg gcttcaggcc ccgagggcgg 840
   tgcctgggat gggccggctc agctttgggg tttcccgagc tccgctctct ctcttccacc 900
   tttcccaacc tgtgtcaggt ccttttacct ggatactcat gatgcggcct cagttctcac 960
   ttccattggg tgttgggttc ctagatcagc caatcagtgt cgccgcggtt cctggttcta 1020
   aagtcctcgc cggcccaccg ggactcagat tctccccaga cgccaaggtt gcgggtcatg 1080
   gagtcccgaa ccctcctcct gctgttctcg ggagccgtgg ccctgatcca gacctgggca 1140
   ggtgagtgcg gggtcgggag ggaaaaagcc tctgcgggaa ggagcgaggg gcccgcccgg 1200
   aggagtaaat ctgcacatat atttaattac agattacaat tacaatcaag gcagaaatga 1260
   tctcattttt acattacaac tctggaaaag gcaatagact gagatgcaag tgtgccccca 1320
   agtgatgggc agaaggagag aaggtgtttt ggatgcattc tagaacacag gtaatctaag 1380
   gagagttgat caaggccgtg gaagagtcct ccagccacta ttggccatca aaggagtcct 1440
   ctgtgtccca ggaatggtcc tgctttggtg tccctggtgt gacccatcac ccgctgggaa 1500
   cagcctgaga gaagtagggc ctctgcacca atgctgctga ggatgtcaga gcacaggaac 1560
   gaggccttgg gaaattacct ggaaatgcga ctgaaatctt ccttcctgag gggtctgggc 1620
   tcttggaaat caaaccctct caggttgggt ggctggacga ttctcctcac acttacaatg 1680
   ggacaagggg aaccaggagg cccccaaggg gatccctggg ttccacacga actcctccta 1740
   ccctcattgt gtgacagcag ccatgcctcc tcctggggat caggatctat tacctgtgcc 1800
   tggagaggag gggactcctc ttctcacccg ctggtctctg gacacatact gtccaattcc 1860
   cctgtggcag ctgtaatgtg tagttcaatg ggcactcatt tgtccccttt taagggtacc 1920
   ctcctttaga atccaggacc ttctaccctg cagagtgtgg ttttgggaga gaagtgcaaa 1980
   atcccacgac aggtgagttg a 2001
<210> 151
   <211> 2001
   <212> DNA
   <213> Homo sapiens
<400> 151
   accattcatc tgcctccaga ctttgctagt cctcagagac actgctgcga gggtagtggg 60
   cagtgggatt agcctcccgc agagctctgg cctcctccag agcttctctg tatcgggagg 120
   gccaggcctg ggggtctttc ttaaagaccc tggccaggaa ctccatgatt tgcatcttgg 180
   tgatttcgcg gctggcccgg gagccccaaa agaactcgta ttcgggcggc tccacgtatg 240
   ggacgcgctg gtacttcagg taattcattt ggacgaactc ctcagtgatg agcttccgca 300
   cgtccccgaa ggtggagtgc ttcttccagg gccgcagccc caggatgcgc agcacgttcc 360
   agacggcgct ctctctggcg ccgcggccct tcacgtagat gaggctcagg atcatgagca 420
   ggaggcctgt catgggcatg cggttgctca gcgccaccct gtccagctcc tcgggctcca 480
   gcgctttgac cagcgcaaac tccatggtgt gcaggctggt tagcctcagg tgcagcccga 540
   acacccgggc gaggatgagg ctggtgcgcc ggaggatgct cctgcaccac ttcttgtagc 600
   tgccgatgac atctttcacc atgtctggaa accagatgat catcttcttc tggtccttga 660
   ccagcacgta ccacatgagc tcgtgcgcct tctgcaccag ctgcgccggg gctggcggtg 720
   ccgggcccgg ctgggccgcg ctcggggcct ggtgggcgcg gccctcctcc gcagcctgct 780
   gcagggcctt cgggtcgccc tcgtcgttcg gggcctgggg aggcggcgcg gcctgcggag 840
   cggccgtcgg gcctagagga gggctctgcg gctctgccag ggtcgcggac ggaggaaccc 900
   cctccgaaac cccagggctg ctgtgcacct cggagttggg ggcctcggct gcaaagttag 960
   ggtcgctcag atccttactt tgttctgaca tgtctgcgcc gtctggcaag ggcagggcct 1020
   ctgcgtccag gagctcttcg agcctgcgct ccctccgcgg attcctggag aggaagtgcg 1080
   cgttgctgcg cgcggcgcct tccgcagcga cgggcagggc ggggcggcgg cgggacgcgg 1140
   cactgcgcct gcgcgaccgc ggccttggcc agcggctggg ggcgggcggg gcagcgcggg 1200
   gccccgacgg ggagggtcca tggcgaggct tcacctgctg ggagagcgat cccggggttt 1260
   cctgtttggg ctgagaagat ctagggcagg agagaagtga gatggatgcg tactggagaa 1320
   ggggccagtt taagggaaag ataccttaag tttttgggat gttggggtca agtttcttgg 1380
   gaaacgctta cctgggtata tccagaatta agttaaatat gagagtccca gagaaaggtc 1440
   tgggcggctt tgtaaacatg agctttatgc gaaccagtga gaggaacagt cgggcttgtc 1500
   caagaaaatg tgtaaactgt aaagtggatg tctgaggttg gaatcctgag aaattccaat 1560
   aaaaaatgat cgactcccta ccaaatcaga taggctataa gaaaagggga aatgcagtgt 1620
   gtcgttaacg taatttccta tcaacccatg tgaactatga actttttaag taggcagtgg 1680
   acttagagca tcattaatgc tttaacgggc ataaatttgg gttttacact tcctttgggt 1740
   aactttgatt ccattttatt tttttccata cttagtccat agcagaagga ttgctatatc 1800
   ttttagcaca aacttgtatg ataatgtctt ttccctctgc ccagcaccct cattcttcaa 1860
   ttcaccttct ctcaggaccc tctctgaagc tactgagtcc tccacgttgg aagccacacc 1920
   caagtctttc ctgagattta aaaaaaaaat aggtcccatg ggtaatatct ggtagtgtct 1980
   ctgagatgac caaagggtag c 2001
<210> 152
   <211> 2001
   <212> DNA
   <213> Homo sapiens
<400> 152
   ctgggatctg acaactgaga agggtctcag cccagcctgc ccctcccctg ccgaccccac      60
   aatcaccctc ccatccttta gcctcctcct tccctctctg tctctcaatg tccccttgcc     120
   tcttctgctc ctcacccaca tcccccacac atcctcccca acccggtagg ctgaggggag     180
   gggagagaaa gtggaggggg tgctagaagg cacatactac aagcaagaca gccactcagc     240
   ttatctcaag cgaccccaca gcatccccag ggcataggcc tcctcccaga tgaataagct     300
   gaggttcaga gaggtgctgt gacttgccca aggccacaca gcggcaccaa gccagtgacg     360
   acagcccagg tcagcctggg gacaaaggct gaatttcagg tttcccactt ccctcctgca     420
   tccacagagt aaagactgag gcttgcctcc gtgggagacc tgcccccagc ttttggggtg     480
   caggtgagga gccctgggca ctctcaccca tagtcctgca ccctccaccg agatggcttc     540
   tggggtgggg tggggctgaa gagggtgaag ctcccctcag cccacccccg ggtgctccct     600
   gtgggccctc ggagaaggaa gcgtcccctc caagctgcct tccagggacg cctagttctg     660
   cagggccagg ggcttcccca ggggcaccag ggcggaagga tctcagggca tcccagttgg     720
   gggctgtgag gaagtccagc caggtaccta cccatctccc accaccacac acttgatggc     780
   ctgcatcgtg tccggagcct ggagagtgtc ggtggtgaca gctcagggcc aggcgcgttt     840
   ctgcgggcgc aaggggtgtg gaggctggtg aggcgcctgc tgaggagcag cggtggtggg     900
   gcaggaggaa acggaagggg aacttactca agctgctctc cccacggtcg tttgcagggg     960
   gcggagcatg cggtctggca gagcagctgg tgggaggggc gctcaggtgc tgggctgagc 1020
   cctggccatc cccacaggcc ccctgcgtgg aggctccagc aggagacctg ggggccctct 1080
   gtgctgggag gctcacttcc tgtccacggg gcgggcccag aggctgttct ccaagaactt 1140
   gagggttcca agaattcagt gcacttcaat tctaagcaga gcatttaatg agtgcctgct 1200
   gtgtgccagg cacagtacca ggtgctggtg atcccaggat gcaagagaca gctggtccct 1260
   ggagtttata ccctttgcag agagggaggc atgacttcat catgggagat cataacagag 1320
   acaggaagtg attgaggcaa cgggggaatc tacagagagg acctcagggt taccgagatg 1380
   gagagctggg ggcctgcaag ctagcattgg agctggctct tgaaacgtgc gccctaaaga 1440
   gggaactcca ggtgggggga gattgcttga gccaggaccc agaggctccc ccaggcaagg 1500
   gcctgtgtgg gagtccctgg gtgggaggat gtggggcgat gaggcaggaa gggaaggtgg 1560
   ggtccgctga tggagaaggg cacattgagg ggaagtggcg ggcagtgccc cacaggagcc 1620
   aagcctattc tgaggccgcc ctgggggacc aggcaccctg gagctagaac tgtggcccat 1680
   ttccctgatg cgaaaactga gggtctgaga agtcatttga ccacctagcc tgaaaccagc 1740
   cctaggctca ggtgactcca agctcttctc caagctgcag ctattggctc cggctgctgt 1800
   ttgaagggac aaaacctgag ttctgcagct tgcagggcgc tcccttcgcc acgagggctc 1860
   tccacgtgga ctcgggtgaa tattcgacca ccccagttgg caccaagttc actgttcatt 1920
   gctatcactc cccacccaca cacctttcca gctccggggg gaagggacgg aggggcgggg 1980
   agaggaagag ccactggtca c 2001
<210> 153
   <211> 2001
   <212> DNA
   <213> Homo sapiens
<400> 153
   tcaggaggcc agtcacttgc ctgtagccca cagttagtgg tagcactggg attccaactt        60
   gggtcccctt catgtcgcct gtatttggtg tcccatttag gcgtccatgc aggttttggc       120
   gtgtctgaag atctgtgtct ccccaagtaa gtgtccccca tggatgccca cccagccgtg       180
   tcctcctgtc tctgaggttt ctccaggaat cgagctcctg gcctggcttg tctgagtgtg       240
   tctgtgggcc agtagcatgc ccctgcccgt ctgggtccct ctgcgtgtct ctgcttgtcc       300
   tagcctgtgt gtgtcagtga cgtctaagag tgtgtgtgcg tgcgtgcgtg cgtgcgtgtg       360
   tgtgtgtgtg tgtgtgtcct tgtccgagga gccgagagag tgagggggaa tgaagggcca       420
   aggaggcctg gctggggctg ggctgggggt ggaggcgggg agaggggcgt cacggcctga       480
   gctgagaggg gagtggagtt ctggaggaat gtttaccaga cacagagccc agagggacag       540
   cgcccagagc ccggatagag agacacggcc tcactggctc aggacagggg gcacagccac       600
   cagggtcccc ttccccctcc tcagctccct ccctggcccc tttaagaaag agctgatcct        660
   ctcctctctt gagttaaccc ctgattgtcc aggtggcccc tgggctctgg ccctggtggg 720
   cggaggcaaa gggggagcca ggggcggaga aagggttgcc caagtctggg agtgagggaa 780
   ggaggcaggg gtgctgagaa ggcggctgct gggcagagcc ggtggcaagg gcctcccctg 840
   ccgctgtgcc aggcaggcag tgccaaatcc ggggagcctg gagctggggg gagggccggg 900
   gacagcccgg ccctgccccc tcccccgctg ggagcccaac aacttctgag gaaagtttgg 960
   cacccatggc gtggcggtgc cccaggatgg gcagggtccc gctggcctgg tgcttggcgc 1020
   tgtgcggctg ggcgtgcatg gcccccaggg gtgagtgatg ggggctcctt ggggcaggga 1080
   tcccctcgga ggggctgggg caggggtagg aggtggggga tgatggcagg tgtggggggc 1140
   ctgggctggc ttagggagtt tccttgggac cacagaaaag ggacttcaag ggagggagac 1200
   acagactcag agacaccaag tgcggcagag ggagagaaag actgagagag gaactgaagg 1260
   gcagggggga cagaagagag gacccggaga gacagacaga caaggagaag ctgagggcag 1320
   gggtgggggc tggacctgag aagggggcca tggggagagg ggctgagctg agggtcgcca 1380
   ggaaggaaga aatctttccg aaggggcacc gagtcaggca gtgggatgag gagggctctg 1440
   gagccctggg ggaaaacgct tctccctgcc aacaccaccc ccctgacccg ccacttgtcc 1500
   ctaccccacc agtccactcc atctctgggg aggaggctct cactttgtct ctctgtctct 1560
   gcctccagct ctgctatctc tgtgggtctc tgtttctctc tctttcacag tctctgcctt 1620
   tatctctccc tgctctctga gtctctctct ctcttctcag cctccctctg tgactgtatc 1680
   ccccctccgc ccccaccttt tcttctctct tgagggctgt ttcttcattt tttctgtctt 1740
   gccttgtatc tgtgctcctc tctgtctcat ctcccctgcc tttctctggc ttctccctcc 1800
   gccaccaccc ttatctcccc ccaccaccac ccttatctct ctcccccaac ccctgattct 1860
   tcccctccat ccaccagcac cagcgcgacc tgttaagtct ctagagacct aagagaggac 1920
   ggagggggca ggggctggcc ctgccaggca gtgcagggcg tgggaggctg ccagatcccg 1980
   agcctccttc ccgacgggat g 2001
<210> 154
   <211> 2001
   <212> DNA
   <213> Homo sapiens
<400> 154
<210> 155
   <211> 2001
   <212> DNA
   <213> Homo sapiens
<400> 155
   gctccccatt gccaggtgct tttcttcctc ctcctcttct tctttttttt ttttgagaca 60
   gagtctcgct ctgccgccca ggctggagtg cagtggtgtg atcttggctc actgcaacct 120
   cctcctcccg ggttcaagcg attatcctgc ctcagcctcc tgagtagctg ggattacagg 180
   cgtgtgccaa catgcctggc taatttttgt atttttagta gagatggggt ttcaccatgt 240
   tggtcaggct ggtctcgaac tcctgacctt gtgatctgcc cacctcagcc tcccaaagag 300
   ctgggattac aggcgtgagc caccacacaa ggccctttct tcctcttctg tcatctgtgg 360
   atttcccatc tgcctcagtg gagtccttga ccttttcctc cctggcccag cagcacattc 420
   tcctctcctc ctttctccca agtcactgac ctagtattta gtgtgtgttc tcagcctcac 480
   cattgacttg ctgtgttacc ttggacaagt cactctccca ctctgacctc aattttcaca 540
   tctctaaaag gagactgcta gactagacgg tgagtgaagt cctgctgagc cctccgagtt 600
   cattctccct tgtccagtca gtccttccct cccaccctcc cctctgggct ccctctctgg 660
   ggccctctcc cccaggggag gcctgctcca gccccagggg agctgcggcc ctggctcctg 720
   ttcccagtcg gagtgaccag ccctgggcct ccttcccagc ttcggccctg gctccatgtg 780
   gcccatctga gaggctggcc tcaagcccgg cggcaactcc acatttctgt ttttcctttt 840
   ttttccctct ttcccggagt taacaagaag cagatgtggc gcacgatggt tggagaggtg 900
   gggggaggaa gggggaggcc ggaccgccag ccagacaggg gggaagggag gggagccagc 960
   agggaggagg aggccagggc ccgccccaca gccactctcg cgcctccgaa cagccacagg 1020
   ggcaaagccc tgtcaccccc aggatccggt catcagggaa agaggacagg gagaccagaa 1080
   gagggccagc tgggacgagg gggcggacgc ccaggaggca acttctgaga cgcagctcct 1140
   gagaggggca gggaccaggc gcgggaggcc agagggggca cagagaacaa accccctcag 1200
   aagtgaagag gagagcggaa ggaaccgaga ggggacggac aggagctgag gaggaaagag 1260
   gaggggagag gggtcaggcc aggcagccaa ggagaagacg tgtggccggg ggctatcaga 1320
   aggaaactgg gacggacggg ccgggctcgg gctgtcctgt ggagcagcag catccccggg 1380
   gccggcagag gcgccagtgg ctgggcggga tgagtctctg agggccactg tggagcgccc 1440
   cgccatggcc ccccgcaccc tctggagctg ctacctctgc tgcctgctga cggcagctgc 1500
   aggggccgcc agctaccctc ctcgaggttt cagcctctac acaggttcca gtggggccct 1560
   cagccccggg gggccccagg cccagattgc cccccggcca gccagccgcc acaggtaaga 1620
   gtctggatcc cagcccgagg cttgggtggt gaggaagggg tcagaatgcc acctctgcct 1680
   ggctctctgc tgtgtccgct aatgcagggc agagggggag tgagaagccc atccatcagg 1740
   tggctcctca catgcccacc ttcccacggg gccttttcca ggaccccctg aacacatatg 1800
   ttccttgggt cacctagggg gtccgctccc acatgcccca gggaagctgc cctgagggga 1860
   gcgggccagg ccttcctgcc agctgccctc tgtatcctag ccccagagcc tcagggagaa 1920
   cttttgtccc cttctagggc tcccttgacc ccatccagta atccataatt ctgacaccac 1980
   tccagctctg ttccctccag c 2001
<210> 156
   <211> 2001
   <212> DNA
   <213> Homo sapiens
<400> 156
   cccaacataa aagaacgcaa ctagtaacca taaagtgttt aaaagtctgg gcttctcccc 60
   accaagaaaa gacgagttgt aatacctgga ctgtgggtct ccagtccgac aagaattggc 120
   tctctgcttc actggtctcg taaggtcact ccctagaatt ggctttctat aggaccattt 180
   aattgagtct atttgaatct tactcaagtc taccacctta taagtatcaa accgaattta 240
   gtaggattcg aaatggtaag gactgcatta aattatttct ctaattcagc agtaaggaaa 300
   gtgaacagaa gtctcggtgt acaaccacgc cccgggttga catctttcct tccacgaaga 360
   cactgggtac aagccttgat gtagctgtta attgccctcc tgtttcctag aaagcttata 420
   gggcagcagt aggaaacggg ttaggtatgc ctttcctggt aggagccgag ctttgggtct 480
   tatctctgcc acttgttcgg catctgatca gtaaaagggg accatgatgg gaggcgaggc 540
   agtagacagc actttgtgac ggttcctaac cagtcgctgt tatcactgag cacattgtcc 600
   tcgtaatcca gaattcatac tgccatttga atattttttg tatttgcaaa gaaaatttta 660
   accacttaac tcaaggctag aaataaaagc aattagtgat tgcaagagaa atccaccggc 720
   agaaggcagg gggagacgac ttaagcctct gattattttt ggaagcttta tatgctttct 780
   agcctttgtc attgaatgga ttaatgtatg tgcctggcat agactaagtg ctttgcttgt 840
   taaactgccc gcgggtcata gcgagcaaac tgggggagcg cccagggaca gtggatggag 900
   tgagtggaga ctcgtgtcct tttaagaaga ggctccattt cttcctcgca tgtcaatgtg 960
   tgcgtgtcat cagttgttcg aggaagctcg cgcatgacgc gacacttgcc gtctgggctc 1020
   tccaggttcg gcctgctgct ttcacagccc agatcttcgg cgtccccgag acgcagccag 1080
   gcgcccggcc cggcccggcc cggccctctg cacagcccca cttcaggcca cgccgtttcc 1140
   ttgaccttcc cccggcaggg tctgaagcgt caccgtccgt ctagagcaga gtcgcgcagg 1200
   cccctgccct gccggtgagg tcggcgccgc gctccggaga gtcggttcct cccgattcag 1260
   acccatatgg cttcagatgg aaccatcatt tctgttccgt catgaccggc ttgcaaggta 1320
   gcaaagcgaa acaaacgtct cgttttcagc ctgtccccgc caccttgaaa taaaaccggc 1380
   aaaaataaaa agagcattct gggaagtact tcatcatttc aacgctcacg aattcaaaac 1440
   ataaacaaag gcttccgagt gccccggcca ggggcgcggg gcgcacggcg ggcccggggc 1500
   aggtaagcgc aggtgcgcgc ccgcccccac ccccggctcc ctccccatcc gctccccgct    1560
   ccccttcccc tttcccttcc ccgcccgctc ccagccgccg ccgccgcccc gcgcacggcc    1620
   tggagcggag gctgcgcagg gcgcggggcg gcgcgggccg ggcgcgggcc gggcggacgg 1680
   ccgcgtcttt cttctcctgg cggtgatgtc attgggcgac ggcggccgag gccggggggc     1740
   ggcggcgggc gcccgcaggt tcccgagccg ctcctgagaa ggcgcctgac agcgggccgg     1800
   ggcgcacgga gaagcgggcc gggccggacc tgctgggccg cgccgagcca atcgccggcg     1860
   ccggccgctc gatgggcgag gcggcggcgg cggcggcggg ggccgcgggc cgggccgccg     1920
   ctccgaggtg aaggcgcgcg cccctccccg cctgcctccc gggccgcagc gatgaattcc     1980
   gccgagcaaa ccgttacgtg g                                              2001
<210> 157
   <211> 2001
   <212> DNA
   <213> Homo sapiens
<400> 157
   cgggagttcg tgaccagcct gaccaacatg gagaaacccc gtctctacta aaaatacaaa 60
   attagctggg catggtggca catgcctata atcccagcta ctcaggaggc tgaggcagga 120
   gaatcgcttg aacccaggaa gctgtggtgg cggtgagcca agatcgtgcc attgcactcc 180
   agcctgggtg acaggagtgt gaaactctgt ctcaaaaaag aaaaaaagac ttgatcagct 240
   tatagccttt atcatctagt cttctattct ctggagagtc agtggggact cttccgagtt 300
   cttgtagcct tttcttaagg cctataggaa gtcatctgtg ctgagcctgc actttctata 360
   acagatggaa aactgaagct cagacatctt aagtggcatt gacttagact atagagctag 420
   ttagtggggg gccagcacca tgacctggcc cctacctcca gcaggcttca cccacccttg 480
   cacccttgca ggaagcactt agaggcttga ggttgccagc tgtggggggt tgggcctgtg 540
   cagtgcctct cagccgccca cgctggtggg gctctgcagt gttagcttct tgtttctcta 600
   ttttctacct aggtttcagt ttttcacttg ggtttgtttc ccctttgtct ctggttcagc 660
   ttccttttcc tgtttggttt taagtaggct ataaaaatca agttgctgtc ttcagagggt 720
   ctgtggtcct ctgatcaaca taggctggtg ggagtacagg actcgcctcc tcagggttcc 780
   ctgtgctgcc acttttcagc catggccaca aggtgagtat tggaaccagt gcaggggaca 840
   gagggatgtc aagaggaaac ctagaatctt aacggtgaat gggctgtaga aattatctca 900
   ggtcccctta gtatactgat cgagaagtcc atgaccagag ggcagggggc ttacatgagg 960
   ttacacagca ggttagtgac agtctgctcc ctctctagcc gctggtcttt ccattacaca 1020
   ggcctgtgtc tcttgatgcc tgtgaccttt aatggcttct gagtcaagga cttacttaga 1080
   aattagcacc tgcctctcat tatagggagg agggtctgag ccatctaggg gaattgttgt 1140
   ttagctcaga ggtacatttg gtgagtcctg gcccttctct ctagaatgac ccgagactgg 1200
   ttgttttttc taaatatagg cagagagggt tctttttatg ataggaactt catgaactta 1260
   atagcaagag tcctgaccct ccacttgaga gacttgtgga cttaggctat tgctgttgag 1320
   cacatgtgtg aataccagcc agtctacaaa ccgcactcct cattgagatt gttctctcta 1380
   aaggtgagat tacattttgg acctgcctct cccctgccct acaactctga aagattatag 1440
   atgataggac agacagactt ttatcctgtg gggtttgggg catttgtttg attgactcat 1500
   gacagctttg ttttttagca taaagtaaca atccctcact ttgaaaatta tttcacaatt 1560
   tccaaagtgc catttcgcca gttttcttat ttgaatcttt tagccgtgtt agattgatga 1620
   gaacgctgtc atcactcctg tttcgtagct gagaaaacct ctgaaaagtt gtggggtttg 1680
   tccaaggtca catggcaggt tggggaagga atggaaccag ggtttaacga ggttggcctc 1740
   atccggtggt tttcgcagca cgctacacag gcacagaagc agcttgcagt gagttgttaa 1800
   aattgacgtg gtgataggta gaacttgtcc agggctgctt attttccagt tggctttatt 1860
   tctgatcctg tcattttagt aactttcctt taactttaga ggactcgtga gtggagcttt 1920
   agaaaacaga atgcttttca tttcaaaggc atatcaactt ccaaagtcat tttcagttgt 1980
   aaattacatt gtgaaaactt c 2001
<210> 158
   <211> 2001
   <212> DNA
   <213> Homo sapiens
<400> 158
   gctggtctca agctcctggc ctcaagcaat cctcccatct tggcctctca aaatgctagg      60
   actacaggca tgagccactg cacctggcct ggacctgtca ttttacagat gaggaaactg     120
   aggcccccag gggacaagac agcctgaagt cactgggatc tgacaactga gaagggtctc     180
   agcccagcct gcccctcccc tgccgacccc acaatcaccc tcccatcctt tagcctcctc     240
   cttccctctc tgtctctcaa tgtccccttg cctcttctgc tcctcaccca catcccccac     300
   acatcctccc caacccggta ggctgagggg aggggagaga aagtggaggg ggtgctagaa     360
   ggcacatact acaagcaaga cagccactca gcttatctca agcgacccca cagcatcccc     420
   agggcatagg cctcctccca gatgaataag ctgaggttca gagaggtgct gtgacttgcc     480
   caaggccaca cagcggcacc aagccagtga cgacagccca ggtcagcctg gggacaaagg     540
   ctgaatttca ggtttcccac ttccctcctg catccacaga gtaaagactg aggcttgcct     600
   ccgtgggaga cctgccccca gcttttgggg tgcaggtgag gagccctggg cactctcacc     660
   catagtcctg caccctccac cgagatggct tctggggtgg ggtggggctg aagagggtga     720
   agctcccctc agcccacccc cgggtgctcc ctgtgggccc tcggagaagg aagcgtcccc     780
   tccaagctgc cttccaggga cgcctagttc tgcagggcca ggggcttccc caggggcacc     840
   agggcggaag gatctcaggg catcccagtt gggggctgtg aggaagtcca gccaggtacc     900
   tacccatctc ccaccaccac acacttgatg gcctgcatcg tgtccggagc ctggagagtg     960
   tcggtggtga cagctcaggg ccaggcgcgt ttctgcgggc gcaaggggtg tggaggctgg    1020
   tgaggcgcct gctgaggagc agcggtggtg gggcaggagg aaacggaagg ggaacttact    1080
   caagctgctc tccccacggt cgtttgcagg gggcggagca tgcggtctgg cagagcagct 1140
   ggtgggaggg gcgctcaggt gctgggctga gccctggcca tccccacagg ccccctgcgt 1200
   ggaggctcca gcaggagacc tgggggccct ctgtgctggg aggctcactt cctgtccacg 1260
   gggcgggccc agaggctgtt ctccaagaac ttgagggttc caagaattca gtgcacttca 1320
   attctaagca gagcatttaa tgagtgcctg ctgtgtgcca ggcacagtac caggtgctgg 1380
   tgatcccagg atgcaagaga cagctggtcc ctggagttta taccctttgc agagagggag 1440
   gcatgacttc atcatgggag atcataacag agacaggaag tgattgaggc aacgggggaa 1500
   tctacagaga ggacctcagg gttaccgaga tggagagctg ggggcctgca agctagcatt 1560
   ggagctggct cttgaaacgt gcgccctaaa gagggaactc caggtggggg gagattgctt 1620
   gagccaggac ccagaggctc ccccaggcaa gggcctgtgt gggagtccct gggtgggagg 1680
   atgtggggcg atgaggcagg aagggaaggt ggggtccgct gatggagaag ggcacattga 1740
   ggggaagtgg cgggcagtgc cccacaggag ccaagcctat tctgaggccg ccctggggga 1800
   ccaggcaccc tggagctaga actgtggccc atttccctga tgcgaaaact gagggtctga 1860
   gaagtcattt gaccacctag cctgaaacca gccctaggct caggtgactc caagctcttc 1920
   tccaagctgc agctattggc tccggctgct gtttgaaggg acaaaacctg agttctgcag 1980
   cttgcagggc gctcccttcg c 2001
<210> 159
   <211> 2001
   <212> DNA
   <213> Homo sapiens
<400> 159
   ggggtctact gctaaaagtg atgtgaaagc cgccatgttg taggcttcca aggcctaaat      60
   cccgtccctg ggaagccagt gaccaaagat gaaatttagc tttgaaacca aatgcagagg     120
   aaagtgatga aaaaaccaca ggctggtgat gcaggtcaga ggaggagatg ctctttcaca     180
   ttcggacttg agagagcccg agagtaccca taggcatggg tcaagagagc tacagtggag     240
   ctcttggggg acagaaagga ggatgtagaa aagaaagggg cagagactgt gcctctagcc     300
   acccatggat acataaggca ttgaatccat tagtgaagag aaataagtaa tgaagcatct     360
   ggggccccgt gtggctccta ttataatgaa tcatgtatgt tctcatatta gttattaagt     420
   acacctggct tggtatatcc gatggcctat tttgattaat ttatctaatt cttttttttt     480
   tttatttcct aggccttggg ttaaaagact cagctatcaa cctggaggat aactgattac     540
   ctagtcagag ttctggtacc tggagacaaa ctaactttga ttaaagcatc agatcaccag     600
   gtcttaaaca ctgggcacca aaccggtcct gggacagggg ctaaaccttg tcagttatga     660
   tcagggagca gtcctagaag aaaatagcac ccaaaggaga acttgctctt ttgggaatga     720
   gaaagagcta atgttttgca ctatggattc agtcaagaag agaaatgctt ttggggcttt     780
   cttaaaaggc caaatgtaaa aagtaccccc atcatttcct aaaactcaaa cctctaggac      840
   tctacctctt gggctaattg ctcatgagcc accaatctgt tctgtcttgc actgtgtgtt      900
   ctgagagcca gcaggttagg acagagggaa catgacgggg tcagaggagg ggctcacctg      960
   actgcacagc tccatgtttt ctgagtacac agcaatctgt cgtctcggct gctgctcctc     1020
   tgacagatag ctggagaaca ggatgaggac atcaaaacca aacttgtctg taaatgcttt     1080
   caagtcactg gtaatattgc tgtgaaatag acaattctga aacgacaaca ggaaatttga     1140
   tattaatgtc aaactgccca gctgcgggga gttgttccac aaatttgtcc agtcttatga     1200
   tgtatttagg agagatattt attatttaag gcaagtgaaa tacagtttgc tttgagaagg     1260
   agtggaaact ttcatgctat ggcttaaaat acactttgct tgtttgtttt cccaaaccat     1320
   gtaagaaagg agtttttgcc attattaata atagtttaga caaagaacag atatcctaaa     1380
   catttttttc taaaatattt attcagatgc ttaatttgca aatcctattt tccctcttgt     1440
   cgcttatagg atctatatac gaatgtctgc tattgttaca atttatcaca ataagaatag     1500
   gaggaagtta tttcttcctg tatttttttt ctaatgatta ttattaactc caaggagaaa     1560
   ttcctacgaa tcaacatagc tgagtttact gggtgatctt tattccagtg tagaattctg     1620
   ggagattttt aaaagagctg tgatgtcaga tttgcttgtt ctatgtggca gctgtggagg     1680
   aatgaataat tgcagaaagg gatcccaata cctcagatta cccagatgtg gctcatatca     1740
   tttgcaaaaa tgttctggct gcttcaagtt ttatttactg tatgtatgta cttatttatt     1800
   tatttttgag acagggtctc acactgtcac ccaggctgga gtgctgtggc gttatctcgg     1860
   ctcactgcaa cctatgcctc ctgggctcaa gcaatcctcc cagttcagcc tcccaagtag     1920
   ctggggctac aggtgtgtat caccatgccc agctaattta ttgtattttt ttgtaggcgt     1980
   caggttctgc cattttgccc a                                               2001
<210> 160
   <211> 2001
   <212> DNA
   <213> Homo sapiens
<400> 160
   atcggcctcg gagacaggca gctctgagtg agtcaggacc ctgcggctct ctcacaactc 60
   ctgaaacaca gagattcctt gtctttcctt tttgctttct cccctaaaaa ttagtaacta 120
   cattttaata agtggatgaa atgaattgat tgaaaatatg ttagtaaaaa catggtacac 180
   ataattgatt tttaaaatca aataatacaa aagagccttt cctatccaga ggcatctatt 240
   gatctattgt gaacactttt ttaggtatcc tttcagaatt tttctctctg gatctaagca 300
   cgtttttttg gtttgttttt gtttgtttgt ttgttttgtt ttttttttgt tttttttttg 360
   tttttttttt ttggagacag ggtcttgctc tgttacccag gctggagtgc agtggcacaa 420
   tctcaactca ctgagacccc cgccctcgcg gggctcaagt gatcctccca cctcagcctc 480
   ctaagtagct gggccacagg catgtgtcca gctaatattt tgtatttttt tagagaccag 540
   gtgtcgccat gttggccagg ctggtctcag actcctggac tcaagtgatc tgccttcctt 600
   ggcctcccaa agtgtcagga ttacaggcat gagccactgc actcggccac atttttaaca 660
   caaacaggaa ctgactctcc acctttgccc agcgtgctct tttaaaaact aaagactaca 720
   tctcaggaat tgttttataa caacatatca gatgagacag cgatgattct atccattttc 780
   cagaggaaga aactaagact caacatttcc ggtgcctatc agaggttact ccagtgtccc 840
   tggtatagct ggtatttaac ccactcccag gaccctaagc ctccgccctc ggcatccgca 900
   gcatccccca accccccaac ctcctgcagt ggactggctg gtagtgggca agagactaat 960
   ctcattccag gaaaacgggc ccctagattt tgtgttctcc gtgtttcttt tccagggagg 1020
   cattttgatg acccccactg gcccttccag gagcctctgt ctgaagggtg gacttggttt 1080
   atcaccccga gaaatgggag cgcctggctg cttggattta gcctgcagct ggggtccccg 1140
   gtggtgtgtg cagggcggcc ttgagcctcc aagggtgcat ccacaaactg cccttccccg 1200
   agtgacccat gggagctcga tggcgtacgt gctgagcacc aggatgtgtc ctgccctgtg 1260
   actcccaggc cccgcccttg cgctcagcac gccctttgtg gatggatttg agggcccctg 1320
   gcctctgatg tgccactccc gctgaggcca gcgaaggagg aagaaggaaa gagcgttgcc 1380
   tgccttgttt tcggggcact cactttttct gggagacagc ggacccaggt tcatcaatgg 1440
   acagcaggat tttcatgtta ttaaaaaggt cattgtaggg gaaatccctg ggggatctgg 1500
   tgagatctcc ctgggggatc tggtgagatc tccctgcctt cccactgctc ctagtaccca 1560
   gaggccaggc caggtcgtgg tgcagggctg agttccagga gggtgcaaac accccaggaa 1620
   cagactgctg cccctccggc cctcatgacc tgctggggaa ggacccccgc aaggagcttc 1680
   tgcaggtgct gtggtctcca cctgctgcac tcaaaattgc ctggagtggg gagaatatca 1740
   atgcctgggc cccgccctga caggtggttc tgtcgcctgt gattcttctc tgcggccagc 1800
   accgagaacc ctgctggggt ttgcttccgt ttaactagtg aatgttgact gagctcctac 1860
   tgcctacacc cagggtttag ggctggggaa ggaataacaa aggtgacaca ttcttgtcct 1920
   tgaagagtcc ccccgcaaag ggagacggga tcatgtcaga gttaagatgg tgaagccaag 1980
   actaggttct gtgctgcctg a 2001
<210> 161
   <211> 2001
   <212> DNA
   <213> Homo sapiens
<400> 161
   gagacagaca aatagcaagg tggtagtggg gttcagaata cacaaaggag agatttccgt 60
   ctaagttttg cctcctgatc atttcttcga ggcgttcttc tctttcgtgc ctaaattctg 120
   gtactttccc agatcttgtc agaagaggac aagggaaaat aggacaatgt ttcctgtttt 180
   ctgagacatt gtatatctcc tcattctcct tatgcatttc ttcttccttt ttctctccct 240
   tccctcctta ggcaggaaac tgccctaatc ttaaccagat gctgcgggca aggtttttta 300
   tttttcccct aacaacaaaa acccactgtg gctgccgcaa aggacaagaa gcctcagatc 360
   caaggtttgt tagaagaatg ttcagactga aaagcaatgt tctatgatga tgaaattcac 420
   agcttttcat aagtttactc tagttagctt cactcatttc ttgcattggc tttcttaatt 480
   acaaatcctc cattttaaga acaatttgca gcctgcagca tgacatatgg cttacacaat 540
   tgcttatagt ccatttacat gtaattttgc aaactggcaa aatgtccagt aggacattta 600
   aaaactagag tgttaagggc tttgagatgt ctcagtgaac aggaagcact gagctactac 660
   ctccccatac cactctcctc ctcctcacta aggagttaac cgttttgatc cttgttggcc 720
   aaagtagaaa acactgcaga aaggccactc ttgaaggatg gggaaatgat tccctatata 780
   ctttgctttt actaacaatc atgtatgtat atatataaat catacaagga attctacaga 840
   tttccaggca gattttattt agccagggaa tgacctaaga agtgcatctc tgttttcatc 900
   tgcagaggct aaaagtttat tgtgatcctc aagtcactac atccctaata ctcagagaat 960
   tgacaggaat gaaaagctaa aagatctagg aagttgcaaa cgttacctgc tgatggaaat 1020
   ctgttgtctg gcttcgtttc ggcagcaaag tgtgggcaca ccatcagggg tgctagattt 1080
   attccatcgg gattgcggaa gtcagctcca gtgatcggct ggaaaatttg ggtaagagga 1140
   ggaggcgggg atagaaatgc tgagagaagg aagggtaggg aggagggaag gaggcagtgg 1200
   atttgcagtg tttctgtgtg tatgcagcat gtgtgccagg gtagggaatt actagctatt 1260
   tagtctcaca agactgtctt tggtggcaaa taacaagacc tcaatatcta ttgactcaaa 1320
   taggagctta ctttctcttt tggtgagtaa aaggaaggac acagccctag tctctcccat 1380
   ccttcttctt caatatcttt attttaatat tgggggatta tatcttttcc tacccctttc 1440
   ctgttaggat ttacctctcc aacattgaaa cctattttca tatttgtgtc tgactctcac 1500
   ctctgggatg cggtaatgaa cataaaaggc tataaaagaa taatgtaatg gaagccatag 1560
   gctccagaat tctttgtatt tttataagct tgttggttgc aagttgtcat tacttccagg 1620
   cagaatagtt ttcccagcct aatgtggttt tgctgaagga ggatatgccc agaaagtcca 1680
   gatgaaactg tctttaaaag taaccaaaaa cagcagtcct ggttatttat gacagcactt 1740
   gaatcaatgc cgtaagttct gatggactca catgtgactc tgttgctaaa actctcaggt 1800
   ggtgggatgc ccgataaaca tcatctgtct caattaaaat ccccaatcta ggacaagtca 1860
   catgagcttc tccaaatgct tccccaacat ccaagcatgt gacagatcat aagaatctag 1920
   ctgattcctc aaaccacacc ccaaacttca ttttcttcca taaacagatg tggggaagga 1980
   gaaaaaaagg cttcttggaa t 2001
<210> 162
   <211> 2001
   <212> DNA
   <213> Homo sapiens
<400> 162
   caagggcctt cttgctgtgt catcccatgg tggaacagca aagagagggc aagagtgtgc      60
   caaaaggggg gcaaactagt ccttttatga aaaatacttt gataacaaac ccactcccat     120
   gataacaaca ttaacccttt cctgacggta gagtccttgt ggcctaatca cctcttaaag     180
   gtgccacatc ttaatactag tatgatggca attacatttc aacatgggtt tcagagggga     240
   ccaacattca aaccatagca gtctcaaaca ctgggctggc agtggaagag gaggctgatc     300
   tttaaagaat ataaattttt tttttttgag acggagtttt gctcttgttg cctaggccag     360
   agtgcaacgg tgtgatcttg ggtcattgca acctctgcct ccccggttca agcgattctc     420
   ctgcctcagc ctcctgagta gctgggatta caggcatgcg ccaccacgcc tggctaattt     480
   tgtattttta gtggagatgg ggtttctcca cgttggtcag gctggtctca gactcctgac     540
   ctcaggtgat ccgcctgcct cagcctccca aagtgctggg attacaggcg tgggccacca     600
   cgcccagcca gaatatgttc tttaaaaaca ggcacaattc catgaagtaa actcacattg     660
   gaatggttaa caggtaaagt gatccagctg gctgaaacaa taggcattca ccggcatcca     720
   aagtacataa agatgagcac tttgagactg cgccactgca ctccagcctg ggcgacagag     780
   tgagactctc tcaccaaaaa aaaaacgtga gcactttcac ctcaagagtc gctaggctca     840
   gatgctgaat gcaccctgaa ggaactctaa agataccagc ctctggagtt ttcacagcca     900
   acagcattcc taggtagact ggttgagaac acggctagtt ggagggtaaa aagaatgact     960
   tgtgaatgct cactaggtct ttgaatatgt aagtagacac gacttcaagc cagttgctca    1020
   gccttgaata tatcctagag tccatcacct tgggctgtga gtggcttgtg cggtcatctg    1080
   tccatctctc agctgggcga gaaacttgat gtcctttctg agaggtggcc ctggctctgc    1140
   ctgggacctg ctactcttct ccaaagtgca ttttttgtgg gtgggttttc actgtgggtc    1200
   ctgggcaggt gagtgctggt gcccagtgtt ggggagcagg gtagatagag gtgtggctgc    1260
   tacctggctg ggcccggact tggcagcctt ccttcatggc cctcagccac agcctgttcc    1320
   atagtttgtt ctcgatgtgt cagctttgtg ttcccatgag aacaatgggc tccttgagga    1380
   caggcattgc atcgtacctg tggtctcttc cctaacgccc cccagtacgc ccagcataga    1440
   cagcacaggg tgcaagcagt cagatgagca gggctcccgt tcccagcacg ggacccagcc    1500
   cgttacctcg ctgctatgtt tcccacgtaa acggagcaat acccaattct aaagaactgt    1560
   tctgaggact aaatggaata gtgtaaaaaa taaattgaca tgaagtgaag gtcaattaaa    1620
   atgcactagc ttggccaggc gcagtgtggc tcatgcctgt aatcccagcg ctttgggagg    1680
   ccgaggaggg aggatcgctg gagcccagga gtttaagacc agccagacaa cacagcaaga    1740
   tcccggctct agcaaaacat taataaaaat tagccgggcg tggcggtgcc caccggtggt    1800
   cccaccttct cgagaggctg aggcgggagg atcgctcgag tcctggacgt cgaggctgca    1860
   atgagccgat ttcgcgccat tgcgctccag cctgggcgac agcgcgagac tccttctcaa 1920
   atatagaatt ttaaaaaggc catctcgact gccgcggggg tcgcgtcctc tccatccccg 1980
   accttggacg caaccccggg c 2001
<210> 163
   <211> 2001
   <212> DNA
   <213> Homo sapiens
<400> 163
   agccttccca ggaggggtct gataccaaag gccaagttaa gaaaatgtct gaggccgggc 60
   acagtggctc acgcctgtaa tcccagcact ttgggaagcc aaggcaggtg gatcacctga 120
   ggtcaggagt tcgagaccag cctggccaat atggaaaaac cccgtctcta ctaaaaatac 180
   aaaaatcagc tgggcatggt ggtggacacc tgtaatccca gctacttggg agactgaggc 240
   aggagaatgg tttgaaccca ggaggcagaa gttgcagtga gccgacatca tgccattgca 300
   ctcagcctgg gcagcaagag cgaaactcca tctcaaaaaa aagaaaaaga aagaaaaaaa 360
   gagaaaacgt ctgagaaccg acggcttgta tttaaaacgt gtgagtgggt ggtggcagga 420
   gaggtagaca gggagcaggg ccggcttggc ggaagctttg ctgtagagag gggcagagat 480
   ggctcggtgg gtggagggag gggcaaacgg tgtttaagaa gaaaaaacga cagcttctgc 540
   gtaggagagg ggcctcctgt ccatctccag ggagaacatg ggttctgtgg ccacaggtct 600
   ccccagggcc tggcatcaag gaggcgccag gcacgacctg cgggatgaaa gcacgtggct 660
   gaagtcctgg tggctgtgtc tgggtgaggg gtgtgagggc gtggcctgag caggtccact 720
   ctcaggggag aggggccgag gccaggaact gggtctgggt gtccacctgg tgcctgggtg 780
   aggtcctgca ggcagcaggt ctgggcttca gtggatgcag ccagtaaggg atgggctgat 840
   cagggaaaac cgtattttca ggggaggagc ccaggaggca gggagctggg cggcggtgga 900
   ggggcctgga gggcgattgt ccagatcaag cccctcgagt aaggcccgag aggctgtggc 960
   ttcagaaatg aaggtcatgg gcgactcggg ccaatgccgc gtccttgacc tgggcgaagt 1020
   gagcggttgc actcacgagt gtggacggcc cttccctaca ggggaccttg gattcgctgg 1080
   gggcggagtg ggggctgggg cccagagaac ccgagtccgg acgctcccgt ccgtgaccct 1140
   ggccgggttc tccgctcctc gcagcttcag tccgcaagtg tgagatgtca ggggacccct 1200
   ggacggcggg gccctcgggc tggcagcgcg cgccgcagga gtttcgattc ggagggtctg 1260
   ggcgaaggcc gagaactcgc gtttccagcg agttcccggg ggcgccgctc cgtcccagag 1320
   gccggggccc ctccggttgc cgggcagagc cgcgccgcgc gcggtcccat tggcgcaggt 1380
   gcgccgcgcc ccgcccaaca ggtagccccg cccgacaggt agccccgccc gcgcctcgcc 1440
   attgggccgc ggccgggtgt cccccgagga ttggcccagg tggcggcccc gcctcgccct 1500
   cattggccgc aggtgccacc gcccgccccg ggaccgcccg ccccgcggat tggccgctgg 1560
   ctccacctcg gtccgccatg ttcggacacc gccccccgcc cccgccggac ggtcccgccc 1620
   cgcgcgcccc ccgctcccgc cccgcgcgcc gccggagttc cgcggagttg accgggtcgg 1680
   cgccgtcggt cctgagcgct gccttccgcg ttccgccgcg gccccacctg gagcccccgc 1740
   cccgcgccat ggccggagct cccggggcgc agcgctgacg gcggcggggg gagcgcgcca 1800
   tgcccagcag gaccggcccc aagatggaag ggagcggcgg ccgcgtccgc ctcaaggcgc 1860
   attacggggg gtgagcggcg gagagggcgg ggagcggcgc gggtgaggca gggagggcgg 1920
   ggagggctca gccgtcgggg ctcctgcgcg agagggagag agggaagggg cggcgaggtc 1980
   gctgcgggcc cggggctgtc g 2001
<210> 164
   <211> 2001
   <212> DNA
   <213> Homo sapiens
<400> 164
   cacacactga caatttggtc caaatgaatt ctaggaaatc tgattaaact cagagggtaa 60
   tgaaaatatt tgtagtattt atattgtgct tagtatttgc caggtactgt cctgaatcat 120
   ttgcatggtt taatctctta gtcctcccaa cagtctattg ggaggtatta ctaacagtct 180
   gtagggaagt actattacta gccccatccc aggattacag acagagaaac tgagacacat 240
   agcagtttca aagcttgtct gaagttactc aattataata tgtcaggttt gtgattggaa 300
   cctagcacta taacttttga gatgatgtgt ttactacagt gcattcctac ttctgtaaat 360
   ctcttctcct tcctttctct gagcaaacac ataaaactgg gaggtaggta ggagaattgg 420
   gatgacattc atttgtggaa gtccatatac tgtgtcatat tcttcatctt cagcactttt 480
   aatctctaaa acatagtgca gtatttagca ggtcatcaat aatttttgtt gttgggtaaa 540
   tggctgatcg aatgaattaa caaaaggact ggatactcca tttctgatca ccatagagat 600
   aaccaaatta cgtttctcaa actctgtcat gctgagggct gcagtgacaa cattttagga 660
   ctagaacaca ttacttggat ttcacaatac aaagatgatt gcagttgtcc ctggtaagct 720
   cagcccacag agcctttcaa ttcagcttgg ggtgagctgc acttcaggca tggggatata 780
   atagtgtgat gaaccattct tctgaaggtt cttctgaaaa gctgctggtc ttgggatact 840
   gtgagagccc ctctcattgc tctttagaag taatttaaat ttttctgcta cattgtctgc 900
   tcagctcgta ttctggtcat agaggaacct gaagccagag aaactagaca aaaaggtaag 960
   agaaaaccaa gcaacaaatg cagtctcagc tgttgctaac gttattcaca ggagtgacaa 1020
   actgaatgaa tcttagggtg agagactggt ctttttacct catttacact tttccttggt 1080
   ctgcactgta gatcagcaga actcatttat cctaagaaat cgatgactaa ttttctgtga 1140
   agcagaatca agaaatctgg tctacattgg ttaaaaataa gtacttggtt caccttgtgc 1200
   tgtgaagtcc aagccaaata attgttgcaa gttgttgaag catttgagtg aagaaaaact 1260
   gaccaagttc actatgactg catgaaatga aatgctgaac tttttggggt tgagggggtt 1320
   ggaaggatgg atggaggaag acagttgttt ccagtctctg aaattcaatt ctgcagtaag 1380
   tttctgtatc accaagtgtc actttggcat acctcgtgca tatttatagt ctaaggcttt 1440
   tcatatagga ttcaaacata gattaaagta gcagggaact tgagagctcc agggaagaat 1500
   atgatacttt aattgcattt gatcatttta ttgaatttgc ctaggaaaat caattaaaac 1560
   acaaaactta atttttgtac ttacagcaga gattatacat tccatcttgt ccctgactat 1620
   agatttttgc cttttgcttt tcattaaata atcaacttca aagtactcag aaggccgatg 1680
   gttctattct gctgggtatg ggcgaactgg aacagggcct tcattccaca aacattttac 1740
   ttgtttcttt ttcttcatct ggcttctcta tgggacatgt tcaaattcta tacataccaa 1800
   agcagtgact tattagacgt atgtaatgtt ttaaaaagat aatagtgtat ttaaattgca 1860
   aatggaatta ctgcaataat atgaaatatg catgattata tggaacagtt gaagagttcc 1920
   caagtgaata ttaattgcct tgtatccttc cttgaggcaa aggcatataa aaataacatg 1980
   aattcacaag tagttagagc c 2001
<210> 165
   <211> 2001
   <212> DNA
   <213> Homo sapiens
<400> 165
   ggagaaacaa acgagactgc ctcccagtaa gcaaaagtga tcacattttc acaaatgaaa 60
   atggtaaaaa gaagtttaaa ttgttaaaga ggaaagaatg aggagccaaa gacaaagaaa 120
   aatcaaagct atcagagttg ctgcagaggt tttactaaac aaagaatttt atgaagcagc 180
   tgaagttcag tacaaaaaga ctccacccta cccagcttct gccgcgactt ttccttgctc 240
   tgaaaactcc agcccacaga acagagctcc tctaactaaa atgcctttag tgctccgttt 300
   taagcattca ctcttttctt attttaaacg tcccactctc acactcagcc aaaaggactt 360
   attgtaggct ttcccagaaa aatttagtag tgtactctct ccaacggtgg atctagaagc 420
   tccggattta caaacatccc aattgtcttt caagtcgttt tccaggtgca aatccgtcag 480
   cccccttctc aaacttcaaa ccagaacctc ctcttcacgc tcttcctgag gcttaacatt 540
   ccacctattt cccaaaccgt gcaataaaca ctacaagcaa agcaaatggc aaactcacca 600
   tcctcgtctt cttcctgggc ttttattgag acaaattgcc ctaataaaac aataagaatg 660
   aggagaggtc ttgcttccgc ccagtttgcc atcatgtcta aatattagac atgtgggttc 720
   tcctgagagt gaaaagtaga ttctgaggtt attgtagcac catgaagtca gctgtgggct 780
   cttctttcag caccagcccc agggcagcct ctgcaaaccc cctttttcag caccagctcc 840
   agcacagtct gcgaaaactt ttttcaagcg atgcagcttt aaataggaag aatgctgcct 900
   ccacttcttt tcctgcccct tttcagcttg ttcaggctca taaccatcca gtgtctgcca 960
   agcaacggtc tgattgatgg taaacaacca aatcagaaca cgcgtctctg tgcctgaact 1020
   ttccctattt catttaactt ttaagcatag atggggctta ataacagaaa caaacatttg 1080
   tttctctttt cttttatttt ttaagaaagg ctgttagaag aaaagggttc tattttcccc 1140
   tccctaaccc ttccccccag acacacaccc tttaactttt cccctataag ctgaaaaaga 1200
   cattccgaag gaaatccagc cttttaccct agttttttta catttagata attcaagact 1260
   ctcttctcca ccttaaatct ttaaacaatc acattttaac cctttctttt gactgaatag 1320
   ggagtattct tatccccttc tcagattcgg tttcttttcc catactctgg aaggagatta 1380
   ttagaatgtt gcccaggtaa agtccaggtt agaaaaccat tttggataaa tgaatttgtg 1440
   atacattgta atttgaatta agaaagcatg tcctttccta atttctccac atctcttgtt 1500
   aaggggccac atcttcaggt tcctcttgtc ccagagagtt ttaccatgtt gaaaaatagt 1560
   ctactaagca gtgcagagga atgggaagta ctctaccttt ggatgaaaaa aggcttgctt 1620
   ctgccttttg tgagtggttc tgccactcaa taattttgta aatgtgcata tagtccttta 1680
   tctctgtgca ctccaatttt ctcatttgag aaaagggctt taccctgacc agtgtacagg 1740
   gtttagtgac actaaaatga gtaatgtatt attaatgaag ctccaaattg caaagagcta 1800
   cataaaagca gatggtggta ttattagctt taagccagct ttgcagggaa tatgatagcc 1860
   ccagttgaac atgcatttta aggaaaatta tcgatgaact tttgtgccac cctcacactt 1920
   gccaagatct taatgacctc agagagttct atacaatcta atgtatttgc caccttgagt 1980
   ctccctagaa tcaggatgga t 2001
<210> 166
   <211> 2001
   <212> DNA
   <213> Homo sapiens
<400> 166
   gaagtataca agcgagggat ctaggttgcg tgctccctat aagaatctaa tgcctgatgt 60
   tctgaggtgg aacagtttca ccctgaaacc atccctcccc atcccctcag tccgtggaaa 120
   aattgtcttc catgaaactg atccctgagg ccaaaaatgt tggggactgc tgttatacag 180
   agctgtagga gagaagaata atgcagaggg aaaaggagtg cctgtctgtt gagactttca 240
   gggggaccta gggactggtt ttgttacagt tgactgggaa ttgtcttgtt tattttattt 300
   tatttttcat ttaaaaaatt tttttgaaat ggagtcttgc tctgttgccc aggctggaga 360
   gcagtggtgc catcttggct cactgcaacc tctgcctccc agataaagtg aagctcctgc 420
   ctcagcctcc tgaatagctg ggattacagg catgcactac cacacctggc taatttttgt 480
   atttttagta gagatgtgtt tcatcatgtc agccaggctg gtcttgaact cctgacctca 540
   agtgatccac ctgcctcagc ctcccaaaat actgagatta caggcatgag ccaccgtgcc 600
   tggccaaagt gtcttgtttt atttttaagt ctttgatcac tctcatttac agtaagtgtt 660
   tgaagagtac attctaaatc atttttctct cttcctttac aggaagtgct caaatgttga 720
   gcacacacac acaaaaaaag aggaaacagt tggataggaa ctcagaaatc atgtgactga 780
   tgactaagtt aaatcttttc tgcttactga aaaggaagag tctgatgatt agttactgat 840
   cctctttgca tttgtaaagc tttggagata ttgaatcatg ttaccatttc tgtttttttc 900
   caccctgttt tcttccatat ttactgaagc tcagaagcag tattgggtct gcaactcatc 960
   cgatgcaagt atttcataca cctactgtgg taagtaaaac cgcaaaacaa ataattgtag 1020
   catcaactat tttgagggta agttttcacg agaaccgtac actgttgtgg ctggaacaca 1080
   cgaaacatca gtgtgttcca gctgctgtgg cggacgctgc cagcaggaaa agcaatagct 1140
   ggcagctgcc ccatgagaat gttctcgacc actcacagag cttgtgagtc tctaaactgt 1200
   gttgtgcttg acctccaagt gcttctgttc cctgcgtcat ctttctctac cctgagccca 1260
   gtttttggtt ctgcccattt ttaataaaac catctgcttc tgagctttca cctcagcctt 1320
   gacacttact agttcttctt cttgagatgg tagagcagtc ccagctgctt cctggagttg 1380
   atttctgctc taagctgtga ctcttgagtg ctcttggaat catccccctc actggatgaa 1440
   agcctggttc tgtcactagc tgtgtgacct taagcaaagt actccagcct ttttagcact 1500
   gaagtgaaag tgattttata gtctacttga taagggtgtt gtgaggtgaa gttttttagc 1560
   atagggtcac atagagacaa taatactctt aatttttttt tggagtcagc cataccctaa 1620
   tactattcac tggctgtgtc actgtcccct gccccagctg ctcattagca agtgtcctgg 1680
   gatctgaagg cacagtgtca ggcctgacag ctgttttggt tcggtacttt ccaccgtgct 1740
   gcttaccatg ggttggctgc ccttttccac ccactagcaa tagctgggag gtttaaagtg 1800
   gtgcttccca aaataccagt tctttgagat tcttttggga aaataggatc tatgacctaa 1860
   tcatctggga gagatttaca gatgtacttt gggttctgag aagttctgca ataaaggcac 1920
   ctgtttaacc acattttcaa tttcatttgt tgtttgtttg tttgtgtttt agtctggagt 1980
   gtatgttaac tctcttgaga a 2001
<210> 167
   <211> 2001
   <212> DNA
   <213> Homo sapiens
<400> 167
   tggccgccat tcccgtggct tccgtgggtt gacagcaggg attctgacaa gatgaggttg        60
   ggggtagggg gtaggtatgg gaatgtgagt ggggtggcgg tggagatgag gagacaggtg       120
   tgggtgggga accctcagtt ccctcccagg gtcctgaccc taatgccggg cggatgcctg       180
   gaccttgatg cctgggccct gccctctcct gaccaaccct gccccggtca cataagctat       240
   gactacagat tgccctgtgg gttaagtggc agtgggacgg gacggggggt ggtccagctg        300
   agacatctgt cctggccggg tggtccaggg ctggcagcag gggtggtact ggatcatttt        360
   ggggccatct acctggggtg tgtgcctcct cagtcctccc tcagcatctc accttgcctc 420
   ctcacagagc ctaaaaccac tcccctcagt ggatctcaag ccgcccctca gaaccattaa 480
   atgaacctgg cttctctcag atctgtgagg ttcaagtcag tggtttcaca tcgggccatc 540
   cagggcttcc ctgggttgag agcaggggca aaccggattc tgccagaatg ggggaggcga 600
   tgggagtggg gcacatgaga gagatgaggg tggggttggg ggcagtgagg gtgaggtggc 660
   agaagggttg gggctggtgc tggggctgga gggtggtggt caggtgacgg agtaggagta 720
   gggatggggg tggaggtgta gctgaggaga tgggggttgg gtggggaatg ggaatgcgaa 780
   tggggtggga gtggggattg gagtgcagtc ttgggggccc acagccctcc ctcttgggtg 840
   ctgatcttga tgcctggcag agcctgtgcc ctgccctctc ctaaccagtc ctgccctggt 900
   cacagcaacc tctgattcca gagcttgggg agaagaacct ggtgtctctc aaggcttttg 960
   gagcaggggt ctgggggcag cccaggctga gacgtcttcc cgcgggtggg ccagggctgg 1020
   cagcaagggc ggcgctggaa tatttggggc tctctatttg gagtggcggg gtcctcagtc 1080
   ctccctcagc ctctcacctt gcctcctcac agagcctggg tcaggttcct tcggccgaac 1140
   tctacgccgt ccctcagaat ggaaacctcg cttctctctg ccccacaatg cgcaagtcag 1200
   tggcgtcaca tccgggcacc cggatgtttc ctggcgggaa ccggatcctg cctgggtggg 1260
   ggtgggagtg ggcgtggggg tgggggtggg ggtgagggtg gaatggctgg gcggatgggc 1320
   atcagggttg agggatgggg tgggggatgt ggggtggggg ttggaatgac aatgaagagg 1380
   gggttgggtc acgggagtga gcattggagt gggggttggg tcacgggagt ggggtcttag 1440
   ggaccctcag ccctccttga gaatcctgag catgatgtct ggcagagcct ggacgctgcc 1500
   ctctcctaag cagcccttcc ctgatcacac gagctctgac ttcagagtcc ccagtggttt 1560
   aagcagcaag ggggctgggg gtggcccagc ctgagaagtc caccaccaga tggtccagga 1620
   ctggcatgaa gggattattt ggggtcctct atctggggta agtgggtcct cagtcctccc 1680
   tcagcatgtc accttgcctc cttacagagc ctgggcccgc ttccctccac cgatctcaag 1740
   ccgcccctca gaccgaaaac ctcgcttctc tctaaccact aatgctcaag tcagtggcgt 1800
   cacatctggg cacctgggct tccctgggtt gagagcaggg gtggaaccgg attctgcctg 1860
   gcttgggctg ggggtgagcg tgaagatgtt tggatgggtg gcgtaaaggg agggattggg 1920
   ggtggtaggg ggagggatgc cagtggtgcc ccggggctta agtcgtcgtg ttggtggtag 1980
   tggggttggg cgtggggttg g 2001
<210> 168
   <211> 2001
   <212> DNA
   <213> Homo sapiens
<400> 168
   cagggcacag ctggcaggca gagcctgaga ccccaggagt gacaataggt gggtagggcc 60
   gggtgctgtg gggtctcctc tgtttctggg ggtagggccc ttggtacaca gccagggtgt 120
   gcactcacct tgactcctgg cactgcctgg gcctcctctg ctgtgctgac tttaggctgt 180
   gtgcctcaca cccagttcgt caccacctgg ttcctggagc acctgcaaga ggaagtgagg 240
   gagcccctca ggctaaagac tgcaagtagg gtcttgggcc tgctagggct gacagctggg 300
   gctggccaga atcccagagc ctcatagttc tggactgcat ggccccctca gaatttaatt 360
   agcaacttca cggcttctca gagagggccg agcctccacc actctgctgg cctaaggcaa 420
   aacctcagag caaccgccac atccctgaga gcagtgggag gtagggcggt ggcagccacc 480
   tcccagcact ctaccatggg ggcagtcggg gtagcctcac gtccgttctg atgcacatgt 540
   gactcctcag tcttatcccc agcagggcct gggaatcttc cctctgccaa ccagaggcag 600
   ctccccgtgc tgacttgcca tgggaccctc agaccaaggc ctccgcctcc cctacgccac 660
   tcacccagga ataagatggc tgccaccccg cctgagctga ctggggagct ctacgagggc 720
   tgatacttga ggagcagggt gggaatctct gggaatctgc cctctgaggt gggggttccc 780
   ccagccctcc tcagtgtctt cctatttatt ccctagaggg ccggcacctc ctcccttctt 840
   ctggggccat ttccctgtct gggacctacc agtctcacgg cagcgcagta caggccgggg 900
   tttcttctct ctatggtggg aggtccctca gttcttcctc agggccctca ccctgaggac 960
   tggccaggtc taagactctt ccccccgcta acttgattcg cgcctcctca caaatgcctt 1020
   cgatccgtca gattcccaag atggccgtcg ggaaagcaca tccgggaacc ctgccgtggt 1080
   tgtccagggc tgacgcagcg tcaatgtggg gcggggcctc ctctctgagg aggaaccacc 1140
   ccccacctct ctgctttttt tccatccctt cctccccacc tcccagctgc cctccaaccc 1200
   ttcctgctca ctccctaagc cttgcggacc tcttcactcc cacctcccca gtccttgaca 1260
   ccccactctc ctttttccag gggtgtcagc agggagtggc ttggtgcagt ctgcgcctcc 1320
   cctccgtgtt gggaggtcac cgtccttcca catctctctt gttttttggc cagttctggg 1380
   acttctttct ctgcttaact gaagctgccc tcctcagaga caacagcctc cccttctgct 1440
   gactctcaaa atggaagtca gaaaattgta catctgggga ccctgatggg gtcctccaga 1500
   gctggcagca gggacagggg aggggcacta ccagtcatgg gtgttccctt ccagccttcc 1560
   tgagaggctg tgcctggact cctgatactg cctgggatgc ctgaccctgt gcatctgagt 1620
   ctttcacctc cgaataagcc cctcccatcc ctgagacctg caaggtgcaa gagagggaga 1680
   atcacatcca tccacagtgg gatagggcct cctagcccta aggcaggcct ccactgccct 1740
   ctgaaatggg gtgggaatgc ccctctgtct ttctcaaggt atgactccta aaagagccta 1800
   tactccttcc ttctcctgtc cagacagcaa catttgttcc ttcgacctct aaattccatt 1860
   ccaagttttt ccactttact gctgtgagtg aaattagtgt catctctccc ataccctgtg 1920
   ttaaaggctt ccttattgtt gcttcttttc ctatcctagt ccctgaaaaa aaccactgtc 1980
   ttttcagcaa cccgtgaccc t 2001
<210> 169
   <211> 2001
   <212> DNA
   <213> Homo sapiens
<400> 169
   gctcgtaatc atcctctgaa cgcttctttt tgagagaaac cacaagaagg tattcaaaga 60
   agtgctgccc cccagcaaag ttgggcaaag agtgctcctg ggccctttct ggttccttta 120
   aagcttcccc tgaattgtcc tggggtggtc ctgaaatcaa gccagagccc atttagtata 180
   atctcattga tccattcctg gagtggagtc tgtgagaatc cgggtgacct ccacacctca 240
   aggccgcctt gccaatgctg agatacctgg atactggcca gaaccaccag tcactcagga 300
   tcagggccaa atgggcagtt tccacacctg ccctggtaga cttgaaacca tggggagcca 360
   agcagaggaa agaaaccact gacacagaag ccacggaaat gtgctggaga ggccacatgg 420
   aggccaaggc cccagggtac cacagagact gcctaacctg tcgggtgctt ctggcagtcc 480
   tggaacaata tggcctgaga gtttagttca tactaagtga ataagaacag gctcaaaggg 540
   catgtgactg tgaccacact agccccagag agggcatgac cagctcttaa aaaccagcag 600
   aggaagcctc ctgacccagt cacatgggga accatgtgga ggccgagata taaaggaaga 660
   ggaggaagga gcatttcaag gaagaaacag tcccgcctaa gagggtcacc ctaaaaacaa 720
   ggaagtaggc agtccagtcc tgacctgctc ggagttgaag cagtcggcgt tggaagagcc 780
   tgaacacccg gcctaccact tgtccttcca tctctgggcc ttcaggacag agcggactcc 840
   cctctcccct aacacagaca gactggtgac agtaagcctg agaaaggggc tgcttcggtc 900
   tccagccaca actctgtgaa gccaagccac gcgctgtctt ccagagaggg aatagaagtt 960
   tccatcctgt gcacccagtg gttgagcaag aaggatgtga cgggacccag gaaggcagca 1020
   gaagccagca ccaacagagt cagcatcctg gcactgcagc gcagatctca gctcctccca 1080
   agagccccag ggtgtggcga gagaaaaggt catgagcaaa cacctctggc tgaaagtagc 1140
   cggaaaggcc tggctactta tgggctaagg gttttgaatc tgggctgcag attgtgcaga 1200
   gagttaattt acaaccttag aacagtggac aatgcttgat agtgtcagac tgtgggcact 1260
   ggaaaccggc tggtctcagg atagggatcc tatacctcca acacttggat tgctgacctt 1320
   tcacttctca acttggtcaa cggaagtcaa aaaccccagc aaggccacct gctgaattct 1380
   agaacttcca ggacttcccg acctagagca catggccaaa cactttctgc agccaggatg 1440
   tcactgccct cccctgatta agataaatag acttggccta ataattatcc aaatagatta 1500
   attcttccaa caaaagaaag agttctggaa gtcaataccc aatagccttc tttcctgatg 1560
   gtgcagtaga gagcaaaatc catctctaag cccagagttc gtgatatttt ggaaatagct 1620
   acaatggtga atttcacccc ctggagagtt ctgtcactgg cacttgttcc agggttccag 1680
   cagagtccct cttctcaaat aggaagcact ggctcagaag actgtcccca gcataaggta 1740
   ggggactgat tgcaccagca gcctgagggt gattccatgg cagtggaaaa catctgggtg 1800
   atctccaccc aacccccacg cctgctcagc cttcctccgg gtcaggccag gtcagctgtt 1860
   ctgcccaagc tacaggcaca gctgctagcc cactgctgtg tctggagact aggtaagaga 1920
   gaaggaatag caaaaaaatc accttggatt ctcacagaaa cccctgaatg agtaaataca 1980
   gggaaggctt ttaccaggaa g 2001
<210> 170
   <211> 2001
   <212> DNA
   <213> Homo sapiens
<400> 170
   aacatttcac aggggtttgg gctgtgagaa acatcctgcc taaccacccg accacaaggc      60
   agacaaaggc ccaactaaag aaacatccct ctcatatctt cctgggcaaa ggtccaaggg     120
   acaccatgat gacatcccac tggaacaagg gccaaaccgc ctcatcatga gaacatcttt     180
   atatcaatat cctgctaggc agcaacccat actgcccaga cccctaccgc ccatacctat     240
   aaattgccca gtctgtaagc agtggtgggc actggcatta ggctggttcc ccacttctgt     300
   aggttattat gctggacata aagcctgcgt ttgctgtcga gccgccctct ttctgtgtgt     360
   gtgtgtgtgt gtgtgtgtgt ctttctttaa cccttgcctt cttttcaaaa cctaacacga     420
   gtagacaaac gttctttctg aagagccaga ttgtaaatat tattgtaggc tttgcaggct     480
   atagctcttc tgcccttgta gtaggaaagc agccacagtc atacttaaag gaatgagcgt     540
   ggctgtgtcc ccataaagct ttctttacgg acactgaaat tttaatttca cataattttc     600
   atgtgctaag aaaatataca tatatatttt gtgagatggt gtgtcgctct cgtcaccctg     660
   gctggagtgc aacggcgcga tctctgctca ctgcaacctc cgctacctgg gttcaagcga     720
   ttctccagcc tcagcctcct gagtagctgc gattacaggc gcccgctacc acgcctggct     780
   aatttttgca tttttagtag agacggggtt tcaccatgtt ggccaggctg ctctcgaact     840
   cctgacctca ggtgatccgc ccgcctcagc ctcccaaagt gctggggcta caggcatgag     900
   ccaccacgcc cggccaagaa aatatagttt ttttattttt tccaactatt tcaaaaggta     960
   aaattcattc ttagctccca ggttgtccaa aaacagacgc gcaggatggc caagcaccag    1020
   agtttatcgt ttgcccataa taatcgggtt gattcggcca aggtttccct cccccaacac    1080
   ggtgcgcact gggaagcgcg aggaccacgt cccttggttt accactgcgc acctcacaag    1140
   gcgcccagtg catccgggta gcgggatgcc ggttggacca acgcgagggt gggcggggtc    1200
   taagagctaa gcgttggggg cggggcggga cagtggggaa gaggacgggt cgagtgctgg    1260
   cttccggcgg aaaagcgcgc gagcaagatg gccaccacca agcgcgtctt gtacgtgggt    1320
   gagcaggagg ggttgctagg cggagtctga gtgaacgcga cccccaaggg tcggggcgtg    1380
   gggtgggacg catctctgaa ccaggaggac ggcgagctgc tgtcaaggcc gggtctctgg 1440
   cggtagtgct ggcgatagct ctggctgtgc ttaaactcct tccaaggttt cccactgtcc 1500
   tcaggaaaaa gtgcagactt cttagcagta cgcacttctc cagcttcttt gctttgcttc 1560
   ccgattcgct gggtctcccg aaaaatgacc cacttgcccc caaggttcag gttatttcac 1620
   acctttgggc ctctgctttt gcgactccct tgcagtgaac agccttgcca cacacctcag 1680
   gtgcgtgaac ccgagacgga atgattattc tgacttccta gcccgctgag ctccagcggg 1740
   ccccgaggag cggtaaagga gtattatggc ccttcggatc ctctcgttca tttgttttac 1800
   aaacattcag tgcagcgacg gctcagcccc agcccctgcc ataaaggaag gagttcccag 1860
   actagccaga aaaaaagtgg aattacctca gtagcacact tttgataagc gctgtagtga 1920
   ggatggatca tgataatatg ggcctggctg cgggacaaat gacccatcca tctggagaag 1980
   cgagggcaaa ttctggatgc g 2001
<210> 171
   <211> 2001
   <212> DNA
   <213> Homo sapiens
<400> 171
   actttcggac gagcacacca gctgtcagtt ataaatgata attaaggcat cttaattatt      60
   aagcacagtg aaaatatgca gtgttcattt tttataaaac ttgtgtccta aggtagaaga     120
   atcctcatac caaccaaatg ctctttctct acctttgatc agcaatttgg ccttttttct     180
   ctctgaggct gataaattgc agcaaccttt gagatactag accctccacc cccaccccag     240
   gtcttccccc aggtctcctt agttataaag gtagcagaat gtgatggttg aaagctgcat     300
   ttgtaaccag cttaccttcg caacaatagc aagagtaaca aagaatccat cctaataaaa     360
   gctctgtgca cagtcagctt aagcaacgta aagtgttcct ttattctgct tagtagcttc     420
   tcccccagag gttaaccatt ttaaagctgg cactcagcct gagaacagga acaaagcatt     480
   actagaaagc agggatcttc ctccaggatc agcatctctg ctttccgtaa ttcttaagaa     540
   atatagaaac tcaatgaaaa ggaagtgata cctttggatg ggcacattcc agcacaggaa     600
   aatcgctgtc ttgtcggctg ctgtgcagaa tgagttgctt gggcaggtat tcctctattc     660
   tcagctcccg ggtctgcctt gtagccgcag ccagcctagg gagcatttac ggcatgtacg     720
   tggtgttcat ccacaataaa aggagactaa ccagctcagc ttggcgctgc tcgcctggag     780
   gcatcagatc cttggaccct ccccatctga ttggctgcta cccggtctag ccgaactcct     840
   ctgccttaac cttctgctgc gttcaataca ttttccctcg ggatacagtc agagaagagc     900
   tggttaggga atcagtggca ggttaaattg agctggcttt aaaaatcagt gacatgattt     960
   tctatgaaga tgtctaatgt ttaattttcc aaagttctac gtaatccaca aggagagacg    1020
   taataaaatg gatatgattt aacaggagcg gcttcatgat tcctccctga aaatacctgg     1080
   agtttaatgc atgcctaaca ccctactgaa gtgatcaggt ggctttttgc agcactgcgt 1140
   gtctgtataa tcaaccttaa atcaaaacag atggggatga cagaatatat taagaggctt 1200
   attaaaagtg ccaggagatg cagttgaata aggctgctga agccatctca agaaagtgtt 1260
   ataaatcatt tacacaaaat gctcttaaga cagttatggt tgattttaga tagctctgat 1320
   aataaaaaca taaagaccaa tgacagacaa aaaggtaaag cctgacttag catctctaga 1380
   agacaagtaa cagaaagaaa aatcaaatct tttactctac aaaagtagtg aaatgagaaa 1440
   agaacaaaag tacaccttca tcatctcctg tccatgtact ggattcaatt tggtcaagga 1500
   cctagaacag ggtattgaaa agtccccaat ggggttttgg tttgttttgt tctttctgtt 1560
   tttttttgtt ttttgttttt tgttgttgtt tctttgtttt gagaccgagt tttgctctgt 1620
   tgcccaggct ggagtgcagt ggcgtgatct ccacgcacta ccccctccac ctcccaggtt 1680
   caagcagttc tcctgcctca gcctcccgag cagctgggat cacaggcacc tgccaccaca 1740
   cccagctaat ttttgtattt ttagtagaga tggggtttca ctatgttggc caggctggtc 1800
   tcgaactcct gacctcaggt gatccgcctg cctcaacctc ccaaaatgct gggattacag 1860
   gcgtgagcca ccacacccag acagttttgt tttgttttgt tttgttttta aaaatgagag 1920
   gcatcaaatg gaagatagtg aattgaccac atacatttaa ccctttctcc ctcacaaagc 1980
   cccactaaaa tgacaataaa g 2001
<210> 172
   <211> 2001
   <212> DNA
   <213> Homo sapiens
<400> 172
   cctagagtaa gggacgcatt ttacactgct atccaaaact catcatagaa acatacacac        60
   acaaaaccaa agcacacata tacaactgag caaatatttc atgacataac actttctctt       120
   actaagggtg acgcgctgaa attttgtatt ctgtcctatt tcatttttta aaaatggtaa       180
   ccatgacctg ctaaattgat ttcattgtcc actaataaat tatgacctca gtttcaaaaa       240
   gattgcttta ggtaaccaat catcttctga gatttataca gattgctcat aattctctcc       300
   tattttttaa aaacatgctg cagtgaactg ctttacactc attttatgac tacttctgag       360
   accaagatcc cggattatgt aattgttatt tacttaaaat tctggtaaaa tgtagccatt       420
   atactggaaa actaaatttt aatcttggat ctgtcaccac catgatatat aaactttggg       480
   caagtccctg cacctctctg gacctcaatc tccccatcag caacctgctg atcctactcc       540
   caggagtgtg ctctaagttg aaagtagatg ccccaccccc tgagtcagcg ccggcaggac       600
   ttctcaccaa gcccttctcc cccttttccg ctccctgttc ctggttccta ggaagcagcc       660
   caaggagaag ggaaaaggca ggtctgggca ggagggagca atgaagggcg gggcagaggg       720
   agggcaggag ggaggccggc cccctagtag gaaatgagac acagtagaaa taacacttta       780
   taagcctctt cctcctccca tctcctggcc tccttccatc ctcctctgcc cagactccgc 840
   ccctcccaga cggtcctcac ttctcttttc cctagactgc agccagcgga gcccgcagcc 900
   ggcccgagcc aggaacccag gtccggagcc tcaacttcag gatgttgaca acattgctgc 960
   cgatactgct gctgtctggc tgggcctttt gtagccaaga cgcctcagat ggtgagtcgg 1020
   gggcacatct cctgcctcag gatggttctg gagaatctca gtctatctgg gcacatggca 1080
   agaccacagg agagcttatc tcacagcatc tgtgtctgca gctggctaga tctctctaca 1140
   gggcaggcag agtcttgggg actggttcgt gtcccaaagc caaggtgagt tagtacattt 1200
   aagcccctga aaagggggag atgaaagagg ctaggggaaa caggatgact ggaaacatga 1260
   gaaagaaacc agcagagagg gtaggagaat cagccccagg gagaggggag aaaggggaac 1320
   tgagggtgat ggtagatagg ggtacatcta ggggagacgg gaagaggctc agaagagaag 1380
   agaaatggag ggaatgggaa gaccctggga aaactgatgg aagaagtggg ggaagagtgg 1440
   ggcagagaga ggttagggga ggctagggaa aatggaagga gactggtcgc agctggtgga 1500
   actggggaga aagagatgct gtgcctaata gaacttatgg gcgatcaggc tactgaagtg 1560
   gccctgttta agcagaaaag ggagttatta ccctccatta taattgcaca ggggcctcct 1620
   ttcccctctc tcacaatccc cgtaacttca gtctccccct cagagaggca gcaaataata 1680
   accagtattc aatgagtgct cactatggtt aatacatgta ttgacccatt taacttgcac 1740
   aaacccctaa aggtgggtaa tattattact atctccattt tatgaggagg aaactgggtc 1800
   acagagtagt taaggaccat gtctagggtt atccataaat atacttattc acatctgcag 1860
   atacaaagca caacttctca aatgcaaaca cagacaggac ccactcacac acacagattt 1920
   acaaccccgg actcatccaa atgtgctctg ggcatcaact ctgtgccagc ctcttttctg 1980
   ggtgtaggaa gcagagatta c 2001
<210> 173
   <211> 2001
   <212> DNA
   <213> Homo sapiens
<400> 173
   cccgtccctg gggtccttga cccaggctct tggggctagc ctatcttctg aggagcacaa      60
   ggtccctggg ggctcaggga agagaaattg gagaaagggg gaggaagccc ccaagatgga     120
   tcacccattg cctggtttcg caggagactg tccgccttca gttctccagc agctcgggga     180
   tcatggccca ctgaaccccc aagcgctttc acccgaaccc aaggaggacg accaggaaag     240
   acgggaactc gcgtagacac gcccggaagc ccttgtcatg taaatagctg tcggggactg     300
   gtgtattgtc gccgccccag ccggcgggac ctggggcgaa tccacaccca ttgtctgctg     360
   cccaaggggc ctccggctgg ggggcgcggc tgcggagttc aaaaggggta tgagcaggag     420
   gggtgtactt ttagttcatt aagttttaat tacaggagtg ctacaagaac acattcttca     480
   ggtttaaaaa gatattaaaa tattacataa gagacctccc ctccctggcc cacctccagc 540
   ctcttaaaaa tttagtgtgt cgccttttag acactttctc aaagcttcac ttatttaaca 600
   ggcacttaag gagcacctac ctgtgccaga aactctccaa atattaactc aacctgacac 660
   cgactcagtg tggccgaata ttactctccc cattttacag agcgggcagc tggtcaagga 720
   agtcgcttgt tgaaagtcac acagtggtgg agcctgtgtg ccaacccagg accctgggga 780
   gctgcctccc cctctcccac gtagtcctga ttctttaagt gtccacatat tcctgtaatg 840
   cctggagttt cagtaattag cagggactta gtgtgttcag agaaaaaaaa agcttttaaa 900
   aattattgtt actgtgtttg taacagtttg gatagagaag gaaaagctgg aatttgggaa 960
   gtgaaggtgg cctcggggta gaacttacct agaccagagc gaattcatcc tgaagaactc 1020
   agagaaagcc ggtgcaggaa gtgggttccc gctctccctg cacaggcaca gtgatgctgc 1080
   cagagctctc ccagaaagac caggaggctt gttctggaga agtcaagccc agggatgtgg 1140
   ctcaggctgg tccaagctct ttggaggagt ccaagcgtgc ccagcccaga gggaggttca 1200
   gaggcactga ccgtcttctg tttgggagga gaagctcact cttggagcca cagccagcac 1260
   taggtcagga cccaggcccc ggcccaggag tggggcaata cccagcgtct accccagatg 1320
   gcaccctgct gtgaactggg cgccctcagc ccctgccttg aggaaggggc aataccacca 1380
   gcgtgtcttt tatcagggaa gatattgctg cagtttggcc gctgcaactt aagagaaaag 1440
   ctaaggggtc ccccagcatc ccttggggtg ccactgcaaa tactggctgg gcctggagat 1500
   gacctgggtc ccattcactt cctagggtga aggaggtcat cattaccacc cctgctttca 1560
   gccatttctt cattcattca atcaacaaac tggctgagct gcaaccctga gccggggaat 1620
   tcagccactc cagacacagc ccctgccctc cgggaagtct cgggagacct ggctagtctg 1680
   gctgggagaa gtcacacgtt gattgtcttg gaagtgagat ggcatttaca caatggaggc 1740
   tgcactgcca gcaggcaaaa ataaccagtt aattcagtgg cttaaagaaa ccaaacctac 1800
   ccacaacgct tgacctccca ttgatccatc tgcgacaccg gcagtggcta ccatttattg 1860
   agtgctgatg gtgtcacctg ggattgactt agtggtctct ggcgctagtt ccgaagttga 1920
   ttctgtctgg agagcttaat gcagtgttca gacctcaggg tccgaacctg agggtcaccc 1980
   aaagatgagt gggacatagc t 2001
<210> 174
   <211> 2001
   <212> DNA
   <213> Homo sapiens
<400> 174
   acacacacac acacccagca cacacctccc cacaatcctc agctgtgggg ggcggttgta 60
   ccaaaggctt caagaagcca ctgggagcac agagcactcc caggctctgc tgtccccgcc 120
   cagctggcct ctatccaggt agggacactg gccttcccca tgtgatcagt ggcagcaggt 180
   gtggctgtcg ggggccctag ccttatctcc taggatcaac tctgaaacga caaccaactg 240
   cactgctacc ctggccgcag aggtacagat gccactgttt gtgcctttca gtgcagtctc 300
   tggtctacag tgacaaaagg actcaagagg aggatgagaa aagttgccgt gtaacttttg 360
   cagggatgag cccaacagaa gcccttcacg gtctccctga cccctgccag caaaaggagc 420
   actcctttag tccaccttta gtccacctaa gacctctcaa gtagctgcat ggctacaccc 480
   ccgttcaatg tgacaaaacc accacaaatg gccaagggta tgacaattgc tagagaacta 540
   caacaggacc tgtctctcac aacaaacccc tctcctctcc aaactaacaa gagactgctt 600
   gccctcacag tgacctgatc ttcctggaga gaagcagcag caagcttctg ttcacaatta 660
   cagggcaagg aatgtcgacg agggtgagaa tcattcctcc ccaggatcga caaaaggcac 720
   agtggaattc tgcagagtcc aggaagtttg gagttgctaa gaaatttgct gctgcagaca 780
   caagccacat agggccttac cagggtcaag gcatgaatct gtgggactga aggtgagtga 840
   cagaagagag acacctgtgt ccaggacacg ctgcacagga atccctggga ctctctctct 900
   gtcatgttcc tggttatcag gacggctcac cagctttctg ctgaggccag ctggacccag 960
   gggagcagcg gggaaccaga aggagcaagg gcatggccat cgggacagac tactcacaag 1020
   acctggctga gctgatggaa ctgctccaga gcctggcgac accagcactg ttgcttgtca 1080
   ctgctgcacc ctgcacacag cgggctctgc aggagccggt agtaccggcg acgggcatac 1140
   cggctgtcca gctccccttc cagttccggg tctgtgcccc cttccccctt cctcttactc 1200
   tgcatataga ctctcaagaa gcacccatac agacgctgga tcatctcttg gaaggttctg 1260
   ggggtgctaa agaacaagac tccgcgcaac atagtgtgga cttcttctcg cagcccctga 1320
   gcaccagtgc ccatcagcaa gcccaggcga gtccatttct ccagcagctc taggctacgc 1380
   aggtagggat ccaggcggct ctccagcagg ccaaaagcgt caaggagtag caaaaggcac 1440
   tggggctcat ccgcagagtt ctcgcattgg gagatggcat tccagaactc aggggagatg 1500
   ttggcctgca gatcgttctg cagcacctcc acgaaccact cctccaggac cgagtgtagc 1560
   ccgtggcccc tcagaacctc caccgccgcc cggagctcct cttcctttgg cgggactgca 1620
   ccgctggtcc gggaagacac ctggggtgca gaaaaccgtg aggcgagggg aacacaacat 1680
   agaggtgggg agaggcgggg aagggaagaa gctgaggggg aggctgcaaa aactccgctg 1740
   ccccctgtcc gtgctgcccg gacgttgggc cgaagctatg gaagggctgt cagcggagct 1800
   gaacgaaacg aaacggaaag gtggtggaaa atggcaggac aggacagagg cggatgatgg 1860
   acagagccgt atgaacccga gggtagcggc cccaaagcgc gtacggtccc ggaaactccg 1920
   cgcggggccg cccctctctt cccaggcctc accagcccca gcgcagccgg cggcaccagg 1980
   ccggtgctca cggtgttcca g 2001
<210> 175
   <211> 2001
   <212> DNA
   <213> Homo sapiens
<400> 175
<210> 176
   <211> 2001
   <212> DNA
   <213> Homo sapiens
<400> 176
   gattctgatg ctacaaatga tagatatcgt tttagcattt tcttacgggt cctagcgatt      60
   ctattcattt ttctttcagt ctctttctct gacttgttca cattgaacaa tttccttttg     120
   ggataggttg ctatttctgt tttcgcaggt ggtttacctg tcttcccagc cagtcacagt     180
   ggtccttgtc cccatggtgg gtccggggca agagagggcc ctgggttggg ggtggggttc     240
   agttgaagat ggggtgagtt ttgaggggag cactacttga gtcccagagg cataggaaac     300
   agcagaggga ggtgggattc ccttatcctc aatgaggatg ggcatggagg gtttggggcg     360
   tggcgctggg aacggcagcc ctccccagcc cacagccgcg catgctccct gggctcccgc     420
   ctcagtgcgc atgttcactg ggcgccttct gcccggcccc ttcgcccacg tgaagaacgc     480
   cagggagctg tgaggcagtg ctgtgtggtt cctgccgtcc ggactctttt tcctctactg     540
   agattcatct ggtaggtgtg caggccagtc atcccggggg ctgaagtgtg agtgagggtg     600
   gagagggcct cgggtgggtc aggcgggtcc cgcttcctgg tctgtggcct ccgagggaga     660
   agggccacga ggtcgtcctc cttcccttca caggctgcga ggccaccggc ggcttcgtgg     720
   tcgtgaaggg gcctggacgg ggaggaaggt gggccgtgga ggggaggcgg tcaggggctc     780
   aggtgaagac ggggtgagtg ctgttggggg gatggaagtc ccgaggtgcc gggaaccccc     840
   gacgacacag ggcagattcc ctgaatgggg tccccggcgg gggcgaggcg ggcggtgaag     900
   aaggggcctg gcacctggga aggctgcggc ctggtgagcg cccccgcagc ggtgtggagt     960
   gcggagcacc cgagtgagaa gcactgcaag gtctcacctc cgccatggaa ggtccgaaaa    1020
   cagtgggaag gagtgggcga ggcagtgcgg tccaaccaaa cttgttgtga gggggggtga    1080
   atggctctag gaagtgggag tgtgcccaaa gcagcaatca cgagaattgt gattcactag    1140
   ggttttcgtg gggagtgcac ttgtgaaact aaacctcatc agaaatgacc tctgtctgcg    1200
   gggcgcagtg gcgctcgcct acgtagtccc agttactggg gacactgagg tgggaggatc    1260
   ccttgagcgg gaggtggagg ctgcagtgag ctgtgatcac gccgctgcac tccagcctga    1320
   gcaacacagc gagaccgcgt gtccaaaaga aatttagaaa aaaatgtcct ctgccttttg    1380
   ccacacgcct taagatgatt gctctgccag cctggccagc agaagtggct ttgtaggcac    1440
   tcagacagcg tacacacgta tgcttaactc tgggacttat cttgagagta ttttcaaaag    1500
   taaaacggca agtttacatt tatccatgga agtgatcgaa tatagcagcc ctgtggagcg    1560
   cacgttccca atcacggttg tctgttttca gtgtgaaata tgagttggcg aggaagatcg      1620
   acctatcggc ctagaccaag acgctacgta gagcctcctg aaatgattgg gcctatgcgg      1680
   gtgagtgctt aaacgttaat tcgatgtttt ctattagtag aaattaattt ttgtgatagc      1740
   gtcgttgcat tagtgtggaa atgctgataa aggtctttcc tgctcataaa aaatgatgat      1800
   ggcatctcat gaaggaaaca ttgattctgg aggatttttt ttttcctctc gtgttcttca      1860
   gcttttgccc atgacttctt tctccggctt tgtttgttaa tgacagattg tacacatgta      1920
   ttccaacaca gagtacaata gcctccaaag tcctcgtgcg tcacttttct cacagtaacc      1980
   tccctgtggg tggagtaacc t                                                2001
<210> 177
   <211> 2001
   <212> DNA
   <213> Homo sapiens
<400> 177
   taaatttgat caaattctct ctcgtgcagt tcaaagacat ctgccagctg ggtgcagtcc      60
   acaccgaaac gtacatcgat acctggcaaa gatagtttgg gggcgcaaaa tatagacagg     120
   aggatgcctc aagacatgga acagaaaaac tcttcctaac ttaaaccgtg cagaccagcc     180
   caagtctggg cacaaagccg gctttatgtg aatatccgtg ggacgatcaa gaacaatctc     240
   cacgtgggtt acatccccac gcccccaccc tttccacttt gggacgcgcc ggccgctggg     300
   cggggtttcg cagaggggcg ggctcgctac ctcgctcgcg caggcactgc agattctccc     360
   aggccagtgg atcccgagcc aactcggccg ggcgctggag gcaggtggcg gtaagttgag     420
   tgctctgatc cagggtttcg ctcagagcgg cggcgaagga gaaattcccc tccccaacca     480
   acaggaggcg ccgaggggcc atggcctcca cggactcccg gctcgcgttc tctgtggcgc     540
   tcgttttacg tcacttcctt cacggatctc tctccgcccc gtcttctccc gtcactagtt     600
   gatgacgcac ccatggcgcc tggttgtcta gacgagccgg tgtaccgact ccccggtgga     660
   tgataactat gagtgcaaaa gcaagacgcg cactttcctg tcacctcgcc ctttcacccc     720
   ctcgggaaga tcaaagtgtc tttagcccaa gcatggcctt gagcgacttc atcacacctg     780
   catgaggtca caagcagtca ctagcgcagg ggggtgaggc gagttctggg tgaaagaaac     840
   ggggcggggc tgaggccaag gaagaggttg gactgtgtct cgcatgctcc tgacgcagaa     900
   ggttttgaac cttttttcac ctcgtctgaa atggctgcct cccagtgtct ctgctgctca     960
   aaatttctct tccaggtgct taaatctcct atcttctatt cgttcaagaa agagtattga    1020
   gtgtctattg cgtgccaggc cactgtgagg gtggttacga tatgtggatg aacaaaatgt    1080
   ggcctatgat ttcaatgagc ctgcagtctg gtggggaatg cacagagcaa aacaggcaac    1140
   tctgatatgg cagggtgtgt gctctgggag cacacagaag aacgttacca agtttggggg    1200
   cgaagagggt cagagagtcc ttggatgaag taaacccgag ttgaaagcta acagggtaaa    1260
   agtgcatctt aactgagtca agaggcgtga agaggcagaa ggaacaatat atgcaaaagc 1320
   ttgatggaga aacagaatag ttgagtttgg ctgaaacaca attggaggga gagtaacaga 1380
   tgagactgaa gagttaggca ggggccaggt catagggagc gttgtaagcc tttaaaaaaa 1440
   atcaactttt attttacaaa tgggggtaca ggtacaggtt tgttacgtgg atatattgca 1500
   cccaggtagg ttgcaggttt gctacatggg tatattgcac ctaatagttt ttcaacccac 1560
   accacccgcc ctccccactc tagttagtag cctggagtgt gtgttgtttc catgtttagg 1620
   tccatgtttg ctcaatgttt agctcccact tataagtgag aatgtcagag gtatttatac 1680
   cagagcaact ccgtcttgaa taggggctag gtaaataagg ctaagacctg ggctgcattc 1740
   ccagtaagtt aaggcattct tagtcacagg atgagatagc tcagcacaag atacaggtca 1800
   taaagacctt gctgataaaa cgggttgcag taaagaagcc agctaaaatc caccaaaacc 1860
   gagatggtga tgagagtgat ctctggtcat cctcattgct acactcccac cagcgccatg 1920
   acagtttaca aatgccatgg caaagtcagg aagttaccgt atatggtcta aaaagtggag 1980
   gtatgaatta atccacccct t 2001
<210> 178
   <211> 2001
   <212> DNA
   <213> Homo sapiens
<400> 178
   tgggatgcaa gacttatgag ggcaaagatt ttgtaaaatt cactatgtat cccaacgttt      60
   actgaccaat agtcataaga tgttttctta gttcattcac tcacctgctt ttctaataat     120
   tatttcatat cgtctgtttt ctcaaattct gttcttttac atcttcacat tctgctaaat     180
   taccgtgctt atttcactga tgaagttgaa gtgtttagaa tggaactcca taacctccta     240
   ccatgatatt tttccactta ccagcatctg taccaacact ctgcctttgc tgtttctgta     300
   aatgaactat tcatgcctct ctttgcagcc agtctttgtt tgcatgttca tcctgtagat     360
   cccatcccct ttcactttca caaggagcca ttcttccctt tctgtcctat atcatcaatt     420
   tccccatgcc tgctggatcc ctcctgtgag cttacataca tactgttatt tcttccatct     480
   tgaaaaacaa acaaaaaacc tctcttgacc acacttcccc tgccagctac catcatattt     540
   ccctcttttc ctttggagct aaactggttg gcaaagttta ttactttctt caattcctgt     600
   tgttctctct gaaacccact ctaaacagaa gtttgatctt tccacactac cgaaactatt     660
   tttataaaag tcactgataa tctctgcttt gctaagtcca aagatcaatt ctctgttctt     720
   attttacctg acaaattcaa taatttgaca gatttatcac tctgtctttc tgaatatatc     780
   tttttcatat agattccagg acaccatatg tttctcggat tttcctatgc tttcctgatc     840
   actcttcctc agtctccttt gatgccttct ctctctctct ttttttcaac tacttaccta     900
   acactggagt ggtccaaggc tcattccttg gacttctcag tctattctcg ccacttctgt     960
   catgtcagtc agtcacacag ctttaaatac catctgtatc gaataatttc taactttttg      1020
   tttctagcct aaaattccct cttgcattcc tgactcttaa acacaagtgc atctttgaca      1080
   ttcctatctg gatgtttaac agacatctca agcttaacat gcctaacctg ctcctgatcc      1140
   tttccccagg ctgggccctc cagtgaactt ctgcatccag ttggcagctc cagacttata      1200
   gttgtcaaca aaaacactgg aatcatcctg cattcctctc tttctttgac acaccataat      1260
   caatctgtca ggaaatcttg ttgcctttac tttcaaaata tattcagaat ctgacctttg      1320
   ccctgcacct ccagctctac caccatcatg caagccacta tcatctccac ctgattttct      1380
   ataatagctt gttaattgtc tctctgcctc tgtcctttcc tcctcttccg cctttgtcca      1440
   ttgtcaaaag catggcctgg atgattcttt tgaaatgtga gtcacatcac atcactcttc      1500
   tcttcacatt tctgtggtag cccccaattc actcagtcat ggcctacaat gctgtgccct      1560
   ctccgtgacc tctctgatct catttttatt tattttgtct ttgcctaact ccactccagc      1620
   cacactggcc tcttggatat tactcagaca agagaggcag cactccacct cagggtgttt      1680
   gtactagttg ttccccctgt ccaaaattat cctccccaaa atactgacat ggaggacact      1740
   ctcacctcct aaaaatctct tctcaattag aagttggatc ttgtaccctc aggactctca      1800
   gttcctctta tcctactttg attttctttt ccacagcatt tatcaccttc tagtatacta      1860
   tatcattaaa gaatttatta tgcttattgt ctcttgtcat cttagtagca cataagagcc      1920
   gtgagagtaa aaatattaat atgttttcat ttcattcgct ggaatatcca ataattgctg      1980
   cccacactta ctatgtgcaa g                                                2001
<210> 179
   <211> 2001
   <212> DNA
   <213> Homo sapiens
<400> 179
   cagcccaccc gcccgaggcg gcggcgacag gacactcgcg ccaaccgaga ccccgccccc      60
   aggggcccgc ccccccaggg ctccgccgcc aaccctgcgt ccccgacgcc cgtcaccgta      120
   tgtccccgcc cacccccggc ctctgccccg cccgcgcagt ccagcctggt gtgggctccc      180
   tgcaacccca ccccctaagc cgggtcccgc ccccacatgg ccccgccctt atctcttctc      240
   agaccccttc catgggttcc aggcaccttc tcgccagggt ggcgaggaga aacaaaacac      300
   atctgtcaca cagccacatg gcctcctctt acttggacag acaagcgtcc ccagcaaagc      360
   ttcccgtcct ctcgataggt gactcgtcct aaatcagctc tgaattcccg caaagacttc      420
   tggaagctgt gctggggttg aaactggtag accaaaacca gaatcaagtc tgttgggagg      480
   ggcctttccc ctgggacggg aattccagtg gtaatgtctt cctggggctt ctccggaagg      540
   tccaatttcc actccaccct gctcattctt tcaccatgta tttctcacaa acactttgag      600
   ctggattgaa ggggaaacat ttgataatgc aatcattaat ggagtatctg ggtattcggc      660
   aggaagtcag tatatctttc acactactgt ttagacttat tcaaaagaaa tttaaaagaa 720
   acatgaaaaa gcctcatcta ctgttcaaca ctacttctta aaactatttt tcgaagtttt 780
   ttcctagtgc caggcacaga gcagtttctt cataactgtt cattgcatga aaacaccaag 840
   tgtgtatttc tgacaactca cttgagggga agggtaagaa tcagttctgt aagcacaact 900
   ctgagctcgc acagagcccc acgatggggg cggggagctg aagactccta ttctaaggtc 960
   tccaggagca aggccaaaag gaggttgtca gcagtatgca cgccgtgaag gaattccaga 1020
   cttaagcaac agactgagaa gcatgaaaaa atgttttgtc aactaccata tctgccagtg 1080
   agaacctgtc agagtaaaaa agaatctgag gctggccagt ccacccggac accttggcct 1140
   ggatcatttc ttcttccata acaccagatt gatgagctgg gtacatcaac ctgaggcata 1200
   agaaagctct ttcccaagca ccgttgaaag gcgcttcaca gtgcttcaac ctgtagcttc 1260
   agcttcatca atctaccagt gctgcacagc tgtctttggt attgaagata gccaagcttc 1320
   agcttctttt tcttcattca aaaaggacag gtcctcaatc caactttttc ttccaattct 1380
   tctcagccaa ccatctcacc acttccttac ctatcacaag gccaccttcc cctgtgcact 1440
   gagggccctt gacctcttta aggcgcccag tgtccacttc ctggacacac cttcccttac 1500
   gaagttgaca gtatctccta agaattcagt gtacacaatg gtgccggagg aaatggcata 1560
   acagaaggcc taagagacgg atggattcat gaaactttgg cttcacaaaa gatcaagttt 1620
   ttcaacacac aggagattaa gtttaccaca tgattcattc aacactagta attttatttc 1680
   aaaatataaa tatttcacat taataaagaa gttacatcag taggttgtca aagcaagtta 1740
   gctaagaaac agcatttgtg tgagaacaga gggtatttat tgtattcgaa tattcttttt 1800
   tacttttgaa ttgcctcctt cacctgagaa cccagggatg atacattttc aaagagttat 1860
   gtaagcaatt acaactctat atcaaatctt agttcaaaat caaattgtac tggaaataca 1920
   cttcagttac ctaaataagt ggcactgcaa agggcaaaga agggttttct ttgggatggc 1980
   tacaatgaga gttacatctg c 2001
<210> 180
   <211> 2001
   <212> DNA
   <213> Homo sapiens
<400> 180
   gattcctcta ctcaagtact ttaagaaaag ttcctgattt ttccccttgt taagcccgag        60
   aaggaggtgg gagggagaat gcctcccctc tgctattcct tggctccagg tttctcttta       120
   agaaatgcaa tcagaccaca tactcctcag catctgagaa cacttcctct ggagtccagt       180
   tctcctttcc aggcagctga gggaagagag actttaaaac tggcagaggg catcaggggt       240
   gaaaggcaac cctgagagta ggaaaaggcc agggagacag gccgattcca gcaggggaga       300
   ctggcccatc tggagcgcca gcacatgcct ggggatttcc tggcctcacc tgcagacacc       360
   ctcccctccc tgagggttgg gatgctcaga gatcacaggg actctcaacc cactgctggg 420
   agtggcccct ctcagacagg catttttatt atcaaatgag caatgagaga ggggagaggt 480
   gaggaactct atgagaaaca tgcagtcttt tatcacgtca tcagttttca gtgagaaaat 540
   ccagaccttc ttacttttac agagatagaa ttcagttctc agggacttgt gaagatgcag 600
   catcatcaaa tcattgtgtg tgtgtgtgtg tgtgtgtgtt gtgttttcct tatcatccac 660
   cttagaaaat attctccaca ttcccatgtt aaaatatttt ttcctaaaat ccatccatcc 720
   acatgccaac atcctaaaga cagtaatata ggttcattgc agaggatttg gaaaatacag 780
   aattgtgacc agaacaaact aaactgcatc cataatttta tcatcgaatt tgatcaacat 840
   tttggtgaat agccaccagt ctgttttcta tctttttagg gaatataagt tccaagaggc 900
   agggatgtca ttcacttctg tgactcagtg ctctgaatca ggcccagtga agactgacgt 960
   gcagcctgta ttggcagaat gaatgaataa atactacaca cgcatatttt gaattgaaat 1020
   aagttgtcag ggtgcagcag agaataacag aaatagaatt gcttggatgt gctgacctag 1080
   atccaagctg ctactggtaa cgtactgtat ctccttgaat aagtcacttc acctctctgg 1140
   gcctgagctg cttcttcagt aaaataatcc ttgtagctaa gatttgttga gcgctcacta 1200
   tgcacactgc catgagcccc tatatgtgta tttctcatca tagctctatg agttacatac 1260
   ctgccatctc cattttatgc atgagaaaat aggcatggag aagttaagtg gtttgcctaa 1320
   agtcacacag caaatttgtg gagagctggg cttgaactca ggcatctggg cttttactct 1380
   ctccagtgga atgcctgggc tgtgggatga gatgacttca caggcctctc ccagctccag 1440
   agttgagaga gagagaggag tggtgcaggg aggtagtagg gatgattatg ctactggcca 1500
   agttcaagaa agtcaattgt agaacaaggt ccagctgatt attacagaga gcaaagttct 1560
   taactagagg acttcctcct ggaatgtgga gagtccacac gggcactagc agagcataat 1620
   gaaagaccag gaattctctg ctgttcaatc agtgctccat cactcagagg cagggctgca 1680
   agagctccta gtgtgtcccc ttcgccttca gggcctccct gtacagccac ccacaggaca 1740
   gtgcaccagg gtcaggcagg gaagtaaata aaaaaaggaa tctgataatt cctgacagtg 1800
   agcagcagct gatgctttta atgggggagg aagactggga atctcgcttg atgatgacct 1860
   aggacttcag tcagcaggac actggcccct ttcctccccc tcccaggctg aaaccttgtg 1920
   ttttgcctta tgtccatggt aaccaagtct cctttttggt gaggaggaat gggcttcagt 1980
   aaacagatct atttgtgtga t 2001
<210> 181
   <211> 2001
   <212> DNA
   <213> Homo sapiens
<400> 181
   tgtagcagtt gccctgcccg ttcttgcagg tgaccttttc ctggaaacag acattctgga 60
   catctaccag gggctcgtgc acaaaggtgt tcactggttt gcaccgcccc tgtgtcatat      120
   tccggcgcct catcatttgg ttacagtagg tggagctgct gctgggggaa ctgtctgagt      180
   ccatatgctg ccgctggaat ttcttggccc gggattcctt gcccagggaa ggctggaccc      240
   agcccagcac cagcagtatc aggacaagca gaaggagccg gacaagagac ttctccagag      300
   ccatggtggc ctcactttcc cagaaaagcc taattgagaa aaggagagag aaggaaaaga      360
   gccctcttag aaaaagaact ccagctccca cctatggctt gcctggttta cactttctgt      420
   ttcaggtcag ccagaaagac gtccatccta cttcctgagg tttttcgtgt aatgagacac      480
   tcacgtgtac ccttttccac gagcgtttta ttcccactct ccaaagcgag gtcttcctcc      540
   cctctaggtg gtgacctaca aagtgaccta aaatcctgtc agttcctatc tttgaatact      600
   ggagtttctt tgatccaaaa tacatttgta cccagtgctc aaatggctca tcgaaacatc      660
   agtttgaata aatcatgaac caaattgtat ccccgacatc cagggccatg ggtgacagag      720
   ccttgttgtc ttggagcata agggaactca ggccagtgcc tgacactggc atcataaaag      780
   attccattgt attccagaga gaaacgctgg cctcccccaa tttggaggct ccttcttctc      840
   cagtcccatg gatgctctgc agcctgcacg ttccaccctg acccctctgc ccccagctga      900
   tgtttgcctg ggccacattt tttcagcgct agcatcaccc aatctttctc agtgctgtgg      960
   actggcccta gctccctctt ctgcaaccct gaagcagaag cggcacctga gaacacctct     1020
   tgggctgctt acctggatct gcagcttggt gtctgagaga gatggaaggc cagtttctgc     1080
   aatcactcag ctcccggggt gtggacctta tacagattac aaaggggttt ggcttccaag     1140
   aggaaaaggg tgggaggagc atctggagct cagttcaaat aaaatcaagg acgtgaggaa     1200
   ggagtggtga atcacccagg ccgccgtctt tctagggctg ctgaagaaag agcaaatctt     1260
   tgcctacgtt agtctttctt cctatgtgaa ccacatctgt ctgctgatgc acaaggagca     1320
   gcagagaaag ccactacagc actggtgccc aaacctggca cactaggaac aaagtccttg     1380
   ttacttcttc gtgggcgttt tcaccaagta gacttggagg ttaacaaagg acgcaaagga     1440
   gaggttctaa tgggagatag ggaattctgg ccaaagctat tccagggctc ctagggagca     1500
   aaacccaagt ctctttctag gagggcattc tcccaacgct acctctcccg gggatgggag     1560
   gcggggtcgg ggggaatgga ggctgtcctg ctgcccccag ctggaaggct tcccagatct     1620
   ggagaagaaa gggggagtaa ctgaataggg aggaggagga ggcctctagg gtgcgctcac     1680
   ccagttctgg ccacttccca cccaccctcc acccctgctc tgggggcatc gagaggatgg     1740
   ggctgttctt atctacagta accactgacc ctgtaggtcg gaattgagta gaggaactaa     1800
   agataaactt aaatcccaaa tctcaaattc tttttgggga aaaaatacaa agaatatcat     1860
   atttattagc agcagaatgc cctcctagaa agagacctgt ttttttgctg agtaacaatt     1920
   tggatacctc taagaattat caaaatatcc cttcgtgctg tgtattcaga ttgtttcagc     1980
   ctttctgtgt aaaaagtggc c 2001
<210> 182
   <211> 2001
   <212> DNA
   <213> Homo sapiens
<400> 182
   gaatgggtag aataaaagtt tgaaattttc cacttcactt ctttgcacaa tctgaggcag 60
   cctctgaaaa cacgatgcca agagccctag gtaatagcag gacaccagga aaatttggtt 120
   ggttaaggca gtatgactcc agagttctgc taataacaac ctgaaaccac catagtggca 180
   gaggaattac attttttaaa aaaaaaaatt ctttcaacag aacacaaaga ctgaaatagg 240
   aggtcactac aaccgcggga gctgccgccc cgccctgcag ggagcacctg gcctgggacc 300
   cgcaggcatt ctctacaagg ggtgcagctg tgcaaatgct cacaggtgac agaaacagag 360
   catctcctgc ccatcacttc atccaacagc cagaggtgac gaagacgacc ctcctgagtg 420
   aggactgagg gtccacaccg cccccccacc ccacacacca tagagggacc acagaatcca 480
   gctcagcccc tcttgtcagc cctggtaaac gcaggcagtg atgtcaccca gaccacaccc 540
   cttcccccaa tgccacttca gggggactca gagtcagaga cttggtctga ggggagcaga 600
   agcaatctgc agaggatggc ggtccaggct cagccaggca tcaacttcag gaccctgagg 660
   gatgaccgaa ggccccgccc acccaccccc aactcccccg accccaccag gatctacagc 720
   ctcaggaccc ccgtcccaat ccttacccct tgccccatca ccatcttcat gcttacctcc 780
   acccccatcc gatccccatc caggcagaat ccagttccac ccctgcccgg aacccagggt 840
   agtaccgttg ccaggatgtg acgccactga cttgcgcatt ggaggtcaga agaccgcgag 900
   attctcgccc tgagcaacga gcgacggcct gacgtcggcg gagggaagcc ggcccaggct 960
   cggtgaggag gcaaggtaag acgctgaggg aggactgagg cgggcctcac ctcagacaga 1020
   gggcctcaaa taatccagtg ctgcctctgc tgccgggcct gggccacccc gcaggggaag 1080
   acttccaggc tgggtcgcca ctacctcacc ccgccgaccc ccgccgcttt agccacgggg 1140
   aactctgggg acagagctta atgtggccag ggcagggctg gttagaagag gtcagggccc 1200
   acgctgtggc aggaatcaag gtcaggaccc cgagagggaa ctgagggcag cctaaccacc 1260
   accctcacca ccattcccgt cccccaacac caaccccacc cccatccccc attccccatt 1320
   cccatcccca cccccacccc tatcctggca gaatccgggc tttgcccctg gtatcaagtc 1380
   acggaagctc cgggaatggc ggccaggcac gtgagtcctg aggttcacat ctacggctaa 1440
   gggagggaag gggttcggta tcgcgagtat ggccgttggg aggcagcgaa agggcccagg 1500
   ccctcctgga agacagtgga gtcctgaggg gacccagcat gccaggacag ggggcccact 1560
   gtacccctgt ctcaaaccga ggcacctttt cattcggcta cgggaatcct agggatgcag 1620
   acccacttca gcagggggtt ggggcccagc cctgcgagga gtcatgggga ggaagaagag 1680
   ggaggactga ggggaccttg gagtccagat cagtggcaac cttgggctgg gggatgctgg     1740
   gcacagtggc caaatgtgct ctgtgctcat tgcgccttca gggtgaccag agagttgagg     1800
   gctgtggtct gaagagtggg acttcaggtc agcagaggga ggaatcccag gatctgcagg     1860
   gcccaaggtg tacccccaag gggcccctat gtggtggaca gatgcagtgg tcctaggatc     1920
   tgccaagcat ccaggtgaag agactgaggg aggattgagg gtacccctgg gacagaatgc     1980
   ggactggggg ccccataaaa a                                               2001
<210> 183
   <211> 2001
   <212> DNA
   <213> Homo sapiens
<400> 183
   cagcggtccg gccgaatctc gccggggtct cctcttcccc tgcaccagcc agcgcctcct      60
   ggctggccag tcccaccctg gctcaccctt cccgaagagc ctgccgagac cactcatcgc     120
   gagctcgcct ctcccgcctc cgcccctcag cgtcctccag acttccgcct tgctcctggg     180
   agggtgatgt ctcatcacac agggaccagc cttgcccaat ccgtcctcag ggcgccgccg     240
   cgacatcaag aggcgcaaac gagaccaccg gtgtcacgtg acccaagcca ccagccccag     300
   accgacgcgc tcctcttaaa gctgccacat cattttctat taagtaaaaa gacagttcta     360
   tctccacctg agtacgtgct tgttctccgg aaggaaatgt gcagtgtgat ggcacctcag     420
   tgtatggggt cagtgggaaa acttcatgct ctttacagag ctcaggaaag tggggtggta     480
   taggttcaag cacttggggt tgccctaagt ttagatggca aacctgatgc agtctaatgt     540
   ctcacattct ggtctctgca cctcaggaca accaacccca tttcacaggc tagaaaaata     600
   gaaactcccc acactgtggg taaaatctct tctgtcacac acagatgaac tttaataaat     660
   tacaaatgca cctgaaaatg ccttcttgat ttcctttcag tttaggcctc aaatgggctc     720
   tcctcaaggc tggacctcaa aggcccagtt tgggccttcg caaatgtctc taacccttga     780
   cttagagttt gaagattcat tccattctgg atgtgaatgc aggtaacacc tagaaagata     840
   agaagtcaca tttcattaag ctttcagggt tctccattat tactctgacc tttgtgagtg     900
   ccacctatag ggcaaactat ccaacctgta tgtatctacc taatccctcc tagggacccc     960
   aaatggctgt tcctcatcac tcagtaccca gtttgctgac gtctacaata ttccgcctct    1020
   catcatcaaa gaagatcatc tgggagaaag gaattccagt cttctgctgc aacctattca    1080
   agacagggca ggagtaacca agctagtatc ttggtttccc aggcctctct gatactttct    1140
   caccctgctc ctcccttatc tccgcatacc tctcaaagtg tgtgatcttg ctgcctggat    1200
   agatttcccg atgaacaaag tacctgaaga ggtcaaagag ctccagtagc tggttggccc    1260
   cttctatctc acttgtcctg caaaacggtg taaaagatgg gattagcaaa gtgaagggcc    1320
   agggctacgt taacttatta agcaaagtta gtaagtataa ccagggaaat caacttgctg    1380
   tttttccggg atggacaggc atcctcagcc tctgtttaac tccattgcct tcctatttga      1440
   cacaattggt gctgggccaa tgttttagct tttgttttga gacagggtct agctctgttg      1500
   cccagactgg agtgcagcga cccaatcacg gctcactgtg gtcacgacct cctgggctca      1560
   agtgatcctc ccacctcagc ctcccaagta gctgggacta caggtgcacg ccactacaat      1620
   tttttttttt tttttggtag agatagggtc tccctctatt gcctaggctg gtctcaaatt      1680
   cctgggctca agtgatcctc ccacctaggc ctcccaaaat gctaagatta caggtgtgag      1740
   tcactgtgtc tggccccact tttttttttt tttttttttt tgagacattg tctcactctg      1800
   tcacccaggc tgaagtgcag tggctccatc tcagctcact gcaacctccg cctcccgggt      1860
   tcaagcgatt ctcctgcctc agcctcccga gtagctagga ttacaggtgc gtgccaccac      1920
   gccctgctaa tttttgtatt tttagtagag acggggtttc accatgttga tcaggctggt      1980
   cttgaactcc cgatctcgtg a                                                2001
<210> 184
   <211> 2001
   <212> DNA
   <213> Homo sapiens
<400> 184
   gaccaggaca cggaatgtgt gggcttgcca atattttgat atataaatca actgttttca        60
   ttatggaagt aacaaaacct caagggaaat agaaaaaaaa attccctgta atcatcccac       120
   ccagctagga ggtctactgt gtgtgttttt gacattttgc tgtcctcgtt ggtgcaaggg       180
   cagatgtgac tgtttatgcc tgtggagggc agaggggttt ttcttcttct tgtctttttt       240
   tagagacagg gtctccctct tttgcccagc ctggatggaa tgcagtggca tgatcccagc       300
   tcactgcagc ctcgaacccc tggcctcaaa ggatcctcct gcctcagcct cccaagtagc       360
   tgggactaca ggcacctgcc accgtgcctg gctgatttat tttttatttg tttgtttaga       420
   gatggggtct ccctacattg gccaggctgg tcttgaactc ctggcctcaa atgttcctcc       480
   tgtcttggcc tcccaaagtt ttgggattat aggcgtgagc catgttgtcg tcattacagc       540
   tacattgaga tataatttat gtgccacaca atgcatgcct ttcaagtgta cagttcagtg       600
   gcattgaggt atatccacag aaccgtacaa ccatcaccac attgagaaaa tttttatcac       660
   cctaaaaaat cctttacaaa atcctttacc ctttagcagt caccccatgt tttgattttt       720
   gctttacatg attttacaag catcttttcc tgttgctgga aaatctcctt acccatcctc       780
   tttggtgact gtcagcaaag gctttcgggt ggtgaaatag cgtggccagg agtgctctag       840
   aacaatggtg ttggtgaggg ctgcagaagc tgaggaccca caggcagaga gggagggatg       900
   gtagaagcag gtggtgagga gggactcggg gcaggacttt gggggtaaaa ggaagcaagt       960
   ctctggaagc agaaccagca gcactgctga tcattgggac gtggagaaag gcgaaggctc      1020
   agggtgtcag caaacttcct agggtgggtg agggttgcca ggtcaataac gaacagggtc      1080
   ccaggaaaag gcggcagcac aggtttgggg agaattagag atgagcttgg ttttggtctt 1140
   gttggatttg ggggcaccag tctctctcct ggcgagagtc ggcagtgggg tgttaggatc 1200
   tggccctcag gaggctgtta gggctgaagc aagagatttg ggagttaagg gtgtgagggc 1260
   cacgtgggag gcctcgggag cctgtgccga tccagggtga gtgtgaagga aagaaaaaag 1320
   agggcccggg gaacactggt attttcgggc ggtggacgag gatgggacgg ccacgagctt 1380
   gtcttaaggt agaaggcaga gcggggccgt aggggcagtg gcgcgggcga gggtggagaa 1440
   cagcgctgag cctcgctggg aggcggccgg agggggctgc agcccccgag ggctccttgc 1500
   agtccggccc cgcagcctgg agacagctcc ggtggtgctg cgctgcccga gactgctgca 1560
   cgagcgggga ctgcggcctc ggctccctgc ctgcccggca gccccccatc cagtcggcct 1620
   gcccggggtg cgcagggtta gcctggagtg agctcgcccc gcccggggtt gcgcggccct 1680
   ggggggtcct ctcgcggcgg ctcctgggac ctgctggcgc cccgggcaga gcccggcccc 1740
   atcccaggag tcgcccggac tggaaggcag tgcgagcact gggacacagc ctccgagctc 1800
   gagcgtgacg ggaagttggg gcaatttgtt agttatccgc cgccaccaag acgcggcacg 1860
   gcgcctggac cggaggggcc ccgcgcgggc gcgaactttg ggctcgggcg agtgggtggt 1920
   gctccgccca gcccgagacg ggcgggcgcg cgggccaatg ggtgccgcct cttggccgcg 1980
   gggggccccg acccgtgggt c 2001
<210> 185
   <211> 2001
   <212> DNA
   <213> Homo sapiens
<400> 185
   cattcacaac aacccattaa tagatgaaga aattgggctc agagaggttg ccatctgtcc      60
   aaagtcacac aggatgagat ggaactgggg tttaaaacca ggtctgttta tcctacaaag     120
   gatggaaaag aaccaaacca aggtgctttt cccagtctca tgcagcactc acacttctga     180
   cacccaaagg tgtgagagtt gtttctccat gcaccaagca agtttctaac agacaccaag     240
   tgagtgtcct ctaattcagt tccgatgcta tcttctggat gatagcatca gattccacaa     300
   gttaaggcac aagactgccc cctcctcccc ctcaccactg cttcagatgc tgaccacaag     360
   tcccaggttg tggcctgtgc ttctgaccaa ccagctgtaa atcaggttcc cacaaaaccc     420
   ccctcaggtt tgattaattt gatggagtgg ctcacagaac tcaggaaaac acttagttta     480
   ctggtttgtt aaaggatatt acaaaggcta taaatgtcta gccagttgga agagatgcac     540
   agggcaaagc atgtgggaag ggtgtggagg cttcagtgac ctctccaggt ataccacccc     600
   ttaggaagct ccatgggttc agctgtccaa atccagttct tttagctttt tatagaggct     660
   tattgtgccg gcctgattga ctaaatcatt ggccatttgg aatcaactca gcctttaggc     720
   ccccacccct ccctgcgggt catgaggtgg gtggggacaa aagttgcaac cctctaatca     780
   catgattgat ccccctggca acaagccccc atccagaggc tatccaggag cccaccaaaa 840
   agtcacctca ctggaacaaa agatgctcct ctcacccggg aattatcaag ggatttagga 900
   gttctgtgtc aggaacgagg gtcaaagacc aaatatttga acaaaagatt agcaccccta 960
   tctacgaggg ttttaggagc tctgcgtcag aaactcaggc gtagagacca gatacatatt 1020
   tcttactata tcatcagacc acgtctttaa agcccgtttt ctttgtacac actctactac 1080
   actctaattg ttagctgttt aagtattaga aaatcattcc ttagaggcaa aatggtgtag 1140
   tatggtgaca gtgtaagcct gtgctctctt caccttccta caactaagtg gtgatgtcaa 1200
   gaggaaaggc taggtgtagg tttgagatcc gcatttggca gcaggggaag ggaagggaca 1260
   gagtgagccc aggaaaaatt gtgaagtggg cagagaatct tcactcccct tcagactctt 1320
   tccccttctc aggcagctcc cccacctctg cactcatttt gccccttggt cactgtagag 1380
   accccaccca atgcttcgga catgatctag acttatctgt tcgcactgcg tttggttata 1440
   tctgtcccca cttgccaagg tccttcacct ttgtgggcct tgaggtcatt gtctctacca 1500
   gctggtggcc tcaggcaagt gactcctctc agggtatttg agcttggagc ccctgaattt 1560
   tcaatactaa tgcccaaaga acatgagtct aaaatggggt tgtctgactt ggagataaga 1620
   gcaaagaaag aatcctttgg tctcccaatc tacagagctg tggatagaaa catttactaa 1680
   tagacagcca tgcttcaaag cagtgtttct catcattttg gctggtatat ggacaaccct 1740
   cctccccatt gtcaatgcca gcagatggaa gtgacacagt ccatctgttg gcccacttca 1800
   ggggccggct gtgtcttctt actatatctt ccttctaaaa tacactttct attggagact 1860
   gctctagtga aggtgacatt ctgaggtgag gtggactctc tactgtccct tattccacca 1920
   gccctgaatc caggagacct aggagggggg tgctctgaag gtgtgaattt tttccttgtt 1980
   gcaggcacat ggtaattgct a 2001
<210> 186
   <211> 2001
   <212> DNA
   <213> Homo sapiens
<400> 186
   cctacgaagt aagcaagctg tgattccaac ccagatttgt gggactccaa agcccactac      60
   taatccactg gaccgtgttc ctggaggcca caaatgctct ttcctcagct gtcttctcag     120
   atcctagaac gggctgatat atggctcaaa agttctttaa cggccaggca cggtggctca     180
   agcctgtaat cccagcactt tggaaggcca aggcaggagg atcacctgag gtccaggagt     240
   tcgagaccaa cctggccaac atggtgaaac cctgtctcta ctaaaaatac aaaaattagc     300
   aggatgtggt agcaggcgcc tgtaatctca gctacttggg aggctgaggc aagagaatcg     360
   cttgaacctg ggaggcagag gttgcagtga gccgagatca caccattgca ctccagcctg     420
   ggcgacagag aatccgtctc aaaagaaaaa aaaaagttgt ctaacatatt aatacaactt     480
   tcaagccaca tgcctcggtc taaaagtagg tgcccccagt acacttttac cccaggtagc    540
   aacttccact caccttaaag tcacatacct ggaggaaggg gatagggagg aaatctaaac    600
   atcttatact tgaatgtaag ctccgttatt tcacggattc aaatgtggtg aaaggaacta    660
   tttttttttc cttttttttt tgagatggag tctcactctg tcgcccaggc gcgatctcgg    720
   ctcactgcaa cctcccgtct cccgtgttca agcaattctc ctgcctcagc ctccagagta    780
   gctaggatta caggcgccca ccaccacgcg caactgattt ttgtattttt agtagagaca    840
   gggtttcacc atgttggcca ggctggtctc aaactcctga cctcaggtga tccgcccgcc    900
   tcggcctccc agagtgctgg gattacaggc gtgagccacc gggaccagac ggaactattc    960
   ttttccacaa aagaacaaac acatacataa cttttgccac gtctgaagac gagggttcta   1020
   atagggcgcg ggtggtgtct caggtatttt gaaaggagct acccaagaat ctcagcttgt   1080
   caggccagtc attccatttg accctatctg gcgcgtattg gggccccgac aaggtaaggg   1140
   gttggcatga agctgcacag tgagataaag ctggaccgga gctagaaccc agaatcaaag   1200
   ccttgccctg tgcggtttct catcgcctac gcagtcgggc cctggcctgg catctccagg   1260
   caggtggcct tggacggaag cccggcctac tggcttcatt tgtgggaccc cgggggtcct   1320
   ctcctccgcc catggagcct ggttactccc acttcctcaa gcctagggcc tggagaagcc   1380
   tttcctggtt cgcggcctca cggtctgcaa ggtcctctta cttccctgaa gccgctccag   1440
   ccccacttcc cgaaggtccc gcttccgggt ggctcgcttc gccacctgcg gtcccctgcc   1500
   tcggcttggg aacgccaagg acatccccca cggaagcctg ggcccacatc cccatgccct   1560
   tgccgcacgt caccgccccg ctggccccga atacgcctcc gccctgcact tcctctctgc   1620
   agcgccgtct ttccggccgg gctcccttct aattggccag ctcagagctt cctcgggtac   1680
   tcggccactc ggggcatcgc ggcggccttt ctagccgctg tcccaagggt tggtctcgcg   1740
   ctttcggctg cgagctctct gtggtgctgg cagcgacatg tggcgcctcc cgggactcct   1800
   gggccgaggt aagggacgtc caagcaacca gcgtcactct gggccgcaaa gaccgaactg   1860
   gagtgggggc gggcccgagg aaacagccga agcgatcgtg ggccgggtta gagccaagga   1920
   aggcggggct tgaaagaggg cggggaagtc aggggctgct ggaatttgag atgtggatgg   1980
   gcctagctag aagagggtgg a                                             2001
<210> 187
   <211> 2001
   <212> DNA
   <213> Homo sapiens
<400> 187
   cgtggcatat caccaggaat gccactgctt aaactcgtct gaggctggga agcccaccca       60
   gaccaacagg cgggccctct ggcttccctg gagggagctc tgtccccagc actcagagaa      120
   cagagtcccc acagcaaagg ccctcagggt gcaacggccg tgtcggaggg ccccttccca      180
   tcttctcttc ttcagcacat gtgcccagct cctgcaccca atgcccaggg gatgcctccc 240
   agatgccagc agcatggtga caggggcagg aggtggcatt gtccttgttc tggatatccc 300
   tgggtgaccc cggtacgtga ctcccctgaa ctccagcggc ctcatctgtg acacaggggg 360
   ccgggccaca tcatcctggg cagtgctgct ctctgcaatt attgagtctg gagtctcttc 420
   ttgtcagaga ttctctttag ggagtttatt ttccatagac taaagactaa acaatagaga 480
   acagagagag gcttctagat acctactgca accttagctt ctcagaaaat acaggtgctt 540
   gggactcttc cacaaaatca ggaatttaaa tggaaaacgt gggccccaag acagctcctt 600
   tagtcaacgg gtctgagtac acattaaagg agggtggagg gagaggggac atagagaggg 660
   aggaaagggg aggagggcac caggaagggc ggagccaagg gagtcctggg ggtggatacg 720
   cacctgcctg taaggggccc tgtggtcctg gcacgtgcct ataagaggcc ctgtcatcgt 780
   gtgttaggtt gtgacacagc tctgccctcc ccaggtgcct tttctgaacc aggaacctaa 840
   gaggagctct cccaccaggg ccagggaagg agggcaccag gacgggcaga gccaaggggg 900
   ttctggaagt ccccggccct ggtgggagag ctcctctctt aggttcctgg ttcagaaaag 960
   gcacctgggg agggcagagc tgtgtcacaa cctaacacac gatgacaggg ccccttacag 1020
   gcacgtcgta tccaccccca ggagcacaca gacagactct cgtccacatg ccctgtctgg 1080
   ggacaggagc ccagcaccca atgcttgttc ctgcttccac ccctttgctg acctccccac 1140
   tcatcagggc tgcgtgccag gtgaccaggg ttctaaccct ggcttcctcc ctgacccctc 1200
   ctcctggaaa catccttcct gacccagcct cagtttacct cattgaaggg tgaattttaa 1260
   ggacccattc agcttttatc tcctgtcact ccatagacat caagacttaa catcccatcc 1320
   ctcccactcc cagctgtcat tccccccccc caatcttcgg cccactccct gtccaggggc 1380
   ccaggtcccc agcgcgggca acccctctgt ccccattccc gggacctgga gcccgcgagg 1440
   gctcaggccg gccaggatgg agttctctat cccagttcaa tttcccccgc ctttttgatg 1500
   tcacatcctg tcccagcctg cttccccgcc ggccgccgcc ctcctccccg ggagagagcg 1560
   aggcgcgcgg gtccctctgc gccacccccg cccccgcccc ttccgagcaa acttttggca 1620
   cccaccgcag cccagcgcgc gttcgtgctc cgcagggcgc gcctctctcc gccaatgcca 1680
   ggcgcgcggg ggagccatta ggaggcgagg agagaggagg gcgcagctcc cgcccagccc 1740
   agccctgccc agccctgccc ggaggcagac gcgccggaac cgggacgcga taaatatgca 1800
   gagcggaggc ttcgcgcagc agagcccgcg cgccgcccgc tccgggtgct gaatccaggc 1860
   gtggggacac gagccaggcg ccgccgccgg agccagcgga gccggggcca gagccggagc 1920
   gcgtccgcgt ccacgcagcc gccggccggc cagcacccag ggccctgcat gccaggtcgt 1980
   tggaggtggc agcgagacat g 2001
<210> 188
   <211> 2001
   <212> DNA
   <213> Homo sapiens
<400> 188
   ctccggcagc ttgtcgcccc acgcttggga ggccagtccg cacgctcggt cgcgggccgt      60
   gcgccctcag atgaagcccg tgcgggccgg ccccagcggc gccccacctc cgggccgcct     120
   cccaggcccg cctcggccct cctgcctctc agcgatcgcc acctcgcgac ccagcctgtg     180
   gacgtgccgc ggcgcccgcg ccggcagccc ggggcctcgg accggttcct tcccgccgcg     240
   tgaggctgac gcaccgacca gcttcccggc tcggccccgc ccggtccgct gcgcccgcgc     300
   ccgtccccgc ccccggctcc ctgacgcccc cggcctggag acgcaacccg acgcgcacaa     360
   ccccagaccc tgcgcgggag tcgggggtcc ttccagttcc cagagcgaga gcgaaagccg     420
   gaggggcctg tgttttcctg cggggttggg gggcgggggg tgcccgcccc gcgccgcccc     480
   gcccagcccc agcccgctca ctcccggcca atccttccgt atctcaatgc gagacaatca     540
   aatcgtacga attacgtgtc caccgtgttc ccggcggagt cttaaagctg aaagggatat     600
   tagagagcat ctagaccagg gcagcatatg tcatttatat ttttagtagc cacagcaaaa     660
   gagtaaaaat aacaggtaca ttttattttt aaaatatttt tatttaaccc cgaatgtgca     720
   aaatattatt tcaaaatata accaacatta aatttattca taagatattt gacttttttt     780
   gtctttgaaa tccagtgtgt attcgatgcg aactagaccc atttcatagt caaatgtggc     840
   tggctagtgc ccactgtatt agacagcgag ggtggaagcc acccgctcat atcataggaa     900
   gaaacaggcc tagagaagtg aggtaaggtg agcaaggatt ggaggtcctc tgatacccag     960
   tcctggtctt ttaacattca gagttgggac tagtctatcc gctttttttt tttccagaca    1020
   gggtctccct gggtcaccca ggctggcctg caatgatgcg atcgcagctc actgcaacct    1080
   cgaactcctg ggctcaagcg atcctcccac ctaagcctcc tgagtagcat gaactacagg    1140
   cgtgcgccac cacgcctggc taattttatt tttagtagaa acgaggtttc accatgttgc    1200
   ccaggctggt ctcaaactcc tcaagagcga ttctcccgcc tcagcctccc aaagtgctgg    1260
   gattactttt tttttttttt tcgaggcgga gtctcgctct gtcgcccagg ctggagtgca    1320
   gtggcgtcat cttggcttac tgcaatctct gcctcccagg ttcaagagat tctcctgcct    1380
   gagcttcccc agtagctggg attacaggcg taagccacca cacactcggc taatttttgt    1440
   attttcagta gagaaggggt tttgccatgt tggccaggct ggtctctagc tctgagctca    1500
   ggtgacccac ccatctcagc ctcccaaagt gctagaattg cagacatgag ccaccgcacc    1560
   tggccttact ttttgttttt tgtttgtttg tttgtttgtt gttttaagaa gacttttttt    1620
   tgagacactc tcacttcctc acccaggctg gagtgcagtg gcgcaatctc gggtcactgc    1680
   aacctccgct tcccgggttt aagtgattct tacgcctcag ctacccgagt agctgggatt    1740
   acaggcctgc gccaccatac ccagctaact tttgtatttt tagtagacat gtggtttcac    1800
   catgttggcc aggctggtct cgaactcctg accttaagtg atctgcctgc ctctgcctcc    1860
   caaagtgctg ggattacagg tgtgagccac ttcaccaagc ctaagaaggt agtttctagg 1920
   tgaaactggc agctttccga catcttgcat gcacattaaa agttgaaata gttcaaatga 1980
   cttaaactca tgtattttac t 2001
<210> 189
   <211> 2001
   <212> DNA
   <213> Homo sapiens
<400> 189
   cgtccgcggg cgtcaatagc gactttcagc acaaaacaaa gatggcggcg gcggcatctc 60
   ggaaatgccc ggatgagact gctaacccct ccgacgcgct cggccccgcc cccttgggaa 120
   cggtctctcg ggttgataag ggacgcacgc ccgaagaact gcctttaacc agcactgggg 180
   acagcggcac gcaggtggcc ggggttggcg aacagccact gcagccgcgc ctgcggccgg 240
   gaagcgagca ttttcgcgcc ctgcacaccc agcagccgca ggaggcggcc cgaggcgcgc 300
   cgcgggggag ccgggggcgg ggcgacagtg ggaggcgacg cagccgagcg ccggcgcccg 360
   ggggtggagc ctccgcccgc tcccaggctc ccaggctgcg gggcccgccc tcgggcgcca 420
   ccgcctccgc ccgcgccgca gcaaaatgag ccgggccgcg cgccccagac cgccgcgcgc 480
   tcggccgccc ccactgccga gaggcccgca gtcgggccgg tggcgcacga gccgggcggg 540
   cgaccgcggg aggcgtccgc gtcatctcgc gtcacctcgc cccgcccgct tcccaggctt 600
   cgagtcccgc cctgcgcgca ggcgcaccgg cgctgctcgg atcctccctt ttcggagatt 660
   tgaatttccc ccagcgaggc gagtgaggcg aaatacccgt atggtgatag ctggcctttt 720
   cgcgccaata ctgtaagtgc tcaccgcagc cctgggaggt gggtcccggc ctcgccccca 780
   cgtttcgcct cctctaggag gccgcccggg cgcggcggcg gcggcggcgg cggcggcatc 840
   cgccctgcca cgcgcaggcc gcagcgcggg gacccaagct gctgcccggg agcggcagtc 900
   gccagtagaa atggacagac cggtcctgtc agagcctgtg gcttttgttg taaacgtcta 960
   gaagttcagt taaattacaa gatctaccag atctaccggc gggcagaatt attactttga 1020
   agttactgaa gttatgcgtc ctgctggaag accttgtaat ttaatgcgtt agtaaagaag 1080
   cacacgtcac cctctcacac attttcaaca atatttttaa agtcgcctct caacatatta 1140
   catgtatttt gtaatcgtct gtgttttagt tcatgcagtt ggaagtatcc atttgagaag 1200
   ggacaaagag aagaattctt gcctgtctgt gccaccacct gctctttgtg accttggcta 1260
   gttacctcac atatgtcccc aagtccccca cccatggtaa ataagaaaac taaggcctga 1320
   tgcgttggct catgcctgta atcccaacac tttggaaggc tgaggtggga ggattgcttg 1380
   agcctagccg gggcaacata gcgagacccg gaggcatggt ggcacacgcc tgtggtccca 1440
   ggtacttgga acgctgaggt gggaggatgg cttgagccag ggagttcagg gctgcagtga 1500
   gctgagatcg cgccactgct ccagcctggg cgacagagca agaccctgtc tcaaaaacac 1560
   acacacacac acacacacac acaaaagcag ctgctggttt ccaagggcct tgcctggaat     1620
   cagtccagaa gctaaattaa tgttaattga tattccttta tccacagaaa gacagcaaga     1680
   aaggtcatgc ggagcggggg acataactaa ccataaatgc taaaggcctg gaggtgacag     1740
   tgcctgctgt ttaaacgcgt gcgggagagt ctggggcctt tagccaggag attgtcaggc     1800
   ttcccagggc ttccctgaaa gaccccagac ccaggggcca gtgctgttga ggtgttggct     1860
   ttcccatgcc aggcgctggg ggaagctgtg acgagatttg agtgtcacat aataaggatc     1920
   atggcaattc aaaggtacag ggaagctagg ctataaggta accagctcgg ttttcctggg     1980
   ggtcttggac aggtgctttg a                                               2001
<210> 190
   <211> 2001
   <212> DNA
   <213> Homo sapiens
<400> 190
   tgggattgca ggcgtgagcc accacgccca gccctattat tatttttgac aacaacagca      60
   aagatctgcc catccgactg caggaattta atacacagcc tttgtcagtg gtagaaattc     120
   tatcactgta tgactcatga gtacataaca atgtgctata ccatacttcc tctttgaggg     180
   ataaagcccc tgcactctaa gggaggattt cattatgcat ttctcttata tcaccagaaa     240
   attatttagt ggaagacagt ttaatatttg acagctttga accatttttt tttctctttc     300
   aaccagtctt tttacattgt ctgcttaaaa acaggaaacc acatatctcc accccagcac     360
   aacccaccat ccaccagcac agaccgtatc attttggatc tacagtcttt ttcttccaag     420
   cctggccagg tgcctgctcc tactttgcag gaaacccctc caggcactcc cggtgcgtgt     480
   tctcagagcc cttctgagaa tattcaggat tgcacttcct gggctgcagc gcaccctgta     540
   ccctcctttt ttaagcctcc tcccctgagt cactgctacc cttccctcct ctaacttccc     600
   cttctctgca gcctccttag caatttctta tcataaagtt tgttttaaag aagaactgcc     660
   cctctaaaaa aataataaac tgtcgagggc ggtagccccg tctagaggtt tccgactttg     720
   ctctgtgccc acctgaagcc tccccagccc gaggtatttg aacttacatt cagtgcttca     780
   cgtctctgtc cggggtgcct ctgtctctca actctgactt ctgagtactg gcagttagaa     840
   caggaaggaa gtcaatgaga aaaaaaaatg ttttagacct acttgaagag gaaaccctgg     900
   gctgtctcaa atcctgttcc tttgcactgc ctatctcttg atcagccaac ttgagcagca     960
   ttcttgtcaa ttaatggcac atgtgcgagc atgacagccc gtgtgacgtg gagatgcatg    1020
   aatgtacacg caagagtgtg gagcctatag ctatgggtat tatgaataat tgtgagggta    1080
   tttctgataa tcaggtcatt gcagtgcata agcctatgga ggtaaggagc ctgaaaccag    1140
   tagcaataaa ttcacatcgg ctttactttg taccactcac ttctgttccc actgggtccc    1200
   cgccagcttc attatcaacc agatacgttg aaagtttgct ggcagtgaat gtagagtgct    1260
   aagaaatgat gattgttaac tgcatatact acggtcctaa atttaaaaat gtgtccttaa     1320
   acagattttt ggagttttgt tggttataat ttcaagacgt agctttgtat tgcactacaa     1380
   acagtcaaat attgttttta atagttttgg ctgatttttc ttcaaatgaa acaaacaagc     1440
   aagcgaaagt actgtctttg atgaaaccta ggaaagaaat tacaaaagga agtaaaactt     1500
   cattaattca tagtcttcca actcagaatt tgtgacaact taacaagtgc caaaatagac     1560
   tttgtaccat atttgaggaa atggtttatg aagtaattta aatgggaaca agtctcggag     1620
   gccatgttta aaccatctga ggaacagttt ttaagcagcc aagtcacatt catgctgtat     1680
   attcccgtag ctgttaccaa taataaatgc ttatttaggt taatatttca atattcagta     1740
   tttacactac atcaatatat ttagatctga ctaagcacac aaatttggag aaactgacgt     1800
   ttattttgtt ttcacctaaa tgtactgcat cttcttcatt tccctttgta aattgatacc     1860
   acagggtttg ccttctactt agagtcaatg aaatcggaat aatcaaggga gtcatttcac     1920
   gtgagaagag attagcctag aaaaatatga aaaggaaaat agtatataat taaaatgagc     1980
   aaaaatacta aaagcctaat a                                               2001
<210> 191
   <211> 2001
   <212> DNA
   <213> Homo sapiens
<400> 191
   agccaaaaga ttggacaccc ctgattgaga acaatgcctg gaactttgta gctctcaaaa 60
   gtttgttcaa tgaatgaatg agtgaatgct tccaatacaa aaccttagaa caggcaagtc 120
   aaaatgtcac caaaaggtgg gaagaagagg cattcacggt actggttgag tgcatagtaa 180
   ataaggtact tgcacaacta tgggcaacaa agagacggct tgggaattat caaacaccaa 240
   ttttgatttt gatgagaaca gtcataaccc aatatttgaa gattttgatg gttgcttctt 300
   ctttgcccct ctaaccaccc cccccacccc agaacgatga agggttaacc aaaacaccac 360
   caaaggcaac tacttattac ctttgttatt cccacataaa aaattgagca gacctattgt 420
   ttaagcagaa gaggcagtcc ctcaatgaag caatatcctg tttgaataat ttaggatggg 480
   gcataggcca gcccacacag cgcatttgtg tgacagggcg tcccactcag acgtctccat 540
   ttccggtatc tagagccggc tgccctctaa aaatccccct gttacagtcg ggatagaaaa 600
   caaaaagctt aatttaccag gaggctaaac agattgtgcc ttcttaggca aatgttccac 660
   ctgcttggcc aattagcttc ctctcccgaa agactcgttc aacaggtttc ctttttctct 720
   tctctgcctc ctgggttggg gaaagaggct gtcacttcgc ttcgaccctc cccttccctc 780
   cctctcggct tttccacagt gggtcggacc aatcgcgccc gctccgacgt gtccaggtcc 840
   gcggccccgg gagcttggcg cggcccggcc tggatgcgct gggcggaggg tgcaggggag 900
   ggcacggcgc cgcttgcttg gcctgcgcac ccggacctag aagccgggac gagccggggc 960
   agagccaggc gcgcggaagt ctgaaggtgc cctccagaca cggccccgat gactgaacta 1020
   cagcaagatg tggaagacac aaagcctgcg aaagtgctcg ggaagaggga gagcaaactt 1080
   ggctcagccc agtgagtatt ctcagctggg acccatccaa ggaggtggga ggagcgcagc 1140
   ctaaacttct ctgccccggg acgcattttg ggtggatgcg gatttctgga gctcctatat 1200
   ggacaatctt gtggcgcgct ctctctgtct ctcactctct ctctctgtct agatattcag 1260
   ttaaggagat ggtttagatt gattgaaaag tctactgtag gatttaaaca aattctctta 1320
   attcaaaact cactggatgt tgggaaaata attttgtaga aaataagcct aaccacattg 1380
   aagctcaaag tcccaacaca gcccttcgtt tagaatgacc attttcacaa attcatttta 1440
   gttcagcaaa taattgagta tctacttgtt caataacgat gggcaataca aggataaatg 1500
   ttattttgga tctgcccttc tggaacttca agagcagagt ggggatgggg gaggcacaaa 1560
   tgaatattac tgtacttcaa gggactttgg tgaccattgc cacattaaac acacagacaa 1620
   ggtatgataa agagtttagc ctataattga gtcttctcta cttttaaaat atttatctat 1680
   tttgcagtct ttggtaaaat tggtttggga ggagaggttg tttttttcct acaaagtggt 1740
   attttctaag cctactgaag tcacttctaa ttcagtctat gattttagac actaaccctt 1800
   ctgagctcta aaactcttgt tctggtatca ggatatgagt aatttaggaa ggagggatat 1860
   gtattatcat aaggttatac tgatgtgata attccacttt gaaatcattt taagttgcca 1920
   aatttttgtg cttagaaacc cggtctaact tctgttttga acttcataac ttacattaat 1980
   tatcactagg ctcaaagtct t 2001
<210> 192
   <211> 2001
   <212> DNA
   <213> Homo sapiens
<400> 192
   aattctctca acagtatttt gaggtaatgg cactggccga tccagtatcc cggctttttt      60
   tcttaagagc caggtagcac caacattttc atgctaaata aacattagga aagctaaaat     120
   tcttgttgac ttatacctgc catgcaaggt ggaataaata tgcatcaaga gtccaaatga     180
   aacagaaaac tggggagaga agaagattaa gagaggcact ttgcaattta ttaaactgtt     240
   aatttgcctt acatgccttt ttaaaagaaa gagctttggc tcaaatgaag aaagaggaat     300
   ataagaatga agattaaatg ggaaggaggc aaaaacttga caggtgaaaa tgacttggaa     360
   atgatagata cataaaaaca aacaaacaaa aacctacaaa catgatcata gctaagaggg     420
   aggaaaatga aaggctatgt actaattcac atatattgac aaaagaaaaa agggtgagtc     480
   cataaacgct ctgacccttg ttgtagatgt gaagcgttcc acttccagaa cttgtgcttt     540
   tattggacag ccggagagat acgtctgtat attaggctcc ttaaatcctt gagtgttata     600
   aatagcagag aaagggatat acactaaaaa aaaaagtaaa taaagttaat gcaatatatg     660
   attagcataa cttgtctttt acaggcattc ttttaagaat cacgtgttaa aacaagtaag 720
   cctcttataa aatgatctga gcccggtgtt acatctgcaa ggaaaatcaa tcatttgctg 780
   gttacttcaa atttgtggtc ttccgaccaa ccgagataaa ctcttttctt ccccgctcag 840
   atcatccgtc tttaccttct tgtatcttgg gatcgctggg agacacgtcg tagtctacct 900
   cctctccctc aaagtggagt tcccgcgtgt ccagattttc tatgatatta ctcatgtcag 960
   cagcaatttt ctgcagtgca gaggtattta cccgtggctc gtttttcagt gacatgttaa 1020
   ctttggacag agtaaaagca aaggggctag gaaagaagaa aacggttaca aagacttagc 1080
   attttgataa cataaaatgc tattccacaa tttgaatcca gacttagata cagcatatgg 1140
   aaacaattca gcctgctctg tgtgagcctc agttatccaa aacaaccttt tagtttacgt 1200
   tcctgtttaa catttaaagg attcctaact tgtaaattaa aacagagtgc ttaattgaaa 1260
   acaagagaaa aaaaaaagaa aaaaaaaagc aacaccacat tgaatgccaa catttctctc 1320
   atccaacttt attaacttgg ataagtaatt caagggagat tggtttggga aagcttattc 1380
   agagaaagaa atagattatt tgctgtaata aatacagctg aagaaagaat atcatgaaaa 1440
   ataagcaact ctaaatattt cctaaagaaa atttagagta tttcacatat tgaaatatat 1500
   agatttagaa atatttttat gatagcataa tatttctaca ttttctctgt aggtgatatc 1560
   ccaagcaaag tgtatattac ctcttcaaga aaggagattt tggccatgac tttacgtgag 1620
   gtataatgac gtatgtgtct tgaaaggaaa ttgattaata tacctattcc gaaagacatc 1680
   tgaggtgcta gaattatact tccctccttt ctttttcttt aaagaaaacc agttctattt 1740
   cattctgaca tttaataaca caagtcgttg cacatcagta cccagggcag aagagttcac 1800
   atggatatta tttttcatgt aaaatattga gattcaagct ttgcacactg tcatcagagt 1860
   tggatgaaac atctgttttg cctgatgtag aaataatctg ttaaaaccag gccctagaat 1920
   ggaaggacgc tcataccagt aactctttgg cacatgttcc tttcttactg caaatcacaa 1980
   acactgagca tccaccacat g 2001
<210> 193
   <211> 2001
   <212> DNA
   <213> Homo sapiens
<400> 193
   acttttatgt ttttatatgg ttaaagtaaa ttcaaagaag gataatattt catgacatgt      60
   gaaaatgata tgaaactcaa atttcaatgt ccgtgaataa agtgacgact gtttttgtca     120
   ttttatatat atatattttt tttgcagagg gactggcttg cactttttaa acattagtta     180
   tattattctc agtacaaccc tataagataa aattcattcc cattttactg atgagcaaac     240
   tgcagcttat gtttcagcca aagtcagaca gttcattaag ggcagagtca tccatggcaa     300
   tctgcctgtc cagatattac actttccccc ggggtaaaag cactggctct aagaatagtg     360
   agaacaaaac atacttataa aagggagttg ggggctggga atggtggctc acgcctgtaa      420
   tcccagcact ttgggaggcc gaggcgggtg gatcacttga gatcaggagt tcgagatcag      480
   cctggccaac atggtgaaac cctgtctcta ctaaaagtac aaaaattagc caggcgtggt      540
   ggcgggtgcc tgtaatccca gttattcggg aggctaagac aggagaatca cttgagtcca      600
   ggaagcggag gttgcagtga gtggagatcg caccattgca ctccaggctg ggcgacagag      660
   cgatactctg tctcaaaaaa aaaaagagag ttgggaagtg ttttgaatgc ttagagggca      720
   gcttgggaac tcaattagaa gaagggacgg tttaagcaga catatttcta agagacttat      780
   atgagttaac tcatttgatc ctcagcactc ctccatgagt tggatgctat tattaacaac      840
   ccaatattag ataaaggatg accttcttaa agacctcttc ccaggaaaaa gactgtgtgt      900
   gggtactctg aagctttggc tcttctggtg gggacaccac tgggtttcca aaggctcatc      960
   cagccaagcg aagtatctat gacctcgtcg tgttcagcgc gcttagggaa ggattctatg     1020
   gactgcactt ttaccctggt gtcctgcatt cttgctccaa gacttctctt accactctca     1080
   ttcccctaaa tgcagcttct gacaccttct agctacgagt tcttgggctc ctccttcgtg     1140
   acttcctttt aagataattt ctgagttaca acaaatcagc atttgaagaa atgcagcaga     1200
   taagttgaaa attcaagtgc tgaggaatgt aaaggcccaa aacaatcccg tagaaagcac     1260
   agaggcagat gacagtcttc cgttaaagat ttcactgctg tagatttctg agagtgctgt     1320
   gtagctgtcc tctttatcct ggtgtttgcc acttcgaaca gtcactttcc cacttggaac     1380
   ttacttgtgg ctttgctgac aaaacaggaa gtgatttgaa tacagtttgt ttttatgacc     1440
   tttcaacctc aggaatttga aaagttttag cagtggattt acagccttgc ttaatacttc     1500
   tatttcttac aaaacaaccg tataggggtt aaagtatcag tgcaaagtcc cgttcaaata     1560
   ttgactcagt aatgagctct acaagccagc ctttgtggca tgttaaattc aaaacaagct     1620
   tagcattttt tttttttttt ttggatagaa actggtgggg gtggggaaag aaagagggaa     1680
   ctgggaagag aaggagggga acttaaacct tgcttcctgc tctgtctttc tcagaaaacc     1740
   aaatatggca tcttccatga ggagcttgtt ttctgaccac gggaaatatg ttgaatcttt     1800
   ccggaggttt ctcaaccatt ccacggaaca ccagtgcatg caggaattca tggacaagaa     1860
   gctgccaggc ataataggaa ggtaacaaaa gggacgttgt tgtcaaaggg acaagcctca     1920
   gactggctgt gcgtcagtga tagatgggtc tcgctcagcc tcgcaggctg ggcacaggga     1980
   taagggcgaa ttactgatag c                                               2001
<210> 194
   <211> 2001
   <212> DNA
   <213> Homo sapiens
<400> 194
   ggcaatgaat tcagggccac ctgatggaaa acttgccttc ctctgtcccc aacacaacga 60
   ccagtacctc cctgacccac ccgctttgag aacctcggct tcacgcagtc tcacggcatg 120
   ttcttcccat ccaagcgcac cacaaacgca ttcccggcta ctccctcctt ccccggggac 180
   tccggcgcct ccataactcc tcccacctcc agtcccagct cctggaggat gtcagagacc 240
   cccagagtct caggagttcc tcagctccag ggccctttgt tccttctctc atcagccaca 300
   tgtttccaca ggtcccctca gccttggtca tcaccgggca ctgtcccacc acccagaaca 360
   acgttcagaa actcccctct ctctgaaccc aacctccctc ctcaacttcc acggcgttca 420
   ctcctttttt ctctcaccgc atgtcctccg accacacgca ggcctcagtc acctcctcct 480
   gcctcggtca cccccctcct gcctcagtca acccctctcc tgcctcggtc acccccatcc 540
   tgcctcagtc agcccccctc ctgcctcggt cacccccctc ctgcctcggt cacccccctc 600
   ctgccttggt cacccccctc ctgcctgtgc tctgcctcag ttaccccctc tcctgcccat 660
   gttctgcctg ttaccccaac tcctgccctt ggtcaccctg ctcctgcctg tgctcggcct 720
   cagtcacccc cctcctgcct cagtcagccc ctctcctacc tatgctctgc cttggtcacc 780
   ccctctcctg cctcagccac cccctctctt gatttcatcc tcacccagct tggatgtcac 840
   tgtccctcat ttcagctgat ttcctcctcc ctacccccca ctgtcatgcc agcccagcag 900
   aactccagcc aggcgtgact tcaatcatcc acctgctcca ggggacattc gggcagacca 960
   ccctgtgcaa aggctgtgac gttgccactc tggaataaca cggtcaccaa cgtgtagaga 1020
   accctcagcc tgcagggcga tcccacaggt cagcatggct tcccactcgt caaacctcct 1080
   ctcctctctt taaacttcac acctcctccc ctgtcccctc tgtggtaact gcttcctaac 1140
   gagcccttga cgatcccagc ctcccggtgc tcacaccctg tgtaggcccg acgcacacac 1200
   ggaattagag ctgacctgtg tgaccagcag aatatggctg aggagacagc gggggtgggt 1260
   gcaggggtga ctttcaggac caggtcttgg aaggcactac agctgcttcc gccttggtct 1320
   cctggatgtt gctctagagg acaccagttg ccatggtggg aagacgctca agtggccctg 1380
   tggagaggcc tatgtggaga ggaactgagg cctcccaccg acggccagct ctgactcgca 1440
   ggccatggag tggcccccgg tacccgttga gccttcagat gatgcagacc cagctgacag 1500
   ccttctgcaa gtttccagcc agagccttcc agccaagctg ctcctgaatt cctgaaacac 1560
   aaaaacagtg agagacgata catggtcatt gtttcaagtg actaaggttt gagatcattt 1620
   gttaggcagc aagagatgac aaatcatctc attttcagca gacaatcccg atcctgcttc 1680
   tcagggtaaa gatcatgagg aggaatctac tttcctgtgt tgctgccagc actgcaacct 1740
   gtgccccacc cttctgcctc ctcccatcgc agacaaggag ctgcctgcct tctccgcagg 1800
   gcttgctggg cagttctggc ctggggtcca ccctcctgct tttgaggagc tgtctgtgct 1860
   cgaagctctc tcctccgccc tttcccttct agctctcccg tgctgctaga cccttcctca 1920
   tgcccctcaa gcctgatgtg gcatcttctg tagtgcgatg agcccatcct tggggtacag 1980
   ctttctttcc ggttactgcc c 2001
<210> 195
   <211> 2001
   <212> DNA
   <213> Homo sapiens
<400> 195
   gtgtgtgtgt gtgtgtttta aggctccccg ggtaaccgtg atgctgacca gctttaggaa 60
   ccactgtcaa tgaaagcaga catagtacaa ggtctaggaa gccccaggtt acgtgtatat 120
   aggaagaaat atatcaggtg aagccttacc agtcccctgc caggggcctg cctttgctct 180
   ggcgttggga gtctgcacac agaatgtgga acttggaacg cacgctggaa cctggcactg 240
   ttgcttcctt ctttgtacag atgacccttg cagtctgacc aggcagcttg aatccaaaga 300
   gccaggctgt cactggggaa ggctggccaa gtctgccaag aggaggctaa aagggatttc 360
   cttggcaccc ggggccatga cgggggttat ccttgcacac tcagaatctc agttaagttg 420
   gcacatggta gcctttagct ctggggcggg ctcaccttaa caggtatgca tggtgaccca 480
   ggcagccagt ttgctaattt actaatgaaa aatacagctg tcatgttggc ggatcatggt 540
   ctcctttccc tctatgtgag ttgcacgtga agccaaatct gaaggttgtt taataagaaa 600
   ccagcacaag caaaattcac cagatggtct ttaagagagc cacacccaca acaggggatg 660
   ctgtgttcca gctcatgagg gagggcatcc tcttcatgcc cagacagggc ctaatgggca 720
   tcaccagctt ccacggtgtc ggcgtggact ctgcagtcag actgacggaa ttcagatcct 780
   ggttctgcac ttaccatttg tacgactttg gcaaagcagt atacccctca agccctcagt 840
   ttcttcatct gtataataaa aataatagca gctacatcat tggattattg taaggattaa 900
   atgaaagaag tgagacgttt aacacaatgt taggcatggt taatcttcat gtctggtagt 960
   tattattatc atcatgacca ctactactat tattgtcctc gtctcctctg atttaggcct 1020
   cactcttcat catagttgtt ctgtgtgtac aggataccta tgaatgatta ggtttccact 1080
   cactaaagtg gaaacatatt ctagtcttgc ctgtttgtaa taaacttagc agccctgcct 1140
   ggagttggtg accctcctct ccacaacact tcatggtacc tttgcccaca gtctcaggtg 1200
   accagtcata tcctttgtga tggtggtgga agctcctgaa tcccaggatt ctgaactggg 1260
   gatgaggtgg aagtcatctc acagcctgtc acacatcctt gtgcctgaat ctgcatcctg 1320
   agttcttgtg tcccctccct gcactcccac ttctaaatag ctcattagtg ctgggacatg 1380
   aaggcagctg cagggcaggg cagggttaaa aggcagggat tgagcaagtc cctctgcagg 1440
   aaagcgtgtg cccatgtaat gtgtctagca gatgtaactt ctcgtctgtc tgggatggag 1500
   attatgagcc cacatgggcc tgtgacttga gcaaaacaac aaaacaaacc aagaaatgtg 1560
   aaacgcacat gcctgtcctt atacgagggc tcagacactt ctaccctgct gaagcaggtg 1620
   gatgctctaa gtggcaccca ctccgcctgt gctggggaca gcagaagggg ccctccaagc 1680
   cagactcggc tctcgtgaac ccttcacgac tggggcctcc ccgctaaaac agcctcattc 1740
   ccacttaaca gctttggctc atgctgttgt cttggctata atgcttccct ccacttaata 1800
   aatcctgccc atatattcag gcccagttta agccccacca ctccttgatc tctggactcc 1860
   tcctccaaat gacagcatgg tctgttttcc agatacaatt cagagctgga ttcggtgttt 1920
   gtttttccag ttatttttgc ttcctctagg aaacttcttt gaaaatgaga ctctccttcc 1980
   ctagcagggg ctgggtgcag a 2001
<210> 196
   <211> 2001
   <212> DNA
   <213> Homo sapiens
<400> 196
   acagatttct ctttcccttg ctcaaatcag cattgcattt tcttcagcaa tgaagtatca 60
   tagtatcaga agaaggaggg gaggtcctaa aggaaaactg aggcattaaa gggaatcttc 120
   tttggagaca gaaagtgttc ctttgatcct tatccttcct gaattaccat ttcatctgta 180
   ctggttttct gtctaagttc cattgctaac caggttaagt aaaggaattg gtcacctcct 240
   cgggcctcag tttccacatc tgtaaagtga agggtctaga ctgtggtctc tatattcact 300
   ttctgtctgt agattgctca tatttctgca ttccttctat tggctgtcca tttccatagc 360
   acttttctgc attgcttctt gtacttttca cctcttattt ctgaattgtg gtcccactat 420
   tttactgaaa ttaacctctc agacatcact gcacatctct aatcatcatt tcttttattt 480
   tttcccctat accctcttgg ttacattcaa tactgacaac ctcagtctga tgagtctgat 540
   cgagtttcag acagttgctt tctaagaaat tttctacatc tcatgttgct ccccttttct 600
   cttccactta tagctcctgt gccctacctt tgtcatttct tatagttttg tgcttggtat 660
   ttacgactct ctctcttttt tttttttttt tgacagggtc tcgttttgtc acccaggcca 720
   gagtgcagtg acatgatcac agctcactgc agcctcaacc tcccaggctc aagtgatcct 780
   cccacctcag cctaccaaat atctgggact acaggcacac gccactatgc ctggctaatc 840
   tgtgtatttt tttgtagagt tggggtttgc catgttgtcc aggctggtct tgaatgcctg 900
   ggctcgacag atcctcccac cttggcctcc caaagtgctg ggattatagg caggagccac 960
   ctcactgtct taatgtaaat tttcccgcac tgacttgtcc gttctgtttt actacttccc 1020
   tctttcccat ctctttgcct ttgttcatgt tattctttcc acctggtttt cttcttcaga 1080
   ctaaaaatta taacaaatca tttgagatct tgcatttgcc aagaatgatt gacatcactt 1140
   tcaattacct agattgaaaa tgaagttggc aaaaatataa attttcatga cttaataaat 1200
   gagcttgcgg aaaaaaagcc agaaagattt gtcaatcaat ttaatacatt attaaagtat 1260
   tatggtttat tgttttacat aaaattagga caccaaaaca ttatttcttt gctgtttgta 1320
   gtttatgctg ttatatatgt gtacccgtta cctcattaca tgttataaat aataagactt 1380
   tttagaagaa aaagatttat attttactat ttcttttttt ttttttttga gatggagtct     1440
   cgctctgtcg cccaggctgg agtgcagtgg tgccatctcg gctcactgca acctctgcct     1500
   cccgagttca agtgattctc ctgcctcagc ctcccaagta ggtgggatta caggtgcctg     1560
   ccaccatgcc cggctaattt ttgtattttt agtaaagacg gggtttcacc atgttggcca     1620
   ggctggtctc aaactcctga cctcagacca cccaccttgg tctcccaaag tgttgggatt     1680
   atagacttga gccaccgcgc tggacctatt ttaatatttc taactgaact ttttttctga     1740
   cttttcaaca ggagacctca cattttcatt ttgcactggg ccttgtaaat tatgtagcca     1800
   gcccgaattg ttgggtctct ctgaggaagc tgaagaatct cagagaaaat acctccagat     1860
   agaattgttt gagtgttctc cacaaaaagg gagctcactg tacaaccact ctggaaggaa     1920
   acacagacac ttcacacaaa gctttcaaat gtttaaggtc ttactcaaaa cgaacataat     1980
   ttaaaatatc acgatacatt g                                               2001
<210> 197
   <211> 2001
   <212> DNA
   <213> Homo sapiens
<400> 197
   aagcatttca gactttagtt ttggtagtac aactcttgta accattcaag tgctgctttg       60
   ctgtcatttc aactcttaaa agaagggaat gggggagggg caagagagtc ttctaagtaa      120
   acaaagatcc caaatccatg aaacctagga tttatcatgg tatgtacatt atgaaattcc      180
   atatattgta aactgtgtag catgtcaaga tttcacatta gcaggtccta gtgtactctt      240
   caatgcacat acggttttta tatgcatatt aataaaggcc catctttggg agaagaaaaa      300
   aggctccctt tcaaatgaaa tcaagcccag gtaaagctga aacccaaccc acagagtact      360
   ggcaccgttt tcaggccaaa agaagacaag ggtttcaatt tcatcatctt gtaatgctga      420
   taaatgcgat gctgtgactg gcaaaatttg cagattacat aggtctttct aaacgtgtgt      480
   aaatatgcaa atatgtattc caatttatgt gcaattccaa acaatttgct gtttgaaatt      540
   aaagaccaac aactcttctg tgagacaata attcgggtca tttacatgaa aagtcttaaa      600
   cagcaaaaat gctacttgca atttacatca tacagtgctc ccatcacaga gaacaatggc      660
   ttcctcaatg gaagttaacg cagtagagag gtatactgag attttttttt taacttttca      720
   gcacagtttg caaatactaa ccaatgaatc aaatttaaaa taaaatagaa atgtttctta      780
   tgcttggtca tcactgctac ttgagtgtta tttttgtgtc ttcagattcc tatcaaacag      840
   gagtgaacct ttttaacagg ttatggtgac attttttcag ctccttccat tttgtcctat     900
   ttccagtgtc agcacaatta gcttgacttg ggggtttctg tctggtttta aaaacctttc     960
   aatggagtgc acgtctgtga acaataaagg aacgaggaca cggggctggg aaaacagcat     1020
   acctcatctg gaatggaaat ctatcgtctc tcgcccttgc acgggtcacg attatacacc     1080
   ttcgtgacct ttagcgggct tacatatgta tctttttgtg ttccgcaggc tatgaccacc      1140
   tgctgtatgt acaatatgtg cttgttacat ttgtacacca tctcccttct agaatttaaa      1200
   gacctagcta ttacatcttg tttactcaat atcattcccc cattaaggaa aaaaaaaaac      1260
   ctacatgaac tgaacatgcc atgtttcaat ctggccccag tggctttttc tctgaaagca      1320
   aacgtgtgtc ttttacacca gggctttctc cccaccccag ggggtgtctt ccatcctttt      1380
   gtggctcagt tgaaggcgaa aagggctcca aaccactaac taaccagagg agagcccctt      1440
   cttccacctc cagggagaat ttcagattta atttgtccga agatagcgtg ctctcttctt      1500
   actcatttgc catcattacg aggaaaacaa accaccacct tggcttcaag atcctgggta      1560
   gaggctcacg gtcttttcaa ccatctttgg cgaggccttg cttccttcca ctcgagtaag      1620
   tcgcggcttg gggccacgga aaggactggg tgagagcccg caatgtcgtt ggaaaaaagc      1680
   agccctccct gcgccccctc accagcccca ccaccccgct ccactcccgg ggcgaagccg      1740
   cgagccagcg gccccgtatg ggcgagactt ggcaggtccg cggggcgcct tctcgccgcc      1800
   gccggactcg gggctccgca gcggccgggc gtcggcgccc gccggccgcg gggcttcgcg      1860
   ctgccgccgc ccggtcccgg agctcccttc ccccaccggc cgccccctcc ccctcgcccg      1920
   cgctcccttt tgttcgctcg ccgcccgggc cgagccgccg ccgccgccgc cgcccggccc      1980
   ggcggcgagg ccgctccacc g                                                2001
<210> 198
   <211> 2001
   <212> DNA
   <213> Homo sapiens
<400> 198
   tgtatgtccc attattgctg gccccatact agacatgtgt ctgcactcaa ttgatagctt        60
   tataatggat gaatgattaa gcctttttat ttaaagctag ttatgttccc acctgagagg       120
   ctattcgcag gggaaaaaca tttgattcaa tcctctttaa gaaagcagtt tggccgggtg       180
   caatggctca catctggaat cccagcactt tgggaggccg aggcaggcag atcacctgag       240
   gtcaggagtt tgagaccagc ctggccaaca tggtgaaacc ccgtctctac taaaaataca       300
   aaaattagct gggcatggtg gcgtgcccct gtatgccagc tactcaggag gctgaggcaa       360
   gagaatcact tgaacccagg aggtggaggt tgcagagagc caagattgtg ccactgcact       420
   ccagcctggg tgacagagca agactctgtc tcaaagcaaa aaaaaaaaaa agagagaaga       480
   aaaaaaaaag tagtttgatc agagagttga tctatgttgt tcatattatg aaaagacttt       540
   aaatcgtttt gtgagaatac aatgttgatc aacttatatg aattcagatt caagaagaga       600
   ctgcagagga tcagggttat taaatagctt cctggcagtc aaagctgttc tggatgcaat       660
   gggctgcctt agaaagtagc ttctgtccct agtattcaag ctaagaggac aatcacttgc       720
   ttggattatc tgcaacatct ctgccatcag atggattctc agactggata atctttaagt       780
   ttcttctaat aatcaatttt atgagtctgt gctgtgaaga gtggtgtctc aagaccaagg      840
   gaacaataga ggaagacgta ttcagctttg gtcttgtgcc aacatttcct agttttaggt      900
   tagcaaattc agaaatctgt taaatgttta ttgagtagct tctatggtcc atacactgtc      960
   ttaagtgctg gggaattaac agtgaccaag aattttagat cgtgacttca tggagctcat     1020
   aatgctaaca attgacattg gacaagtaac tacaagtttg atgaatctta ccaaaggaat     1080
   aattgaagga gcctataata aggcgatttg gcaaagttgg agtcaggagg gttggggaaa     1140
   agctatcaag gagagatatt taagatgaga ccttagttct tcaaagattg gtagaatctg     1200
   cttagtgaag aagataatga agtgagtagt tcttaaattt caatttatga gtataaaacc     1260
   tgagggatta gaattttttc ttaaaggtga accagatttc agtctgagta taaaacgttt     1320
   ttatgtaata aatctgttta acaaataaaa ttagatccag caatcccact gttgggtata     1380
   catccaaagg aaatgaaatc agtatgttaa tgaggtatct gcactccacg cttattgcag     1440
   cactattcac tagagccaac atatggaatc aacctaagtg tctatcaatg gatgaatgtg     1500
   taaagaaatt gtagatatat acacaatgga atactatcca tccttaaaca aagaaggagg     1560
   ctgggcaagg gagctcatgc ctatgatcct agcgctttgg gaggccaagg caggaagatc     1620
   ccttgaggcc aggaatttta gaccagccta agcaacatag aaagacccca tgtctacaaa     1680
   aaaaattttt aaacatgatc agggcatggt gatgcatgcc tgtaatcctt gctatatgtg     1740
   aggctgaggt gggaggattg ttgagcccgg gagtttgagg gtgcagtgag ctatgatcat     1800
   gccactgcac tccagcagag accacccaga atagagatgc atctactccc agccaggggt     1860
   aagagagagg gaaccagcaa aacttaacca gcaaaactgc ccttaacaga aaatagtgca     1920
   tcctagccat cctccaggcc cactgagtgc aaagccactc agctaggtaa gaagagtttt     1980
   gacacctccc catcctcctg g                                               2001
<210> 199
   <211> 2001
   <212> DNA
   <213> Homo sapiens
<400> 199
   ggtgttgttc ttgaacctcc cggtgtttga gagatggtgg gctgccctgt caagggcact      60
   ggctggaagt tgtggcttct actcctggct ctatgtgtga tctgaccagc tcatcaggct     120
   ccacaattag ctttctgcaa tgtgggagtg gattcttaca aaatgtagaa ctctactttc     180
   taccctgctc ggggtctgca tgaaagatgg cgggggtaag ctttggctgc aaaatggtaa     240
   atatttcctg agtgtctact gtgtacagtg cagtgaggct ggtttctgcc gtacatggta     300
   tatagttatc caacagacac tagctggcat gggctcttac tgtgtgactt taggcgagtt     360
   acttaacctt tctcttgacg ttcctttttt cacttttttt tttttttttt gagacaaggc     420
   cttgctggag tgacaagctc attaggttcc acaattagcc ttctgcaatg ttgtccaggc     480
   tggagtacag tggcacaatc atagctcact gcagcctcaa actcctggga tcaagtgatc 540
   ctccagcctc gggatttttt tcacttttat tattttattt gtttgtttgt ttatttattt 600
   atttatttga gatggagtct cactctgcct cccaggctgg agtgcagtgg tgcgatctcg 660
   gctcactgca acctccgcct ctcaggttca agtgattctc ctgcctcagc ctcccgagta 720
   gctgggacta caggcacacg ccaccacacc tggctgattt ttgtattttt agtagagaga 780
   gggtttcgcc atattggcca ggctggtctc gaactcctga cctcaagtga tcctcctgcc 840
   ttggcctccc aaagtgctgg gattacaagc atgagccact gcgcctggca cttttaaaac 900
   agagacaata cttacctcat aggtttattg tgagagtggc aacagcttgc aatgggatga 960
   ggcacatggt aaggtgctca atatgtgtga actattataa cggtcatagt taccattaag 1020
   ggaagaccta tctgtgctac attaatgggg gataataatt actttattcg ctgggcgtgg 1080
   tggttcatgc ctataatccc aacactttgg gaggctgagg cgggaggatc acttgaggcc 1140
   aggagttgga gaccagcctg ggcaacatgg caaaacccca tctctactaa aaatacaaaa 1200
   actagctggg cgtggtggtg ggcgtctgta atcccagcta ctcgggaggc tgagaattgc 1260
   tggaacctgg gaggcagagg ttgcagtgag ccgagatcac gccattgcat ttcagccagg 1320
   gcaacaagag caaaattctg tctctaataa taataataat aacaacaaca acaacaacaa 1380
   caacaacttc attcaaccaa atacattgta catctcacct ggcctgccca taagcaatgg 1440
   agacactgaa ataaacacga ttcctgccct caaggtctaa tggaggaaag agatgtacac 1500
   agctattcta gagggaaatt gacatgaaca gaagttctca tcctgtgctc caggatcaca 1560
   ctggaggagg gagatgctcc cagagggtct tgggaaggct ttccaatctg gagctggaag 1620
   aatgtttgcc caagagatgt acacagttat tctagaggga aattgacatg aacagaagtt 1680
   ctcatcctgt gctccaggat cacactggag gagggagatg ctcccaaagg gtcttgggaa 1740
   ggctttccaa tctggagctg gaagaatgtt tgccagagat gaaaggaggg gaagggaatt 1800
   ccgggcagag ggaagggcgc aggcaacagc taggaggcgc agatgcaggc tgggtgtgga 1860
   gggtgcgagt aggctggagt acaccaggga gttcccagca ggccactagg agccacccag 1920
   gatgcccggc aacctcatga gtcagacaag cagggctgtg tctatctgga agtttccagc 1980
   caagctaggt aaaacacatg g 2001
<210> 200
   <211> 2001
   <212> DNA
   <213> Homo sapiens
<400> 200
   gatccccggt gagaaagtgg gggccacttc tcttcctcac cgtcctgcct ggggttccag 60
   agctgataag agggacagat ttcctagttc tgagatgcgg ggatctgctg agtcctccct 120
   cagggctctg ggactcctcc ctctgctgac ctgaggctcc actcctcaga ccatagccct 180
   ctcctcattg acaccccaaa ggcacagcaa agacacatca catgggccac cgtgcctgaa 240
   accacccagg gctgaggtcc ccactgatct ggactccaag gtcccttgag tcctttctct 300
   tcttcctccc cttgactctt ggctgggttg ttctcctcta ccacgccccg ccgcagacct 360
   gagtcaccgt cccttggatt cctcaggccg agtgaagaga catctcggtc tgagacagag 420
   gtggggtggg caccctgtcc tgggatccct tcatgcctcc ctcatttccc agcagggccc 480
   gggccttccc tctgctcccc tgaagccgca ttcttggtac caatatcccc ttccctacgt 540
   cagcctcaga tgccgacgtc agaatttgcg ttcctggtcg ccatgaccag ggcttcccag 600
   ggccgacatc aggggcagag ccctccctga gcgtcccgac attgatgctg gcagaaccta 660
   ggcgcggctc ccgccgaacc agccgggccc tggtcacacc aagctctgac tcccagagtc 720
   ccctgcggct taagcggcgg gagagagtgg gggcgggggt gggggggggg tgacagccca 780
   gcctgagaag tctgccctca ggtggtccag agctggcagc agaggcagcg ctgtattatt 840
   tagggctttc tatctgggtt gggggcgtcc tcagtcctcc ctcagcctct caccttgcct 900
   cctcacagag cctgggcccg ctctcttcag ccgatctcaa gcctgtccct cagaacgaga 960
   acctcgcttc tctctgatcc ccgaggcgta agttagtggc gtcacatccg gtccccgggg 1020
   cttccctggg ttgacagcag gggcggagtg gattctgccg gaactggggt gggagttggg 1080
   gtgggagtga gggtgggagt ccgggtgggg tggggtacgg gtgaggatgg ggttggggca 1140
   ggggtggtgg tgaagatgag tgtggggagg ggcgtggagg tgggaactgg gatggggatc 1200
   ttggggctgt gggtcgtggt gggggtgggg gtggggtctt tcagtcatcc ctcagtgtct 1260
   cgaccttgat gcctggaaaa gcctggaccc ctgttttgtg ctgaggatgt cttctcccct 1320
   taaaccaggt ctcttgactc ggagtcttcc aaggtggcag tgaggggagg gttgtggtag 1380
   gggtgacaac attgccaggg tcttccaggg ctgacaagag gggctcagct ggattctgtg 1440
   gcccctttgt gaggggtggg tggaccctcg gtcctcactc aggagggtct tcctcctggc 1500
   ttttgtctct ttgatgaagt gatgggtggg agatgctcag tttccgtcac ctctgatcat 1560
   ttgcccagct gcatcccttg cagagcatgg gtgcggttcc caggccagat ggtccctggg 1620
   gagctgcaac acccgtgatt gtaatgacct atgggagaag acgcacacct tcccaacggg 1680
   gctcttccca agccacaagt agacttggca aactttttgt cctaaagggt atattttcta 1740
   caagaaagag gctgagcatc ggggtgtgga gaggtcctgc tgttttgagt ttagctgata 1800
   ttttatagtc caatgcagtg atgtctggtt ttctaaccct cattcctagt aaaaatatgt 1860
   gttttaaatg tctgccaagt gtatattgaa tacatatata acatttcatg aaagaatacc 1920
   taccttacgc tgtgtggttc actgcttatt ctatttctgg tctattcttt atattctatt 1980
   ataataattt ttctcttttt t 2001
<210> 201
   <211> 2001
   <212> DNA
   <213> Homo sapiens
<400> 201
   tttatgggac aggatcacac acagggtggt gctactttct agagccaaat ctcaagtaag      60
   gtgtccaggg gtcagttgca acagaaggca cattagccac agcagtctca gagaacactg     120
   ctagaagaag aacctacaca tggccatgca atgaggctga ggggcaggcc taagtggaag     180
   cagaccaagt aaagaggtca gagagcccat ggaaggagaa gagggaaaga cagttccaag     240
   agtaaagcag cctaggttaa ggtgcagaaa attcagcatg gatactttta agagagctgt     300
   gcaaggctct ccttacctaa aatcacactc cgtatacaga catacccaca aacaatcaca     360
   tcatgttctt tgctcaagat tgaacttgct gaaactgtga tgtgctacta ctcctaccgg     420
   gggtagaggg tagtctgctg ctgtacttct cctctctggt tactgatgca attatctata     480
   gaagtataat gccgctctct gtaagcaatt ttaggtaaaa tccatgggat attttcacag     540
   tgagctagcc tacagagagg gcaaaagttt ctgggttctt aaagcagctg ccgccaatgc     600
   aagctctgta caggaggaat ttcttttttt tttttcgttt tattattata ctttaagttt     660
   tagggtacat gtacacattg tgcaggttag ttacatatgt atacatgtgc cacgctggtg     720
   cgctgcaccc actaactcgt catctagcat taggtatatc tcccaatgct atccctcccc     780
   cctcccccca ccccacaaca gtccccagag tgtgatgttt ccctacactc cacctcatgc     840
   acaagcccaa atatatcaca gcttctctac agaaatgcat aattcactgg cgaagttaat     900
   agacattggc caagaggatg atctcctcac tatggaatca gctacagtct taggtagcat     960
   gcaaactcca gtctgactcc aatcccttat atcttccatc gtaaagtatt attgccaact    1020
   ttgtttcttt ccaatttttg tatgttctgc agtttatgta aaggctagca aaaacatgaa    1080
   tactttatta tacatatggc aaatagtctc aaattgtaat gactcatttt tatcctctta    1140
   tactgttgta aaattcagaa caacatgtat tcactatagg agtatgaaaa ttctttttac    1200
   caatgtacat tttaaataac attgtggtaa agagaagcta catatttcac attctgattc    1260
   aactcactgt atccaacgag taaaagatcc tagaaagaat cttacatttc taaaattctt    1320
   atagactaag agtgagcgat aggaacaata aaaatatttc tgtaagtgct cttggtaaac    1380
   agtcttaaag tcagaagaac acttatccaa aatgccctac attattgtgc aatcaaagta    1440
   gataacttta acttcaaaaa caactcagtc ttccaaccca attcttaaaa ctctaagctc    1500
   ttcacaaaaa atttttagcc aatttaaact tatttctaca ctcagttatg tttaaaacaa    1560
   cataatgttt gcataatttt ttaaaaaaca ttctaactca atgtttttat ttatctaata    1620
   ttaattacca gatataaaat ctttaaagta gcccaatatt tttcaaaata atttctgcat    1680
   agatgattgt cattcagttt ataaaggttt atttataatt ttgtgatagg aataagaaaa    1740
   gacaaaacct ctgaaacaaa ctcacacaca aaagcaaaat acaaaacaaa acagaaaaaa    1800
   tacagtgaac ttacttctgg gaaaatggaa gcaaggaatc tagaggtatg gtttatccac    1860
   tgagctcctt gtttagaaga aagttattca tattttctcc tggtctctac catgtatcta      1920
   gaatttgctt taactatttg acttctgtga aaagtttctg tgaagactgc tttcagagtg      1980
   gaggttatga tctcaattct g                                                2001
<210> 202
   <211> 2001
   <212> DNA
   <213> Homo sapiens
<400> 202
   gtgttcctct tatttgttct cccagctgcc aggtgaactc ttgaactgca aatcacattg 60
   ttttcctctc ctgcttggaa tctgctcctt ggggcccact gtttgtggaa tggagtcctt 120
   gctgtgtcta cccaacccta ctccactcac ctctctccta tgtcaactta ctgccctcat 180
   ccctggcctc cactcattct cttcctaccc tttgcccctg ctgttctctc tgaacaaagt 240
   tcagctccct cgttccctgg gtgctctagg gttagggaca agttcttatt tgcttacatg 300
   ttcctcccac aaggcctggg aggccagctc tgcatttcct agcttgcagc aggtatgttg 360
   taaatctctc cggcaggctg ccatgctttt gatccccttc cttcctgacc tcaacctgac 420
   agatgcactg ctgttattac cattttatgg agaggatgtg ggccttggaa agggaggaga 480
   ctttataagg gtcatgcaac cacatgtagc aaatgattca tatgaggctt tcaaaccaat 540
   acccccagca gggctgtggg ggtgctggca aggggcccca tcctctgccc cccggagtcc 600
   tgcaagtgcc cctagtgcgg ctcctctcgt cctgccttac ctcttttcct tgctctgctg 660
   ccactgcctc cttgctgttc tgaatgctcc aggcctccct gccactgcca gcatttgcta 720
   gggcatctcc atctgcccag aacacccttt cttcaaatgt cactagggct ttttgcattg 780
   aacctcattt aaccttaaag aggccaccct ctttaaaacc ccaagccccc tcccccggca 840
   ttccctgtcc tcctcactct gctctccttt ttctcttttc acttatcccc ttctgacatt 900
   tgagatcatt tactcatttc atatgttttt gtttacttcc atctccctgc tactaacaca 960
   gaaactgcaa gagggccggg atctttgtca ctgtcatgcc gtgaccctaa cacttagtgc 1020
   agggcctgat ttgcaggagg tgcccagtga agaaagcctt gctcagggtc tctcggagct 1080
   gcggacaggg tcatcatggg ggcagccctc tcctctgggg tctcccagtc cctccctgcc 1140
   caaaaacctg gctcacctgg ctcccctctg tcctgtgtcc gcactgtagg ccgggctggc 1200
   ctctccccgg ctgtaggccg cagcctctgg caggcttgtg gtcctgccct tctctcctcc 1260
   cacatagggg ctggaacaca gaagctgaga gaaactgctt ggccagctct caagcccttg 1320
   tctgctctca agcccttgtc tgctctcaag cccttgtctg ctctcaagcc cttgtctggg 1380
   cttatggagc tcctctcctt cctccctctc ttcctgcatg ggtgtgacct ttgtgcctgt 1440
   cgctggggct gccctgggag gacttctggg aggttggagc ccttacatct caaagccagg 1500
   gattcctttg acactacttg taccaaaaag ctgtgtaccc cagcagtgtt tccaaagtga 1560
   tatcctggtc ttccctgatc tttcactgtg tgcccttcag gcactcaagc tgttaatatc     1620
   acacgagctt aataggctcc cctgagatgg tcctcatgtg aaaggattgc tctgtaaggt     1680
   gtacgttttt cctctctccc catgtttctg ctctaaaata tggcttgtct tatgcttagc     1740
   atcccctgac attccctgga aaaatatttg tgaagcatcc atctgtagtg tgtgggatcc     1800
   agatgagagc tgggtcttag tgagaggaat gagaggctta gaggttccct ccctgcataa     1860
   ccaccttgga aagtcagatg cggctcccca ttttgcaggt aaggaaacag gctcagggag     1920
   ggatgtcact ttgtttgggt ttgcacaata agtagattac gtaacacatt tgtgtaagac     1980
   tgggaaagaa atcaaggggc c                                               2001
<210> 203
   <211> 2001
   <212> DNA
   <213> Homo sapiens
<400> 203
   tcctatggct aaagtgctca aaggggaaaa aggagtttca aaaatgttct tggaaatacc      60
   atctctcacg aattcttcgg cctctgctgt cccaatgtca cttgtctgag atgtaaacag     120
   aggagttctg agaaagaagc tgaacttgca tttctccctg tttctatttg ttccaaactt     180
   gtggcatttc taacaggatg aagcggaaga gaaagggaaa gagacaaaag tgtagaaaga     240
   tggaagatcc cagctgcaaa tggccatttg cagttagatg gaacagctgc tgacgttcag     300
   ggaaatgcat gtctctcttc agatgggaag gagcagtgga aaggggtgac gagttcctgg     360
   ctggccacca atcatcccat ctttctgtgc cggttcctca tctggaaagt gggagtgata     420
   cttgtgcttg cttttcctac ccacaaagat tattgtgaga gctataatac ggtgagatac     480
   agaatcctgc ttttaaaaat acaaagcaga atcaagatgt caataataag gatagtaatt     540
   gtgttagtta tctgcaatca tctattatag ctagtcgtct aggatcctgg atcgttctcc     600
   tggttttact acagttttgg atcagctcac ccccaaatcc cttgctgaag ggtggagctc     660
   tgtcagccat gggcagggaa ccacttcctc ttgcctttct actttctgtc tttcaaacat     720
   gcccagggtc tttgcacttg ctgttccccc tgcctggtac ctctctcctg tggcttgccc     780
   cagagctgat ccttgtcttt gtccacttct cagcgaggat ggcacttcag ggagcccttc     840
   ccttactatc gcagagagag caggccctcc ccagtcatgt ccaacccaga actctgtttt     900
   gttttcttca tagccctagc atcacagaaa atcaccctgt gcattcatgg atgtccacgg     960
   gggcaagggc tttgtgttgc ttaacccagc atcctgaacc gtgtttgttg aatgaataca    1020
   gaaccccgtt tgctctggga gagcacagaa aacagtcttc tatcatatat catagccagc    1080
   tgcaaacagc agatggcttc ccatatccca gagagtaaga accagagaga gagagaaaga    1140
   gagagagttt gggtctttct cctctgtgcc tgctctctcc agagaaactg gaggggtagc    1200
   agttagcatt cccccgctgg ttccaccaag cacagtcaag gtctctagga catggccacc    1260
   cctcacctgt ggaagcggtc ctgctggggt gggtgggtgt tagttggttc tggtttgggt      1320
   cagagacacc cagtggccca ggtgggcgtg gggccagggc gcagacgaga aggggcacga      1380
   gggctccgct ccgaggaccc agcggcaagc accggtcccg ggcgcgcccc agcccaccca      1440
   ctcgcgtgcc cacggcggca ttattcccta taaggatctg aacgatccgg gggcggcccc      1500
   gccccgttac cccttgcccc cggccccgcc ccctttttgg agggccgatg aggtaatgcg      1560
   gctctgccat tggtctgagg gggcgggccc caacagcccg aggcggggtc cccgggggcc      1620
   cagcgctata tcactcggcc gcccaggcag cggcgcagag cgggcagcag gcaggcggcg      1680
   ggcgctcaga cggcttctcc tcctcctctt gctcctccag ctcctgctcc ttcgccggga      1740
   ggccgcccgc cgagtcctgc gccagcgccg aggcagcctc gctgcgcccc atcccgtccc      1800
   gccgggcact cggagggcag cgcgccggag gccaaggttg ccccgcacgg cccggcgggc      1860
   gagcgagctc gggctgcagc agccccgccg gcggcgcgca cggcaacttt ggagaggcga      1920
   gcagcagccc cggcagcggc ggcagcagcg gcaatgaccc cttggctcgg gctcatcgtg      1980
   ctcctgggca gctggagcct g                                                2001
<210> 204
   <211> 2001
   <212> DNA
   <213> Homo sapiens
<400> 204
   ggtccccgag gggccccgac cgttggcagg agccgcgggg ttgcctctga tgcctggaca 60
   aggagggaga agcaatggga gtcattccat cccgaggtgc cacccattcc tatctccgga 120
   cgcctcttcc caaatcctgc ggcctctgac aaatgcctca caatgtctgg gctgacctcg 180
   aaaattcagg tacgaccggt cccattctac agcggagggc accgaggctc agagaggcca 240
   acggggctgt tttcgggtaa catctacctg atccagatcc cgcacgtgcc ttccccctgc 300
   cctagcctcc gtcccgcatg agagaggggg tacttaatca ccgggcttcg gtgccaaagc 360
   ctgcgtcctc gggcactggg cggacaccca ggccccgccg ccagccactc cagaaccggc 420
   ccaaaacacg gcggccgtcc atctcccgag ccaggacagc gcgcatgtgc gtccagaggg 480
   gcagacccgc gctccagcca gcgcgctgat tggtcagctg gagtgtcaat cggaatggtg 540
   accgggcggg actcagagcc tgaggaggcg gggcttttcc tccgcccagg aaggaaatgc 600
   agaaatgcct tcctatgctt ttagggagag cgctgtagca gaagaatttg atctgatcaa 660
   aaaacctgtc ttagcaaaaa acctgaagcc gcctttgcaa aaattatatt acaaaattat 720
   gacagcgaaa gagatctgat ctaaccaacc cgcagcttgc ctttaacctc taaacggtcc 780
   ttggtcattc ctgggcttgg gccaagctaa ctttgggaga aattagttcg tagtctaaat 840
   gataattgcc cttcccccga actaaactgg cttcatagag ttaatgaaag gccaccaggt 900
   taggagtagt agagggacct gaattctgct aagggttaga ggtaactgat taccagcggt 960
   tattccggag gtcacagtgt ttgcagcttc cccagctcac gattgtccaa cctgagattg 1020
   gcctttggag atgtctttcc aggcttttgc atttctgacg gccggatcac cccacccgga 1080
   ccctcaactc ttggctctac tagtcctgtg gcccctcgaa gtcaggagat cgagaccatc 1140
   ctggctaaca cggtgaaacc acgtctccac caaaaataca aaaaaattag ccgggcgtgg 1200
   tggcgggcgc ctgtagtccc agctactcgg gaggctgagg caggagaatg gcgtgaaccc 1260
   gggaggcgga gtttgcagtg agccgagatc gtgccactgc actccagcct gggcgacgag 1320
   cgagactccg tctcaaaaaa aaaaaaaaaa gaaaaaagaa aaagaaagta gaagaacaca 1380
   gaaaaaaaag aataatataa tgactgccca cattcctacc atcagaatta gtcattgtga 1440
   aacaatatat atatgtatag gtttcctgtt tttcttgttt aagaaatgaa agggataaaa 1500
   gtaaattccc ctttactgcg tgcttccagt tttcactccc tccccagtct ccactttgag 1560
   tttggtgtga acattttcct aatgtgtttt ggtttttgtt ttttggagac agagtctcgc 1620
   tctgtcaccc aggctagagt gcagtggccc gatctcgact cactgcaact tccacctccc 1680
   gggttcaagc gattcccctg cctttgcctc ctgagtagct ggaattacag gtgcggtgcc 1740
   cgccaccaca cccggctaat tttttgtatt ttagtgacgg ggtttcacca tgtcgcccag 1800
   gctggtctcg aactcctgag ctcaggcaat ccgcccgcct tggcctccca aagtgctagg 1860
   attacagacg tgagccactg tgcccggcca ctaatgtgtt tttttgcttg ctctgtcatc 1920
   caggctggag tgcagtggca caatcacagc tcactgcagc ctcaacctcc caggctcaaa 1980
   cgatcctccc acttcagtct c 2001
<210> 205
   <211> 2001
   <212> DNA
   <213> Homo sapiens
<400> 205
   ctgcaactct atataccagg caactagttg tcaaatgcta attactgtca tcatccttaa       60
   agacttgggc ggcaatgttt ccattatttt cacgatgggg aaacgcaggt tccagaggtt      120
   gagcaatggc cacgaagcca cactgctagg acctgagggt gtgagcttag ctgcaacctc      180
   cctctttggc ccctgctcat accaagccag gtgggtgaac tgagagtcca ccgagacgcc      240
   cagcacttca cagcccagct tgcggaagtc ctctgcacgg ttgctgaacg cgatgatctc      300
   ggtggggcac acaaaagtga agtccagagg gtagaaaaag aggaccacgt acttccctgg      360
   ggaggaggga cacagaggag ttagggccca gcttctctca tgcacggccc cgcttctacc      420
   tgggccccct ccgggcggcg ctcacctttg tagtccgaca gcttcacctc tttgaaggcg      480
   ccatcaacca ccgctgtggc cttgaagtca ggggctggct ttccgatgcg cgcgttaccg      540
   gaggccatga ctgaaagctg agacccccgc ccggtcagtg cgcccgggaa gacactttgt      600
   cctcccaacc caaggtcgcg ctgcgtgctg ggccctatga ctgagtcagc acggcggagg      660
   cgacagcact aaccctcacc ctcccgggta cccggtctac gaggcccgga ggctgccgga      720
   gacccgctct cctgaccaga gcacgagtga cccagccact gccaccagca ccccacgagc      780
   gagtcggccg gagggcggtg tcgcaagccc ccagcccaga aggagacagt gaggcccgca      840
   cgagcagggg gcgggaagtc gggcgatcag gcctgcgctg caaggctgtg gcctcggttt      900
   cccccgcaag gacaaaggag gccactatag cggatggaaa gcaaaggcgg ccagcactaa      960
   agtcaggatc gggccaaagc agcctgaagg gggtcctccc gccaggtgca ctccgggtgt     1020
   tcatacctgc gtgggcaaag gctagacgca cggacgatca cacgcgtgga cccgcgttct     1080
   cagcgccaag tgagccctgg gccgcgaagc cttttatgcc cggcccgaac caagacgtgc     1140
   acggcgcggg ggagggagcg gacgagcgga gcggtcgggt ccactgcgcc cgcaggtgcg     1200
   ggggagcgtg cggccgggtc catgcgcctg cgggcggcgg ggggagacgc gttgccttcg     1260
   gccgggacca ctgcacctgc ccgcgtgggt aatgcgcccg ccgcagactc cgcgcacgac     1320
   tccgcctggg agcgcgttgg gggccgttgg agtccagcat ggcgcggacc ccgggggcgc     1380
   tgctgctggc gcctctgcta ctcctgcagc tggcgacccc tgccctggtc taccaggact     1440
   atcagtactt agggcagcag ggcgaaggtg acagctggga gcagctgagg ctgcagcatc     1500
   taaagggtaa cctcaggcgg cggggatgac gatgcccaaa ggctccccgt tgccctactt     1560
   ctcctgctgg ctgcaaaggg accttcctcg gcgggctgca aatctccacc cagctccgca     1620
   ctggaggcgt ccccttgggc tggttacttt tctctaaggc tcggtttact cttggatata     1680
   ttgaaataga tactgcttta aagcagctcc caggggaaga gagggtgtgg gcaggacact     1740
   gaagctgtca ctcggggata ggtgccagga agggcagaga cagagaaaga agggggcggg     1800
   caagcttggg aaatcagaga gggtccgatt gcagaagacc cgtgaccagt gcagcgagaa     1860
   cgacaagcga aagtcccata tggaaagatt tggggaaaga atgtccaagg aagagggaat     1920
   agcaaatgcc aaggtgaaaa gtcctgaagg agaaatggaa agaggtcggt gaggtgtggc     1980
   agagacatgg gtgtgggagg g                                               2001
<210> 206
   <211> 2001
   <212> DNA
   <213> Homo sapiens
<400> 206
   cccactggga tcccctgccc actagacttg tccccatcct gaggcagcct gttcctcctg      60
   acccccgaaa gcttcccacc catctccctt cggcctgaag ccagcccccc tcctctctcc     120
   cttctgttta cctcccaggt ttgggctcta ttttctactc cttccacctc ttcctgagta     180
   acaaggacac tcccttcatc cacaccccat gcctcctgcc ttgcttcctg taccttcaag     240
   tagttggaag aggtcatggg tgagaattcc ttctccttgg cctagtctgg cccaggtctg     300
   gagagaaggc aatggagatg tgaagtggag gccaggtgtg gtggctacgc ctataatccc     360
   agcactttgg gagtccgagg tgggcggatc acctgaggct aggaatttga gaccagcctg 420
   gacagcatgc tgaaaccctg cctctactaa aaatacaaaa attagccagg tgtggaggca 480
   tgcacctgca gtcccagcta ctcgggaggc taaggcacaa gaattgcttg aacccgggag 540
   gcggaggttg cagtgagcag agatcgcgcc actgcactcc agcctgggtc acagaatgag 600
   actctgtctc aaaaaaaaaa aaaaaaaaaa aaagaaattg aagtggaaaa tgagctggca 660
   ggggtggtca ggcagggggt gagcaatgag ggctcctgag ctgaggaagc aggggcgggg 720
   ggaggatggg ggctagggag tcctcagaag ttcccggaga tactccctca ctggacagag 780
   tggggttccc gatgacgtta cttgaggcct gggagacggc ctctgcccct cactgctcct 840
   ccccctccta aggtcaacat ttatgtgtcc tagacctaga gaggtcccag gacacgccac 900
   tgtcccgcct tccccattgc ccgccccact ggccagtccc cacgcccaca cacccaaggc 960
   tgccccatct ggcgctgatt atcctgctgc tgccgccacc gctgctgctg ctctgcaaaa 1020
   ttcagctgct gcctctgtct tgaggacccc agcgcctttc ccccggggcc atgctgcctg 1080
   cagccacagc ctccctcctg gggcccctcc tcactgcctg cgccctgctg ccttttgccc 1140
   agggccagac ccccaactac accaggtagg tctcttggca tcttccagac agctccagtg 1200
   aacttcagac ccccgattcc tttggggtta tgcctggaga tattttgggt tcccttgctg 1260
   acacaccact ccccagattc cccaccgcct cacccttctc tggcccccaa atcctcccca 1320
   tctgctgcaa gaccttggtc tcctgcaggc caccactgct cccattcccc caccttcgtc 1380
   ctctggtctc ttaagtctcc ccacccaccc agatccttga ccccccatag aacccaagag 1440
   agagccaagc tcctgaactt gcctggtagc tggattccag gaaggaattc gagagggatt 1500
   gtagggggag gagggggcag tgagtggaga cttgggagtc agaatctgaa gtagtttgga 1560
   acaggggcag ggctaaggtg tggagctcgc ggagcctggc ctccaggttc aatcagaggg 1620
   ccatcgccac tgcagctccc tccatctctc caccccctcc cccccaccgc ctcgtggctg 1680
   tgtccacatc tggaagtagt taggcggctc ctgtccctgg actgccaggc agagtgaggc 1740
   gggagtctgg aaggggggcc gaggaggatt ccgggaattc ctgttggggt gggggtgggg 1800
   actcttgggg ttggagggat ggtcgagttg ccaggcccac ttttccctta tctttgtggg 1860
   agtctgggac accccagccc tgtcctggct ggtggaagct gccccaggtc tctcaaggcc 1920
   tttccatttc atcccccagc ttccctcctt ggagtttcat cctcatcacc tcatccccat 1980
   cttgccagtg ctcccccgtc c 2001
<210> 207
   <211> 2001
   <212> DNA
   <213> Homo sapiens
<400> 207
   tgactgagaa ggaatagctg atacagaggg gagggaattt ctgaaggagg gagtgaatca 60
   tcaacaccat cacatggctt agaaaagtgg accctggatt tggcaatgat ggacataatc 120
   caggtggact ttgagcctcc tcctgccccc agtggccact gcattctgca gccctgaact 180
   ctacctctac ctctgctttt cttctgcaaa atgaacctct tatatacaat tctgtttcct 240
   acatcagtta ctaaactttg aggcatttga gccaagggga cccgtactcg caccaagttt 300
   catcacaggt gtctaatgtg attacagggg cagcacggtg ccctgcacgg gaaagtactg 360
   gggtcagaga gaccagagtt caaatcgtga ctctttaccc ctgcgatccc tttggtgcaa 420
   atgacttaat ccgtctctgc ctcagctttc tccactgtta attgggcata atggacacaa 480
   caatggtgag gggctcttgc aggaatgaag actaggatga ggatgaggat gagggatacg 540
   tccataaagc ccctgcacac cttccatttc ctgagcactc ccaggaagtc tttgctgacc 600
   acgccaaatt agattatctt tgcttagcca ttaggccgct gccctatatt gggaggcaaa 660
   tatacctctc agcttcggtg gccaggtccc ctggcaccca cttagtcctc agtctgcacc 720
   taaaagcaag ccttccgagg gtcttgcctt gcaggtgtag ctcccacccc ccacgctggg 780
   gtggtccggg gaccctctgt ttcctcagtg gtggggaagg gacccgtggg ttcgtgggtg 840
   ctgaccgacg aatgtgcgga ggagggcgcg ccccagcatg gggggcgggg gtgcttctta 900
   ggatggcttg gaggggggcg cctgcagggc tggacggcgg acgggggaca ctgcactttc 960
   tgatgcggtc ggggaagccg ggagtcagcc tactggagtc cgctgccatc cctggccaga 1020
   agcagccagc gccaccccaa gctccccagc ccctcgcccg tgccggggcc gccggggtcc 1080
   gagttagggg agagtggggg gcggccaccg ctgcaccgcg cggccttcga aggactgccc 1140
   tgcggcgccg cgttcccccg aggccacccc ctcccgccgt caacactccc cggggcgggg 1200
   gctcacctgt gtggtctggg tcgcctccga ggccgagtcc ctcgttgcca agcccagcag 1260
   gcctcgctcc cccgtgactg caccgccggc ccgctccagc ttcccgagta ccccacctgt 1320
   ctccccggct tccttccggc cctttgtgtt ttcttctctt gctccgcccc ctcgcctgcc 1380
   cgctaagcac ttaagctggt gaagtcaccc tactgccacc tggcagtcac tttcgctgtt 1440
   ggtggcaacc caggctggaa cccagctctt gccggaaccc ctgccccatc cttgaggctt 1500
   gtcccgacac cgagtaggtt ctagttcctt tgtgccttcc aaatgactcc cttcctccag 1560
   caatcacagc cgcaacagcc atgtattgcg cccccgccct aggccaggcg tgggcatcac 1620
   ctttgcactc ctttctgcag ccgtgtgtgg tgcccactgt gacctgcact tcacagacgg 1680
   gtcatctgag ttgtgcaggc cactaaggga gcctggagtc aaccccaggc ctgcttggct 1740
   cccaggcctt ctctttccat tgctctttgt cggtcccctg ctgcctcctg tccctcccca 1800
   actctgactt gtggagggaa gcctttgaca tgtgggggta cctttaatac cgatatcact 1860
   tgttaccgga gaggggtccc gatccagacc ccaagcgagg gttcttggac ttctcgcaag 1920
   aaagaattcg ggtgagtccg gtagagtgag agcaagttta ttaaggaagt atagaaaaca 1980
   ggccgggtgc ggtggctcac g 2001
<210> 208
   <211> 2001
   <212> DNA
   <213> Homo sapiens
<400> 208
   tgaaggatgg ggaaatgatt ccctatatac tttgctttta ctaacaatca tgtatgtata 60
   tatataaatc atacaaggaa ttctacagat ttccaggcag attttattta gccagggaat 120
   gacctaagaa gtgcatctct gttttcatct gcagaggcta aaagtttatt gtgatcctca 180
   agtcactaca tccctaatac tcagagaatt gacaggaatg aaaagctaaa agatctagga 240
   agttgcaaac gttacctgct gatggaaatc tgttgtctgg cttcgtttcg gcagcaaagt 300
   gtgggcacac catcaggggt gctagattta ttccatcggg attgcggaag tcagctccag 360
   tgatcggctg gaaaatttgg gtaagaggag gaggcgggga tagaaatgct gagagaagga 420
   agggtaggga ggagggaagg aggcagtgga tttgcagtgt ttctgtgtgt atgcagcatg 480
   tgtgccaggg tagggaatta ctagctattt agtctcacaa gactgtcttt ggtggcaaat 540
   aacaagacct caatatctat tgactcaaat aggagcttac tttctctttt ggtgagtaaa 600
   aggaaggaca cagccctagt ctctcccatc cttcttcttc aatatcttta ttttaatatt 660
   gggggattat atcttttcct acccctttcc tgttaggatt tacctctcca acattgaaac 720
   ctattttcat atttgtgtct gactctcacc tctgggatgc ggtaatgaac ataaaaggct 780
   ataaaagaat aatgtaatgg aagccatagg ctccagaatt ctttgtattt ttataagctt 840
   gttggttgca agttgtcatt acttccaggc agaatagttt tcccagccta atgtggtttt 900
   gctgaaggag gatatgccca gaaagtccag atgaaactgt ctttaaaagt aaccaaaaac 960
   agcagtcctg gttatttatg acagcacttg aatcaatgcc gtaagttctg atggactcac 1020
   atgtgactct gttgctaaaa ctctcaggtg gtgggatgcc cgataaacat catctgtctc 1080
   aattaaaatc cccaatctag gacaagtcac atgagcttct ccaaatgctt ccccaacatc 1140
   caagcatgtg acagatcata agaatctagc tgattcctca aaccacaccc caaacttcat 1200
   tttcttccat aaacagatgt ggggaaggag aaaaaaaggc ttcttggaat agaaaggata 1260
   ataaatggga tctgtaatag actcagtttc tagctggatt ccatgcataa accctcaaaa 1320
   atacaggctt atgcctctgg attttaagag ctgctaacag gctaggcgca gtggttcacg 1380
   cctgtaatcc caggcctttg ggaggccaaa gcatgtggat cacctaaggt caggagttcg 1440
   agactagcct ggccaacata tagtgaaacc ccatctctac taaaaaaaaa aaacacaaaa 1500
   attagctggg catggtggca cacacctgta gtgccagcta cttgggaagc tgaggcagga 1560
   gaattgcttg aacccaaggg gcagaggttg cagtgagcca agatcgtgcc actgcactcc 1620
   agcctgggta acagagcgag actccatctc tcacacacac acacacacac acacacaaaa 1680
   aaaaaaaaaa aaaaaaaagt tgctaatgaa ccaacatagt ctcctagagg gaaaccaggt     1740
   ccagacttga acagtttaaa tttcactcat tttaatccag aaaaggacat actgggggaa     1800
   ggcatccaga tgtgccctag tggttttcaa gtcccgactc ttcccctctc tcccaagaac     1860
   ccaggcaacg cccaccccaa aacccccacc tcatggtcag agcattcctg ctgatgttac     1920
   tcagaatgtg tcaggatcag gaggacaagt gaggggggag gcgtgcaatg tgatcagaac      1980
   cctatgattt ctgattcctg a                                               2001
<210> 209
   <211> 2001
   <212> DNA
   <213> Homo sapiens
<400> 209
   tattcctatg tttccaaata gaaatgaaaa ttctgaaatt tcagataatt cccccctcca      60
   aatcccaatt ctcaatttag cttttctgag ccttgtccct accgtgaaat cttactagct     120
   caccccgaaa cgccggggtc ttggtcctct gggggactag tgagtcctgc tcctctcggc     180
   tttgctccgt ccctgccccg gcccctccgc gggagtctgg ggtcgcggcc cggtcaggcg     240
   ccaagccggc aggggggcgc ggtccacttg aggccacagc tccccaggtc cagggctcgg     300
   gccatccgcg ctgtcccttc cgcgggctct tgctgttctt cgccaggagg ctttgcactc     360
   cgggccgcat gggtcctgga taaggacctc aagaggttgt agtggcgccc cttcgctccg     420
   gcgtactgtc tgaaccctgt gcccaaagag ggctgtggca gttcctgcac caaccgcctg     480
   gagcccatac ctaagccctc tgggcacgag ggacctcctc tcccccgctc ccccctcccc     540
   ccatcccaac tccagcccca aaggaagcaa tgcgcgcgtc cgagagcagg agcgcgcgtg     600
   gctgaggaaa gaaagggaag gcaaccgggc agcccaggcc ccgccccgcc gctcccccac     660
   ccgtgcgctt ataaagcaca ggaaccagag ctggccactc agtggtttct tggtgacact     720
   ggatagaaca gctcaagcct tgccacttcg ggcttctcac tgcagctggg cttggacttc     780
   ggagttttgc cattgccagt gggacgtctg agactttctc cttcaagtac ttggcagatc     840
   actctcttag caggtaggtg ccgcagaccc tgcgggttaa gaggtggggt ggggggcagt     900
   gcttgccaag gccctaaact gggagcgctg ggtgagggga acaacccact ttggagggtt     960
   ctctgagaga tagatacacc ccatatcctg ggcccagctc gtgcacacag ctggaggtcc    1020
   agagacccag tcccctctgc tccgtcagcc aagttccaag aagttgagca gagaccctct    1080
   gggagcctgg cggggtgcag cggcctcccc tgcggggcct gtcacccggc cggcgcgtgc    1140
   aaacgcctct ggcgcctctc tgcgcggagg gagataagcg tctgagccag ggaaagcgcg    1200
   ggctaaaccc gcctcgccgg ggcccctgcc cgccctccgt gccccgcccg ggcggtgcag    1260
   ctggcccggg tgctcacgct cgactctctt tcttcttttc cagggtctgc gcttcgcagc    1320
   cgggatgaag ctggtttccg tcgccctgat gtacctgggt tcgctcgcct tcctaggcgc    1380
   tgacaccgct cggttggatg tcgcgtcgga gtttcgaaag aagtgagtcc gggcagcgcc     1440
   ttcccccttg ctggtacctg gcaggcaagg ggaactgacc gttggtcccg aaggtctaga    1500
   agtgaatggg agcagggaca ggcctgggcg tcacctgaac gcacgcgaat cgggtctgct     1560
   tgtgttttcc aggtggaata agtgggctct gagtcgtggg aagagggaac tgcggatgtc     1620
   cagcagctac cccaccgggc tcgctgacgt gaaggccggg cctgcccaga cccttattcg     1680
   gccccaggac atgaagggtg cctctcgaag ccccgaagac aggtaactac gccctgtgct     1740
   gtccagggac gggagggaag gaaggtgtgc gggaggagtt ctctgtctcc actcccctgg     1800
   cccgggggat cgtcggggct ggaccgcagc tcagatggcg cgagcagttt ccagctccct     1860
   ctggctctag aatggctccc gttcccggtg ttggggccaa agctctgctt gatggggtct     1920
   caagttgcct ttcttccccc tccccccgcc cgcagcagtc cggatgccgc ccgcatccga     1980
   gtcaagcgct accgccagag c                                               2001
<210> 210
   <211> 2001
   <212> DNA
   <213> Homo sapiens
<400> 210
   tatgaaaaac atagtgaagt gaaagcaaac atctgtccag aagggaatag aacaataaaa        60
   atgcccaata ataaatttaa tttgaatgtg aagaactaat atgaagcaaa taacacagtc       120
   attgtgaggg atacataaaa agacttgagg aaatgaggag tggctttttc ttggatagag       180
   aaattctaaa atagaaagat agatattgtc cctaagttaa gatgtaattt aaaaataata       240
   cctagtgtct cacctggaat attttagcac ctgatcaaat gattctgaag ttcatctgaa       300
   agacaaatgt gtcagagtag acagaaaagg aaaaagcaat gagggagggc aagcattatt       360
   tcgcattaca aatataatta agctacagca attaaaatag tatactagtg gtacaggtac       420
   aaacgactca atggaagaaa attaaaataa gagagttaca tacaaaattg agtggggatg       480
   taggtgatga taaaagagcc acttcaaaac agtgggtgaa acagggattc aacaaatgat       540
   gtagagtccc taccacttaa ttttgttcag atggatcaat tttttttttg tttgtttgtt       600
   ttgttttttt tttttttttg agatgaggtc tcactttgtt gcccaggctg gagtgcagtg       660
   gcacgatctc ggctcactgc aacttccacc tcccgggttc aagcaattat ccagcctcag       720
   cctcccgagt agctgagact acaggcgcgt gccactatgc ccggctaatt ttttgaattt       780
   ttagtagaga tggggtttca ccatgttagc caggatgatc tcgatctact gacctcgtga       840
   tctgcccgct tgggcctccc aaagggctgg gattacaggc gtgagccact gcgcctggcc       900
   cggatcaatg attttgaatg tacaatcatg caacaaaaac tatgaatgtc ttatcatatt       960
   ggagtgggta agatcattgc aagcatgaca gcagactcgc gaaggcacaa gaaaatgatt      1020
   tatgaaagca gatataaaat gggtattaac atatataata tttatttcta tgatggcgaa      1080
   tttgtccgaa acacattgtt ttcttagagt aacctgggcc tcatattctc caacgccatt 1140
   ggaagtgaaa ggaaggttgg gcgggggaaa aagaaaagtg ctttgtaagt tcattttcgt 1200
   tgtcagaaga gcttgtggtt taggttctcc acagaggcag gaaaccttca gtcacgtggc 1260
   aaatacccca ctgagtgcgc atgctgcaca gatctgttgc tgcgcatgcg cctttctgcc 1320
   caccttctgt ctaggcagag ctctgcaagg agaggttgtg tcttcgttct ttccgccatc 1380
   ttcgttcttt ccaacatctt cgttctttct cactgaccga gactcagccg gtaggtctgc 1440
   agagtggtct tcctggtaat ttagttgtga gtgaatgtgt ggaggagcca gcgggcttag 1500
   gacaggtccc gtggcacagt ccgtggcttt gagggaaaag ggcctcgcgg tggtcctccg 1560
   ccttccccca ggtcctgatg caggcgccat gggccggtaa tcgtggctgg gctggaacga 1620
   gggaggaagg taggccgtgg agggggtaga tcgcctgaag atggtgcgag tcctgggggt 1680
   gggtgctgtt agaggtgtct gagtcccgaa aacgcctgga actctctgag ggaggacagt 1740
   ttctggactc ttcgttaggg atgcgagaag ggatggtgtg ggtggtaagg aaggggccag 1800
   gaaactggga acgctgtggg ctggtgacta cggccccgag gtctgtagag tgcctggcag 1860
   aggtgtccag tgaggaacat aaccttaact ctgtgtcgca acttctcatc tcagccatgg 1920
   acctcatgga aaggaatggt tgaggctgtg cattccaaca aaacttgatt tttcggggag 1980
   tggggaggag gtggtggtgg c 2001
<210> 211
   <211> 2001
   <212> DNA
   <213> Homo sapiens
<400> 211
   cccaaacatg ctttactgga acttacgccc tggcataagg gccacaacaa aagagagagc      60
   gaattctctg gcttgctgac tccttggaaa aaaccggtag ggattttttt attaggcaaa     120
   gcacaggaat tgacgtcaga ggcaggatgt gctgctgggc aaagcatacg agaagtgggg     180
   tatgcaggtc agcattactt ggttgcaatg gttatcttga ggaatgggcc aactggtggt     240
   ctggccagtg gcaacaaggc tgtaaatcaa ttattcagca ttccttccca aggtgggaca     300
   cccggcaaca ttgtttatct cctaaggcca gttcctggaa ttaagtgaaa ggatgactaa     360
   tggacatgtt gtcagtgagg tagtggtgtg ggttttgtga ccagtgggaa tgcacgaaag     420
   aatgctttag cggggagtga gctgaagcca agccccatcc ctactctgtc tcaaagtgag     480
   ttcagaaaag gggatttaaa gaattctttt tttttttttt tttttttttg agacagagtc     540
   ttgctctgtc gcccaggctg gagtgcagtg gcgccatctt ggctcactgc aagctccgcc     600
   ccccgggttc atgccattct cctgcctcag cctcccaagt agctgggact gcaggtgcct     660
   accaccaagc ccagctaatt ttttgtattt tttttttagt agagacgggg tttcaccatg     720
   ttagccagga tggtctcgat ctcctgacct cgtgatctgc ccgccttagc ctcccaaagt     780
   gctgggatta caggcatgag cctccgcccc cggccttaaa taattcttaa aggaagtaaa      840
   gttaactttg aaagaactat caggatttgg attgactgaa aggagtgggg aagcttaggg      900
   aggaggtgct tgccagacac tgggtcatgg cagtggtcgg tgaagctgca gttgcctagg      960
   gcagggatgg agagagagtc tgggcatgag gagagggtct cgggatgttt ggctggacta     1020
   gattttacag aaagccttat ccaggctttt aaaattactc tttccagact tcatctgaga     1080
   ctccttcttc agccaacatt ccttagccct gaatacattt cctatcctca tctttccctt     1140
   cttttttttc ctttctttta catgtttaaa tttaaaccat tcttcgtgac cccttttctt     1200
   gggagattca tggcaagaac gagaagaatg atggtgcttg ttaggggatg tcctgtctct     1260
   ctgaactttg gggtcctatg cattaaataa ttttcctgac gagctcaagt gctccctctg     1320
   gtctacaatc cctggcggct ggccttcatc ccttgggcaa gcattgcata cagctcatgg     1380
   ccctccctct accataccct ccacccccgt tcgcctaagc tcccttctcc gggaatttca     1440
   tcatttccta gaacagccag aacatttgtg gtctatttct ctgttagtgt ttaaccaacc     1500
   atctgttcta aaagaagggc tgaactgatg gaaggaatgc tgttagcctg agactcagga     1560
   agacaacttc tgcagggtca ctccctggct tctggaggaa agagaaggag ggcagtgctc     1620
   cagtggtaca gaagtgagac ataatggaat caggcttcac ctccaaggac acctatctaa     1680
   gccattttaa ccctcgggat tacctagaaa aatattacaa gtttggttct aggcactctg     1740
   cagaaagcca gattcttaag caccttctga aaaatctttt caagatattc tgcctaggta     1800
   agtctgttgt ctgcatgtct ccccactaat gtgagtcata tagatggagt ctcagggcac     1860
   gactgggttt tgtgtctctc gttgttgctt cacagccctt ttggcatcac ccatttattt     1920
   aactaggata aaaacgaata ttggtatagc gattccacag tttacaaagt gcttttgtat     1980
   ccactgtctc acttgatcaa g                                               2001
<210> 212
   <211> 2001
   <212> DNA
   <213> Homo sapiens
<400> 212
   gaatgggtag aataaaagtt tgaaattttc cacttcactt ctttgcacaa tctgaggcag      60
   cctctgaaaa cacgatgcca agagccctag gtaatagcag gacaccagga aaatttggtt     120
   ggttaaggca gtatgactcc agagttctgc taataacaac ctgaaaccac catagtggca     180
   gaggaattac attttttaaa aaaaaaaatt ctttcaacag aacacaaaga ctgaaatagg     240
   aggtcactac aaccgcggga gctgccgccc cgccctgcag ggagcacctg gcctgggacc     300
   cgcaggcatt ctctacaagg ggtgcagctg tgcaaatgct cacaggtgac agaaacagag     360
   catctcctgc ccatcacttc atccaacagc cagaggtgac gaagacgacc ctcctgagtg     420
   aggactgagg gtccacaccg cccccccacc ccacacacca tagagggacc acagaatcca     480
   gctcagcccc tcttgtcagc cctggtaaac gcaggcagtg atgtcaccca gaccacaccc 540
   cttcccccaa tgccacttca gggggactca gagtcagaga cttggtctga ggggagcaga 600
   agcaatctgc agaggatggc ggtccaggct cagccaggca tcaacttcag gaccctgagg 660
   gatgaccgaa ggccccgccc acccaccccc aactcccccg accccaccag gatctacagc 720
   ctcaggaccc ccgtcccaat ccttacccct tgccccatca ccatcttcat gcttacctcc 780
   acccccatcc gatccccatc caggcagaat ccagttccac ccctgcccgg aacccagggt 840
   agtaccgttg ccaggatgtg acgccactga cttgcgcatt ggaggtcaga agaccgcgag 900
   attctcgccc tgagcaacga gcgacggcct gacgtcggcg gagggaagcc ggcccaggct 960
   cggtgaggag gcaaggtaag acgctgaggg aggactgagg cgggcctcac ctcagacaga 1020
   gggcctcaaa taatccagtg ctgcctctgc tgccgggcct gggccacccc gcaggggaag 1080
   acttccaggc tgggtcgcca ctacctcacc ccgccgaccc ccgccgcttt agccacgggg 1140
   aactctgggg acagagctta atgtggccag ggcagggctg gttagaagag gtcagggccc 1200
   acgctgtggc aggaatcaag gtcaggaccc cgagagggaa ctgagggcag cctaaccacc 1260
   accctcacca ccattcccgt cccccaacac caaccccacc cccatccccc attccccatt 1320
   cccatcccca cccccacccc tatcctggca gaatccgggc tttgcccctg gtatcaagtc 1380
   acggaagctc cgggaatggc ggccaggcac gtgagtcctg aggttcacat ctacggctaa 1440
   gggagggaag gggttcggta tcgcgagtat ggccgttggg aggcagcgaa agggcccagg 1500
   ccctcctgga agacagtgga gtcctgaggg gacccagcat gccaggacag ggggcccact 1560
   gtacccctgt ctcaaaccga ggcacctttt cattcggcta cgggaatcct agggatgcag 1620
   acccacttca gcagggggtt ggggcccagc cctgcgagga gtcatgggga ggaagaagag 1680
   ggaggactga ggggaccttg gagtccagat cagtggcaac cttgggctgg gggatgctgg 1740
   gcacagtggc caaatgtgct ctgtgctcat tgcgccttca gggtgaccag agagttgagg 1800
   gctgtggtct gaagagtggg acttcaggtc agcagaggga ggaatcccag gatctgcagg 1860
   gcccaaggtg tacccccaag gggcccctat gtggtggaca gatgcagtgg tcctaggatc 1920
   tgccaagcat ccaggtgaag agactgaggg aggattgagg gtacccctgg gacagaatgc 1980
   ggactggggg ccccataaaa a 2001
<210> 213
   <211> 2001
   <212> DNA
   <213> Homo sapiens
<400> 213
   gccaggggat ggggaaatgg agtcaggtca cagccatgcc tggaacctgc actaacgccc     60
   tccccatccc agaccacaga gagccagcca ggaaagtcat tccacaccca tgtttcagaa    120
   aacattcaag gtcagatcct ttttacctaa atcctgcttt catttgggtt tgcaccaggc    180
   tcatccccct cctccagcaa gccttcctgg atttattcac tcagtggatg ccattccact 240
   ggggcaaacc tttccggcac ctctcctgac ctaaggcaca gtcctgcccc aatcccctta 300
   ccctcctacc cagatcactc ccaagttaac cacttgagtg atccacagca ggctgtcagc 360
   cccggggact ggcttccagc tcctgcccaa gcccaaaaca ggaaatgggg ctccaaggct 420
   gaagccttga tcttaacaca ggcaaggtta aatgctgcca aaataaatga tttaaaaaaa 480
   ccctggccca ctgaggtgga agtgaccggg agataatctc ctgctgttca ttaaccatgc 540
   cgctgcctct acctcaggca gggcagggca ggcaggcagg ccactggaag gctgactcaa 600
   gtgtttcctt ccttaactta gaagctcctc ctatcctcag accagggaca caggacacag 660
   agggacagga tgggggtggg ggggtgacat ctctagatag tggagggcat cttgagagca 720
   agaggcagat gccccctgca gccagcttct acgtttagcg gggtaatcaa cattcaacct 780
   cgccctggag gaagacacct ggctttcctt cccccgtagg gcaaggctgc gcctctccac 840
   tcccctgccg gacaggcgcc cgcagttccc ctcccctttg aggaaaggga ggcaggcccg 900
   gaggttctaa gaacggcccc aagatttccc ttcttgaggt ttccatagcc ccaaagcctc 960
   tgaatctcat tcctaacttt acttgacaat ctactcctaa cgttcgccac cacttaaacc 1020
   tcccccaggc ctcccaggga ccaccagtgg ctgagcccca cacagcgcac gtttgggaag 1080
   cttgaagacg accccgaaat gcgaagaacc aacgtttctg tcggtttacg cccttaagca 1140
   cgaggcaagg gctcactggc ccggctgaag ttcggggtcc tgatccccca ccagccccgc 1200
   cctgttaggc ctttagccgt ttctgtgccc ccgcgccccc tactcactgc gtgtgctggg 1260
   cgcggcctgc gccgaagacc tcgtccagcg ccaccgtggc ctggcccagg aacttgtcga 1320
   cgccgatgag cgagcggtgc atggtggtga gcaccagctc gcaggcggcg gcggagctcg 1380
   cggcccaggg cgccgggccc gcgtcggcct cctgcgcccg cagcaggcca tccagggccc 1440
   ccggcggcag ctcgaaggag cactcctcac gccactcggg gcagccgtgc gtcttctcca 1500
   ccaccgacgt actgtacttc tcgcggccca cctggatcac cgtgtacgcg tcgctggtgc 1560
   tgcccgctcc cgagctcttg ccccgcagcc cgcgggcccg cagcaccgtc acctggacgt 1620
   gcgtgggcag ccagcgggaa ggccccgccg ccggctccgc gccccgcacc agggccatgg 1680
   cggagaagcg gggggcgagg tctggccgag ccggtgcaga ccccaggacc tggtgacaaa 1740
   cccggccgcg gcagaaggcg gtcaggaacc aactctgagc gccgccgcag ctgcgggctg 1800
   ggctgggccg ggccgaccgg ccgccgcctc cccgcctcac cgggccgctg gccgcgggcc 1860
   gcgccgcgac gtcagcgccc cgccccctgc cccgccccgc gcccgccgcc ccaaccggcg 1920
   aggcggtgcg acagggatgc gggcacgcag ctcgcgcccc ggcagccgca gaccagcccg 1980
   gagcgggaag gggcggtgcg c 2001
<210> 214
   <211> 2001
   <212> DNA
   <213> Homo sapiens
<400> 214
   gtttgctgtt agttatctca tacctaaagg gctattatca ttattaatat ttttattttt 60
   tgagccagag tttcgctctt gttgcccatg ctggagtgca atggcgtgat cttggctcac 120
   tgcaacccct gcctcctggg ttcaagtgat tctcctgcct cagcctccca aataactggg 180
   attacaggga tgcaccacca cacccagcta attttgtatt ttcagtagag atggggtttc 240
   accatgttgg tcaagccggt cttgaactcc tgacctcagg tgatctaccc gcctcggcct 300
   cccaaagtgc tgggattgca ggcgtgagcc accacgccca gccctattat tatttttgac 360
   aacaacagca aagatctgcc catccgactg caggaattta atacacagcc tttgtcagtg 420
   gtagaaattc tatcactgta tgactcatga gtacataaca atgtgctata ccatacttcc 480
   tctttgaggg ataaagcccc tgcactctaa gggaggattt cattatgcat ttctcttata 540
   tcaccagaaa attatttagt ggaagacagt ttaatatttg acagctttga accatttttt 600
   tttctctttc aaccagtctt tttacattgt ctgcttaaaa acaggaaacc acatatctcc 660
   accccagcac aacccaccat ccaccagcac agaccgtatc attttggatc tacagtcttt 720
   ttcttccaag cctggccagg tgcctgctcc tactttgcag gaaacccctc caggcactcc 780
   cggtgcgtgt tctcagagcc cttctgagaa tattcaggat tgcacttcct gggctgcagc 840
   gcaccctgta ccctcctttt ttaagcctcc tcccctgagt cactgctacc cttccctcct 900
   ctaacttccc cttctctgca gcctccttag caatttctta tcataaagtt tgttttaaag 960
   aagaactgcc cctctaaaaa aataataaac tgtcgagggc ggtagccccg tctagaggtt 1020
   tccgactttg ctctgtgccc acctgaagcc tccccagccc gaggtatttg aacttacatt 1080
   cagtgcttca cgtctctgtc cggggtgcct ctgtctctca actctgactt ctgagtactg 1140
   gcagttagaa caggaaggaa gtcaatgaga aaaaaaaatg ttttagacct acttgaagag 1200
   gaaaccctgg gctgtctcaa atcctgttcc tttgcactgc ctatctcttg atcagccaac 1260
   ttgagcagca ttcttgtcaa ttaatggcac atgtgcgagc atgacagccc gtgtgacgtg 1320
   gagatgcatg aatgtacacg caagagtgtg gagcctatag ctatgggtat tatgaataat 1380
   tgtgagggta tttctgataa tcaggtcatt gcagtgcata agcctatgga ggtaaggagc 1440
   ctgaaaccag tagcaataaa ttcacatcgg ctttactttg taccactcac ttctgttccc 1500
   actgggtccc cgccagcttc attatcaacc agatacgttg aaagtttgct ggcagtgaat 1560
   gtagagtgct aagaaatgat gattgttaac tgcatatact acggtcctaa atttaaaaat 1620
   gtgtccttaa acagattttt ggagttttgt tggttataat ttcaagacgt agctttgtat 1680
   tgcactacaa acagtcaaat attgttttta atagttttgg ctgatttttc ttcaaatgaa 1740
   acaaacaagc aagcgaaagt actgtctttg atgaaaccta ggaaagaaat tacaaaagga 1800
   agtaaaactt cattaattca tagtcttcca actcagaatt tgtgacaact taacaagtgc 1860
   caaaatagac tttgtaccat atttgaggaa atggtttatg aagtaattta aatgggaaca 1920
   agtctcggag gccatgttta aaccatctga ggaacagttt ttaagcagcc aagtcacatt 1980
   catgctgtat attcccgtag c 2001
<210> 215
   <211> 2001
   <212> DNA
   <213> Homo sapiens
<400> 215
   tatttagtgt gtgttctcag cctcaccatt gacttgctgt gttaccttgg acaagtcact 60
   ctcccactct gacctcaatt ttcacatctc taaaaggaga ctgctagact agacggtgag 120
   tgaagtcctg ctgagccctc cgagttcatt ctcccttgtc cagtcagtcc ttccctccca 180
   ccctcccctc tgggctccct ctctggggcc ctctccccca ggggaggcct gctccagccc 240
   caggggagct gcggccctgg ctcctgttcc cagtcggagt gaccagccct gggcctcctt 300
   cccagcttcg gccctggctc catgtggccc atctgagagg ctggcctcaa gcccggcggc 360
   aactccacat ttctgttttt cctttttttt ccctctttcc cggagttaac aagaagcaga 420
   tgtggcgcac gatggttgga gaggtggggg gaggaagggg gaggccggac cgccagccag 480
   acagggggga agggagggga gccagcaggg aggaggaggc cagggcccgc cccacagcca 540
   ctctcgcgcc tccgaacagc cacaggggca aagccctgtc acccccagga tccggtcatc 600
   agggaaagag gacagggaga ccagaagagg gccagctggg acgagggggc ggacgcccag 660
   gaggcaactt ctgagacgca gctcctgaga ggggcaggga ccaggcgcgg gaggccagag 720
   ggggcacaga gaacaaaccc cctcagaagt gaagaggaga gcggaaggaa ccgagagggg 780
   acggacagga gctgaggagg aaagaggagg ggagaggggt caggccaggc agccaaggag 840
   aagacgtgtg gccgggggct atcagaagga aactgggacg gacgggccgg gctcgggctg 900
   tcctgtggag cagcagcatc cccggggccg gcagaggcgc cagtggctgg gcgggatgag 960
   tctctgaggg ccactgtgga gcgccccgcc atggcccccc gcaccctctg gagctgctac 1020
   ctctgctgcc tgctgacggc agctgcaggg gccgccagct accctcctcg aggtttcagc 1080
   ctctacacag gttccagtgg ggccctcagc cccggggggc cccaggccca gattgccccc 1140
   cggccagcca gccgccacag gtaagagtct ggatcccagc ccgaggcttg ggtggtgagg 1200
   aaggggtcag aatgccacct ctgcctggct ctctgctgtg tccgctaatg cagggcagag 1260
   ggggagtgag aagcccatcc atcaggtggc tcctcacatg cccaccttcc cacggggcct 1320
   tttccaggac cccctgaaca catatgttcc ttgggtcacc tagggggtcc gctcccacat 1380
   gccccaggga agctgccctg aggggagcgg gccaggcctt cctgccagct gccctctgta 1440
   tcctagcccc agagcctcag ggagaacttt tgtccccttc tagggctccc ttgaccccat 1500
   ccagtaatcc ataattctga caccactcca gctctgttcc ctccagccct ccagccagac 1560
   acagatcaga ggccggagtg aatggggcca ggagtataga actgcaatcc ccaagagggg 1620
   caggctcatc aatggcttat tctctactta accccaagct ggcctgcccg tcttctcctg 1680
   gaggggtggg gtggaagcca gtgttcagat cacccacagg gcccgggctg gctatgcgca 1740
   cacagcatgc caatctgggg actgagacag gcccagaaag ggacacacat gattgtgtac 1800
   attcagaggc tccccgggac cccaccacca tgaggattga ctgctctgtg gttttccatt 1860
   ctgcgcgtct ccccatcttg aaaagtcttt atctgctcct gctacacagg aaagcgtggg 1920
   ggatagggag ggtggcgatg ccaaagccct tgctctcact gatccccaag ggttctgcat 1980
   ctttggagcc ttcttcagcc c 2001
<210> 216
   <211> 2001
   <212> DNA
   <213> Homo sapiens
<400> 216
   ttttgaatta tttgccatga aagcctttcg gtgtcccctc catccccccc aaaaaaaaga      60
   aagagaaaaa gaaaaaaggc cctagcacta ctagaactca agcagaaatt cacattctcc     120
   cagcactcac gtaggataag aaatagaatt ctaccccaag tccaaaaaag agtctggccc     180
   aatatcaaaa tagactttac ctccaaagca gtttgatatt tattaaacac tacctttttc      240
   tcttcattaa catctcttgc ctcctggtaa tttgcccttt acttttcctc tgtttctcca     300
   gctttgaaat cacacgccat tttttttaag acaatgtaac aatgtgcaag atattttttg      360
   ttgttgtttt ttgcactttt gacataccct atccccaaac acaccccaaa ccaggacact     420
   gtggaattaa tacagtttgg aaattgggta tagtttagtg actcagtgtt agttaagatg     480
   ctggcaacag atgttggcaa aggctatgga atgagtccca agaatgacaa accttacatt     540
   actccaaagg tttgaaatcc aggatttctc tggttaatat ggttaatagt tccactgctg     600
   gttttaattg tcttagagtg gaaggaggtg tcatattttc aggccctagt aatctcttcc     660
   ttcagaattc ttctgaataa aaagcttagc aacaaaatca ctagagttag agtgctatgc     720
   cttcttgatt taatactaaa acatggaaca aatgaaacca ctctggcctt tttccaacct     780
   ctacagatcc ctcattctta tacttctagt ccgtcttttc ttcctcataa caccccaatt     840
   ccctttttgc tctttctttt cctcctcaaa tcatatctct gcattgtctc gctatgtggc     900
   cacccttgat ctccagtgac ctccaaaggg caaaacgccc ataacaagac aacaactgca     960
   gtaacaatcc agtccaaaag tatttgccaa gagtttattc cgcggttagc accaaactct    1020
   ccatctattt tgccactgca aacagtgaac ccatagttcc ccaaaacaat catagttcct    1080
   catgcttcac tcttggcagg gctgaggaca ggaaagaagt gaaaataaaa tcttccatta    1140
   ctaccctcag tctagccaga agtatacccc accacacaag gagatggcag ccaagtcttc    1200
   actccctcag agtttctcct caggctcctc actcacctaa ctcttccatt gtcctacatc    1260
   caagatgctc ttgtctcaca ggccgtgagg aacagctttg gcatgtgctg aagaagagga 1320
   ccgcaaagga actggatgag acagcataag gcagcctcta taaacctttt tggttcctgt 1380
   atttgcttag cacttcctct cttttgattt ggtgagcttt tttttttttt tcataggtta 1440
   cacagtttta aaaatgtgac tgacatagaa aggtagatag taagatttgc attatggcag 1500
   tgcattggcc agggtgataa agattcaaaa ttaattttgc aaaatttgag gacctactct 1560
   gtgccaggat tcatcttgga ttcatgggaa tggcagactt atacttgcgt tttttccaag 1620
   tgtaggtacc ccactctgag catctttctt ttttcttttt tttttttctg tgaagctggg 1680
   agacacagag gcatagtctc cttcttaaaa tttgcatcag agttgaccag catccatttc 1740
   attttgttgt cttatttatt cagaaattat ggttatctgc tttcctgcat ggaacgtaaa 1800
   tccttaaaag tagggcagaa gtggattcat gcttggattc tcagggcctg gtttactcag 1860
   tggtcccaaa agcaaacatc tatagggtgg tcacaaagaa ggaagccagg tttgacacca 1920
   gatgttaaac tgtgtgcatc tcaattaagc ataatccctg cctagataca ctgccttata 1980
   gcaatttctt tggaaagtaa c 2001
<210> 217
   <211> 2001
   <212> DNA
   <213> Homo sapiens
<400> 217
   ggagtgggct gcatgtgccg cgcggggagc ccgcccgact tcggggaggc agcggggcgc 60
   cgtggccccg cggctcagct ccctgccggg tcgggtgtcc cagccctgcg acttccgggg 120
   cccggcggct cctcggggag ctcgtccaca ggcagcctcc ggggcgagag agcgcgcggc 180
   gcgggcgccg ggacggtccg cggcacgcgg gtggagccgc cggcgctcgg gctcccgcag 240
   gggcggcggg cggggcgggg tgaggcgggc cggccgccct cccctctcct ccctcccgca 300
   gcccgccctc ccttcctccg gtcccgcagc caattaggcg gccccctcgg gcgggaggcg 360
   tggggcgctg cataaagcgg cggggagctg ccaccccggg agcgggctgc gcagtgtccg 420
   ggccgagccg gtgcgccgca gactagggcg cctcgggcca gggagcgcgg aggagccatg 480
   gccaccgcta acggggccgt ggaaaacggg cagccggaca ggaagccgcc ggccctgccg 540
   cgccccatcc gcaacctgga ggtcaagttc accaaggtga ggcgggcgcc cctcccaccc 600
   gacggccgcg ggcccctgcg ctgggcagcc agactcgggg cggcgggggc gagggagcag 660
   cgggccgggg gtccgccggg cgccgccgag acccgagcct ccctgcccgg gcgcggctct 720
   ccagaaaccg cacctttcgc gggacgtctc gggcgggatc gcaggcggcg gggctcggcg 780
   ctgtgagcct cgaagccggg aaaccgaggc cgtccagcgc ccgcgcgggg ccgggccgcc 840
   cgcatccctg cgaggccccg acgtgtccct gcgctgcccg ctttgatcgc gagtggaggt 900
   gcgcgccgcg ttgactcgga gcgcgctggt tttggggagc tccctcccca aaaacccctg 960
   cgatgatttc cagggtggac gagtctggct ttgagagggt cggagggtcc gagagcgcca      1020
   agagagagtc cgggaggcag atggcggagc tgcagttgga agactgggga agcgggtcct      1080
   ccgagcaaag cgcagcgggc gccttccgtg cttagtttat acccggctca gcagcagaac      1140
   gaggaggagt aggaaggtgc tcgcttactg acgaaacctc cccggagccc agaggtcccg      1200
   gtgtttacac acaaggcgcg cgctccggaa ggtcgcagac ctgggccttt gggaagcggt      1260
   gggtcgcgag gccgcagaag cgcacggttc tctgcctcct cgctctgtgg gctgggaggc      1320
   tacagcgttg gcagaacggc gagattacct gcccggggca gaaaggaata aaagaagatg      1380
   gtgaggcctc cgaggcctct gagctggggc tgaggagggc cgagagaccc gggtccggga      1440
   gcgcgacccg atggaggaca cccccaggct gtctggcgtg cgcgtctgtc gtgggccggt      1500
   gctggggcac tttagaggca caggccaggc cgagagccat tgccttaact ttccccacct      1560
   ttggcttctc ttgatttttg cttttaccca tagcccccaa ctccagctga caccgcactt      1620
   agagaatttc ctcatccgtt ttactttaag aagtagcaat tatctttctc aaagaatttt      1680
   aatcaagcgg agaaagttgc agggcactat gtgtggcctt cagatttggg tctcttggca      1740
   cttaattccc aatgtcggct gagcctctga tcacaagaca tttaaattaa ctgttttcag      1800
   aaggctctgt aatcaacaaa gacacgtcgc tgtaaactaa acgctggaaa ttattcttaa      1860
   tgtagtttta tttgtaatat cagcttcaga gtgaaaattc caacacgaat tctgggaaga      1920
   gtgaaaatgg tacccgaatt ttgtaagacc ctaaccccag gtgcttggct gctgctactg      1980
   atggttaagt ggggaggttg t                                                2001
<210> 218
   <211> 2001
   <212> DNA
   <213> Homo sapiens
<400> 218
   aggcaggaga atcacttgaa cccgggaggc gtgggttgca gtgagccgag atcgcgccgt 60
   ggcactccag cctgggcggc cagagtgaaa ctccgtttca aaaaaataaa ttaataaatt 120
   aaattaaagg tatgtgaatg caatctctcg ctgacaaaat atctttatgt actgtagatc 180
   ttggcaacct tagcatacga ttttgttttc tttccttatt atgttaaaaa ctttcccatt 240
   ttcacttaaa ggaagcaagc actttacagc ttctttttgg catatttcaa tcgccagcat 300
   tgctaccctt gtactttggg gccattatta agtaccataa gggttaattg aacacaagca 360
   ctgagatacc accaggacag tcactctgat aactgcgacc gctcctaagt gactaaggta 420
   cagagagcgc agccagccaa cagcatgggg actcaggaca aagggaggat taccatccca 480
   gggcagggct gactcaagag cttatcacac tactcagaag ggctggcgac ttaaaattta 540
   tgtattgcct atttctggaa ttttccaatt aatattttgg aacctcaggt aactgaaagc 600
   acagaaagga aaaccctgga taagggaaga ctactgtagt tggagcagag aattgcaata 660
   cctaaaatat ttgccatcta gtctttcaca gaacaagttt gccagctcta gttttagagg      720
   agcaaaatga aagatagcaa atttctcagc tcaccagttc ctaaagttgc gagtgcatcc      780
   agatgattca gtgcagctca gtatgttcca ctctgggcca caagggaaag cgaagctgga      840
   gcttccttgt ctccacctcc gggagaaatt tgtccagaga ccggcagcac tgcttccgca      900
   ggcgaacaac gccgcttgct cttcaaagtc ctttctgagg ccagatcaga aggtcccact      960
   ccttttaagc cccttattgc ccaatcattt ttccagtcac gtgtctgaag cagtttatcc     1020
   tgattggatt ttcagttgtt gtccagcttg attgccaaaa gttttcctcc aattgctact     1080
   caaaggaaag aatgagggtg gggcttcagg gctgctttcc aggttgattg agctgctgat     1140
   gggcatcctc cctcctctct ctcttgttca gagtctacca ctttctctgc agtgtgcctg     1200
   ggccctcagc tattggggat gagaagggat gcggagtggg aggcagtcca catgtctgtt     1260
   ccaacaaagc tcagaggtga gcgagttaaa aagcagatgt ctttccagta ggagccccag     1320
   gggacagggt gggtctcaca gcccttgaag accgagctcc cgtaggagtt tccaggtgtc     1380
   caaacagggt cctcactgtg agtcttcaac agctttctcc aagcagcagc ccactcccct     1440
   cttaatccat ttccaggggt caaaggtcta tcctggttcc ctcccatgcc ctctcacttc     1500
   cttccctctg tctctccctc ctccctcctc cttcactacc ccctccctgg aaccttctca     1560
   gctgaatttc aggcccaccc tttcactctc tttacaggct ttgcaacaca gattagaaag     1620
   cgggggaggg ggagcctcaa gagtcccttg acaggtaaga aatatttata tttccacccc     1680
   cattccaacg caatgcaatc aagtctcaaa attaaaacga aaatcatttg caaattaaaa     1740
   tgttctaaag taccatctcc tgataaaact ttaaaaatac ctttctgggc cggatgtggt     1800
   gactcacatc tgtaatccca gcacttttga aggccgaggc agcagatcac atgagcccat     1860
   tagtttgaga ccagcctggc caacatgatg aaaacctgtc tcttattctt tataaagtaa     1920
   aatacaaaaa tttcctgggc gtggccgtgc gtgcctgtaa tcccaggtac tcgggaggct     1980
   gaggcagaat aatcacttga a                                               2001
<210> 219
   <211> 2001
   <212> DNA
   <213> Homo sapiens
<400> 219
   gaggctaagc cggcagatca tttgatgtca ggagttcgag aacagcctga ccaaaattgt      60
   gaaatcccat ctctgttaaa aatacaaaaa aattagccag acatagtggc acacatttgt     120
   aatctagcta ctcaggaggc tgaagcagga gaattgcttg aacccgggag gtggtggttg     180
   cagtgattcg agatccgtgc cactgcactg caacaaagca aaagcaagac tctgtctcaa     240
   aaaaaaaagg acaaggacaa ggtcagaggc aaaacaggag gaacagagga aggaagggac     300
   aaagagggac cccaatgcag actggacgag tgcagagggc agggatggaa agtcacgtcc     360
   tgagctgttt gctgttgcag aatctcagca gctgggccat aaagaaggga cttgacgctg 420
   tcccagtctc tacacttggc acccttgacc tctctcatcc cacctttccc taggccgagg 480
   tcacacctgc tttcattttg tttttttttg tttttttttt ttaagacggg gtctcgctag 540
   attgccctga cgggagtgca gtggctattc aagggcacca tgatagtgca ctgcggcctg 600
   gaattcctgg gctcaagcga tcctcccacc ccagcctctc gagttgctgg gactacaggc 660
   gtgtgcccct gcacccagct gcgcccgttt ttgtcctcct gtctggcttc tcttcatgat 720
   ccccacccct ttattcggcc tcaggggaac cccgtggtct attccgtgag accgagcaga 780
   gttttgggga gccccgactc ttggatctcc ctgctggctc acctttctct gttcggtctc 840
   caaaccagac cagcaatggc cccaggaaga aggtggcgct gggagaagtt ccctaagcta 900
   tgtatgccta cctgtcccct tcctgacttt tcggttcccc aaacccgagg ctggacctac 960
   ctttaatcag atccacgatg cgcagctcga agggaatgtc gttcttcttg gcaaagatgt 1020
   aaacagcgcg gcagggctgg gacagcaggt ccaggtacag ctccaggccc atagtgggga 1080
   ccgaccgacc tgaccggaaa ccagagggag tcagcaaact ccagtgccag gactcgggtc 1140
   tcggacctca gaccacgccc ctgcggctac gcaccccgac cgctgtgccc cattgggcgg 1200
   cacggacgcc tgggacactc tttagtccaa ttgcgggcgg cgggcggcct gtctcagggc 1260
   tgggcccgcc tggctcctgt gggatcaggg tgggtggcca agagaccgtt gtctctcagg 1320
   gggacgcaca gagaggcccc agtcccatcc ctttcgtgcc tggctcgctc atttcactta 1380
   gcataatgtc ttcaaggtgc atctgtgttg aagcgtgtgt cagaatttcc caccgtccgc 1440
   attttagagg aggagacaga cactgaatga ttatagaccc agaaagtctg gctccaaagt 1500
   ccatcctctt ggctcaacac tgcagagtcc ttggtgccag caagtggcaa agcctcaggg 1560
   cttacgttca gtgttttgtg gtcactcgct tcactgcagt cgcactccct ggaatgtcac 1620
   agccttggtc ttcctgcccc tgccagcaca gtgccatcta tcgcattccc tgggtttatt 1680
   tcttcctttc tccaggcctg tgtttctcca tctttaaaat gacagggaag ctagagccct 1740
   ccagagtcct tgcaagtcca cccccctgga ttcatctgct gagccgtaag cccaggcctg 1800
   ccccttgctg ccatggagcc tgtcaaatag tatcagggtc acatgtcatt ttctaaaacg 1860
   aatcccactg aaagtgtgtc agccaacgca taatgttttc tagagaataa ggaacatttc 1920
   tgcaaataaa gtggaagtaa caactgtttt attttattta gatagagtct cactctcttg 1980
   cccagggtag aatgcagtgg c 2001
<210> 220
   <211> 1147
   <212> DNA
   <213> Homo sapiens
<400> 220
   gggggaggag ccaagatggc cgaataggaa cagctccggt ctacagctcc cagcgtgagc 60
   gacgcagaag acgggtgatt tctgcatttc catctgaggt accgggttca tctcactagg 120
   gagtgccaga cagtgggcgc aggccactgt gtgcgcgcac cgtgcgcgag ccgaagcagg 180
   gcgaggcatt gcctcacctg ggaagcgcaa ggggtcaggg agttcccttt ccgagtcaaa 240
   gaaaggggtg acggacgcac ctggaaaatc gggtcactcc cacccgaata ttgcgctttt 300
   cagaccggct taagaaacgg cgcaccacga gactatatcc cacacctggc tcagagggtc 360
   ctacgcccac ggaatctcgc tgattgctag cacagcagtc tgagatcaaa ctgcaaggcg 420
   gcaacgaggc tgggggaggg gcgcccgcca ttgcccaggc ttgcttaggt aaacaaagca 480
   gccgggaagc tcgaactggg tggagcccac cacagctcaa ggaggcctac ctgcctctgt 540
   aggctccacc tctgggggca gggcacagac aaacaaaaag acagcagtaa cctctgcaga 600
   cttaagtgtc cctgtctgac agctttgaag agagcagtgg ttctcccagc acgcagctgg 660
   agatctgaga acgggcagac tgcctcctca agtgggtccc tgacccctga cccccgagca 720
   gcctaactgg gaggcacccc ccagcagggc acactgacac ctcacacggc agggtattcc 780
   aacagacctg cagctgaggg tcctgtctgt tagaaggaaa actaacaacc agaaaggaca 840
   tctacacgaa aacccatctg tacatcacca tcatcaaaga ccaaaagtag ataaaaccac 900
   aaagatgggg aaaaaacaga acagaaaaac tggaaactct aaaacgcaga ggccctctcc 960
   tcctccaaag gaacgcagtt cctcaccagc aacagaacaa agctggatgg agaatgattt 1020
   tgacgagctg agagaagaag gcttcagacg atcaaattac tctgagctac aggaggacat 1080
   tcaaaccaaa ggcaaagaag ttgaaaactt tgaaaaaaat ttagaagaat gtataactag 1140
   aataacc 1147
<210> 221
   <211> 20
   <212> DNA
   <213> Artificial Sequence
<220>
   <223> Synthetic Primer
<400> 221
   ttttttgagt taggtgtggg 20
<210> 222
   <211> 23
   <212> DNA
   <213> Artificial Sequence
<220>
   <223> Synthetic Primer
<400> 222
   tctcactaaa aaataccaaa caa 23
<210> 223
   <211> 13
   <212> DNA
   <213> Artificial Sequence
<220>
   <223> Synhetic Primer
<400> 223
   gggtgggagt gat 13
<210> 224
   <211> 18
   <212> DNA
   <213> Artificial Sequence
<220>
   <223> Synthetic Primer
<400> 224
   cgagcgcggc tacagctt 18
<210> 225
   <211> 20
   <212> DNA
   <213> Artificial Sequence
<220>
   <223> Synthetic Primer
<400> 225
   ccttaatgtc acgcacgatt 20
<210> 226
   <211> 18
   <212> DNA
   <213> Artificial Sequence
<220>
   <223> Synthetic Primer
<400> 226
   accaccacgg ccgagcgg 18
<210> 227
   <211> 22
   <212> DNA
   <213> Artificial Sequence
<220>
   <223> Synthetic Primer
<400> 227
   tgtcgtcgga aattggcagt at 22
<210> 228
   <211> 22
   <212> DNA
   <213> Artificial Sequence
<220>
   <223> Synthetic Primer
<400> 228
   caaagaccag ctgcaaggaa ct 22
<210> 229
   <211> 17
   <212> DNA
   <213> Artificial Sequence
<220>
   <223> Synthetic Primer
<400> 229
   tctttcctgt gatcttc 17
<210> 230
   <211> 18
   <212> DNA
   <213> Artificial Sequence
<220>
   <223> synthetic primer
<400> 230
   cgcgagtcga agtagggc 18
<210> 231
   <211> 23
   <212> DNA
   <213> Artificial Sequence
<220>
   <223> synthetic primer
<400> 231
   acccgatttt ccaaatacga ccg 23
<210> 232
   <211> 21
   <212> DNA
   <213> Artificial Sequence
<220>
   <223> synthetic primer
<400> 232
   tgtgtgtgag ttgaagtagg g 21
<210> 233
   <211> 26
   <212> DNA
   <213> Artificial Sequence
<220>
   <223> synthetic primer
<400> 233
   acccaatttt ccaaatacaa ccatca 26